(19) Europäisches Patentamt
European Patent Office
Office européen des brevets

(11) EP 2 674 161 A1

(12) EUROPEAN PATENT APPLICATION

(43) Date of publication:
**18.12.2013 Bulletin 2013/51**

(21) Application number: **13174939.2**

(22) Date of filing: **11.11.2011**

(51) Int Cl.:
*A61K 31/675* (2006.01) *A61K 31/4164* (2006.01)
*A61P 29/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.11.2010 EP 10190922**

(62) Document number(s) of the earlier application(s) in accordance with Art. 76 EPC:
**11781808.8 / 2 637 649**

(71) Applicant: **Akron Molecules GmbH**
**1030 Vienna (AT)**

(72) Inventors:
- **Penninger, Josef**
  **1130 Vienna (AT)**
- **Neely, Graham Gregory**
  **Sydney, 2015 (AU)**
- **McManus, Shane**
  **1030 Vienna (AT)**
- **Nilsson, Henrik**
  **1030 Vienna (AT)**

(74) Representative: **Sonn & Partner Patentanwälte**
**Riemergasse 14**
**1010 Wien (AT)**

Remarks:
•This application was filed on 03-07-2013 as a divisional application to the application mentioned under INID code 62.
•Claims filed after the date of receipt of the divisional application (Rule 68(4) EPC).

(54) **FMO3 inhibitors for treating pain**

(57) The present invention relates to new therapies to treat pain and related diseases, as well as pharmaceutical compounds for use in said therapies.



Printed by Jouve, 75001 PARIS (FR)

## Description

The present invention relates to the field of method of treatment of pain and the provision of pharmaceutical compounds suitable for such treatments.

Acute and chronic pain affects millions of people after injury or surgery and those suffering from diseases like arthritis, cancer, and diabetes. Nociception (the detection of noxious or damaging stimuli) serves a crucial biological purpose: it alerts living organisms to environmental dangers, inducing the sensation of pain, reflex withdrawal and complex behavioural and emotional responses, which protect the organism from further damage. Noxious stimuli are detected by specialized high threshold primary sensory neurons (nociceptors), which transfer signals to the spinal cord and then transmit them to the brain for higher level processing that results in the conscious awareness of the sensation called pain. The functional importance of pain perception is exemplified by individuals with defects in nociception; patients with congenital insensitivity to pain do not survive past their twenties.

Two basic types of pain can be distinguished - acute and chronic. Acute or nociceptive pain is generally self-limiting and serves a protective biological function by warning of ongoing tissue damage caused by noxious chemical, thermal and mechanical stimuli. Examples of nociceptive pain include: postoperative pain, pain associated with trauma, and the pain associated with arthritis. Chronic pain, on the other hand, serves no protective biological function, and reflects either poor resolution of the painful stimuli, or is itself a disease process. Chronic pain is unrelenting and not self-limiting and can persist for years and even decades after the initial injury. Chronic pain is predominantly neuropathic in nature and may involve damage either to the peripheral or central nervous systems.

It is a goal of the present invention to provide methods of treating, ameliorating or suppressing pain, in particular by the use of novel compounds for this purpose.

The present invention therefore provides the use of new classes of compounds for the treatment, prevention or reduction of pain. These compounds are given in the claims, and especially include Tenofovir (PMPA), dasatinib, AMG-706 ( motesanib ), BIRB 796 ( Doramapimod ), EKB-569 ( Pelitinib ), sorafenib , Vandetanib, CI-1033 ( Canertinib ), NSC161613, N6-Benzyladenosine-5'-phosphate, p-Aminobenzoly PAB-J acid, NSC47091, cilomilast , Nicotinamide ( Nicotinamide ), IBMX, Roflumilast, Filaminast, Piclamilast, V11294, CC-10004 ( Apremilast ), LAS31025 ( Arofylline ), CP80633 ( Atizoram ), Catramilast/Atopik ( Catramilast ), BRL-61063 ( Cimpyfylline ), Daxalipram/mesopram ( Daxalipram ), Doxofylline, Drotaverine, Efloxate, Etamiphylline, Etazolate, Etofylline, Glaucine Hydrobromide ( Broncholytine ), GRC3886 ( oglemilast ), oxtriphyllin ( Choline theophyllinate ), Pumafentrine, Revamilast, Tofimilast, Tolafentrine, Seoanin ( Trapidil ), GW 842470 (AWD 12-281), CDP-840, YM-976, CI-1018, D-4418, Lirimilast, SCH-351591, RPL-554, IPL-455903 (HT-0712), GSK256066, Zardaverine, Vardenafil, OPC-6535 ( Tetomilast ), IC485, L-826,141, ONO-6126, CI-1044, MK-0873, T-2585, R1533 (MEM-1414), Ronomilast ( ELB-353 ), UK-500,001, AN2728 , DE-103, Tofisopam, (R)-Tofisopam ( Dextofisopam ), (S)-Tofisopam ( Levotofisopam (USAN) ), EKB-568, SU-14813, LY-333531 ( Ruboxistaurin ), CGP-52421, SKI-606 ( Bosutinib ), Roscovitine, Tenofovir (PMPA), Methimazole, Adefovir dipivoxil (Bis-POM PMEA) ( Adefovir ), Acetazolamide, midostaurin ( PKC-412 ), tozasertib ( MK-0457, VX 680 ) or lestaurtinib ( CEP-701 ). A further compound is imatinib (STI-571), which is preferably used in the treatment on non-inflammatory pain, especially in the treatment of neuropathic pain. Alternative names or identification of the compounds are given in brackets. Chemical structures of some of these compounds are given as follows:

| | | |
|---|---|---|
| V11294 | -[(3-cyclopentyloxy-4-methoxyphenyl) methyl]-N-ethyl-8-propan-2-ylpurin-6-amine: | 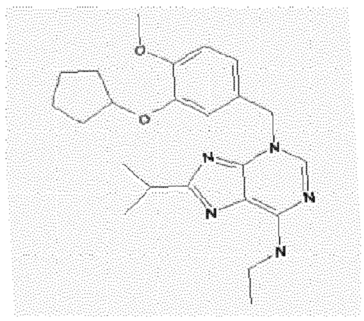 |

(continued)

| | | |
|---|---|---|
| GW 842470 (AWD 12-281) | N-(3,5-dichloropyridin-4-yl)-2-[1-[(4-fluorophenyl)methyl]-5-hydroxyindol-3-yl]-2-oxoacetamide | 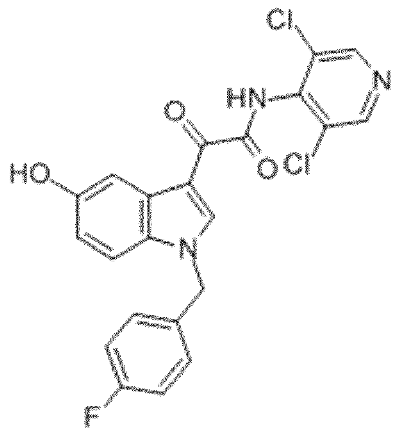 |
| CDP-840 | 4- [(2R) -2- [3-(Cyclopentyloxy)-4-methoxyphenyl]-2-phenylethyl]-pyridine hydrochloride | 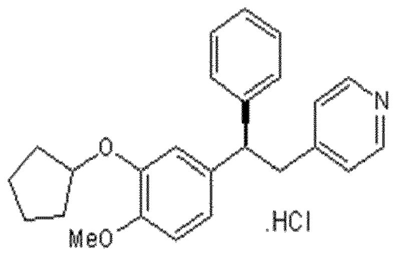 |
| YM-976 | 4-(3-Chlorophenyl)-1,7-diethylpyrido [2,3-d]pyrimidin-2(1H)-one | 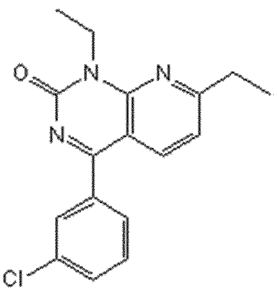 |
| CI-1018 | N-[9-Methyl-4-oxo-1-phenyl-3,4,6,7-tetrahydro-pyrrolo[3,2,1-jk][1,4] benzodiazepin-3(R)-yl]pyridine-4-carboxamide | 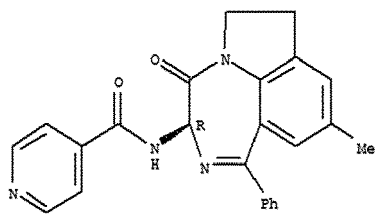 |
| D-4418 | -(3,5-Dichloro-4-pyridinyl)-8-methoxyquinoline-5-carboxamid | 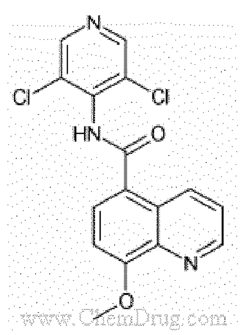 |

(continued)

| | | |
|---|---|---|
| SCH-351591 | 3,5-dichloro-4-(8-methoxy-2-(trifluorome-thyl)quinolin-5-ylcarbox-amido)pyridine-1-oxide | 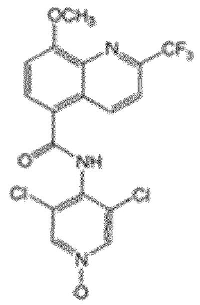 SCH 351591 |
| RPL-554 | N-{2-[(2E)-2-(mesitylimino)-9,10-dimethoxy-4-oxo-6,7-dihydro-2H-pyrimido[6,1-a]-isoquinolin-3(4H)-yl] ethyl}urea | 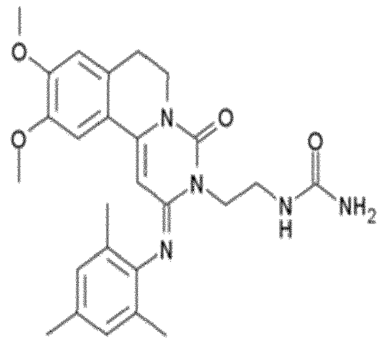 |
| IPL-455903 (HT-0712) | (3R,5R)-5-(3-(cyclopentyloxy)-4-methoxyphenyl)-3-(3-methylbenzyl) piperidin-2-one | 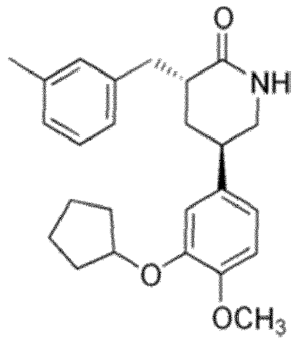 |
| GSK256066 | 6-[[3-[(Dimethyla-mino)carbonyl]phenyl] su lfonyl]-4-[(3-methoxyphenyl)amino]-8-methyl-3-quinolinecarboxamide | 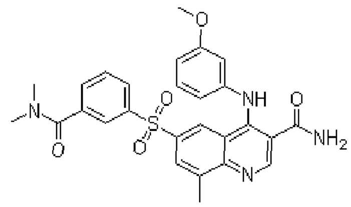 |
| OPC-6535 | 6-[2-(3,4-diethoxyphenyl)-1,3-thiazol-4-yl]pyridine-2-carboxylic acid | 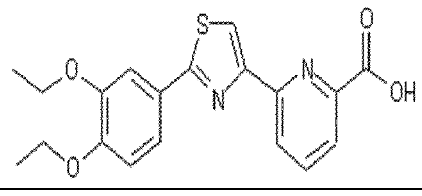 |

(continued)

| | | |
|---|---|---|
| L-826,141 | 4-[2-[3,4-Bis(difluoromethoxy)phe nyl]-2-[4-[2,2,2-trifluoro-1-hydroxy-1-(trifluorome-thyl)ethyl]phenyl]ethyl ]-3-methylpyridine N-oxide | 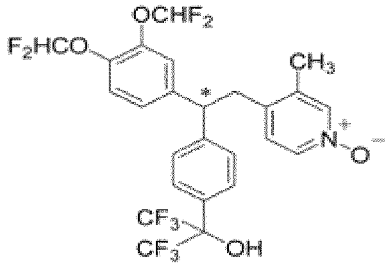 **L-826,141 (Enantiomer 1)** |
| CI-1044 | N-(9-amino-4-oxo-1-phenyl-3,4,6,7-tetrahydro(1,4)diazepin o(6,7,1-hi)indol-3-yl)nicotinamide | 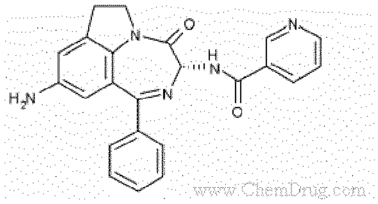 |
| MK-0873 | (-)-trans-2-(3'-(3-(N-Cycloprpropylcarbamoyl)-4-oxo-1,4-dihydro-1,8-naphthyridin-1-yl)-3-fluorobiphenyl-4-yl) cycloprpanecarboxylic acid | |
| T-2585 | | 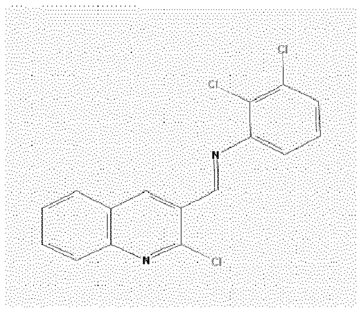 |
| AN2728 | 5-(4-cyanophenoxy)-2,3-dihydro-1-hydroxy-2,1-benzoxaborole | 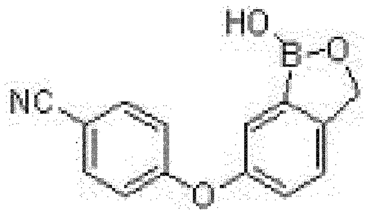 |
| SU-14813 | (S,Z)-5-((5-fluoro-2-oxoindolin-3-ylidene)methyl)-N-(2-hydroxy-3-morpholinopropyl)-2,4-dimethyl-1H-pyrrole-3-carboxamide | 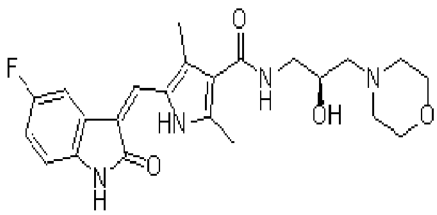 |

(continued)

| | | |
|---|---|---|
| CGP-52421 | | 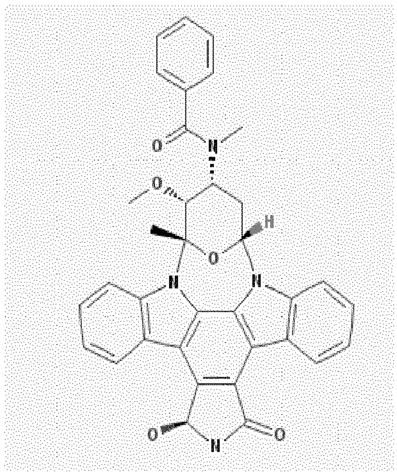 |
| NSC161613 | 2-[[2-[(2-aminoacetyl)amino]-3-(2,4-dinitrophenyl)sulfanylp ropanoyl]amino] pentaned ioic acid | 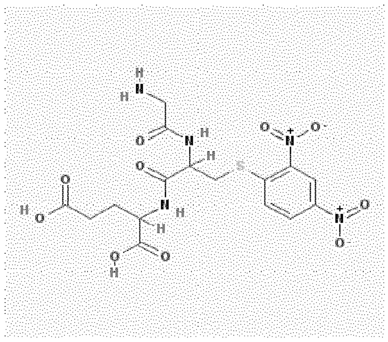 |
| NSC47091 | 2-(3-carboxyprop-2-enoylamino)-6-[[(2)-3-carboxyprop-2-enoyl]amino] benzoic acid | 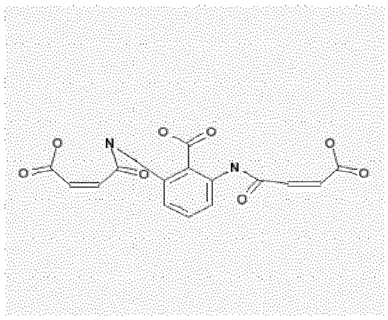 |

In preferred embodiments the inventive compound is an inhibitor (i.e. an antagonist) or modulator of any one of FRK, PDE4D, LPAR3, CAMK1D, CSNK1G3 or FMO3. Inhibitors or ligands (i.e. binders) or modulators of FRK, PDE4D, LPAR3, CAMK1D, CSNK1G3 or FMO3 can be used in the treatment of pain in a subject. In preferred embodiments the FRK, PDE4D, LPAR3, CAMK1D, CSNK1G3 or FMO3 modulator is a selective FRK, PDE4D, LPAR3, CAMK1D, CSNK1G3 or FMO3 modulator. By "selective" it is meant that the affinity for one of FRK, PDE4D, LPAR3, CAMK1D or FMO3 is at least 10-fold, preferably 25-fold, more preferred 100-fold, still preferred 150-fold higher than the affinity of the other targets selected from FRK, PDE4D, LPAR3, CAMK1D or FMO3.

Surprisingly it has been shown that some of these targets, such as FRK, FMO3, LPAR3 can be both activated, e.g. by an agonist, or inhibited (e.g. by an inhibitor or antagonist) for an anti-pain effect in a patient. The group of agonists and antagonists is refered to herein as "modulators". Although in most cases an inhibitor is preferred for greater effect, it seems that modulation of activity of these targets in any direction reduces the pain or sensation of pain. Preferably the modulator is a strong binder to these targets, especially with a Kd of 1000nM or less or an IC50 of 1000 nM or less. Preferred lower values for Kd and IC50 are 800 nM or less, 600 nM or less, 500 nM or less, 400 nM or less, 300 nM or less, 200 nM or less, 100 nM or less, 50 nM or less or even 30 nM or less.

In particular preferred are FMO3 modulators or inhibitors for use in the treatment of pain. Such compounds are e.g. Tenofovir and Methimazole.

In particular preferred are FRK modulators or inhibitors for use in the treatment of pain. Such compounds are

e.g. dasatinib, motesanib, Doramapimod, Pelitinib, sorafenib, Vandetanib and Canertinib.

Preferably the inhibitor is selected from compounds with a Kd of lower than 3000 nM, preferably lower than 2000 nM, especially preferred lower than 1000 nM. In particular, these compounds can be selected from dasatinib (Kd 0.31 nM), motesanib (Kd 99 nM), Doramapimod (Kd 360 nM), Pelitinib (Kd 190 nM), sorafenib (Kd 440 nM) and Vandetanib (Kd 480 nM). The Kd may also be up to 750 nM or up to 500 nM.

Kd, Ki or IC50 values of course relate to the binding or inhibiting capability of a given compound on a given target, such as one of FRK, PDE4D, LPAR3, CAMK1D, CSNK1G3 or FMO3, as associated herein.

In preferred embodiments the FRK modulator is selected from compounds comprising a substituted pyridine, quinoline, isoquinoline or pyridine group.

In especially preferred embodiments the FRK modulator is an FRK inhibitor. An inhibitor or antagonist is a compound that lowers or inhibits the activity of a given target here FRK. This can be achieved by binding to the target, e.g. but not necessarily to the catalytic center, and preventing the catalytic activity of the target.

Preferably an inhibitor or modulator, in particular of FRK, is selected from compounds comprising a substituted pyrimidine, quinoline, isoquinoline or pyridine group, such as from motesanib (AMG-706), Pelitinib (EKB-569), sorafenib (sorafenib), Vandetanib (Vandetanib), canertinib ( CI-1033 ).

Preferably an inhibitor or modulator, in particular of FRK, is selected from compounds comprising a substituted aniline group with a Kd value less than 1000 nM, such as dasatinib ( dasatinib ), motesanib ( AMG-706 ), Doramapimod ( BIRB 796 ), Pelitinib ( EKB-569 ), sorafenib ( sorafenib ), Vandetanib ( Vandetanib ).

Preferably an inhibitor or modulator, in particular of FRK, is selected from compounds comprising a chlorine, fluorine or chlorine and fluorine substituted aniline group. Such compounds are e.g. dasatinib, Pelitinib, sorafenib, Vandetanib and canertinib.

In preferred embodiments an FRK inhibitor selected from compounds comprising an aniline group, selected from dasatinib ( dasatinib ), motesanib ( AMG-706 ), doramapimod ( BIRB 796 ), pelitinib ( EKB-569 ), sorafenib ( sorafenib ), vandetanib ( vandetanib ), canertinib ( CI-1033 ) and imatinib ( STI-571 ) is for use in the treatment of neuropathic pain, such as trigeminal neuralgia, such as post-herpetic neuralgia, such as painful diabetic neuropathy, such as painful diabetic peripheral neuropathy, such as diabetic polyneuropathy, such as sciatic pain, such as radiculopathy, such as radicular pain or such as non-inflammatory neuropathic pain. The aniline group is preferably a substituted aniline group and is e.g. selected from a chloride and/or fluoride substituted aniline group or a carbonyl substituted aniline. Preferably the aniline group is a carbonyl substitution, such as in dasatinib ( dasatinib ), motesanib ( AMG-706 ), doramapimod ( BIRB 796 ), pelitinib ( EKB-569 ), sorafenib ( sorafenib ), canertinib ( CI-1033 ) and imatinib ( STI-571 ), and can be used in the treatment of neuropathic pain.

In preferred embodiments the FRK inhibitor is a selective FRK inbibitor. By "selective" it is meant that the affinity for FRK is at least 10-fold, preferably 25-fold, more preferred 100-fold, still preferred 150-fold higher than the affinity for other tyrosine kinase receptors, especially one or both of FLT3 or c-Kit.

In further embodiments there is provided an LPAR3 inhibitor or modulator for use in the treatment of pain. Such a compound is e.g. selected from NSC161613, NSC47091, N6-Benzyladenosine-5'-phosphate, p-Aminobenzoly PAB-J acid.

Surprisingly it could be shown that in case of LPAR3 both activators or agonists and inhibitors or antagonsits can be effective in the treatment of pain. Thus any artificial change in the expression or activity of LPAR3 can ameliorate pain. Preferably the LPAR3 modulator is selected from compounds comprising N6-Benzyladenosine-5'-phosphate, p-Aminobenzoly PAB-J acid, NSC161613 and NSC47091. In particular preferred cases the LPAR3 modulator is an LPAR3 inhibitor. Such an inhibitor is e.g. p-Aminobenzoly PAB-J acid, NSC161613 or NSC47091.

In further embodiments there is provided a PDE4D inhibitor or modulator for use in the treatment of pain. Such a compound may be selected from cilomilast, Roflumilast, Filaminast, Piclamilast, V11294, Luteolin, Apremilast, Arofylline, Atizoram, Catramilast, Cimpyfylline, Daxalipram, Doxofylline, drotaverin, Efloxate, Etamiphylline, Etazolate, Etofylline, Broncholytine, Irimilast, oglemilast, Choline theophyllinate, Pumafentrine, Revamilast, Ronomilast, Tofimilast, Tolafentrine, Trapidil, GW 842470 (AWD 12-281), CDP-840, YM-976, CI-1018, D-4418, Lirimilast, SCH-351591, RPL-554, IPL-455903 (HT-0712), GSK256066, Zardaverine, Vardenafil, Tetomilast, IC485, L-826,141, ONO-6126, CI-1044, MK-0873, T-2585, R1533 (MEM-1414), UK-500,001, AN2728, DE-103, Tofisopam, Dextofisopam, Levotofisopam (USAN).

Preferably the PDE4D inhibitor is selected from a compound comprising a 1,2-dioxy-aryl group with an $IC_{50}$ value of 1100 nM or less, preferably 1050 nM or less, preferably 1000 nM or less, preferably 950 nM or less, preferably 950 nM or less, preferably 900 nM or less. Such a PDE4D inhibitor is preferably selected from cilomilast ( cilomilast, $IC_{50}$ of 11 nM), Roflumilast ( Roflumilast, $IC_{50}$ of 0.68 nM), Filaminast ( Filaminast, $IC_{50}$ of 1000 nM ), Piclamilast ( Piclamilast, $IC_{50}$ of 0.02 nM) , ( V11294, $IC_{50}$ of 200 nM ), Apremilast ( CC-10004 ), Atizoram ( CP80633 ), Catramilast ( Catramilast ), Daxalipram ( Daxalipram/mesopram, , $IC_{50}$ of 1100 nM ), drotaverin ( Drotaverine ), ( Glaucine Hydrobromide ), oglemilast ( GRC3886, $IC_{50}$ of 166 nM ), Pumafentrine ( Pumafentrine ), Revamilast ( Revamilast, $IC_{50}$ of 2.7 nM ) , Tolafentrine ( Tolafentrine, $IC_{50}$ of 30 nM ), ( CDP-840, $IC_{50}$ of 2.1 nM ), ( RPL-554 ), ( IPL-455903 (HT-0712) ), Zardaverine ( Zardaverine, $IC_{50}$ of 390 nM ), Tetomilast ( OPC-6535, $IC_{50}$ of 70 nM ), ( L-826,141, $IC_{50}$ of 2.4

nM ), Ronomilast ( ELB353 , $IC_{50}$ of 3.5 nM), Tofisopam ( Tofisopam, $IC_{50}$ of 900 nM ) .

In a further preferred embodiment the PDE4D inhibitor or modulator comprises a 1,2-dioxy-aryl group substituted with an alkyl or flour-alkyl group, or 1,2-dioxy-aryl group condensed in a furan ring containing oxygen. Such a compound is e.g. selected from cilomilast ( cilomilast ), Roflumilast ( Roflumilast ), Filaminast ( Filaminast ), Piclamilast ( Piclamilast ), ( V11294 ), Apremilast ( CC-10004 ), Daxalipram ( Daxalipram/mesopram ), drotaverin ( Drotaverine ), broncholytine ( Glaucine Hydrobromide ), oglemilast ( GRC3886 ), Pumafentrine ( Pumafentrine ), Revamilast ( Revamilast ), Tolafentrine ( Tolafentrine ), ( RPL-554 ), Zardaverine ( Zardaverine ), Tetomilast ( OPC-6535 ), ( L-826,141 ), Ronomilast ( ELB353 ), Tofisopam ( Tofisopam ).

In another aspect of PDE4D inhibitors or modulators there is provided a PDE4D inhibitor or modulator comprising a 3,5-dichlor-pyridine group or a pyridine group that is not condensed in a 2 ring structure. Such compounds may be selected from Roflumilast ( Roflumilast ), Piclamilast ( Piclamilast ), oglemilast ( GRC3886 ), Revamilast ( Revamilast ), GW 842470 (AWD 12-281), D-4418, SCH-351591.

Also provided is a PDE4D inhibitor or modulator comprising a quinoline, isoquinoline or pyrimidine group. Such a compound can be selected from drotaverine ( Drotaverine ), Pumafentrine ( Pumafentrine ), Tolafentrine ( Tolafentrine ), Trapidil ( Seoanin ), D-4418, SCH-351591, RPL-554, SK256066, T-2585, Ronomilast ( ELB353 ).

In a further embodiment the PDE4D inhibitor or modulator is selected from compounds comprising an aniline group, in particu-particular preferred a carbonyl- or chlorine-substituted aniline. Such compounds are e.g. Apremilast ( CC-10004 ), Arofylline ( LAS31025 ), CI-1044, T-2585.

The PDE4D inhibitor or modulator for use according to the invention may comprise a purine ring. Such a compound can be selected from ( IBMX ), ( V11294 ), Arofylline ( LAS31025 ), Cimpyfylline ( BRL-61063 ), Doxofylline ( Doxofylline ), Etamiphylline ( Etamiphylline ), Etofylline ( Etofylline ), Choline theophyllinate ( oxtriphyllin ), Theophylline ( Theophylline ). These compounds are preferably used for the treatment of neuropathic pain, especially non-inflammatory neuropathic pain.

In a further embodiment the PDE4D inhibitor or modulator for use according to the invention is essentially the only active pharmaceutical ingredient of the composition or medicament according to the invention, such as the only active pharmaceutical ingredient, in particular the only anti-pain compound, of the composition or medicament according to the invention. Preferably the PDE4D inhibitor or modulator for use according to the invention is not combined with or used in combination with a phospholipase inhibitor. Especially preferred, CI-1018 is not combined with or used in combination with a phospholipase inhibitor when used according to the invention.

In further embodiments there is provided a CAMK1D inhibitor or modulator for use in the treatment of pain. Such a compound may be selected from Bosutinib, Pelitinib, EKB-568, SU-14813, Ruboxistaurin, CGP-52421.

The CAMK1D inhibitor or modulator preferably comprises a chlorine-substituted aniline group. Such compounds can be selected from Bosutinib ( SKI-606 ) and Pelitinib ( EKB-569 ).

Preferably the CAMK1D inhibitor is selected from the group of bosutinib ( SKI-606 ), pelitinib ( EKB-569 ), EKB-568 and CGP-52421 for use in the treatment of pain.

In preferred embodiments the CAMK1D modulator is a selective CAMK1D modulator. By "selective" it is meant that the affinity for CAMK1D is at least 10-fold, preferably 25-fold, more preferred 100-fold, still preferred 150-fold higher than the affinity for other tyrosine kinase receptors, especially one or both of FLT3 or c-Kit.

In further embodiments there is provided a CSNK1G3 inhibitor or modulator for use in the treatment of pain. Such compound can be roscovitine.

The CSNK1G3 inhibitor or modulator preferably comprises a purine ring. Such compound can be roscovitine.

In preferred embodiments the CSNK1G3 inhibitor is a selective CSNK1G3 inbibitor. By "selective" it is meant that the affinity for CSNK1G3 is at least 10-fold, preferably 25-fold, more preferred 100-fold, still preferred 150-fold higher than the affinity for other tyrosine kinase receptors, especially one or both of FLT3 or c-Kit.

In preferred embodiments of the invention the compound for use in the treatment of pain comprises a quinoline or isoquinoline group. Such compounds are e.g. Bosutinib, Pelitinib ( EKB-569 ), drotaverin ( Drotaverine ), Pumafentrine ( Pumafentrine ), Tolafentrine ( Tolafentrine ), D-4418, SCH-351591, RPL-554, ( GSK256066 ), T-2585, Ronomilast ( ELB353 ), preferably the compound being an inhibitor of FRK, PDE4D or CAMK1D. These compounds are preferably for use in the treatment of neuropathic pain, preferably non-inflammatory neuropathic pain.

In preferred embodiments of the invention the compound for use in the treatment of pain comprises a chlorine-substituted aniline group. Such compounds are e.g. selected from dasatinib ( dasatinib ), bosutinib (SKI-606), sorafenib ( sorafenib ), Canertinib ( CI-1033 ), Arofylline ( LAS31025 ), T-2585, Pelitinib ( EKB-569 ), preferably the compound being an inhibitor of FRK, PDE4D or CAMK1D. These compounds are preferably for use in the treatment of neuropathic pain, preferably non-inflammatory neuropathic pain.

The inventive compound can be used in combination with other active analgesic/anti-pain compounds, preferably only with those described herein or above or in the claims, or used as single active analgesic/anti-pain compound.

In preferred embodiments the compound for use according to the invention comprises a 1,2-Dioxyaryl group. Especially preferred the compound comprises a 1,2-Dioxyaryl group substituted with a basic residue, such as Vandetanib

( Vandetanib ), SKI-606 ( Bosutinib ). The compound may comprise a 1,2-Dioxyaryl group substituted with a cycloaliphatic residue, such as cilomilast ( cilomilast ), Roflumilast ( Roflumilast ), Filaminast ( Filaminast ), Piclamilast ( Piclamilast ), V11294, CP80633 ( Atizoram ), Catramilast/Atopik ( Catramilast ), CDP-840 , IPL-455903 (HT-0712). The compound may comprise a 1,2-dioxy-aryl substituted with an alkyl residue, such as CC-10004 ( Apremilast ), Daxalipram/mesopram ( Daxalipram ), Drotaverine ( drotaverin ), Glaucine Hydrobromide ( Broncholytine ), Pumafentrine ( Pumafentrine ), Tolafentrine ( Tolafentrine ), RPL-554, Zardaverine ( Zardaverine ), OPC-6535 ( Tetomilast ), Tofisopam ( Tofisopam ). The compound may comprise a 1,2-dioxy-aryl substituted with a fluor-alkyl group, such as GRC3886 ( oglemilast ), Revamilast ( Revamilast ), Zardaverine ( Zardaverine ), L-826,141, ELB353 ( Ronomilast ). The compound may comprise a 1,2-dioxy-aryl in a condensed furan ring with an oxygen, such as GRC3886 ( oglemilast ), Revamilast ( Revamilast ).

In preferred embodiments the compound for use according to the invention comprises an indole group, especially an indole group as part of a ringsystem, such as CGP-52421, midostaurin ( PKC-412 ).

In preferred embodiments the compound for use according to the invention comprises a substituted indole, such as GW 842470 (AWD 12-281), AMG-706 ( motesanib ), SU-14813 , LY-333531 ( Ruboxistaurin ), SKI-606 ( Bosutinib ).

In preferred embodiments the compound for use according to the invention comprises a purine ring, such as N6-Benzyladenosine-5'-phosphate, V11294, LAS31025 ( Arofylline ), BRL-61063 ( Cimpyfylline ), Doxofylline ( Doxofylline ), Etamiphylline ( Etamiphylline ), Etofylline ( Etofylline ), oxtriphyllin ( Choline theophyllinate ), Roscovitine ( Roscovitine ), Tenofovir (PMPA) ( Tenofovir ), Remofovir (Pradefovir), Adefovir dipivoxil (Bis-POM PMEA) ( Adefovir ).

In preferred embodiments the compound for use according to the invention comprises a sulfone or sulfonic acid or sulfonamide group, especially a methyl-suflone, such as Lirimilast ( Lirimilast ). The compound may comprise a siaryl-sulfone, such as GSK256066, Vardenafil ( Vardenafil). The compound may comprise a sulfonic acid group, such as p-Aminobenzoly PAB-J acid. The compound may comprise a Sulfonamide group, such as Tolafentrine ( Tolafentrine ), Acetazolamide ( Acetazolamide ).

In preferred embodiments the compound for use according to the invention comprises a pyridine group. Preferably the compound comprises an unsubstituted pyridine radical, such as Nicotinamide ( Nicotinamide ), or CI-1044. The compound may comprise a 3,5 -Dichlorpyridine group, such as Roflumilast ( Roflumilast ), Piclamilast ( Piclamilast ), GRC3886 ( oglemilast ), Revamilast ( Revamilast ), GW 842470 (AWD 12-281), D-4418, SCH-351591. The compound may comprise a substituted pyridine group, such as AMG-706 ( motesanib ), sorafenib ( sorafenib ), Etazolate ( Etazolate ), Tofimilast ( Tofimilast ), GSK256066, MK-0873.

In preferred embodiments the compound for use according to the invention comprises a quinolone or isoquinoline or condensed isoquinoline group. The compound may comprise a quinolone group such as EKB-569 ( Pelitinib ), D-4418, SCH-351591, GSK256066, MK-0873 , SKI-606 ( Bosutinib ). The compound may comprise a isoquinoline or condensed isoquinoline group, such as Drotaverine ( drotaverin ), Pumafentrine ( Pumafentrine ), Tolafentrine ( Tolafentrine ), RPL-554, ELB353 ( Ronomilast ).

In preferred embodiments the compound for use according to the invention comprises a pyrimidine group, such as Vandetanib ( Vandetanib ), CI-1033 ( Canertinib ), Seoanin ( Trapidil ), tozasertib ( MK-0457, VX 680 ).

In preferred embodiments the compound for use according to the invention is a compound with 3 nitrogens in a ring structures, such as YM-976.

In preferred embodiments the compound for use according to the invention comprises a carbonic or phosphoric acid group. The compound may comprise a carbonic acid group, such as OPC-6535 ( Tetomilast ). The compound may comprise a phosphoric acid group, such as N6-Benzyladenosine-5'-phosphate, Tenofovir (PMPA) ( Tenofovir ).

In preferred embodiments the compound for use according to the invention comprises an esther group, such as Remofovir (Pradefovir) ( Pradefovir ), Adefovir dipivoxil (Bis-POM PMEA) ( Adefovir ).

In preferred embodiments the compound for use according to the invention comprises a aniline group, especially preferred a chloride and/or fluoride substituted aniline group. The compound may comprise a Chlor-fluor-aniline, auch as EKB-569 ( Pelitinib ), CI-1033 ( Canertinib ). The compound may comprise a Chlor-aniline or dichlor-aniline, such as dasatinib ( dasatinib ), sorafenib ( sorafenib ), LAS31025 ( Arofylline ), T-2585 , SKI-606 ( Bosutinib ). The compound may comprise a Fluor-aniline, such as Vandetanib ( Vandetanib ), SU-14813.

In preferred embodiments the compound for use according to the invention comprises a carbonyl-substituted aniline, such as dasatinib ( dasatinib ), AMG-706 ( motesanib ), BIRB 796 ( Doramapimod ), EKB-569 ( Pelitinib ), sorafenib ( sorafenib ), CI-1033 ( Canertinib ), p-Aminobenzoly PAB-J acid, CC-10004 ( Apremilast ), CI-1044 , NSC47091.

In preferred embodiments the compound for use according to the invention comprises a diaryl-thioether group, such as tozasertib ( MK-0457, VX 680 ).

In preferred embodiments the compound for use according to the invention comprises a 1,3-Dioxyaryl group, such as Efloxate ( Efloxate ).

In preferred embodiments the compound for use according to the invention is selected from Methimazole, AN2728, NSC161613, especially a compound without one or more or all of the above mentioned groups.

The present invention also provides a method of treating pain in a subject comprising the administration of a

therapeutic compound selected from the compounds of table 1. In a related aspect the present invention provides the use of a compound of table 1 for the manufacture of an analgesic or a medicament for the treatment of pain in a subject. The invention is further defined by the subject matter of the claims.

The inventive compounds have been identified by a thorough screening system based on genetic analysis, starting from drosophila hits. *Drosophila* (fruit flies) respond to noxious stimuli, and have become a powerful model organism for studying genetics, including the genetics of nociception. For instance, the TRP channel *PAINLESS* was previously identified as a heat-responsive channel mediating thermal-based nociception in fly larvae. Using genome-wide neuronal-specific RNAi knock-down, the present invention provides a global screen for an innate behavior and identify hundreds of novel genes implicated in nociception in the fly, including the $\alpha 2\delta$-family calcium channel subunit *straightjacket* or the phospholipid kinase PI3Kgamma. The initial drosophila screen yielded targets having homologous targets in various organisms, including humans. For example, observation of the mammalian straightjacket ortholog, $\alpha 2\delta 3$, and PI3Kgamma in nociception was confirmed in knock-out mice that exhibit significantly impaired basal pain sensitivity and delayed thermal hyperalgesia after inflammation. In humans, single nucleotide polymorphisms (SNPs) in $\alpha 2\delta 3$ or PIK3CG were found that are associated with reduced acute pain sensitivity in healthy volunteers and chronic postsurgical back pain. Based on the validated genetic data various compounds have been identified that are capable of treating or suppressing pain in various organisms, in particular in humans. In a further screening, several compounds have been identified which modulate or antagonize or inhibit, that is lower the targets activity in vivo or as can be determined in an in vitro assay as described herein or known in the art (e.g. Enzyme activity assay to determine $IC_{50}$ values), which are active in the treatment or reduction of pain in a subject. In particular the compounds to be used in any form of treatment according to the present invention are selected from any one of (1S,2S)-2-(2-(N-((3-benzimidazol-2-yl)propyl)-N-methylamino)ethyl)-6-fluoro-1,2,3,4-tetrahydro-1-isopropyl-2-naphtyl cyclopropanecarboxylate dihydrochloride, (5-(2-methoxy-5-chloro-5-phenyl)furan-2-ylcarbonyl)guanidine, (6S)-5,6,7,8-tetrahydrofolic acid, (T,G)-A-L, 1 alpha-hydroxyergocalciferol, 1-(1-cyclohexylethylamino)-4-phenylphthalazine, 1-(2-methyl-4-methoxyphenyl)-4-((2-hydroxyethyl)amino)-6-trifluoromethoxy-2,3-dihydropyrrolo(3,2-c)quinoline, 1-(2,3-dichlorobenzoyl)-5-methoxy-2-methyl-(2-(mopholin-4-yl)ethyl)-1H-indole, 1-(2,3-dihydro-1,4-benzodioxin-5-yl)-4-((5-(4-fluorophenyl)-3-pyridinyl)methyl) piperazine, 1-(6-((3-methoxyestra-1,3,5(10)-trien-17-yl)amino)hexyl)-1H-pyrrole-2,5-dione, 1-adamantyl propargyl ether, 1-aminobenzotriazole, 1-aminooxy-3-aminopropane, 1-hydrazino-4-(3,5-dimethyl)-l-pyrazolyl-5H-pyridazino(4,5-b)indole, 1-hydroxymethylmidazolam, 1-hydroxypyrene, 1-Methyl-4-phenylpyridinium, 1-Nitropyren-8-ol, 1-phosphatidyl-1D-myo-inositol 3-phosphates, 1-stearoyl-2-oleoyl-sn-glycero-3-phosphocholine, 1,1-bis(3'-indolyl)-1-(4-t-butylphenyl)methane, 1,1-dimethylbutyl-1-deoxy-Delta(9)-THC, 1,1,1-trichloro-2-(4-hydroxyphenyl)-2-(4-methoxyphenyl) ethane, 1,2-bis(diphenylphosphino)ethane, 1,2-di-(4-sulfamidophenyl)-4-butylpyrazolidine-3,5-dione, 1,2-diacyl-sn-glycero-3-phosphocholines, 1,2-ethanedithiol, 1,2-oleoylphosphatidylcholine, 1,2,4-triazines, 1,25-dihydroxy-21-(3-hydroxy-3-methylbutyl)-23-yne-26,27-hexafluorocholecalciferol, 1,25-dihydroxyergocalciferol, 1,25D3, 1,3-Dcg, 1,3-dihydroxy-4,4,5,5-tetramethyl-2-(4-carboxyphenyl)tetrahydroimidazole, 1,3-dipropyl-8-(3-noradamantyl)xanthine, 1,3,5-trimethylbenzene, 1,7-dioxa-2,6-diaza-4,4-dioxide-4,7a-dithia-3H,5H-benzo(cd)pentalene, 10-deoxymethynolide, 10-propargyl-10-deazaaminopterin, 10-undecynoic acid, 10,10-bis(4-pyridinylmethyl)-9(10H)-anthracenone, 11-cis-retinal, 11-hydroxycannabinol, 12-Hht, 13-Lox, 13-oxo-9,11-octadecadienoic acid, 15 hete, 15-Hydroxy-11 alpha,9 alpha-(epoxymethano)prosta-5,13-dienoic Acid, 17-(allylamino)-17-demethoxygeldanamycin, 17alpha-ethynylestradiol, 1843U89, 1D-myo-inositol 1,3,4,5-tetrakisphosphate, 1H-indole, 1H-pyrazole, 1H-pyrazolo(3,4-b)pyridine, 2 APB, 2-(1-(3-dimethylaminopropyl)-5-methoxyindol-3-yl)-3-(1H-indol-3-yl)maleimide, 2-(1-methyl-4-piperidinyl)-6-(2-phenylpyrazolo(1,5-a)pyridin-3-yl)-3(2H)-pyridazinone, 2-(2-hydroxyethylsulfanyl)-3-methyl-1,4-naphthoquinone, 2-(3,4-dimethoxyphenyl)-5-amino-2-isopropylvaleronitrile, 2-(4-amino-3-methylphenyl)-5-fluorobenzothiazole, 2-(4-morpholinoanilino)-6-cyclohexylaminopurine, 2-(4-morpholinyl)-8-phenyl-4H-1-benzopyran-4-one, 2-(4-toluidino)-6-naphthalenesulfonic acid, 2-(cyclohexylmethylidenehydrazino)adenosine, 2-AAF, 2-acetylthiomethyl-3-(4-methylbenzoyl) propionic acid, 2-AG, 2-amino-1-methyl-6-phenylimidazo(4,5-b)pyridine, 2-amino-3,4-dimethylimidazo(4,5-f)quinoline, 2-aminoethoxydiphenyl borate, 2-AP, 2-CADO, 2-chloro-5-nitrobenzanilide, 2-cyano-3-hydroxy-N-(4-(trifluoromethyl) phenyl)-2-hepten-6-ynamide, 2-cyanomethylthiopyridine-4-carbonitrile, 2-cyclopentyl-5-(5-isoquinolylsulfonyl)-6-nitro-1H-benzo(D)imidazole, 2-DG, 2-hydroxy-4-(2,2,3,3,3-pentafluoropropoxy)benzoic acid, 2-hydroxyamino-1-methyl-6-phenylimidazo(4,5-b)pyridine, 2-hydroxyamino-3-methylimidazolo(4,5-f)quinoline, 2-ME, 2-methoxyacetic acid [2-[2-[3-(1H-benzoimidazol-2-yl)propylmethyl-amino]ethyl]-6-fluoro-1-isopropyl-tetralin-2-yl] ester, 2-methyl-1-((4-methyl-5-isoquinolinyl)sulfonyl)homopiperazine, 2-N-(4-(1-azitrifluoroethyl)benzoyl)-1,3-bis-(mannos-4-yloxy)-2-propylamine, 2-Naftol, 2-oxothiazolidine-4-carboxylic acid, 2-phenyl-4-oxohydroquinoline, 2,2,2-trichloroethane-1,1-diol, 2,2'-(hydroxynitrosohydrazono)bis-ethanamine, 2,2'-azobis(2,4-dimethylvaleronitrile), 2,2'-bipyridine, 2,2',4,4'-tetrachlorobiphenyl, 2,3-bis(3'-hydroxybenzyl)butane-1,4-diol, 2,3-dihydroxyterephthalamide, 2,3,4-tri-O-acetylarabinopyranosyl isothiocyanate, 2,4-diaminoquinazoline, 2,4-thiazolidinedione, 21-hydroxy-9beta,10alpha-pregna-5,7-diene-3-ol-20-one, 25-desacetylrifabutin, 25-Hydroxycholesterol, 25(OH)D3, 3-((4-(4-chlorophenyl)piperazin-1-yl)methyl)-1H-pyrrolo(2,3-b)pyridine, 3-(2-hydroxy-4-(1,1-dimethylheptyl)phenyl)-4-(3-hydroxypropyl)cyclohexanol, 3-(2h)-pyridazinone, 3-(cyclopentyloxy)-N-(3,5-dichloro-4-pyridyl)-4-methoxybenzamide, 3-aminopyrazole, 3-beta-(2-(diethylamino)ethoxy)androst-5-en-17-one, 3-BHA, 3-hydroxybutanal, 3-hydroxyflunitrazepam, 3-Hydroxyquinine, 3-isobutyl-1-methyl-Xanthine, 3-keto-desogestrel, 3-methoxy-4-aminoazobenzene, 3-Methoxymorphinan, 3-Methoxyoestradiol, 3-methylcholanthrene, 3-MI, 3,3',4,5'-tetrahydroxystilbene, 3,4-DCI, 3,4,5-trihydroxybenzamidoxime, 4-(3-3,4-p-menthadien-(1,8)-yl)olivetol, 4-(3-Butoxy-4-methoxybenzyl)-2-imidazolidinone, 4-(4-(4-chlorophenyl)-4-hydroxy-1-piperidinyl)-1-(4-fluorophenyl)-1-butanol, 4-(4-(N-benzoylamino)anilino)-6-methoxy-7-(3-(1-morpholino)propoxy)quinazoline, 4-(4-fluorophenyl)-2-(4-hydroxyphenyl)-5-(4-pyridyl)imidazole, 4-(5-benzo(1,3)dioxol-5-yl-4-pyridin-2-yl-1H-imidazol-2-yl)benzamide, 4-(benzodioxan-5-yl)-1-(indan-2-yl)piperazine, 4-(N-methyl-N-nitrosamino)-1-(3-pyridyl)-1-butanone, 4-AP, 4-azidosalicylic acid, 4-dimethylamino-3',4'-dimethoxychalcone, 4-hydroxy-N-desmethyltamoxifen, 4-hydroxyacetophenone, 4-hydroxycoumarin, 4-hydroxyestradiol-17 beta, 4-hydroxynon-2-enal, 4-hydroxytriazolam, 4-methyl-N-(3-(4-methylimidazol-1-yl)-5-(trifluoromethyl)phenyl)-3-((4-pyridin-3-ylpyrimidin-2-yl)amino)benzamide, 4-phenylbutyric acid, 4-S-cysteaminylphenol, 4-sulfophenylmethallyl ether, 4,4'-DDE, 4,4'-dipyridyl disulfide, 4,8-dimethoxy-7-hydroxyfuro(2,3-b)quinoline, 4'-epidoxorubicin, 4'-N-benzoylstaurosporine, 4(2'-aminoethyl)amino-1,8-dimethylimidazo(1,2-a)quinoxaline, 4alpha-phorbol 12,13-didecanone, 4alphaPDD, 5-((1,2-dihydro-2-oxo-3H-indol-3-ylidene)methyl)-2,4-dimethyl-1H-pyrrole-3-propanoic acid, 5-(4'-(N-piperidinyl)phenylazo)indazole, 5-7-oxo-zeaenol, 5-AC, 5-acetylneuraminyl-(2-3)-galactosyl-(1-4)-(fucopyranosyl-(1-3))-N-acetylglucosamine, 5-azido-1H-indole-3-acetic acid, 5-HT, 5-methoxy-N,N-diisopropyltryptamine, 5-Mop, 5,10-methylenetetrahydrofolate, 5,6-dimethylxanthenoneacetic acid, 5'-O-(((2-decanoylamino-3-phenylpropyloxycarbonyl)amino)sulfonyl)uridine, 6 beta-hydroxycortisol, 6-Aminochrysene-1,2-dihydrodiol, 6-chloro-2-pyridylmethyl nitrate, 6-deoxy-6-bromoascorbic acid, 6-hydroxydexamethasone, 6-Mercaptopurine, 6,6'-oxybis(2,2-dimethylhexanoic acid), 64Gd, 7-(1,1-dimethylethyl)-6-(2-ethyl-2H-1,2,4-triazol-3-ylmethoxy)-3-(2-fluorophenyl)-1,2,4-triazolo(4,3-b)pyridazine, 7-benzylamino-6-chloro-2-piperazino-4-pyrrolidinopteridine, 7-benzyloxyquinoline, 7-CDL, 7-hydroxystaurosporine, 7-ketocholesterol, 7,8-BF, 7,8-dihydroneopterin, 7'-Isothiocyanato-11-hydroxy-1',1'-dimethylheptylhexahydrocannabinol, 7C3MT, 7H-Pyrrolo(2,3-d)pyrimidine, 8-((4-bromo-2,3-dioxobutyl)thio)-adenosine 3',5'-cyclic monophosphate, 8-(2,6-dichlorophenyl)-10-methyl-3-((4-morpholin-4-ylphenyl) amino)-2,4,10-triazabicyclo(4.4.0)deca-1,3,5,7-tetraen-9-one, 8-(3-chlorostyryl)caffeine, 8-anilinonaphthalene-1-sulfonic acid, 8-Hydroxy-2-(di-n-propylamino)tetralin, 8-Isoprostane, 8,10-bis((2,2-dimethyl-1-oxopropyl)oxy)-11-methyl-1234-tetrahydro-6H-benzo(beta)quinolizin-6-one, 9-(4'-aminophenyl)-9H-pyrido(3,4-b)indole, 9-anthroic acid, 9-CRA, 9-hydroxy-risperidone, 9,10-anthraquinone, 9H-xanthene, A 71915, A-300-I, a-ADP, A73025, Abbott, abciximab, Absele, ABT-737, acetamide 45, Aceton, acetonitrile, acetyl-11-ketoboswellic acid, acetylcholine, acetylvalerenolic acid, Aclarubicin, Acolen, ACON, ACT D, actinium, Actosin, adalimumab, Adalin, Adanon, Adfeed, adinazolam, Adofeed, Adrenor, Adrin, AEBSF, Aeromax, afloqualone, AGMATINE, AIDSVAX, ajoene, ajulemic acid, alachlor, Aladerm, alaninate, Alat, Alcolo, Alcuronium, Aldara, ALDO, Aldrich, alemtuzumab, Alfarol, Alfentanil, ALIMTA, aliskiren, Alli, ALLN, alloxazine, allyl isothiocyanate, almokalant, aloesin, Alprenolol, Alvesco, AM 1387, AM 251, Am 80, AMD 070, Amiloride, Aminacrine, Amine BB, amino-polyethyleneoxide-sulfonate, aminoflavone, Amiodarone, Amlodipine, Amphotericin B, amprenavir, Amrinone, amsonic acid, Amygdalin, AN 207, Anaboleen, anacardic acid, Anandamide, Anco, Andrographis, Androtine, Aneol, Ang II, Anisomycin, Anon, Anthocyanins, anthra(1,9-cd)pyrazol-6(2H)-one, anthracene, anthralin, Anthricin, anthrone, antibiotic G 418, antibiotic H107, Antimycin A, Anyvim, APAP, APDC, Aphidicolin, Aphloiol, apicidin, Apigenin, apocynin, Apotransferrin, aprepitant, APRL, AQ4N, arabinogalactan, Arac, Aralen, Arasine, Areca, Arecoline, Areether, argatroban, aripiprazole, Aron, Artein, Artra, arvanil, asiatic acid, asiaticoside, Asmax, Asmol, ASTA, astatine, Astemizole, Astragaloside A, atazanavir, ATL 146e, Atorel, atorvastatin, Atovaquone, ATRA, Atropine, Auranofin, AuTM, auxin, avasimibe, AVE 0118, avicularin, Avid, Axert, Axsain, Aza-dC, Aza-deoxycytidine, azacyclonol, Azadc, azamulin, azaspirane, azelaic acid, azelastine, azelnidipine, azido ruthenium, Azine, Azithromycin, Azobisisobutyramidinium dichloride, Azole, Azoles, Azolidine, Azophen, Azor, BA (VAN), Ba 0108E, bacitracin, Baclofen, bacterial lysate, bafilomycin A1, Bagren, baicalein, Barbiturate, Barnidipine, BAY 11-7085, BB-K8, BCNU, Beflavin, Belt, benazepril, bendamustine, Benidipine, benzamidine, benzimidazolide, Benzodiazepines, Benzodioxoles, Benzphetamine, benzydamine N-oxide, benzylamine, benzyloxycarbonylleucyl-leucyl-leucine aldehyde, benzyloxycarbonylvalyl-alanyl-aspartyl fluoromethyl ketone, beractant, berberine, bergamottin, bergaptol, beta-glycerophosphoric acid, beta-lapachone, beta-Naphthoflavone, beta-propiolactone, Bethanechol, betulinic acid, bexarotene, Bezafibrate, BG 9928, BGC945, biapigenin, BIBX 1382BS, biphenyl-4-ol, BIRB 796, bisindolylmaleimide I, bisindolylmaleimide III, Bisoprolol, bisperoxovanadium, Bisphenol A-Glycidyl Methacrylate, bizelesin, BL1521, Bla-S, Blow, BM 41.440, BML 241, BMS 310705, BMS204352, BMS453, Bo-Xan, Boltin, Bonopen, boron, Borrelia-burgdorferi, bortezomib, bosentan, bosutinib, botrocetin, BPDE, BR-II, Brake, bredinin, Brefeldin A, Bromazepam, bromo-cis-stilbene, brucine, bryostatin 1, Budesonide, bufalin, bufuralol, Bumetanide, BuOH, Bupivacaine, Buprenorphine, BUPROPION, Buspirone, Busulfan, Buthionine Sulfoximine, Butyrate, butyrolactone I, C 1027, C 76, CACP, Calcijex, Calcimycin, calphostin C, Calyculin, Camptothecin, Canef, cangrelor, Cannabinoids, Cannabis, Cantharidin, CAPE, Capsaicin, capsaicinoids, capsazepine, Carbachol, Carbamazepine, carbapenem, Carbapenems, carbobenzoxy-leucyl-leucyl-norvalinal, Carbolines, Carboxyethyl-phenethylamino-ethylcarboxamidoadenosine, Cardiolipins, carebastine, CARNOSOL, carrageenans, carvacrol, carvedilol, Casodex, caspofungin, casticin, catechins, CB 3717, Cbdca, CCPA, CD 437, CDP 840, Cefoxitin, celecoxib, cephalomannine, cephalosporins, cepharanthine, cerebrolysin, cerivastatin, Cetomacrogol, cetrorelix, cetuximab, CGP 12177, CGS 15943A, CGS 21680,

CH-THF, CH2CHO, Chalcone, CHAPS, Chinine, Chitosan, Chloramphenicol, chlorophenyl-ethane, chlorophyllin, chlorophyllypt, chlorpromazine, Chlorpropham, Chlorzoxazone, Cholestanol, CHOLINE, Chonsurid, chromophore, Chrysin, chymostatin, CI1033, cicaprost, cifostodine, ciglitazone, Cilazapril, Cilomilast, cilostazol, Cimetidine, cinacalcet, cinitapride, cinnamic aldehyde, cionin, Cipol N, Ciprofloxacin, Ciprol, cis-9, trans-11-conjugated linoleic acid, Cisapride, Citalopram, Citox, CITRULLINE, clebopride, clevidipine, clobazam, Clodronic Acid, clofarabine, Clofibric Acid, Clomipramine, Clonazepam, Clonidine, clopidogrel, clotiazepam, Clozapine, clozapine N-oxide, CNI 1493, Co 2-1970, Coagulin, Colchicine, compactin, CONT, Cotinine, Cotrim, coumarin, CRA 024781, CRA 026440, Crestor, Crodacid, Crypt-2,2,2, cryptdin 3, cryptotanshinone, cryptoxanthin, CUBE, CVT 3146, cyanidin 3-rutinoside, cyanidin-3-glucoside, cyanoginosin-LA, Cyclandelate, cyclohexyl carbamic acid 3'-carbamoylbiphenyl-3-yl ester, cyclohexyl-methyl, cyclopamine, Cyclopentenone, cyclopiazonic acid, Cyproheptadine, Cyproterone Acetate, cystathionine, cysteamine, cysteinyl-leukotriene, Cytarabine, cytochalasin B, Cytochalasin D, cytochalasin E, D 22888, D 23129, DA 8159, Dacarbazine, DADSO, daidzein, danaproid, Dapsone, Daral, Darifenacin, darunavir, dasatinib, Daunorubicin, Dayfen, DBPC, DDB, DDE, Debrisoquin, decursin, Deethylamiodarone, deferiprone, Deferoxamine, deguelin, dehydroaripiprazole, Dehydroepiandrosterone Sulfate, dehydroxymethylepoxyquinomicin, Delavirdine, delta8-THC, Denagard, denbinobin, denileukin diftitox, denopamine, Depas, deramciclane, desethylchloroquine, desflurane, desisobutyrylciclesonide, desmethylazelastine, Desmethyldeprenyl, Devazepide, Dexfenfluramine, dexloxiglumide, Dextropropoxyphene, dFdC, DFMO, DHEA, DHLA, di-(1-isoquinolinyl)-di-(pyridyl-2')butane, Diaben, Diacomit, diadenosine tetraphosphate, Dial, Diamide, DIAN, diarsenic trioxide, Dibenzanthracene, Dicid, Diclofenac, Dicyclohexylcarbodiimide, diethyl maleate, Diethyl-benzoquinone-imine, Digicor, Digitin, Digoxin, Dihydroqinghaosu, Dihydroxycholecalciferols, diisopropyl fluorophosphate, dillapiol, Diltiazem, Dimethadione, dimethyl fumarate, Dimethyl Sulfoxide, dimethyl-hydrazide, dimethylamino-purine, dimuonium, dinitrophenol, Dinoprostone, dioxirane, Dipalmitoyl, diphenylalanine, Diphenylamine, diphenyleneiodonium, Dipyridamole, Dipyrone, discodermolide, Diterpenes, Dithionite, diuretic, Diuron, divinyl benzene, dl-Ipr, DMGG, DMPX, DMSO, Dobutamine, Doca, Doconexent, dodecyl-phosphocholine, dodecyloctaethyleneglycol monoether, Domperidone, DOTA, Doxazosin, Doxorubicin, Doxycycline, DPC 681, DPCPX, Droxia, DTMC, dulcin, Durapatite, DX 9065a, Dxms, Dynatra, E 10, E 3330, E-MIX 80, E.O., EACA, ebastine, ebrotidine, Echinomycin, Econ, econazole, ecteinascidin 743, Edetic Acid, Edex, efavirenz, EGCg, EGTA, eletriptan, Elicide, Empecid, Enalapril, Endocannabinoids, endomorphin 1, Enediynes, enflurane, enone, Enoximone, entacapone, Entex, enzastaurin, EOS, EPC-K(1), EPEG, EPIB, epibatidine, Epicar, Epoprostenol, epoxybergamottin, epsilon-viniferin, erastin, ergosterol-5,8-peroxide, Eril, erlotinib, erucin, Eryc, erythritol anhydride, esterbut-3, Estriol, ET18-Ome, Etfc cpd, Ethacrynic Acid, Ethan, Ethinyloestradiol, Ethylmorphine, Ethynodiol Diacetate, Eticol, Etidronic Acid, Etodolac, Etoposide, etoricoxib, etravirine, Eufor, Eugenol, eupatilin, everolimus, Evex, Evodin, exenatide, Exosurf, Expectorants, Extina, Ezerin, ezetimib, Facet, Facid, facile, Factor IIa, FAMP, Fanchinine, Farnesyl-PP, farnesylthiosalicylic acid, febuxostat, felbamate, Felodipine, Fenfluramine, fenitrothion, fenofibric acid, Fenretinide, Fentanyl, ferulic acid, Filipin, fingolimod, fipronil, fisetin, Flanin F, Flavon, flavonols, flavopiridol, Flavyl, FLCZ, Flecainide, Floxacillin, flufenamic acid, Flunitrazepam, fluorexon, Fluorouracil, fluvoxamine, FOLATE-ANALOG, fondaparinux, Fonofos, Format, Formyl-Tetrahydrofolate, Forskolin, fosamprenavir, Foscarnet, FR 120480, FR 235222, fraxin, FTY 720P, fucoidan, fulvestrant, fumagillin, Fura-2, furafylline, Furamon, Furylfuramide, Gabexate, gadolinium, Gadolinium DTPA, galactocerebroside, galactomannan, galangin, galaturonate, gallic acid, Gallogen, gambierol, Gambogic acid, gamma-butyric-acid, Ganciclovir, gastrin 17, gatifloxacin, gefitinib, Geldanamycin, Gemfibrozil, gemtuzumab, Gentamicins, gepirone, geraniol, geranylcoumarin, Gestodene, GF 120918, GGTI 298, GI 129471, gingerol, ginsenoside Rd, ginsenoside Rf, ginsenoside Rg1, ginsenoside Rh2, Ginsenosides, GLCa, Gliclazide, Glumin, Glyoxal, Gnidimacrin, GnRH, Go 6976, gossypol, GR 79236X, gramicidin S, Granisetron, Gravistat, Grofo, Guggulsterone, GW 4064, GW 501516, H 89, Halan, halofuginone, harmine, Harzol, hassium, HDMTX, Hecogenin, Hectorol, Heet, helenalin, Hemicholinium 3, herbimycin, hesperadin, HESPERETIN, Hexadimethrine, hexarelin, Hgln, himbacine, Hk, Hocus, HOE 33342, honokiol, Horner, HS 1200, HU 211, HyateC, Hydoxin, hydride, Hydromorphone, Hydroxychloroquine, hydroxycotinine, hydroxylamine, Hydroxytryptophol, Hyhorin, Hypaque, hyperforin, hypericin, Hypericum-perforatum, hypochlorous acid, iberin, IBMX, ibopamine, ibudilast, IC 831423, icariin, icaritin, icilin, ICRF 193, IDS 23, Ifosfamide, Ikarugamycin, ilimaquinone, Iloprost, Imadyl, imatinib, imidafenacin, imidazo-pyridine, imidazolidin-2-one, imidazolidin-one, imidazolidine, Imidazoline, imidazolyl-disulfide, Imipenem, Imizin, Immulina, Immunoferon, Impulsin, Imrecoxib, Imutex, Indinavir, indiplon, indirubin, indole-3-acetic acid, indole-3-methanol, indolin-2-one, indolin-one, infliximab, inhibin B, INOmax, inositol-1,3,4,5-tetrakisphosphate, inulin, Iodoacetamide, iodomethane, iodoresiniferatoxin, Ionomycin, ionophore, Iopanoic Acid, Iophendylate, IPADE, IPOMEANOL, Iressa, irinotecan, irisolidone, Isatin, isaxonine, isoamylol, isobutylmethyl-Xanthine, Isodonol, isoflavone, isoflurane, Isol, Isoliquiritigenin, isometronidazole, isoprenoids, Isopropyl Thiogalactoside, Isoprostanes, Isorhamnetin, isosilybin A, Isosorbide Dinitrate, isothiocyanates, Isotretinoin, Isradipine, istradefylline, Itraconazole, ivabradine, Ivermectin, ixabepilone, jadomycin B, Jexin, JHW 015, JTE 013, K 252, K-PAM, K-SR, kaempferol, kaempferol-3-0-(2,3,4-tri-0-acetyl-alpha-1-rhamnopyranoside), KAFA, Kaken, Kamalin, Kaolin, Kathon 886, KB 141, Kemi, kenpaullone, Ketamine, Keto-desogestrel, Keto-pgflalpha, ke-ketoglutarate, Kipca, KMD 3213, KMTB, Kojic acid, KR-31543, KRM 1648, L 365260, L 740,093, L-454,560, L-696,474, L-T3, LAAM, lacidipine, lactacystin, lactisole, lamotrigine, Lanol, lansoprazole, lapatinib, laquinimod, latrunculin A, latrunculin B, lavendustin A, LBH589,

leflunomide, lenalidomide, Lendorm, Lentinan, leptomycin B, Leucovorin, Leukotriene C4, Leukotriene D4, leukotrienes, Leupeptin, Levamisole, Levitra, levobupivacaine, Levonorgestrel, levugen, liarozole, Lidocaine, lilopristone, Lipoate, Lipofectamine, lipoteichoic acid, Lipoxins, lissamine rhodamine B, lithocholic acid, LMWH, LNAC, lonafarnib, Loperamide, lopinavir, Loratadine, Lorazepam, Lorex, lorglumide, Losartan, Lovan, loxiglumide, LUF 5831, lupeol, luteolin, LY 117018, LY 293111, LY231514, LYCOPENE, lysophosphatidic acid, Lysophosphatidylcholines, Lysophosphatidylglycerol, lysyl-arginyl-alanyl-lysyl-alanyl-lysyl-threonyl-threonyl-lysyl-lysyl-arginine, M&B22948, Malix, manidipine, manumycin, maraviroc, Matrine, MCYST-LR, Me-nle-asp-phe-NH2, mead ethanolamide, MeAsO(OH)2, Mebumal, Mechlorethamine, Medroxyprogesterone 17-Acetate, Mefenamic Acid, Megalomicin, Melarsoprol, Melatol, meletin, melitten, meloxicam, Melphalan, Memantine, menadiol, Menhaden oil, menthofuran, Meperidine, Mephenytoin, mesalamine, Mesaton, Meth, methanandamide, methanethiosulfonate ethylammonium, Methimazole, methionyl-leucyl-phenylalanine, Methorphan, Methoxsalen, Methoxy-psoralen, methoxyamine, methoxychlor, methoxymorphinan, methyl chloroformate, Methyl glycine, Methyl paraben, methyl salicylate, methyl tryptophan, methyl-dopa, methyl-phosphorothioate, methyl-Pyridinium, methylamine, Methylamylnitrosamine, Methylene-tetrahydrofolate, methylenetetrahydrofolates, methylglyoxal, methylnaltrexone, methyloxidanyl, methylparaben, methylphosphate, Methylprednisolone, methylxanthines, Metoclopramide, Metopiron, Metribolone, mevalonic acid, micafungin, miconazole, Mictonorm, Midazolam, Mifepristone, MIII, Milrinone, Mimosine, mirtazapine, Mit-C, mithramycin, MitoTracker-Red, Mitoxantrone, mizolastine, MLN8054, mofarotene, Monensin, mono-N-demethyladinazolam, mono(2-ethylhexyl) phthalate, monoethylglycinexylidide, monomethylarsonic acid, monoterpenes, monuron, MORIN, morpholine, morusin, motexafin gadolinium, Motuporin, moxifloxacin, MPEG, Muraglitazar, mutalipocin II, mycophenolic acid, Mycose, Myocol, myricetin, myxothiazol, N-(2-cyclohexyloxy-4-nitrophenyl) methanesulfonamide, N-(2-hydroxypropyl)methacrylamide, N-(3-(4-chlorophenyl)-2-(3-cyanophenyl)-1-methylpropyl)-2-methyl-2-((5-(trifluoromethyl)pyridin-2-yl)oxy)propanamide, N-(3-methoxyphenyl)-4-chlorocinnamanilide, N-(3-oxododecanoyl)homoserine lactone, N-(4-(6-(4-(1-(4-fluorophenyl)ethyl)piperazin-1-yl)pyrimidin-4-yloxy)benzo (d) thiazol-2-yl)acetamide, N-(4-(6-(4-trifluoromethylphenyl)pyrimidin-4-yloxy)benzothiazol-2-yl)acetamide, N-(4-cyano-benzo(b) thiophene-2-carbonyl)guanidine, N-(5-(((5-(1,1-dimethylethyl)-2-oxazolyl)methyl)thio)-2-thiazolyl)-4-piperidinecarboxamide, N-acetylcysteine lysinate, n-acetylmuramyl-1-alanyl-d-isoglutamine, N-acetylneuraminic acid, N-desmethylclobazam, N-ethylmaleimide, N-methyl-N-(trimethylsilyl)trifluoroacetamide, N-methylsulfonyl-6-(2-propargyloxyphenyl)hexanamide, N-oleoyldopamine, N-phenyl-1-naphthylamine, N,N,N',N'-tetramethylethylenediamine, N(6)-cyclohexyl-2-O-methyladenosine, N(6)-cyclopentyladenosine, N3-IQ, Nadroparin, naftifine, nal-NH2, NALS, nanchangmycin, Naproxen, naratriptan, narbonolide, NARIGENIN, Narkotil, Nasol, natalizumab, nateglinide, Naxy, nebivolol, Nefazodone, nefiracetam, Nelfinavir, Neomycin, Neopterin, Neostigmine, Neut, Nevirapine, NFBA, Nialk, Nicardipine, Niflumic Acid, nimesulide, niobium, nitecapone, nitroanilide, nitroaspirin, Nitrofurans, NITROPYRENE, nitrosamines, Nitrosoanabasine, Nitrosocysteine, nitrosulindac, Nizatidine, NK 104, NK314, NMDA, NN 703, Noan, Nobiletin, NOC 18, Nocodazole, nodularin, Nodularin v, nolatrexed, Nonoxynol, noralfentanil, norbuprenorphine, Norclozapine, Nordihydroguaiaretic Acid, Norethindrone, noreximide, norfluoxetine, Norgestrel, norharman, norketobemidone, norlaudanosoline, normeperidine, Nortilidine, norverapamil, novobiocin, NS-187, NSC 23766, NSC 366140, NSC 663284, NSC-134754, NU2058, number-one, nutlin 3, NVP-AEW541, Nylon, O-(chloroacetylcarbamoyl)fumagillol, O-desethylreboxetine, O-Due, o-quinone, obovatol, OCDD, octanediol, Octoxynol, Octreotide, Okadaic Acid, olanzapine, olefins, oleoylethanolamide, olmelin, olmesartan, olomoucine, olomoucine II, Oltipraz, omalizumab, omega-agatoxin, omega-Conotoxin GVIA, omega-N-Methylarginine, Omeprazole, omeprazole sulfone, onapristone, ONCB, Ondansetron, ONO4819, Optef, OR 1246, oroxylin A, Orphenadrine, Osten, osteum, OSU 03012, Ouabain, OVEX, Ovex, oxaliplatin, Oxarol, oxaspirodion, oxatomide, Oxazepam, oxcarbazepine, Oxotremorine, oxotremorine M, Oxymorphone, Oxyntomodulin, Oxytrol, p-ABA, p-XSC, p-Xylol, Paclitaxel, paeonol, palladium, palmitoleate, PALMITOYL, Palmitoylcarnitine, pamidronate, panaxadiol, panepoxydone, pantoprazole, Papaverine, Papite, PAPP, parecoxib, Paroxetine, Parsal, Parthenolide, PC 314, PCA 4230, PCSO, PD 134308, PD 144795, PD 180988, PD 98059, pectin, Pemetrexed, Penicillins, Penite, Pentagastrin, Pentoxifylline, Peplomycin, peppermint oil, Pepstatin A, Perazine, Pergolide, Perillol, Perilymph, periodate, perospirone, perovskite, PFPA, Phebestin, phen, phenolate, Phenols, phenoxodiol, Phenprocoumon, Phentermine, phenyl-propionamide, phenyl-Pyridinium, Phenytoin, pheophorbide a, phloretin, PHOB, phorate, phorbol, phorbol 12-phenylacetate 13-acetate 20-homovanillate, phosphatidylethanolamines, Phosphatidylinositol 4,5-Diphosphate, phosphatidylinositol phosphate, PtdIns(4,5)P2, phytanic acid, Picibanil, picric acid, pifithrin, Pilot, pimecrolimus, pioglitazone, pipecoloxylidide, piperidine, piperine, Pira, pirinixic acid, Piroxicam, PKC412, plumbagin, Pluronic p 85, PMDT, PMPA, PMSF, PNPP, Podophyllotoxin, polidocanol, poly-gamma-glutamate, ponicidin, poractant alfa, posaconazole, potassium tellurate(IV), PQQ Cofactor, pranlukast, Pravastatin, Prazosin, PRDL, Precursor mrna, Prednisone, pregnane, Pregnanes, Pregnanolone, pregnenolone 16alpha-carbonitrile, Pregnyl, preussin, Primidone, Proadifen, Proanthocyanidins, Probenecid, Probucol, Procasil, Procetofen, procyanidin B2, Prodix, prolactin, polymeric, Propafenone, Propanesulfonate, Propofol, propyl pyrazole triol, propyne, prostratin, protopanaxadiol, protopanaxatriol, PS 15, Pseudohypericin, Pseudomonas-exotoxin, Psoralens, psychosine-3'-sulfate ester, PTBP, pteridine, Pterostilbene, PURAC, Puromycin, putrescine, Pyocyanine, Pyra, pyranones, pyrazole, Pyrethrins, pyridazine, Pyrimethamine, pyrimidin-2-one beta-ribofuranoside, Pyro, pyrogallol sulfonphthalein, pyrrole-2-carboxylic acid, pyrrolidine dithiocarbamic acid, pyrroloazepinone, Qingkailing,

quercitrin, quetiapine, Quicifal, quinazoline, Quinolinium, Quinpirole, quinuclidin-3'-yl-1-phenyl-1,2,3,4-tetrahydroisoquinoline-2-carboxylate monosuccinate, quinupris-quinupristin-dalfopristin, R-138727, R-99224, Raloxifene, raltitrexed, Ramipril, ramiprilat, RAMP, Ranitidine, RAPA, rasagiline, rebamipide, reboxetine, remifentanil, renzapride, repaglinide, Resiniferotoxin, resiquimod, Retardex, Riacon, Ribavirin, Riboflavin, Rifabutin, rifamycins, Rifocin, rimonabant, risedronic acid, risperidone, Ristocetin, Ritonavir, rituximab, Ro 13-8996, Ro 23-7553, Ro 23-7637, Ro 24-7429, Ro 31-6233, Ro 31-7549, Ro 31-8220, RO4383596, Robitet, rofecoxib, roflumilast, rokitamycin, Rolipram, romidepsin, rooperol, ropivacaine, roscovitine, rosiglitazone, rosmarinic acid, rosuvastatin, Roxithromycin, Rozevin, RPR 121056, RU 58668, ruboxistaurin, rugosin E, rutecarpine, Rutin, S-(beta-p-methoxypropiophenone)thiamine, S-Nitroso-N-Acetylpenicillamine, S-Nitrosothiols, S-phenyl-N-acetylcysteine, sabarubicin, sabcomeline, Safingol, Safrole, SAGA, SAHA, saikosaponin, Salicin, salvin, samarium, SAMe, sanguinarine, sapogenins, Saquinavir, Sarasar, Sarna, sauchinone, saxatilin, SB 218078, SB 225002, SB 415286, SB-705498, scandium, SCH 66712, schizandrer A, scoparone, Scopoletin, Score, SDX 308, Selegiline, seocalcitol, Sep-Pak, Serad, sertindole, sevoflurane, SEW2871, shikonin, siderophore, Sildenafil, silvestrol, silybin, Sincalide, Sizofiran, SK-7041, SK&F 106528, SM 7368, Sodium pentosan poly sulfate, Sodium Salicylate, sorafenib, sorbinil, Sorbo, Sorbose, spiroglumide, Spironolactone, squamocin, SR 144528, SR 27897, SR 48692, SR 80327A, SR 90107A-ORG 31540, ST 638, stallimycin, Stanozolol, staurosporine, Stearin, Stereoisomerism, Steviol, Stevioside, STIL, stilbene-disulphonate, Stilbenes, Stim, Streptomycin, Styrene, styrene-methylmethacrylate copolymer, SU 5416, SU 6668, SU 9516, suberate, suberosin, succinic semialdehyde, Sufentanil, Suldox, sulfadoxine-pyrimethamine, Sulfamethazine, sulfamethoxazole hydroxylamine, Sulfaphenazole, Sulfasalazine, sulfate cellufine, sulfate-sulfate, sulfidonitrogen(.), Sulfinpyrazone, sulfo-N-succinimidyl oleate, sulfo-succinimidyl-oleate, sulfogalactosylglycerolipid, sulfones, sulfonic acid, sulfonyl-phenyl-ethyl, Sulforafan, Sulindac, sulindac sulfone, sultopride, sunitinib, Synthos, T 0070907, T 0901317, Tacrine, Tacrolimus, tadalafil, Tamogel, Tamoxifen, tandospirone, Tangeretin, tanshinone, taurocholic acid, Taurodeoxycholic Acid, tauroursodeoxycholic acid, tautomycetin, TAXOTERE, TBDZ, TBHQ, TCAT, technetium, Tegafur, Teleocidin, telithromycin, Temazepam, temozolomide, temsirolimus, terbinafine, terephthalic acid, Terfenadine, teriflunomide, terrein, territrem A, territrem B, territrem C, tertiapin, tetra-mu3-sulfido-tetrairon, tetrachloroethene, tetradecanoyl-phorbol-acetate, Tetrahydrocannabinol, tetramethylrhodamine, tetramethylsilane, tetraphene, Tetraprenol, tetrasulfanide, Thalidomide, Thapsigargin, thiamine disulfide, thiazole, Thiazolidinediones, thioacetamide, Thioacetazone, thiobenzamide, thiocoraline, Thiole, Thiopental, thioredoxin dithiol, thioridazine, Thiostrepton, thrombin receptor peptide SFLLRNP, thrombin Tokushima, Thromboxane A2, thromboxane B2, Thyminose, thymol, thymoquinone, Thyrotropin, Ticlopidine, Tilidine, tipranavir, tirilazad, titanium alloy (TiAl6V4), TMC-95A, Tmndga, TMSI, Tobrex, tocotrienols, tofisopam, tolrestat, Tolterodine, toluene, TOLUENE-DITHIOL, topiramate, Topotecan, Toremifene, Tosylarginine Methyl Ester, Tosyllysine Chloromethyl Ketone, Tosylphenylalanyl Chloromethyl Ketone, TPN+, Tracer, Tramadol, trans-resveratrol, trastuzumab, Trazodone, Tremode, Tremorine, Tretinoin, Triad, Triamcinolone, triazolam, triazoles, tributylstannane, trichostatins, Triclosan, triethylamine, Trifluoperazine, trimethylaminocarboxyldihydroboran, trioctyl phosphine oxide, Triolein, tripterine, triptolide, triterpenoids, troglitazone, Troleandomycin, TTNPB, tubocapsanolide A, Tunicamycin, tyrphostin AG 1478, tyrphostin AG-490, tyrphostin AG17, U 0126, Ubizol, Ufur, UK 157147, Uprima, uranyl acetate, urethane, Urex, urinastatin, Urso, USAN, valerenic acid, valspodar, venlafaxine, Verapamil, verlukast, vesnarinone, Viagra, Vigil, vincristine, vinflunine, vinorelbine, vinpocetine, violacein, Vira-A, voriconazole, vorozole, VX680, Warfarin, WAY-169916, Win 55212-2, withaferin A, WLN: QR BG, WLN: RVR, WLN: ZSWR, xanthohumol, Xaxa, Xylit, Y 27632, yristate, Z 338, zafirlukast, Zalcitabine, zardaverine, ZD 9331, Zearalenone, Zeldox, zerumbone, Zidovudine, zileuton, Zocor, zopiclone, Zymosan, Trospium chloride, Valproic Acid. These compounds interact with newly identified key regulators of pain sensation and nociception. Mechanistically the inventive treatments involve the modulation of the genes and gene function or interaction with the gene products, in particular proteins, of the genes listed in table 1 or the human orthologues of the Drosophila genes described in Neely et al., 2010, or preferably the human genes listed in table 1.

According to the investigation described herein it was found that these compounds modify at least one gene or gene product thereof selected from the CACNA2D3 (gene for calcium channel subunit alpha-2-delta-3), PIK3CG, or any one of the human genes of table 1, column 2, or any of the human orthologues of the Drosophila genes described in Neely et al., 2010, or any drosophila gene of table 1 column 3, in particular preferred any one of CG10033, CG10095, CG10142, CG10153, CG10158, CG10186, CG10186, CG10228, CG10265, CG1031, CG10332, CG10332, CG10481, CG10537, CG10550, CG1058, CG10583, CG10603, CG10603, CG10612, CG10641, CG10667, CG10686, CG10689, CG10689, CG10691, CG10706, CG10711, CG10728, CG10746, CG10800, CG10823, CG1086, CG10882, CG10932, CG10936, CG10954, CG10988, CG1100, CG1100, CG1101, CG11033, CG11081, CG11183, CG1119, CG11280, CG1130, CG11352, CG11456, CG11508, CG1152, CG11555, CG11577, CG11586, CG11590, CG11592, CG11594, CG11637, CG11637, CG11638, CG11642, CG11715, CG1180, CG1180, CG11820, CG11857, CG11865, CG11878, CG11893, CG11895, CG1193, CG11930, CG11942, CG11967, CG11992, CG12004, CG12030, CG12035, CG12052, CG12079, CG12082, CG12093, CG12131, CG12135, CG12199, CG12209, CG12235, CG12269, CG12290, CG12334, CG12373, CG12559, CG12637, CG1264, CG12641, CG12641, CG12645, CG12663, CG12749, CG12796, CG12797, CG12831, CG12878, CG12932, CG12938, CG12954, CG12975, CG13035, CG13047, CG13061, CG13069, CG13074,

CG13096, CG13110, CG13130, CG13130, CG13162, CG13194, CG13196, CG13196, CG13243, CG13308, CG13319, CG13403, CG13559, CG13575, CG13623, CG1371, CG1387, CG13998, CG14028, CG1406, CG14065, CG14086, CG14214, CG14217, CG14240, CG14252, CG14274, CG14351, CG14362, CG14374, CG14374, CG14376, CG14506, CG14514, CG14590, CG14659, CG14710, CG14750, CG14755, CG14804, CG14818, CG14940, CG14952, CG14980, CG15059, CG15120, CG15153, CG15167, CG15254, CG15307, CG15321, CG15324, CG15427, CG15570, CG15604, CG15604, CG15884, CG16778, CG1683, CG16840, CG16852, CG16854, CG16873, CG16899, CG16932, CG16975, CG17003, CG17027, CG1709, CG17137, CG17146, CG17150, CG17189, CG17234, CG1725, CG17255, CG17266, CG17293, CG17295, CG17521, CG1759, CG17612, CG17673, CG17697, CG17943, CG1800, CG1804, CG18069, CG18088, CG18130, CG18249, CG18332, CG18332, CG18350, CG18350, CG18350, CG18350, CG18372, CG1845, CG18480, CG18624, CG18624, CG18666, CG1915, CG1921, CG1921, CG1966, CG1968, CG1982, CG2038, CG2060, CG2079, CG2100, CG2109, CG2128, CG2158, CG2161, CG2257, CG2257, CG2286, CG2346, CG2371, CG2371, CG2503, CG2522, CG2747, CG2848, CG2848, CG2872, CG2872, CG2901, CG3000, CG30004, CG30004, CG30005, CG30039, CG30050, CG30073, CG30291, CG30342, CG30383, CG30384, CG30404, CG3083, CG3105, CG31065, CG31068, CG31110, CG31267, CG31299, CG31300, CG31623, CG31713, CG31716, CG31718, CG3181, CG3184, CG31841, CG31842, CG31876, CG31886, CG31908, CG31936, CG31955, CG31962, CG32016, CG32025, CG32045, CG32057, CG32121, CG3213, CG32148, CG32150, CG32176, CG32193, CG32219, CG32227, CG3224, CG32278, CG32296, CG32296, CG32313, CG32333, CG32346, CG3241, CG32531, CG32533, CG32540, CG32604, CG32614, CG32678, CG32678, CG32678, CG32678, CG3269, CG32698, CG3270, CG32703, CG32736, CG32779, CG32779, CG32779, CG32792, CG3291, CG3295, CG3298, CG33002, CG33106, CG33106, CG33106, CG33106, CG33106, CG33106, CG33128, CG33135, CG33147, CG33149, CG33166, CG33202, CG33261, CG33275, CG33275, CG3330, CG33346, CG33350, CG3344, CG33484, CG33500, CG33500, CG3351, CG33512, CG33512, CG33530, CG33547, CG33547, CG33653, CG33980, CG34059, CG34140, CG34140, CG34159, CG3421, CG34339, CG34341, CG34364, CG34383, CG34400, CG34401, CG34401, CG34416, CG3474, CG3520, CG3569, CG3581, CG3604, CG3613, CG3619, CG3619, CG3619, CG3707, CG3711, CG3717, CG3735, CG3905, CG3943, CG3949, CG3955, CG3981, CG4013, CG4040, CG4083, CG4094, CG4109, CG4217, CG42250, CG4247, CG4247, CG4260, CG4279, CG4279, CG4351, CG4365, CG4396, CG4459, CG4477, CG4502, CG4550, CG4560, CG4584, CG4587, CG4612, CG4633, CG4633, CG4648, CG4655, CG4673, CG4692, CG4698, CG4742, CG4775, CG4795, CG4798, CG4799, CG4806, CG4807, CG4830, CG4875, CG4875, CG4887, CG4946, CG4975, CG4977, CG5003, CG5012, CG5012, CG5013, CG5014, CG5014, CG5121, CG5134, CG5160, CG5179, CG5183, CG5198, CG5201, CG5219, CG5219, CG5261, CG5287, CG5330, CG5405, CG5411, CG5473, CG5499, CG5516, CG5519, CG5565, CG5580, CG5611, CG5643, CG5644, CG5703, CG5723, CG5725, CG5725, CG5727, CG5757, CG5788, CG5800, CG5818, CG5819, CG5821, CG5842, CG5884, CG5889, CG5890, CG5902, CG5934, CG5940, CG5949, CG5969, CG5977, CG5986, CG6006, CG6098, CG6136, CG6143, CG6177, CG6210, CG6249, CG6340, CG6395, CG6457, CG6496, CG6536, CG6549, CG6553, CG6571, CG6575, CG6582, CG6583, CG6605, CG6620, CG6637, CG6673, CG6703, CG6721, CG6724, CG6822, CG6824, CG6930, CG6930, CG6946, CG6948, CG6963, CG6971, CG6971, CG6972, CG6987, CG6998, CG7006, CG7007, CG7007, CG7010, CG7015, CG7025, CG7059, CG7081, CG7099, CG7100, CG7109, CG7129, CG7145, CG7160, CG7175, CG7175, CG7187, CG7194, CG7211, CG7223, CG7225, CG7292, CG7311, CG7342, CG7358, CG7371, CG7376, CG7422, CG7430, CG7443, CG7467, CG7494, CG7494, CG7497, CG7507, CG7556, CG7583, CG7636, CG7664, CG7708, CG7708, CG7712, CG7728, CG7730, CG7800, CG7800, CG7811, CG7816, CG7856, CG7873, CG7946, CG7957, CG8005, CG8008, CG8009, CG8014, CG8014, CG8029, CG8039, CG8048, CG8107, CG8110, CG8114, CG8203, CG8233, CG8288, CG8325, CG8326, CG8394, CG8432, CG8436, CG8440, CG8487, CG8520, CG8625, CG8631, CG8651, CG8732, CG8764, CG8849, CG8912, CG8914, CG8985, CG8985, CG9022, CG9022, CG9032, CG9067, CG9102, CG9160, CG9172, CG9231, CG9280, CG9311, CG9323, CG9350, CG9388, CG9447, CG9453, CG9460, CG9519, CG9537, CG9548, CG9603, CG9636, CG9636, CG9650, CG9696, CG9739, CG9742, CG9753, CG9753, CG9825, CG9825, CG9901, CG9901, CG9948, as well as their orthologues, in particular human orthologues. Furthermore preferred genes (or gene targets) for the inventive treatment are selected from CG10882, CG10033, CG10095, CG10096, CG10096, CG10098, CG10142, CG10153, CG10158, CG10186, CG10186, CG10200, CG10228, CG1031, CG10315, CG10332, CG10332, CG10481, CG10540, CG10550, CG1058, CG10583, CG10603, CG10603, CG10612, CG10641, CG10667, CG10689, CG10691, CG10711, CG10728, CG10746, CG10754, CG10800, CG10823, CG1086, CG10872, CG10932, CG10936, CG10954, CG10988, CG10992, CG1100, CG1100, CG1101, CG11033, CG11081, CG11081, CG11183, CG1119, CG11280, CG11339, CG11419, CG11456, CG11508, CG11512, CG1152, CG11555, CG11577, CG11586, CG11590, CG11592, CG11637, CG11637, CG11638, CG11715, CG1180, CG1180, CG11820, CG11857, CG11865, CG11878, CG11893, CG11895, CG1193, CG11942, CG11953, CG11967, CG11992, CG12004, CG12030, CG12035, CG12079, CG12082, CG12082, CG12083, CG12131, CG12135, CG12209, CG12235, CG12334, CG12373, CG12532, CG12637, CG12645, CG12663, CG12785, CG12796, CG12797, CG12831, CG12915, CG12927, CG12932, CG12938, CG12954, CG12975, CG13035, CG13047, CG13069, CG13074, CG13096, CG13110, CG13162, CG13196, CG13243, CG13308, CG13319, CG13388, CG13403, CG13534, CG13548, CG13623, CG1371, CG1375, CG13777, CG13788, CG13802, CG13802, CG1387, CG13922, CG13998,

CG14028, CG1406, CG14075, CG14086, CG14198, CG14214, CG14217, CG14240, CG14252, CG14255, CG14274, CG14351, CG14353, CG14365, CG14374, CG14374, CG14376, CG14429, CG14442, CG14506, CG14514, CG14707, CG14707, CG14750, CG14804, CG14818, CG14940, CG14980, CG15071, CG15120, CG15167, CG15238, CG15241, CG15254, CG15321, CG15324, CG15427, CG15433, CG15570, CG15604, CG15604, CG15884, CG16725, CG16775, CG16778, CG16852, CG16854, CG16873, CG16899, CG16932, CG17003, CG17027, CG1709, CG17137, CG17146, CG17150, CG17189, CG17203, CG17234, CG1725, CG17266, CG17293, CG17295, CG17386, CG17521, CG17650, CG17673, CG17697, CG17943, CG1800, CG18026, CG1804, CG18069, CG18088, CG18130, CG18213, CG18249, CG18332, CG18332, CG18350, CG18350, CG18350, CG18350, CG18372, CG1847, CG18480, CG18557, CG18624, CG18624, CG18666, CG18671, CG18671, CG18771, CG18816, CG18816, CG18833, CG1915, CG1922, CG1966, CG1968, CG1982, CG2038, CG2052, CG2060, CG2100, CG2109, CG2128, CG2151, CG2158, CG2161, CG2257, CG2257, CG2286, CG2309, CG2346, CG2371, CG2371, CG2503, CG2522, CG2685, CG2698, CG2713, CG2747, CG2848, CG2848, CG2848, CG2872, CG2872, CG3000, CG30005, CG30025, CG30025, CG30025, CG30025, CG30039, CG30050, CG30073, CG30113, CG30186, CG30291, CG30352, CG30383, CG30384, CG30404, CG30427, CG30474, CG3083, CG3105, CG31065, CG31067, CG31067, CG31068, CG31103, CG31110, CG31170, CG31183, CG31267, CG31299, CG31300, CG31309, CG31364, CG31406, CG31623, CG31654, CG31713, CG31718, CG3181, CG3184, CG31841, CG31842, CG31876, CG31893, CG31908, CG31936, CG31955, CG31962, CG32006, CG32016, CG32025, CG32057, CG3213, CG32131, CG32148, CG32150, CG32176, CG32219, CG32227, CG3224, CG32278, CG32283, CG32313, CG32333, CG32381, CG3240, CG3240, CG3241, CG32467, CG32478, CG32531, CG32614, CG32678, CG32678, CG32678, CG32678, CG3269, CG32779, CG32792, CG3291, CG3298, CG33002, CG3330, CG33346, CG3344, CG33530, CG3421, CG3520, CG3581, CG3604, CG3612, CG3613, CG3619, CG3619, CG3707, CG3711, CG3717, CG3717, CG3733, CG3735, CG3905, CG3943, CG3949, CG3955, CG3955, CG3981, CG3996, CG4073, CG4083, CG4109, CG4110, CG4217, CG4247, CG4247, CG4279, CG4279, CG4351, CG4365, CG4396, CG4453, CG4459, CG4477, CG4550, CG4560, CG4581, CG4584, CG4587, CG4609, CG4612, CG4633, CG4633, CG4648, CG4655, CG4673, CG4698, CG4742, CG4772, CG4775, CG4795, CG4798, CG4799, CG4806, CG4830, CG4863, CG4875, CG4887, CG4946, CG4975, CG4977, CG4994, CG5003, CG5012, CG5012, CG5013, CG5014, CG5014, CG5121, CG5160, CG5179, CG5183, CG5198, CG5201, CG5219, CG5219, CG5254, CG5261, CG5287, CG5330, CG5380, CG5411, CG5473, CG5499, CG5507, CG5516, CG5519, CG5541, CG5565, CG5580, CG5611, CG5625, CG5643, CG5644, CG5703, CG5711, CG5714, CG5723, CG5725, CG5725, CG5727, CG5751, CG5757, CG5788, CG5818, CG5819, CG5820, CG5821, CG5842, CG5884, CG5889, CG5890, CG5902, CG5934, CG5940, CG5949, CG5969, CG5977, CG5986, CG5989, CG6006, CG6006, CG6027, CG6066, CG6098, CG6136, CG6143, CG6177, CG6210, CG6249, CG6294, CG6340, CG6375, CG6457, CG6496, CG6553, CG6571, CG6582, CG6583, CG6605, CG6620, CG6637, CG6673, CG6703, CG6721, CG6724, CG6822, CG6852, CG6881, CG6901, CG6930, CG6930, CG6946, CG6948, CG6963, CG6971, CG6971, CG6972, CG6987, CG6998, CG7006, CG7007, CG7007, CG7010, CG7015, CG7042, CG7042, CG7059, CG7100, CG7109, CG7129, CG7145, CG7160, CG7172, CG7175, CG7175, CG7187, CG7194, CG7211, CG7223, CG7225, CG7307, CG7311, CG7342, CG7358, CG7371, CG7376, CG7422, CG7430, CG7436, CG7443, CG7462, CG7467, CG7494, CG7494, CG7513, CG7520, CG7556, CG7580, CG7583, CG7636, CG7664, CG7693, CG7708, CG7708, CG7712, CG7726, CG7728, CG7730, CG7778, CG7800, CG7800, CG7811, CG7816, CG7856, CG7873, CG7946, CG7957, CG8005, CG8008, CG8009, CG8014, CG8014, CG8029, CG8039, CG8039, CG8048, CG8107, CG8110, CG8114, CG8137, CG8203, CG8233, CG8288, CG8325, CG8326, CG8394, CG8432, CG8436, CG8440, CG8487, CG8520, CG8625, CG8631, CG8651, CG8663, CG8732, CG8764, CG8771, CG8849, CG8912, CG8914, CG9022, CG9022, CG9032, CG9067, CG9102, CG9160, CG9172, CG9231, CG9280, CG9288, CG9305, CG9311, CG9323, CG9343, CG9350, CG9388, CG9447, CG9453, CG9460, CG9519, CG9548, CG9588, CG9603, CG9633, CG9636, CG9636, CG9650, CG9678, CG9696, CG9696, CG9720, CG9742, CG9753, CG9753, CG9825, CG9825, CG9901, CG9901, CG9948, CG9948, CG9983, as well as their orthologues, in particular human orthologues (such as described in Neely et al., 2010, incorporated herein by reference). These genes are referred herein as "pain genes". Preferred genes are selected from CG10095, CG10096, CG10098, CG10158, CG10481, CG11033, CG11456, CG11577, CG11586, CG11590, CG11592, CG11820, CG11967, CG12004, CG12334, CG12785, CG12797, CG13096, CG13162, CG13623, CG1371, CG14351, CG14442, CG14514, CG14980, CG16725, CG16854, CG1804, CG18088, CG18130, CG18213, CG18249, CG18480, CG1968, CG2052, CG2747, CG30005, CG31103, CG31267, CG31955, CG3213, CG32150, CG3224, CG32792, CG33346, CG3996, CG4110, CG4351, CG4477, CG4946, CG5516, CG5565, CG5819, CG5969, CG5986, CG6136, CG6294, CG6340, CG6553, CG6583, CG6637, CG6724, CG6852, CG6901, CG7006, CG7042, CG7175, CG7358, CG7376, CG7556, CG7728, CG7800, CG8233, CG8325, CG8436, CG8771, CG9067, CG9288, CG9636, CG9650. These genes, as well as their orthologue counterparts, in particular human orthologs (Neely et al., 2010), or their respective gene products are preferred targets for therapy according to the present invention. According to the present invention function of at least one of these genes is modified by the inventive compounds, in particular the small molecules given in table 1. In preferred embodiments the compound modulates at least two, three, four, five or six or more of these genes (or orthologues). Further compounds suitable to modulate gene function include the administration of therapeutic proteins or nucleic acids,

such as transgenes or inhibitory nucleic acids (RNAi molecules, siRNA, antisense RNA or DNA). Such interfering nucleic acids bind messages of the genes leading to degradation and reduced gene expression. Preferred therapeutic proteins include the gene products of these genes (as agonists) or antibodies which specifically bind these proteins (as antagonists, but also as agonists if protein activity is increased - such as by binding and blocking an inhibitor binding site). The inventive compounds can act as either agonist by increasing the gene function (via mRNA regulation or interaction with the protein) of a protein in the enzymatic pathway of any one of the above listed genes or an antagonist in said pathways. The antagonizing or activating (agonist) activity of the compounds acts preferably on the identified pain genes (including their gene product) themselves or on a binding partner thereof. With the inventive methods it is possible to suppress pain or, alternatively to increase pain, e.g. to treat hyposensitivities. Depending on the goal an antagonist or agonist of the gene targets may be used. In preferred embodiments antagonists of the pain genes are used.

The subject to be treated according to the present invention can be any non-human animal or a human. Preferably the subject is a mammal, in particular preferred embodiments a human.

According to the present invention pain and conditions associated with pain, e.g. itching or depression, can be treated or prevented, in particular in the meaning of a prophylactic administration. "Preventing" or "prevention" herein does not require absolute success in the sense of an absolute prevention of pain but indicates a reduced risk of developing a disease or painful condition, or developing pain with reduced severity. Likewise, "treatment" shall not be construed as an absolute cure, but may also relate to amelioration or suppression of pain or pain associated conditions.

Pain and pain associated conditions and diseases to be treated according to the present invention can include acute pain, chronic pain, somatogenic pain, neuropathic pain, psychogenic pain, heat induced pain, physical pain and nociception in general, or hyperalgesia. In particular embodiments the pain is selected from neuropathic pain, inflammatory pain, nociceptive pain, rheumatic pain, headache, low back pain, pelvic pain, myofascial pain, vascular pain, migraine, wound associated pain, inflammatory pain, arthritic pain, diabetic pain, pain from cancer or somatic visceral pain, all in both acute and chronic forms. The pain can also be related to phantom pain, pain from a part of the body that has been lost or from which the brain no longer receives physical signals.

Pain can be generally classified to two broad categories, acute and chronic. The treatment of any acute or chronic pain is subject matter of the present invention. Acute pain is usually associated with a specific cause such as a specific injury and is often sharp and severe. Acute pain begins suddenly and is not persistent. Chronic pain is long-term pain, with a typical duration of more than three months leading to significant psychological and emotional problems. Chronic pain is generally associated with clinical conditions characterised by chronic and/or degenerative lesions. Common examples of chronic pain are neuropathic pain (e.g. painful diabetic neuropathy, post-herpetic neuralgia), rheumatoid arthritis, osteoarthritis, fibromyalgia, back pain, headache, carpal tunnel syndrome, cancer pain, and chronic post-surgical pain. Pain can also be divided into a number of different subtypes according to differing pathophysiology, including nociceptive, inflammatory and neuropathic pain. Also some types of pain can be classified in multiple categories, for example pain associated with cancer can have a nociceptive and neuropathic component. Nociceptive pain consists of somatic pain (musculo-skeletal pain) and visceral pain (pain associated with the viscera, which encompass the organs of the abdominal cavity). Common causes of somatic pain include cancer metastasis such as to the bone and postsurgical pain from a surgical incision in addition to musculo-skeletal disorders such as dystrophinopathy, myalgia and polymyositis. Nociceptive pain also includes tissue injury-induced pain and inflammatory pain such as that associated with arthritis. Another type of inflammatory pain is visceral pain which includes pain associated with gastrointestinal disorders (GI) such as functional bowel disorder (FBD) and inflammatory bowel disease (IBD). Further examples of visceral pain include the pain associated with dysmenorrhea, cystitis and pancreatis and pelvic pain. Additional pain types include dysfunctional pain such as fibromyalgia, Temporomandibular Joint Disorder (TMJ), Irritable bowel syndrome (IBS) and musculo-skeletal pain). Neuropathic pain is caused by damage to the peripheral or central nervous system. Examples of central neuropathic pain include pain from spinal cord injury, multiple sclerosis, strokes and fibromyalgia. Diabetes and related metabolic disorders are a common cause of peripheral neuropathic pain (diabetic neuropathy). Some of the human conditions and pathologies characterised by the presence of neuropathic pain include, but are not limited to, cancer (cancer neuropathy), HIV neuropathy, Parkinson’s disease, epilepsy, immunodeficiency, post-herpetic syndromes, trauma, ischaemia, sciatica, multiple sclerosis, peripheral neuropathy, trigeminal neuralgia, back pain, phantom limb pain, carpal tunnel syndrome, central post-stroke pain and pain associated with chronic alcoholism, hypothyroidism, uraemia, spinal cord injury, and vitamin deficiency. Preferably the pain is neuropathic pain, such as trigeminal neuralgia, such as post-herpetic neuralgia, such as painful diabetic neuropathy, such as painful diabetic peripheral neuropathy, such as diabetic polyneuropathy, such as sciatic pain, such as radiculopathy, such as radicular pain or such as non-inflammatory neuropathic pain. Pain may be selected from fibromyalgia, postoperative pain, trigeminal neuralgia, post-herpetic neuralgia, painful diabetic neuropathy, painful diabetic peripheral neuropathy, diabetic polyneuropathy, sciatic pain, radiculopathy, radicular pain, lumbar pain. Preferably the pain is caused by the conditions as mentioned above related to the given pain type. In particular the pain type can be the only pain type in a subject. E.g. preferably a neuropathic pain is caused by affected nerves but not caused by inflammation, i.e. neuropathic pain is the only pain in the subject and is non-inflammatory.

"About" is used to refer to certain dosages that can vary from a given value, nevertheless with the same effects as the indicated dose. In some embodiments "about" may refer to +/- 20% or 10% of a given value.

Preferably the compound is administered in a dosage sufficient to treat or prevent pain or associated conditions and diseases. Administration can e.g. be a singe dose administration or a successive or repeated administration, e.g. twice a day, daily or in an interval of at least 1 day, at least 2 days, at least 3 days, at least 1 week, preferably at least 2 weeks, at least 4 weeks, at least 8 weeks or even more preferred at least 12 weeks. Preventive administrations are usually a short time before expected pain, if controllable or foreseeable - such as in scheduled surgery - e.g. up to 1hour (h), 2h, 3h, 4h, 5h, 6h, 8h, 10h, 12h or up to 24h or even up to 48h beforehand, as well as any interval in between.

According to a further preferred embodiment of the present invention, the compound is provided in a pharmaceutical composition or a medicament, in particular an analgesics. The composition or medicament may comprise a pharmaceutical carrier. Pharmaceutical carrier substances serve for a better tolerance of the medicament and allow for a better solubility as well as a better bioavailability of the active substances contained in the medicament. Examples of this are emulsifiers, thickening agents, redox components, starch, alcohol solutions, polyethylene glycol or lipids. The choice of a suitable pharmaceutical carrier is highly dependent on the manner of administration. For oral administrations, liquid or solid carriers may be used, for injections, liquid final compositions are required. For cellular targeting, such as for inhibitory nucleic acids, suitable vehicles can be included such as liposomes or microsomes.

Preferably, the medicament or the compound to be used according to the invention comprises buffer substances or tonic substances. By means of a buffer, the pH of the medicament can be adjusted to physiological conditions, and moreover, pH fluctuations can be attenuated, or buffered, respectively. An example thereof is a phosphate buffer. Tonic substances serve for adjusting the osmolarity and may comprise ionic substances, such as, e.g., inorganic salts, such as NaCl, or also non-ionic substances, such as, e.g. glycerol or carbohydrates.

The inventive compound or medicament can be administered topical, enteral or parenteral, in particular preferred oral or rectal, intravenous, intraarterial, intramuscular, subcutaneous, intradermal or intraperitoneal, transdermal, transmucosal or inhalational. Preferred routes of administration of the inventive agent according to the present invention are parenteral routes, preferably intraperitoneal or intravenous administration, intravenous administration being specifically preferred. Intravenous administration can be performed e.g. via bolus injection or by continuous intravenous delivery over a longer time period (e.g. 30 min to 6 h, especially 1 to 3 h). Further routes include oral or transdermal or subcutaneous routes. In particular preferred is oral administration. For digestible agents, such as active proteins, peptides or siRNA, parenteral routes are preferred.

The medicament or the compound to be used according to the invention can be prepared to be suitable for oral or intranasal administration. These administration forms of the medicament of the present invention allow for a rapid an uncomplicated uptake of the active substances via the mucous membranes. For a nasal intake, nose drops or nose sprays are suitable. For an oral administration, solid or liquid medicaments may, e.g., be taken directly or in a dissolved or diluted state, respectively.

The medicament or compound to be used according to the invention can be prepared for an intravenous, intraarterial, intramuscular, intravascular, systemic, intraperitoneal or subcutaneous administration. For this purpose, e.g., injections or transfusions are suitable. Administrations directly into the bloodstream have the advantage that the active substances of the medicament will be distributed in the entire body and will quickly reach the target tissue or cells, in particular the peripheral nerves, spinal cord cells or brain cells.

The compound may be administered in a effective therapeutic dose. Effective doses are in the range of dosages known for the compounds for other, non-pain related administrations. In particular, for a specific use a dosage can be determined by a simple test using drosophila or mouse test systems. Further possible therapeutic doses of the compounds for the inventive treatment can be the same dosage disclosed or approved for other therapeutic uses for each of these compounds. Example dosages are at least 0.01 mg/kg, at least 0.1 mg/kg, at least 1 mg/kg, at least 10 mg/kg and/or up to 1 mg/kg, up to 10 mg/kg, up to 100 mg/kg, up to 1 g/kg, and any dosages in between. Preferred dosage ranges are between 0.01 mg/kg and 1 g/kg, preferably between 0.1 mg/kg and 100 mg/kg.

The examples show that the inventive pain tests revealed pharmaceutical compounds that are well known to be therapeutically applicable for the treatment of human conditions and diseases. The compounds may now also be used for the treatment of pain and pain associated secondary diseases. Of course the full list of compounds according to table 1 provides new therapeutic concepts.

The present invention also relates to a method of modulating the gene expression or gene function in a cell, wherein the gene is selected from one or more of the genes listed in table 1, in particular selected from the genes above, or an ortholog counterpart thereof, comprising administering a compound of table 1 to said cell. The cell can be a nerve cell, including pain or thermosensitive nerve cells, and/or preferably selected from spinal cord cells, brain cells or peripheral nerve cells. The cell can be of the "pain matrix" such as the thalamus, the S1 and S2 somatosensory cortex, the cingulum, amygdala, hypothalamus, or the motor cortex. The inventive administration be be for treatment, alleviation or prevention of pain or hyperalgesia in a subject.

In a further aspect the present invention relates to method of screening active compounds suitable for the

treatment of pain comprising testing for modulation, including suppression or activation, preferably suppression, of gene activity of any one of the genes listed above or given in table 1. The test may comprise recombinantly expressing the gene product in a suitable host cell or cell lines, such as mammal cell lines, in particular CHO cells, contacting said cell or cell line with a candidate compound and detecting a deviation in gene function when compared to normal levels without contacting with the compound. Further tests compromising binding of the compound to the gene product (the expressed protein) and detecting binding events. Other tests include the use of animal models and testing the compounds for behavioural changes when exposed to pain, such tests are disclosed in the examples. Additional, information on optimal dosages can be obtained with these tests.

**Table 1:** List of therapeutic compounds: Small Molecule: name of compound (see list of synonyms below), Interacting Gene Symbol: human gene name of therapeutic target, Interacting GeneID: uman gene ID, Drosophila ID: ortholog Drosophila gene ID;

| Small Molecule | Interacting Gene Symbol | Interacting GeneID | Drosophila Id |
|---|---|---|---|
| (1S,2S)-2-(2-(N-((3-benzimidazol-2-yl)propyl)-N-methylamino)ethyl)-6-fluoro-1,2,3,4-tetrahydro-1-isopropyl-2-naphtyl cyclopropanecarboxylate dihydrochloride | CYP3A4, | 1576, | CG2060, |
| (5-(2-methoxy-5-chloro-5-phenyl)furan-2-ylcarbonyl) guanidine | CYP3A4, | 1576, | CG2060, |
| (6S)-5,6,7,8-tetrahydrofolic acid | TYMS, | 7298, | CG3181, |
| (T,G)-A-L | FOXP3, | 50943, | CG16899, |
| 1 alpha-hydroxyergocalciferol | CYP3A4, | 1576, | CG2060, |
| 1-(1-cyclohexylethylamino)-4-phenylphthalazine | CYP3A4, | 1576, | CG2060, |
| 1-(2-methyl-4-methoxyphenyl)-4-((2-hydroxyethyl) amino)-6-trifluoromethoxy-2,3-dihydropyrrolo(3,2-c) quinoline | CYP3A4, | 1576, | CG2060, |
| 1-(2,3-dichlorobenzoyl)-5-methoxy-2-methyl-(2-(mopholin-4-yl)ethyl)-1H-indole | CNR2, | 1269, | CG12796, |
| 1-(2,3-dihydro-1,4-benzodioxin-5-yl)-4-((5-(4-fluorophenyl)-3-pyridinyl)methyl)piperazine | HDAC3, | 8841, | CG2128, |
| 1-(6-((3-methoxyestra-1,3,5(10)-trien-17-yl)amino) hexyl)-1H-pyrrole-2,5-dione | NFKB1,S1PR1,ADORA 1, | 4790,1901,134, | CG11992,CG12796,CG 9753, |
| 1-adamantyl propargyl ether | NFKB1, | 4790, | CG11992, |
| 1-aminobenzotriazole | CYP4F3,CYP4A11,CYP 3A5, | 4051,1579,1577, | CG2060,CG2060,CG20 60, |
| 1-aminooxy-3-aminopropane | SLC25A21, | 89874, | CG5254, |
| 1-hydrazino-4-(3,5-dimethyl)-1-pyrazolyl-5H-pyridazino (4,5-b)indole | PDE4A, | 5141, | CG14940, |
| 1-hydroxymethylmidazolam | CYP3A4, | 1576, | CG2060, |
| 1-hydroxypyrene | GSTT1, | 2952, | CG30005, |
| 1-Methyl-4-phenylpyridinium | NFKB1, | 4790, | CG11992, |

(continued)

| Small Molecule | Interacting Gene Symbol | Interacting GeneID | Drosophila Id |
|---|---|---|---|
| 1-Nitropyren-8-ol | CYP3A5,CYP4F3, | 1577,4051, | CG2060,CG2060, |
| 1-phosphatidyl-1D-myo-inositol 3-phosphates | KHDRBS1,NCF4, | 10657,4689, | CG3613,CG5821,CG71 29, |
| 1-stearoyl-2-oleoyl-sn-glycero-3-phosphocholine | NCF4, | 4689, | CG7129, |
| 1,1-bis(3'-indolyl)-1-(4-t-butylphenyl)methane | PSMD9, | 5715, | CG9588, |
| 1,1-dimethylbutyl-1-deoxy-Delta(9)-THC | CNR2, | 1269, | CG12796, |
| 1,1,1-trichloro-2-(4-hydroxyphenyl)-2-(4-methoxyphenyl) ethane | CYP3A4, | 1576, | CG2060, |
| 1,2-bis(diphenylphosphino)ethane | ME1, | 4199, | CG5889, |
| 1,2-di-(4-sulfamidophenyl)-4-butylpyrazolidine-3,5-dione | UBE2L3, | 7332, | CG5788, |
| 1,2-diacyl-sn-glycero-3-phosphocholines | DLD, | 1738, | CG7430, |
| 1,2-ethanedithiol | ME3, | 10873, | CG5889, |
| 1,2-oleoylphosphatidylcholine | TXNRD1,TXNRD2, | 7296,10587, | CG2151,CG2151, |
| 1,2,4-triazines | FGFR1, | 2260, | CG7223, |
| 1,25-dihydroxy-21-(3-hydroxy-3-methylbutyl)-23-yne-26,27-hexafluorocholecalciferol | SMAD6, | 4091, | CG5201, |
| 1,25-dihydroxyergocalciferol | TRPV6, | 55503, | CG5842, |
| 1,25D3 | SLC2A1,THOC4,NFKB 1,LARP6,CYP3A7,CYP 3A4,UGT1A4,UGT1A3 , UGT2B15,UGT2B17, TRPV5,TRPV6,GPR17 7,PPP2CA, CDC2,CDK 2,PSMD9, | 6513,10189,4790, 55323,1551,1576, 54657,54659,7366 , 7367,56302,5550 3,79971,5515,983, 1017,5715, | CG1086,CG1101,CG11 992,CG17386,CG2060 , CG2060,CG4772,CG4 772,CG4772,CG4772, CG5842,CG5842,CG62 10,CG7109,CG8203,C G8203,CG9588, |
| 1,3-Dcg | PRDX6, | 9588, | CG3083, |
| 1,3-dihydroxy-4,4,5,5-tetramethyl-2-(4-carboxyphenyl) tetrahydroimid azole | CCRN4L, | 25819, | CG31299, |
| 1,3-dipropyl-8-(3-noradamantyl)xanthine | ADORA1, | 134, | CG9753, |
| 1,3,5-trimethylbenzene | CELSR1,ME2, | 9620,4200, | CG11895,CG5889, |

(continued)

| Small Molecule | Interacting Gene Symbol | Interacting GeneID | Drosophila Id |
|---|---|---|---|
| 1,7-dioxa-2,6-diaza-4,4-dioxide-4,7a-dithia-3H,5H-benzo(cd)pentalene | LPAR3,LPAR2, | 23566,9170, | CG12796,CG12796, |
| 10-deoxymethynolide | NDUFAB1, | 4706, | CG9160, |
| 10-propargyl-10-deazaaminopterin | RFC1, | 5981, | CG1119, |
| 10-undecynoic acid | CYP4A11, | 1579, | CG2060, |
| 10,10-bis(4-pyridinylmethyl)-9(10H)-anthracenone | KCNQ5, | 56479, | CG12915, |
| 11-cis-retinal | SAG, | 6295, | CG5711, |
| 11-hydroxycannabinol | CNR2,CNR1, | 1269,1268, | CG12796,CG12796, |
| 12-Hht | LPAR2, | 9170, | CG12796, |
| 13-Lox | CD36, | 948, | CG7422, |
| 13-oxo-9,11-octadecadienoic acid | UGT2B7, | 7364, | CG4772, |
| 15 hete | TXNRD1,TXNRD2, | 7296,10587, | CG2151,CG2151, |
| 15-Hydroxy-11 alpha,9 alpha-(epoxymethano)prosta-5,13-dienoic Acid | PSMD9, | 5715, | CG9588, |
| 17-(allylamino)-17-demethoxygeldanamycin | NFKB1,CYP3A5,ECD, | 4790,1577,11319, | CG11992,CG2060,CG5 714, |
| 17alpha-ethynylestradiol | CYP3A4, | 1576, | CG2060, |
| 1843U89 | TYMS, | 7298, | CG3181, |
| 1D-myo-inositol 1,3,4,5-tetrakisphosphate | INPPSA, | 3632, | CG31110, |
| 1H-indole | GABARAP,CNR2,SFRS 1, | 11337,1269,6426, | CG12334,CG12796,CG 6987, |
| 1H-pyrazole | HNRNPA1, | 3178, | CG9983, |
| 1H-pyrazolo(3,4-b)pyridine | CDC2,CDK2, | 983,1017, | CG8203,CG8203, |
| 2 APB | TRPV1,TRPV6,TRPV2, TRPV3, | 7442,55503,51393 , 162514, | CG5842,CG5842,CG58 42,CG5842, |
| 2-(1-(3-dimethylaminopropyl)-5-methoxyindol-3-yl)-3-(1H-indol-3-yl)maleimide | NFKB1, | 4790, | CG11992, |
| 2-(1-methyl-4-piperidinyl)-6-(2-phenylpyrazolo(1,5-a) pyridin-3-yl)-3(2H)-pyridazinone | ADORA1, | 134, | CG9753, |

(continued)

| Small Molecule | Interacting Gene Symbol | Interacting GeneID | Drosophila Id |
|---|---|---|---|
| 2-(2-hydroxyethylsulfanyl)-3-methyl-1,4-naphthoquinone | CDC2, | 983, | CG8203, |
| 2-(3,4-dimethoxyphenyl)-5-amino-2-isopropylvaleronitrile | CYP3A5,CYP3A4, | 1577,1576, | CG2060,CG2060, |
| 2-(4-amino-3-methylphenyl)-5-fluorobenzothiazole | NFKB1, | 4790, | CG11992, |
| 2-(4-morpholinoanilino)-6-cyclohexylaminopurine | AURKA, | 6790, | CG6620, |
| 2-(4-morpholinyl)-8-phenyl-4H-1-benzopyran-4-one | NFKB1,S1PR1,CCNA2, LETMD1,CD36,FYN,PS MD9, | 4790,1901,890,25 875,948,2534,571 5, | CG11992,CG12796,CG 5940,CG5989,CG7422 , CG7873,CG9588, |
| 2-(4-toluidino)-6-naphthalenesulfonic acid | CYP3A4, | 1576, | CG2060, |
| 2-(cyclohexylmethylidenehydraz ino)adenosine | ADORA2A, | 135, | CG9753, |
| 2-AAF | CYP4F3,CYP3A5, | 4051,1577, | CG2060,CG2060, |
| 2-acetylthiomethyl-3-(4-methylbenzoyl)propionic acid | NFKB1, | 4790, | CG11992, |
| 2-AG | CNR2,CNR1, | 1269,1268, | CG12796,CG12796, |
| 2-amino-1-methyl-6-phenylimidazo(4,5-b)pyridine | UGT1A1,UGT1A7, | 54658,54577, | CG4772,CG4772, |
| 2-amino-3,4-dimethylimidazo(4,5-f)quinoline | CYP3A4,CYP3A5, | 1576,1577, | CG2060,CG2060, |
| 2-aminoethoxydiphenyl borate | TRPV1,TRPV3,TRPV6, TRPV2, | 7442,162514,5550 3,51393, | CG5842,CG5842,CG58 42,CG5842, |
| 2-AP | NFKB1,SERPINC1, | 4790,462, | CG11992,CG8137, |
| 2-CADO | NFKB1, | 4790, | CG11992, |
| 2-chloro-5-nitrobenzanilide | PSMD9, | 5715, | CG9588, |
| 2-cyano-3-hydroxy-N-(4-(trifluoromethyl)phenyl)-2-hepten-6-ynamide | NFKB1, | 4790, | CG11992, |
| 2-cyanomethylthiopyridine-4-carbonitrile | CYP3A4, | 1576, | CG2060, |
| 2-cyclopentyl-5-(5-isoquinolylsulfonyl)-6-nitro-1H-benzo (D)imidazole | CELSR1,NFKB1,GPR1 2,CNR2,TRPV1,ME2,M E3,PSMD9, | 9620,4790,2835,1 269,7442,4200,10 873,5715, | CG11895,CG11992,CG 12796,CG12796,CG58 42,CG5889,CG5889,C G9588, |
| 2-DG | SLC2A1,CCNB1,GLRX 2,GBF1, | 6513,891,51022,8 729, | CG1086,CG5940,CG68 52,CG8487, |

(continued)

| Small Molecule | Interacting Gene Symbol | Interacting GeneID | Drosophila Id |
|---|---|---|---|
| 2-hydroxy-4-(2,2,3,3,3-pentafluoropropoxy)benzoic acid | PSMD9, | 5715, | CG9588, |
| 2-hydroxyamino-1-methyl-6-phenylimidazo(4,5-b) pyridine | UGT1A1, | 54658, | CG4772, |
| 2-hydroxyamino-3-methylimidazolo(4,5-f)quinoline | PLXNB1, | 5364, | CG11081, |
| 2-ME | NFKB1,SMN1,LARP6,T YMS, CCNB1,PSMD9, | 4790,6606,55323, 7298,891,5715, | CG11992,CG16725,CG 17386,CG3181,CG594 0,CG9588, |
| 2-methoxyacetic acid [2-[2-[3-(1H-benzoimidazol-2-yl) propyl-methyl-amino]ethyl]-6-fluoro-1-isopropyl-tetralin-2-yl] ester | CYP3A4, | 1576, | CG2060, |
| 2-methyl-1-((4-methyl-5-isoquinolinyl)sulfonyl)homopip erazine | SMN1, | 6606, | CG16725, |
| 2-N-(4-(1-azitrifluoroethyl)benzoyl)-1,3-bis-(mannos-4-yloxy)-2-propylamine | SLC2A1, | 6513, | CG1086, |
| 2-Naftol | GSTT1, | 2952, | CG30005, |
| 2-oxothiazolidine-4-carboxylic acid | NFKB1, | 4790, | CG11992, |
| 2-phenyl-4-oxohydroquinoline | CDC2, | 983, | CG8203, |
| 2,2,2-trichloroethane-1,1-diol | SLC2A1, | 6513, | CG1086, |
| 2,2'-(hydroxynitrosohydrazono)bis-ethanamine | NFKB1, | 4790, | CG11992, |
| 2,2'-azobis(2,4-dimethylvaleronitrile) | CDK9, | 1025, | CG5179, |
| 2,2'-bipyridine | ME1, | 4199, | CG5889, |
| 2,2',4,4'-tetrachlorobiphenyl | NFKB1, | 4790, | CG11992, |
| 2,3-bis(3'-hydroxybenzyl)butane-1,4-diol | CCNA2, | 890, | CG5940, |
| 2,3-dihydroxyterephthalamide | CELSR1,ME3,ME2, | 9620,10873,4200, | CG11895,CG5889,CG5 889, |
| 2,3,4-tri-O-acetylarabinopyranosyl isothiocyanate | TRPA1, | 8989, | CG5751, |
| 2,4-diaminoquinazoline | SMN2, | 6607, | CG16725, |
| 2,4-thiazolidinedione | SLC2A4, | 6517, | CG1086, |

(continued)

| Small Molecule | Interacting Gene Symbol | Interacting GeneID | Drosophila Id |
|---|---|---|---|
| 21-hydroxy-9beta,10alpha-pregna-5,7-diene-3-ol-20-one | UGT2B7, | 7364, | CG4772, |
| 25-desacetylrifabutin | CYP3A4, | 1576, | CG2060, |
| 25-Hydroxycholesterol | CYP4F2, | 8529, | CG2060, |
| 25(OH)D3 | TRPV6, | 55503, | CG5842, |
| 3-((4-(4-chlorophenyl)piperazin-1-yl)methyl)-1H-pyrrolo (2,3-b)pyridine | HDAC3, | 8841, | CG2128, |
| 3-(2-hydroxy-4-(1,1-dimethylheptyl)phenyl)-4-(3-hydroxypropyl)cyclohexanol | CNR1,CNR2, | 1268,1269, | CG12796,CG12796, |
| 3-(2h)-pyridazinone | ADORA1, | 134, | CG9753, |
| 3-(cyclopentyloxy)-N-(3,5-dichloro-4-pyridyl)-4-methoxybenzamide | PDE4A, | 5141, | CG14940, |
| 3-aminopyrazole | CCNA2,CDK2, | 890,1017, | CG5940,CG8203, |
| 3-beta-(2-(diethylamino)ethoxy)androst-5-en-17-one | IGF2R, | 3482, | CG14255, |
| 3-BHA | UGT1A6, | 54578, | CG4772, |
| 3-hydroxybutanal | TFAP4, | 7023, | CG7664, |
| 3-hydroxyflunitrazepam | CYP3A4, | 1576, | CG2060, |
| 3-Hydroxyquinine | CYP3A4, | 1576, | CG2060, |
| 3-isobutyl-1-methyl-Xanthine | CADPS, | 8618, | CG18026, |
| 3-keto-desogestrel | CYP3A4, | 1576, | CG2060, |
| 3-methoxy-4-aminoazobenzene | CYP4B1, | 1580, | CG2060, |
| 3-Methoxymorphinan | CYP3A4, | 1576, | CG2060, |
| 3-Methoxyoestradiol | CYP3A4, | 1576, | CG2060, |
| 3-methylcholanthrene | PLXNB1,CYP3A7,CYP 3A4,CYP3A5,CYP4F3, UGT1A1,SERPINB3,PS MD9, | 5364,1551,1576,1 577,4051,54658,6 317,5715, | CG11081,CG2060,CG2 060,CG2060,CG2060, CG4772,CG8137,CG94 53,CG9460,CG9588, |

(continued)

| Small Molecule | Interacting Gene Symbol | Interacting GeneID | Drosophila Id |
|---|---|---|---|
| 3-MI | CYP3A4, | 1576, | CG2060, |
| 3,3',4,5'-tetrahydroxystilbene | NFKB1,FGFR4, | 4790,2264, | CG11992,CG7223, |
| 3,4-DCI | NFKB1, | 4790, | CG11992, |
| 3,4,5-trihydroxybenzamidoxime | NFKB1, | 4790, | CG11992, |
| 4-(3-3,4-p-menthadien-(1,8)-yl)olivetol | CNR1,CNR2, | 1268,1269, | CG12796,CG12796, |
| 4-(3-Butoxy-4-methoxybenzyl)-2-imidazolidinone | PDE4A, | 5141, | CG14940, |
| 4-(4-(4-chlorophenyl)-4-hydroxy-1-piperidinyl)-1-(4-fluorophenyl)-1-butanol | CYP3A4, | 1576, | CG2060, |
| 4-(4-(N-benzoylamino)anilino)-6-methoxy-7-(3-(1-morpholino)propoxy)quinazoli ne | AURKA, | 6790, | CG6620, |
| 4-(4-fluorophenyl)-2-(4-hydroxyphenyl)-5-(4-pyridyl) imidazole | POLD1,CDC2, | 5424,983, | CG5949,CG8203, |
| 4-(5-benzo(1,3)dioxol-5-yl-4-pyridin-2-yl-1H-imidazol-2-yl)benzamide | SMN1,SMAD7, | 6606,4092, | CG16725,CG5201, |
| 4-(benzodioxan-5-yl)-1-(indan-2-yl)piperazine | HDAC3, | 8841, | CG2128, |
| 4-(N-methyl-N-nitrosamino)-1-(3-pyridyl)-1-butanone | CYP4F3,CYP3A5, | 4051,1577, | CG2060,CG2060, |
| 4-AP | PLXNB2,FGFR3, | 23654,2261, | CG11081,CG7223, |
| 4-azidosalicylic acid | UGT1A3, UGT1A4, | 54659,54657, | CG4772,CG4772, |
| 4-dimethylamino-3',4'-dimethoxychalcone | LARP6, | 55323, | CG17386, |
| 4-hydroxy-N-desmethyltamoxifen | UGT1A4, UGT1A3, | 54657,54659, | CG4772,CG4772, |
| 4-hydroxyacetophenone | ECD, | 11319, | CG5714, |
| 4-hydroxycoumarin | UGT1A6, | 54578, | CG4772, |
| 4-hydroxyestradiol-17 beta | PLXNB2, | 23654, | CG11081, |
| 4-hydroxynon-2-enal | PDHX,TRPA1, | 8050,8989, | CG5261,CG5751, |
| 4-hydroxytriazolam | CYP3A4, | 1576, | CG2060, |

(continued)

| Small Molecule | Interacting Gene Symbol | Interacting GeneID | Drosophila Id |
|---|---|---|---|
| 4-methyl-N-(3-(4-methylimidazol-1-yl)-5-(trifluoromethyl) phenyl)-3-((4-pyridin-3-ylpyrimidin-2-yl)amino) benzamide | CYP3A4, | 1576, | CG2060, |
| 4-phenylbutyric acid | SMN2, | 6607, | CG16725, |
| 4-S-cysteaminylphenol | SERPINB6, | 5269, | CG8137,CG9453,CG94 60, |
| 4-sulfophenylmethallyl ether | ECD, | 11319, | CG5714, |
| 4,4'-DDE | CYP3A4, | 1576, | CG2060, |
| 4,4'-dipyridyl disulfide | CYP3A4, | 1576, | CG2060, |
| 4,8-dimethoxy-7-hydroxyfuro(2,3-b)quinoline | SMN1, | 6606, | CG16725, |
| 4'-epidoxorubicin | GPR177, | 79971, | CG6210, |
| 4'-N-benzoylstaurosporine | POLD1,CDK2,CDC2, | 5424,1017,983, | CG5949,CG8203,CG82 03, |
| 4(2'-aminoethyl)amino-1,8-dimethylimidazo(1,2-a) quinoxaline | CCNB1,AURKA,CDC2, | 891,6790,983, | CG5940,CG6620,CG82 03, |
| 4alpha-phorbol 12,13-didecanone | TRPV4, | 59341, | CG5842, |
| 4alphaPDD | TRPV4, | 59341, | CG5842, |
| 5-((1,2-dihydro-2-oxo-3H-indol-3-ylidene)methyl)-2,4-dimethyl-1H-pyrrole-3-propanoic acid | FGFR1, | 2260, | CG7223, |
| 5-(4'-(N-piperidinyl)phenylazo)indazole | CCNA2,CDK2, | 890,1017, | CG5940,CG8203, |
| 5-7-oxo-zeaenol | NFKB1, | 4790, | CG11992, |
| 5-AC | GALR1, | 2587, | CG2872, |
| 5-acetylneuraminyl-(2-3)-galactosyl-(1-4)-(fucopyranosyl-(1-3))-N-acetylglucosamine | SLC2A1, | 6513, | CG1086, |
| 5-azido-1H-indole-3-acetic acid | KDELR1, | 10945, | CG5183, |
| 5-HT | SERPINC1, | 462, | CG8137, |
| 5-methoxy-N,N-diisopropyltryptamine | CYP3A4, | 1576, | CG2060, |
| 5-Mop | CYP3A4, | 1576, | CG2060, |

(continued)

| Small Molecule | Interacting Gene Symbol | Interacting GeneID | Drosophila Id |
|---|---|---|---|
| 5,10-methylenetetrahydrofolate | TYMS, | 7298, | CG3181, |
| 5,6-dimethylxanthenoneacetic acid | NFKB1, | 4790, | CG11992, |
| 5'-O-(((2-decanoylamino-3-phenylpropyloxycarbonyl) amin o)sulfonyl)uridine | FYN, | 2534, | CG7873, |
| 6 beta-hydroxycortisol | CYP3A4, | 1576, | CG2060, |
| 6-Aminochrysene-1,2-dihydrodiol | CYP4F3,CYP3A5, | 4051,1577, | CG2060,CG2060, |
| 6-chloro-2-pyridylmethyl nitrate | COG6, | 57511, | CG1968, |
| 6-deoxy-6-bromoascorbic acid | SLC2A1, | 6513, | CG1086, |
| 6-hydroxydexamethasone | CYP3A4, | 1576, | CG2060, |
| 6-Mercaptopurine | TXNRD2, | 10587, | CG2151, |
| 6,6'-oxybis(2,2-dimethylhexanoic acid) | UGT2B7,UGT2B17,UG T1A3, | 7364,7367,54659, | CG4772,CG4772,CG47 72, |
| 64Gd | TXNRD2,TXNRD1,TRP A1,TRPV1,CD36, | 10587,7296,8989, 7442,948, | CG2151,CG2151,CG57 51,CG5842,CG7422, |
| 7-(1,1-dimethylethyl)-6-(2-ethyl-2H-1,2,4-triazol-3-ylmethoxy)-3-(2-fluorophenyl)-1,2,4-triazolo(4,3-b) pyridazine | CYP3A4, | 1576, | CG2060, |
| 7-benzylamino-6-chloro-2-piperazino-4-pyrrolidinopteridine | PDE4A, | 5141, | CG14940, |
| 7-benzyloxyquinoline | CYP3A4, | 1576, | CG2060, |
| 7-CDL | UGT8, | 7368, | CG4772, |
| 7-hydroxystaurosporine | NFKB1,TYMS,CCNA2, POLD1,CDC2,CDK2,P SMD9, | 4790,7298,890,54 24,983,1017,5715, | CG11992,CG3181,CG5 940,CG5949,CG8203, CG8203,CG9588, |
| 7-ketocholesterol | DYNLL1, | 8655, | CG6998, |
| 7,8-BF | CYP3A4,CYP3A5,CYP3 A7, | 1576,1577,1551, | CG2060,CG2060,CG20 60, |
| 7,8-dihydroneopterin | NFKB1, | 4790, | CG11992, |
| 7'-Isothiocyanato-11-hydroxy-1',1'-dimethylheptylhexahydrocann abinol | CNR1, | 1268, | CG12796, |

(continued)

| Small Molecule | Interacting Gene Symbol | Interacting GeneID | Drosophila Id |
|---|---|---|---|
| 7C3MT | NFKB1,CYP3A4,CCNB 1,PSMD9, | 4790,1576,891,57 15, | CG11992,CG2060,CG5 940,CG9588, |
| 7H-Pyrrolo(2,3-d)pyrimidine | TYMS, | 7298, | CG3181, |
| 8-((4-bromo-2,3-dioxobutyl)thio)-adenosine 3',5'-cyclic monophosphate | PDE4A, | 5141, | CG14940, |
| 8-(2,6-dichlorophenyl)-10-methyl-3-((4-morpholin-4-ylphenyl)amino)-2,4,10-triazabicyclo(4.4.0)deca-1,3,5,7-tetraen-9-one | LPAR2, | 9170, | CG12796, |
| 8-(3-chlorostyryl)caffeine | ADORA2A, | 135, | CG9753, |
| 8-anilinonaphthalene-1-sulfonic acid | SERPINC1, | 462, | CG8137, |
| 8-Hydroxy-2-(di-n-propylamino)tetralin | FGFR3, | 2261, | CG7223, |
| 8-Isoprostane | CADPS, | 8618, | CG18026, |
| 8,10-bis((2,2-dimethyl-1-oxopropyl)oxy)-11-methyl-1234-tetrahydro-6H-benzo(beta)quinolizin-6-one | CNR2, | 1269, | CG12796, |
| 9-(4'-aminophenyl)-9H-pyrido(3,4-b)indole | CYP3A4, | 1576, | CG2060, |
| 9-anthroic acid | VDAC1,OPN4, | 7416,94233, | CG17137,CG4550, |
| 9-CRA | UGT2B15,CD36, | 7366,948, | CG4772,CG7422, |
| 9-hydroxy-risperidone | CYP4F3,CYP3A5,CYP3 A4, | 4051,1577,1576, | CG2060,CG2060,CG20 60, |
| 9,10-anthraquinone | CYP4F3,CYP3A5, | 4051,1577, | CG2060,CG2060, |
| 9H-xanthene | CYP3A4,UGT1A6, | 1576,54578, | CG2060,CG4772, |
| A 71915 | NPR1, | 4881, | CG31183, |
| A-300-I | SLC2A4,NFKB1,S1PR 1,SMN2,FOXP3,HDAC 3,GALR1,PEG3,TYMS, UBE2L3,AURKB,CDC2 ,MLL,ATP1B2, | 6517,4790,1901,6 607,50943,8841,2 587,5178,7298,73 32,9212,983,4297, 482, | CG1086,CG11992,CG1 2796,CG16725,CG168 99,CG2128,CG2872,C G31364,CG6930,CG31 81,CG5788,CG6620,C G8203,CG8651,CG866 3, |
| a-ADP | SERPINC1, | 462, | CG8137, |
| A73025 | SERPINC1, | 462, | CG8137, |

(continued)

| Small Molecule | Interacting Gene Symbol | Interacting GeneID | Drosophila Id |
|---|---|---|---|
| Abbott | PSMD12, | 5718, | CG1100, |
| abciximab | SAG,SERPINC1, | 6295,462, | CG5711,CG8137, |
| Absele | SLC2A1, | 6513, | CG1086, |
| ABT-737 | NFKB1, | 4790, | CG11992, |
| acetamide 45 | PDE4A, | 5141, | CG14940, |
| Aceton | CD36, | 948, | CG7422, |
| acetonitrile | CYP3A4, | 1576, | CG2060, |
| acetyl-11-ketoboswellic acid | NFKB1, | 4790, | CG11992, |
| acetylcholine | PLXNA2,DLD, | 5362,1738, | CG11081,CG7430, |
| acetylvalerenolic acid | NFKB1, | 4790, | CG11992, |
| Aclarubicin | SMN2,SMN1, | 6607,6606, | CG16725,CG16725, |
| Acolen | CYP3A4, | 1576, | CG2060, |
| ACON | CYP3A5,CYP3A4,CYP3 A7,TXNRD1,DLL1,SA G, | 1577,1576,1551,7 296,28514,6295, | CG2060,CG2060,CG20 60,CG2151,CG3619,C G5711, |
| ACT D | SLC2A3,MRPL41,BRD 8,CYP3A4,TXNRD1,TX NRD2,KHDRBS1,SNR PG, | 6515,64975,10902 , 1576,7296,10587, 10657,6637, | CG1086,CG12954,CG1 4514,CG2060,CG2151 , CG2151,CG3613,CG5 821,CG9742, |
| actinium | VDAC1, | 7416, | CG17137, |
| Actosin | SLC2A4,NFKB1,LPAR1 ,PDE1B, PDE4A,SMN1, PSMD9, | 6517,4790,1902,5 153,5141,6606,57 15, | CG1086,CG11992,CG1 2796,CG14940,CG149 40,CG16725,CG9588, |
| adalimumab | PSMD12,CD36, | 5718,948, | CG1100,CG7422, |
| Adalin | ECD, | 11319, | CG5714, |
| Adanon | CYP3A4,CYP3A5,FGF R3, | 1576,1577,2261, | CG2060,CG2060,CG72 23, |
| Adfeed | PSIP1, | 11168, | CG7946, |
| adinazolam | CYP3A4, | 1576, | CG2060, |
| Adofeed | UGT2B7,CD36, | 7364,948, | CG4772,CG7422, |

(continued)

| Small Molecule | Interacting Gene Symbol | Interacting GeneID | Drosophila Id |
|---|---|---|---|
| Adrenor | SLC2A4,NFKB1,CNR1, LPAR2,TBXAS1,TYMS, ECD, FGFR4,FGFR3,FY N, | 6517,4790,1268,9 170,6916,7298,11 319,2264,2261,25 34, | CG1086,CG11992,CG1 2796,CG12796,CG206 0,CG3181,CG5714,CG 7223,CG7223,CG7873, |
| Adrin | KCNQ5,PDE4A,CYP4F 2,CYP4A11,UBE2L3,F GFR3, | 56479,5141,8529, 1579,7332,2261, | CG12915,CG14940,CG 2060,CG2060,CG5788 ,CG7223, |
| AEBSF | CTSB, | 1508, | CG10992, |
| Aeromax | DHX8,DHX34, | 1659,9704, | CG10689,CG1375,CG1 4198, |
| afloqualone | UGT1A3, UGT1A4, | 54659,54657, | CG4772,CG4772, |
| AGMATINE | CCNA2, | 890, | CG5940, |
| AIDSVAX | SFRS1, | 6426, | CG6987, |
| ajoene | CCNB1, | 891, | CG5940, |
| ajulemic acid | CNR1, | 1268, | CG12796, |
| alachlor | CYP3A4,CYP3A7, | 1576,1551, | CG2060,CG2060, |
| Aladerm | CDK2, | 1017, | CG8203, |
| alaninate | FGFR1, | 2260, | CG7223, |
| Alat | NFKB1,CYP3A4,CYP3 A5,FGFR3, | 4790,1576,1577,2 261, | CG11992,CG2060,CG2 060,CG7223, |
| Alcolo | TXNRD2, | 10587, | CG2151, |
| Alcuronium | FGFR3, | 2261, | CG7223, |
| Aldara | NFKB1, | 4790, | CG11992, |
| ALDO | CYP3A4,HDAC3,TMBI M4,GAD1, | 1576,8841,51643, 2571, | CG2060,CG2128,CG69 01,CG7811, |
| Aldrich | ACTR2, | 10097, | CG9901, |
| alemtuzumab | CADPS, | 8618, | CG18026, |
| Alfarol | CYP3A4, | 1576, | CG2060, |

(continued)

| Small Molecule | Interacting Gene Symbol | Interacting GeneID | Drosophila Id |
|---|---|---|---|
| Alfentanil | ANKHD1,CYP3A4,CYP 3A5,FGFR3, | 54882,1576,1577, 2261, | CG18671,CG2060,CG2 060,CG7223, |
| ALIMTA | TBXAS1,TYMS, | 6916,7298, | CG2060,CG3181, |
| aliskiren | CYP3A4, | 1576, | CG2060, |
| Alli | CNR1, | 1268, | CG12796, |
| ALLN | NFKB1,CD36, | 4790,948, | CG11992,CG7422, |
| alloxazine | ADORA2B, | 136, | CG9753, |
| allyl isothiocyanate | TRPA1, | 8989, | CG5751, |
| almokalant | UGT2B7, | 7364, | CG4772, |
| aloesin | CDK2, | 1017, | CG8203, |
| Alprenolol | GSTO1,DNALI1, | 9446,7802, | CG6673,CG6971, |
| Alvesco | CYP3A4, | 1576, | CG2060, |
| AM 1387 | CNR1, | 1268, | CG12796, |
| AM 251 | CNR1, | 1268, | CG12796, |
| Am 80 | NFKB1, | 4790, | CG11992, |
| AMD 070 | CYP3A4, | 1576, | CG2060, |
| Amiloride | ACCN1,SLC25A21,CC NB1,SERPINB3, | 40,89874,891,631 7, | CG1058,CG5254,CG59 40,CG8137,CG9453,C G9460, |
| Aminacrine | NFKB1, | 4790, | CG11992, |
| Amine BB | SERPINB1, | 1992, | CG8137,CG9453,CG94 60, |
| amino-polyethyleneoxide-sulfonate | SERPINC1, | 462, | CG8137, |
| aminoflavone | CYP4F3,CYP3A5, | 4051,1577, | CG2060,CG2060, |
| Amiodarone | CYP3A4,FGFR3, | 1576,2261, | CG2060,CG7223, |
| Amlodipine | CYP3A4, | 1576, | CG2060, |
| Amphotericin B | UGT8, | 7368, | CG4772, |
| amprenavir | CYP3A7,CYP3A4, | 1551,1576, | CG2060,CG2060, |

(continued)

| Small Molecule | Interacting Gene Symbol | Interacting GeneID | Drosophila Id |
|---|---|---|---|
| Amrinone | SMARCA1, | 6594, | CG8625, |
| amsonic acid | CDC2, | 983, | CG8203, |
| Amygdalin | LPAR2, | 9170, | CG12796, |
| AN 207 | LARP6, | 55323, | CG17386, |
| Anaboleen | SLC2A1, NFKB1, UGT2 B15,UGT1A4,UGT1A3 , UGT2B17,UGT1A9,U BE2L3,CCNA2, | 6513,4790,7366,5 4657,54659,7367, 54600,7332,890, | CG1086,CG11992,CG4 772,CG4772,CG4772, CG4772,CG4772,CG57 88,CG5940, |
| anacardic acid | AURKA, | 6790, | CG6620, |
| Anandamide | NFKB1,CNR1,CNR2,T RPV1,CD36,CDK2, | 4790,1268,1269,7 442,948,1017, | CG11992,CG12796,CG 12796,CG5842,CG742 2,CG8203, |
| Anco | NFKB1,PSIP1, | 4790,11168, | CG11992,CG7946, |
| Andrographis | TRPV4, | 59341, | CG5842, |
| Androtine | UGT2B17, | 7367, | CG4772, |
| Aneol | CCNB1,POLD1,CDC2, | 891,5424,983, | CG5940,CG5949,CG82 03, |
| Ang II | LPAR2,TXNRD3,SERPI NC1, | 9170,114112,462, | CG12796,CG2151,CG8 137, |
| Anisomycin | PSMD12,NFKB1,TXNR | 5718,4790,10587, | CG1100,CG11992,CG2 |
| | D2, | | 151, |
| Anon | TRPV1, | 7442, | CG5842, |
| Anthocyanins | CYP3A4, | 1576, | CG2060, |
| anthra(1,9-cd)pyrazol-6(2H)-one | SLC2A4,NFKB1,SMN1 , CYP3A4,CCNB1,CDC 2,PSMD9, | 6517,4790,6606,1 576,891,983,5715, | CG1086,CG11992,CG1 6725,CG2060,CG5940 , CG8203,CG9588, |
| anthracene | CD226,CYP3A5,CYP4 F3,FYN,PSIP1, | 10666,1577,4051, 2534,11168, | CG13162,CG2060,CG2 060,CG7873,CG7946, |
| anthralin | NFKB1,CYP3A5,CYP3 A7, | 4790,1577,1551, | CG11992,CG2060,CG2 060, |
| Anthricin | CYP3A4, | 1576, | CG2060, |
| anthrone | KCNQ5, | 56479, | CG12915, |

(continued)

| Small Molecule | Interacting Gene Symbol | Interacting GeneID | Drosophila Id |
|---|---|---|---|
| antibiotic G 418 | CCNA2, | 890, | CG5940, |
| antibiotic H107 | PSMD12, | 5718, | CG1100, |
| Antimycin A | TXNRD2,CCNA2,POL D1,CDC2,PSMD9, | 10587,890,5424,9 83,5715, | CG2151,CG5940,CG59 49,CG8203,CG9588, |
| Anyvim | CYP3A4, | 1576, | CG2060, |
| APAP | CYP3A4,UGT1A4,UGT 1A3,UGT1A1, | 1576,54657,54659 , 54658, | CG2060,CG4772,CG47 72,CG4772, |
| APDC | THOC4,NFKB1,LARP6, SMAD7, | 10189,4790,55323 , 4092, | CG1101,CG11992,CG1 7386,CG5201, |
| Aphidicolin | NFKB1, TYMS, | 4790,7298, | CG11992,CG3181, |
| Aphloiol | NFKB1, | 4790, | CG11992, |
| apicidin | NFKB1,CDK2, | 4790,1017, | CG11992,CG8203, |
| Apigenin | ACAT1,NFKB1,UGT1A 4, UGT1A1, UGT2 B7,U GT1A3,UGT1A6,CCNB 1,CCNA2,POLD1,EXO SC1,GADD45GIP1,CD C2,CDK2, | 38,4790,54657,54 658,7364,54659,5 4578,891,890,542 4,51013,90480,98 3,1017, | CG10932,CG11992,CG 4772,CG4772,CG4772 , CG4772,CG4772,CG5 940,CG5940,CG5949, CG6249,CG7172,CG82 03,CG8203, |
| apocynin | NFKB1, | 4790, | CG11992, |
| Apotransferrin | PSMD9, | 5715, | CG9588, |
| aprepitant | CYP3A4, | 1576, | CG2060, |
| APRL | SLC2A4,NFKB1,LPAR2 ,NCF4,FYN, SERPINB3, | 6517,4790,9170,4 689,2534,6317, | CG1086,CG11992,CG1 2796,CG7129,CG7873 , CG8137,CG9453,CG9 460, |
| AQ4N | CYP3A5,CYP4F3, | 1577,4051, | CG2060,CG2060, |
| arabinogalactan | DHX9, | 1660, | CG9323, |
| Arac | NFKB1, | 4790, | CG11992, |
| Aralen | NFKB1,C9orf5,DHPS, | 4790,23731,1725, | CG11992,CG2698,CG8 005, |
| Arasine | SLC2A4, | 6517, | CG1086, |

(continued)

| Small Molecule | Interacting Gene Symbol | Interacting GeneID | Drosophila Id |
|---|---|---|---|
| Areca | NFKB1, | 4790, | CG11992, |
| Arecoline | CCNB1,POLD1,CDC2, | 891,5424,983, | CG5940,CG5949,CG82 03, |
| Areether | CYP3A4, | 1576, | CG2060, |
| argatroban | CYP3A4,SERPINC1, | 1576,462, | CG2060,CG8137, |
| aripiprazole | CYP3A4, | 1576, | CG2060, |
| Aron | TXNRD1,TXNRD2, | 7296,10587, | CG2151,CG2151, |
| Artein | NFKB1,SMN1,CYP4F2, CYP3A5,CYP3A4,CD3 6,CDK2,PSMD9, | 4790,6606,8529,1 577,1576,948,101 7,5715, | CG11992,CG16725,CG 2060,CG2060,CG2060 , CG7422,CG8203,CG9 588, |
| Artra | NFKB1,GSTT1,CD36, | 4790,2952,948, | CG11992,CG30005,CG 7422, |
| arvanil | TRPV1,CD36, | 7442,948, | CG5842,CG7422, |
| asiatic acid | POLD1,CDC2, | 5424,983, | CG5949,CG8203, |
| asiaticoside | SMAD7, | 4092, | CG5201, |
| Asmax | PLXNB1,NFKB1,PDE4 A, SMN1,CYP4F3,CYP3 A5,SERPINB6,ADORA 2A, | 5364,4790,5141,6 606,4051,1577,52 69,135, | CG11081,CG11992,CG 14940,CG16725,CG20 60,CG2060,CG8137,C G9453,CG9460,CG975 3, |
| Asmol | NFKB1,LPAR2,PDE4A, SMN1, | 4790,9170,5141,6 606, | CG11992,CG12796,CG 14940,CG16725, |
| ASTA | RFC1,FOXP3,CYP3A5, CYP4F3,SERPINB6,PA FAH1B1, | 5981,50943,1577, 4051,5269,5048, | CG1119,CG16899,CG2 060,CG2060,CG8137, CG9453,CG9460,CG84 40, |
| astatine | LGI1,SERPINC1, | 9211,462, | CG12927,CG8137, |
| Astemizole | CYP3A4, | 1576, | CG2060, |
| Astragaloside A | NFKB1, | 4790, | CG11992, |
| atazanavir | CYP3A4, UGT1A4,UGT 1A1,UGT1A3, | 1576,54657,54658 , 54659, | CG2060,CG4772,CG47 72,CG4772, |
| ATL 146e | ADORA2A, | 135, | CG9753, |

(continued)

| Small Molecule | Interacting Gene Symbol | Interacting GeneID | Drosophila Id |
|---|---|---|---|
| Atorel | LPAR2, | 9170, | CG12796, |
| atorvastatin | SLC2A4,NFKB1,CYP3 A5,CYP3A4,CD36,SCA RB2,SERPINC1, | 6517,4790,1577,1 576,948,950,462, | CG1086,CG11992,CG2 060,CG2060,CG7422, CG7422,CG8137, |
| Atovaquone | CYP3A4, | 1576, | CG2060, |
| ATRA | PSMD12,THOC4,NFKB 1,IGF2R, SMN1,FOXP3 ,GFRA1,GFRA2,TRAM 1,CYP4B1,CYP4A11,T BXAS1,CYP3A7,TXNR D2,FZR1,MED9,KHDR BS1,C1orf107,UGT2 B 15,KDELR2,PDHX,UB E2L3,CCNA1,CCNB1,P OLD1,CD36,FGR,PSIP 1,SERPINC1,CDK2,CD C2,CDK5,PAFAH1B1, MLL,PSMD9, | 5718,10189,4790, 3482,6606,50943, 2674,2675,23471, 1580,1579,6916,1 551,10587,51343, 55090,10657,2704 2,7366,11014,805 0,7332,8900,891,5 424,948,2268,111 68,462,1017,983,1 020,5048,4297,57 15, | CG1100,CG1101,CG11 992,CG14255,CG1672 5,CG16899,CG17203, CG17203,CG18833,CG 2060,CG2060,CG2060 , CG2060,CG2151,CG3 000,CG30113,CG3613 , CG5821,CG3735,CG4 772,CG5183,CG5261, CG5788,CG5940,CG59 40,CG5949,CG7422,C G7873,CG7946,CG813 7,CG8203,CG8203,CG 8203,CG8440,CG8651 ,CG9588, |
| Atropine | FGFR3, | 2261, | CG7223, |
| Auranofin | NFKB1, | 4790, | CG11992, |
| AuTM | PARD6A, | 50855, | CG5884, |
| auxin | NFKB1, | 4790, | CG11992, |
| avasimibe | CYP3A4, | 1576, | CG2060, |
| AVE 0118 | FGFR3, | 2261, | CG7223, |
| avicularin | UGT1A9, | 54600, | CG4772, |
| Avid | CNR2, | 1269, | CG12796, |
| Axert | CYP3A4, | 1576, | CG2060, |
| Axsain | CNR1,TRPV1,DLD,PSI P1, | 1268,7442,1738,1 1168, | CG12796,CG5842,CG7 430,CG7946, |

(continued)

| Small Molecule | Interacting Gene Symbol | Interacting GeneID | Drosophila Id |
|---|---|---|---|
| Aza-dC | S1PR1, | 1901, | CG12796, |
| Aza-deoxycytidine | PEG3, | 5178, | CG31364,CG6930, |
| azacyclonol | CYP3A4, | 1576, | CG2060, |
| Azadc | LARP6,FZR1,PEG3,DY NLL1,FYN, PAFAH1B1, ATP1B2, | 55323,51343,5178 , 8655,2534,5048,4 82, | CG17386,CG3000,CG3 1364,CG6930,CG6998 , CG7873,CG8440,CG8663, |
| azamulin | CYP3A4, | 1576, | CG2060, |
| azaspirane | NFKB1, | 4790, | CG11992, |
| azelaic acid | TXNRD1, | 7296, | CG2151, |
| azelastine | NFKB1,CYP3A4, | 4790,1576, | CG11992,CG2060, |
| azelnidipine | CYP3A4,CD36, | 1576,948, | CG2060,CG7422, |
| azido ruthenium | VDAC1, | 7416, | CG17137, |
| Azine | TXNRD1, | 7296, | CG2151, |
| Azithromycin | NFKB1, | 4790, | CG11992, |
| Azobisisobutyramidinium dichloride | CDK9, | 1025, | CG5179, |
| Azole | CYP3A4, | 1576, | CG2060, |
| Azoles | CYP3A4, | 1576, | CG2060, |
| Azolidine | NFKB1, | 4790, | CG11992, |
| Azophen | CYP3A7,CYP3A5, | 1551,1577, | CG2060,CG2060, |
| Azor | PLXNA2,CYP3A43,CY P3A4,CYP3A5, | 5362,64816,1576, 1577, | CG11081,CG2060,CG2 060,CG2060, |
| BA (VAN) | CYP3A4,CYP3A7, | 1576,1551, | CG2060,CG2060, |
| Ba 0108E | SERPINC1, | 462, | CG8137, |
| bacitracin | SERPINB1, | 1992, | CG8137,CG9453,CG94 60, |
| Baclofen | FGFR3, | 2261, | CG7223, |
| bacterial lysate | NAP1L4, | 4676, | CG5330, |

(continued)

| Small Molecule | Interacting Gene Symbol | Interacting GeneID | Drosophila Id |
|---|---|---|---|
| bafilomycin A1 | SFRS1, | 6426, | CG6987, |
| Bagren | CYP3A4, | 1576, | CG2060, |
| baicalein | CCNB1,POLD1,CDC2, | 891,5424,983, | CG5940,CG5949,CG82 03, |
| Barbiturate | DYNC1H1,SLC2A1, | 1778,6513, | CG17150,CG1086, |
| Barnidipine | CYP3A4, | 1576, | CG2060, |
| BAY 11-7085 | NFKB1, | 4790, | CG11992, |
| BB-K8 | SMN1, | 6606, | CG16725, |
| BCNU | GSR,UBE2L3,GLRX, | 2936,7332,2745, | CG2151,CG5788,CG68 52, |
| Beflavin | CYP4F3,CYP3A5,GSR, HADHB, | 4051,1577,2936,3 032, | CG2060,CG2060,CG21 51,CG4581, |
| Belt | DDX18, | 8886, | CG6375, |
| benazepril | RBM6, | 10180, | CG4887, |
| bendamustine | NFKB1, | 4790, | CG11992, |
| Benidipine | CYP3A4, | 1576, | CG2060, |
| benzamidine | SERPINC1, | 462, | CG8137, |
| benzimidazolide | UBE2L3,DYNLL2,DYN LL1, | 7332,140735,8655 | CG5788,CG6998,CG69 98, |
| Benzodiazepines | KCNQ2,PDE4A,CYP3A 4,FGFR3, | 3785,5141,1576,2 261, | CG12915,CG14940,CG 2060,CG7223, |
| Benzodioxoles | CNR1, | 1268, | CG12796, |
| Benzphetamine | CYP4F11,CYP3A4, | 57834,1576, | CG2060,CG2060, |
| benzydamine N-oxide | FMO3, | 2328, | CG3006, |
| benzylamine | SLC2A4, | 6517, | CG1086, |
| benzyloxycarbonylleucyl-leucyl-leucine aldehyde | NFKB1,S1PR1,SMN1, CYP3A4,KHDRBS1,SM AD6,CCNB1,POLD1,A URKA, CDC2,CDK2,PS MD9, | 4790,1901,6606,1 576,10657,4091,8 91,5424,6790,983, 1017,5715, | CG11992,CG12796,CG 16725,CG2060,CG361 3,CG5821,CG5201,CG 5940,CG5949,CG6620 , CG8203,CG8203,CG9 588, |

(continued)

| Small Molecule | Interacting Gene Symbol | Interacting GeneID | Drosophila Id |
|---|---|---|---|
| benzyloxycarbonylvalyl-alanyl-aspartyl fluoromethyl ketone | TYMS, | 7298, | CG3181, |
| beractant | NFKB1, | 4790, | CG11992, |
| berberine | CYP3A4,CCNB1, | 1576,891, | CG2060,CG5940, |
| bergamottin | CYP3A4, | 1576, | CG2060, |
| bergaptol | CYP3A4, | 1576, | CG2060, |
| beta-glycerophosphoric acid | TXNRD2,TXNRD1, | 10587,7296, | CG2151,CG2151, |
| beta-lapachone | NFKB1, | 4790, | CG11992, |
| beta-Naphthoflavone | CYP4F3,CYP3A4,CYP3 A5,UGT1A4,UGT1A1, UGT1A3,UGT1A9,UGT 1A6, | 4051,1576,1577,5 4657,54658,54659 , 54600,54578, | CG2060,CG2060,CG20 60,CG4772,CG4772,C G4772,CG4772,CG477 2, |
| beta-propiolactone | SFRS1, | 6426, | CG6987, |
| Bethanechol | FGFR3, | 2261, | CG7223, |
| betulinic acid | NFKB1, | 4790, | CG11992, |
| bexarotene | PSMD12,PSIP1, | 5718,11168, | CG1100,CG7946, |
| Bezafibrate | NFKB1,LPAR3,CYP3A 4, | 4790,23566,1576, | CG11992,CG12796,CG 2060, |
| BG 9928 | ADORA1, | 134, | CG9753, |
| BGC945 | TYMS, | 7298, | CG3181, |
| biapigenin | CYP3A4, | 1576, | CG2060, |
| BIBX 1382BS | CHM, | 1121, | CG8432, |
| biphenyl-4-ol | UGT1A4,UGT1A3, | 54657,54659, | CG4772,CG4772, |
| BIRB 796 | DLG1, | 1739, | CG1725, |
| bisindolylmaleimide I | NFKB1,CYP3A4,GPR1 77, | 4790,1576,79971, | CG11992,CG2060,CG6 210, |
| bisindolylmaleimide III | CDK2, | 1017, | CG8203, |
| Bisoprolol | CYP3A4, | 1576, | CG2060, |
| bisperoxovanadium | NFKB1, | 4790, | CG11992, |

(continued)

| Small Molecule | Interacting Gene Symbol | Interacting GeneID | Drosophila Id |
|---|---|---|---|
| Bisphenol A-Glycidyl Methacrylate | CLTB, | 1212, | CG6948, |
| bizelesin | SERPINB3, | 6317, | CG8137,CG9453,CG94 60, |
| BL1521 | PSMD9, | 5715, | CG9588, |
| Bla-S | NFKB1, | 4790, | CG11992, |
| Blow | CCNA2,FRK, | 890,2444, | CG5940,CG7873, |
| BM 41.440 | CCNB1,POLD1,CDC2, | 891,5424,983, | CG5940,CG5949,CG82 03, |
| BML 241 | S1PR3, | 1903, | CG12796, |
| BMS 310705 | CYP3A4, | 1576, | CG2060, |
| BMS204352 | KCNQ4,KCNQ5,UGT2 B7, | 9132,56479,7364, | CG12915,CG12915,CG 4772, |
| BMS453 | CDK2, | 1017, | CG8203, |
| Bo-Xan | CHM, | 1121, | CG8432, |
| Boltin | SERPINC1, | 462, | CG8137, |
| Bonopen | ME3, | 10873, | CG5889, |
| boron | TXNRD1, | 7296, | CG2151, |
| Borrelia-burgdorferi | CNR2, | 1269, | CG12796, |
| bortezomib | NFKB1,CYP4F3,CYP3A 5,CYP3A4,KHDRBS1,P SIP1,PSMD9, | 4790,4051,1577,1 576,10657,11168, 5715, | CG11992,CG2060,CG2 060,CG2060,CG3613, CG5821,CG7946,CG95 88, |
| bosentan | CYP3A4, | 1576, | CG2060, |
| bosutinib | CDK2,PSMD9, | 1017,5715, | CG8203,CG9588, |
| botrocetin | ACTR2, | 10097, | CG9901, |
| BPDE | NFKB1,GSTT1, | 4790,2952, | CG11992,CG30005, |
| BR-II | TBXAS1,TYMS, | 6916,7298, | CG2060,CG3181, |
| Brake | LPAR3,CD36, | 23566,948, | CG12796,CG7422, |
| bredinin | CCNA2, | 890, | CG5940, |

(continued)

| Small Molecule | Interacting Gene Symbol | Interacting GeneID | Drosophila Id |
|---|---|---|---|
| Brefeldin A | SLC2A4,GABARAPL2,I GF2R, LARP6,KDELR2, CCNB1,CTBP1,GBF1, | 6517,11345,3482, 55323,11014,891, 1487,8729, | CG1086,CG12334,CG1 4255,CG17386,CG518 3,CG5940,CG7583,CG 8487, |
| Bromazepam | CYP3A4, | 1576, | CG2060, |
| bromo-cis-stilbene | CCNB1, | 891, | CG5940, |
| brucine | FGFR3, | 2261, | CG7223, |
| bryostatin 1 | CDK2,CDC2,PSMD9, | 1017,983,5715, | CG8203,CG8203,CG95 88, |
| Budesonide | CYP3A4,CYP3A5, | 1576,1577, | CG2060,CG2060, |
| bufalin | CCNA2, | 890, | CG5940, |
| bufuralol | CYP3A4, | 1576, | CG2060, |
| Bumetanide | FGFR3, | 2261, | CG7223, |
| BuOH | ME1,FGFR3,FGR, | 4199,2261,2268, | CG5889,CG7223,CG78 73, |
| Bupivacaine | KCNQ2, | 3785, | CG12915, |
| Buprenorphine | CYP3A4, | 1576, | CG2060, |
| BUPROPION | CYP3A4,CCKAR,GAD1 , | 1576,886,2571, | CG2060,CG6881,CG78 11, |
| Buspirone | CYP3A4, | 1576, | CG2060, |
| Busulfan | GSTT1, | 2952, | CG30005, |
| Buthionine Sulfoximine | LARP6,GSR,SERPINB6 , | 55323,2936,5269, | CG17386,CG2151,CG8 137,CG9453,CG9460, |
| Butyrate | NFKB1,HDAC3,GSTT2 , CCNA2,CCNB1,CDC2 ,CDK2, | 4790,8841,2953,8 90,891,983,1017, | CG11992,CG2128,CG3 0005,CG5940,CG5940 , CG8203,CG8203, |
| butyrolactone I | UBE2L3,CCNB1,POLD 1,CDC2,CDK2,CDK5, | 7332,891,5424,98 3,1017,1020, | CG5788,CG5940,CG59 49,CG8203,CG8203,C G8203, |
| C 1027 | CCNB1,POLD1,CDC2, | 891,5424,983, | CG5940,CG5949,CG82 03, |
| C 76 | RPL11, | 6135, | CG7726, |

(continued)

| Small Molecule | Interacting Gene Symbol | Interacting GeneID | Drosophila Id |
|---|---|---|---|
| CACP | NFKB1,LPAR1,MRPL4 1,VDAC1,DNAH5,LAR P6,TXNRD3,NPR2,ZS CAN1,TYMS, CCNA2,G PR177,AURKA,CASK,S ERPINB6,SERPINB3,D DOST,PSMD9, | 4790,1902,64975, 7416,1767,55323, 114112,4882,2843 12,7298,890,7997 1,6790,8573,5269, 6317,1650,5715, | CG11992,CG12796,CG 12954,CG17137,CG17 150,CG17386,CG2151 , CG31183,CG31364,C G6930,CG3181,CG594 0,CG6210,CG6620,CG 6703,CG8137,CG9453 , CG9460,CG8137,CG9 453,CG9460,CG9022, CG9588, |
| Calcijex | PSMD12,TRPV6, | 5718,55503, | CG1100,CG5842, |
| Calcimycin | PSMD12,NFKB1,DYNL L1,FGR, | 5718,4790,8655,2 268, | CG1100,CG11992,CG6 998,CG7873, |
| calphostin C | NFKB1,UGT1A1,UGT1 A4,UGT1A3,FGFR4, | 4790,54658,54657 , 54659,2264, | CG11992,CG4772,CG4 772,CG4772,CG7223, |
| Calyculin | SLC2A1,SLC2A4,NFK B1,TXNRD2,TXNRD1, | 6513,6517,4790,1 0587,7296, | CG1086,CG1086,CG11 992,CG2151,CG2151, |
| Camptothecin | PHB,NFKB1,S1PR1,BR D8,CCNA2,CCNB1, | 5245,4790,1901,1 0902,890,891, | CG10691,CG11992,CG 12796,CG14514,CG59 40,CG5940, |
| Canef | CYP3A4,CYP3A5, | 1576,1577, | CG2060,CG2060, |
| cangrelor | PRDX6,PSMD9, | 9588,5715, | CG3083,CG9588, |
| Cannabinoids | CNR1,CNR2,TRPA1,A NK1, | 1268,1269,8989,2 86, | CG12796,CG12796,CG 5751,CG7462, |
| Cannabis | CNR1,CNR2,CYP3A4, GSR, | 1268,1269,1576,2 936, | CG12796,CG12796,CG 2060,CG2151, |
| Cantharidin | TYMS, | 7298, | CG3181, |
| CAPE | NFKB1,SMN1, | 4790,6606, | CG11992,CG16725, |
| Capsaicin | TRPV1, | 7442, | CG5842, |
| capsaicinoids | TRPV1, | 7442, | CG5842, |
| capsazepine | TRPV1, | 7442, | CG5842, |

(continued)

| Small Molecule | Interacting Gene Symbol | Interacting GeneID | Drosophila Id |
|---|---|---|---|
| Carbachol | NFKB1,LPAR2,LPAR3, TAOK2,PDE1A,FGFR3, | 4790,9170,23566, 9344,5136,2261, | CG11992,CG12796,CG 12796,CG14217,CG14 940,CG7223, |
| Carbamazepine | CYP4F3,CYP3A4,CYP3 A5,HDAC3, | 4051,1576,1577,8 841, | CG2060,CG2060,CG20 60,CG2128, |
| carbapenem | VDAC1, | 7416, | CG17137, |
| Carbapenems | VDAC1,UGT1A3,UGT1 A4, | 7416,54659,54657 , | CG17137,CG4772,CG4 772, |
| carbobenzoxy-leucyl-leucyl-norvalinal | AURKA, | 6790, | CG6620, |
| Carbolines | CDK5,CDK2, | 1020,1017, | CG8203,CG8203, |
| Carboxyethyl-phenethylamino-ethylcarboxamidoadenosine | ADORA2A, | 135, | CG9753, |
| Cardiolipins | GLRX2, | 51022, | CG6852, |
| carebastine | CYP3A4, | 1576, | CG2060, |
| CARNOSOL | CCNB1, | 891, | CG5940, |
| carrageenans | SERPINC1, | 462, | CG8137, |
| carvacrol | TRPA1,TRPV3, | 8989,162514, | CG5751,CG5842, |
| carvedilol | UGT2B7,UGT2B4,UGT 1A1, | 7364,7363,54658, | CG4772,CG4772,CG47 72, |
| Casodex | NFKB1,CYP4F3,CYP3A 5, | 4790,4051,1577, | CG11992,CG2060,CG2 060, |
| caspofungin | CYP3A4, | 1576, | CG2060, |
| casticin | CCNA2,CDC2, | 890,983, | CG5940,CG8203, |
| catechins | UGT1A1, | 54658, | CG4772, |
| CB 3717 | TYMS, | 7298, | CG3181, |
| Cbdca | AURKB,SERPINB3, | 9212,6317, | CG6620,CG8137,CG94 53,CG9460, |
| CCPA | ADORA1, | 134, | CG9753, |
| CD 437 | TXNRD2, | 10587, | CG2151, |
| CDP 840 | PDE4A, | 5141, | CG14940, |
| Cefoxitin | VDAC1,DPAGT1, | 7416,1798, | CG17137,CG5287, |

(continued)

| Small Molecule | Interacting Gene Symbol | Interacting GeneID | Drosophila Id |
|---|---|---|---|
| celecoxib | NFKB1,UBE2L3,CCNB 1,CDK2,PSMD9, | 4790,7332,891,10 17,5715, | CG11992,CG5788,CG5 940,CG8203,CG9588, |
| cephalomannine | CYP3A4, | 1576, | CG2060, |
| cephalosporins | CAPZA1,VDAC1, | 829,7416, | CG10540,CG17137, |
| cepharanthine | NFKB1, | 4790, | CG11992, |
| cerebrolysin | SLC2A1, | 6513, | CG1086, |
| cerivastatin | NFKB1,CYP3A5,CYP3 A4,CDK2, | 4790,1577,1576,1 017, | CG11992,CG2060,CG2 060,CG8203, |
| Cetomacrogol | UGT1A4,UGT1A3, | 54657,54659, | CG4772,CG4772, |
| cetrorelix | PSIP1, | 11168, | CG7946, |
| cetuximab | CHM, | 1121, | CG8432, |
| CGP 12177 | DYNLL1, | 8655, | CG6998, |
| CGS 15943A | ADORA2A, | 135, | CG9753, |
| CGS 21680 | TRPV1,ADORA2A, | 7442,135, | CG5842,CG9753, |
| CH-THF | TYMS, | 7298, | CG3181, |
| CH2CHO | DPAGT1,TRPA1,FGFR 3, | 1798,8989,2261, | CG5287,CG5751,CG72 23, |
| Chalcone | CCNA2,CCNB1,POLD1 , CDC2,PSMD9, | 890,891,5424,983, 5715, | CG5940,CG5940,CG59 49,CG8203,CG9588, |
| CHAPS | NFKB1, | 4790, | CG11992, |
| Chinine | CYP4F3,CYP3A4,CYP3 A5,CYP3A7,UGT2B7, | 4051,1576,1577,1 551,7364, | CG2060,CG2060,CG20 60,CG2060,CG4772, |
| Chitosan | ME1, | 4199, | CG5889, |
| Chloramphenicol | CYP3A4,UGT1A4,UGT 1A3,CLTB, | 1576,54657,54659 , 1212, | CG2060,CG4772,CG47 72,CG6948, |
| chlorophenyl-ethane | CYP3A4, | 1576, | CG2060, |
| chlorophyllin | NFKB1,CCNB1, | 4790,891, | CG11992,CG5940, |
| chlorophyllypt | RFC1, | 5981, | CG1119, |

(continued)

| Small Molecule | Interacting Gene Symbol | Interacting GeneID | Drosophila Id |
|---|---|---|---|
| chlorpromazine | CYP4F11,GSR,UGT1A 4,UGT1A3,TRPA1, | 57834,2936,54657 , 54659,8989, | CG2060,CG2151,CG47 72,CG4772,CG5751, |
| Chlorpropham | FGFR3, | 2261, | CG7223, |
| Chlorzoxazone | CYP4F3,CYP3A5,CYP3 A4, | 4051,1577,1576, | CG2060,CG2060,CG20 60, |
| Cholestanol | CYP3A4, | 1576, | CG2060, |
| CHOLINE | PLXNA2,CHDH,FGFR3 ,SLC5A7, | 5362,55349,2261, 60482, | CG11081,CG1152,CG9 519,CG7223,CG7708, |
| Chonsurid | CA10, | 56934, | CG9678, |
| chromophore | LPAR2,OPN4,SAG, | 9170,94233,6295, | CG12796,CG4550,CG5 711, |
| Chrysin | UGT1A1,UGT1A6, | 54658,54578, | CG4772,CG4772, |
| chymostatin | CAPN2,CAPNS1, | 824,826, | CG8107,CG8107, |
| CI1033 | PSMD9, | 5715, | CG9588, |
| cicaprost | CCNA2, | 890, | CG5940, |
| cifostodine | SERPINB1, | 1992, | CG8137,CG9453,CG94 60, |
| ciglitazone | NFKB1,CD36,PSMD9, | 4790,948,5715, | CG11992,CG7422,CG9 588, |
| Cilazapril | PGC, | 5225, | CG10872, |
| Cilomilast | PDE4B, | 5142, | CG14940, |
| cilostazol | CYP3A4, | 1576, | CG2060, |
| Cimetidine | CYP4F3,CYP3A5,CYP3 A4,CYP3A7, | 4051,1577,1576,1 551, | CG2060,CG2060,CG20 60,CG2060, |
| cinacalcet | LPAR2,LPAR3,CYP3A4 , | 9170,23566,1576, | CG12796,CG12796,CG 2060, |
| cinitapride | CYP3A4, | 1576, | CG2060, |
| cinnamic aldehyde | TRPA1, | 8989, | CG5751, |
| cionin | SERPINB6, | 5269, | CG8137,CG9453,CG94 60, |

(continued)

| Small Molecule | Interacting Gene Symbol | Interacting GeneID | Drosophila Id |
|---|---|---|---|
| Cipol N | PSMD12,NFKB1,FOXP 3,DLG3,CYP3A5,CYP3 A4,TXNRD1,UGT2B7, CCNB1,GPR177,DYNL L1,PSIP1,SERPINC1, | 5718,4790,50943, 1741,1577,1576,7 296,7364,891,799 71,8655,11168,46 2, | CG1100,CG11992,CG1 6899,CG1725,CG2060 , CG2060,CG2151,CG4 772,CG5940,CG6210, CG6998,CG7946,CG81 37, |
| Ciprofloxacin | CYP3A4, | 1576, | CG2060, |
| Ciprol | CYP4X1,UGT1A3, | 260293,54659, | CG2060,CG4772, |
| cis-9, trans-11-conjugated linoleic acid | CD36, | 948, | CG7422, |
| Cisapride | CYP3A4, | 1576, | CG2060, |
| Citalopram | CYP3A4, | 1576, | CG2060, |
| Citox | CYP3A4, | 1576, | CG2060, |
| CITRULLINE | DYNLL1,FGFR3, | 8655,2261, | CG6998,CG7223, |
| clebopride | FGFR3, | 2261, | CG7223, |
| clevidipine | CYP3A4, | 1576, | CG2060, |
| clobazam | CYP3A4, | 1576, | CG2060, |
| Clodronic Acid | DDOST, | 1650, | CG9022, |
| clofarabine | PAFAH1B1, | 5048, | CG8440, |
| Clofibric Acid | NFKB1,CYP3A4,UGT1 A3, | 4790,1576,54659, | CG11992,CG2060,CG4 772, |
| Clomipramine | CYP3A4, | 1576, | CG2060, |
| Clonazepam | CYP3A5,CYP3A4,CYP4 F3,TYMS, UGT1A4,UG T1A3, | 1577,1576,4051,7 298,54657,54659, | CG2060,CG2060,CG20 60,CG3181,CG4772,C G4772, |
| Clonidine | S1PR1,TBXAS1,TYMS, | 1901,6916,7298, | CG12796,CG2060,CG3 181, |
| clopidogrel | CYP4F3,CYP3A4,CYP3 A5,CDK9,ME1,SERPIN C1, | 4051,1576,1577,1 025,4199,462, | CG2060,CG2060,CG20 60,CG5179,CG5889,C G8137, |
| clotiazepam | CYP3A4, | 1576, | CG2060, |
| Clozapine | SLC2A1,PSMD12,CYP 3A4,CYP3A5,CYP4F3, GAD1,PSIP1, | 6513,5718,1576,1 577,4051,2571,11 168, | CG1086,CG1100,CG20 60,CG2060,CG2060,C G7811,CG7946, |

(continued)

| Small Molecule | Interacting Gene Symbol | Interacting GeneID | Drosophila Id |
|---|---|---|---|
| clozapine N-oxide | CYP3A4,FMO3, | 1576,2328, | CG2060,CG3006, |
| CNI 1493 | DHPS, | 1725, | CG8005, |
| Co 2-1970 | GSTO1,DNALI1, | 9446,7802, | CG6673,CG6971, |
| Coagulin | NFKB1,S1PR1, | 4790,1901, | CG11992,CG12796, |
| Colchicine | PLXNB2,CADPS,CYP3 A4, | 23654,8618,1576, | CG11081,CG18026,CG 2060, |
| compactin | CDK2,PSMD9, | 1017,5715, | CG8203,CG9588, |
| CONT | SMN1,CYP3A4, | 6606,1576, | CG16725,CG2060, |
| Cotinine | GSTT1,FMO1,UGT1A9 , UGT1A1,UGT1A4, | 2952,2326,54600, 54658,54657, | CG30005,CG3006,CG4 772,CG4772,CG4772, |
| Cotrim | CYP4F3,CYP3A5, | 4051,1577, | CG2060,CG2060, |
| coumarin | CYP3A4,SERPINC1, | 1576,462, | CG2060,CG8137, |
| CRA 024781 | HDAC3, | 8841, | CG2128, |
| CRA 026440 | HDAC3, | 8841, | CG2128, |
| Crestor | CYP3A4, | 1576, | CG2060, |
| Crodacid | PSMD12,CD36, | 5718,948, | CG1100,CG7422, |
| Crypt-2,2,2 | COG6, | 57511, | CG1968, |
| cryptdin 3 | NFKB1, | 4790, | CG11992, |
| cryptotanshinone | CYP3A4, | 1576, | CG2060, |
| cryptoxanthin | TBXAS1,TYMS, | 6916,7298, | CG2060,CG3181, |
| CUBE | NFKB1, | 4790, | CG11992, |
| CVT 3146 | ADORA2A, | 135, | CG9753, |
| cyanidin 3-rutinoside | NFKB1, | 4790, | CG11992, |
| cyanidin-3-glucoside | NFKB1,CCNB1, | 4790,891, | CG11992,CG5940, |
| cyanoginosin-LA | PPP2CA, | 5515, | CG7109, |
| Cyclandelate | SERPINC1, | 462, | CG8137, |

(continued)

| Small Molecule | Interacting Gene Symbol | Interacting GeneID | Drosophila Id |
|---|---|---|---|
| cyclohexyl carbamic acid 3'-carbamoylbiphenyl-3-yl ester | TRPA1, | 8989, | CG5751, |
| cyclohexyl-methyl | CDK2, | 1017, | CG8203, |
| cyclopamine | CDK2,PSMD9, | 1017,5715, | CG8203,CG9588, |
| Cyclopentenone | CCNA2, | 890, | CG5940, |
| cyclopiazonic acid | NFKB1,CYP3A4, | 4790,1576, | CG11992,CG2060, |
| Cyproheptadine | UGT1A3, | 54659, | CG4772, |
| Cyproterone Acetate | SERPINC1, | 462, | CG8137, |
| cystathionine | ME1, | 4199, | CG5889, |
| cysteamine | STK39, | 27347, | CG7693, |
| cysteinyl-leukotriene | GPR177, | 79971, | CG6210, |
| Cytarabine | NFKB1,DNAH5,CYP3A 4,TRPV1,PAFAH1B1, | 4790,1767,1576,7 442,5048, | CG11992,CG17150,CG 2060,CG5842,CG8440 , |
| cytochalasin B | SLC2A1,SLC2A4,SMN 1,DNAH5,CD36, | 6513,6517,6606,1 767,948, | CG1086,CG1086,CG16 725,CG17150,CG7422 , |
| Cytochalasin D | CADPS,CD36,PSIP1, | 8618,948,11168, | CG18026,CG7422,CG7 946, |
| cytochalasin E | SLC2A1, | 6513, | CG1086, |
| D 22888 | PDE4A, | 5141, | CG14940, |
| D 23129 | KCNQ2,KCNQ4,KCNQ 3, | 3785,9132,3786, | CG12915,CG12915,CG 12915, |
| DA 8159 | PLXNB1,CYP3A5,CYP 3A4, | 5364,1577,1576, | CG11081,CG2060,CG2 060, |
| Dacarbazine | CYP3A5,CYP4F3, | 1577,4051, | CG2060,CG2060, |
| DADSO | NFKB1,CYP3A4,TRPA 1,TRPV1, | 4790,1576,8989,7 442, | CG11992,CG2060,CG5 751,CG5842, |
| daidzein | NFKB1,UGT1A8, | 4790,54576, | CG11992,CG4772, |
| danaproid | SERPINC1, | 462, | CG8137, |
| Dapsone | CYP4F3,CYP3A5,CYP3 A4, | 4051,1577,1576, | CG2060,CG2060,CG20 60, |

(continued)

| Small Molecule | Interacting Gene Symbol | Interacting GeneID | Drosophila Id |
|---|---|---|---|
| Daral | PLXNA2,CYP4B1,CYP 3A4,TRPV6,KCNIP3,P SMD9, | 5362,1580,1576,5 5503,30818,5715, | CG11081,CG2060,CG2 060,CG5842,CG5890, CG9588, |
| Darifenacin | CYP4F3,CYP3A5, | 4051,1577, | CG2060,CG2060, |
| darunavir | CYP3A4, | 1576, | CG2060, |
| dasatinib | CYP3A4,PSMD9, | 1576,5715, | CG2060,CG9588, |
| Daunorubicin | NFKB1,UGT8,GPR177 ,PSIP1, | 4790,7368,79971, 11168, | CG11992,CG4772,CG6 210,CG7946, |
| Dayfen | NFKB1,FOXP3,SERPIN B6,SERPINB4,ADORA 2A, | 4790,50943,5269, 6318,135, | CG11992,CG16899,CG 8137,CG9453,CG9460 , CG8137,CG9453,CG9 460,CG9753, |
| DBPC | UGT1A6, | 54578, | CG4772, |
| DDB | UBE2L3, | 7332, | CG5788, |
| DDE | PSMD9, | 5715, | CG9588, |
| Debrisoquin | CYP3A4, | 1576, | CG2060, |
| decursin | NFKB1, | 4790, | CG11992, |
| Deethylamiodarone | CYP3A4, | 1576, | CG2060, |
| deferiprone | UGT1A6, | 54578, | CG4772, |
| Deferoxamine | SLC2A1,DPAGT1,CCN A2,POLD1,CDC2,CDK 2, | 6513,1798,890,54 24,983,1017, | CG1086,CG5287,CG59 40,CG5949,CG8203,C G8203, |
| deguelin | NFKB1, | 4790, | CG11992, |
| dehydroaripiprazole | CYP3A4, | 1576, | CG2060, |
| Dehydroepiandrosterone Sulfate | CYP3A7, | 1551, | CG2060, |
| dehydroxymethylepoxyquino micin | NFKB1, | 4790, | CG11992, |
| Delavirdine | CYP3A4, | 1576, | CG2060, |
| delta8-THC | CNR1,CNR2,CADPS,T RPA1,POLD1,CDC2, | 1268,1269,8618,8 989,5424,983, | CG12796,CG12796,CG 18026,CG5751,CG594 9,CG8203, |
| Denagard | CYP3A4, | 1576, | CG2060, |

(continued)

| Small Molecule | Interacting Gene Symbol | Interacting GeneID | Drosophila Id |
|---|---|---|---|
| denbinobin | NFKB1, | 4790, | CG11992, |
| denileukin diftitox | PSIP1, | 11168, | CG7946, |
| denopamine | UGT2B7, | 7364, | CG4772, |
| Depas | CYP3A4, | 1576, | CG2060, |
| deramciclane | CYP3A4, | 1576, | CG2060, |
| desethylchloroquine | CYP3A4, | 1576, | CG2060, |
| desflurane | TRPV1, | 7442, | CG5842, |
| desisobutyrylciclesonide | CYP3A4, | 1576, | CG2060, |
| desmethylazelastine | CYP3A4, | 1576, | CG2060, |
| Desmethyldeprenyl | CYP3A4, | 1576, | CG2060, |
| Devazepide | CCKBR,CCKAR, | 887,886, | CG6881,CG6881, |
| Dexfenfluramine | CD36, | 948, | CG7422, |
| dexloxiglumide | CYP3A5,CYP4F3,CCK AR, | 1577,4051,886, | CG2060,CG2060,CG68 81, |
| Dextropropoxyphene | CYP3A4, | 1576, | CG2060, |
| dFdC | NFKB1,LARP6,TXNRD 1,TXNRD2,UBE2L3,G PR177, | 4790,55323,7296, 10587,7332,79971 , | CG11992,CG17386,CG 2151,CG2151,CG5788 ,CG6210, |
| DFMO | NFKB1,SLC25A21,CC NB1,CDK2, | 4790,89874,891,1 017, | CG11992,CG5254,CG5 940,CG8203, |
| DHEA | SLC2A1,SLC2A4,NFK B1,FOXP3,CYP3A5,CY P4F3,CYP3A4,CYP3A7 , | 6513,6517,4790,5 0943,1577,4051,1 576,1551, | CG1086,CG1086,CG11 992,CG16899,CG2060 , CG2060,CG2060,CG2 060, |
| DHLA | NFKB1, | 4790, | CG11992, |
| di-(1-isoquinolinyl)-di-(pyridyl-2')butane | CCNB1, | 891, | CG5940, |
| Diaben | CYP3A4, | 1576, | CG2060, |
| Diacomit | CYP4F3,CYP3A4,CYP3 A5, | 4051,1576,1577, | CG2060,CG2060,CG20 60, |
| diadenosine tetraphosphate | CDC2, | 983, | CG8203, |

(continued)

| Small Molecule | Interacting Gene Symbol | Interacting GeneID | Drosophila Id |
|---|---|---|---|
| Dial | NFKB1,SERPINB3,PAF AH1B1, | 4790,6317,5048, | CG11992,CG8137,CG9 453,CG9460,CG8440, |
| Diamide | NFKB1,HNRNPA1, | 4790,3178, | CG11992,CG9983, |
| DIAN | UGT1A6, | 54578, | CG4772, |
| diarsenic trioxide | SLC2A1,NFKB1,CCNA 2,CCNB1,POLD1,GPR 177,GSTO1,FGFR4,C DK2,CDC2,PSMD9, | 6513,4790,890,89 1,5424,79971,944 6,2264,1017,983,5 715, | CG1086,CG11992,CG5 940,CG5940,CG5949, CG6210,CG6673,CG72 23,CG8203,CG8203,C G9588, |
| Dibenzanthracene | CYP3A5,CYP3A7, | 1577,1551, | CG2060,CG2060, |
| Dicid | CYP4F3,CYP3A5, | 4051,1577, | CG2060,CG2060, |
| Diclofenac | CYP3A4,UGT2B7,UGT 1A6, | 1576,7364,54578, | CG2060,CG4772,CG47 72, |
| Dicyclohexylcarbodiimide | VDAC1, | 7416, | CG17137, |
| diethyl maleate | CD36, | 948, | CG7422, |
| Diethyl-benzoquinone-imine | CYP3A4, | 1576, | CG2060, |
| Digicor | NFKB1, | 4790, | CG11992, |
| Digitin | VDAC1, | 7416, | CG17137, |
| Digoxin | CYP3A4, | 1576, | CG2060, |
| Dihydroqinghaosu | UGT1A9,UGT2B7, | 54600,7364, | CG4772,CG4772, |
| Dihydroxycholecalciferols | FGR, | 2268, | CG7873, |
| diisopropyl fluorophosphate | ECD, | 11319, | CG5714, |
| dillapiol | CYP3A4, | 1576, | CG2060, |
| Diltiazem | CYP3A5,CYP3A4,CYP4 F3, | 1577,1576,4051, | CG2060,CG2060,CG20 60, |
| Dimethadione | CYP3A4, | 1576, | CG2060, |
| dimethyl fumarate | NFKB1, | 4790, | CG11992, |
| Dimethyl Sulfoxide | PSMD12,PSIP1, | 5718,11168, | CG1100,CG7946, |
| dimethyl-hydrazide | PDE4A, | 5141, | CG14940, |

(continued)

| Small Molecule | Interacting Gene Symbol | Interacting GeneID | Drosophila Id |
|---|---|---|---|
| dimethylamino-purine | POLD1,CDC2, | 5424,983, | CG5949,CG8203, |
| dimuonium | LPAR2, | 9170, | CG12796, |
| dinitrophenol | SLC2A4,SLC2A1, | 6517,6513, | CG1086,CG1086, |
| Dinoprostone | PSMD12,NFKB1,LPAR 2,PDE4A, CADPS,ONE CUT2,TRPV1,CCKBR, ANK1, | 5718,4790,9170,5 141,8618,9480,74 42,887,286, | CG1100,CG11992,CG1 2796,CG14940,CG180 26,CG1922,CG5842,C G6881,CG7462, |
| dioxirane | TBXAS1,TYMS, | 6916,7298, | CG2060,CG3181, |
| Dipalmitoyl | LPAR2,PDE4A, | 9170,5141, | CG12796,CG14940, |
| diphenylalanine | UGT1A1, | 54658, | CG4772, |
| Diphenylamine | UGT1A3, | 54659, | CG4772, |
| diphenyleneiodonium | NFKB1,MRPL41,TXNR D1,FGR, | 4790,64975,7296, 2268, | CG11992,CG12954,CG 2151,CG7873, |
| Dipyridamole | SLC2A1,SLC2A4,NFK B1,DLD, | 6513,6517,4790,1 738, | CG1086,CG1086,CG11 992,CG7430, |
| Dipyrone | CYP4F3,CYP3A4,CYP3 A5, | 4051,1576,1577, | CG2060,CG2060,CG20 60, |
| discodermolide | CYP3A4, | 1576, | CG2060, |
| Diterpenes | NFKB1, | 4790, | CG11992, |
| Dithionite | CYP3A4, | 1576, | CG2060, |
| diuretic | TRPV5,FGFR3, | 56302,2261, | CG5842,CG7223, |
| Diuron | CYP3A4, | 1576, | CG2060, |
| divinyl benzene | SERPINC1, | 462, | CG8137, |
| dl-Ipr | IGF2R,PDE4A,MFF,FG FR3, | 3482,5141,56947, 2261, | CG14255,CG14940,CG 30404,CG7223, |
| DMGG | SLC2A1,SLC2A4,CYP3 A4,SERPINC1, | 6513,6517,1576,4 62, | CG1086,CG1086,CG20 60,CG8137, |
| DMPX | ADORA2A, | 135, | CG9753, |
| DMSO | CYP3A7,CYP3A4,UBE 2L3,CD36, | 1551,1576,7332,9 48, | CG2060,CG2060,CG57 88,CG7422, |

(continued)

| Small Molecule | Interacting Gene Symbol | Interacting GeneID | Drosophila Id |
|---|---|---|---|
| Dobutamine | UGT1A3,UGT1A4, | 54659,54657, | CG4772,CG4772, |
| Doca | UGT2B7, | 7364, | CG4772, |
| Doconexent | NFKB1,UGT1A4,UGT1 A3, | 4790,54657,54659 , | CG11992,CG4772,CG4 772, |
| dodecyl-phosphocholine | ECD, | 11319, | CG5714, |
| dodecyloctaethyleneglycol monoether | SLC2A1, | 6513, | CG1086, |
| Domperidone | CYP3A4, | 1576, | CG2060, |
| DOTA | PLXNB2, | 23654, | CG11081, |
| Doxazosin | ANAPC10,NFKB1,CCN A2, | 10393,4790,890, | CG11419,CG11992,CG 5940, |
| Doxorubicin | NFKB1,LARP6,TXNRD 1,TXNRD2,GSR,TYMS, OPN4,SMAD7,UBE2L3 , CCNA2,CCNB1,POLD 1,AURKB,DLD, CDC2,C DK2,PSMD9, | 4790,55323,7296, 10587,2936,7298, 94233,4092,7332, 890,891,5424,921 2,1738,983,1017,5 715, | CG11992,CG17386,CG 2151,CG2151,CG2151 , CG3181,CG4550,CG5 201,CG5788,CG5940, CG5940,CG5949,CG66 20,CG7430,CG8203,C G8203,CG9588, |
| Doxycycline | NFKB1,CYP3A4,PRDX | 4790,1576,9588,1 | CG11992,CG2060,CG3 |
| | 6,DPAGT1, | 798, | 083,CG5287, |
| DPC 681 | CYP3A4, | 1576, | CG2060, |
| DPCPX | ADORA1, | 134, | CG9753, |
| Droxia | CYP3A4,SMAD7,POLD 1,CDC2, | 1576,4092,5424,9 83, | CG2060,CG5201,CG59 49,CG8203, |
| DTMC | DHX16, | 8449, | CG10689,CG1375, |
| dulcin | UGT1A9, | 54600, | CG4772, |
| Durapatite | CD36,ANK1,CSNK2B, | 948,286,1460, | CG7422,CG7462,CG89 14, |
| DX 9065a | SERPINC1, | 462, | CG8137, |

(continued)

| Small Molecule | Interacting Gene Symbol | Interacting GeneID | Drosophila Id |
|---|---|---|---|
| Dxms | SLC2A5,PSMD12,NFK B1,BRD8,PDE4B,PDE 4A, SMN1,CYP4A11,C YP4Z1,CYP4X1,CYP3A 4,CYP3A5,CYP3A7,UG T1A1,NAP1L1,TRPV6, CCNB1,CD36,PSIP1,S LC46A1,SERPINC1,SE RPINB3,CDK2,CDC2,P SMD9, | 6518,5718,4790,1 0902,5142,5141,6 606,1579,199974, 260293,1576,1577 , 1551,54658,4673, 55503,891,948,11 168,113235,462,6 317,1017,983,571 5, | CG1086,CG1100,CG11 992,CG14514,CG1494 0,CG14940,CG16725, CG2060,CG2060,CG20 60,CG2060,CG2060,C G2060,CG4772,CG533 0,CG5842,CG5940,CG 7422,CG7946,CG8008 , CG8137,CG8137,CG9 453,CG9460,CG8203, CG8203,CG9588, |
| Dynatra | CNR1,LPAR2,SMN1,T BXAS1,TYMS, SAG,RA SA3,CCKBR,FGFR3,G AD2,FYN,SERPINB1,A DORA2A, ADORA1, | 1268,9170,6606,6 916,7298,6295,22 821,887,2261,257 2,2534,1992,135,1 34, | CG12796,CG12796,CG 16725,CG2060,CG318 1,CG5711,CG6721,CG 6881,CG7223,CG7811 , CG7873,CG8137,CG9 453,CG9460,CG9753, CG9753, |
| E 10 | NFKB1, | 4790, | CG11992, |
| E 3330 | NFKB1, | 4790, | CG11992, |
| E-MIX 80 | CYP4F2,CYP3A4, | 8529,1576, | CG2060,CG2060, |
| E.O. | GSTT1, | 2952, | CG30005, |
| EACA | SFRS1,SERPINC1, | 6426,462, | CG6987,CG8137, |
| ebastine | CYP4F12,CYP4F3, | 66002,4051, | CG2060,CG2060, |
| ebrotidine | CYP3A4,CYP3A5, | 1576,1577, | CG2060,CG2060, |
| Echinomycin | NFKB1, | 4790, | CG11992, |
| Econ | NFKB1,CYP4F2,CYP3A 4,CCRN4L, UGT1A1,C D36,FYN, | 4790,8529,1576,2 5819,54658,948,2 534, | CG11992,CG2060,CG2 060,CG31299,CG4772 , CG7422,CG7873, |
| econazole | CYP3A4,CDK2, | 1576,1017, | CG2060,CG8203, |
| ecteinascidin 743 | CYP4F3,CYP3A4,CYP3 A5, | 4051,1576,1577, | CG2060,CG2060,CG20 60, |

(continued)

| Small Molecule | Interacting Gene Symbol | Interacting GeneID | Drosophila Id |
|---|---|---|---|
| Edetic Acid | SLC2A1,PLXNB2,CELS R1,LYRM4,ME2,POLD 1,CAPN2,CAPNS1,CD C2, | 6513,23654,9620, 57128,4200,5424, 824,826,983, | CG1086,CG11081,CG1 1895,CG3717,CG5889 , CG5949,CG8107,CG8 107,CG8203, |
| Edex | DYNC1H1,ATP1B1, | 1778,481, | CG17150,CG8663, |
| efavirenz | CYP4F3,CYP3A5,CYP3 A4, | 4051,1577,1576, | CG2060,CG2060,CG20 60, |
| EGCg | NFKB1,CYP3A4,CDK2, PSMD9, | 4790,1576,1017,5 715, | CG11992,CG2060,CG8 203,CG9588, |
| EGTA | CD36,YES1,CAPN2,CA PNS1, | 948,7525,824,826, | CG7422,CG7873,CG81 07,CG8107, |
| eletriptan | CYP3A4, | 1576, | CG2060, |
| Elicide | TRPV1, | 7442, | CG5842, |
| Empecid | THOC4,CYP3A5,CYP3 A4,CYP3A7,TRPA1,TR PV1, | 10189,1577,1576, 1551,8989,7442, | CG1101,CG2060,CG20 60,CG2060,CG5751,CG5842, |
| Enalapril | TXNRD2,TXNRD1, | 10587,7296, | CG2151,CG2151, |
| Endocannabinoids | NFKB1,CNR2,CNR1,T RPV1, | 4790,1269,1268,7 442, | CG11992,CG12796,CG 12796,CG5842, |
| endomorphin 1 | OPN4, | 94233, | CG4550, |
| Enediynes | CCNA2, | 890, | CG5940, |
| enflurane | DYNC1H1,TRPV1, | 1778,7442, | CG17150,CG5842, |
| enone | ME3, | 10873, | CG5889, |
| Enoximone | PDE4A, | 5141, | CG14940, |
| entacapone | UGT1A9, | 54600, | CG4772, |
| Entex | FMO3, | 2328, | CG3006, |
| enzastaurin | TYMS,UBE2L3, | 7298,7332, | CG3181,CG5788, |
| EOS | S1PR1, | 1901, | CG12796, |
| EPC-K(1) | NFKB1, | 4790, | CG11992, |

(continued)

| Small Molecule | Interacting Gene Symbol | Interacting GeneID | Drosophila Id |
|---|---|---|---|
| EPEG | NFKB1,CYP3A5,CYP3 A4,TFAM, UGT1A1,CC NA2,CCNB1,POLD1,G PR177,SERPINB3,CDK 2,CDC2,MLL, | 4790,1577,1576,7 019,54658,890,89 1,5424,79971,631 7,1017,983,4297, | CG11992,CG2060,CG2 060,CG4217,CG4772, CG5940,CG5940,CG59 49,CG6210,CG8137,C G9453,CG9460,CG820 3,CG8203,CG8651, |
| EPIB | CYP4A11,CD36, | 1579,948, | CG2060,CG7422, |
| epibatidine | FGFR3, | 2261, | CG7223, |
| Epicar | SPAST,SERPINB3, | 6683,6317, | CG5977,CG8137,CG94 53,CG9460, |
| Epoprostenol | NFKB1,TAOK2,DYNC1 H1,CYP3A4,SERPINC1 , | 4790,9344,1778,1 576,462, | CG11992,CG14217,CG 17150,CG2060,CG813 7, |
| epoxybergamottin | CYP3A4, | 1576, | CG2060, |
| epsilon-viniferin | CYP4F3,CYP3A5, | 4051,1577, | CG2060,CG2060, |
| erastin | VDAC2, | 7417, | CG17137, |
| ergosterol-5,8-peroxide | NFKB1, | 4790, | CG11992, |
| Eril | SMN1, | 6606, | CG16725, |
| erlotinib | CYP3A4,TYMS,UBE2L 3,PSMD9, | 1576,7298,7332,5 715, | CG2060,CG3181,CG57 88,CG9588, |
| erucin | TXNRD1, | 7296, | CG2151, |
| Eryc | CYP4F11,CYP3A4,CYP 3A5,SMAD7, | 57834,1576,1577, 4092, | CG2060,CG2060,CG20 60,CG5201, |
| erythritol anhydride | GSTT1, | 2952, | CG30005, |
| esterbut-3 | TYMS, | 7298, | CG3181, |
| Estriol | CYP3A7, | 1551, | CG2060, |
| ET18-Ome | NFKB1, | 4790, | CG11992, |
| Etfc cpd | CYP3A4, | 1576, | CG2060, |
| Ethacrynic Acid | NFKB1,GPR177, | 4790,79971, | CG11992,CG6210, |
| Ethan | CELSR1,ME2,ME1, | 9620,4200,4199, | CG11895,CG5889,CG5 889, |

(continued)

| Small Molecule | Interacting Gene Symbol | Interacting GeneID | Drosophila Id |
|---|---|---|---|
| Ethinyl-oestradiol | CYP3A4, | 1576, | CG2060, |
| Ethylmorphine | CYP4F11,OPN4, | 57834,94233, | CG2060,CG4550, |
| Ethynodiol Diacetate | SERPINC1, | 462, | CG8137, |
| Eticol | SH3D19, | 152503, | CG7129, |
| Etidronic Acid | TFAP4, | 7023, | CG7664, |
| Etodolac | UGT1A9,CCNA2,CCN B1,POLD1,SERPINB3, CDK2,CDC2, | 54600,890,891,54 24,6317,1017,983, | CG4772,CG5940,CG59 40,CG5949,CG8137,C G9453,CG9460,CG820 3,CG8203, |
| Etoposide | NFKB1,CCNA2,PSIP1,CDC2,CDK2, | 4790,890,11168,9 83,1017, | CG11992,CG5940,CG7 946,CG8203,CG8203, |
| etoricoxib | CYP3A4, | 1576, | CG2060, |
| etravirine | CYP3A4, | 1576, | CG2060, |
| Eufor | CYP4F3,CYP3A4,CYP3 A5,PSMD9, | 4051,1576,1577,5 715, | CG2060,CG2060,CG20 60,CG9588, |
| Eugenol | UBE2L3,TRPV3, | 7332,162514, | CG5788,CG5842, |
| eupatilin | UGT1A3,UGT1A4,CCN B1,POLD1,CDC2,CDK 2, | 54659,54657,891, 5424,983,1017, | CG4772,CG4772,CG59 40,CG5949,CG8203,C G8203, |
| everolimus | ACAT1,CYP3A4, | 38,1576, | CG10932,CG2060, |

(continued)

| Small Molecule | Interacting Gene Symbol | Interacting GeneID | Drosophila Id |
|---|---|---|---|
| Evex | SLC2A1,SLC2A4,CHD H, NFKB1,GABARAPL1 ,CNR1,IGF2R, SMN1,F OXP1,CYP3A5,CYP4F 3,TBXAS1,CYP3A4,PR DX6,TYMS, DLL4,OPN 4,WNT9A,WNT9B,UG T1A4,UGT2B17,UGT2 B15,UGT1A1,UGT1A3 , UGT1A10,UGT1A9,U BE2L3,TRPV6,ME3,CC NA2,CCNB1,POLD1,A URKB,AURKA, FGFR1, SCARB2,CD36,YES1,S ERPINC1,SERPINB3,C DK2,CDC2,SMARCA1, DHX9,PSMD9,HNRNP A1, | 6513,6517,55349, 4790,23710,1268, 3482,6606,27086, 1577,4051,6916,1 576,9588,7298,54 567,94233,7483,7 484,54657,7367,7 366,54658,54659, 54575,54600,7332 , 55503,10873,890, 891,5424,9212,67 90,2260,950,948,7 525,462,6317,101 7,983,6594,1660,5 715,3178, | CG1086,CG1086,CG11 52,CG9519,CG11992, CG12334,CG12796,CG 14255,CG16725,CG16 899,CG2060,CG2060, CG2060,CG2060,CG30 83,CG3181,CG3619,C G4550,CG4698,CG469 8,CG4772,CG4772,CG 4772,CG4772,CG4772 , CG4772,CG4772,CG5 788,CG5842,CG5889, CG5940,CG5940,CG59 49,CG6620,CG6620,C G7223,CG7422,CG742 2,CG7873,CG8137,CG 8137,CG9453,CG9460 , CG8203,CG8203,CG8 625,CG9323,CG9588, CG9983, |
| Evodin | CYP3A4, | 1576, | CG2060, |
| exenatide | PDHX, | 8050, | CG5261, |
| Exosurf | NFKB1, | 4790, | CG11992, |
| Expectorants | NFKB1, | 4790, | CG11992, |
| Extina | CYP3A4,CYP4F3,CYP4 F2,CYP3A5,UGT1A3,U GT1A1,UGT1A9,UGT1 A4, | 1576,4051,8529,1 577,54659,54658, 54600,54657, | CG2060,CG2060,CG20 60,CG2060,CG4772,C G4772,CG4772,CG477 2, |
| Ezerin | FGFR3, | 2261, | CG7223, |
| ezetimib | CD36, | 948, | CG7422, |
| Facet | FOXP3, | 50943, | CG16899, |
| Facid | RFC1,CNR2,TYMS,FG FR4,SLC46A1,KHSRP, | 5981,1269,7298,2 264,113235,8570, | CG1119,CG12796,CG3 181,CG7223,CG8008, CG8912, |
| facile | MRPL13,TYMS, | 28998,7298, | CG10603,CG3181, |

(continued)

| Small Molecule | Interacting Gene Symbol | Interacting GeneID | Drosophila Id |
|---|---|---|---|
| Factor IIa | SERPINC1, | 462, | CG8137, |
| FAMP | NFKB1,SERPINB6,PS MD9, | 4790,5269,5715, | CG11992,CG8137,CG9 453,CG9460,CG9588, |
| Fanchinine | CYP3A4,TYMS,SMAD7 ,UBE2L3, | 1576,7298,4092,7 332, | CG2060,CG3181,CG52 01,CG5788, |
| Farnesyl-PP | TXNRD2,TXNRD1, | 10587,7296, | CG2151,CG2151, |
| farnesylthiosalicylic acid | UBE2L3, | 7332, | CG5788, |
| febuxostat | CYP4F3,CYP3A5,UGT 1A4,UGT1A3, | 4051,1577,54657, 54659, | CG2060,CG2060,CG47 72,CG4772, |
| felbamate | CYP3A4, | 1576, | CG2060, |
| Felodipine | CYP3A4,CYP3A5, | 1576,1577, | CG2060,CG2060, |
| Fenfluramine | CYP4F3,CYP3A5, | 4051,1577, | CG2060,CG2060, |
| fenitrothion | TBXAS1,TYMS, | 6916,7298, | CG2060,CG3181, |
| fenofibric acid | CYP4A11,CYP3A4, | 1579,1576, | CG2060,CG2060, |
| Fenretinide | UBE2L3, | 7332, | CG5788, |
| Fentanyl | PLXNB1,NFKB1,CYP3 A5,CYP3A4, | 5364,4790,1577,1 576, | CG11081,CG11992,CG 2060,CG2060, |
| ferulic acid | CCNB1,POLD1,CDC2, | 891,5424,983, | CG5940,CG5949,CG82 03, |
| Filipin | CD36,YES1, | 948,7525, | CG7422,CG7873, |
| fingolimod | S1PR2,CNR1,S1PR5,S 1PR1,CDK2, | 9294,1268,53637, 1901,1017, | CG12796,CG12796,CG 12796,CG12796,CG82 03, |
| fipronil | CYP3A4, | 1576, | CG2060, |
| fisetin | NFKB1,CD36, | 4790,948, | CG11992,CG7422, |
| Flanin F | GSR, | 2936, | CG2151, |
| Flavon | CYP3A5,CYP3A4,DYN LL1,CDK2, | 1577,1576,8655,1 017, | CG2060,CG2060,CG69 98,CG8203, |
| flavonols | CCNB1,CD36, | 891,948, | CG5940,CG7422, |

(continued)

| Small Molecule | Interacting Gene Symbol | Interacting GeneID | Drosophila Id |
|---|---|---|---|
| flavopiridol | NFKB1,UGT1A1,CDK9 , UBE2L3,CCNB1,CDC 2,CDK2,CDK5, | 4790,54658,1025, 7332,891,983,101 7,1020, | CG11992,CG4772,CG5 179,CG5788,CG5940, CG8203,CG8203,CG82 03, |
| Flavyl | KCNQ3,KCNQ2,CYP3A 5,CYP4F3,CYP3A4, | 3786,3785,1577,4 051,1576, | CG12915,CG12915,CG 2060,CG2060,CG2060 , |
| FLCZ | CYP3A4,TXNRD1,UGT 2B7, | 1576,7296,7364, | CG2060,CG2151,CG47 72, |
| Flecainide | KCNIP2, | 30819, | CG5890, |
| Floxacillin | CYP3A4, | 1576, | CG2060, |
| flufenamic acid | NFKB1,TRPV1, | 4790,7442, | CG11992,CG5842, |
| Flunitrazepam | CYP3A5,UGT1A3,UGT 1A1,UGT2B7, | 1577,54659,54658 , 7364, | CG2060,CG4772,CG47 72,CG4772, |
| fluorexon | GPR177, | 79971, | CG6210, |
| Fluorouracil | SLC2A1,NFKB1,TAOK 2,LARP6,CYP3A4,TBX AS1,TYMS, DTYMK,UB E2L3,CCNA2,CCNB2, SERPINB6, | 6513,4790,9344,5 5323,1576,6916,7 298,1841,7332,89 0,9133,5269, | CG1086,CG11992,CG1 4217,CG17386,CG206 0,CG2060,CG3181,CG 5757,CG5788,CG5940 , CG5940,CG8137,CG9 453,CG9460, |
| fluvoxamine | CYP3A4, | 1576, | CG2060, |
| FOLATE-ANALOG | TYMS, | 7298, | CG3181, |
| fondaparinux | SERPINC1, | 462, | CG8137, |
| Fonofos | CYP3A4, | 1576, | CG2060, |
| Format | CADPS,ELAVL2, | 8618,1993, | CG18026,CG4396, |
| Formyl-Tetrahydrofolate | TYMS, | 7298, | CG3181, |
| Forskolin | SLC2A5,SLC2A4,SLC2 A2,SLC2A1,LYZ,NFKB 1,S1PR1,PDE4A,CYP3 A4,TRPV1,FGFR4,TFA P4,ATP1B1, | 6518,6517,6514,6 513,4069,4790,19 01,5141,1576,744 2,2264,7023,481, | CG1086,CG1086,CG10 86,CG1086,CG1180,C G11992,CG12796,CG1 4940,CG2060,CG5842 , CG7223,CG7664,CG8 663, |
| fosamprenavir | CYP3A4, | 1576, | CG2060, |

(continued)

| Small Molecule | Interacting Gene Symbol | Interacting GeneID | Drosophila Id |
|---|---|---|---|
| Foscarnet | NFKB1, | 4790, | CG11992, |
| FR 120480 | CCKAR, | 886, | CG6881, |
| FR 235222 | NFKB1, | 4790, | CG11992, |
| fraxin | API5, | 8539, | CG6582, |
| FTY 720P | S1PR1, | 1901, | CG12796, |
| fucoidan | NFKB1,SERPINC1, | 4790,462, | CG11992,CG8137, |
| fulvestrant | UGT2B15,UGT1A8,UG T1A10,CD36, | 7366,54576,54575 , 948, | CG4772,CG4772,CG47 72,CG7422, |
| fumagillin | CYP4V2, | 285440, | CG2060, |
| Fura-2 | FGFR3, | 2261, | CG7223, |
| furafylline | CYP3A4, | 1576, | CG2060, |
| Furamon | FGFR3, | 2261, | CG7223, |
| Furylfuramide | THOC4,TXNRD2, | 10189,10587, | CG1101,CG2151, |
| Gabexate | SERPINC1, | 462, | CG8137, |
| gadolinium | CELSR1,ME2,ME3, | 9620,4200,10873, | CG11895,CG5889,CG5 889, |
| Gadolinium DTPA | TXNRD2,TXNRD1,CD 36, | 10587,7296,948, | CG2151,CG2151,CG74 22, |
| galactocerebroside | UGT8, | 7368, | CG4772, |
| galactomannan | CSNK2B, | 1460, | CG8914, |
| galangin | CDC2, | 983, | CG8203, |
| galaturonate | CYP4F3,CYP3A5,UGT 1A3,UGT1A4,UGT1A1 0, | 4051,1577,54659, 54657,54575, | CG2060,CG2060,CG47 72,CG4772,CG4772, |
| gallic acid | POLD1,CDC2, | 5424,983, | CG5949,CG8203, |
| Gallogen | CDK2, | 1017, | CG8203, |
| gambierol | TRPV1, | 7442, | CG5842, |
| Gambogic acid | NFKB1,POLD1,CDC2, | 4790,5424,983, | CG11992,CG5949,CG8 203, |
| gamma-butyric-acid | KCNIP3, | 30818, | CG5890, |

(continued)

| Small Molecule | Interacting Gene Symbol | Interacting GeneID | Drosophila Id |
|---|---|---|---|
| Ganciclovir | PDHX, | 8050, | CG5261, |
| gastrin 17 | NFKB1,CCKBR, | 4790,887, | CG11992,CG6881, |
| gatifloxacin | NFKB1, | 4790, | CG11992, |
| gefitinib | SERPINB3,PSMD9, | 6317,5715, | CG8137,CG9453,CG94 60,CG9588, |
| Geldanamycin | NFKB1,CNR2,CDK9,C CNB1,POLD1,CDK2,C DC2,FAM162A, | 4790,1269,1025,8 91,5424,1017,983, 26355, | CG11992,CG12796,CG 5179,CG5940,CG5949 , CG8203,CG8203,CG9 231, |
| Gemfibrozil | CYP3A4,UGT2B7, | 1576,7364, | CG2060,CG4772, |
| gemtuzumab | NOMO1, | 23420, | CG1371, |
| Gentamicins | UGT8,FGFR3, | 7368,2261, | CG4772,CG7223, |
| gepirone | CYP3A4, | 1576, | CG2060, |
| geraniol | CYP3A5, | 1577, | CG2060, |
| geranylcoumarin | CYP3A4, | 1576, | CG2060, |
| Gestodene | CYP3A4, | 1576, | CG2060, |
| GF 120918 | CYP3A4, | 1576, | CG2060, |
| GGTI 298 | CDK2,PSMD9, | 1017,5715, | CG8203,CG9588, |
| GI 129471 | PSMD12, | 5718, | CG1100, |
| gingerol | NFKB1,CCNA2, | 4790,890, | CG11992,CG5940, |
| ginsenoside Rd | CYP3A4, | 1576, | CG2060, |
| ginsenoside Rf | CYP3A4, | 1576, | CG2060, |
| ginsenoside Rg1 | CDK2, | 1017, | CG8203, |
| ginsenoside Rh2 | CDK2, | 1017, | CG8203, |
| Ginsenosides | NFKB1,FGFR4, | 4790,2264, | CG11992,CG7223, |
| GLCa | SERPINC1,DHX9, | 462,1660, | CG8137,CG9323, |
| Gliclazide | SLC2A4,CD36, | 6517,948, | CG1086,CG7422, |

(continued)

| Small Molecule | Interacting Gene Symbol | Interacting GeneID | Drosophila Id |
|---|---|---|---|
| Glumin | THOC4,PQBP1,FOXP2 ,PDHX, | 10189,10084,9398 6,8050, | CG1101,CG11820,CG1 6899,CG5261, |
| Glyoxal | TRPV6, | 55503, | CG5842, |
| Gnidimacrin | CDK2, | 1017, | CG8203, |
| GnRH | LPAR2,SMAD7,FGFR1 'PSIP1, | 9170,4092,2260,1 1168, | CG12796,CG5201,CG7 223,CG7946, |
| Go 6976 | NFKB1, | 4790, | CG11992, |
| gossypol | TXNRD1,ME2,ME1,ME 3, | 7296,4200,4199,1 0873, | CG2151,CG5889,CG58 89,CG5889, |
| GR 79236X | ADORA1, | 134, | CG9753, |
| gramicidin S | HDHD1A, | 8226, | CG5565, |
| Granisetron | CYP3A4, | 1576, | CG2060, |
| Gravistat | SERPINC1, | 462, | CG8137, |
| Grofo | CYP3A5,CYP3A4,CYP4 F3, | 1577,1576,4051, | CG2060,CG2060,CG20 60, |
| Guggulsterone | NFKB1, | 4790, | CG11992, |
| GW 4064 | UGT2B15, | 7366, | CG4772, |
| GW 501516 | NFKB1, | 4790, | CG11992, |
| H 89 | CYP3A4, | 1576, | CG2060, |
| Halan | PLXNB1,CYP4F3,CYP3 A4,CYP3A5, | 5364,4051,1576,1 577, | CG11081,CG2060,CG2 060,CG2060, |
| halofuginone | RPL10,SMAD7, | 6134,4092, | CG17521,CG5201, |
| harmine | CCNA2,CDK5,CDC2,C DK2, | 890,1020,983,101 7, | CG5940,CG8203,CG82 03,CG8203, |
| Harzol | CCNB1,POLD1,CDC2, | 891,5424,983, | CG5940,CG5949,CG82 03, |
| hassium | CCNA2,FGFR2,ANK1, CDC2, | 890,2263,286,983, | CG5940,CG7223,CG74 62,CG8203, |
| HDMTX | RFC1,NFKB1,TXNRD2 , TXNRD1,TYMS,SLC4 6A1,SERPINC1, | 5981,4790,10587, 7296,7298,113235 , 462, | CG1119,CG11992,CG2 151,CG2151,CG3181, CG8008,CG8137, |

(continued)

| Small Molecule | Interacting Gene Symbol | Interacting GeneID | Drosophila Id |
|---|---|---|---|
| Hecogenin | UGT1A4, | 54657, | CG4772, |
| Hectorol | CYP3A4, | 1576, | CG2060, |
| Heet | CYP4F3,CYP3A5, | 4051,1577, | CG2060,CG2060, |
| helenalin | NFKB1, | 4790, | CG11992, |
| Hemicholinium 3 | FGFR3,SLC5A7, | 2261,60482, | CG7223,CG7708, |
| herbimycin | NFKB1,PSMD9, | 4790,5715, | CG11992,CG9588, |
| hesperadin | AURKB, | 9212, | CG6620, |
| HESPERETIN | CDK2, | 1017, | CG8203, |
| Hexadimethrine | SERPINC1, | 462, | CG8137, |
| hexarelin | CD36, | 948, | CG7422, |
| Hgln | CCKBR, | 887, | CG6881, |
| himbacine | FGFR3, | 2261, | CG7223, |
| Hk | SLC2A4,VDAC1,EXOS C1, | 6517,7416,51013, | CG1086,CG17137,CG6 249, |
| Hocus | SLC26A6,NFKB1,S1P R3,CYP3A7,CYP3A4,C CRN4L, UGT1A4,UGT1 A3,UGT1A1,UGT2B7, TRPV1,FGFR3,SERPIN C1, | 65010,4790,1903, 1551,1576,25819, 54657,54659,5465 8,7364,7442,2261, 462, | CG11895,CG11992,CG 12796,CG2060,CG206 0,CG31299,CG4772,C G4772,CG4772,CG477 2,CG5842,CG7223,CG 8137, |
| HOE 33342 | UBE2L3, | 7332, | CG5788, |
| honokiol | UBE2L3, | 7332, | CG5788, |
| Horner | ODZ1, | 10178, | CG5723, |
| HS 1200 | CCNA2,CDK2, | 890,1017, | CG5940,CG8203, |
| HU 211 | CNR1,CNR2,TRPV1, | 1268,1269,7442, | CG12796,CG12796,CG 5842, |
| HyateC | LMAN1, | 3998, | CG6822, |
| Hydoxin | DPAGT1,SERPINC1, | 1798,462, | CG5287,CG8137, |
| hydride | ME1,ME2,ME3, | 4199,4200,10873, | CG5889,CG5889,CG58 89, |

(continued)

| Small Molecule | Interacting Gene Symbol | Interacting GeneID | Drosophila Id |
|---|---|---|---|
| Hydromorphone | CYP3A4, | 1576, | CG2060, |
| Hydroxychloroquine | UBE2L3, | 7332, | CG5788, |
| hydroxycotinine | UGT1A4, | 54657, | CG4772, |
| hydroxylamine | PLXNB2,SLC25A21, | 23654,89874, | CG11081,CG5254, |
| Hydroxytryptophol | UGT1A6, | 54578, | CG4772, |
| Hyhorin | SERPINC1, | 462, | CG8137, |
| Hypaque | CLTA, | 1211, | CG6948, |
| hyperforin | CYP3A4, | 1576, | CG2060, |
| hypericin | NFKB1, | 4790, | CG11992, |
| Hypericum-perforatum | CYP3A4, | 1576, | CG2060, |
| hypochlorous acid | NFKB1,ATP1B1, | 4790,481, | CG11992,CG8663, |
| iberin | TXNRD1,UGT1A1, | 7296,54658, | CG2151,CG4772, |
| IBMX | PDE1B,PDE1C,PDE4A, | 5153,5137,5141, | CG14940,CG14940,CG 14940, |
| ibopamine | SMARCA1, | 6594, | CG8625, |
| ibudilast | PDE4A, | 5141, | CG14940, |
| IC 831423 | SERPINC1, | 462, | CG8137, |
| icariin | PDE4A, | 5141, | CG14940, |
| icaritin | FGFR4, | 2264, | CG7223, |
| icilin | TRPA1,TRPV1, | 8989,7442, | CG5751,CG5842, |
| ICRF 193 | NFKB1, | 4790, | CG11992, |
| IDS 23 | NFKB1, | 4790, | CG11992, |
| Ifosfamide | CYP4F3,CYP3A4,CYP3 A5, | 4051,1576,1577, | CG2060,CG2060,CG20 60, |
| Ikarugamycin | SFRS1, | 6426, | CG6987, |
| ilimaquinone | NFKB1, | 4790, | CG11992, |
| Iloprost | DYNC1H1,RBM6, | 1778,10180, | CG17150,CG4887, |

(continued)

| Small Molecule | Interacting Gene Symbol | Interacting GeneID | Drosophila Id |
|---|---|---|---|
| Imadyl | PSIP1, | 11168, | CG7946, |
| imatinib | FOXP3,CYP3A4,FZR1, CCNA2,POLD1,FGFR4 , CDC2,CDK5,CDK2,PS MD9, | 50943,1576,51343 , 890,5424,2264,98 3,1020,1017,5715, | CG16899,CG2060,CG3 000,CG5940,CG5949, CG7223,CG8203,CG82 03,CG8203,CG9588, |
| imidafenacin | CYP3A4, | 1576, | CG2060, |
| imidazo-pyridine | CYP3A4, | 1576, | CG2060, |
| imidazolidin-2-one | PDE4A, | 5141, | CG14940, |
| imidazolidin-one | PDE4A, | 5141, | CG14940, |
| imidazolidine | TXNRD2,TXNRD1, | 10587,7296, | CG2151,CG2151, |
| Imidazoline | NFKB1,ADORA1, | 4790,134, | CG11992,CG9753, |
| imidazolyl-disulfide | TXNRD1, | 7296, | CG2151, |
| Imipenem | VDAC1, | 7416, | CG17137, |
| Imizin | CYP4F11,CYP3A4,UG T1A4, | 57834,1576,54657 , | CG2060,CG2060,CG47 72, |
| Immulina | NFKB1, | 4790, | CG11992, |
| Immunoferon | NFKB1, | 4790, | CG11992, |
| Impulsin | CNR1, | 1268, | CG12796, |
| Imrecoxib | CYP3A4, | 1576, | CG2060, |
| Imutex | CYP4F3,CYP3A5,CYP3 A7, | 4051,1577,1551, | CG2060,CG2060,CG20 60, |
| Indinavir | SLC2A4,CYP3A4,CYP4 F3,CYP3A7,CYP3A5,U GT1A1,GPR177, | 6517,1576,4051,1 551,1577,54658,7 9971, | CG1086,CG2060,CG20 60,CG2060,CG2060,C G4772,CG6210, |
| indiplon | CYP3A4,CYP3A5, | 1576,1577, | CG2060,CG2060, |
| indirubin | NFKB1,CDK2,CDC2,CDK5, | 4790,1017,983,1020, | CG11992,CG8203,CG8203,CG8203, |
| indole-3-acetic acid | GAD2,GAD1, | 2572,2571, | CG7811,CG7811, |
| indole-3-methanol | NFKB1, | 4790, | CG11992, |
| indolin-2-one | CCKBR, | 887, | CG6881, |

(continued)

| Small Molecule | Interacting Gene Symbol | Interacting GeneID | Drosophila Id |
|---|---|---|---|
| indolin-one | CDK2, | 1017, | CG8203, |
| infliximab | SLC2A4,PSMD12,NFK B1,FOXP3,ELAVL1, | 6517,5718,4790,5 0943,1994, | CG1086,CG1100,CG11 992,CG16899,CG4396 , |
| inhibin B | SMAD7, | 4092, | CG5201, |
| INOmax | PLXNB2,NFKB1,LPAR 2,DNAH5,CYP4F3,CYP 3A5,CYP3A4,TXNRD1, TRPV1,DYNLL1,FGFR 3,FGFR1,GAD2,SERPI NB3, | 23654,4790,9170, 1767,4051,1577,1 576,7296,7442,86 55,2261,2260,257 2,6317, | CG11081,CG11992,CG 12796,CG17150,CG20 60,CG2060,CG2060,C G2151,CG5842,CG699 8,CG7223,CG7223,CG 7811,CG8137,CG9453 ,CG9460, |
| inositol-1,3,4,5-tetrakisphosphate | RASA3, | 22821, | CG6721, |
| inulin | PSIP1, | 11168, | CG7946, |
| Iodoacetamide | TRPA1, | 8989, | CG5751, |
| iodomethane | CHD2, | 1106, | CG3733, |
| iodoresiniferatoxin | TRPV1, | 7442, | CG5842, |
| Ionomycin | NFKB1,FGFR3,CAPNS 1, | 4790,2261,826, | CG11992,CG7223,CG8 107, |
| ionophore | NFKB1,DYNLL1,FGFR 4,YES1, | 4790,8655,2264,7 525, | CG11992,CG6998,CG7 223,CG7873, |
| Iopanoic Acid | PSMD9, | 5715, | CG9588, |
| lophendylate | TXNRD2,TXNRD1, | 10587,7296, | CG2151,CG2151, |
| IPADE | CDK2, | 1017, | CG8203, |
| IPOMEANOL | CYP4F3,CYP4B1,CYP3 A4,CYP3A5, | 4051,1580,1576,1 577, | CG2060,CG2060,CG20 60,CG2060, |
| Iressa | CYP3A4,UBE2L3,SPA G9,PSMD9, | 1576,7332,9043,5 715, | CG2060,CG5788,CG81 10,CG9588, |
| irinotecan | NFKB1,CYP3A4,TYMS, UGT1A7,UGT1A1,UGT 1A4,UGT1A9,UGT1A3 ,CDC2, | 4790,1576,7298,5 4577,54658,54657 , 54600,54659,983, | CG11992,CG2060,CG3 181,CG4772,CG4772, CG4772,CG4772,CG47 72,CG8203, |
| irisolidone | NFKB1, | 4790, | CG11992, |

(continued)

| Small Molecule | Interacting Gene Symbol | Interacting GeneID | Drosophila Id |
|---|---|---|---|
| Isatin | CNR2, | 1269, | CG12796, |
| isaxonine | CYP4F3,CYP3A5, | 4051,1577, | CG2060,CG2060, |
| isoamylol | CYP3A4, | 1576, | CG2060, |
| isobutyl-methyl-Xanthine | CAMK2B, | 816, | CG18069, |
| Isodonol | NFKB1, | 4790, | CG11992, |
| isoflavone | CYP3A4, | 1576, | CG2060, |
| isoflurane | DYNC1H1,TRPV1, | 1778,7442, | CG17150,CG5842, |
| Isol | ME1, | 4199, | CG5889, |
| Isoliquiritigenin | NFKB1, | 4790, | CG11992, |
| isometronidazole | CHM, | 1121, | CG8432, |
| isoprenoids | PSMD9, | 5715, | CG9588, |
| Isopropyl Thiogalactoside | LETMD1, | 25875, | CG5989, |
| Isoprostanes | FGFR3, | 2261, | CG7223, |
| Isorhamnetin | NFKB1, | 4790, | CG11992, |
| isosilybin A | NFKB1, | 4790, | CG11992, |
| Isosorbide Dinitrate | SERPINC1, | 462, | CG8137, |
| isothiocyanates | NFKB1, | 4790, | CG11992, |
| Isotretinoin | CYP3A4,SFRS1,PAFA H1B1, | 1576,6426,5048, | CG2060,CG6987,CG84 40, |
| Isradipine | CYP3A4, | 1576, | CG2060, |
| istradefylline | ADORA2A, | 135, | CG9753, |
| Itraconazole | CYP4F3,CYP3A5,CYP3 A4, | 4051,1577,1576, | CG2060,CG2060,CG20 60, |
| ivabradine | CYP3A4, | 1576, | CG2060, |
| Ivermectin | GPR177, | 79971, | CG6210, |
| ixabepilone | CYP3A4, | 1576, | CG2060, |
| jadomycin B | AURKB, | 9212, | CG6620, |

(continued)

| Small Molecule | Interacting Gene Symbol | Interacting GeneID | Drosophila Id |
|---|---|---|---|
| Jexin | FGFR3, | 2261, | CG7223, |
| JHW 015 | CNR2, | 1269, | CG12796, |
| JTE 013 | S1PR2,S1PR1,S1PR3, | 9294,1901,1903, | CG12796,CG12796,CG 12796, |
| K 252 | POLD1,CDC2, | 5424,983, | CG5949,CG8203, |
| K-PAM | LMAN1,SERPINC1, | 3998,462, | CG6822,CG8137, |
| K-SR | SAG,PPP2CA,BDP1, | 6295,5515,55814, | CG5711,CG7109,CG93 05, |
| kaempferol | NFKB1,CYP3A4,CD36, MLL, | 4790,1576,948,42 97, | CG11992,CG2060,CG7 422,CG8651, |
| kaempferol-3-O-(2,3,4-tri-O-acetyl-alpha-l-rhamnopyranoside) | CYP3A4, | 1576, | CG2060, |
| KAFA | CYP4F3,CYP3A5, | 4051,1577, | CG2060,CG2060, |
| Kaken | THOC4,NFKB1,S1PR1, WDR92,BRD8,TXNRD 1,TXNRD2,TYMS,KHD RBS1,UBE2L3,CCNA2, FGR, PSIP1,CDK2,SNR PG, | 10189,4790,1901, 116143,10902,729 6,10587,7298,106 57,7332,890,2268, 11168,1017,6637, | CG1101,CG11992,CG1 2796,CG14353,CG145 14,CG2151,CG2151,C G3181,CG3613,CG582 1,CG5788,CG5940,CG 7873,CG7946,CG8203 ,CG9742, |
| Kamalin | CCNA2,POLD1,CDC2, | 890,5424,983, | CG5940,CG5949,CG82 03, |
| Kaolin | SERPINC1, | 462, | CG8137, |
| Kathon 886 | DLD, | 1738, | CG7430, |
| KB 141 | TXNRD2, | 10587, | CG2151, |
| Kemi | UGT2B4,UGT1A9,UGT 2B7,GSTO1,DNALI1,D YNLL1, | 7363,54600,7364, 9446,7802,8655, | CG4772,CG4772,CG47 72,CG6673,CG6971,C G6998, |
| kenpaullone | CDC2,CDK5, | 983,1020, | CG8203,CG8203, |
| Ketamine | SLC2A1,FGFR3, | 6513,2261, | CG1086,CG7223, |
| Keto-desogestrel | CYP3A4, | 1576, | CG2060, |
| Keto-pgf1alpha | SERPINC1, | 462, | CG8137, |
| ketoglutarate | CELSR1,ME2, | 9620,4200, | CG11895,CG5889, |

(continued)

| Small Molecule | Interacting Gene Symbol | Interacting GeneID | Drosophila Id |
|---|---|---|---|
| Kipca | NFKB1,SERPINC1,CD C2, | 4790,462,983, | CG11992,CG8137,CG8 203, |
| KMD 3213 | CYP3A4, | 1576, | CG2060, |
| KMTB | CTBP1, | 1487, | CG7583, |
| Kojic acid | NFKB1, | 4790, | CG11992, |
| KR-31543 | CYP3A4, | 1576, | CG2060, |
| KRM 1648 | CYP3A4, | 1576, | CG2060, |
| L 365260 | CCKBR, | 887, | CG6881, |
| L 740,093 | CCKBR, | 887, | CG6881, |
| L-454,560 | PDE4A, | 5141, | CG14940, |
| L-696,474 | SLC2A1, | 6513, | CG1086, |
| L-T3 | CYP3A4,TXNRD1,TXNRD2, | 1576,7296,10587, | CG2060,CG2151,CG2151, |
| LAAM | CYP3A4, | 1576, | CG2060, |
| lacidipine | NFKB1, | 4790, | CG11992, |
| lactacystin | NFKB1,TXNRD2,TXNR D1,SMAD7,PSMD9, | 4790,10587,7296, 4092,5715, | CG11992,CG2151,CG2 151,CG5201,CG9588, |
| lactisole | TAS1R2, | 80834, | CG7145, |
| lamotrigine | UGT1A3,UGT1A4, | 54659,54657, | CG4772,CG4772, |
| Lanol | ACAT2,ACAT1,PLXNB 2,PQBP1,NFKB1,CNR 1,LPAR2,VDAC1,DNA H5,CYP3A4,CYP4F3,C YP3A5,TXNRD2,DLL1, COG2,SCARB2,CD36, SERPINC1,CDC2,PAFA H1B1, | 39,38,23654,1008 4,4790,1268,9170, 7416,1767,1576,4 051,1577,10587,2 8514,22796,950,9 48,462,983,5048, | CG10932,CG10932,CG 11081,CG11820,CG11 992,CG12796,CG1279 6,CG17137,CG17150, CG2060,CG2060,CG20 60,CG2151,CG3619,C G6177,CG7422,CG742 2,CG8137,CG8203,CG 8440, |
| lansoprazole | CYP4F3,CYP3A4,CYP3 A5, | 4051,1576,1577, | CG2060,CG2060,CG20 60, |
| lapatinib | CYP3A4,PSMD9, | 1576,5715, | CG2060,CG9588, |
| laquinimod | CYP3A4, | 1576, | CG2060, |

(continued)

| Small Molecule | Interacting Gene Symbol | Interacting GeneID | Drosophila Id |
|---|---|---|---|
| latrunculin A | DNAH5, | 1767, | CG17150, |
| latrunculin B | SMN1, | 6606, | CG16725, |
| lavendustin A | FGFR4, | 2264, | CG7223, |
| LBH589 | PSMD9, | 5715, | CG9588, |
| leflunomide | FYN, | 2534, | CG7873, |
| lenalidomide | PAFAH1B1, | 5048, | CG8440, |
| Lendorm | CYP3A4, | 1576, | CG2060, |
| Lentinan | TAOK2, | 9344, | CG14217, |
| leptomycin B | PHB,NFKB1,CCNB1,P SMD9, | 5245,4790,891,57 15, | CG10691,CG11992,CG 5940,CG9588, |
| Leucovorin | NFKB1,TBXAS1,TYMS, | 4790,6916,7298, | CG11992,CG2060,CG3 181, |
| Leukotriene C4 | NFKB1,GPR177, | 4790,79971, | CG11992,CG6210, |
| Leukotriene D4 | NFKB1,LPAR2, | 4790,9170, | CG11992,CG12796, |
| leukotrienes | LPAR2,CYP4F3,NAP1L 1, | 9170,4051,4673, | CG12796,CG2060,CG5 330, |
| Leupeptin | NFKB1,CAPNS1,CAPN 2, | 4790,826,824, | CG11992,CG8107,CG8 107, |
| Levamisole | DPAGT1, | 1798, | CG5287, |
| Levitra | CYP3A4,CYP3A5, | 1576,1577, | CG2060,CG2060, |
| levobupivacaine | CYP3A4, | 1576, | CG2060, |
| Levonorgestrel | DLL1, | 28514, | CG3619, |
| levugen | SLC2A1,SLC2A5,SLC2 A3,SLC2A2,SLC2A4, | 6513,6518,6515,6 514,6517, | CG1086,CG1086,CG10 86,CG1086,CG1086, |
| liarozole | TBXAS1, | 6916, | CG2060, |
| Lidocaine | CYP3A4,TRPV1, | 1576,7442, | CG2060,CG5842, |
| lilopristone | CYP3A4, | 1576, | CG2060, |
| Lipoate | NFKB1,DLAT, | 4790,1737, | CG11992,CG5261, |
| Lipofectamine | GPR177, | 79971, | CG6210, |

(continued)

| Small Molecule | Interacting Gene Symbol | Interacting GeneID | Drosophila Id |
|---|---|---|---|
| lipoteichoic acid | NFKB1,CD36, | 4790,948, | CG11992,CG7422, |
| Lipoxins | LPAR2, | 9170, | CG12796, |
| lissamine rhodamine B | CYP3A4, | 1576, | CG2060, |
| lithocholic acid | NFKB1,CYP3A4,UGT2 B7, | 4790,1576,7364, | CG11992,CG2060,CG4 772, |
| LMWH | BRD8,SMN1,TBXAS1, TYMS,PDHX, NAP1L4, SAG,FGFR1,PSIP1,SE RPINC1,SERPINB1,CD K2, | 10902,6606,6916, 7298,8050,4676,6 295,2260,11168,4 62,1992,1017, | CG14514,CG16725,CG 2060,CG3181,CG5261,CG5330,CG571 1,CG7 223,CG7946,CG8137, CG8137,CG9453,CG94 60,CG8203, |
| LNAC | NFKB1,CD36,PSIP1,P SMD9, | 4790,948,11168,5 715, | CG11992,CG7422,CG7 946,CG9588, |
| lonafarnib | NFKB1, | 4790, | CG11992, |
| Loperamide | CYP3A4, | 1576, | CG2060, |
| lopinavir | CYP3A4, | 1576, | CG2060, |
| Loratadine | CYP3A4, | 1576, | CG2060, |
| Lorazepam | PLXNA2,ECD, | 5362,11319, | CG11081,CG5714, |
| Lorex | CYP3A4, | 1576, | CG2060, |
| lorglumide | CCKBR,CCKAR, | 887,886, | CG6881,CG6881, |
| Losartan | CYP3A4, | 1576, | CG2060, |
| Lovan | ME1, | 4199, | CG5889, |
| loxiglumide | CCKAR, | 886, | CG6881, |
| LUF 5831 | ADORA1, | 134, | CG9753, |
| lupeol | POLD1,CDC2, | 5424,983, | CG5949,CG8203, |
| luteolin | CYP3A4,CYP3A5,CCN B1,EXOSC1, | 1576,1577,891,51 013, | CG2060,CG2060,CG59 40,CG6249, |
| LY 117018 | FGFR1, | 2260, | CG7223, |
| LY 293111 | CCNA2,CDK2,PSMD9, | 890,1017,5715, | CG5940,CG8203,CG95 88, |
| LY231514 | TBXAS1,TYMS, | 6916,7298, | CG2060,CG3181, |

(continued)

| Small Molecule | Interacting Gene Symbol | Interacting GeneID | Drosophila Id |
|---|---|---|---|
| LYCOPENE | NFKB1,PSMD9, | 4790,5715, | CG11992,CG9588, |
| lysophosphatidic acid | NFKB1,S1PR4,S1PR1, LPAR2,LPAR1,S1PR5, S1PR3,SMN1,SMAD7, CD36,ANK1,FYN, | 4790,8698,1901,9 170,1902,53637,1 903,6606,4092,94 8,286,2534, | CG11992,CG12796,CG 12796,CG12796,CG12 796,CG12796,CG1279 6,CG16725,CG5201,C G7422,CG7462,CG787 3, |
| Lysophosphatidylcholines | LPAR2, | 9170, | CG12796, |
| Lysophosphatidylglycerol | NFKB1, | 4790, | CG11992, |
| lysyl-arginyl-alanyl-lysyl-alanyl-lysyl-threonyl-threonyllysyl-lysyl-arginine | NFKB1,KHDRBS1,PAR D6A, | 4790,10657,50855, | CG11992,CG3613,CG5 821,CG5884, |
| M&B22948 | LPAR2, | 9170, | CG12796, |
| Malix | CYP3A4, | 1576, | CG2060, |
| manidipine | CYP3A4, | 1576, | CG2060, |
| manumycin | NFKB1, | 4790, | CG11992, |
| maraviroc | CYP3A4, | 1576, | CG2060, |
| Matrine | UBE2L3, | 7332, | CG5788, |
| MCYST-LR | PPP2CA, | 5515, | CG7109, |
| Me-nle-asp-phe-NH2 | CCKBR, | 887, | CG6881, |
| mead ethanolamide | CNR1, | 1268, | CG12796, |
| MeAsO(OH)2 | GSTO1, | 9446, | CG6673, |
| Mebumal | SLC2A1, | 6513, | CG1086, |
| Mechlorethamine | NFKB1,CCNB1,POLD1 ,CDC2, | 4790,891,5424,98 3, | CG11992,CG5940,CG5 949,CG8203, |
| Medroxyprogesterone 17-Acetate | CYP3A4,DLL4,SERPIN C1,PSMD9, | 1576,54567,462,5 715, | CG2060,CG3619,CG81 37,CG9588, |
| Mefenamic Acid | UGT1A9, | 54600, | CG4772, |
| Megalomicin | CD36, | 948, | CG7422, |
| Melarsoprol | NFKB1, | 4790, | CG11992, |

(continued)

| Small Molecule | Interacting Gene Symbol | Interacting GeneID | Drosophila Id |
|---|---|---|---|
| Melatol | CYP4F3,CYP3A5,GSR,ARR3, | 4051,1577,2936,407, | CG2060,CG2060,CG2151,CG5711, |
| meletin | NFKB1,CYP3A4,CYP3 A5,UGT1A6,UGT1A3, UGT1A4,CCNB1,POLD 1,CDC2,CDK2,PSMD9, | 4790,1576,1577,5 4578,54659,54657 , 891,5424,983,101 7,5715, | CG11992,CG2060,CG2 060,CG4772,CG4772, CG4772,CG5940,CG59 49,CG8203,CG8203,C G9588, |
| melitten | NFKB1, | 4790, | CG11992, |
| meloxicam | NFKB1, | 4790, | CG11992, |
| Melphalan | CCNB1,PAFAH1B1, | 891,5048, | CG5940,CG8440, |
| Memantine | CYP3A4, | 1576, | CG2060, |
| menadiol | UGT1A4,UGT1A3,UGT 1A6, | 54657,54659,5457 8, | CG4772,CG4772,CG47 72, |
| Menhaden oil | CYP4F3,CYP3A5, | 4051,1577, | CG2060,CG2060, |
| menthofuran | CYP3A4, | 1576, | CG2060, |
| Meperidine | CYP3A4, | 1576, | CG2060, |
| Mephenytoin | CYP3A4,CYP4F3,CYP3 A5, | 1576,4051,1577, | CG2060,CG2060,CG20 60, |
| mesalamine | NFKB1, | 4790, | CG11992, |
| Mesaton | NFKB1,CCNA2, | 4790,890, | CG11992,CG5940, |
| Meth | NFKB1,CYP3A4, | 4790,1576, | CG11992,CG2060, |
| methanandamide | CNR1,CADPS, | 1268,8618, | CG12796,CG18026, |
| methanethiosulfonate ethylammonium | TRPA1, | 8989, | CG5751, |
| Methimazole | FMO3, | 2328, | CG3006, |
| methionyl-leucylphenylalanine | LPAR2,TRPV2, | 9170,51393, | CG12796,CG5842, |
| Methorphan | CYP4F3,CYP3A4,CYP3 A5, | 4051,1576,1577, | CG2060,CG2060,CG20 60, |
| Methoxsalen | CYP3A4, | 1576, | CG2060, |
| Methoxy-psoralen | PRPF19, | 27339, | CG5519, |
| methoxyamine | SERPINC1, | 462, | CG8137, |
| methoxychlor | CYP3A4, | 1576, | CG2060, |

(continued)

| Small Molecule | Interacting Gene Symbol | Interacting GeneID | Drosophila Id |
|---|---|---|---|
| methoxymorphinan | CYP3A4, | 1576, | CG2060, |
| methyl chloroformate | TYMS, | 7298, | CG3181, |
| Methyl glycine | CYP4F3,CYP3A5, | 4051,1577, | CG2060,CG2060, |
| Methyl paraben | TRPA1, | 8989, | CG5751, |
| methyl salicylate | UGT1A6, | 54578, | CG4772, |
| methyl tryptophan | FOXP3, | 50943, | CG16899, |
| methyl-dopa | UGT1A6, | 54578, | CG4772, |
| methyl-phosphorothioate | TYMS, | 7298, | CG3181, |
| methyl-Pyridinium | CCKBR, | 887, | CG6881, |
| methylamine | SLC2A4,CYP4F3,CYP3 A5, | 6517,4051,1577, | CG1086,CG2060,CG20 60, |
| Methylamylnitrosamine | CYP4F3,CYP3A5, | 4051,1577, | CG2060,CG2060, |
| Methylene-tetrahydrofolate | TYMS, | 7298, | CG3181, |
| methylenetetrahydrofolates | TYMS, | 7298, | CG3181, |
| methylglyoxal | NFKB1,HAGH,TRPV6, | 4790,3029,55503, | CG11992,CG4365,CG5 842, |
| methylnaltrexone | S1PR3, | 1903, | CG12796, |
| methyloxidanyl | CYP3A4, | 1576, | CG2060, |
| methylparaben | TRPA1, | 8989, | CG5751, |
| methylphosphate | CDK9, | 1025, | CG5179, |
| Methylprednisolone | NFKB1,CYP3A4,SERPI NC1, | 4790,1576,462, | CG11992,CG2060,CG8 137, |
| methylxanthines | PDE1B, | 5153, | CG14940, |
| Metoclopramide | NFKB1, | 4790, | CG11992, |
| Metopiron | CYP4F3,CYP3A4,CYP3 A5, | 4051,1576,1577, | CG2060,CG2060,CG20 60, |
| Metribolone | NFKB1,OPN4,CCNA1, PSIP1, | 4790,94233,8900, 11168, | CG11992,CG4550,CG5 940,CG7946, |
| mevalonic acid | NFKB1, | 4790, | CG11992, |

(continued)

| Small Molecule | Interacting Gene Symbol | Interacting GeneID | Drosophila Id |
|---|---|---|---|
| micafungin | CYP3A4, | 1576, | CG2060, |
| miconazole | CYP4F3,CYP3A4,CYP3 A5, | 4051,1576,1577, | CG2060,CG2060,CG20 60, |
| Mictonorm | CYP3A4, | 1576, | CG2060, |
| Midazolam | PLXNB1,CYP4F3,CYP3 A5,CYP3A4, | 5364,4051,1577,1 576, | CG11081,CG2060,CG2 060,CG2060, |
| Mifepristone | CYP3A4,PRDX6,UBE2 L3,CCNA2,CDK2, | 1576,9588,7332,8 90,1017, | CG2060,CG3083,CG57 88,CG5940,CG8203, |
| MIII | FGFR1, | 2260, | CG7223, |
| Milrinone | PDE4A, | 5141, | CG14940, |
| Mimosine | CCNA2, | 890, | CG5940, |
| mirtazapine | CYP3A4, | 1576, | CG2060, |
| Mit-C | NFKB1, | 4790, | CG11992, |
| mithramycin | NDUFV2, | 4729, | CG5703, |
| MitoTracker-Red | TFAM, | 7019, | CG4217, |
| Mitoxantrone | UBE2L3,PAFAH1B1, | 7332,5048, | CG5788,CG8440, |
| mizolastine | CYP3A4, | 1576, | CG2060, |
| MLN8054 | AURKA, | 6790, | CG6620, |
| mofarotene | PSMD9, | 5715, | CG9588, |
| Monensin | CCNA2,CCNB1,POLD1 , CDC2,CDK2,PSMD9, | 890,891,5424,983, 1017,5715, | CG5940,CG5940,CG59 49,CG8203,CG8203,C G9588, |
| mono-N-demethyladinazolam | CYP3A4, | 1576, | CG2060, |
| mono(2-ethylhexyl) phthalate | CELSR2, | 1952, | CG11895, |
| monoethylglycinexylidide | CYP3A4, | 1576, | CG2060, |
| monomethylarsonic acid | SLC2A1, | 6513, | CG1086, |
| monoterpenes | NFKB1, | 4790, | CG11992, |
| monuron | TRPV1, | 7442, | CG5842, |

(continued)

| Small Molecule | Interacting Gene Symbol | Interacting GeneID | Drosophila Id |
|---|---|---|---|
| MORIN | SMAD7,CD36, | 4092,948, | CG5201,CG7422, |
| morpholine | ME1, | 4199, | CG5889, |
| morusin | NFKB1, | 4790, | CG11992, |
| motexafin gadolinium | CYP3A4,TXNRD1, | 1576,7296, | CG2060,CG2151, |
| Motuporin | PPP2CA, | 5515, | CG7109, |
| moxifloxacin | NFKB1, | 4790, | CG11992, |
| MPEG | CYP3A4, | 1576, | CG2060, |
| Muraglitazar | UGT1A4,UGT1A3, | 54657,54659, | CG4772,CG4772, |
| mutalipocin II | MLL, | 4297, | CG8651, |
| mycophenolic acid | UGT2B7,UGT1A9,UGT 1A8,UGT1A4,UGT1A3 ,UGT1A7, | 7364,54600,54576 , 54657,54659,545 77, | CG4772,CG4772,CG47 72,CG4772,CG4772,C G4772, |
| Mycose | LPAR2, | 9170, | CG12796, |
| Myocol | PSMD12, | 5718, | CG1100, |
| myricetin | NFKB1,CD36, | 4790,948, | CG11992,CG7422, |
| myxothiazol | TXNRD2, | 10587, | CG2151, |
| N-(2-cyclohexyloxy-4-nitrophenyl)methanesulfonami de | NFKB1,UBE2L3, | 4790,7332, | CG11992,CG5788, |
| N-(2-hydroxypropyl)methacrylamide | TXNRD2,TXNRD1, | 10587,7296, | CG2151,CG2151, |
| N-(3-(4-chlorophenyl)-2-(3-cyanophenyl)-1-methylpropyl)-2-methyl-2-((5-(trifluoromethyl)pyridin-2-yl)oxy)propanamide | CNR1, | 1268, | CG12796, |
| N-(3-methoxyphenyl)-4-chlorocinnamanilide | TRPV1, | 7442, | CG5842, |
| N-(3-oxododecanoyl)homoserine lactone | NFKB1, | 4790, | CG11992, |
| N-(4-(6-(4-(1-(4-fluorophenyl)ethyl)piperazin-1-yl) pyrimidin-4-yloxy)benzo(d)thiazol-2-yl)acetamide | TRPV1, | 7442, | CG5842, |
| N-(4-(6-(4-trifluoromethylphenyl)pyrimidi n-4-yloxy) benzothiazol-2-yl)acetamide | TRPV1, | 7442, | CG5842, |
| N-(4-cyano-benzo(b)thiophene-2-carbonyl)guanidine | CYP3A4,CYP3A7, | 1576,1551, | CG2060,CG2060, |

(continued)

| Small Molecule | Interacting Gene Symbol | Interacting GeneID | Drosophila Id |
|---|---|---|---|
| N-(5-(((5-(1,1-dimethylethyl)-2-oxazolyl)methyl)thio)-2-thiazolyl)-4-piperidinecarboxamide | UBE2L3, | 7332, | CG5788, |
| N-acetylcysteine lysinate | NFKB1, | 4790, | CG11992, |
| n-acetylmuramyl-l-alanyl-d-isoglutamine | NFKB1, | 4790, | CG11992, |
| N-acetylneuraminic acid | PGC,CNR2,KHDRBS1, SERPINC1, | 5225,1269,10657, 462, | CG10872,CG12796,CG 3613,CG5821,CG8137 |
| N-desmethylclobazam | CYP3A4, | 1576, | CG2060, |
| N-ethylmaleimide | KCNQ2,HNRNPA1, | 3785,3178, | CG12915,CG9983, |
| N-methyl-N-(trimethylsilyl)trifluoroacetami de | TYMS, | 7298, | CG3181, |
| N-methylsulfonyl-6-(2-propargyloxyphenyl)hexanami de | CYP3A5,CYP4F3, | 1577,4051, | CG2060,CG2060, |
| N-oleoyldopamine | TRPV1, | 7442, | CG5842, |
| N-phenyl-1-naphthylamine | SLC5A7, | 60482, | CG7708, |
| N,N,N',N'-tetramethylethylenediamine | ME1, | 4199, | CG5889, |
| N(6)-cyclohexyl-2-O-methyladenosine | ADORA1, | 134, | CG9753, |
| N(6)-cyclopentyladenosine | ADORA1, | 134, | CG9753, |
| N3-IQ | CYP3A5,CYP4F3, | 1577,4051, | CG2060,CG2060, |
| Nadroparin | SERPINC1, | 462, | CG8137, |
| naftifine | CYP4F3,CYP3A5, | 4051,1577, | CG2060,CG2060, |
| nal-NH2 | CCKBR, | 887, | CG6881, |
| NALS | SMN1, | 6606, | CG16725, |
| nanchangmycin | NDUFAB1, | 4706, | CG9160, |
| Naproxen | UGT2B7,UGT1A1, | 7364,54658, | CG4772,CG4772, |
| naratriptan | SFRS1, | 6426, | CG6987, |
| narbonolide | NDUFAB1, | 4706, | CG9160, |
| NARIGENIN | SLC2A4,CYP3A4,UGT 1A3, | 6517,1576,54659, | CG1086,CG2060,CG47 72, |
| Narkotil | STX8, | 9482, | CG4109, |

(continued)

| Small Molecule | Interacting Gene Symbol | Interacting GeneID | Drosophila Id |
|---|---|---|---|
| Nasol | TYMS, | 7298, | CG3181, |
| natalizumab | FOXP3, | 50943, | CG16899, |
| nateglinide | CYP3A4, | 1576, | CG2060, |
| Naxy | NFKB1,CYP3A4,CYP3 A5, | 4790,1576,1577, | CG11992,CG2060,CG2 060, |
| nebivolol | NFKB1,CDK2, | 4790,1017, | CG11992,CG8203, |
| Nefazodone | CYP4F3,CYP3A4,CYP3 A5, | 4051,1576,1577, | CG2060,CG2060,CG20 60, |
| nefiracetam | FGFR3, | 2261, | CG7223, |
| Nelfinavir | CYP4F3,CYP3A4,CYP3 A5, | 4051,1576,1577, | CG2060,CG2060,CG20 60, |
| Neomycin | SERPINB1, | 1992, | CG8137,CG9453,CG94 60, |
| Neopterin | NFKB1, | 4790, | CG11992, |
| Neostigmine | FGFR3, | 2261, | CG7223, |
| Neut | SLC25A21,UBE2L3, | 89874,7332, | CG5254,CG5788, |
| Nevirapine | CYP3A4, | 1576, | CG2060, |
| NFBA | CYP3A4, | 1576, | CG2060, |
| Nialk | ECD, | 11319, | CG5714, |
| Nicardipine | CYP3A5,CYP3A4,DLL1 ,CD36, | 1577,1576,28514, 948, | CG2060,CG2060,CG36 19,CG7422, |
| Niflumic Acid | UGT1A9, | 54600, | CG4772, |
| nimesulide | SERPINB3, | 6317, | CG8137,CG9453,CG94 60, |
| niobium | ME1, | 4199, | CG5889, |
| nitecapone | NFKB1, | 4790, | CG11992, |
| nitroanilide | SERPINC1, | 462, | CG8137, |
| nitroaspirin | NFKB1, | 4790, | CG11992, |
| N itrofu ra ns | GSR, DLD, | 2936,1738, | CG2151,CG7430, |
| NITROPYRENE | CYP3A4,CYP4F3,CYP3 A5, | 1576,4051,1577, | CG2060,CG2060,CG20 60, |

(continued)

| Small Molecule | Interacting Gene Symbol | Interacting GeneID | Drosophila Id |
|---|---|---|---|
| nitrosamines | CYP3A5,CYP4F3,UGT 1A7, | 1577,4051,54577, | CG2060,CG2060,CG47 72, |
| Nitrosoanabasine | CYP3A4, | 1576, | CG2060, |
| Nitrosocysteine | NFKB1,LPAR2, | 4790,9170, | CG11992,CG12796, |
| nitrosulindac | NFKB1, | 4790, | CG11992, |
| Nizatidine | FGFR3, | 2261, | CG7223, |
| NK 104 | NFKB1,CD36, | 4790,948, | CG11992,CG7422, |
| NK314 | CDC2, | 983, | CG8203, |
| NMDA | DLG1,FYN, | 1739,2534, | CG1725,CG7873, |
| NN 703 | CYP3A4, | 1576, | CG2060, |
| Noan | SLC2A1,PDE4A,CYP3 A4, | 6513,5141,1576, | CG1086,CG14940,CG2 060, |
| Nobiletin | NFKB1,CYP3A4,GSR,U GT1A4,UGT1A1,UGT1 A3,CD36, | 4790,1576,2936,5 4657,54658,54659 , 948, | CG11992,CG2060,CG2 151,CG4772,CG4772, CG4772,CG7422, |
| NOC 18 | CYP3A4, | 1576, | CG2060, |
| Nocodazole | NFKB1,TAOK2,CCNB1 , BICD2,COX7A2L, | 4790,9344,891,23 299,9167, | CG11992,CG14217,CG 5940,CG6605,CG9603 , |
| nodularin | PPP2CA, | 5515, | CG7109, |
| Nodularin v | PPP2CA, | 5515, | CG7109, |
| nolatrexed | TYMS, | 7298, | CG3181, |
| Nonoxynol | NFKB1, | 4790, | CG11992, |
| noralfentanil | CYP3A4, | 1576, | CG2060, |
| norbuprenorphine | UGT1A3, | 54659, | CG4772, |
| Norclozapine | CYP3A4, | 1576, | CG2060, |
| Nordihydroguaiaretic Acid | NFKB1, | 4790, | CG11992, |
| Norethindrone | CYP3A4,TYMS, | 1576,7298, | CG2060,CG3181, |
| noreximide | CDC2, | 983, | CG8203, |

(continued)

| Small Molecule | Interacting Gene Symbol | Interacting GeneID | Drosophila Id |
|---|---|---|---|
| norfluoxetine | CYP4F3,CYP3A5,CYP3 A4, | 4051,1577,1576, | CG2060,CG2060,CG20 60, |
| Norgestrel | TYMS, | 7298, | CG3181, |
| norharman | CYP3A4, | 1576, | CG2060, |
| norketobemidone | CYP3A4, | 1576, | CG2060, |
| norlaudanosoline | NFKB1, | 4790, | CG11992, |
| normeperidine | CYP3A4, | 1576, | CG2060, |
| Nortilidine | CYP3A4, | 1576, | CG2060, |
| norverapamil | CYP3A4,CYP3A5, | 1576,1577, | CG2060,CG2060, |
| novobiocin | GPR177, | 79971, | CG6210, |
| NS-187 | YES1, | 7525, | CG7873, |
| NSC 23766 | NFKB1,ACTR2, | 4790,10097, | CG11992,CG9901, |
| NSC 366140 | CYP3A4,CYP4F3,CYP3 A5, | 1576,4051,1577, | CG2060,CG2060,CG20 60, |
| NSC 663284 | CDC2, | 983, | CG8203, |
| NSC-134754 | SLC2A1, | 6513, | CG1086, |
| NU2058 | CDK2, | 1017, | CG8203, |
| number-one | SMN1, | 6606, | CG16725, |
| nutlin 3 | NFKB1,MRPL41,UBE2 L3, | 4790,64975,7332, | CG11992,CG12954,CG 5788, |
| NVP-AEW541 | PSMD9, | 5715, | CG9588, |
| Nylon | CADPS, | 8618, | CG18026, |
| O-(chloroacetylcarbamoyl)fumag illol | CYP4V2,CCNA2,POLD 1,CDC2,CDK2, | 285440,890,5424, 983,1017, | CG2060,CG5940,CG59 49,CG8203,CG8203, |
| O-desethylreboxetine | CYP3A4, | 1576, | CG2060, |
| O-Due | LRRN2,CYP3A4,CYP4F 3,CYP3A5, | 10446,1576,4051, 1577, | CG11280,CG2060,CG2 060,CG2060, |
| o-quinone | TXNRD2,TXNRD1,DL D, | 10587,7296,1738, | CG2151,CG2151,CG74 30, |
| obovatol | NFKB1, | 4790, | CG11992, |

(continued)

| Small Molecule | Interacting Gene Symbol | Interacting GeneID | Drosophila Id |
|---|---|---|---|
| OCDD | CYP3A7,CYP3A5, | 1551,1577, | CG2060,CG2060, |
| octanediol | ME1, | 4199, | CG5889, |
| Octoxynol | UGT1A6,UGT1A1,UGT 1A3,UGT2B4,UGT2B1 5,UGT2B7,UGT1A4, | 54578,54658,5465 9,7363,7366,7364, 54657, | CG4772,CG4772,CG47 72,CG4772,CG4772,C G4772,CG4772, |
| Octreotide | PLXNA2,CD36,PSMD9 , | 5362,948,5715, | CG11081,CG7422,CG9 588, |
| Okadaic Acid | SLC2A1,SLC2A4,CTSB , NFKB1,TXNRD2,POL D1,AURKB, CDC2,ACS L3, | 6513,6517,1508,4 790,10587,5424,9 212,983,2181, | CG1086,CG1086,CG10 992,CG11992,CG2151 , CG5949,CG6620,CG8 203,CG8732, |
| olanzapine | CYP4F3,CYP3A5,FMO 3,UGT1A4, | 4051,1577,2328,5 4657, | CG2060,CG2060,CG30 06,CG4772, |
| olefins | ME1, | 4199, | CG5889, |
| oleoylethanolamide | CNR1, | 1268, | CG12796, |
| olmelin | UGT1A3, UGT1A4, | 54659,54657, | CG4772,CG4772, |
| olmesartan | NFKB1, | 4790, | CG11992, |
| olomoucine | POLD1,CDK2,CDC2, | 5424,1017,983, | CG5949,CG8203,CG82 03, |
| olomoucine II | CDK9, | 1025, | CG5179, |
| Oltipraz | NFKB1,CYP3A4,UGT1 A4,UGT1A6,UGT1A3, UGT1A1, | 4790,1576,54657, 54578,54659,5465 8, | CG11992,CG2060,CG4 772,CG4772,CG4772, CG4772, |
| omalizumab | CYP3A4, | 1576, | CG2060, |
| omega-agatoxin | FGFR3, | 2261, | CG7223, |
| omega-Conotoxin GVIA | FGFR3, | 2261, | CG7223, |
| omega-N-Methylarginine | FGFR3, | 2261, | CG7223, |
| Omeprazole | PGC,CYP4F3,CYP3A4, CYP3A5, | 5225,4051,1576,1 577, | CG10872,CG2060,CG2 060,CG2060, |
| omeprazole sulfone | CYP3A4, | 1576, | CG2060, |
| onapristone | CYP3A4, | 1576, | CG2060, |

(continued)

| Small Molecule | Interacting Gene Symbol | Interacting GeneID | Drosophila Id |
|---|---|---|---|
| ONCB | PPP3R1, | 5534, | CG14353, |
| Ondansetron | CYP3A4, | 1576, | CG2060, |
| ONO4819 | FGFR3, | 2261, | CG7223, |
| Optef | RFC1,NFKB1,DNAH5, CYP3A5,CYP3A7,CYP3 A4,DLL1,UGT1A1,ME 1,SFRS1,SERPINC1, | 5981,4790,1767,1 577,1551,1576,28 514,54658,4199,6 426,462, | CG1119,CG11992,CG1 7150,CG2060,CG2060 , CG2060,CG3619,CG4 772,CG5889,CG6987, CG8137, |
| OR 1246 | NFKB1, | 4790, | CG11992, |
| oroxylin A | POLD1,CDC2, | 5424,983, | CG5949,CG8203, |
| Orphenadrine | CYP3A4, | 1576, | CG2060, |
| Osten | CYP3A4,FGFR1, | 1576,2260, | CG2060,CG7223, |
| osteum | SERPINC1, | 462, | CG8137, |
| OSU 03012 | CCNA2,CDK2, | 890,1017, | CG5940,CG8203, |
| Ouabain | LRRN2,NFKB1,ATP4B, | 10446,4790,496, | CG11280,CG11992,CG 8663, |
| OVEX | CYP3A7,UGT1A3, | 1551,54659, | CG2060,CG4772, |
| Ovex | CYP3A5,CYP3A4,UGT 1A1,SERPINC1, | 1577,1576,54658, 462, | CG2060,CG2060,CG47 72,CG8137, |
| oxaliplatin | NFKB1,TYMS,SLC25A 21,POLD1,CDC2,CDK 2, | 4790,7298,89874, 5424,983,1017, | CG11992,CG3181,CG5 254,CG5949,CG8203, CG8203, |
| Oxarol | PLXNA2,PSMD9, | 5362,5715, | CG11081,CG9588, |
| oxaspirodion | NFKB1, | 4790, | CG11992, |
| oxatomide | CYP3A4, | 1576, | CG2060, |
| Oxazepam | UGT2B7,UGT2B15, | 7364,7366, | CG4772,CG4772, |
| oxcarbazepine | CYP3A5,CYP3A4, | 1577,1576, | CG2060,CG2060, |
| Oxotremorine | FGFR3, | 2261, | CG7223, |
| oxotremorine M | FGFR3, | 2261, | CG7223, |
| Oxymorphone | CYP3A4, | 1576, | CG2060, |

(continued)

| Small Molecule | Interacting Gene Symbol | Interacting GeneID | Drosophila Id |
|---|---|---|---|
| Oxyntomodulin | CCKAR, | 886, | CG6881, |
| Oxytrol | CYP3A5,CYP4F3,CYP3 A4, | 1577,4051,1576, | CG2060,CG2060,CG20 60, |
| p-ABA | SERPINC1, | 462, | CG8137, |
| p-XSC | NFKB1, | 4790, | CG11992, |
| p-Xylol | CELSR1,ME2, | 9620,4200, | CG11895,CG5889, |
| Paclitaxel | NFKB1,TAOK2,CYP4F 3,CYP3A4,CYP3A5,TX NRD2,TXNRD1,TYMS, UBE2L3,CCNB1,POLD 1,AURKB, CDC2,CDK2,CDKS,PSMD9,COX7A 2L, | 4790,9344,4051,1 576,1577,10587,7 296,7298,7332,89 1,5424,9212,983,1 017,1020,5715,9167, | CG11992,CG14217,CG 2060,CG2060,CG2060 , CG2151,CG2151,CG3 181,CG5788,CG5940, CG5949,CG6620,CG8203,CG8203,CG 8203,C G9588,CG9603, |
| paeonol | NFKB1, | 4790, | CG11992, |
| palladium | CELSR1,DNAH5,ME2, | 9620,1767,4200, | CG11895,CG17150,CG 5889, |
| palmitoleate | ME2,ME1,ME3, | 4200,4199,10873, | CG5889,CG5889,CG58 89, |
| PALMITOYL | UGT1A4,UGT1A3, | 54657,54659, | CG4772,CG4772, |
| Palmitoylcarnitine | PANK2, | 80025, | CG5725, |
| pamidronate | DDOST, | 1650, | CG9022, |
| panaxadiol | CDK2, | 1017, | CG8203, |
| panepoxydone | NFKB1, | 4790, | CG11992, |
| pantoprazole | CYP3A4, | 1576, | CG2060, |
| Papaverine | PDE4A,FGFR3, | 5141,2261, | CG14940,CG7223, |
| Papite | NFKB1,TRPA1, | 4790,8989, | CG11992,CG5751, |
| PAPP | HDAC3, | 8841, | CG2128, |
| parecoxib | CYP3A4, | 1576, | CG2060, |
| Paroxetine | CYP4F3,CYP3A5, | 4051,1577, | CG2060,CG2060, |
| Parsal | PSMD12,NFKB1, | 5718,4790, | CG1100,CG11992, |
| Parthenolide | NFKB1, | 4790, | CG11992, |

(continued)

| Small Molecule | Interacting Gene Symbol | Interacting GeneID | Drosophila Id |
|---|---|---|---|
| PC 314 | NPFFR2, | 10886, | CG10823, |
| PCA 4230 | UBE2L3,CCNA2, | 7332,890, | CG5788,CG5940, |
| PCSO | CNR2, | 1269, | CG12796, |
| PD 134308 | CCKBR, | 887, | CG6881, |
| PD 144795 | NFKB1, | 4790, | CG11992, |
| PD 180988 | CYP3A4, | 1576, | CG2060, |
| PD 98059 | SLC2A5,SLC2A4,NFK B1,SMN1,CYP3A4,UG T1A1,CDK2, | 6518,6517,4790,6 606,1576,54658,1 017, | CG1086,CG1086,CG11 992,CG16725,CG2060 , CG4772,CG8203, |
| pectin | ACAT1, | 38, | CG10932, |
| Pemetrexed | TBXAS1,TYMS,SLC46 A1, | 6916,7298,113235 , | CG2060,CG3181,CG80 08, |
| Penicillins | VDAC1, | 7416, | CG17137, |
| Penite | PSMD12,NFKB1, | 5718,4790, | CG1100,CG11992, |
| Pentagastrin | CCKAR,CCKBR, | 886,887, | CG6881,CG6881, |
| Pentoxifylline | NFKB1,CCNB1, | 4790,891, | CG11992,CG5940, |
| Peplomycin | NFKB1, | 4790, | CG11992, |
| peppermint oil | CYP3A4, | 1576, | CG2060, |
| Pepstatin A | CYP3A4, | 1576, | CG2060, |
| Perazine | CYP3A4, | 1576, | CG2060, |
| Pergolide | NFKB1, | 4790, | CG11992, |
| Perillol | NFKB1, | 4790, | CG11992, |
| Perilymph | SERPINB3, | 6317, | CG8137,CG9453,CG94 60, |
| periodate | CLTA, | 1211, | CG6948, |
| perospirone | CYP3A4, | 1576, | CG2060, |
| perovskite | TXNRD1,TXNRD2, | 7296,10587, | CG2151,CG2151, |
| PFPA | ECD, | 11319, | CG5714, |

(continued)

| Small Molecule | Interacting Gene Symbol | Interacting GeneID | Drosophila Id |
|---|---|---|---|
| Phebestin | FOXP3, | 50943, | CG16899, |
| phen | NFKB1, | 4790, | CG11992, |
| phenolate | ME1, | 4199, | CG5889, |
| Phenols | UGT1A3,UGT1A4, | 54659,54657, | CG4772,CG4772, |
| phenoxodiol | CDK2, | 1017, | CG8203, |
| Phenprocoumon | CYP3A4, | 1576, | CG2060, |
| Phentermine | CYP4F3,CYP3A5, | 4051,1577, | CG2060,CG2060, |
| phenyl-propionamide | CYP3A4, | 1576, | CG2060, |
| phenyl-Pyridinium | MPP1, | 4354, | CG6703, |
| Phenytoin | CYP4F3,CYP3A4,CYP3 A5,UGT1A6,UGT1A1, | 4051,1576,1577,5 4578,54658, | CG2060,CG2060,CG20 60,CG4772,CG4772, |
| pheophorbide a | CCNA1,POLD1,CDC2, | 8900,5424,983, | CG5940,CG5949,CG82 03, |
| phloretin | SLC2A1,SLC2A2, | 6513,6514, | CG1086,CG1086, |
| PHOB | CYP4B1,CYP4F3,CYP3 A4,CYP4X1,CYP3A5,G STT1,UGT1A1,UGT1A 6,FGFR3, | 1580,4051,1576,2 60293,1577,2952, 54658,54578,2261 , | CG2060,CG2060,CG20 60,CG2060,CG2060,C G30005,CG4772,CG47 72,CG7223, |
| phorate | CYP3A4, FMO1, | 1576,2326, | CG2060,CG3006, |
| phorbol | CADPS,TRPV1,TRPV4, CD36, | 8618,7442,59341, 948, | CG18026,CG5842,CG5 842,CG7422, |
| phorbol 12-phenylacetate 13-acetate 20-homovanillate | TRPV1, | 7442, | CG5842, |
| phosphatidylethanolamines | GABARAP,GABARAPL 2,GABARAPL1,SERPIN C1, | 11337,11345,2371 0,462, | CG12334,CG12334,CG 12334,CG8137, |
| Phosphatidylinositol 4,5-Diphosphate | KCNQ2,KHDRBS2,KH DRBS3,TRPV6, RASA2, PSIP1, | 3785,202559,1065 6,55503,5922,111 68, | CG12915,CG3613,CG5 821,CG3613,CG5821, CG5842,CG6721,CG79 46, |
| phosphatidylinositol phosphate, PtdIns(4,5)P2 | RASA2, | 5922, | CG6721, |
| phytanic acid | UGT1A4,UGT1A3, | 54657,54659, | CG4772,CG4772, |

(continued)

| Small Molecule | Interacting Gene Symbol | Interacting GeneID | Drosophila Id |
|---|---|---|---|
| Picibanil | TAOK2, | 9344, | CG14217, |
| picric acid | TRPA1, | 8989, | CG5751, |
| pifithrin | NFKB1,CCNB1, | 4790,891, | CG11992,CG5940, |
| Pilot | MLL, | 4297, | CG8651, |
| pimecrolimus | NFKB1, | 4790, | CG11992, |
| pioglitazone | SLC2A5,ACAT1,NFKB 1,CYP3A5,CYP3A4,CD 36,SERPINC1,PSMD9, | 6518,38,4790,157 7,1576,948,462,57 15, | CG1086,CG10932,CG1 1992,CG2060,CG2060 , CG7422,CG8137,CG9 588, |
| pipecoloxylidide | CYP3A4, | 1576, | CG2060, |
| piperidine | SMN1, | 6606, | CG16725, |
| piperine | CYP3A4,UGT1A3,UGT 1A4,TRPV1, | 1576,54659,54657 , 7442, | CG2060,CG4772,CG47 72,CG5842, |
| Pira | LYZ,CYP3A4, | 4069,1576, | CG1180,CG2060, |
| pirinixic acid | CYP4A11,CYP4X1, | 1579,260293, | CG2060,CG2060, |
| Piroxicam | SERPINB3, | 6317, | CG8137,CG9453,CG94 60, |
| PKC412 | POLD1,CDC2, | 5424,983, | CG5949,CG8203, |
| plumbagin | NFKB1,POLD1,CDC2, | 4790,5424,983, | CG11992,CG5949,CG8 203, |
| Pluronic p 85 | SLC2A1, | 6513, | CG1086, |
| PMDT | ME1, | 4199, | CG5889, |
| PMPA | FMO3, | 2328, | CG3006, |
| PMSF | PSIP1, | 11168, | CG7946, |
| PNPP | PPEF1, | 5475, | CG6571, |
| Podophyllotoxin | CYP3A4,PAFAH1B1, | 1576,5048, | CG2060,CG8440, |
| polidocanol | UGT1A3,UGT1A4, | 54659,54657, | CG4772,CG4772, |
| poly-gamma-glutamate | TYMS, | 7298, | CG3181, |
| ponicidin | NFKB1, | 4790, | CG11992, |

(continued)

| Small Molecule | Interacting Gene Symbol | Interacting GeneID | Drosophila Id |
|---|---|---|---|
| poractant alfa | CD36, | 948, | CG7422, |
| posaconazole | CYP3A4, | 1576, | CG2060, |
| potassium tellurate(IV) | UGT8, | 7368, | CG4772, |
| PQQ Cofactor | TXNRD1, | 7296, | CG2151, |
| pranlukast | GPR177, | 79971, | CG6210, |
| Pravastatin | NFKB1,CYP3A5,CYP4F 3,CYP3A4,CD36, | 4790,1577,4051,1 576,948, | CG11992,CG2060,CG2 060,CG2060,CG7422, |
| Prazosin | CCNA2, | 890, | CG5940, |
| PRDL | NFKB1,CYP3A5,CYP3 A4,DPAGT1, | 4790,1577,1576,1 798, | CG11992,CG2060,CG2 060,CG5287, |
| Precursor mrna | DHX16, | 8449, | CG10689,CG1375, |
| Prednisone | SERPINC1, | 462, | CG8137, |
| pregnane | CYP3A4,CYP3A7, | 1576,1551, | CG2060,CG2060, |
| Pregnanes | UGT2B7,UGT2B11, | 7364,10720, | CG4772,CG4772, |
| Pregnanolone | CYP3A4, | 1576, | CG2060, |
| pregnenolone 16alpha-carbonitrile | CYP3A4, | 1576, | CG2060, |
| Pregnyl | NFKB1,NDUFA6, | 4790,4700, | CG11992,CG7712, |
| preussin | CDK2, | 1017, | CG8203, |
| Primidone | CYP3A4, | 1576, | CG2060, |
| Proadifen | CYP4F3,CYP3A7,CYP3 A5, | 4051,1551,1577, | CG2060,CG2060,CG20 60, |
| Proanthocyanidins | NFKB1, | 4790, | CG11992, |
| Probenecid | TRPV2,GPR177, | 51393,79971, | CG5842,CG6210, |
| Probucol | NFKB1,FGR, | 4790,2268, | CG11992,CG7873, |
| Procasil | PSMD9, | 5715, | CG9588, |
| Procetofen | CYP3A4,UBE2L3, | 1576,7332, | CG2060,CG5788, |
| procyanidin B2 | NFKB1, | 4790, | CG11992, |

(continued)

| Small Molecule | Interacting Gene Symbol | Interacting GeneID | Drosophila Id |
|---|---|---|---|
| Prodix | CYP3A4, | 1576, | CG2060, |
| prolactin, polymeric | ECD, | 11319, | CG5714, |
| Propafenone | CYP3A4, | 1576, | CG2060, |
| Propanesulfonate | UGT1A4,UGT1A3, | 54657,54659, | CG4772,CG4772, |
| Propofol | SLC2A1,PLXNB1,CYP3 A4,UGT1A1,UGT1A9, | 6513,5364,1576,5 4658,54600, | CG1086,CG11081,CG2 060,CG4772,CG4772, |
| propyl pyrazole triol | UGT2B15, | 7366, | CG4772, |
| propyne | ME3, | 10873, | CG5889, |
| prostratin | NFKB1, | 4790, | CG11992, |
| protopanaxadiol | FGFR4, | 2264, | CG7223, |
| protopanaxatriol | FGFR4, | 2264, | CG7223, |
| PS 15 | CYP3A4, | 1576, | CG2060, |
| Pseudohypericin | CYP3A4, | 1576, | CG2060, |
| Pseudomonas-exotoxin | KDELR1,FGFR2, | 10945,2263, | CG5183,CG7223, |
| Psoralens | CYP3A4, | 1576, | CG2060, |
| psychosine-3'-sulfate ester | S1PR3, | 1903, | CG12796, |
| PTBP | UGT1A6, | 54578, | CG4772, |
| pteridine | PDE4A, | 5141, | CG14940, |
| Pterostilbene | PSMD9, | 5715, | CG9588, |
| PURAC | SLC2A4, | 6517, | CG1086, |
| Puromycin | KHDRBS1, | 10657, | CG3613,CG5821, |
| putrescine | SLC25A21,TRPV1, | 89874,7442, | CG5254,CG5842, |
| Pyocyanine | NFKB1, | 4790, | CG11992, |
| Pyra | KCNQ2,KCNQ3,SERPI NB3, | 3785,3786,6317, | CG12915,CG12915,CG 8137,CG9453,CG9460 , |
| pyranones | NFKB1, | 4790, | CG11992, |

(continued)

| Small Molecule | Interacting Gene Symbol | Interacting GeneID | Drosophila Id |
|---|---|---|---|
| pyrazole | FYN, | 2534, | CG7873, |
| Pyrethrins | CYP4F3,CYP3A4,CYP3 A5, | 4051,1576,1577, | CG2060,CG2060,CG20 60, |
| pyridazine | ADORA1, | 134, | CG9753, |
| Pyrimethamine | TBXAS1,TYMS,DHPS, | 6916,7298,1725, | CG2060,CG3181,CG80 05, |
| pyrimidin-2-one beta-ribofuranoside | POLD1,CDC2, | 5424,983, | CG5949,CG8203, |
| Pyro | CCNA2,CCNB1,CDK2, PSMD9, | 890,891,1017,571 5, | CG5940,CG5940,CG82 03,CG9588, |
| pyrogallol sulfonphthalein | NFKB1, | 4790, | CG11992, |
| pyrrole-2-carboxylic acid | PSMD9, | 5715, | CG9588, |
| pyrrolidine dithiocarbamic acid | NFKB1, | 4790, | CG11992, |
| pyrroloazepinone | CDK5, | 1020, | CG8203, |
| Qingkailing | NFKB1, | 4790, | CG11992, |
| quercitrin | CYP3A4, | 1576, | CG2060, |
| quetiapine | CYP3A4, | 1576, | CG2060, |
| Quicifal | CYP3A4, | 1576, | CG2060, |
| quinazoline | NFKB1, TYMS, | 4790,7298, | CG11992,CG3181, |
| Quinolinium | CYP3A4, | 1576, | CG2060, |
| Quinpirole | FGFR3, | 2261, | CG7223, |
| quinuclidin-3'-yl-1-phenyl-1,2,3,4-tetrahydroisoquinoline-2-carboxylate monosuccinate | CYP3A4,CYP4F3,CYP3 A5, | 1576,4051,1577, | CG2060,CG2060,CG20 60, |
| quinupristin-dalfopristin | CYP3A4, | 1576, | CG2060, |
| R-138727 | CYP3A4, | 1576, | CG2060, |
| R-99224 | ME1, | 4199, | CG5889, |
| Raloxifene | CYP3A4,SERPINC1, | 1576,462, | CG2060,CG8137, |
| ra Ititrexed | TBXAS1,TYMS,UBE2L 3, | 6916,7298,7332, | CG2060,CG3181,CG57 88, |
| Ramipril | DYNC1H1, | 1778, | CG17150, |

(continued)

| Small Molecule | Interacting Gene Symbol | Interacting GeneID | Drosophila Id |
|---|---|---|---|
| ramiprilat | NFKB1, | 4790, | CG11992, |
| RAMP | CYP4F3,CYP3A4,CYP3 A5,CYP3A7,UGT1A1,U GT1A6,UGT2A3,GPR1 77, | 4051,1576,1577,1 551,54658,54578, 79799,79971, | CG2060,CG2060,CG20 60,CG2060,CG4772,C G4772,CG4772,CG621 0, |
| Ranitidine | CYP4F3,CYP3A5,CYP3 A4,FGFR3, | 4051,1577,1576,2 261, | CG2060,CG2060,CG20 60,CG7223, |
| RAPA | SLC2A1,EIF3J,GABAR APL2,GABARAPL1,SM N1,CYP3A4,CYP3A5,N PR1,UBE2L3,TRPV6,T RPV5,CCNA2,CD36,C DK2, PSMD9, | 6513,8669,11345, 23710,6606,1576, 1577,4881,7332,5 5503,56302,890,9 48,1017,5715, | CG1086,CG12131,CG1 2334,CG12334,CG167 25,CG2060,CG2060,C G31183,CG5788,CG58 42,CG5842,CG5940,C G7422,CG8203,CG958 8, |
| rasagiline | NFKB1, | 4790, | CG11992, |
| rebamipide | AURKB, | 9212, | CG6620, |
| reboxetine | CYP3A4, | 1576, | CG2060, |
| remifentanil | ELAVL1, | 1994, | CG4396, |
| renzapride | CYP4F3,CYP3A5, | 4051,1577, | CG2060,CG2060, |
| repaglinide | CYP3A4, | 1576, | CG2060, |
| Resiniferotoxin | TRPV1, | 7442, | CG5842, |
| resiquimod | GSTO1,DNALI1, | 9446,7802, | CG6673,CG6971, |
| Retardex | PDE4A,UGT1A4,UGT1 A3,PSIP1, | 5141,54657,54659 , 11168, | CG14940,CG4772,CG4 772,CG7946, |
| Riacon | NFKB1, | 4790, | CG11992, |
| Ribavirin | NFKB1, | 4790, | CG11992, |
| Riboflavin | GSR, | 2936, | CG2151, |
| Rifabutin | CYP4F3,CYP3A4,CYP3 A5,POLD1,CDC2, | 4051,1576,1577,5 424,983, | CG2060,CG2060,CG20 60,CG5949,CG8203, |
| rifamycins | GPR177, | 79971, | CG6210, |

(continued)

| Small Molecule | Interacting Gene Symbol | Interacting GeneID | Drosophila Id |
|---|---|---|---|
| Rifocin | CYP3A4, | 1576, | CG2060, |
| rimonabant | CNR1, | 1268, | CG12796, |
| risedronic acid | DDOST, | 1650, | CG9022, |
| risperidone | LPAR3,CYP3A4, | 23566,1576, | CG12796,CG2060, |
| Ristocetin | ACTR2, | 10097, | CG9901, |
| Ritonavir | SLC2A4,NFKB1,CYP3 A7,CYP3A4,CD36, | 6517,4790,1551,1 576,948, | CG1086,CG11992,CG2 060,CG2060,CG7422, |
| rituximab | NFKB1,DNAH5, | 4790,1767, | CG11992,CG17150, |
| Ro 13-8996 | CYP3A4, | 1576, | CG2060, |
| Ro 23-7553 | CDK2, | 1017, | CG8203, |
| Ro 23-7637 | CYP3A4, | 1576, | CG2060, |
| Ro 24-7429 | NFKB1, | 4790, | CG11992, |
| Ro 31-6233 | SLC2A4, | 6517, | CG1086, |
| Ro 31-7549 | LPAR2, | 9170, | CG12796, |
| Ro 31-8220 | NFKB1,MAPK15,FGFR 4, | 4790,225689,2264 , | CG11992,CG2309,CG7 223, |
| RO4383596 | FGFR1,FGFR2, | 2260,2263, | CG7223,CG7223, |
| Robitet | PHB,UGT1A1,UGT1A6 , DPAGT1,PSMD9, | 5245,54658,54578 , 1798,5715, | CG10691,CG4772,CG4 772,CG5287,CG9588, |
| rofecoxib | NFKB1, | 4790, | CG11992, |
| roflumilast | PDE4A,PDE4B,CYP3A 4, | 5141,5142,1576, | CG14940,CG14940,CG 2060, |
| rokitamycin | DPAGT1, | 1798, | CG5287, |
| Rolipram | NFKB1,PDE4B,PDE4C, PDE4A, ADORA2A, | 4790,5142,5143,5 141,135, | CG11992,CG14940,CG 14940,CG14940,CG97 53, |
| romidepsin | NFKB1, | 4790, | CG11992, |
| rooperol | CYP3A4, | 1576, | CG2060, |
| ropivacaine | CYP3A4, | 1576, | CG2060, |

(continued)

| Small Molecule | Interacting Gene Symbol | Interacting GeneID | Drosophila Id |
|---|---|---|---|
| roscovitine | CDK9,UBE2L3,CCNA2 , POLD1,CDK5,CDC2,C DK2,PSMD9, | 1025,7332,890,54 24,1020,983,1017, 5715, | CG5179,CG5788,CG59 40,CG5949,CG8203,C G8203,CG8203,CG958 8, |
| rosiglitazone | SLC2A4,CYP3A4,CD3 6,ACSL4, | 6517,1576,948,21 82, | CG1086,CG2060,CG74 22,CG8732, |
| rosmarinic acid | NFKB1, | 4790, | CG11992, |
| rosuvastatin | CYP3A4,CYP3A5, | 1576,1577, | CG2060,CG2060, |
| Roxithromycin | NFKB1,CYP3A4, | 4790,1576, | CG11992,CG2060, |
| Rozevin | GPR177,CDC2, | 79971,983, | CG6210,CG8203, |
| RPR 121056 | CYP3A4, | 1576, | CG2060, |
| RU 58668 | NFKB1,PSMD9, | 4790,5715, | CG11992,CG9588, |
| ruboxistaurin | CYP3A4, | 1576, | CG2060, |
| rugosin E | NFKB1, | 4790, | CG11992, |
| rutecarpine | CYP3A4,TRPV1, | 1576,7442, | CG2060,CG5842, |
| Rutin | NFKB1, | 4790, | CG11992, |
| S-(beta-p-methoxypropiophenone)thiami ne | CYP3A4, | 1576, | CG2060, |
| S-Nitroso-N-Acetylpenicillamine | NFKB1,SERPINB3, | 4790,6317, | CG11992,CG8137,CG9453,CG9460, |
| S-Nitrosothiols | NFKB1,LPAR2, | 4790,9170, | CG11992,CG12796, |
| S-phenyl-N-acetylcysteine | GSTT1, | 2952, | CG30005, |
| sabarubicin | GPR177, | 79971, | CG6210, |
| sabcomeline | FGFR3, | 2261, | CG7223, |
| Safingol | TYMS, | 7298, | CG3181, |
| Safrole | GSTT1, | 2952, | CG30005, |
| SAGA | SMARCA1, | 6594, | CG8625, |
| SAHA | NFKB1,HDAC3,KPNA2 , POLD1,AURKB,AURK A,CDC2, | 4790,8841,3838,5 424,9212,6790,98 3, | CG11992,CG2128,CG4 799,CG5949,CG6620, CG6620,CG8203, |

(continued)

| Small Molecule | Interacting Gene Symbol | Interacting GeneID | Drosophila Id |
|---|---|---|---|
| saikosaponin | NFKB1, | 4790, | CG11992, |
| Salicin | LPAR2, | 9170, | CG12796, |
| salvin | CCNA2,PSMD9, | 890,5715, | CG5940,CG9588, |
| samarium | DCP1B,EXOSC1,SNRP G, | 196513,51013,663 7, | CG11183,CG6249,CG9 742, |
| SAMe | ELP3, | 55140, | CG15433, |
| sanguinarine | NFKB1, | 4790, | CG11992, |
| sapogenins | UGT1A4, | 54657, | CG4772, |
| Saquinavir | CYP3A5,CYP3A4,CYP3 A7, | 1577,1576,1551, | CG2060,CG2060,CG20 60, |
| Sarasar | CYP3A4, | 1576, | CG2060, |
| Sarna | NFKB1,CYP3A4,TRPA 1,TRPV1,TRPV4, | 4790,1576,8989,7 442,59341, | CG11992,CG2060,CG5 751,CG5842,CG5842, |
| sauchinone | NFKB1, | 4790, | CG11992, |
| saxatilin | PSMD9, | 5715, | CG9588, |
| SB 218078 | POLD1,CDC2, | 5424,983, | CG5949,CG8203, |
| SB 225002 | LPAR2, | 9170, | CG12796, |
| SB 415286 | ACTR2, | 10097, | CG9901, |
| SB-705498 | TRPV1, | 7442, | CG5842, |
| scandium | CELSR1,ME2,HNRNPH 3, | 9620,4200,3189, | CG11895,CG5889,CG6 946, |
| SCH 66712 | CYP3A4, | 1576, | CG2060, |
| schizandrer A | NPFFR2, | 10886, | CG10823, |
| scoparone | PSIP1, | 11168, | CG7946, |
| Scopoletin | UGT1A3, | 54659, | CG4772, |
| Score | SERPINC1, | 462, | CG8137, |
| SDX 308 | NFKB1, | 4790, | CG11992, |
| Selegiline | KHDRBS1, | 10657, | CG3613,CG5821, |

(continued)

| Small Molecule | Interacting Gene Symbol | Interacting GeneID | Drosophila Id |
|---|---|---|---|
| seocalcitol | PSMD9, | 5715, | CG9588, |
| Sep-Pak | ECD, | 11319, | CG5714, |
| Serad | CYP3A4, | 1576, | CG2060, |
| sertindole | CYP3A4, | 1576, | CG2060, |
| sevoflurane | NFKB1,TRPV1, | 4790,7442, | CG11992,CG5842, |
| SEW2871 | S1PR1, | 1901, | CG12796, |
| shikonin | NFKB1,FGFR4, | 4790,2264, | CG11992,CG7223, |
| siderophore | VDAC1, | 7416, | CG17137, |
| Sildenafil | PLXNA3,PDE4A,CYP3 A5,CYP3A4, | 55558,5141,1577, 1576, | CG11081,CG14940,CG 2060,CG2060, |
| silvestrol | POLD1,CDC2, | 5424,983, | CG5949,CG8203, |
| silybin | NFKB1,CYP3A4,UGT1 A1,UBE2L3,CCNB1,C DK2,PSMD9, | 4790,1576,54658, 7332,891,1017,57 15, | CG11992,CG2060,CG4 772,CG5788,CG5940, CG8203,CG9588, |
| Sincalide | CCKAR, | 886, | CG6881, |
| Sizofiran | TAOK2, | 9344, | CG14217, |
| SK-7041 | CCNB1, | 891, | CG5940, |
| SK&F 106528 | SFRS1, | 6426, | CG6987, |
| SM 7368 | NFKB1, | 4790, | CG11992, |
| Sodium pentosan poly sulfate | SERPINC1, | 462, | CG8137, |
| Sodium Salicylate | NFKB1, | 4790, | CG11992, |
| sorafenib | NFKB1,CYP3A4,CCNA 2,CCNB1,POLD1,CDC 2,PAFAH1B1,PSMD9, | 4790,1576,890,89 1,5424,983,5048,5 715, | CG11992,CG2060,CG5 940,CG5940,CG5949, CG8203,CG8440,CG95 88, |
| sorbinil | NFKB1, | 4790, | CG11992, |
| Sorbo | NFKB1,SORD, | 4790,6652, | CG11992,CG1982, |
| Sorbose | SLC2A1, | 6513, | CG1086, |

(continued)

| Small Molecule | Interacting Gene Symbol | Interacting GeneID | Drosophila Id |
|---|---|---|---|
| spiroglumide | CCKBR, | 887, | CG6881, |
| Spironolactone | NFKB1, | 4790, | CG11992, |
| squamocin | CDC2,PSMD9, | 983,5715, | CG8203,CG9588, |
| SR 144528 | CNR1,CNR2, | 1268,1269, | CG12796,CG12796, |
| SR 27897 | CCKAR, | 886, | CG6881, |
| SR 48692 | SERPINB1, | 1992, | CG8137,CG9453,CG94 60, |
| SR 80327A | SERPINC1, | 462, | CG8137, |
| SR 90107A-ORG 31540 | SERPINC1, | 462, | CG8137, |
| ST 638 | FGFR4, | 2264, | CG7223, |
| stallimycin | UBE2L3, | 7332, | CG5788, |
| Stanozolol | SERPINC1, | 462, | CG8137, |
| staurosporine | SLC2A4,NFKB1,LPAR3 , CYP3A4,TXNRD2,CC NB1,POLD1,AURKA,F YN, CDC2,CDK2,PSMD 9,ACTR2, | 6517,4790,23566, 1576,10587,891,5 424,6790,2534,98 3,1017,5715,1009 7, | CG1086,CG11992,CG1 2796,CG2060,CG2151 , CG5940,CG5949,CG6 620,CG7873,CG8203, CG8203,CG9588,CG99 01, |
| Stearin | CD36, | 948, | CG7422, |
| Stereoisomerism | CDK2, | 1017, | CG8203, |
| Steviol | NFKB1, | 4790, | CG11992, |
| Stevioside | NFKB1, | 4790, | CG11992, |
| STIL | OPN4,SERPINC1, | 94233,462, | CG4550,CG8137, |
| stilbene-disulphonate | VDAC1, | 7416, | CG17137, |
| Stilbenes | CYP3A4, | 1576, | CG2060, |
| Stim | SLC2A4,CYP4F3,CYP3 A4,CYP3A5,CYP3A7,C CNB1,POLD1,CDC2,A DORA1,ADORA2A, | 6517,4051,1576,1 577,1551,891,542 4,983,134,135, | CG1086,CG2060,CG20 60,CG2060,CG2060,C G5940,CG5949,CG820 3,CG9753,CG9753, |

(continued)

| Small Molecule | Interacting Gene Symbol | Interacting GeneID | Drosophila Id |
|---|---|---|---|
| Streptomycin | VDAC1, | 7416, | CG17137, |
| Styrene | PLXNB1,GSTT1,SERPI NC1, | 5364,2952,462, | CG11081,CG30005,CG 8137, |
| styrene-methylmethacrylate copolymer | POLD1,CDC2, | 5424,983, | CG5949,CG8203, |
| SU 5416 | PSMD9, | 5715, | CG9588, |
| SU 6668 | FGFR1, | 2260, | CG7223, |
| SU 9516 | TYMS,CCNB1,CDC2,C DK2, | 7298,891,983,101 7, | CG3181,CG5940,CG82 03,CG8203, |
| suberate | NPR1, | 4881, | CG31183, |
| suberosin | NFKB1, | 4790, | CG11992, |
| succinic semialdehyde | CCKBR, | 887, | CG6881, |
| Sufentanil | CYP3A4, | 1576, | CG2060, |
| Suldox | DHPS, | 1725, | CG8005, |
| sulfadoxine-pyrimethamine | DHPS, | 1725, | CG8005, |
| Sulfamethazine | CYP3A4, | 1576, | CG2060, |
| sulfamethoxazole hydroxylamine | CYP3A4, | 1576, | CG2060, |
| Sulfaphenazole | CYP4F3,CYP3A4,CYP3 A5, | 4051,1576,1577, | CG2060,CG2060,CG20 60, |
| Sulfasalazine | NFKB1,SLC46A1, | 4790,113235, | CG11992,CG8008, |
| sulfate cellufine | LMAN1, | 3998, | CG6822, |
| sulfate-sulfate | CYP3A7, | 1551, | CG2060, |
| sulfidonitrogen(.) | CYP3A4,DLL1,FGFR3, CAPN3, | 1576,28514,2261, 825, | CG2060,CG3619,CG72 23,CG8107, |
| Sulfinpyrazone | CYP3A4, UGT1A9, UGT 1A7,UGT1A1,CLTB, | 1576,54600,54577 , 54658,1212, | CG2060,CG4772,CG47 72,CG4772,CG6948, |
| sulfo-N-succinimidyl oleate | CD36, | 948, | CG7422, |
| sulfo-succinimidyl-oleate | CD36, | 948, | CG7422, |
| sulfogalactosylglycerolipid | KHDRBS1, | 10657, | CG3613,CG5821, |
| sulfones | TYMS, | 7298, | CG3181, |

(continued)

| Small Molecule | Interacting Gene Symbol | Interacting GeneID | Drosophila Id |
|---|---|---|---|
| sulfonic acid | SERPINC1, | 462, | CG8137, |
| sulfonyl-phenyl-ethyl | CNR2, | 1269, | CG12796, |
| Sulforafan | NFKB1,CYP3A4,TXNR D1,UGT1A1,CCNB1, | 4790,1576,7296,5 4658,891, | CG11992,CG2060,CG2 151,CG4772,CG5940, |
| Sulindac | NFKB1,FMO3, | 4790,2328, | CG11992,CG3006, |
| sulindac sulfone | NFKB1, | 4790, | CG11992, |
| sultopride | CYP4F3,CYP3A5, | 4051,1577, | CG2060,CG2060, |
| sunitinib | CYP3A4, | 1576, | CG2060, |
| Synthos | CADPS,CSNK2B, | 8618,1460, | CG18026,CG8914, |
| T 0070907 | NFKB1, | 4790, | CG11992, |
| T 0901317 | CYP3A4,CD36,PSMD9 , | 1576,948,5715, | CG2060,CG7422,CG95 88, |
| Tacrine | CDK9, | 1025, | CG5179, |
| Tacrolimus | NFKB1,FOXP3,CYP3A 5,CYP3A4,UGT2B7,C D36, | 4790,50943,1577, 1576,7364,948, | CG11992,CG16899,CG 2060,CG2060,CG4772 ,CG7422, |
| tadalafil | CYP3A4, | 1576, | CG2060, |
| Tamogel | CYP3A4,UBE2L3,PSM D9, | 1576,7332,5715, | CG2060,CG5788,CG95 88, |
| Tamoxifen | NFKB1,CYP4F3,CYP3A 5,CYP3A4,TXNRD1,U GT1A4,UGT1A3,UBE2 L3,TRPV6,CCNA2,CC NB1,POLD1,FGFR4,C D36,PSIP1,SERPINC1, SERPINB3,CDK2,CDC 2,PSMD9, | 4790,4051,1577,1 576,7296,54657,5 4659,7332,55503, 890,891,5424,226 4,948,11168,462,6 317,1017,983,571 5, | CG11992,CG2060,CG2 060,CG2060,CG2151, CG4772,CG4772,CG57 88,CG5842,CG5940,C G5940,CG5949,CG722 3,CG7422,CG7946,CG 8137,CG8137,CG9453 , CG9460,CG8203,CG8 203,CG9588, |
| tandospirone | CYP3A4, | 1576, | CG2060, |
| Tangeretin | CYP3A4,PSMD9, | 1576,5715, | CG2060,CG9588, |
| tanshinone | NFKB1,CYP3A4, | 4790,1576, | CG11992,CG2060, |
| taurocholic acid | FGFR4, | 2264, | CG7223, |

(continued)

| Small Molecule | Interacting Gene Symbol | Interacting GeneID | Drosophila Id |
|---|---|---|---|
| Taurodeoxycholic Acid | NFKB1, | 4790, | CG11992, |
| tauroursodeoxycholic acid | UBE2L3, | 7332, | CG5788, |
| tautomycetin | NFKB1, | 4790, | CG11992, |
| TAXOTERE | NFKB1,TAOK2,CYP4F 3,CYP3A5,CYP3A4,CC NB1,POLD1,SERPINB3,CDC2,PAFAH 1B1,PS MD9, | 4790,9344,4051,1 577,1576,891,542 4,6317,983,5048,5715 , | CG11992,CG14217,CG 2060,CG2060,CG2060 , CG5940,CG5949,CG8137,CG9453,CG 9460, CG8203,CG8440,CG95 88, |
| TBDZ | CYP3A5,CYP3A4,CYP4 F3, | 1577,1576,4051, | CG2060,CG2060,CG20 60, |
| TBHQ | UGT1A4,UGT1A6,UGT 1A9,UGT2B7,UGT1A3 , | 54657,54578,5460 0,7364,54659, | CG4772,CG4772,CG47 72,CG4772,CG4772, |
| TCAT | ZNF24, | 7572, | CG31364,CG6930, |
| technetium | ONECUT2, | 9480, | CG1922, |
| Tegafur | TAOK2, | 9344, | CG14217, |
| Teleocidin | PSMD12, | 5718, | CG1100, |
| telithromycin | CYP3A4, | 1576, | CG2060, |
| Temazepam | CYP3A4, | 1576, | CG2060, |
| temozolomide | TXNRD2,TXNRD1,POL D1,CDC2, | 10587,7296,5424, 983, | CG2151,CG2151,CG59 49,CG8203, |
| temsirolimus | CYP3A4,PSMD9, | 1576,5715, | CG2060,CG9588, |
| terbinafine | CYP4F3,CYP3A5,CCN A2, | 4051,1577,890, | CG2060,CG2060,CG59 40, |
| terephthalic acid | HDHD1A, | 8226, | CG5565, |
| Terfenadine | CYP3A5,CYP3A4, | 1577,1576, | CG2060,CG2060, |
| teriflunomide | NFKB1, | 4790, | CG11992, |
| terrein | CCNB1,POLD1,CDC2, | 891,5424,983, | CG5940,CG5949,CG82 03, |
| territrem A | CYP3A4, | 1576, | CG2060, |
| territrem B | CYP3A5, | 1577, | CG2060, |
| territrem C | CYP3A5, | 1577, | CG2060, |

(continued)

| Small Molecule | Interacting Gene Symbol | Interacting GeneID | Drosophila Id |
|---|---|---|---|
| tertiapin | FGFR3, | 2261, | CG7223, |
| tetra-mu3-sulfido-tetrairon | ELP3, | 55140, | CG15433, |
| tetrachloroethene | CYP3A4, | 1576, | CG2060, |
| tetradecanoyl-phorbol-acetate | NFKB1, | 4790, | CG11992, |
| Tetrahydrocannabinol | CNR2,CNR1,UBE2L3, TRPV2,CCNA2,ANK1, | 1269,1268,7332,5 1393,890,286, | CG12796,CG12796,CG 5788,CG5842,CG5940 ,CG7462, |
| tetramethylrhodamine | ACTR2, | 10097, | CG9901, |
| tetramethylsilane | CELSR1,ME2, | 9620,4200, | CG11895,CG5889, |
| tetraphene | CYP3A5,CYP3A7, | 1577,1551, | CG2060,CG2060, |
| Tetraprenol | TBXAS1,TYMS, | 6916,7298, | CG2060,CG3181, |
| tetrasulfanide | NFKB1, | 4790, | CG11992, |
| Thalidomide | NFKB1, | 4790, | CG11992, |
| Thapsigargin | LYZ,NFKB1,PDE1A,CD K2, | 4069,4790,5136,1 017, | CG1180,CG11992,CG1 4940,CG8203, |
| thiamine disulfide | NFKB1, | 4790, | CG11992, |
| thiazole | TRPV1, | 7442, | CG5842, |
| Thiazolidinediones | NFKB1,CYP3A4,TFAM, PSMD9, | 4790,1576,7019,5 715, | CG11992,CG2060,CG4 217,CG9588, |
| thioacetamide | ADORA2A, | 135, | CG9753, |
| Thioacetazone | FMO3,FMO1, | 2328,2326, | CG3006,CG3006, |
| thiobenzamide | FMO1, | 2326, | CG3006, |
| thiocoraline | CYP3A4, | 1576, | CG2060, |
| Thiole | NFKB1,CYP4F3,CYP3A 5, | 4790,4051,1577, | CG11992,CG2060,CG2 060, |
| Thiopental | PLXNB1,NFKB1, | 5364,4790, | CG11081,CG11992, |
| thioredoxin dithiol | NFKB1,TXNRD1,PDHX , | 4790,7296,8050, | CG11992,CG2151,CG5 261, |
| thioridazine | CYP3A4, | 1576, | CG2060, |

(continued)

| Small Molecule | Interacting Gene Symbol | Interacting GeneID | Drosophila Id |
|---|---|---|---|
| Thiostrepton | RPL11, | 6135, | CG7726, |
| thrombin receptor peptide SFLLRNP | TXNRD1,TXNRD2, | 7296,10587, | CG2151,CG2151, |
| thrombin Tokushima | SERPINC1, | 462, | CG8137, |
| Thromboxane A2 | LPAR2,TAOK2,HADHB ,FGFR3, | 9170,9344,3032,2 261, | CG12796,CG14217,CG 4581,CG7223, |
| thromboxane B2 | TAOK2,SERPINC1, | 9344,462, | CG14217,CG8137, |
| Thyminose | DLD, | 1738, | CG7430, |
| thymol | TRPV3, | 162514, | CG5842, |
| thymoquinone | UBE2L3, | 7332, | CG5788, |
| Thyrotropin | LPAR2,TXNRD2,TXNR D1,ERP29, | 9170,10587,7296, 10961, | CG12796,CG2151,CG2 151,CG7225, |
| Ticlopidine | CYP3A4,CYP4F3,CYP3 A5,SERPINC1, | 1576,4051,1577,4 62, | CG2060,CG2060,CG20 60,CG8137, |
| Tilidine | CYP3A4, | 1576, | CG2060, |
| tipranavir | CYP3A4, | 1576, | CG2060, |
| tirilazad | CYP3A4, | 1576, | CG2060, |
| titanium alloy (TiAl6V4) | ELAVL2, | 1993, | CG4396, |
| TMC-95A | SLC5A7, | 60482, | CG7708, |
| Tmndga | POLD1,CDC2, | 5424,983, | CG5949,CG8203, |
| TMSI | TYMS, | 7298, | CG3181, |
| Tobrex | SMN1, | 6606, | CG16725, |
| tocotrienols | CYP4F2,CYP3A4,UGT 1A1, | 8529,1576,54658, | CG2060,CG2060,CG47 72, |
| tofisopam | CYP3A4, | 1576, | CG2060, |
| tolrestat | NFKB1, | 4790, | CG11992, |
| Tolterodine | CYP4F3,CYP3A5, | 4051,1577, | CG2060,CG2060, |

(continued)

| Small Molecule | Interacting Gene Symbol | Interacting GeneID | Drosophila Id |
|---|---|---|---|
| toluene | PLXNB1,CELSR1,STX 8,ME2, | 5364,9620,9482,4 200, | CG11081,CG11895,CG 4109,CG5889, |
| TOLUENE-DITHIOL | ME3, | 10873, | CG5889, |
| topiramate | CYP3A4,UGT2B7, | 1576,7364, | CG2060,CG4772, |
| Topotecan | CYP3A4,TXNRD1,TXN RD2, | 1576,7296,10587, | CG2060,CG2151,CG21 51, |
| Toremifene | ECD,SERPINC1, | 11319,462, | CG5714,CG8137, |
| Tosylarginine Methyl Ester | SERPINC1, | 462, | CG8137, |
| Tosyllysine Chloromethyl Ketone | SMAD7, | 4092, | CG5201, |
| Tosylphenylalanyl Chloromethyl Ketone | NFKB1, | 4790, | CG11992, |
| TPN+ | UGT1A4,UGT1A3, | 54657,54659, | CG4772,CG4772, |
| Tracer | GSTO2, | 119391, | CG6673, |
| Tramadol | NFKB1, | 4790, | CG11992, |
| trans-resveratrol | NFKB1,SMN1,CYP3A5 , CYP4F3,CYP3A4,CDC 2,PSMD9, | 4790,6606,1577,4 051,1576,983,571 5, | CG11992,CG16725,CG 2060,CG2060,CG2060 , CG8203,CG9588, |
| trastuzumab | LARP6,ECD,CCNA2,C DK2,PSMD9, | 55323,11319,890, 1017,5715, | CG17386,CG5714,CG5 940,CG8203,CG9588, |
| Trazodone | CYP3A4, | 1576, | CG2060, |
| Tremode | CYP3A4,CYP3A5, | 1576,1577, | CG2060,CG2060, |
| Tremorine | PLXNA2, | 5362, | CG11081, |
| Tretinoin | TXNRD1, | 7296, | CG2151, |
| Triad | WDR92,PRDX6, | 116143,9588, | CG14353,CG3083, |
| Triamcinolone | PLXNA2, | 5362, | CG11081, |
| triazolam | CYP3A4, | 1576, | CG2060, |
| triazoles | CYP3A4, | 1576, | CG2060, |
| tributylstannane | CYP3A5,CYP4F3, | 1577,4051, | CG2060,CG2060, |

(continued)

| Small Molecule | Interacting Gene Symbol | Interacting GeneID | Drosophila Id |
|---|---|---|---|
| trichostatins | CCNA2, | 890, | CG5940, |
| Triclosan | RCP9, | 27297, | CG4875, |
| triethylamine | TXNRD1, | 7296, | CG2151, |
| Trifluoperazine | UGT1A4, | 54657, | CG4772, |
| trimethylaminocarboxyldihydr oboran | NFKB1, | 4790, | CG11992, |
| trioctyl phosphine oxide | UBE2L3, | 7332, | CG5788, |
| Triolein | PQBP1, | 10084, | CG11820, |
| tripterine | NFKB1,PSMD9, | 4790,5715, | CG11992,CG9588, |
| triptolide | NFKB1,CYP3A4, | 4790,1576, | CG11992,CG2060, |
| triterpenoids | NFKB1,PDE4A, | 4790,5141, | CG11992,CG14940, |
| troglitazone | SLC2A1,NFKB1,CYP3 A5,CYP3A7,CYP3A4,U GT1A6,POLD1,CD36, DLD, CDC2,CDK2,PSM D9, | 6513,4790,1577,1 551,1576,54578,5 424,948,1738,983, 1017,5715, | CG1086,CG11992,CG2 060,CG2060,CG2060, CG4772,CG5949,CG74 22,CG7430,CG8203,C G8203,CG9588, |
| Troleandomycin | CYP3A7,CYP4F3,CYP3 A5,CYP3A4, | 1551,4051,1577,1 576, | CG2060,CG2060,CG20 60,CG2060, |
| TTNPB | UGT2B15, | 7366, | CG4772, |
| tubocapsanolide A | PSMD9, | 5715, | CG9588, |
| Tunicamycin | SLC2A1,CCNA2, | 6513,890, | CG1086,CG5940, |
| tyrphostin AG 1478 | S1PR3,GBF1,PSMD9, | 1903,8729,5715, | CG12796,CG8487,CG9 588, |
| tyrphostin AG-490 | FOXP3, | 50943, | CG16899, |
| tyrphostin AG17 | CDK2, | 1017, | CG8203, |
| U 0126 | SLC2A5,NFKB1,SMN1 , SMAD6,CCNA2,AURK A,CDK2, | 6518,4790,6606,4 091,890,6790,101 7, | CG1086,CG11992,CG1 6725,CG5201,CG5940 , CG6620,CG8203, |
| Ubizol | CYP3A4,FGFR3, | 1576,2261, | CG2060,CG7223, |
| Ufur | TYMS, | 7298, | CG3181, |

(continued)

| Small Molecule | Interacting Gene Symbol | Interacting GeneID | Drosophila Id |
|---|---|---|---|
| UK 157147 | UGT1A1, | 54658, | CG4772, |
| Uprima | FGFR3, | 2261, | CG7223, |
| Uran | RBM5, | 10181, | CG4887, |
| uranyl acetate | NFKB1, | 4790, | CG11992, |
| urethane | ELAC1, | 55520, | CG3298, |
| Urex | UGT1A1,FGFR3, | 54658,2261, | CG4772,CG7223, |
| urinastatin | SERPINC1, | 462, | CG8137, |
| Urso | CYP3A4,ME2,ME1,ME 3, | 1576,4200,4199,1 0873, | CG2060,CG5889,CG58 89,CG5889, |
| USAN | NFKB1,GSR,UGT2B7, SERPINB3,CDK2, | 4790,2936,7364,6 317,1017, | CG11992,CG2151,CG4 772,CG8137,CG9453, CG9460,CG8203, |
| valerenic acid | NFKB1, | 4790, | CG11992, |
| valspodar | CYP3A4, | 1576, | CG2060, |
| venlafaxine | CYP3A4, | 1576, | CG2060, |
| Verapamil | CYP3A5,CYP3A4,ME1, GPR177,FGFR3,DHPS, | 1577,1576,4199,7 9971,2261,1725, | CG2060,CG2060,CG58 89,CG6210,CG7223,C G8005, |
| verlukast | GPR177, | 79971, | CG6210, |
| vesnarinone | CYP3A4, | 1576, | CG2060, |
| Viagra | SMN1, | 6606, | CG16725, |
| Vigil | CYP3A5,CYP3A4, | 1577,1576, | CG2060,CG2060, |
| vincristine | CYP4F3,CYP3A5,CYP3A4,CCNB1,GP R177, | 4051,1577,1576,891,7 9971, | CG2060,CG2060,CG2060,CG5940,CG 6210, |
| vinflunine | CYP4F3,CYP3A5,COX 7A2L, | 4051,1577,9167, | CG2060,CG2060,CG96 03, |
| vinorelbine | CYP4F3,CYP3A5,TYM S, | 4051,1577,7298, | CG2060,CG2060,CG31 81, |
| vinpocetine | PDE1C, | 5137, | CG14940, |
| violacein | PSMD9, | 5715, | CG9588, |

(continued)

| Small Molecule | Interacting Gene Symbol | Interacting GeneID | Drosophila Id |
|---|---|---|---|
| Vira-A | NFKB1,LPAR2,PDE4A, FOXP3,DLL1,TRPV1,S FRS1,FGFR3,ADORA2 A, ADORA1,ACTR2, | 4790,9170,5141,5 0943,28514,7442, 6426,2261,135,13 4,10097, | CG11992,CG12796,CG 14940,CG16899,CG36 19,CG5842,CG6987,C G7223,CG9753,CG975 3,CG9901, |
| voriconazole | CYP3A4,FMO3,FMO1, | 1576,2328,2326, | CG2060,CG3006,CG30 06, |
| vorozole | SERPINB1, | 1992, | CG8137,CG9453,CG94 60, |
| VX680 | AURKA, | 6790, | CG6620, |
| Warfarin | CYP4F2,CYP4F3,CYP3 A4,CYP3A5,SERPINC1 , | 8529,4051,1576,1 577,462, | CG2060,CG2060,CG20 60,CG2060,CG8137, |
| WAY-169916 | NFKB1, | 4790, | CG11992, |
| Win 55212-2 | SLC2A4,CNR2,CNR1, | 6517,1269,1268, | CG1086,CG12796,CG1 2796, |
| withaferin A | NFKB1, | 4790, | CG11992, |
| WLN: QR BG | C9orf5, | 23731, | CG2698, |
| WLN: RVR | EXOSC1, | 51013, | CG6249, |
| WLN: ZSWR | CCNA2, | 890, | CG5940, |
| xanthohumol | CYP3A4,UGT1A3,UGT 1A4, | 1576,54659,54657 , | CG2060,CG4772,CG47 72, |
| Xaxa | NFKB1,UGT1A6,CDK9 , FGFR3,CD36,SERPIN C1, | 4790,54578,1025, 2261,948,462, | CG11992,CG4772,CG5 179,CG7223,CG7422, CG8137, |
| Xylit | TXNRD1, | 7296, | CG2151, |
| Y 27632 | SMN1, | 6606, | CG16725, |
| yristate | NFKB1,CD226,RPL10, TXNRD1,SLC25A21,S AG,ECD, UBE2L3,TRP V4,CCNA2,HNRNPH3, FGFR1,CD36,FGR,MA PK8IP3,SERPINB10,C DK2,SMARCA1,ADOR A2A, | 4790,10666,6134, 7296,89874,6295, 11319,7332,59341 , 890,3189,2260,94 8,2268,23162,527 3,1017,6594,135, | CG11992,CG13162,CG 17521,CG2151,CG525 4,CG5711,CG5714,CG 5788,CG5842,CG5940 , CG6946,CG7223,CG7 422,CG7873,CG8110, CG8137,CG9453,CG94 60,CG8203,CG8625,C G9753, |
| Z 338 | UGT1A9,UGT1A1, | 54600,54658, | CG4772,CG4772, |

(continued)

| Small Molecule | Interacting Gene Symbol | Interacting GeneID | Drosophila Id |
|---|---|---|---|
| zafirlukast | CYP3A4, | 1576, | CG2060, |
| Zalcitabine | TFAM, | 7019, | CG4217, |
| zardaverine | PDE4A, | 5141, | CG14940, |
| ZD 9331 | TYMS, | 7298, | CG3181, |
| Zearalenone | CYP3A4, | 1576, | CG2060, |
| Zeldox | CYP3A4, | 1576, | CG2060, |
| zerumbone | POLD1,CD36,CDC2, | 5424,948,983, | CG5949,CG7422,CG82 03, |
| Zidovudine | UGT2B7,UGT1A9,GPR 177,FGFR4, | 7364,54600,79971 , 2264, | CG4772,CG4772,CG62 10,CG7223, |
| zileuton | CYP4F3,CYP3A5, | 4051,1577, | CG2060,CG2060, |
| Zocor | NFKB1,S1PR3,CYP3A 5,CYP3A4,CD36,FYN, SERPINC1,CDK2,PSM D9, | 4790,1903,1577,1 576,948,2534,462,101 7,5715, | CG11992,CG12796,CG 2060,CG2060,CG7422,CG7873,CG813 7,CG8 203,CG9588, |
| zopiclone | CYP3A4, | 1576, | CG2060, |
| Zymosan | RFC1,NAP1L1,FGR,N DUFAB1, | 5981,4673,2268,4 706, | CG1119,CG5330,CG78 73,CG9160, |
| Trospium chloride | CYP3A4 | 1576 | CG2060, |
| Valproic Acid | PLXNB1,CYP3A4,SMN 2,NFKB1,TYMS,SMN1, SLC2A4,SLC2A1,GSR, CYP3A4,CYP3A4,CYP3 A4,CYP4B1, | 5364,1576,6607,4 790,7298,6606,65 17,6513,2936,157 6,1576,1576,1580 | CG11081,CG2060,CG1 6725,CG11992,CG318 1,CG16725,CG1086,C G1086,CG2151,CG206 0,CG2060,CG2060,CG 2060, |

The inventive pharmaceutical compounds suitable for the treatment of pain are also known by the following synonyms, trade names and CAS designations:

(1S,2S)-2-(2-(N-((3-benzimidazol-2-yl)propyl)-N-methylamino)ethyl)-6-fluoro-1,2,3,4-tetrahydro-1-isopropyl-2-naphtyl cyclopropanecarboxylate dihydrochloride, (1S,2S)-2-(2-(N-((3-benzimidazol-2-yl)propyl)-N-methylamino) ethyl)-6-fluoro-1,2,3,4-tetrahydro-1-isopropyl-2-naphtyl cyclopropanecarboxylate dihydrochloride, NNC 55-0396; (5-(2-methoxy-5-chloro-5-phenyl)furan-2-ylcarbonyl)guanidine, (5-(2-methoxy-5-chloro-5-phenyl)furan-2-ylcarbonyl)guanidine, (5-(2-methoxy-5-chlorophenyl)furan-2-ylcarbonyl)guanidine, KR-32570;
(6S)-5,6,7,8-tetrahydrofolic acid, (6S)-5,6,7,8-TETRAHYDROFOLATE, (6S)-5,6,7,8-tetrahydrofolic acid, (6S)-5,6,7,8-tetrahydropteroylglutamate;
(T,G)-A-L, (2S)-2-amino-3-(4-hydroxyphenyl)propanoic acid; (2S)-2-aminopentanedioic acid; (2S)-2-aminopropanoic acid; (2S)-2,6-diaminohexanoic acid, (2S)-2-aminoglutaric acid; (2S)-2-amino-3-(4-hydroxyphenyl)propionic acid; (2S)-2-aminopropionic acid;
(2S)-2,6-diaminohexanoic acid, (L-Tyrosine-L-glutamic acid)-poly(DL-alanine)-poly(L-lysine);
1 alpha-hydroxyergocalciferol, 1 alpha-hydroxyergocalciferol, 1alpha(OH)2D2, 1alpha-hydroxyvitamin D2;
1-(1-cyclohexylethylamino)-4-phenylphthalazine, 1-(1-cyclohexylethylamino)-4-phenylphthalazine, MKC 963, MKC-963; 1-(2-methyl-4-methoxyphenyl)-4-((2-hydroxyethyl)amino)-6-trifluoromethoxy-2,3-dihydropyrrolo(3,2-c) quinoline, 1-(2-methyl-4-methoxyphenyl)-4-((2-hydroxyethyl)amino)-6-trifluoromethoxy-2,3-dihydropyrrolo(3,2-c) quinoline, 1-(4-methoxy-2-methylphenyl)-4-((2-hydroxyethyl)amino)-6-trifluoromethoxy-2,3-dihydropyrrolo(3,2-c) quinoline, KR 60436; 1-(2,3-dichlorobenzoyl)-5-methoxy-2-methyl-(2-(mopholin-4-yl)ethyl)-1H-indole, 1-(2,3-dichlorobenzoyl)-5-methoxy-2-methyl-(2-(mopholin-4-yl)ethyl)-1H-indole, GW405833;
1-(2,3-dihydro-1,4-benzodioxin-5-yl)-4-((5-(4-fluorophenyl)-3-pyridinyl)methyl)piperazine, 1-(2,3-dihydro-1,4-benzodioxin-5-yl)-4-((5-(4-fluorophenyl)-3-pyridinyl)methyl)piperazine, SLV 313, SLV-313;
1-(6-((3-methoxyestra-1,3,5(10)-trien-17-yl)amino)hexyl)-1H-pyrrole-2,5-dione, 1-(6-((3-methoxyestra-1,3,5(10)-trien-17-yl)amino)hexyl)-1H-pyrrole-2,5-dione, U 73122, U-73,122; 1-adamantyl propargyl ether, 1-adamantyl propargyl ether, 1APE; 1-aminobenzotriazole, 1-aminobenzotriazole;
1-aminooxy-3-aminopropane, 1-aminooxy-3-aminopropane; 1-hydrazino-4-(3,5-dimethyl)-1-pyrazolyl-5H-pyridazino(4,5-b)indole, 1-hydrazino-4-(3,5-dimethyl)-1-pyrazolyl-5H-pyridazino(4,5-b)indole;
1-hydroxymethylmidazolam, 1-hydroxymethylmidazolam; 1-hydroxypyrene, 1-hydroxypyrene, 1-pyrenol;
1-Methyl-4-phenylpyridinium, 1 Methyl 4 phenylpyridine, 1 Methyl 4 phenylpyridinium, 1 Methyl 4 phenylpyridinium Chloride; 1-Nitropyren-8-ol, 1-Nitro-8-hydroxypyrene, 1-Nitropyren-8-ol, 1-Nitropyrene-8-ol;
1-phosphatidyl-1D-myo-inositol 3-phosphates, 1,2-Diacyl-sn-glycero-3-phospho-(1'-myo-inositol-3'-phosphate), 1-O-(3-sn-phosphatidyl)-1D-myo-inositol 3-(dihydrogen phosphate), 1-Phosphatidyl-1D-myo-inositol 3-phosphate;
1-stearoyl-2-oleoyl-sn-glycero-3-phosphocholine, 1-SOPC, 1-stearoyl-2-oleoyl lecithin, 1-stearoyl-2-oleoyl-sn-3-glycerophosphocholine;
1,1-bis(3'-indolyl)-1-(4-t-butylphenyl)methane, 1,1-bis(3'-indolyl)-1-(4-t-butylphenyl)methane, 1,1-bis(3'-indolyl)-1-(p-t-butylphenyl)methane, DIM-C-pPhtBu;
1,1-dimethylbutyl-1-deoxy-Delta(9)-THC, 1,1-dimethylbutyl-1-deoxy-Delta(9)-THC, JWH-133, JWH1333;
1,1,1-trichloro-2-(4-hydroxyphenyl)-2-(4-methoxyphenyl)ethane, 1,1,1-trichloro-2-(4-hydroxyphenyl)-2-(4-methoxyphenyl)ethane, mono-OH-M;
1,2-bis(diphenylphosphino)ethane, 1,2-bis(diphenylphosphino)ethane, bis(diphenylphosphine)ethane, C(CP (c1ccccc1)c2ccccc2)P(c3ccccc3)c4ccccc4;
1,2-di-(4-sulfamidophenyl)-4-butylpyrazolidine-3,5-dione, 1,2-di-(4-sulfamidophenyl)-4-butylpyrazolidine-3,5-dione;
1,2-diacyl-sn-glycero-3-phosphocholines, (3-sn-phosphatidyl)choline, 1,2-diacyl-sn-glycero-3-phosphocholine, 1,2-diacyl-sn-glycero-3-phosphocholines;
1,2-ethanedithiol, 1,2-ethanedithiol, 2-mercaptoethanol disulfide, BisEDT;
1,2-oleoylphosphatidylcholine, 1,2-dioleoyl glycerophosphocholine, 1,2-dioleoyl-sn-glycerol-3-ethylphosphocholine, 1,2-dioleoylglycerophosphocholine; 1,2,4-triazines, 1,2,4-triazines;
1,25-dihydroxy-21-(3-hydroxy-3-methylbutyl)-23-yne-26,27-hexafluorocholecalciferol, 1,25-dihydroxy-21-(3-hydroxy-3-methylbutyl)-23-yne-26,27-hexafluorocholecalciferol, 1alpha,25-dihydroxy-20S-21-(3-hydroxy-3-methylbutyl)-23-yne-26,27-hexafluorocholecalciferol, Ro 438-2582;
1,25-dihydroxyergocalciferol, 1 alpha,25-dihydroxyvitamin D2, 1,25-(OH)2D2, 1,25-dihydroxyergocalciferol;
1,25D3, (1alpha,3beta,5Z,7E)-9,10-secocholesta-5,7,10(19)-triene-1,3,25-triol, (1R,3S)-5-{2-[(1R,3aS,7aR)-1-((R)-5-Hydroxy-1,5-dimethyl-hexyl)-7a-methyl-octahydro-inden-4-ylidene]-ethylidene}-4-methylene-cyclohexane-1,3-diol, (1R,3S,5Z)-5-[(2E)-2-[(1R,3aS,7aR)-1-[(1R)-5-hydroxy-1,5-dimethyl-hexyl]-7a-methyl-2,3,3a,5,6,7-hexahydro-1H-inden-4-ylidene]ethylidene]-4-methylene-cyclohexane-1,3-diol;
1,3-Dcg, (2-hydroxy-3-octanoyloxy-propyl) octanoate, (2-hydroxy-3-octanoyloxypropyl) octanoate, 1,3-Dcg;

1,3-dihydroxy-4,4,5,5-tetramethyl-2-(4-carboxyphenyl)tetrahydroimidazole, 1,3-dihydroxy-4,4,5,5-tetramethyl-2-(4-carboxyphenyl)tetrahydroimidazole, 1,3-DTCTI, 2-(4-carboxyphenyl)-;
1,3-dipropyl-8-(3-noradamantyl)xanthine, 1,3-dipropyl-8-(3-noradamantyl)xanthine;
1,3,5-trimethylbenzene, 1,3,5-trimethylbenzene, 3,5-dimethyltoluene, Cc1cc(C)cc(C)c1;
1,7-dioxa-2,6-diaza-4,4-dioxide-4,7a-dithia-3H,5H-benzo(cd)pentalene, 1,7-dioxa-2,6-diaza-4,4-dioxide-4,7a-dithia-3H,5H-benzo(cd)pentalene, HEP-IV;
10-deoxymethynolide, 10-deoxymethynolide;
10-propargyl-10-deazaaminopterin, 10-propargyl-10-deazaaminopterin, pralatrexate;
10-undecynoic acid, 10-undecynoic acid;
10,10-bis(4-pyridinylmethyl)-9(10H)-anthracenone, 10,10-bis(4-pyridinylmethyl)-9(10H)-anthracenone, XE 991, anthracenone, XE-991;
11-cis-retinal, (2E,4Z,6E,8E)-3,7-dimethyl-9-(2,6,6-trimethylcyclohex-1-en-1-yl)nona-2,4,6,8-tetraenal, 11-cis-retinal, 11-cis-Retinene;
11-hydroxycannabinol, 11-hydroxycannabinol;
12-Hht, (5E,8E,10E)-12-hydroxyheptadeca-5,8,10-trienoic acid, 12-Hht, 12-hydroxy-5,8,10-heptadecatrienoic acid;
13-Lox, (9E,11E)-13-hydroxyoctadeca-9,11-dienoic acid, 13-Hodd, 13-Hode;
13-oxo-9,11-octadecadienoic acid, 13-oxo-9,11-octadecadienoic acid;
15 hete, (15S)-15-Hydroxy-5,8,11-cis-13-trans-eicosatetraenoate, (15S)-15-Hydroxy-5,8,11-cis-13-trans-icosatetraenoate, (15S,5Z,8Z,11Z,13E)-15-Hydroxyeicosatetraenoic acid;
15-Hydroxy-11 alpha,9 alpha-(epoxymethano)prosta-5,13-dienoic Acid, (15S)-Hydroxy-11 alpha, 9 alpha-(epoxymethano)prosta-5Z, 13E-dienoic Acid, (15S)hydroxy-9alpha,11alpha-(epoxymethano)prosta-5,13-dienoic acid, 11 alpha,9 alpha-Epoxymethano PGH2;
17-(allylamino)-17-demethoxygeldanamycin, 17-(allylamino)-17-demethoxy-geldanamycin, 17-(allylamino)-17-demethoxygeldanamycin, 17-AAG;
17alpha-ethynylestradiol, 17-Ethinyl-3,17-estradiol, 17-Ethinyl-3,17-oestradiol, 17-Ethinylestradiol;
1843U89, 1843U, 1843U89, 2-(5-(((1,2-dihydro-3-methyl-1-oxobenzo(f)quinazolin-9-yl)methyl)amino)-1-oxo-2-isoindolinyl)glutaric acid;
1D-myo-inositol 1,3,4,5-tetrakisphosphate, 1D-myo-inositol 1,3,4,5-tetrakis(dihydrogen phosphate), 1D-myo-inositol 1,3,4,5-tetrakisphosphate, D-myo-Inositol 1,3,4,5-tetrakisphosphate;
1H-indole, 1H-indole, 2,3-Benzopyrrole, c1ccc2[nH]ccc2c1;
1H-pyrazole, 1,2-Diazole, 1H-Pyrazol, 1H-pyrazole;
1H-pyrazolo(3,4-b)pyridine, 1H-pyrazolo(3,4-b)pyridine;
2 APB, ()-2-Amino-4-phosphonobutyric acid, ()-AP-4, 2 APB;
2-(1-(3-dimethylaminopropyl)-5-methoxyindol-3-yl)-3-(1H-indol-3-yl)maleimide, 2-(1-(3-dimethylaminopropyl)-5-methoxyindol-3-yl)-3-(1H-indol-3-yl)maleimide, Gö6983;
2-(1-methyl-4-piperidinyl)-6-(2-phenylpyrazolo(1,5-a)pyridin-3-yl)-3(2H)-pyridazinone, 2-(1-methyl-4-piperidinyl)-6-(2-phenylpyrazolo(1,5-a)pyridin-3-yl)-3(2H)-pyridazinone, FR194921;
2-(2-hydroxyethylsulfanyl)-3-methyl-1,4-naphthoquinone, 2-(2-hydroxyethylsulfanyl)-3-methyl-1,4-naphthoquinone, CPD 5, CPD-5;
2-(3,4-dimethoxyphenyl)-5-amino-2-isopropylvaleronitrile, 1-isopropyl-1-N-methylpropylamino-(3,4-dimethoxyphenyl)acetonitrile, 2-(3,4-dimethoxyphenyl)-5-amino-2-isopropylvaleronitrile, 2-(3,4-dimethoxyphenyl)-5-amino-2-isopropylvaleronitrile hydrochloride;
2-(4-amino-3-methylphenyl)-5-fluorobenzothiazole, 2-(4-amino-3-methylphenyl)-5-fluorobenzothiazole, 2-NMPh-FBzT, 5F-203;
2-(4-morpholinoanilino)-6-cyclohexylaminopurine, 2-(4-morpholinoanilino)-6-cyclohexylaminopurine, reversine;
2-(4-morpholinyl)-8-phenyl-4H-1-benzopyran-4-one, 2-(4-morpholinyl)-8-phenyl-4H-1-benzopyran-4-one, LY 294002, LY-294002;
2-(4-toluidino)-6-naphthalenesulfonic acid, 2,6-TNS, 2-(4-toluidino)-6-naphthalenesulfonic acid, 2-(4-toluidino)-6-naphthalenesulfonic acid, monopotassium salt;
2-(cyclohexylmethylidenehydrazino)adenosine, 2-(cyclohexylmethylidenehydrazino)adenosine, Binodenoson, WRC 0470;
2-AAF, 2-(Acetylamino)fluorene, 2-AAF, 2-Acetamidofluorene;
2-acetylthiomethyl-3-(4-methylbenzoyl)propionic acid, 2-acetylthiomethyl-3-(4-methylbenzoyl)propionic acid, esonarimod, KE 298;
2-AG, (5Z,8Z,11Z,14Z)-Eicosatetraenoic acid, 2-hydroxy-1-(hydroxymethyl)ethyl ester, (5Z,8Z,11Z,14Z)-icosa-5,8,11,14-tetraenoic acid (2-hydroxy-1-methylol-ethyl) ester, (5Z,8Z,11Z,14Z)-icosa-5,8,11,14-tetraenoic acid [2-hydroxy-1-(hydroxymethyl)ethyl] ester;
2-amino-1-methyl-6-phenylimidazo(4,5-b)pyridine, 2-amino-1-methyl-6-phenylimidazo(4,5-b)pyridine;

2-amino-3,4-dimethylimidazo(4,5-f)quinoline, 2-amino-3,4-dimethylimidazo(4,5-f)quinoline;
2-aminoethoxydiphenyl borate, 2-aminoethoxydiphenyl borate, 2-APB compound, 2APB;
2-AP, 2-AP, 4H-1,3-Thiazin-2-amine, 5,6-dihydro-, monohydrobromide, 5,6-Dihydro-4H-1,3-thiazin-2-amine hydrobromide;
2-CADO, (2R,3R,4S,5R)-2-(6-amino-2-chloro-9-purinyl)-5-(hydroxymethyl)tetrahydrofuran-3,4-diol, (2R,3R,4S,5R)-2-(6-amino-2-chloro-purin-9-yl)-5-(hydroxymethyl)oxolane-3,4-diol, (2R,3R,4S,5R)-2-(6-amino-2-chloro-purin-9-yl)-5-(hydroxymethyl)tetrahydrofuran-3,4-diol;
2-chloro-5-nitrobenzanilide, 2-chloro-5-nitrobenzanilide, GW9662;
2-cyano-3-hydroxy-N-(4-(trifluoromethyl)phenyl)-2-hepten-6-ynamide, 2-cyano-3-hydroxy-N-(4-(trifluoromethyl) phenyl)-2-hepten-6-ynamide, 2-cyano-3-hydroxy-N-(4-(trifluoromethyl)phenyl)-2-hepten-6-ynoic acid, FK 778;
2-cyanomethylthiopyridine-4-carbonitrile, 2-(cyanomethylsulfanyl)pyridine-4-carbonitrile, 2-(cyanomethylthio)isonicotinonitrile, 2-(cyanomethylthio)pyridine-4-carbonitrile;
2-cyclopentyl-5-(5-isoquinolylsulfonyl)-6-nitro-1H-benzo(D)imidazole, 2-cyclopentyl-5-(5-isoquinolylsulfonyl)-6-nitro-1H-benzo(D)imidazole, compound-1;
2-DG, (2S,4R,5S,6R)-6-(hydroxymethyl)oxane-2,4,5-triol, (2S,4R,5S,6R)-6-(hydroxymethyl)tetrahydropyran-2,4,5-triol, (2S,4R,5S,6R)-6-methyloltetrahydropyran-2,4,5-triol;
2-hydroxy-4-(2,2,3,3,3-pentafluoropropoxy)benzoic acid, 2-hydroxy-4-(2,2,3,3,3-pentafluoropropoxy)benzoic acid, UR 1505, UR-1505;
2-hydroxyamino-1-methyl-6-phenylimidazo(4,5-b)pyridine, 2-hydroxyamino-1-methyl-6-phenylimidazo(4,5-b)pyridine;
2-hydroxyamino-3-methylimidazolo(4,5-f)quinoline, 2-hydroxyamino-3-methylimidazolo(4,5-f)quinoline;
2-ME, (17beta)-2-Methoxyestra-1,3,5(10)-triene-3,17-diol, (8R,9S,13S,14S,17S)-2-methoxy-13-methyl-6,7,8,9,11,12,14,15,16,17-decahydrocyclopenta[a]phenanthrene-3,17-diol, 1,3,5(10)-ESTRATRIEN-2,3,17-BETA-TRIOL 2-METHYL ETHER;
2-methoxyacetic acid [2-[2-[3-(1H-benzoimidazol-2-yl)propylmethyl-amino]ethyl]-6-fluoro-1-isopropyl-tetralin-2-yl] ester, 2-methoxyacetic acid [2-[2-[3-(1H-benzoimidazol-2-yl)propyl-methyl-amino]ethyl]-6-fluoro-1-isopropyl-tetralin-2-yl] ester, d020748, mibefradil;
2-methyl-1-((4-methyl-5-isoquinolinyl)sulfonyl)homopiperazine, 2-methyl-1-((4-methyl-5-isoquinolinyl)sulfonyl) homopiperazine, dimethylfasudil, H 1152;
2-N-(4-(1-azitrifluoroethyl)benzoyl)-1,3-bis-(mannos-4-yloxy)-2-propylamine, 2-N-(4-(1-azitrifluoroethyl)benzoyl)-1,3-bis-(mannos-4-yloxy)-2-propylamine, 2-N-4-(1-azi-2,2,2-trifluoroethyl)benzoyl-1,3-bis(D-mannos-4-yloxy)-2-propylamine, ATB-BMPA;
2-Naftol, .beta.-Hydroxynaphthalene, .beta.-Monoxynaphthalene, .beta.-Naftol;
2-oxothiazolidine-4-carboxylic acid, 2-oxo-4-thiazolidine carboxylic acid, 2-oxothiazolidine-4-carboxylate, 2-oxothiazolidine-4-carboxylic acid;
2-phenyl-4-oxohydroquinoline, 2-phenyl-4-oxohydroquinoline; 2,2,2-trichloroethane-1,1-diol, 1,1,1-trichloro-2,2-dihydroxyethane, 1,1,1-trichloro-2,2-ethanediol, 2,2,2-trichloro-1,1-ethanediol;
2,2'-(hydroxynitrosohydrazono)bis-ethanamine, 1-(N-(2-aminoethyl)-N-(2-ammonioethyl)amino)diazen-1-ium-1,2-diolate, 2,2'-(hydroxynitrosohydrazono)bis-ethanamine, DETA NONOate;
2,2'-azobis(2,4-dimethylvaleronitrile), 2,2'-azobis(2,4-dimethyl)valeronitrile, 2,2'-azobis(2,4-dimethylvaleronitrile), ABDVN-2,2';
2,2'-bipyridine, 2,2'-Bipyridin, 2,2'-bipyridine, 2,2'-bipyridyl;
2,2',4,4'-tetrachlorobiphenyl, 1,1'-Biphenyl, 2,2',4,4'-tetrachloro-, 2,2',4,4'-tetrachloro-1,1'-biphenyl, 2,2',4,4'-tetrachlorobiphenyl;
2,3-bis(3'-hydroxybenzyl)butane-1,4-diol, 2,3-BHBBD, 2,3-bis(3'-hydroxybenzyl)butane-1,4-diol, 2,3-bis(3-hydroxybenzyl)butane-1,4-diol;
2,3-dihydroxyterephthalamide, 2,3-DHPA, 2,3-dihydroxyterephthalamide;
2,3,4-tri-O-acetylarabinopyranosyl isothiocyanate, 2,3,4-tri-O-acetylarabinopyranosyl isothiocyanate;
2,4-diaminoquinazoline, 2,4-diaminoquinazoline;
2,4-thiazolidinedione, 2,4-thiazolidinedione, thiazolidinedione;
21-hydroxy-9beta,10alpha-pregna-5,7-diene-3-ol-20-one, 21-hydroxy-9beta,10alpha-pregna-5,7-diene-3-ol-20-one;
25-desacetylrifabutin, 25-desacetylrifabutin, LM 565, LM-565;
25-Hydroxycholesterol, (3BETA)-CHOLEST-5-ENE-3,25-DIOL, (3S,8S,9S,10R,13R,14S,17R)-17-[(1R)-5-hydroxy-1,5-dimethyl-hexyl]-10,13-dimethyl-2,3,4,7,8,9,11,12,14,15,16,17-dodecahydro-1H-cyclopenta[a]phenanthren-3-ol, (3S,8S,9S,10R,13R,14S,17R)-17-[(1R)-5-hydroxy-1,5-dimethylhexyl]-10,13-dimethyl-2,3,4,7,8,9,11,12,14,15,16,17-dodecahydro-1H-cyclopenta[a]phenanthren-3-ol;
25(OH)D3, (1S,3Z)-3-[(2E)-2-[(1R,3aS,7aR)-1-[(1R)-5-hydroxy-1,5-dimethyl-hexyl]-7a-methyl-2,3,3a,5,6,7-hexahydro-1H-inden-4-ylidene]ethylidene]-4-methylene-cyclohexan-1-ol, (1S,3Z)-3-[(2E)-2-[(1R,3aS,7aR)-1-[(1R)-5-hydroxy-1,5-dimethylhexyl]-7a-methyl-2,3,3a,5,6,7-hexahydro-1H-inden-4-ylidene]ethylidene]-4-methylene-1-cyclohexanol, (1S,3Z)-3-[(2E)-2-[(1R,3aS,7aR)-1-[(2R)-6-hydroxy-6-methyl-heptan-2-yl]-7a-methyl-2,3,3a,5,6,7-hexahydro-1H-inden-4-ylidene]ethylidene]-4-methylidene-cyclohexan-1-ol;
3-((4-(4-chlorophenyl)piperazin-1-yl)methyl)-1H-pyrrolo(2,3-b)pyridine, 3-((4-(4-chlorophenyl)piperazin-1-yl)methyl)-1H-pyrrolo(2,3-b)pyridine, CPMPP-3, L 745,870;
3-(2-hydroxy-4-(1,1-dimethylheptyl)phenyl)-4-(3-hydroxypropyl)cyclohexanol, 3-(2-hydroxy-4-(1,1-dimethylheptyl)phenyl)-4-(3-hydroxypropyl)cyclohexanol, CP 55940, CP 56667;
3-(2h)-pyridazinone, 2H-pyridazin-3-one, 3 (2H)-Pyridazinone, 3(2H)-Pyridazinone;
3-(cyclopentyloxy)-N-(3,5-dichloro-4-pyridyl)-4-methoxybenzamide, 3-(cyclopentyloxy)-N-(3,5-dichloro-4-pyridyl)-4-methoxybenzamide, 3-cyclopentyloxy-N-(3,5-dichloro-4-pyridyl)-4-methoxybenzamide, CPODPMB;
3-aminopyrazole, 3-aminopyrazole, 3-APzl;
3-beta-(2-(diethylamino)ethoxy)androst-5-en-17-one, 3-beta-(2-(diethylamino)ethoxy)androst-5-en-17-one, 3beta-(2-diethylaminoethoxy)-androst-5-en-17-one hydrochloride, U 18, 666A;
3-BHA, 2(3)-tert-Butyl-4-hydroxyanisole, 2-(1,1-Dimethylethyl)-4-methoxy-phenol, 2-Butyl-4-hydroxyanisole;
3-hydroxybutanal, 3-hydroxybutanal, 3-hydroxybutyraldehyde, acetaldol;
3-hydroxyflunitrazepam, 3-hydroxyflunitrazepam;
3-Hydroxyquinine, (3S,4R,6R)-6-[(S)-hydroxy-(6-methoxy-4-quinolyl)methyl]-3-vinyl-3-quinuclidinol, (3S,4R,6R)-6-[(S)-hydroxy-(6-methoxy-4-quinolyl)methyl]-3-vinyl-quinuclidin-3-ol, (4R,5S,7R)-5-ethenyl-7-[(S)-hydroxy-(6-methoxyquinolin-4-yl)methyl]-1-azabicyclo[2.2.2]octan-5-ol;
3-isobutyl-1-methyl-Xanthine, 3-isobutyl-1-methyl-Xanthine;
3-keto-desogestrel, 13-ethyl-17-hydroxy-11-methylene-18,19-dinor-17alpha-pregn-4-en-20-yn-3-one, 3-keto-desogestrel, 3-ketodesogestrel;
3-methoxy-4-aminoazobenzene, 3-methoxy-4-aminoazobenzene;
3-Methoxymorphinan, 3-Methoxymorphinan, Morphinan, 3-methoxy-;
3-Methoxyoestradiol, (17-beta)-3-Methoxyestra-1,3,5(10)-trien-17-ol, (17S)-3-methoxy-13-methyl-6,7,8,9,11,12,14,15,16,17-decahydrocyclopenta[a]phenanthren-17-ol, 17-beta-Estradiol 3-methyl ether;
3-methylcholanthrene, 1,2-Dihydro-3-methylbenz(j)aceanthrylene, 20-MC, 20-Methylcholanthrene;
3-MI, .beta.-Methylindole, 1H-Indole, 3-methyl-, 3-methyl-1H-indole;
3,3',4,5'-tetrahydroxystilbene, 3'-hydroxyresveratol, 3,3',4',5-tetrahydroxystilbene, 3,3',4,5'-tetrahydroxy stilbene;
3,4-DCI, 1H-2-Benzopyran-1-one, 3,4-dichloro-, 3,4-DCI, 3,4-Dcl; 3,4,5-trihydroxybenzamidoxime, 3,4,5-trihydroxybenzamidoxime, trimidox, VF 233;
4-(3-3,4-p-menthadien-(1,8)-yl)olivetol, 4-(3-3,4-p-menthadien-(1,8)-yl)olivetol, abn-cbd, abnormal cannabidiol;
4-(3-Butoxy-4-methoxybenzyl)-2-imidazolidinone, 4-(3-Butoxy-4-methoxybenzyl)-2-imidazolidinone, Ro 1724, Ro 20 1724;
4-(4-(4-chlorophenyl)-4-hydroxy-1-piperidinyl)-1-(4-fluorophenyl)-1-butanol, 4-(4-(4-chlorophenyl)-4-hydroxy-1-piperidinyl)-1-(4-fluorophenyl)-1-butanol, hydroxyhaloperidol, reduced haloperidol;
4-(4-(N-benzoylamino)anilino)-6-methoxy-7-(3-(1-morpholino)propoxy)quinazoline, 4-(4-(N-benzoylamino)anilino)-6-methoxy-7-(3-(1-morpholino)propoxy)quinazoline, ZM 447439, ZM-447439;
4-(4-fluorophenyl)-2-(4-hydroxyphenyl)-5-(4-pyridyl)imidazole, 4-(4-fluorophenyl)-2-(4-hydroxyphenyl)-5-(4-pyridyl)imidazole, SB 202190, SB-202190;
4-(5-benzo(1,3)dioxol-5-yl-4-pyridin-2-yl-1H-imidazol-2-yl)benzamide, 4-(5-benzo(1,3)dioxol-5-yl-4-pyridin-2-yl-1H-imidazol-2-yl)benzamide, SB 431542, SB-431542;
4-(benzodioxan-5-yl)-1-(indan-2-yl)piperazine, 4-(benzodioxan-5-yl)-1-(indan-2-yl)piperazine, S 15535, S-15535;
4-(N-methyl-N-nitrosamino)-1-(3-pyridyl)-1-butanone, 4-(N-methyl-N-nitrosamino)-1-(3-pyridyl)-1-butanone;
4-AP, .gamma.-Aminopyridine, 4-Aminopyridine, 4-Aminopyridine 10;
4-azidosalicylic acid, 4-AzHBA, 4-azido-2-hydroxybenzoic acid, 4-azidosalicylic acid;
4-dimethylamino-3',4'-dimethoxychalcone, 4-dimethylamino-3',4'-dimethoxychalcone, CH 11;
4-hydroxy-N-desmethyltamoxifen, 4-hydroxy-N-demethyltamoxifen, 4-hydroxy-N-desmethyltamoxifen, 4-hydroxy-N-desmethyltamoxifen, (Z)-isomer;
4-hydroxyacetophenone, 1-(4-hydroxyphenyl)ethanone, 4-hydroxyacetophenone, p-hydroxyacetophenone;
4-hydroxycoumarin, 4-hydroxycoumarin;
4-hydroxyestradiol-17 beta, 4-hydroxyestradiol, 4-hydroxyestradiol-17 alpha, 4-hydroxyestradiol-17 beta;
4-hydroxynon-2-enal, 4-Hydroxy-2,3-nonenal, 4-Hydroxy-2-nonenal, 4-hydroxynon-2-enal;
4-hydroxytriazolam, 4-hydroxytriazolam;
4-methyl-N-(3-(4-methylimidazol-1-yl)-5-(trifluoromethyl)phenyl)-3-((4-pyridin-3-ylpyrimidin-2-yl)amino)benzamide, 4-methyl-N-(3-(4-methylimidazol-1-yl)-5-(trifluoromethyl)phenyl)-3-((4-pyridin-3-ylpyrimidin-2-yl)amino)benzamide, AMN107, nilotinib;

4-phenylbutyric acid, 4-phenylbutyrate, 4-phenylbutyric acid, 4-phenylbutyric acid, calcium salt;
4-S-cysteaminylphenol, 4-S-cysteaminylphenol;
4-sulfophenylmethallyl ether, 4-sulfophenylmethallyl ether, 4-sulfophenylmethallyl ether, sodium salt, SPME;
4,4'-DDE, 1,1'-(2,2-dichloroethene-1,1-diyl)bis(4-chlorobenzene), 1,1'-(Dichloroethenylidene)bis(4-chlorobenzene), 1,1'-(Dichloroethenylidene)bis[4-chlorobenzene];
4,4'-dipyridyl disulfide, 4,4'-dipyridine disulfide, 4,4'-dipyridyl disulfide, 4,4'-dithiodipyridine;
4,8-dimethoxy-7-hydroxyfuro(2,3-b)quinoline, 4,8-dimethoxy-7-hydroxyfuro(2,3-b)quinoline, haplopine;
4'-epidoxorubicin, (1S,3S)-3,5,12-trihydroxy-3-(hydroxyacetyl)-10-methoxy-6,11-dioxo-1,2,3,4,6,11-hexahydrotetracen-1-yl 3-amino-2,3,6-trideoxy-alpha-L-arabino-hexopyranoside, 4'-Epiadriamycin, 4'-epidoxorubicin;
4'-N-benzoylstaurosporine, 4'-N-benzoyl staurosporine, 4'-N-benzoylstaurosporine, benzoylstaurosporine;
4(2'-aminoethyl)amino-1,8-dimethylimidazo(1,2-a)quinoxaline, 4(2'-aminoethyl)amino-1,8-dimethylimidazo(1,2-a)quinoxaline, BMS-345541, BMS345541;
4alpha-phorbol 12,13-didecanone, 4alpha-phorbol 12,13-didecanone;
4alphaPDD, 4-.alpha.-Phorbol 12,13-didecanoate, 4-.alpha.-Phorbol didecanoate, 4-alpha-Phorbol 12,13-didecanoate; 5-((1,2-dihydro-2-oxo-3H-indol-3-ylidene)methyl)-2,4-dimethyl-1H-pyrrole-3-propanoic acid, 5-((1,2-dihydro-2-oxo-3H-indol-3-ylidene)methyl)-2,4-dimethyl-1H-pyrrole-3-propanoic acid, TSU 68, TSU-68;
5-(4'-(N-piperidinyl)phenylazo)indazole, 5-(4'-(N-piperidinyl)phenylazo)indazole;
5-7-oxo-zeaenol, 5-7-oxo-zeaenol, 7-oxozeaenol;
5-AC, 1,3,5-Triazin-2(1H)-one, 4-amino-1-beta-D-ribofuranosyl-, 2-(beta-D-Ribofuranosyl)-4-amino-1,3,5-triazin-2-one, 4-Amino-1-(beta-D-ribofuranosyl)-1,3,5-triazin-2(1H)-one;
5-acetylneuraminyl-(2-3)-galactosyl-(1-4)-(fucopyranosyl-(1-3))-N-acetylglucosamine, 5-acetylneuraminyl-(2-3)-galactosyl-(1-4)-(fucopyranosyl-(1-3))-N-acetylglucosamine;
5-azido-1H-indole-3-acetic acid, 5-AIAA, 5-azido-(7-3H)-indol-3-ylacetic acid, 5-azido-1H-indole-3-acetic acid;
5-HT, 1H-Indol-5-ol, 3-(2-aminoethyl)-, 3-(2-aminoethyl)-1H-indol-5-ol, 3-(2-Aminoethyl)indol-5-ol;
5-methoxy-N,N-diisopropyltryptamine, 5-MeO-DIPT, 5-methoxy-N,N-bis(1-methylethyl)-1H-indole-3-ethanamine, 5-methoxy-N,N-diisopropyltryptamine;
5-Mop, 4-methoxy-7-furo[3,2-g]chromenone, 4-Methoxy-7H-furo(3,2-g) (1)benzopyran-7-one, 4-methoxy-7H-furo[3,2-g]chromen-7-one; 5,10-methylenetetrahydrofolate, 5,10-methylene-5,6,7,8-tetrahydrofolate, 5,10-methylenetetrahydrofolate, 5,10-methylenetetrahydrofolate monohydrochloride, (L-Glu)-isomer; 5,6-dimethylxanthenoneacetic acid, 5,6-dimethyl xanthenone acetic acid, 5,6-dimethyl-9-oxo-9H-xanthene-4-acetic acid, 5,6-dimethylxanthenone-4-acetic acid;
5'-O-(((2-decanoylamino-3-phenylpropyloxycarbonyl)amino)sulfonyl)uridine, 5'-O-(((2-decanoylamino-3-phenylpropyloxycarbonyl)amino)sulfonyl)uridine, PP55;
6 beta-hydroxycortisol, 6 beta-hydroxycortisol, 6beta-hydroxycortisol;
6-Aminochrysene-1,2-dihydrodiol, (1S,2S)-6-amino-1,2-dihydrochrysene-1,2-diol, 1,2-Chrysenediol, 1,2-dihydro-6-amino-, trans-, 6-Aminochrysene-1,2-dihydrodiol;
6-chloro-2-pyridylmethyl nitrate, 6-chloro-2-pyridylmethyl nitrate;
6-deoxy-6-bromoascorbic acid, 6-bromo-6-deoxy-ascorbic acid, 6-bromo-6-deoxy-L-ascorbic acid, 6-bromo-6-deoxyascorbic acid;
6-hydroxydexamethasone, 6-hydroxydexamethasone;
6-Mercaptopurine, 1,7-Dihydro-6H-purine-6-thione, 6 Mercaptopurine, 6 Mercaptopurine Monohydrate;
6,6'-oxybis(2,2-dimethylhexanoic acid), 6,6'-oxybis(2,2-dimethylhexanoic acid), gemcabene, PD 72953;
64Gd, 64Gd, gadolinio, gadolinium;
7-(1,1-dimethylethyl)-6-(2-ethyl-2H-1,2,4-triazol-3-ylmethoxy)-3-(2-fluorophenyl)-1,2,4-triazolo(4,3-b)pyridazine, 7-(1,1-dimethylethyl)-6-(2-ethyl-2H-1,2,4-triazol-3-ylmethoxy)-3-(2-fluorophenyl)-1,2,4-triazolo(4,3-b)pyridazine, TPA 023, TPA-023;
7-benzylamino-6-chloro-2-piperazino-4-pyrrolidinopteridine, 7-benzylamino-6-chloro-2-piperazino-4-pyrrolidinopteridine;
7-benzyloxyquinoline, 7-benzyloxyquinoline;
7-CDL, (2S,4R)-N-[2-chloro-1-[(2R,3R,4S,5R,6R)-3,4,5-trihydroxy-6-(methylthio)-2-tetrahydropyranyl]propyl]-1-methyl-4-propyl-2-pyrrolidinecarboxamide, (2S,4R)-N-[2-chloro-1-[(2R,3R,4S,5R,6R)-3,4,5-trihydroxy-6-(methylthio)tetrahydropyran-2-yl]propyl]-1-methyl-4-propyl-pyrrolidine-2-carboxamide, (2S,4R)-N-[2-chloro-1-[(2R,3R,4S,5R,6R)-3,4,5-trihydroxy-6-methylsulfanyl-oxan-2-yl]propyl]-1-methyl-4-propyl-pyrrolidine-2-carboxamide;
7-hydroxystaurosporine, 7-hydroxy-staurosporine, 7-hydroxystaurosporine, UCN 01;
7-ketocholesterol, 7-ketocholesterol;
7,8-BF, .alpha.-Naphthoflavone, .alpha.-Naphthylflavone, 2-phenyl-4-benzo[h]chromenone;
7,8-dihydroneopterin, 7,8-dihydro-neopterin, 7,8-dihydroneopterin;
7'-Isothiocyanato-11-hydroxy-1',1'-dimethylheptylhexahydrocannabinol, 7'-Isothiocyanato-11-hydroxy-1',1'-dimethylheptylhexahydrocannabinol, AM841;
7C3MT, (2R,3R)-3,5,7-trihydroxy-2-[(2R,3R)-2-(4-hydroxy-3-methoxy-phenyl)-3-(hydroxymethyl)-2,3-dihydro-1,4-benzodioxin-7-yl]chroman-4-one, (2R,3R)-3,5,7-trihydroxy-2-[(2R,3R)-2-(4-hydroxy-3-methoxy-phenyl)-3-methylol-2,3-dihydro-1,4-benzodioxin-7-yl]chroman-4-one, (2R,3R)-3,5,7-trihydroxy-2-[(2R,3R)-2-(4-hydroxy-3-methoxy-phenyl)-3-(hydroxymethyl)-2,3-dihydro-1,4-benzodioxin-7-yl]-4-chromanone;
7H-Pyrrolo(2,3-d)pyrimidine, 7H-Pyrrolo(2,3-d)pyrimidine; 8-((4-bromo-2,3-dioxobutyl)thio)-adenosine 3',5'-cyclic monophosphate, 8-((4-bromo-2,3-dioxobutyl)thio)-adenosine 3',5'-cyclic monophosphate;
8-(2,6-dichlorophenyl)-10-methyl-3-((4-morpholin-4-ylphenyl)amino)-2,4,10-triazabicyclo(4.4.0)deca-1,3,5,7-tetraen-9-one, 8-(2,6-dichlorophenyl)-10-methyl-3-((4-morpholin-4-ylphenyl)amino)-2,4,10-triazabicyclo(4.4.0)deca-1,3,5,7-tetraen-9-one, PD0173952;
8-(3-chlorostyryl)caffeine, 8-(3-chlorostyryl)caffeine; 8-anilinonaphthalene-1-sulfonic acid, 1-(phenylamino)-8-naphthalenesulfonic acid, 1-Anilino-8-naphthalenesulfonate, 1-anilino-8-naphthalenesulfonic acid;
8-Hydroxy-2-(di-n-propylamino)tetralin, 8-Hydroxy-2-(di-n-propylamino)tetralin, 8-Hydroxy-2-(di-n-propylamino)tetralin Hydrobromide, 8-Hydroxy-2-(di-n-propylamino)tetralin Hydrobromide, (+-)-Isomer;
8-Isoprostane, (5Z,13E,15S)-9alpha,11alpha,15-trihydroxy-8beta-prosta-5,13-dien-1-oic acid, (Z)-7-[(1S,2R,3R,5S)-3,5-dihydroxy-2-[(E,3S)-3-hydroxyoct-1-enyl]cyclopentyl]hept-5-enoic acid, 8-Epi PGF-2alpha;
8,10-bis((2,2-dimethyl-1-oxopropyl)oxy)-11-methyl-1234-tetrahydro-6H-benzo(beta)quinolizin-6-one, 8,10-bis((2,2-dimethyl-1-oxopropyl)oxy)-11-methyl-1234-tetrahydro-6H-benzo(beta)quinolizin-6-one, Sch35966;
9-(4'-aminophenyl)-9H-pyrido(3,4-b)indole, 9-(4'-aminophenyl)-9H-pyrido(3,4-b)indole, aminophenylnorharman, APNH cpd;
9-anthroic acid, 9-anthracenecarboxylic acid, 9-anthroic acid, 9-carboxyanthracene;
9-CRA, (2E,4E,6Z,8E)-3,7-dimethyl-9-(2,6,6-trimethyl-1-cyclohexenyl)nona-2,4,6,8-tetraenoic acid, 9 Cis Retinoic Acid, 9-cis-RA;
9-hydroxy-risperidone, 3-(2-(4-(6-fluoro-3-(1,2-benzisoxazolyl))-1-piperidinyl)ethyl)-6,7,8,9-tetrahydro-9-hydroxy-2-methyl-4H-pyrido(1,2-a)pyrimidin-4-one, 9-hydroxy-risperidone, 9-hydroxyrisperidone;
9,10-anthraquinone, 9,10-Anthracendion, 9,10-anthracenedione, 9,10-Anthrachinon;
9H-xanthene, 10H-9-oxaanthracene, 9H-xanthene, C1c2ccccc2Oc3ccccc13;

A 71915, A 71915;
A-300-I, (2E,4E,6R)-7-(4-(dimethylamino)phenyl)-N-hydroxy-4,6-dimethyl-7-oxo-2,4-heptadienamide, (2E,4E,6R)-7-(4-dimethylaminophenyl)-4,6-dimethyl-7-oxo-hepta-2,4-dienehydroxamic acid, (2E,4E,6R)-7-(4-dimethylaminophenyl)-4,6-dimethyl-7-oxohepta-2,4-dienehydroxamic acid;
a-ADP, (S)-1-C-(7-Amino-1H-pyrazolo(4,3-d)pyrimidin-3-yl)-1,4-anhydro-D-ribitol, 5-(trihydrogen diphosphate), 5'-Adenylphosphoric acid, 5'-ADP;
A73025, A73025;
Abbott, 1,2,4-Triazolidine-3,5-dione, 1,2-bis(1-methylethyl)-, 1,2,4-Triazolidine-3,5-dione, 1,2-bis(1-methylethyl)-(9CI), 1,2,4-Triazolidine-3,5-dione, 1,2-diisopropyl-;
abciximab, abciximab, c7E3 Fab, CentoRx;
Absele, (+)-Ethyl 1-(alpha-methylbenzyl)imidazole-5-carboxylate, (+)-Etomidate, (d)-Etomidate;
ABT-737, ABT-737;
acetamide 45, acetamide 45, acetamide-45;
Aceton, .beta.-Ketopropane, 2-Propanone, Aceton;
acetonitrile, acetonitrile, CC#N, CH3-C#N;
acetyl-11-ketoboswellic acid, 3-O-acetyl-11-keto-boswellic acid, acetyl-11-keto-beta-boswellic acid, acetyl-11-ketoboswellic acid;
acetylcholine, 2-acetyloxy-N,N,N-trimethylethanaminium, acetylcholine, ACh;
acetylvalerenolic acid, acetylvalerenolic acid;
Aclarubicin, Aclacin, Aclacinomycin A, Aclaplastin;
Acolen, (4R)-4-[(5S,8R,9S,10S,13R,14S,17R)-10,13-dimethyl-3,7,12-trioxo-1,2,4,5,6,8,9,11,14,15,16,17-dodecahydrocyclopenta[a]phenanthren-17-yl]pentanoic acid, (4R)-4-[(5S,8R,9S,10S,13R,14S,17R)-3,7,12-triketo-10,13-dimethyl-1,2,4,5,6,8,9,11,14,15,16,17-dodecahydrocyclopenta[a]phenanthren-17-yl]valeric acid, (5beta)-3,7,12-trioxocholan-24-oic acid;
ACON, (2E,4E,6E,8E)-3,7-dimethyl-9-(2,6,6-trimethyl-1-cyclohexenyl)nona-2,4,6,8-tetraen-1-ol, (2E,4E,6E,8E)-3,7-dimethyl-9-(2,6,6-trimethylcyclohex-1-en-1-yl)nona-2,4,6,8-tetraen-1-ol, (all-E)-3,7-Dimethyl-9-(2,6,6-trimethyl-1-cyclohexen-1-yl)-2,4,6,8-nonatetraen-1-ol;
ACT D, Err:508, 10,10'-[(2-Amino-4,6-dimethyl-3-oxo-3H-phenoxazine-1,9-Diyl)bis(carbonylimino)]bis-[dodecahydro-6,13-diisopropyl-2,5,9-trimethyl-1H-Pyrrolo-(2,1-1)(1,4,7,10,13)oxatetra-azacyclohexadecine]-1,4,7,11,14, 1H-Pyrrolo(2,1-1)-(1,4,7,10,13)oxatetraazacyclohexadecine;

actinium, 89Ac, actinio, actinium;
Actosin, 2-methoxy-2-methyl-4-phenyl-3,4-dihydropyrano[5,6-c]chromen-5-one, 2H,5H-Pyrano(3,2-c)(1)benzopyran-5-one, 3,4-dihydro-2-methoxy-2-methyl-4-phenyl-, 2H,5H-Pyrano[3,2-c][1]benzopyran-5-one, 3,4-dihydro-2-methoxy-2-methyl-4-phenyl-; adalimumab, Abbott brand of adalimumab, adalimumab, D2E7 antibody;
Adalin, (.alpha.-Bromo-.alpha.-ethylbutyryl)carbamide, (.alpha.-Bromo-.alpha.-ethylbutyryl)urea, (2-Brom-2-ethylbutyryl)harnstoff;
Adanon, (+)-Methadone,
(+-)-Methadone, (-)-(R)-6-(Dimethylamino)-4,4-diphenyl-3-heptanone;
Adfeed, (-)-(2R)-2-(2-fluorobiphenyl-4-yl)propanoic acid, (-) - Flurbiprofen, (2R)-2-(2-Fluoro-1,1'-biphenyl-4-yl)propanoic acid;
adinazolam, adinazolam;
Adofeed, (C3-C24) Fatty acids, 2-arylpropanoic acid, 2-Arylpropionate;
Adrenor, (−)-Norepinephrine, (-)-(R)-Norepinephrine, (-)-alpha-(Aminomethyl)protocatechuyl alcohol;
Adrin, (+)-3,4-dihydroxy-alpha-((methylamino)methyl)benzyl alcohol, (+)-adrenaline, (-)-Adrenaline;
AEBSF, 4-(2-Aminoethyl)benzenesulfonyl fluoride, 4-(2-Aminoethyl)benzenesulfonylfluoride, 4-beta-Aminoethylbenzolsulfofluoride;
Aeromax, (+-)-4-Hydroxy-alpha'-(((6-(4-phenylbutoxy)hexyl)amino)methyl)-m-xylene-alpha,alpha'-diol, (+-)-4-Hydroxy-alpha1-(((6-(4-phenylbutoxy)hexyl)amino)methyl)-1,3-benzenedimethanol, 1,3-Benzenedimethanol, 4-hydroxy-alpha(sup 1)-(((6-(4-phenylbutoxy)hexyl)amino)methyl)-, (+-) -;
afloqualone, afloqualone;
AGMATINE, (4-Aminobutyl) guanidine, (4-AMINOBUTYL)GUANIDINE, (4-Aminobutyl)guanidinium sulphate;
AIDSVAX, AIDSVAX, ALVAC-HIV, vCP1521;
ajoene, 4,5,9-trithiadodeca-1,6,11-triene 9-oxide, ajoene; ajulemic acid, ajulemic acid;
alachlor, 2-chloro-N-(2,6-diethylphenyl)-N-(methoxymethyl)acetamide, alachlor;
Aladerm, 5-amino-4-keto-valeric acid, 5-amino-4-oxo-pentanoic acid, 5-Amino-4-oxopentanoate;
alaninate, 2-aminopropanoate, alaninate, alanine anion;
Alat, 1,4-Dihydro-2,6-dimethyl-4-(2-nitrophenyl)-3,5-pyridinedicarboxylic acid dimethyl ester, 2,6-Dimethyl-3,5-dicarbomethoxy-4-(2-nitrophenyl)-1,4-dihydropyridine, 2,6-dimethyl-4-(2-nitrophenyl)-1,4-dihydropyridine-3,5-dicarboxylic acid dimethyl ester;
Alcolo, 1-Hydroxy-1-methylethyl, 1-Methylethanol, 1-Methylethyl alcohol;
Alcuronium, Alcuronium, Alcuronium Chloride, Alcuronium Dichloride;
Aldara, (1-isobutylimidazo[4,5-c]quinolin-4-yl)amine, 1-(2-Methylpropyl)-1H-imidazole[4,5-c]quinoline-4-amine, 1-(2-methylpropyl)-1H-imidazo[4,5-c]quinolin-4-amine;
ALDO, .gamma.-[4-(p-Chlorphenyl)-4-hydroxpiperidino]-p-fluorbutyrophenone, 1-(3-p-Fluorobenzoylpropyl)-4-p-chlorophenyl-4-hydroxypiperidine, 1-Butanone, 4-(4-(4-chlorophenyl)-4-hydroxy-1-piperidinyl)-1-(4-fluorophenyl)-;
Aldrich, 1,3-di-O-methylpyrogallol, 1,3-dimethoxy-2-hydroxybenzene, 1,3-dimethyl pyrogallate;
alemtuzumab, alemtuzumab, Berlex brand of alemtuzumab, Campath; Alfarol, (1S,3R,5Z)-5-[(2E)-2-[(1R,7aR)-1-[(1R)-1,5-dimethylhexyl]-7a-methyl-2,3,3a,5,6,7-hexahydro-1H-inden-4-ylidene]ethylidene]-4-methylene-cyclohexane-1,3-diol, (1S,3R,5Z)-5-[(2E)-2-[(1R,7aR)-1-[(1R)-1,5-dimethylhexyl]-7a-methyl-2,3,3a,5,6,7-hexahydro-1H-inden-4-ylidene]ethylidene]-4-methylenecyclohexane-1,3-diol, (1S,3R,5Z)-5-[(2E)-2-[(1R,7aR)-7a-methyl-1-[(2R)-6-methylheptan-2-yl]-2,3,3a,5,6,7-hexahydro-1H-inden-4-ylidene]ethylidene]-4-methylidene-cyclohexane-1,3-diol;
Alfentanil, Alfenta, Alfentanil, Alfentanil Hydrochloride;
ALIMTA, ALIMTA;
aliskiren, 2(S),4(S),5(S),7(S)-N-(2-carbamoyl-2-methylpropyl)-5-amino-4-hydroxy-2,7-diisopropyl-8-(4-methoxy-3-(3-methoxypropoxy)phenyl)octanamid hemifumarate, aliskiren, CGP 060536B;
Alli, (−)-Tetrahydrolipstatin, (-)-Tetrahydrolipstatin, (2S)-2-formamido-4-methyl-valeric acid [(1S)-1-[[(2S,3S)-3-hexyl-4-keto-oxetan-2-yl]methyl]dodecyl] ester;
ALLN, (2S)-2-[(2S)-2-acetamido-4-methylpentanamido]-N-[(1S)-1-formylpentyl]-4-methylpentanamide, (2S)-2-[[(2S)-2-acetamido-4-methyl-1-oxopentyl]amino]-N-[(1S)-1-formylpentyl]-4-methylpentanamide, (2S)-2-[[(2S)-2-acetamido-4-methyl-pentanoyl]amino]-4-methyl-N-[(2S)-1-oxohexan-2-yl]pentanamide;
alloxazine, Alloxazin, alloxazine, benzo[g]pteridine-2,4(1H,3H)-dione;
allyl isothiocyanate, allyl isothiocyanate;
almokalant, almokalant;
aloesin, aloesin;
Alprenolol, 1-(o-Allylphenoxy)-3-(isopropylamino)-2-propanol, Alfeprol, Alpheprol;
Alvesco, Alvesco, Ciclesonide;
AM 1387, AM 1387, AM-1387, AM1387;

AM 251, AM 251, AM-251, AM251;
Am 80, 4-((5,6,7,8-tetrahydro-5,5,8,8-tetramethyl-2-naphthalenyl)carbamoyl)benzoic acid, Am 80, AM-80;
AMD 070, AMD 070, AMD-070, AMD070;
Amiloride, Alphapharm Brand of Amiloride Hydrochloride, Amidal, Amiduret Trom;
Aminacrine, 9 Aminoacridine, 9-Aminoacridine, Acridinamine; Amine BB, (C12-18)Qalkylamine, (C12-C18) Alkylamine, 1-Aminododecane;
amino-polyethyleneoxide-sulfonate, amino-polyethyleneoxide-sulfonate, NH2-PEO-SO3, PEO-SO3;
aminoflavone, 5-amino-2-(4-amino-3-fluorophenyl)-6,8-difluoro-7-methyl-4H-1-benzopyran-4-one, aminoflavone, NSC 686288;
Amiodarone, (2-butyl-1-benzofuran-3-yl)(4-{[2-(diethylamino)ethyl]oxy}-3,5-diiodophenyl)methanone, (2-butyl-1-benzofuran-3-yl)-[4-(2-diethylaminoethoxy)-3,5-diiodo-phenyl]methanone, (2-butyl-1-benzofuran-3-yl)-[4-(2-diethylaminoethoxy)-3,5-diiodophenyl]methanone;
Amlodipine, Almirall Brand of Amlodipine Besilate, Amlodipine, Amlodipine Besylate;
Amphotericin B, Amphocil, Amphotericin, Amphotericin B; amprenavir, 141W94, Agenerase, amprenavir;
Amrinone, 5-Amino-(3,4'-bipyridine)-6(1H)-one, Amrinon, Amrinone;
amsonic acid, 4,4'-diacetamido-2,2'-stilbene disulfonate, 4,4-diaminostilbene-2,2'-disulfonic acid, amsonic acid;
Amygdalin, Amygdalin, Amygdaloside, Laetrile;
AN 207, AN 207;
Anaboleen, (5a,17b)-17-Hydroxyandrostan-3-one, (5alpha,17beta)-17-hydroxyandrostan-3-one, (5S,8R,9S,10S, 13S,14S,17S)-17-hydroxy-10,13-dimethyl-1,2,4,5,6,7,8,9,11,12,14,15,16,17-tetradecahydrocyclopenta[a]phenanthren-3-one;
anacardic acid, 6-(8(Z),11(Z),14-pentadecatrienyl)salicylic acid, 6-(8,11,14-pentadecatrienyl)salicylic acid, 6-nonadecyl salicylic acid;
Anandamide, (5Z,8Z,11Z,14Z)- N-(2-Hydroxyethyl)- 5,8,11,14-eicosatetraenamide, (5Z,8Z,11Z,14Z)-N-(2-hydroxyethyl)-5,8,11,14-eicosatetraenamide, (5Z,8Z,11Z,14Z)-N-(2-hydroxyethyl)icosa-5,8,11,14-tetraenamide;
Anco, ( -)-Ibuprofen, (+-)-2-(p-isobutylphenyl)propionic acid, (+-)-alpha-methyl-4-(2-methylpropyl)benzeneacetic acid;
Andrographis, (3E,4S)-3-[2-[(1R,4aS,5R,6R,8aS)-6-hydroxy-5,8a-dimethyl-2-methylene-5-methylol-decalin-1-yl] ethylidene]-4-hydroxy-tetrahydrofuran-2-one, (3E,4S)-3-[2-[(lR,4aS,5R,6R,8aS)-6-hydroxy-5-(hydroxymethyl)-5,8a-dimethyl-2-methylene-1-decalinyl]ethylidene]-4-hydroxy-2-tetrahydrofuranone, (3E,4S)-3-[2-[(1R,4aS,5R,6R, 8aS)-6-hydroxy-5-(hydroxymethyl)-5,8a-dimethyl-2-methylene-decalin-1-yl]ethylidene]-4-hydroxy-tetrahydrofuran-2-one;
Androtine, (3alpha,5alpha)-3-hydroxyandrostan-17-one, (3R,5S,8R,9S,10S,13S,14S)-3-hydroxy-10,13-dimethyl-1,2,3,4,5,6,7,8,9,11,12,14,15,16-tetradecahydrocyclopenta[a]phenanthren-17-one, 3-alpha-Hydroxy-17-androstanone;
Aneol, (+-)-3-Benzoyl-alpha-methylbenzeneacetic acid, 19583 RP, 2-(3-benzoylphenyl)propanoic acid;
Ang II, (3S)-3-amino-4-[[(1S)-1-[[(1S)-1-[[(1S)-2-[[(1S,2S)-1-[[(1S)-2-[(2S)-2-[[(1S)-1-(benzyl)-2-hydroxy-2-keto-ethyl]carbamoyl]pyrrolidin-1-yl]-1-(3H-imidazol-4-ylmethyl)-2-keto-ethyl]carbamoyl]-2-methyl-butyl]amino]-1-(4-hydroxybenzyl)-2-keto-ethyl]carbamoyl], (3S)-3-amino-4-[[(1S)-4-guanidino-1-[[(1S)-1-[[(1S)-2-[[(1S,2S)-1-[[(1S)-2-[(2S)-2-[[(1S)-2-hydroxy-2-oxo-1-(phenylmethyl)ethyl]carbamoyl]pyrrolidin-1-yl]-1-(3H-imidazol-4-ylmethyl)-2-oxo-ethyl]carbamoyl]-2-methyl-butyl]amino]-1-[(4-hydroxyphenyl)methyl]-, (3S)-3-amino-4-[[(1S)-4-guanidino-1-[[[(1S)-1-[[[(1S)-2-[[(1S,2S)-1-[[[(1S)-2-[(2S)-2-[[[(1S)-2-hydroxy-2-oxo-1-(phenylmethyl)ethyl]amino]-oxomethyl]-1-pyrrolidinyl]-1-(3H-imidazol-4-ylmethyl)-2-oxoethyl]amino]-oxomethyl]-2-methylbutyl]amino]-1-[(4-hydrox;
Anisomycin, Anisomycin, Flagecidin;
Anon, Anon, Anone, Cicloesanone;
Anthocyanins, Anthocyanidin, Anthocyanidins, Anthocyanin;
anthra(1,9-cd)pyrazol-6(2H)-one, anthra(1,9-cd)pyrazol-6(2H)-one, SP600125;
anthracene, anthracene, Anthrazen, c1ccc2cc3ccccc3cc2c1;
anthralin, 1,8-dihydroxy-9(10H)-anthracenone, 1,8-dihydroxy-9-anthrone, 1,8-dihydroxyanthracen-9(10H)-one;
Anthricin, (-)-Anthricin, (-)-Deoxypodophyllotoxin, (-)-Desoxypodophyllotoxin;
anthrone, 9(10H)-anthracenone, 9,10-dihydro-9-oxoanthracene, anthracen-9(10H)-one;
antibiotic G 418, antibiotic G 418;
antibiotic H107, antibiotic H107, H 107;
Antimycin A, Antimycin A, Antimycin A1;
Anyvim, Aminobenzene, Aminophen, Anilin;
APAP, 222 AF, 4'-Hydroxyacetanilide, 4-(Acetylamino)phenol;
APDC, 1-Pyrrolidinecarbodithioic acid, 1-Pyrrolidinecarbodithioic acid & Z-100, Ammonium pyrrolidine dithiocarbamate;

Aphidicolin, Aphidicolin, Aphidicolin, (3-S-(3alpha,4beta,4abeta,6aalpha,8alpha,9alpha,11aalpha,11balpha))-Isomer, ICI 69653;
Aphloiol, 1,3,6,7-tetrahydroxy-2-[(3R,4R,5S,6R)-3,4,5-trihydroxy-6-(hydroxymethyl)-2-tetrahydropyranyl]-9-xanthenone, 1,3,6,7-tetrahydroxy-2-[(3R,4R,5S,6R)-3,4,5-trihydroxy-6-(hydroxymethyl)oxan-2-yl]xanthen-9-one, 1,3,6,7-tetrahydroxy-2-[(3R,4R,5S,6R)-3,4,5-trihydroxy-6-(hydroxymethyl)tetrahydropyran-2-yl]xanthen-9-one;
apicidin, apicidin, apicidin C, cyclo(N-O-methyl-tryptophyl-isoleucyl-pipecolinyl-2-amino-8-oxodecanoyl);
Apigenin, 2-(p-hydroxyphenyl)-5,7-dihydroxychromone, 4′,5,7-Trihydroxyflavone, 4′,5,7-Trihydroxyflavone; apocynin, 1-(4-hydroxy-3-methoxyphenyl)ethan-1-one, 4'-hydroxy-3'-methoxyacetophenone, Acetovanillone;
Apotransferrin, Apotransferrin;
aprepitant, (2R)-(1R)-3,5-bis(trifluoromethylphenyl)ethoxy)-(3S)-(4-fluoro)phenyl-4-(3-(5-oxo-1H,4H-1,2,4-triazole) methyl-morpholine, aprepitant, Emend;
APRL, 1-ethyl-2-acetyl-sn-glycero-3-phosphocholine, 1-O-Alkyl-2-acetyl-sn-glycero-3-phophocholine, 1-O-Alkyl-2-acetyl-sn-glyceryl-3-phosphorylcholine;
AQ4N, 1,4-bis-((2-(dimethylamino)ethyl)amino)-5,8-dihydroxyanthracene-9,10-dione, 1,4-bis-((2-(dimethylamino-N-oxide)ethyl)amino)-5,8-dihydroxyanthracene-9,10-dione, AQ4N;
arabinogalactan, arabinogalactan;
Arac, Arac;
Aralen, ( -)-Chloroquine, (+)-Chloroquine, (+)-N4-(7-Chloro-4-quinolinyl)-N1,N1-diethyl-1,4-pentanediamine;
Arasine, 1H-Imidazole-4-carboxamide, 5-amino-1-beta-D-ribofuranosyl-, 5-Amino-1-.beta.-D-ribofuranosyl-1H-imidazole-4-carboxamide, 5-Amino-1-beta-D-ribofuranosyl-1H-imidazole-4-carboxamide;
Areca, Areca, Areca catechu, Betel Nut;
Arecoline, Arecaline, Arecholin, Arecholine;
Areether, 109716-83-8 (UNSPECIFIED 12 POSITION), 3,12-Epoxy-12H-pyrano(4,3-j)-1,2-benzodioxepin, 10-ethoxydecahydro-3,6,9-trimethyl-, (3S-(3alpha,5alpha,6alpha,8aalpha,9beta,10beta,12beta,12aalpha))-, 3,12-Epoxy-12H-pyrano(4,3-j)-1,2-benzodioxepin, decahydro-10-ethoxy-3,6,9-trimethyl-, (3R-(3alpha,5abeta,6beta, 8abeta,9alpha,10alpha,12beta,12aR*))-;
argatroban, 4-methyl-1-(N(2)-(3-methyl-1,2,3,4-tetrahydro-8-quinolinesulfonyl)-L-arginyl)-2-piperidinecarboxylic acid, Acova, argatroban;
aripiprazole, 7-(4-(4-(2,3-dichlorophenyl)-1-piperazinyl)butyloxy)-3,4-dihydro-2(1H)-quinolinone, Abilify, aripiprazole;
Aron, 2-Carboxyethyl acrylate oligomers, 2-Propenoic acid, 2-Propenoic acid, homopolymer;
Artein, (1S,3R,7S,8S,8aR)-1,2,3,7,8,8a-Hexahydro-3,7-dimethyl-8-(2-(2R,4R)-(tetrahydro-4-hydroxy-6-oxo-2H-pyran-2-yl)ethyl)-1-naphthalenyl (S)-2-methyl-butyrate, (1S-(1alpha(R),3alpha,7beta,8beta(2S,4S), 8abeta))-(1,2,3,7,8,8a-hexahydro-3,7-dimethyl-8-(2-(tetrahydro-4-hydroxy-6-oxo-2H-pyran-2-yl)ethyl)-1-naphthyl) 2-methylbutanoate, (1S-(1alpha(R*),3alpha,7beta,8beta(2S*,4S*),8abeta))-2-Methylbutanoic acid 1,2,3,7,8,8a-hexahydro-3,7-dimethyl-8-(2-(tetrahydro-4-hydroxy-6-oxo-2H-pyran-2-yl)ethyl)-1-naphthalenyl ester;
Artra, .alpha.-Hydroquinone, .beta.-Quinol, 1,4-Benzenediol; arvanil, arvanil;
asiatic acid, asiatic acid;
asiaticoside, asiaticoside;
Asmax, 1,3-Dimethyl-2,6-dioxo-1,2,3,6-tetrahydropurine, 1,3-dimethyl-3,7-dihydro-1H-purine-2,6-dione, 1,3-dimethyl-7H-purine-2,6-dione;
Asmol, (+-) -Albuterol, (+-) -alpha (sup 1)-(((1,1-Dimethylethyl)amino)methyl)-4-hydroxy-1,3-benzenedimethanol, (+-)-Salbutamol;
ASTA, (+-)-Cyclophosphamide, (+-)-N,N-Bis(2-chloroethyl)tetrahydro-2H-1,3,2-oxazaphosphorin-2-amine 2-oxide, (-)-Cyclophosphamide;
astatine, 85At, Astat, astate;
Astemizole, Alacan Brand of Astemizole, Alermizol, Alonga Brand of Astemizole;
Astragaloside A, Astragaloside A, Astragaloside IV, Astramembrannin I;
atazanavir, 3,12-bis(1,1-dimethylethyl)-8-hydroxy-4,11-dioxo-9-(phenylmethyl)-6-((4-(2-pyridinyl)phenyl)methyl)-2,5,6,10,13-pentaazatetradecanedioic acid dimethyl ester, atazanavir, atazanavir sulfate;
ATL 146e, 4-(3-(6-amino-9-(5-ethylcarbamoyl-3,4-dihydroxytetrahydrofuran-2-yl)-9H-purin-2-yl)prop-2-ynyl)cyclohexanecarboxylic acid methyl ester, ATL 146e, ATL-146e;
Atorel, .beta.-D-Ribofuranoside, hypoxanthine-9, .beta.-Inosine, 6H-Purin-6-one, 9-.beta.-D-arabinofuranosyl-1,9-dihydro-;
atorvastatin, atorvastatin, atorvastatin calcium, atorvastatin, calcium salt;
Atovaquone, 2-(4-(4'-chlorophenyl)cyclohexyl)-3-hydroxy-1,4-naphthoquinone, 2-(trans-4-(4-chlorophenyl)cyclohexyl)-3-hydroxy-1,4-naphthoquinone, 566C;

ATRA, (2E,4E,6E,8E)-3,7-dimethyl-9-(2,6,6-trimethyl-1-cyclohexenyl)nona-2,4,6,8-tetraenoic acid, (2E,4E,6E,8E)-3,7-dimethyl-9-(2,6,6-trimethylcyclohex-1-en-1-yl)nona-2,4,6,8-tetraenoic acid, (2E,4E,6E,8E)-3,7-Dimethyl-9-(2,6,6-trimethylcyclohex-1-enyl)nona-2,4,6,8-tetraenoic acid;
Atropine, Anaspaz, AtroPen, Atropin Augenöl;
Auranofin, Auranofin, Auranofin Recordati Brand, Auranofin Robapharm Brand;
AuTM, (1,2-dicarboxyethylthio)gold, 1,2-dicarboxyethanethiolatogold(I), 1,2-dicarboxyethylsulfanylgold;
auxin, (indol-3-yl)acetate, 1H-indol-3-ylacetate, 2-(1H-indol-3-yl)acetate;
avasimibe, 2,6-bis(1-methylethyl)phenyl ((2,4,6-tris(1-methylethyl)phenyl)acetyl)sulfamate, avasimibe, CI 1011;
AVE 0118, AVE 0118, AVE-0118, AVE0118;
avicularin, avicularin;
Avid, Abamectin, ABAMECTIN (4:1 MIXTURE), Abamectin [ANSI];
Axert, Almogran, Almotriptan, Almotriptan (USAN);
Axsain, (6E)-N-(4-hydroxy-3-methoxybenzyl)-8-methylnon-6-enamide, (6E)-N-{[4-hydroxy-3-(methyloxy)phenyl] methyl}-8-methylnon-6-enamide, (E)-8-Methyl-N-vanillyl-6-nonenamide;
Aza-dC, Aza-dC;
Aza-deoxycytidine, Aza-deoxycytidine;
azacyclonol, azacyclonol;
Azadc, 1,3, 5-Triazin-2(1H)-one, 4-amino-1-(2-deoxy--D-erythro-pentofuranosyl)-, 2'-Deoxy-5-azacytidine, 4-amino-1-(2-deoxy-beta-D-erythro-pentofuranosyl)-1,3,5-triazin-2(1H)-one;
azamulin, azamulin;
azaspirane, antiprimod, atiprimod, azaspirane;
azelaic acid, 1,7-dicarboxyheptane, 1,7-Heptanedicarboxylic acid, 1,9-nonanedioic acid;
azelastine, 4-((4-chlorophenyl)methyl)-2- (hexahydro-1-methyl-1H-azepin-4-yl)-1(2H)- phthalazinone HCl, 4-(p-chlorobenzyl)-2-(N-methylperhydroazepinyl-(4))-1-(2H)-phthalazinone, A 5610;
azelnidipine, 3-(1-diphenylmethylazetidin-3-yl)-5-isopropyl-2-amino-1,4-dihydro-6-methyl-4-(3-nitrophenyl)-3,5-pyridinedicarboxylate, azelnidipine, CS 905;
azido ruthenium, azido ruthenium;
Azine, Azabenzene, Azine, Piridina;
Azithromycin, Azadose, Azithromycin, Azithromycin Dihydrate; Azobisisobutyramidinium dichloride, 2,2'-Azobis(2-amidinopropane) dihydrochloride, 2,2'-Azobis(2-amidinopropane)hydrochloride, 2,2'-Azobis(2-methylpropionamidine) dihydrochloride;
Azole, (4H)-1,2,4-Triazol-3-amine, .DELTA.2-1,2,4-Triazoline, 5-imino-, 1,2,4-Triazol-3-amine;
Azoles, Azoles;
Azolidine, 1-Azacyclopentane, Azacyclopentane, Azolidine;
Azophen, .beta.-Antipyrine, 1,2-Dihydro-1,5-dimethyl-2-phenyl-3H-pyrazol-3-one, 1,5-dimethyl-2-phenyl-1,2-dihydro-3H-pyrazol-3-one;
Azor, 4H-(1,2,4)Triazolo(4,3-a)(1,4)benzodiazepine, 8-chloro-1-methyl-6-phenyl-, 4H-s-Triazolo(4,3-a)(1,4)benzodiazepine, 8-chloro-1-methyl-6-phenyl-, 4H-[1,2,4]Triazolo[4,3-a][1,4]benzodiazepine, 8-chloro-1-methyl-6-phenyl-;
BA (VAN), 1,1'-(Oxybis(methylene))bisbenzene, 1,1'-[oxybis(methylene)]dibenzene, 1,1-(Oxybis(methylene))bisbenzene; Ba 0108E, Ba 0108E, Barium chloride, Barium chloride (BaCl2);
bacitracin, bacitracin, bacitracins;
Baclofen, Alphapharm Brand of Baclofen, Apo Baclofen, Apo-Baclofen;
bacterial lysate, bacterial immunostimulant, bacterial lysate, Broncho-Vaxom;
bafilomycin A1, bafilomycin A1;
Bagren, (5'alpha)-2-bromo-12'-hydroxy-2'-(1-methylethyl)-5'-(2-methylpropyl)-3',6',18-trioxoergotaman, (5'alpha)-2-bromo-12'-hydroxy-2'-(1-methylethyl)-5'-(2-methylpropyl)ergotaman-3',6',18-trione, (5'alpha)-2-bromo-12'-hydroxy-5'-(2-methylpropyl)-2'-(propan-2-yl)-3',6',18-trioxoergotaman; baicalein, 5,6,7-trihydroxy-2-phenyl-4-chromenone, 5,6,7-Trihydroxy-2-phenyl-4H-1-benzopyran-4-one, 5,6,7-trihydroxy-2-phenyl-4H-chromen-4-one;
Barbiturate, 1,2,3,4,5,6-Hexahydro-2,4,6-pyrimidinetrione, 1,3-diazinane-2,4,6-trione, 2,4,6(1H,3H,5H)-Pyrimidinetrione;
Barnidipine, (+)-(3'S,4S)-1-Benzyl-3-pyrrolidinyl methyl 1,4-dihydro-2,6-dimethyl-4-(m-nitrophenyl)-3,5-pyridinedicarboxylate, 2,6-dimethyl-4-(3-nitrophenyl)-1,4-dihydropyridine-3,5-dicarboxylic acid 03-[(3S)-1-(benzyl)pyrrolidin-3-yl] O5-methyl ester, 2,6-dimethyl-4-(3-nitrophenyl)-1,4-dihydropyridine-3,5-dicarboxylic acid O5-methyl O3-[(3S)-1-(phenylmethyl)-3-pyrrolidinyl] ester;
BAY 11-7085, 3-((4-(1,1-dimethylethyl)phenyl)sulfonyl)-2-propenenitrile, BAY 11-7083, BAY 11-7085;
BB-K8, (2S)-4-amino-N-[(1R,2S,3S,4R,5S)-5-amino-2-(3-amino-3-deoxy-alpha-D-glucopyranosyloxy)-4-(6-amino-6-deoxy-alpha-D-glucopyranosyloxy)-3-hydroxycyclohexyl]-2-hydroxybutanamide, (2S)-4-amino-N-[(1R,2S,3S, 4R,5S)-5-amino-2-[(2S,3R,4S,5S,6R)-4-amino-3,5-dihydroxy-6-(hydroxymethyl)oxan-2-yl]oxy-4-[(2R,3R,4S,5S,6R)-6-(aminomethyl)-3,4,5-trihydroxy-oxan-2-yl]oxy-3-hydroxy-cyclohexyl]-2-hydroxy-butanamide, (2S)-4-amino-N-[(1R,2S,3S,4R,5S)-5-amino-2-[(2S,3R,4S,5S,6R)-4-amino-3,5-dihydroxy-6-(hydroxymethyl)oxan-2-yl]oxy-4-[(2R,3R,4S,5S,6R)-6-(aminomethyl)-3,4,5-trihydroxyoxan-2-yl]oxy-3-hydroxycyclohexyl]-2-hydroxybutanamide;
BCNU, 1,3-Bis(.beta.-chloroethyl)-1-nitrosourea, 1,3-bis(2-chloroethyl)-1-nitroso-urea, 1,3-Bis(2-chloroethyl)-1-nitrosourea;
Beflavin, ( )-Riboflavin, (−)-Riboflavin, (-)-Riboflavin;
Belt, (1alpha,2alpha,3aalpha,4beta,7beta,7aalpha) - 1,2,4,5,6,7,8,8-Octachloro-2,3,3a,4,7,7a-hexahydro-4,7-methano-1H-indene, (1alpha,2beta,3aalpha,4beta,7beta,7aalpha) - 1,2,4,5,6,7,8,8-Octachloro-2,3,3a,4,7,7a-hexahydro-4,7-methano-1H-indene, .gamma.-Chlordan;
benazepril, Beecham brand of benazepril hydrochloride, benazapril, benazepril;
bendamustine, bendamustin, bendamustine, bendamustine hydrochloride;
Benidipine, (+-)-(R*)-3-((R*)-1-Benzyl-3-piperidyl) methyl 1,4-dihydro-2,6-dimethyl-4-(m-nitrophenyl)-3,5-pyridinedicarboxylate, 2,6-dimethyl-4-(3-nitrophenyl)-1,4-dihydropyridine-3,5-dicarboxylic acid O3-[(3R)-1-(benzyl)-3-piperidyl] O5-methyl ester, 2,6-dimethyl-4-(3-nitrophenyl)-1,4-dihydropyridine-3,5-dicarboxylic acid O5-methyl O3-[(3R)-1-(phenylmethyl)-3-piperidinyl] ester;
benzamidine, benzamidine, benzenecarboximidamide, phenylamidine; benzimidazolide, benzimidazol-1-ide, benzimidazolide, bim;
Benzodiazepines, Benzodiazepine, Benzodiazepine Compounds,
BENZODIAZEPINE CPDS;
Benzodioxoles, 1,3-Dioxaindans, 1,3-Dioxindans, Benzodioxoles;
Benzphetamine, Benzfetamine, Benzphetamine, Didrex;
benzydamine N-oxide, benzydamine N-oxide;
benzylamine, (Aminomethyl)benzene, (Phenylmethyl)amine, 1-phenylmethanamine;
benzyloxycarbonylleucyl-leucyl-leucine aldehyde, benzyloxycarbonylleucyl-leucyl-leucine aldehyde;
benzyloxycarbonylvalyl-alanyl-aspartyl fluoromethyl ketone, Ac-DEVD-CMK, Ac-ZVAD-FMK, benzyloxycarbonylvalyl-alanyl-aspartyl fluoromethyl ketone;
beractant, Abbott brand of beractant, beractant, Ross brand of beractant;
berberine, 7,8,13,13a-tetradehydro-9,10-dimethoxy-2,3-[methylenebis(oxy)]berbinium, 9,10-dimethoxy-2,3-(methylenedioxy)-7,8,13,13a-tetradehydroberbinium, 9,10-dimethoxy-5,6-dihydro[1,3]dioxolo[4,5-g]isoquino[3,2-a]isoquinolin-7-ium;
bergamottin, 5-geranoxypsoralen, 8-geranyloxypsoralen, bergamottin;
bergaptol, 4-hydroxy-7H-furo[3,2-g]chromen-7-one, 5-Hydroxyfuranocoumarin, bergaptol;
beta-glycerophosphoric acid, 2-glycerophosphoric acid, beta-glycerol phosphate, beta-glycerophosphate;
beta-lapachone, beta-lapachone;
beta-Naphthoflavone, 5,6-Benzoflavone, beta Naphthoflavone, beta-Naphthoflavone;
beta-propiolactone, 1,3-propiolactone, 2-oxetanone, 3-hydroxypropionic acid beta-lactone;
Bethanechol, Bethanechol, Bethanechol Chloride, Bethanecol; betulinic acid, betulic acid, betulinic acid;
bexarotene, 3-methyl-TTNEB, 4-(1-(3,5,5,8,8-pentamethyl-5,6,7,8-tetrahydro-2-naphthyl)ethenyl)benzoic acid, bexarotene;
Bezafibrate, Azufibrat, Azupharma Brand of Bezafibrate, Bayer Brand of Bezafibrate;
BG 9928, 3-(4-(2,6-dioxo-1,3-dipropyl-2,3,6,7-tetrahydro-1H-purin-8-yl)-bicyclo(2.2.2)oct-1-yl)propionic acid, BG 9928, BG-9928;
BGC945, BGC 945, BGC945;
biapigenin, bi-apigenin, biapigenin;
BIBX 1382BS, BIBX 1382BS, BIBX-1382BS, BIBX1382BS; biphenyl-4-ol, 4-biphenylol, 4-diphenylol, 4-Hydroxybiphenyl;
BIRB 796, 1-(5-tert-butyl-2-p-tolyl-2H-pyrazol-3-yl)-3(4-(2-morpholin-4-yl-ethoxy)naph- thalen-1-yl)urea, BIRB 796, BIRB796;
bisindolylmaleimide I, 2-(1-(3-dimethylaminopropyl)indol-3-yl)-3-(indol-3-yl)maleimide, 3-(1-(3-(dimethylamino)propyl)-1H-indol-3-yl)-4-(1H-indol-3-yl)-1H-pyrrole-2,5-dione, BIS-1 cpd;
bisindolylmaleimide III, 3-[1-(3-aminopropyl)-1H-indol-3-yl]-4-(1H-indol-3-yl)-1H-pyrrole-2,5-dione, bis-(III) indolylmaleimide, bisindolylmaleimide III;
Bisoprolol, Bisoprolol, Bisoprolol Fumarate, Bisoprolol Fumarate (1:1) Salt, (+-)-Isomer;
bisperoxovanadium, bisperoxovanadium, bpV vanadium compound;
Bisphenol A-Glycidyl Methacrylate, 2-Propenoic acid, 2-methyl-, (1-methylethylidene)bis(4,1-phenyleneoxy(2-hydroxy-3,1-propanediyl)) ester, homopolymer, Adaptic, Bis GMA;
bizelesin, bizelesin, U-78779;
BL1521, BL 1521, BL1521;

Bla-S, (2R,3R,6S)-3-[[(3S)-3-amino-5-(carbamimidoyl-methyl-amino)pentanoyl]amino]-6-(4-amino-2-oxo-pyrimidin-1-yl)-3,6-dihydro-2H-pyran-2-carboxylic acid, (2R,3R,6S)-3-[[(3S)-3-amino-5-(carbamimidoyl-methylamino)-1-oxopentyl]amino]-6-(4-amino-2-oxo-1-pyrimidinyl)-3,6-dihydro-2H-pyran-2-carboxylic acid, (2R,3R,6S)-3-[[(3S)-3-amino-5-(carbamimidoyl-methylamino)pentanoyl]amino]-6-(4-amino-2-oxopyrimidin-1-yl)-3,6-dihydro-2H-pyran-2-carboxylic acid;
Blow, (-)-Cocaine, (1R,2R,3S,5S)-2-(methoxycarbonyl)tropan-3-yl benzoate, (1R,2R,3S,5S)-2-Methoxycarbonyltropan-3-yl benzoate;
BM 41.440, 1-Hexadecylmercapto-2-methoxymethyl-3-propyl phosphoric acid monocholine ester, 1-Hexadecylthio-2-methoxymethyl-rac-glycero-3-phosphocholine, 3,5-Dioxa-9-thia-4-phosphapentacosan-1-aminium, 4-hydroxy-7-(methoxymethyl)-N,N,N-trimethyl-, (2S-(1(R*(R*)),2alpha,3abeta,7abeta))-;
BML 241, BML 241, BML-241, BML241;
BMS 310705, BMS 310705;
BMS204352, (5-chloro-2-methoxyphenyl)-1,3-dihydro-3-fluoro-6-(trifluoromethyl)-2H-indol-2-one, BMS 204352, BMS-204352;
BMS453, BMS 453, BMS-453, BMS453;
Bo-Xan, (3R,3'R)-Beta,Beta-Carotene-3,3'-Diol, Lutein, (3R,3'R,6'R)-Lutein, (3R,3'R,6S)-4,5-Didehydro-5,6-Dihydro-Beta,Beta-Carotene-3,3'-Diol;
Boltin, (7beta,8xi,9beta,13alpha,14beta,17alpha)-17-ethynyl-17-hydroxy-7-methylestr-5(10)-en-3-one, (7R,8R,9S,13S,14S,17R)-17-ethynyl-17-hydroxy-7,13-dimethyl-1,2,4,6,7,8,9,11,12,14,15,16-dodecahydrocyclopenta[a]phenanthren-3-one, Boltin;
Bonopen, (2S,5R,6R)-6-((2R)-2-Methylenamino-2-phenylacetamido-3,3-dimethyl-7-oxo-4-thia-1-azabicyclo(3.2.0)heptan-2-carbonsaeure, (alpha-(Methyleneamino)benzyl)penicillin, 3,3-Dimethyl-6-(2-(methyleneamino)-2-phenylacetamidol-7-oxo-4-thia-1-azabicyclo(3.2.0)heptane-2-carboxylic acid;
boron, Bor, boracium, bore;
Borrelia-burgdorferi, Borrelia-burgdorferi;
bortezomib, bortezomib, PS 341, PS-341;
bosentan, 4-t-butyl-N-(6-(2-hydroxyethoxy)-5-(2-methoxyphenoxy)-2,2'-bipyrimidin-4-yl)benzenesulfonamide, Actelion brand of bosentan monohydrate, bosentan;
bosutinib, 4-((2,4-dichloro-5-methoxyphenyl)amino)-6-methoxy-7-(3-(4-methyl-1-piperazinyl)propoxy)-3-quinolinecarbonitrile, 4-[(2,4-dichloro-5-methoxyphenyl)amino]-6-methoxy-7-[3-(4-methylpiperazin-1-yl)propoxy]quinoline-3-carbonitrile, bosutinib;
botrocetin, botrocetin;
BPDE, (+)-7beta,8alpha-Dihydroxy-9alpha,10alpha-oxy-7,8,9,10-tetrahydrobenzo(a)pyrene, (+-)-(E)-7,8-Dihydroxy-9,10-epoxy-7,8,9,10-tetrahydrobenzo(a)pyrene, (+-)-anti-BPDE;
BR-II, BR-II;
Brake, 1-Methyl-3-phenyl-5-(3-(trifluoromethyl)phenyl)-4(1H)-pyridinone, 1-Methyl-3-phenyl-5-(3-(trifluoromethyl)phenyl)-4-pyridone, 1-Methyl-3-phenyl-5-(alpha,alpha,alpha-trifluoro-m-tolyl)-4-pyridone;
bredinin, bredinin;
Brefeldin A, Ascotoxin, Brefeldin A, Cyanein;
Bromazepam, 1A Brand of Bromazepam, Aliud Brand of Bromazepam, Anxyrex;
bromo-cis-stilbene, bromo-cis-stilbene;
brucine, brucine;
bryostatin 1, bryostatin 1;
Budesonide, Budesonide, Budesonide, (R)-Isomer, Budesonide, (S)-Isomer;
bufalin, bufalin, bufalin, (3alpha,5beta)-isomer;
bufuralol, bufuralol, bufuralol, (DL)-(+-)-isomer, bufuralol, hydrochloride;
Bumetanide, AstraZeneca Brand of Bumetanide, Atlantis Brand of Bumetanide, Bumedyl;
BuOH, 1-butanol, 1-Butyl alcohol, 1-Hydroxybutane;
Bupivacaine, 1-Butyl-N-(2,6-dimethylphenyl)-2-piperidinecarboxamide, Abbott Brand of Bupivacaine Hydrochloride, Astra Brand of Bupivacaine Hydrochloride;
Buprenorphine, 6029 M, 6029-M, 6029M;
BUPROPION, (-)-2-(tert-Butylamino)-3'-chloropropiophenone, (- )-2-(tert-Butylamino)-3'-chlorpropiophenon, (+-)-1-(3-chlorophenyl)-2-((1,1-dimethylethyl)amino)-1-propanone;
Buspirone, Anxut, Apo Buspirone, Apo-Buspirone;
Busulfan, Busulfan, Busulfan GlaxoSmithKline Brand, Busulfan Orphan Brand;
Buthionine Sulfoximine, Buthionine Sulfoximine;
Butyrate, 1-Butyric acid, 1-Propanecarboxylic acid, 2-butanoate;
butyrolactone I, alpha-oxo-beta-(4-hydroxyphenyl)-gamma-(4-hydroxy-m-3,3-dimethylallylbenzyl)-gamma-methoxycarbonyl-gamma-butyrolactone, butyrolactone I;
C 1027, C 1027, C-1027, C1027 chromophore;
C 76, C 76;
CACP, azanide; dichloroplatinum, CACP, cis Pt II;
Calcijex, (1R,5Z)-5-[(2E)-2-[(1R,7aR)-1-(5-hydroxy-1,5-dimethyl-hexyl)-7a-methyl-2,3,3a,5,6,7-hexahydro-1H-inden-4-ylidene]ethylidene]-4-methylene-cyclohexane-1,3-diol, (1R,5Z)-5-[(2E)-2-[(1R,7aR)-1-(5-hydroxy-1,5-dimethylhexyl)-7a-methyl-2,3,3a,5,6,7-hexahydro-1H-inden-4-ylidene]ethylidene]-4-methylenecyclohexane-1,3-diol, (1R,5Z)-5-[(2E)-2-[(1R,7aR)-1-(6-hydroxy-6-methyl-heptan-2-yl)-7a-methyl-2,3,3a,5,6,7-hexahydro-1H-inden-4-ylidene]ethylidene]-4-methylidene-cyclohexane-1,3-diol;
Calcimycin, A 23187, A-23187, A23187;
calphostin C, calphostin C;
Calyculin, Calyculin, Calyculin A, Calyculin A from Discodermia calyx;
Camptothecin, Camptothecin, Camptothecine;
Canef, Canef, Cranoc, Fluindostainin sodium;
cangrelor, AR C69931MX, AR-C69931MX, cangrelor;
Cannabinoids, Cannabinoids;
Cannabis, Bhang, Cannabis, Cannabis indica;
Cantharidin, Cantharides, Cantharidin, Cantharidine;
CAPE, (E)-3-(3,4-dihydroxyphenyl)acrylic acid 2-phenylethyl ester, (E)-3-(3,4-dihydroxyphenyl)prop-2-enoic acid 2-phenylethyl ester, 2-phenylethyl (E)-3-(3,4-dihydroxyphenyl)prop-2-enoate;
Capsaicin, 8 Methyl N Vanillyl 6 Nonenamide, 8-Methyl-N-Vanillyl-6-Nonenamide, Alacan Brand of Capsaicin;
capsaicinoids, capsaicinoids;
capsazepine, capsazepine;
Carbachol, Alcon Brand 1 of Carbachol, Alcon Brand 2 of Carbachol, Allphar Brand of Carbachol;
Carbamazepine, Amizepine, Carbamazepine, Carbamazepine Acetate;
carbapenem, (5R)-1-azabicyclo[3.2.0]hept-2-en-7-one, 2,3-didehydro-1-carbapenam, carbapenem;
Carbapenems, Antibiotics, Carbapenem, Carbapenem Antibiotics, Carbapenems;
carbobenzoxy-leucyl-leucyl-norvalinal, benzyloxycarbonyl-leucyl-leucyl-norvalinal, carbobenzoxy-leucyl-leucyl-norvalinal, carbobenzoxyl-leucinyl-leucinyl-norvalinal-H;
Carbolines, Beta Carbolines, Beta-Carbolines, Carbolines;
Carboxyethyl-phenethylamino-ethylcarboxamidoadenosine, Carboxyethyl-phenethylamino-ethylcarboxamidoadenosine;
Cardiolipins, Cardiolipin, Cardiolipins, Diphosphatidylglycerols;
carebastine, carebastine;
CARNOSOL, CARNOSOL;
carrageenans, Carrageenan, carrageenans, carrageenin;
carvacrol, carvacrol;
carvedilol, Atlana Pharma brand of carvedilol, BM 14190, BM-14190;
Casodex, Bicalutamide, Casodex, Casodex (TN);
caspofungin, Cancidas, caspofungin, caspofungin acetate;
casticin, 3',5-dihydroxy-3,4',6,7-tetramethoxyflavone, casticin, vitexicarpin;
catechins, catechin, catechins;
CB 3717, CB 3717;
Cbdca, (SP-4-2)-diammine[cyclobutane-1,1-dicarboxylato(2-)-kappa(2)O,O']platinum, 1,1-Cyclobutanedicarboxylate diammine platinum(II), ammonia; cyclobutane-1,1-dicarboxylic acid;
platinum(+2) cation;
CCPA, (2R,3R,4S,5R)-2-[2-chloro-6-(cyclopentylamino)-9-purinyl]-5-(hydroxymethyl)tetrahydrofuran-3,4-diol, (2R,3R,4S,5R)-2-[2-chloro-6-(cyclopentylamino)purin-9-yl]-5-(hydroxymethyl)oxolane-3,4-diol, (2R,3R,4S,5R)-2-[2-chloro-6-(cyclopentylamino)purin-9-yl]-5-(hydroxymethyl)tetrahydrofuran-3,4-diol;
CD 437, 6-(3-(1-adamantyl)-4-hydroxyphenyl)-2-naphthalenecarboxylic acid, AHPN, CD 437;
CDP 840, CDP 840;
Cefoxitin, Cefoxitin, Cefoxitin Sodium, Mefoxin;
celecoxib,
4-(5-(4-methylphenyl)-3-(trifluoromethyl)-1H-pyrazol-1-yl)benzenesulfonamide, Celebrex, celecoxib;
cephalomannine, cephalomannine, taxol B;
cephalosporins, cephalosporins;
cepharanthine, cepharanthin, cepharanthine;
cerebrolysin, cerebrolysin, cerebrolysine, FPF 1070;

cerivastatin, 6-Heptenoic acid, 7-(4-(4-fluorophenyl)-5-(methoxymethyl)-2,6-bis(1-methylethyl)-3-pyridinyl)-3,5-dihydroxy-, monosodium salt, (S-(R*,S*-(E)))-, 7-(4-(4-fluorophenyl)-2,6-diisopropyl-5-(methoxymethyl)pyrid-3-yl)-3,5-dihydroxy-6-heptenoate sodium salt, Bay w 6228;
Cetomacrogol, alpha-Hexadecyl-omega-Hydroxypoly(oxy-1,2-Ethanediyl), Brij 52, Brij 56;
cetrorelix, ASTA Medica brand of cetrorelix acetate, cetrorelix, cetrorelix acetate;
cetuximab, C225, cetuximab, Erbitux;
CGP 12177, 4-(3-tert-butylamino-2-hydroxypropoxy)benzimidazol-2-one, 4-(3-tert-butylamino-2-hydroxypropoxy) benzimidazol-2-one hydrochloride, (+-)-isomer, 4-(3-tert-butylamino-2-hydroxypropoxy)benzimidazol-2-one, (+-)-isomer;
CGS 15943A, (1,2,4)Triazolo(1,5-c)quinazolin-5-amine, 9-chloro-2-(2-furanyl)-, 9-Chloro-2-(2-furanyl)-(1,2,4)triazolo(1,5-c)quinazolin-5-amine, 9-Chloro-2-(2-furanyl)-[1,2,4]triazolo[1,5-c]quinazolin-5-amine;
CGS 21680, 2-(4-(2-carboxyethyl)phenethylamino)-5'-N-ethylcarboxamidoadenosine, Benzenepropanoic acid, 4-(2-((6-amino-9-(N-ethyl-beta-D-ribofuranuronamidosyl)-9H-purin-2-yl)amino)ethyl)-, CGS 21680;
CH-THF, 2-[[4-(3-amino-1-keto-5,6,6a,7-tetrahydro-4H-imidazo[3,4-f]pteridin-10-ium-8-yl)benzoyl]amino]glutaric acid, 2-[[4-(3-amino-1-oxo-5,6,6a,7-tetrahydro-4H-imidazo[3,4-f]pteridin-10-ium-8-yl)benzoyl]amino]pentanedioic acid, 2-[[4-(3-amino-1-oxo-5,6,6a,7-tetrahydro-4H-imidazo[3,4-f]pteridin-10-ium-8-yl)phenyl]carbonylamino]pentanedioic acid;
CH2CHO, Acetaldehyd, Acetaldehyde, Acetaldehyde (natural); Chalcone, 1,3-Diphenyl-2-Propen-1-One, Benzalacetophenone, Benzylideneacetophenone;
CHAPS, 3-((3-Cholamidopropyl)dimethylammonio)-1-propanesulfonate, 3-((3-Cholamidopropyl)dimethylammonium)-1-propanesulfonate, 3-[(3-Cholamidopropyl)dimethylammonio]-1-propanesulfonate;
Chinine, (-)-Quinine, (5-ethenyl-1-azabicyclo[2.2.2]octan-7-yl)-(6-methoxyquinolin-4-yl)methanol, (6-methoxy-4-quinolyl)-(5-vinyl-2-quinuclidinyl)methanol;
Chitosan, Chitosan, Poliglusam;
Chloramphenicol, Chloramphenicol, Chlornitromycin, Chlorocid; chlorophenyl-ethane, chlorophenyl-ethane;
chlorophyllin, chlorophyllin, chlorophyllin a, chlorophyllin copper complex;
chlorophyllypt, Chlorophyllipt, chlorophyllypt;
chlorpromazine, 3-(2-chloro-10H-phenothiazin-10-yl)-N,N-dimethyl-1-propanamine, 3-(2-chloro-10H-phenothiazin-10-yl)-N,N-dimethylpropan-1-amine, 3-(2-chlorophenothiazin-10-yl)-N,N-dimethyl-propan-1-amine;
Chlorpropham, Chlorpropham, CIPC, Isopropyl N-(3-Chlorophenol)carbamate;
Chlorzoxazone, Chlorzoxazone, McNeil Brand of Chlorzoxazone, Ortho Brand of Chlorzoxazone;
Cholestanol, 5 alpha Cholestan 3 alpha ol, 5 alpha Cholestan 3 beta ol, 5 alpha-Cholestan-3 alpha-ol;
CHOLINE, (2-Hydroxyethyl)trimethylammonium, (2-Hydroxyethyl)trimethylammonium chloride, (beta-hydroxyethyl) trimethylammonium;
Chonsurid, (2S,3S,4S,5R,6R)-6-[(2R,3R,4R,5R,6R)-3-acetamido-2,5-dihydroxy-6-sulfooxy-oxan-4-yl]oxy-3,4,5-trihydroxy-oxane-2-carboxylic acid, (2S,3S,4S,5R,6R)-6-[(2R,3R,4R,5R,6R)-3-acetamido-2,5-dihydroxy-6-sulfooxy-tetrahydropyran-4-yl]oxy-3,4,5-trihydroxy-tetrahydropyran-2-carboxylic acid, (2S,3S,4S,5R,6R)-6-[(2R,3R,4R,5R,6R)-3-acetamido-2,5-dihydroxy-6-sulfooxyoxan-4-yl]oxy-3,4,5-trihydroxyoxane-2-carboxylic acid; chromophore, chromophore, chromophores;
Chrysin, 4H-1-Benzopyran-4-one, 5,7-dihydroxy-2-phenyl-, 4H-1-Benzopyran-4-one, 5,7-dihydroxy-2-phenyl-(9CI), 5,7-dihydroxy-2-phenyl-4-chromenone;
chymostatin, chymostatin;
CI1033, Canertinib, CI 1033, CI-1033;
cicaprost, 13,14-didehydro-16,20-dimethyl-3-oxa-18,18,19,19-tetradehydro-6-carbaprostaglandin 12, 5-(7-hydroxy-6-(3-hydroxy-4-methylnona-1,6-diynyl)-bicyclo(3.3.0)octan-3-yliden)-3-oxapentanoic acid, cicaprost;
cifostodine, cifostodine;
ciglitazone, 5-(4-(1-methylcyclohexylmethoxy)benzyl)thiazolidine-2,4-dione, ADD 3878, ADD-3878;
Cilazapril, Cilazapril, Cilazapril Hydrate, Cilazapril Monohydrobromide;
Cilomilast, Cilomilast;
cilostazol, 6-(4-(1-cyclohexyl-1H-tetrazol-5-yl)butoxy)-3,4-dihydro-2(1H)-quinolinone, cilostazol, OPC 13013;
Cimetidine, Altramet, Biomet, Biomet400;
cinacalcet, alpha-methyl-N-(3-(3-(trifluoromethyl)phenyl)propyl)-1-naphthalenemethanamine, (alphaR)-hydrochloride, AMG 073, AMG073;
cinitapride, Almirall brand of cinitapride tartrate, Blaston, Cidine;
cinnamic aldehyde, 3-phenylprop-2-enaldehyde, beta-phenylacrolein, cinnamaldehyde;
cionin, Asn-Tyr(SO3)-Tyr(SO3)-Gly-Trp-Met-Asp-Phe-NH2, cionin;
Cipol N, (R-(R*,R*-(E)))-Cyclic(L-alanyl-D-alanyl-N-methyl-L-leucyl-N-methyl-L-leucyl-N-methyl-, 1,4,7,10,13,16,19,22,25,28,31-undecaazacyclotritriacontane-2,5,8,11,14,17,20,23,26,29,32-, 30-ethyl-33-[(Z,1S,2R)-1-hydroxy-2-methyl-hex-4-enyl]-1,4,7,10,12,15,19,25,28-nonamethyl-6,9,18,24-tetrakis(2-methylpropyl)-3,21-di(propan-2-yl)-1,4,7,10,13,16,19,22,25,28,31-undecazacyclotritriacontane-2,5,8,11,14,17,20,23,26,29,32-undecone;
Ciprofloxacin, Bay 09867, Bay-09867, Bay09867;
Ciprol, 2-(4-(2,2-Dichlorocyclopropyl)phenoxy)2-methylpropanoic acid, 2-(p-(2,2-Dichlorocyclopropyl)phenoxy)-2-methylpropionic acid, 2-[4-(2,2-dichlorocyclopropyl)phenoxy]-2-methyl-propanoic acid;
cis-9, trans-11-conjugated linoleic acid, c9-t11-CLA, cis-9, trans-11-conjugated linoleic acid;
Cisapride, Cisapride, Propulsid, R 51619;
Citalopram, Citalopram, Cytalopram, Escitalopram;
Citox, .alpha.,.alpha.-Bis(p-chlorophenyl)-.beta.,.beta.,.beta.-trichlorethane, 1,1'-(2,2,2-trichloroethane-1,1-diyl)bis(4-chlorobenzene), 1,1'-(2,2,2-Trichloroethylidene)bis(4-chlorobenzene);
CITRULLINE, (2S)-2-amino-5-(aminocarbonylamino)pentanoic acid, (2S)-2-amino-5-(carbamoylamino)pentanoic acid, (2S)-2-amino-5-ureido-pentanoic acid;
clebopride, clebopride, clebopride fumarate (1:1), clebopride maleate;
clevidipine, butyroxymethyl methyl 4-(2',3'-dichlorophenyl)-2,6-dimethyl-1,4-dihydropyridine-3,5-dicarboxylate, clevidipine;
clobazam, clobazam;
Clodronic Acid, Bonefos, Cl2MDP, Clodronate;
clofarabine, 2-chloro-2'-arabino-fluoro-2'-deoxyadenosine, 2-chloro-2'-fluoroarabino-2'-deoxyadenosine, 2-chloro-9-(2-deoxy-2-fluoro-beta-D-arbinofuranosyl)adenine;
Clofibric Acid, 2-(4-Chlorophenoxy)-2-methylpropionic Acid, Clofibric Acid, Clofibrinic Acid;
Clomipramine, Anafranil, Chlomipramine, Chlorimipramine; Clonazepam, Antelepsin, Clonazepam, Rivotril;
Clonidine, Catapres, Catapresan, Catapressan;
clopidogrel, clopidogrel, clopidogrel bisulfate, clopidogrel, (+)(S)-isomer;
clotiazepam, clotiazepam;
Clozapine, Clozapine, Clozaril, Leponex;
clozapine N-oxide, clozapine N-oxide;
CNI 1493, CNI 1493, CNI-1493, N,N'-bis(3,5-diacetylphenyl)decanediamide tetrakis(amidinohydrazone) tetrahydrochloride;
Co 2-1970, 1-[(3S,5S,8R,9S,10S,13S,14S,17S)-3-hydroxy-10,13-dimethyl-3-(trifluoromethyl)-1,2,4,5,6,7,8,9,11,12,14,15,16,17-tetradecahydrocyclopenta[a]phenanthren-17-yl]ethanone, 3alpha-Hydroxy-3beta-(trifluoromethyl)-5alpha-pregnan-20-one, 3alpha-Hydroxy-3beta-trifluoromethyl-5alpha-pregnan-20-one;
Coagulin, antigens, cd142, blood coagulation factor iii, cd142 antigens;
Colchicine, Colchicine;
compactin, 6-demethylmevinolin, compactin, CS 500;
CONT, 1-(.beta.-Ethylol)-2-methyl-5-nitro-3-azapyrrole, 1-(.beta.-Hydroxyethyl)-2-methyl-5-nitroimidazole, 1-(2-Hydroxy-1-ethyl)-2-methyl-5-nitroimidazole;
Cotinine, Cotinine, Scotine;
Cotrim, 3-(p-Aminophenylsulfonamido)-5-methylisoxazole, 3-(para-Aminophenylsulphonamido)-5-methylisoxazole, 3-Sulfanilamido-5-methylisoxazole;
coumarin, 1,2-Benzopyrone, 2-Propenoic acid, 3-(2-hydroxyphenyl)-, d-lactone, 2H-1-Benzopyran-2-one;
CRA 024781, CRA 024781, CRA-024781, CRA024781;
CRA 026440, CRA 026440, CRA-026440, CRA026440;
Crestor, calcium (E,3R,5S)-7-[4-(4-fluorophenyl)-2-(methyl-methylsulfonyl-amino)-6-propan-2-yl-pyrimidin-5-yl]-3,5-dihydroxy-hept-6-enoate, calcium (E,3R,5S)-7-[4-(4-fluorophenyl)-2-(methyl-methylsulfonylamino)-6-propan-2-ylpyrimidin-5-yl]-3,5-dihydroxyhept-6-enoate, calcium (E,3R,5S)-7-[4-(4-fluorophenyl)-6-isopropyl-2-(mesyl-methyl-amino)pyrimidin-5-yl]-3,5-dihydroxy-hept-6-enoate;
Crodacid, 1-Tridecanecarboxylic acid, C14 fatty acid, CH3-[CH2]12-COOH;
Crypt-2,2,2, 13,16,21,24-Hexaoxa-1,10-diazabicyclo-(8,8,8)-hexacosane, 2,2,2-Cryptand, 4,7,13,16,21,24-Hexaoxa-1,10-diazabicyclo(8.8.8)hexacosane;
cryptdin 3, cryptdin 3, cryptdin-3;
cryptotanshinone, cryptotanshinone;
cryptoxanthin, beta-caroten-3-ol, beta-cryptoxanthin, cryptoxan-cryptoxanthin;
CUBE, (-)-cis-Rotenone, (-)-Rotenone, (1)Benzopyrano(3,4-b)furo(2,3-h)(1)benzopyran-6(6aH)-one, 1,2,12,12a-tetrahydro-2-alpha-isopropenyl-8,9-dimethoxy-;
CVT 3146, (1-(9-(3,4-dihydroxy-5-(hydroxymethyl)oxolan-2-yl)-6-aminopurin-2-yl)pyrazol-4-yl)-N-methylcarboxamide, CVT 3146, CVT-3146;
cyanidin 3-rutinoside, cyanidin 3-rutinoside;

cyanidin-3-glucoside, cyanidin-3-glucoside;
cyanoginosin-LA, cyanoginosin-LA, cyanogynosin-LA, microcystin LA;
Cyclandelate, Cyclandelate, Cyclospasmol;
cyclohexyl carbamic acid 3'-carbamoylbiphenyl-3-yl ester, cyclohexyl carbamic acid 3'-carbamoylbiphenyl-3-yl ester, URB 597, URB-597;
cyclohexyl-methyl, cyclohexyl-methyl;
cyclopamine, cyclopamine;
Cyclopentenone, 1-cyclopent-2-enone, 2-Cyclopenten-1-one, 2-Cyclopenten-1-one (8CI)(9CI);
cyclopiazonic acid, cyclopiazonic acid;
Cyproheptadine, Antergan, Cyproheptadine, Dihexazin;
Cyproterone Acetate, Androcur, Cyproterone Acetate, Cyproterone Acetate, (1 alpha,2 alpha)-Isomer;
cystathionine, 2-amino-4-[(2-amino-2-carboxyethyl)sulfanyl]butanoic acid, cystathionine, DL-Allocystathionine;
cysteamine, 2-amino-1-ethanethiol, 2-aminoethanethiol, beta-Aminoethanethiol;
cysteinyl-leukotriene, Cys-LT, cysteinyl-leukotriene;
Cytarabine, Ara-C, Arabinofuranosylcytosine, Arabinosylcytosine; cytochalasin B, cytochalasin B;
Cytochalasin D, Cytochalasin D;
cytochalasin E, cytochalasin E;
D 22888, D 22888;
D 23129, D 20443, D 23129, D-20443;
DA 8159, 5-(2-propyloxy-5-(1-methyl-2-pyrollidinylethylamidosulfonyl)phenyl)-1-methyl-3-propyl-1,6-dihydro-7H-pyrazolo (4,3-d) pyrimidine-7-one, DA 8159, DA-8159; Dacarbazine, 5-(3,3-Dimethyl-1-triazeno)imidazole-4-carboxamide, Biocarbazine, Dacarbazine;
DADSO, 2-Propene-1-sulfinic acid, thio-, S-allyl ester, 2-Propene-1-sulfinothioic acid S-2-propenyl ester, 2-Propene-1-sulfinothioic acid, S-2-propenyl ester (9CI);
daidzein, daidzein, diadzein;
danaproid, danaparoid, danaproid, danaproid sodium;
Dapsone, 4,4'-Diaminophenyl Sulfone, Avlosulfone, DADPS;
Daral, 3-[(2E)-2-[1-(5,6-dimethylhept-3-en-2-yl)-7a-methyl-2,3,3a,5,6,7-hexahydro-1H-inden-4-ylidene]ethylidene]-4-methylidene-cyclohexan-1-ol, 3-[(2E)-2-[1-(5,6-dimethylhept-3-en-2-yl)-7a-methyl-2,3,3a,5,6,7-hexahydro-1H-inden-4-ylidene]ethylidene]-4-methylidenecyclohexan-1-ol, 3-[(2E)-2-[7a-methyl-1-(1,4,5-trimethylhex-2-enyl)-2,3,3a,5,6,7-hexahydro-1H-inden-4-ylidene]ethylidene]-4-methylene-1-cyclohexanol;
Darifenacin, 2-[(3S)-1-[2-(2,3-dihydro-1-benzofuran-5-yl)ethyl]pyrrolidin-3-yl]-2,2-di(phenyl)acetamide, 2-[(3S)-1-[2-(2,3-dihydro-1-benzofuran-5-yl)ethyl]pyrrolidin-3-yl]-2,2-di(phenyl)ethanamide, 2-[(3S)-1-[2-(2,3-dihydrobenzofuran-5-yl)ethyl]-3-pyrrolidinyl]-2,2-di(phenyl)acetamide;
darunavir, darunavir, darunavir ethanolate, Prezista; dasatinib, (18F)-N-(2-chloro-6-methylphenyl)-2-(6-(4-(2-hydroxyethyl)piperazin-1-yl)-2-methylpyrimidin-4-ylamino)thiazole-5-carboxamide, BMS 354825, BMS-354825;
Daunorubicin, Cerubidine, Dauno Rubidomycine, Dauno-Rubidomycine;
Dayfen, 1-Methyl-4-piperidyl benzhydryl ether, 132-18-3 (HCL), 4-(Benzhydryloxy)-1-methylpiperidine;
DBPC, (2,5-Cyclohexadiene-1,4-diylidene)-dimalononitrile, 1-Hydroxy-4-methyl-2,6-di-tert-butylbenzene, 2,6-Bis (1,1-dimethylethyl)-4-methylphenol;
DDB, DDB;
DDE, 1,1-Dichloro-2,2-bis(4'-chlorophenyl)ethylene, 1-chloro-4-[2,2-dichloro-1-(4-chlorophenyl)ethenyl]benzene, 4,4'-DDE;
Debrisoquin, Debrisoquin, Debrisoquine, Tendor;
decursin, decursin, decursinol;
Deethylamiodarone, (2-butyl-1-benzofuran-3-yl)-[4-(2-ethylaminoethoxy)-3,5-diiodo-phenyl]methanone, (2-butyl-1-benzofuran-3-yl)-[4-(2-ethylaminoethoxy)-3,5-diiodophenyl]methanone, (2-Butyl-3-benzofuranyl)(4-(2-(ethylamino)ethoxy)-3,5-diiodophenyl)methanone;
deferiprone, 1,2-dimethyl-3-hydroxy-4-pyridinone, 1,2-dimethyl-3-hydroxypyrid-4-one, 1,2-dimethyl-3-hydroxypyridin-4-one;
Deferoxamine, Deferoxamine, Deferoxamine B, Deferoxamine Mesilate;
deguelin, deguelin;
dehydroaripiprazole, dehydroaripiprazole;
Dehydroepiandrosterone Sulfate, Dehydroepiandrosterone Sulfate, Dehydroisoandrosterone Sulfate, DHA Sulfate;
dehydroxymethylepoxyquinomicin, dehydroxymethylepoxyquinomicin, DHMEQ cpd;
Delavirdine, Agouron Brand of Delavirdine Mesylate, Delavirdine, Delavirdine Mesylate;
delta8-THC, (-)-.delta.-(sup8)-trans-Tetrahydrocannabinol, (-)-.DELTA.6-Tetrahydrocannabinol, (-)-.DELTA.8-Tetrahydrocannabinol;

Denagard, (4R,5S,6S,8R,9AR,10R)-5-HYDROXY-4,6,9,10-TETRAMETHYL-1-OXO-6-VINYLDECAHYDRO-3A,9-PROPANOCYCLOPENTA[8]ANNULEN-8-YL {[2-(DIETHYLAMINO)ETHYL]SULFANYL}ACETATE, Denagard, Denagard (TN);
denbinobin, 5-hydroxy-3,7-dimethoxy-1,4-phenanthraquinone, denbinobin;
denileukin diftitox, DAB(389)-IL-2, DAB(389)-interleukin 2, DAB(389)IL-2;
denopamine, (3,4-dimethoxyphenethylaminomethyl)-4-hydroxybenzyl alcohol, 1-(p-hydroxyphenyl)-2-((3,4-dimethoxyphenethyl)amino)ethanol, denopamine;
Depas, 4-(2-chlorophenyl)-2-ethyl-9-methyl-6H-thieno[3,2-f][1,2,4]triazolo[4,3-a][1,4]diazepine, 4-(o-Chlorophenyl)-2-ethyl-9-methyl-6H-thieno(3,2-f)-s-triazolo(4,3-a)(1,4)diazepine, 4H-s-Triazolo(3,4-c)thieno(2,3-e)(1,4)-diazepine, 6-(o-chlorophenyl)-8-ethyl-1-methyl-;
deramciclane, 2-phenyl-2-(dimethylaminoethoxy)-1,7,7-trimethylbicyclo(2.2.1)heptane hemifumarate, deramciclane, deramciclane, (1R,2S,4R)-isomer;
desethylchloroquine, deethylchloroquine, desethylchloroquine, desethylchloroquine dihydrochloride;
desflurane, Baxter Anaesthesia brand of desflurane, Baxter brand of desflurane, desflurane;
desisobutyrylciclesonide, desisobutyryl-ciclesonide, desisobutyrylciclesonide;
desmethylazelastine, desmethylazelastine;
Desmethyldeprenyl, (1-methyl-2-phenyl-ethyl)-propargyl-amine, 1-Phenyl-2-(N-2-propynyl)aminopropane, alpha-Methyl-N-2-propynylbenzeneethanamine;
Devazepide, Devazepide, L-364,718, MK 329;
Dexfenfluramine, Dexfenfluramine, Dexfenfluramine Hydrochloride, Redux;
dexloxiglumide, dexloxiglumide;
Dextropropoxyphene, D Propoxyphene, D-Propoxyphene, Darvon; dFdC, 122111-03-9 (HYDROCHLORIDE), 2', 2'-DiF-dC, 2',2'-DIFLUORODEOXYCYTIDINE;
DFMO, .alpha.-DFMO HCl, 2,5-diamino-2-(difluoromethyl)pentanoic acid, 2,5-diamino-2-(difluoromethyl)valeric acid;
DHEA, (+)-Dehydroisoandrosterone, (3-beta)-3-Hydroxyandrost-5-en-17-one, (3beta)-3-hydroxyandrost-5-en-17-one;
DHLA, ()-6,8-Dimercaptooctanoic acid, ()-Dihydrolipoic acid, 6,8-bis-sulfanyloctanoic acid;
di-(1-isoquinolinyl)-di-(pyridyl-2')butane, di-(1-isoquinolinyl)-di-(pyridyl-2')butane, S-147;
Diaben, 1-butyl-3-(4-methylphenyl)sulfonyl-urea, 1-butyl-3-(4-methylphenyl)sulfonylurea, 1-Butyl-3-(4-methylphenylsulfonyl)urea;
Diacomit, (E)-1-(1,3-benzodioxol-5-yl)-4,4-dimethyl-pent-1-en-3-ol, (E)-1-(1,3-benzodioxol-5-yl)-4,4-dimethylpent-1-en-3-ol, 1-(1,3-Benzodioxol-5-yl)-4,4-dimethyl-1-penten-3-ol;
diadenosine tetraphosphate, diadenosine tetraphosphate;
Dial, 1,4-Pentanediamine, N4-(6-chloro-2-methoxy-9-acridinyl)-N1,N1-diethyl-, 2-Methoxy-6-chloro-9-diethylaminopentylaminoacridine, 3-Chloro-7-methoxy-9-(1-methyl-4-diethylaminobutylamino)acridine;
Diamide, Diamide, Diazodicarboxylic Acid Bis(N,N-dimethyl)amide, Diazodicarboxylic Acid Bisdimethylamide;
DIAN, .beta.,.beta.'-Bis(p-hydroxyphenyl)propane, .beta.-Di-(p-hydroxyphenyl)propane, 2,2-(4,4'-Dihydroxydiphenyl)propane;
diarsenic trioxide, Arsenic trioxide, arsenic(III) oxide, As203; Dibenzanthracene, 1,2,3,4-DIBENZANTHRACENE, 1,2:3,4-Dibenzanthracene, 1,2:3,4-Dibenzoanthracene;
Dicid, Alfa-Tox, Antigal, Bassadinon;
Diclofenac, Dichlofenal, Diclofenac, DICLOFENAC NA;
Dicyclohexylcarbodiimide, DCCD, Dicyclohexylcarbodiimide; diethyl maleate, diethyl maleate;
Diethyl-benzoquinone-imine, Diethyl-benzoquinone-imine;
Digicor, 4-[(3S,5R,8R,9S,10S,13R,14S,17S)-3-[(2R,4S,5S,6R)-5-[(2S,4S,5S,6R)-5-[(2S,4S,5S,6R)-4,5-dihydroxy-6-methyl-oxan-2-yl]oxy-4-hydroxy-6-methyl-oxan-2-yl]oxy-4-hydroxy-6-methyl-oxan-2-yl]oxy-14-hydroxy-10,13-dimethyl-1,2,3,4,5,6,7,8,9,11,12,15,16,17-tetradeca, 4-[(3S,5R,8R,9S,10S,13R,14S,17S)-3-[(2R,4S,5S,6R)-5-[(2S,4S,5S,6R)-5-[(2S,4S,5S,6R)-4,5-dihydroxy-6-methyl-tetrahydropyran-2-yl]oxy-4-hydroxy-6-methyl-tetrahydropyran-2-yl]oxy-4-hydroxy-6-methyl-tetrahydropyran-2-yl]oxy-14-hydroxy-10,13-dimethyl-1,2,3,4,5, 4-[(3S,5R,8R,9S,10S,13R,14S,17S)-3-[(2R,4S,5S,6R)-5-[(2S,4S,5S,6R)-5-[(2S,4S,5S,6R)-4,5-dihydroxy-6-methyloxan-2-yl]oxy-4-hydroxy-6-methyloxan-2-yl]oxy-4-hydroxy-6-methyloxan-2-yl]oxy-14-hydroxy-10,13-dimethyl-1,2,3,4,5,6,7,8,9,11,12,15,16,17-tetradecahyd;
Digitin, .beta.-D-Galactopyranoside, (2.alpha.,3.beta.,5.alpha.,15.beta.,25R)-2,15-dihydroxyspirostan-3-yl O-.beta.-D-glucopyranosyl-(1.fwdarw.3)-O-.beta.-D-galactopyranosyl-(1.fwdarw.2)-O-[.beta.-D-xylopyranosyl-(1.fwdarw.3)]-O-.beta.-D-glucopyranosyl-(1.fwdarw.4, Digitin, Digitogenin, glycoside;
Digoxin, AWD.pharma Brand of Digoxin, Bertek Brand of Digoxin, Digacin;

Dihydroqinghaosu, 3,12-Epoxy-12H-pyrano(4,3-j)-1,2-benzodioxepin-10-ol, decahydro-3,6,9-trimethyl-, (3R,5aS, 6R,8aS,9R,10S,12R,12aR)-, 3,12-Epoxy-12H-pyrano(4,3-j)-1,2-benzodioxepin-10-ol, decahydro-3,6,9-trimethyl-, (3R-(3alpha,5abeta,6beta,8abeta,9alpha,10alpha,12beta,12aR*))-, Dihydroartemisinin;
Dihydroxycholecalciferols, Dihydroxycholecalciferols, Dihydroxyvitamins D;
diisopropyl fluorophosphate, bis(propan-2-yl) fluorophosphate, Diisopropoxyphosphoryl fluoride, diisopropyl fluorophosphate; dillapiol, dillapiol;
Diltiazem, Aldizem, Cardil, Cardizem;
Dimethadione, 5,5-Dimethyl-2,4-oxazolidinedione, 5,5-Dimethyloxazolidine-2,4-dione, Dimethadione;
dimethyl fumarate, dimethyl fumarate, dimethylfumarate, Fumaderm;
Dimethyl Sulfoxide, Dimethyl Sulfoxide, Dimethyl Sulphoxide, Dimethylsulfoxide;
dimethyl-hydrazide, dimethyl-hydrazide;
dimethylamino-purine, dimethylamino-purine;
dimuonium, (mu(+)e(-))2, dimuonium, Mu2;
dinitrophenol, dinitrophenol;
Dinoprostone, Dinoprostone, PGE2, PGE2 alpha;
dioxirane, dioxirane;
Dipalmitoyl, 1,2-Di-O-palmitoyl-3-sn-glyceryl-O-phosphoric acid, 1,2-dihexadecanoyl-sn-glycero-3-phosphate, 1,2-Dipalmitoyl-3-sn-phosphatidic acid;
diphenylalanine, diphenylalanine;
Diphenylamine, Diphenylamine;
diphenyleneiodonium, diphenylene iodonium, diphenyleneiodium chloride, diphenyleneiodonium;
Dipyridamole, Antistenocardin, Apo-Dipyridamole, Apotex Brand of Dipyridamole;
Dipyrone, Algopyrin, Analgin, Biopyrin;
discodermolide, discodermolide;
Diterpenes, Cembrane Diterpenes,
Cembranes, Diterpenes;
Dithionite, Dithionite, Hyposulfite, Sodium Dithionite; diuretic, diuretic, diuretics;
Diuron, 3-(3,4-Dichlorophenyl)-1,1-dimethylurea, DCMU, Diuron; divinyl benzene, divinyl benzene;
dl-Ipr, (+)-Isoprenaline, (+)-Isoproterenol, (+-)-Isoprenaline; DMGG, 1,1-Dimethyl biguanide, 1,1-Dimethylbiguanide, 1115-70-4 (HCL);
DMPX, 1H-Purine-2,6-dione, 3,7-dihydro-3,7-dimethyl-1-(2-propynyl)-, 3,7-Dimethyl-1-(2-propynyl)xanthine, 3,7-dimethyl-1-prop-2-ynyl-purine-2,6-dione;
DMSO, (CH3)2SO, (DMSO), (methanesulfinyl)methane;
Dobutamine, Boehringer Ingelheim Brand of Dobutamine Hydrochloride, Dobucor, Dobuject;
Doca, 11-Dehydroxycorticosterone, 11-Deoxycorticosterone, 11-Desoxycorticosterone;
Doconexent, (4Z,7Z,10Z,13Z,16Z,19Z)-4,7,10,13,16,19-Docosahexaenoic acid, (4Z,7Z,10Z,13Z,16Z,19Z)-docosa-4,7,10,13,16,19-hexaenoic acid, (4Z,7Z,10Z,13Z,16Z,19Z)-Docosahexaenoic acid;
dodecyl-phosphocholine, dodecyl-phosphocholine;
dodecyloctaethyleneglycol monoether, dodecyloctaethyleneglycol monoether;
Domperidone, Aliud Brand of Domperidone Maleate, Apo Domperidone, Apo-Domperidone;
DOTA, 1,4,7,10-Dota, 1,4,7,10-tetraazacyclododecane- 1,4,7,10-tetraacetic acid, 1,4,7,10-Tetraazacyclododecane-1,4,7,10-tetraacetic acid;
Doxazosin, 1 (4-Amino-6,7-dimethoxy-2-quinazolinyl)-4-((2,3-dihydro-1,4-benzodioxin-2-yl)carbonyl)piperazine, Alfamedin, Aliud Brand of Doxazosin Mesylate;
Doxorubicin, Adriablastin, Adriablastine, Adriamycin; Doxycycline, alpha 6 Deoxyoxytetracycline, alpha-6-Deoxyoxytetracycline, BMY 28689;
DPC 681, DPC 681;
DPCPX, 1,3-Dipropyl-8-cyclopentylxanthine, 1,3-Dpcpx, 1H-Purine-2,6-dione, 8-cyclopentyl-3,7-dihydro-1,3-dipropyl-;
Droxia, 1-HYDROXYUREA, Biosupressin, Carbamohydroxamic acid;
DTMC, 1,1-bis(4-chlorophenyl)-2,2,2-trichloroethanol, 1,1-Bis(chlorophenyl)-2,2,2-trichloroethanol, 1,1-bis(p-chlorophenyl)-2,2,2-trichloroethanol;
dulcin, dulcin, p-ethoxyphenylurea, phenetolcarbamide;
Durapatite, Alveograf, Calcitite, Calcium Hydroxyapatite;
DX 9065a, DX 9065a;
Dxms, (11beta,16alpha)-9-Fluoro-11,17,21-trihydroxy-16-methylpregna-1,4-diene-3,20-dione, (11beta,16beta)-9-fluoro-11,17,21-trihydroxy-16-methylpregna-1,4-diene-3,20-dione, (3H)-Dexamethasone;
Dynatra, (3H)-Dopamine, .alpha.-(3,4-Dihydroxyphenyl)-.beta.-aminoethane, .Beta.-(3,4-Dihydroxyphenyl)ethylamine hydrochloride;
E 10, E 10, E-10, N-(2-aminoethyl)-N-(2-(octylamino)ethyl)glycine monohydrochloride, mixt. with N,N-bis(2-(octylamino)ethyl)glycine monohydrochloride;
E 3330, E 3330;
E-MIX 80, .gamma.-Tocopherol, 2,7,8-trimethyl-2-(4,8,12-trimethyltridecyl)-6-chromanol, 2,7,8-trimethyl-2-(4,8,12-trimethyltridecyl)chroman-6-ol;
E.O., 1,2-Epoxyethane, Alpha,beta-oxidoethane, alpha-Hydro-omega-hydroxypoly(oxy(methyl-1,2-ethanediyl)), (chlorome-(chloromethyl)oxirane polymer;

EACA, .epsilon. S, .epsilon.-Aminocaproic acid, .epsilon.-Aminohexanoic acid;
ebastine, 4-diphenylmethoxy-1-(3-(4-tert-butylbenzoyl)propyl)piperidine, Almirall brand of ebastine, Bactil;
ebrotidine, 4-bromo-N-(((2-(((-((diaminomethylene)amino)-4-thiazolyl)methyl)thio)ethyl)amino)methylene)benzenesulfonamide, ebrotidine;
Echinomycin, Echinomycin, NSC 526417, NSC-526417;
Econ, ()-alpha-Tocopherol, (+)-alpha-Tocopherol acetate, (+)-alpha-Tocopheryl acetate;
econazole, 1-(2,4-Dichloro-beta-((p-chlorobenzyl)oxy)phenethyl)imidazole, 1-{2-(4-chlorobenzyloxy)-2-(2,4-dichlorophenyl)ethyl}-1H-imidazole, Clc1ccc(COC(Cn2ccnc2)c3ccc(Cl)cc3Cl)cc1;
ecteinascidin 743, ecteinascidin 743, ET 743, ET-743;
Edetic Acid, Calcium Disodium Edetate, Calcium Disodium Versenate, Calcium Tetacine;
Edex, (11alpha,13E,15S)-11,15-dihydroxy-9-oxoprost-13-en-1-oic acid, (11alpha,13E,15S)-11,15-Dihydroxy-9-oxoprost-13-enoic acid, (13E)-(15S)-11alpha,15-Dihydroxy-9-oxoprost-13-enoate;
efavirenz, 6-chloro-4-cyclopropylethynyl-4-trifluoromethyl-1,4-dihydro-2H-3,1-benzoxazin-2-one, Bristol-Myers Squibb brand of efavirenz, DMP 266;
EGCg, (−)-cis-2-(3,4,5-Trihydroxyphenyl)-3,4-dihydro-1(2H)-benzopyran-3,5,7-triol 3-gallate, (−)-cis-3,3′,4′,5,5′,7-Hexahydroxy-flavane-3-gallate, (−)-Epigallocatechin gallate;
EGTA, (Ethylenebis(oxyethylenenitrilo))tetraacetic acid, 1,2-Bis(2-aminoethoxyethane)-N,N,N',N'-tetraacetic acid, 1,2-Bis(2-dicarboxymethylaminoethoxy)ethane;
eletriptan, 3-(1-methyl-2-pyrrolidinylmethyl)-5-(2-(phenylsulfonyl)ethyl)-1H-indole hydrobromide, eletriptan, eletriptan hydrobromide;
Elicide, Elicide, Estivin, Ethylmercurithiosalicylate sodium;
Empecid, (2-Chlorophenyl)diphenyl-1-imidazolylmethane, (Chlorotrityl)imidazole, 1-((2-Chlorophenyl)diphenylmethyl)-1H-imidazole;
Enalapril, Enalapril, Enalapril Maleate, MK 421;
Endocannabinoids, CANNABINOID RECEPT MODULATORS, Cannabinoid Receptor Modulators, Cannabinoids, Endogenous;
endomorphin 1, (Dmt1)endomorphin-1, endomorphin 1, endomorphin-1;
Enediynes, Enediyne Group, Enediynes;
enflurane, 2-chloro-1,1,2-trifluoroethyl difluoromethyl ether, 2-chloro-1-(difluoromethoxy)-1,1,2-trifluoroethane, Alyrane;
enone, enone;
Enoximone, Enoximone, Fenoximone, MDL 17043;
entacapone, 2-cyano-N,N-diethyl-3-(3,4-dihydroxy-5-nitrophenyl)propenamide, Comtan, Comtess;
Entex, 4-Methylmercapto-3-methylphenyl dimethyl thiophosphate, Bay-Bassa, Baycid;
enzastaurin, enzastaurin, LY317615.HCl;
EOS, EOS;
EPC-K(1), ascorbic acid-2-(3,4-dihydro-2,5,7,8-tetramethyl-2-(4,8,12-trimethyltridecyl)-2H-1-benzopyran-6-yl hydrogen phosphate), EPC-K, EPC-K(1);
EPEG, (-)-Etoposide, (5R,5aR,8aR,9S)-9-[[(2R,4aR,6R,7R,8R,8aS)-7,8-dihydroxy-2-methyl-4,4a,6,7,8,8a-hexahydropyrano[5,6-d][1,3]dioxin-6-yl]oxy]-5-(4-hydroxy-3,5-dimethoxy-phenyl)-5a,8,8a,9-tetrahydro-5H-isobenzofurano[6,5-f][1,3]benzodioxol-6-one, (5R,5aR,8aR,9S)-9-[[(2R,4aR,6R,7R,8R,8aS)-7,8-dihydroxy-2-methyl-4,4a,6,7,8,8a-hexahydropyrano[5,6-d][1,3]dioxin-6-yl]oxy]-5-(4-hydroxy-3,5-dimethoxyphenyl)-5a,8,8a,9-tetrahydro-5H-isobenzofurano[6,5-f][1,3]benzodioxol-6-one;
EPIB, .alpha.-(p-Chlorophenoxy)isobutyric acid, ethyl ester, .alpha.-p-Chlorophenoxyisobutyryl ethyl ester, 19 more names available;
epibatidine, epibatidine;
Epicar, (+)-pilocarpine, (3S,4R)-3-ethyl-4-[(1-methyl-1H-imidazol-5-yl)methyl]dihydrofuran-2(3H)-one, (3S,4R)-3-ethyl-4-[(3-methyl-4-imidazolyl)methyl]-2-tetrahydrofuranone;
Epoprostenol, Epoprostanol, Epoprostenol, Epoprostenol Sodium; epoxybergamottin, epoxybergamottin;

epsilon-viniferin, epsilon-viniferin;
erastin, erastin;
ergosterol-5,8-peroxide, 3-hydroxy-5,7-epidioxyergosta-6,22-diene, 5,8-epidioxyergosta-6,22-dien-3-ol, ergosterol endoperoxide;
Eril, (5-ISOQUINOLINESULFONYL)HOMOPIPERAZINE, 1-(5-Isoquinolinesulfonyl)homopiperazine, 1-(5-Isoquinolinesulphonyl)homopiperazine;
erlotinib, CP 358,774, CP 358774, CP-358,774;
erucin, erucin;
Eryc, 6-(4-dimethylamino-3-hydroxy-6-methyl-oxan-2-yl)oxy-14-ethyl-7,12,13-trihydroxy-4-(5-hydroxy-4-methoxy-4,6-dimethyl-oxan-2-yl)oxy-3,5,7,9,11,13-hexamethyl-1-oxacyclotetradecane-2,10-dione, 6-(4-dimethylamino-3-hydroxy-6-methyl-tetrahydropyran-2-yl)oxy-14-ethyl-7,12,13-trihydroxy-4-(5-hydroxy-4-methoxy-4,6-dimethyl-tetrahydropyran-2-yl)oxy-3,5,7,9,11,13-hexamethyl-1-oxacyclotetradecane-2,10-dione, 6-(4-dimethylamino-3-hydroxy-6-methyl-tetrahydropyran-2-yl)oxy-14-ethyl-7,12,13-trihydroxy-4-(5-hydroxy-4-methoxy-4,6-dimethyl-tetrahydropyran-2-yl)oxy-3,5,7,9,11,13-hexamethyl-1-oxacyclotetradecane-2,10-quinone;
erythritol anhydride, erythritol anhydride;
esterbut-3, esterbut-3;
Estriol, 16 alpha Hydroxy Estradiol, 16 Epiestriol, 16 Hydroxyestradiol;
ET18-Ome, ( )-ET-18-OCH3, ( )-ET-18-OMe, (2-methoxy-3-octadecoxy-propyl) 2-trimethylammonioethyl phosphate;
Etfc cpd, 2H-1-Benzopyran-2-one, 7-ethoxy-4-(trifluoromethyl)-, 7-ethoxy-4-(trifluoromethyl)-2-chromenone, 7-Ethoxy-4-(trifluoromethyl)-2H-1-benzypyran-2-one;
Ethacrynic Acid, Edecrin, Etacrynic Acid, Ethacrinic Acid; Ethan, Aethan, Alkanes, C1-2, Alkanes, C2-3;
Ethinyl-oestradiol, Ethinyl-oestradiol;
Ethylmorphine, Dionine, Ethomorphine, Ethylmorphine;
Ethynodiol Diacetate, (3 beta, 17 alpha)-19-Norpregn-4-en-20-yne-3,17 diol Diacetate, Continuin, Ethyndiol Diacetate;
Eticol, Chinorta, Chinorto, diethyl (4-nitrophenyl) phosphate; Etidronic Acid, (1-hydroxyethylene)diphosphonic acid, (1-hydroxyethylene)diphosphonic acid, Tetrapotassium Salt, 1,1-hydroxyethylenediphosphonate;
Etodolac, AY 24236, AY-24,236, AY-24236;
Etoposide, alpha-D-Glucopyranosyl Isomer Etoposide, Baxter Brand of Etoposide, Baxter Oncology Brand of Etoposide;
etoricoxib, Arcoxia, etoricoxib, L-791456;
etravirine, etravirine, R165335, TMC 125;
Eufor, (+) or (-)-N-Methyl-3-phenyl-3-((alpha,alpha,alpha-trifluoro-p-tolyl)oxy)propylamine, (+) or (-)-N-Methyl-gamma-(4-(trifluoromethyl)phenoxy)benzenepropanamine, (+-)-N-Methyl-3-phenyl-3-((alpha,alpha,alpha-trifluoro-p-tolyl)oxy)propylamine;
Eugenol, 1,3,4-Eugenol, 1-Hydroxy-2-methoxy-4-allylbenzene, 1-Hydroxy-2-methoxy-4-prop-2-enylbenzene;
eupatilin, eupatilin;
everolimus, 40-O-(2-hydroxyethyl)-rapamycin, Certican, everolimus;
Evex, (+)-3,17beta-Estradiol, (13S,17S)-13-methyl-7,8,9,11,12,13,14,15,16,17-decahydro-6H-cyclopenta[a]phenanthrene-3,17-beta-diol, (17beta)-estra-1(10),2,4-triene-3,17-diol;
Evodin, (4aS,6aR,8aR,8bR,9aS,12R,12aS,14aR,14bR)-12-(3-furyl)-6,6,8a,12a-tetramethyldecahydro-3H-oxireno[d]pyrano[4',3':3,3a][2]benzofuro[5,4-f]isochromene-3,8,10(6H,9aH)-trione, 7,16-Dioxo-7,16-dideoxylimondiol, Citrolimonin;
exenatide, AC 2993, AC 2993 LAR, Byetta;
Exosurf, Alevaire, Enuclene, Exosurf;
Expectorants,
Expectorants, Mucolytic Agents, Mucolytics;
Extina, (+-)-cis-1-Acetyl-4-(p-((2-(2,4-dichlorophenyl)-2-(imidazol-1-ylmethyl)-1,3-dioxolan-4-yl)methoxy)phenyl) piperazine, (-)-Ketoconazole, (2S,4R)-ketoconazole;
Ezerin, (3aS,8aR)-1,3a,8-trimethyl-1,2,3,3a,8,8a-hexahydropyrrolo[2,3-b]indol-5-yl methylcarbamate, (3aS-cis)-1,2,3,3a,8,8a-Hexahydro-1,3a,8-trimethylpyrrolo(2,3-b)indol-5-ol methylcarbamate (ester), 1,2,3,3abeta,8abeta-Hexahydro-1,3a,8-trimethylpyrrolo(2,3-b)-indol-5-yl methylcarbamate;
ezetimib, ezetimib;
Facet, 3,7-Dichloro-8-quinolinecarboxylic acid, 3,7-Dichloroquinoline-8-carboxylic acid, 8-Quinolinecarboxylic acid, 3,7-dichloro-;
Facid, (2S)-2-[[4-[(2-amino-4-keto-1H-pteridin-6-yl)methylamino]benzoyl]amino]glutaric acid, (2S)-2-[[4-[(2-amino-4-oxo-1H-pteridin-6-yl)methylamino]benzoyl]amino]pentanedioic acid, (2S)-2-[[4-[(2-amino-4-oxo-1H-pteridin-6-yl)methylamino]phenyl]carbonylamino]pentanedioic acid;
facile, facile;
Factor IIa, Factor IIa;
FAMP, 2-F-ara-AMP, 2-Fluoro-9-(5-O-phosphono-beta-D-arabinofuranosyl)-9H-purin-6-amine, 2-Fluoro-ARA AMP;
Fanchinine, (+)-Tetrandrine, (+-)-Tetrandine, (+-)-Tetrandine; DL-Tetrandine;
Farnesyl-PP, (2E,6E)-3,7,11-trimethyldodeca-2,6,10-trien-1-yl trihydrogen diphosphate, (2E,6E)-Farnesyl diphosphate, (2E,6E)-Farnesyl pyrophosphate;
farnesylthiosalicylic acid, farnesylthiosalicylic acid, S-farnesylthiosalicylic acid, S-trans,trans-farnesylthiosalicylic acid;
febuxostat, 2-(3-cyano-4-isobutoxyphenyl)-4-methyl-5-thiazolecarboxylic acid, febuxostat, TEI 6720;
felbamate, 2-phenyl-1,3-propanediol dicarbamate, ADD-03055, Essex brand of felbamate;
Felodipine, 1A Brand of Felodipine, AbZ Brand of Felodipine, Agon;
Fenfluramine, Fenfluramine, Fenfluramine Hydrochloride, Fenfluramine Hydrochloride, (+-)-Isomer;
fenitrothion, fenitrothion, MEP, O,O-dimethyl O-(3-methyl-4-nitrophenyl) thiophosphate;
fenofibric acid, 2-(4-(4'-chlorophenoxy)phenoxy)propionic acid, fenofibric acid, fenofibric acid potassium salt;
Fenretinide, 13-cis-Isomer Fenretinide, 4
Hydroxyphenylretinamide, 4-HPR;
Fentanyl, Cephalon Brand of Fentanyl Buccal OraVescent, Fentanest, Fentanyl;
ferulic acid, 4-hydroxy-3-methoxycinnamic acid, 8,8'-diferulic acid, ferulic acid;
Filipin, Desoxylagosin, Filimarisin, Filipin;
fingolimod, 2-amino-2-(2-(4-octylphenyl)ethyl)-1,3-propanediol hydrochloride, fingolimod, fingolimod hydrochloride;
fipronil, 5-amino-1-[2,6-dichloro-4-(trifluoromethyl)phenyl]-4-[(trifluoromethyl)sulfinyl]-1H-pyrazole-3-carbonitrile, fipronil;
fisetin, 2-(3,4-Dihydroxyphenyl)-3,7-dihydroxy-4H-1-benzopyran-4-one, 2-(3,4-dihydroxyphenyl)-3,7-dihydroxy-4H-chromen-4-one, 3,3',4',7-Tetrahydroxyflavone;
Flanin F, 1H-Purin-6-amine, flavin dinucleotide, 1H-Purin-6-amine, flavine dinucleotide, Adenine-flavin dinucleotide;
Flavon, 2-Phenyl-.gamma.-benzopyrone, 2-Phenyl-4-benzopyron, 2-phenyl-4-chromenone;
flavonols, a flavonol, flavonols;
flavopiridol, (-)cis-5,7-dihydroxy-2-(2-chlorophenyl)-8-(4-(3-hydroxy-1-methyl)piperidinyl)-4H-1-benzopyran-4-one, flavopiridol, HMR 1275;
Flavyl, 1-Propanamine, 3-(10,11-dihydro-5H-dibenzo(a,d)cyclohepten-5-ylidene)-N,N-dimethyl-, 1-Propanamine, 3-(10,11-dihydro-5H-dibenzo[a,d]cyclohepten-5-ylidene)-N,N-dimethyl-, 10,11-Dihydro-5-(gamma-dimethylaminopropylidene)-5H-dibenzo(a,d)cycloheptene;
FLCZ, .alpha.-(2,4-Difluorophenyl)-.alpha.-(1H-1,2,4-triazol-1-ylmethyl)-1H-1,2,4-triazole-1-ethanol, 1H-1,2,4-Triazole-1-ethanol, .alpha.-(2,4-difluorophenyl)-.alpha.-(1H-1,2,4-triazol-1-ylmethyl)-, 1H-1,2,4-Triazole-1-ethanol, alpha-(2,4-difluorophenyl)-alpha-(1H-1,2,4-triazol-1-ylmethyl)-;
Flecainide, 3M Brand of Flecainide Acetate, Alphapharm Brand of Flecainide Acetate, Alpharma Brand of Flecainide Acetate;
Floxacillin, Floxacillin, Flucloxacillin, Fluorochloroxacillin; flufenamic acid, 2-[3-(trifluoromethyl)anilino]benzoic acid, 2-[[3-(TRIFLUOROMETHYL)PHENYL]AMINO] BENZOIC ACID, 3'-trifluoromethyldiphenylamine-2-carboxylic acid;
Flunitrazepam, 1A Brand of Flunitrazepam, betapharm Brand of Flunitrazepam, ct Arzneimittel Brand of Flunitrazepam;
fluorexon, fluorexon;
Fluorouracil, 5 Fluorouracil, 5 Fluorouracil biosyn, 5 FU Lederle;
fluvoxamine, (1E)-5-methoxy-1-[4-(trifluoromethyl)phenyl]pentan-1-one O-(2-aminoethyl)oxime, fluvoxamine;
FOLATE-ANALOG, FOLATE-ANALOG;
fondaparinux, Arixtra, fondaparinux, fondaparinux sodium; Fonofos, Dyfonate, Dyphonate, Fonofos;
Format, 2-Pyridine carboxylic acid, 3,6-dichloro-, 2-Pyridinecarboxylic acid, 3,6-dichloro-, 3,6-Dichloro-2-pyridinecarboxylic acid;
Formyl-Tetrahydrofolate, Formyl-Tetrahydrofolate;
Forskolin, Coleonol, Forskolin;
fosamprenavir, (3-(((4-aminophenyl)sulfonyl)(2-methylpropyl)amino)-1-(phenylmethyl)-2-(phosphonooxy)propyl) carbamic acid C-(tetrahydro-3-furanyl) ester, fos-amprenavir, fosamprenavir;
Foscarnet, Foscarnet, Foscarnet Barium (2:3) Salt, Foscarnet Calcium (2:3) Salt;
FR 120480, FR 120480;
FR 235222, FR 235222, FR-235222, FR235222;
fraxin, fraxin;

FTY 720P, FTY 720P, FTY-720P, FTY720P;
fucoidan, fucan sulfate, fucan sulfate Hor-1, fucoidan; fulvestrant, 7-(9-(4,4,5,5,5-pentafluoropentylsulfinyl)nonyl) estra-1,3,5(10)-triene-3,17-diol, AstraZeneca brand of fulvestrant, Faslodex;
fumagillin, (2E,4E,6E,8E)-10-({(3R,4S,5S,6R)-5-methoxy-4-[(2R,3R)-2-methyl-3-(3-methylbut-2-en-1-yl)oxiran-2-yl]-1-oxaspiro[2.5]oct-6-yl}oxy)-10-oxodeca-2,4,6,8-tetraenoic acid, 2,4,6,8-decatetraenedioic acid, 4-(1,2-epoxy-1,5-dimethyl-4-hexenyl)-5-methoxy-1-oxaspiro(2,5)oct-6-yl ester, Fugillin;
Fura-2, Fura 2, Fura-2;
furafylline, furafylline;
Furamon, (2-Furylmethyl)trimethylammonium iodide, 2-Furanmethanaminium, N,N,N-trimethyl-, iodide, 2-Furanmethanaminium, N,N,N-trimethyl-, iodide (9CI); Furylfuramide, AF 2, AF-2, AF2;
Gabexate, Foy, Gabexate, Gabexate Mesilate;
gadolinium, 64Gd, gadolinio, gadolinium;
Gadolinium DTPA, Berlex Brand of Gadopentetate Dimeglumine, Gadolinium Diethylenetriaminepenta acetic Acid, Gadolinium Diethylenetriaminepenta-acetic Acid;
galactocerebroside, galactocerebroside;
galactomannan, galactomannan;
galangin, 3,5,7-trihydroxy-2-phenyl-4H-benzopyran-4-one, 3,5,7-trihydroxy-2-phenyl-4H-chromen-4-one, 3,5,7-Trihydroxyflavone;
galaturonate, (2R,3S,4S,5R)-3,4,5,6-tetrahydroxyoxane-2-carboxylic acid, (2R,3S,4S,5S,6R)-3,4,5,6-tetrahydroxyoxane-2-carboxylic acid, (2S,3R,4S,5R,6R)-3,4,5,6-tetrahydroxyoxane-2-carboxylic acid;
gallic acid, 3,4,5-trihydroxybenzoic acid, gallic acid, Pyrogallol-5-carboxylic acid;
Gallogen, (1)Benzopyrano(5,4,3-cde)(1)benzopyran-5,10-dione, 2,3,7,8-tetrahydroxy-, 2,3,7,8-Tetrahydroxy(1) benzopyrano(5,4,3-cde)(1)benzopyran-5,10-dione, 2,3,7,8-Tetrahydroxy(1)benzopyrano(5,4,3-cde)-(1)benzopyran-5,10-dione;
gambierol, gambierol;
Gambogic acid, Gambogic acid;
gamma-butyric-acid, gamma-butyric-acid;
Ganciclovir, BIOLF-62, BW-759, Cytovene;
gastrin 17, gastrin 17;
gatifloxacin, 1-cyclopropyl-1,4-dihydro-6-fluoro-8-methoxy-7-(3-methyl-1-piperazinyl)-4-oxo-3-quinolinecarboxylic acid, AM 1155, AM-1155;
gefitinib, 4-(3'-chloro-4'-fluoroanilino)-7-methoxy-6-(3-morpholinopropoxy)quinazoline, COc1cc2ncnc(Nc3ccc(F)c(Cl)c3)c2cc1OCCCN4CCOCC4, gefitinib;
Geldanamycin, 2-Azabicyclo[16.3.1]docosa-4,6,10,18,21-pentaene-3,20,22-trione, 9,13-dihydroxy-8,14,19-trimethoxy-4,10,12,16-tetramethyl-, 9-carbamate (8CI), 2-azabicyclo[16.3.1]docosa-4,6,10,18,21-pentaene-3,20,22-trione, 9-[(aminocarbonyl)oxy]-13-hydroxy-8,14,19-trimethoxy-4,10,12,16-tetramethyl-, (4E,6Z,8S,9S,10E,12S, 13R,14S,16R)-, 2-Azabicyclo[16.3.1]docosa-4,6,10,18,21-pentaene-3,20,22-trione, 9-[(aminocarbonyl)oxy]-13-hydroxy-8,14,19-trimethoxy-4,10,12,16-tetramethyl-, [8S-(4E,6Z,8R*,9R*,10E,12R*,13S*,14R*,16S*)]-;
Gemfibrozil, 1A Brand of Gemfibrozil, Alphapharm Brand of Gemfibrozil, Apo Gemfibrozil;
gemtuzumab, CMA 676, CMA-676, gemtuzumab;
Gentamicins, G Myticin, G-Myticin, Garamycin;
gepirone, gepirone;
geraniol, (2E)-3,7-dimethyl-2,6-octadien-1-ol, (2E)-3,7-dimethylocta-2,6-dien-1-ol, (E)-3,7-dimethyl-2,6-octadien-1-ol; geranylcoumarin, geranylcoumarin;
Gestodene, 13-ethyl-17-hydroxy-18,19-dinor-17 alpha-pregna-4,15-dien-20-yn-3-one, 17-alpha-ethinyl-13-ethyl-17 beta-hydroxy-4,15-gonadien-3-one, Gestoden;
GF 120918, Elacridar, GF 120918, GF-120918;
GGTI 298, GGTI 298, GGTI-298;
GI 129471, GI 129471;
gingerol, (6)-gingerol, 6-gingerol, gingerol;
ginsenoside Rd, ginsenoside Rd, ginsenoside-Rd;
ginsenoside Rf, ginsenoside Rf;
ginsenoside Rg1, ginsenoside Rg1, ginsenoside-Rg(1), sanchinoside C(1);
ginsenoside Rh2, ginsenoside Rh2;
Ginsenosides, Ginsenosides, Panaxosides, Sanchinosides;
GLCa, (2S,3R,4S,5R)-3,4,5-trihydroxy-6-keto-pipecolinic acid, (2S,3R,4S,5R)-3,4,5-trihydroxy-6-oxo-2-piperidinecarboxylic acid, (2S,3R,4S,5R)-3,4,5-trihydroxy-6-oxo-piperidine-2-carboxylic acid;
Gliclazide, Alphapharm Brand of Gliclazide, Diabrezide, Diaglyk; Glumin, (2S)-2,5-diamino-5-keto-valeric acid, (2S)-2,5-diamino-5-oxo-pentanoic acid, (2S)-2,5-diamino-5-oxopentanoic acid;
Glyoxal, Ethanedial, Ethanedione, Glyoxal;
Gnidimacrin, Gnidimacrin;
GnRH, cystorelin, dirigestran, factrel;
Go 6976, Go 6976, Go-6976, Go6976;
gossypol, gossypol;
GR 79236X, (2S,3S,4S,5R)-2-[6-[(2-hydroxycyclopentyl)amino]-9-purinyl]-5-(hydroxymethyl)tetrahydrofuran-3,4-diol, (2S,3S,4S,5R)-2-[6-[(2-hydroxycyclopentyl)amino]purin-9-yl]-5-(hydroxymethyl)oxolane-3,4-diol, (2S,3S,4S,5R)-2-[6-[(2-hydroxycyclopentyl)amino]purin-9-yl]-5-(hydroxymethyl)tetrahydrofuran-3,4-diol;
gramicidin S, 1,10-anhydro(L-leucyl-D-phenylalanyl-L-prolyl-L-valyl-L-ornithyl-L-leucyl-D-phenylalanyl-L-prolyl-L-valyl-L-ornithine), Cyclo(L-valyl-L-ornithyl-L-leucyl-D-phenylalanyl-L-prolyl-L-valyl-L-ornithyl-L-leucyl-D-phenylalanyl-L-prolyl), Gramicidin C;
Granisetron, 1-Methyl-N-(endo-9-Methyl-9-Azabicyclo(3.3.1)non-3-yl)-1H-Indazole-3-Carboxamide, BRL 43694, BRL 43694A;
Gravistat, Gravistat;
Grofo, Bonidel, Brodan, Chloropyrifos solution;
Guggulsterone, (17E)-Pregna-4,17(20)-diene-3,16-dione, (17E)-pregna-4,17-diene-3,16-dione, (8R,9S,10R,13S,14S,17E)-17-ethylidene-10,13-dimethyl-1,2,6,7,8,9,11,12,14,15-decahydrocyclopenta[a]phenanthrene-3,16-dione;
GW 4064, GW 4064, GW-4064;
GW 501516, GW 1516, GW 501516, GW-1516;
H 89, H 87, H 89, H-87;
Halan, (R)-2-Bromo-2-chloro-1,1,1-trifluoroethane, 1,1,1-Trifluoro-2-bromo-2-chloroethane, 1,1,1-Trifluoro-2-chloro-2-bromoethane;
halofuginone, 7-bromo-6-chlorofebrifugine, halofuginone, halofunginone;
harmine, harmine;
Harzol, (3beta)-stigmast-5-en-3-ol, (3S,8S,9S,10R,13R,14S,17R)-17-[(1R,4R)-4-ethyl-1,5-dimethyl-hexyl]-10,13-dimethyl-2,3,4,7,8,9,11,12,14,15,16,17-dodecahydro-1H-cyclopenta[a]phenanthren-3-ol, (3S,8S,9S,10R,13R,14S,17R)-17-[(1R,4R)-4-ethyl-1,5-dimethylhexyl]-10,13-dimethyl-2,3,4,7,8,9,11,12,14,15,16,17-dodecahydro-1H-cyclopenta[a]phenanthren-3-ol;
hassium, 108Hs, hahnium, hassio;
HDMTX, ()Amethopterin, (+)-Amethopterin, 2-[[4-[(2,4-diaminopteridin-6-yl)methyl-methyl-amino]benzoyl]amino]glutaric acid;
Hecogenin, Hecogenin;
Hectorol, (1R,3S,5Z)-5-[(2E)-2-[(1R,3aS,7aR)-1-[(E,2R,5R)-5,6-dimethylhept-3-en-2-yl]-7a-methyl-2,3,3a,5,6,7-hexahydro-1H-inden-4-ylidene]ethylidene]-4-methylidene-cyclohexane-1,3-diol, (1R,3S,5Z)-5-[(2E)-2-[(1R,3aS,7aR)-1-[(E,2R,5R)-5,6-dimethylhept-3-en-2-yl]-7a-methyl-2,3,3a,5,6,7-hexahydro-1H-inden-4-ylidene]ethylidene]-4-methylidenecyclohexane-1,3-diol, (1R,35,5Z)-5-[(2E)-2-[(1R,3aS,7aR)-7a-methyl-1[(E,1R,4R)-1,4,5-trimethylhex-2-enyl]-2,3,3a,5,6,7-hexahydro-1H-inden-4-ylidene]ethylidene]-4-methylene-cyclohexane-1,3-diol;
Heet, Heet;
helenalin, helenalin;
Hemicholinium 3, Hemicholinium, Hemicholinium 3;
herbimycin, geldanamycin, 17-demethoxy-15-methoxy-11-O-methyl-, (15R)-, herbimycin, herbimycin A;
hesperadin, hesperadin;
HESPERETIN, 3',5,7-Trihydroxy-4'-methoxyflavanone, 4H-1-Benzopyran-4-one, 2,3-dihydro-5,7-dihydroxy-2-(3-hydroxy-4-methoxyphenyl)-, (S)-, 5,7,3'-Trihydroxy-4'-methoxyflavanone;
Hexadimethrine, 1,5-Dimethyl-1,5-Diazaundecamethylene Polymethobromide, Hexadimethrine, Hexadimethrine Bromide;
hexarelin, hexarelin;
Hgln, (+-)-Glutamine, (2R)-2,5-diamino-5-oxopentanoic acid, .gamma.-Glutamine;
himbacine, himbacine, NSC-23969, NSC23969;
Hk, Hk;
Hocus, (-)(5.alpha.,6.alpha.)-7,8-Didehydro-4,5-epoxy-17-methylmorphinan-3,6-diol, (-)-Heroin hydrochloride, (-)-Morphine;
HOE 33342, H33342, HOE 33342, HOE-33342;
honokiol, honokiol;
Horner, (+)-(5Z,7E)-26,26,26,27,27,27-Hexafluoro-9,10-secocholesta-5,7,10(19)-triene-1alpha,3beta,25-triol, (1R,3S,5Z)-5-[(2E)-2-[(1R,3aS,7aR)-7a-methyl-1-[(1R)-6,6,6-trifluoro-5-hydroxy-1-methyl-5-(trifluoromethyl)hexyl]-2,3,3a,5,6,7-hexahydro-1H-inden-4-ylidene]ethylidene]-4-methylene-cyclohexane-1,3-diol, (1R,3S,5Z)-5-[(2E)-2-[(1R,3aS,7aR)-7a-methyl-1-[(1R)-6,6,6-trifluoro-5-hydroxy-1-methyl-5-(trifluoromethyl)hexyl]-2,3,3a,5,6,7-hexahydro-1H-inden-4-ylidene]ethylidene]-4-methylenecyclohexane-1,3-diol;
HS 1200, HS 1200, HS-1200, HS1200;
HU 211, 1,1-dimethylheptyl-11-hydroxytetrahydrocannabinol, 1,1-dimethylheptyl-7-hydroxy-delta(6)-tetrahydrocannabinol, 11-hydroxy-delta(8) -tetrahydrocannabinol-dimethylheptyl;
HyateC, antihemophilic factor, blood coagulation factor viii, coagulation factor viii;
Hydoxin, 2-methyl-3-hydroxy-4,5-bis(hydroxy-methyl) pyridine, 2-Methyl-3-hydroxy-4,5-bis(hydroxymethyl)pyridine, 2-Methyl-3-hydroxy-4,5-di(hydroxymethyl)pyridine;
hydride, hydride, hydrogen anion;
Hydromorphone, Dihydromorphinone, Dilaudid, Hydromorphon;
Hydroxychloroquine, Hydroxychlorochin, Hydroxychloroquine, Hydroxychloroquine Sulfate;
hydroxycotinine, 1-methyl-3-hydroxy-5-(3-pyridyl)-2-pyrrolidinone, 3'-hydroxycotinine, hydroxycotinine;
hydroxylamine, dihydridohydroxidonitrogen, H2NHO, HYDROXYAMINE; Hydroxytryptophol, Hydroxytryptophol;
Hyhorin, Conestoral, Conjugated Estrogens, Conjugated estrogens: sodium estrone sulfate;
Hypaque, 3,5-diacetamido-2,4,6-triiodo-benzoic acid; sodium, 3,5-diacetamido-2,4,6-triiodobenzoic acid; sodium, Diatrizoate;
hyperforin, hyperforin, octahydrohyperforin, tetrahydrohyperforin;
hypericin, hypericin, mono-(123I)iodohypericin;
Hypericum-perforatum, Hypericum-perforatum;
hypochlorous acid, Chlor(I)-saeure, chloranol, HClO;
iberin, iberin;
IBMX, 1-methyl-3-(2-methylpropyl)-3,7-dihydro-1H-purine-2,6-dione, 1-methyl-3-(2-methylpropyl)-3,9-dihydro-1H-purine-2,6-dione, 1-methyl-3-(2-methylpropyl)-7H-purine-2,6-dione;
ibopamine, Escandine, ibopamine, ibopamine hydrochloride;
ibudilast, 3-isobutyryl-2-isopropylpyrazolo(1,5-a)pyridine, ibudilast, KC 404;
IC 831423, IC 831423;
icariin, icariin;
icaritin, icaritin;
icilin, AG-3-5 compound, icilin;
ICRF 193, ICRF 193;
IDS 23, IDS 23, IDS-23, Rheuma-Hek;
Ifosfamide, Asta Z 4942, Holoxan, Ifosfamide;
Ikarugamycin, Ikarugamycin;
ilimaquinone, ilimaquinone;
Iloprost, Ciloprost, CoTherix Brand of Iloprost, Iloprost;
Imadyl, (+-)-6-Chloro-alpha-methylcarbazole-2-acetic acid, (.+-.)-6-Chloro-.alpha.-methylcarbazole-2-acetic acid, 2-(6-Chloro-9H-carbazol-2-yl)propanoic acid;
imatinib, CGP 57148, CGP-57148, CGP57148B;
imidafenacin, imidafenacin, KRP 197, KRP-197;
imidazo-pyridine, imidazo-pyridine;
imidazolidin-2-one, 1,3-ethyleneurea, 2-imidazolidinone, 2-imidazolidone;
imidazolidin-one, imidazolidin-one;
imidazolidine, C1CNCN1, imidazolidine;
Imidazoline, Imidazoline;
imidazolyl-disulfide, imidazolyl-disulfide;
Imipenem, Anhydrous Imipenem, Imipemide, Imipenem;
Imizin, 10,11-Dihydro-N,N-dimethyl-5H-dibenz[b,f]azepine-5-propanamine hydrochloride, 3-(5,6-dihydrobenzo[b][1]benzazepin-11-yl)-N,N-dimethyl-propan-1-amine hydrochloride, 3-(5,6-dihydrobenzo[b][1]benzazepin-11-yl)-N,N-dimethylpropan-1-amine hydrochloride;
Immulina, Immulina;
Immunoferon, Immunoferon, Inmunoferon;
Impulsin, Anandamide (16:0), Hexadecanamide, N-(2-hydroxyethyl)-, Hydroxyethylpalmitamide;
Imrecoxib, Imrecoxib;
Imutex, 1,3-Diaza-2,4-cyclopentadiene, 1,3-Diaza-2,4-cyclopentadiene-, 1,3-Diazole;
Indinavir, Crixivan, Indinavir, Indinavir Sulfate;
indiplon, indiplon, NBI 34060;

indirubin, indigo red, indirubin;
indole-3-acetic acid, 1H-indol-3-ylacetic acid, 2-(indol-3-yl)ethanoic acid, 3-Indolylessigsaeure;
indole-3-methanol, 1H-indol-3-ylmethanol, 3-hydroxymethylindole, 3-indolylcarbinol;
indolin-2-one, 3Z-3-((1H-pyrrol-2-yl)-methylidene)-1-(1-piperidinylmethyl)-1,3-2H-indol-2-one, indolin-2-one, tetrahydroindolinone;
indolin-one, indolin-one;
infliximab, Centocor brand of infliximab, Essex brand of infliximab, infliximab;
inhibin B, inhibin B;
INOmax, Amidogen, oxo-, INOmax, Mononitrogen monoxide;
inositol-1,3,4,5-tetrakisphosphate, inositol-1,3,4,5-tetrakisphosphate;
inulin, (1,2-beta-D-fructosyl)n, (2,1-beta-D-Fructosyl)n, (2->1)-beta-D-fructofuranan;
Iodoacetamide, Iodoacetamide;
iodomethane, CH3I, Iodmethan, iodomethane;
iodoresiniferatoxin, I-RTX cpd, iodo-resiniferatoxin, iodoresiniferatoxin;
Ionomycin, Ionomycin, SQ 23377, SQ-23377;
ionophore, ionophore, ionophores;
Iopanoic Acid, Cholevid, Iodopanoic Acid, Iopagnost;
Iophendylate, Ethiodan, Iodophendylate, Iofendylate;
IPADE, 1H-Purin-6-amine, N-(3-methyl-2-butenyl)-, 1H-Purin-6-amine, N-(3-methyl-2-butenyl)- (9CI), 3-methylbut-2-enyl-(7H-purin-6-yl)amine;
IPOMEANOL, 1-(3-Furanyl)-4-hydroxy-1-pentanone, 1-(3-Furyl)-4-hydroxy-1-pentanone, 1-(3-Furyl)-4-hydroxy-4-pentanone;
Iressa, (3-chloro-4-fluoro-phenyl)-[7-methoxy-6-(3-morpholinopropoxy)quinazolin-4-yl]amine, 4-(3'-Chloro-4'-fluoroanilino)-7-methoxy-6-(3-morpholinopropoxy)quinazoline, 4-Quinazolinamine, N-(3-chloro-4-fluorophenyl)-7-methoxy-6-(3-(4-morpholinyl)propoxy)-;
irinotecan, 7-ethyl-10-hydroxycamptothecin, ALIRI cpd, Camptosar;
irisolidone, irisolidone;
Isatin, 1H-Indole-2,3-dione, 2,3-Diketoindoline, 2,3-Dioxo-2,3-dihydroindole;
isaxonine, isaxonine, isopropylamino-2-pyrimidine phosphate, N-isopropyl-amino-2-pyrimidine orthophosphate;
isoamylol, 1-HYDROXY-3-METHYLBUTANE, 3-methyl-l-butanol, 3-methylbutan-1-ol;
isobutyl-methyl-Xanthine, isobutyl-methyl-Xanthine;
Isodonol, (1S,4AR,5S,6S,14S)-1,5,6,14-tetrahydroxy-4,4-dimethyl-8-methylenedecahydro-1H-6,11b-(epoxymethano)-6a,9-methanocyclohepta[a]naphthalen-7(8H)-one, Isodonol, Oridonin;
isoflavone, 3-phenyl-4H-1-benzopyran-4-one, 3-phenyl-4H-chromen-4-one, 3-Phenylchromone;
isoflurane, 1-chloro-2,2,2-trifluoroethyl difluoromethyl ether, 2-chloro-2-difluoromethoxy-1,1,1-trifluoroethane, Aerrane;
Isol, ()-2-Methyl-2,4-pentanediol, (+-)-2-Methyl-2,4-pentanediol, (4r)-2-Methylpentane-2,4-Diol;
Isoliquiritigenin, (2E)-1-(2,4-Dihydroxyphenyl)-3-(4-hydroxyphenyl)-2-propen-1-one, (2E)-1-(2,4-dihydroxyphenyl)-3-(4-hydroxyphenyl)prop-2-en-1-one, (E)-1-(2,4-Dihydroxyphenyl)-3-(4-hydroxyphenyl)-2-propen-1-one;
isometronidazole, isometronidazole;
isoprenoids, isoprenoid, isoprenoids;
Isopropyl Thiogalactoside, IPTG, Isopropyl 1 Thio beta D galactopyranoside, Isopropyl 1-Thio-beta-D-galactopyranoside;
Isoprostanes, Isoprostane,
Isoprostanes;
Isorhamnetin, Isorhamnetin;
isosilybin A, isosilybin A, isosilybin B;
Isosorbide Dinitrate, Cardonit 40, Dilatrate, Iso Bid; isothiocyanates, isothiocyanates;
Isotretinoin, 13 cis Retinoic Acid, 13-cis-Retinoic Acid, Accutane;
Isradipine, Dynacirc, Isradipine, Isradipine, (+-)-Isomer;
istradefylline, 8-(2-(3,4-dimethoxyphenyl)ethenyl)-1,3-diethyl-3,7-dihydro-7-methyl-1H-purine-2,6-dione, istradefylline, KW 6002;
Itraconazole, Itraconazole, R 51211, R-51211;
ivabradine, 7,8-dimethoxy-3-(3-(((4,5-dimethoxybenzocyclobutan-1-yl)methyl)methylamino)propyl)-1,3,4,5-tetrahydro-2H-benzazepin-2-one, ivabradine, S 16257;
Ivermectin, Eqvalan, Ivermectin, Ivermectin Merck Brand; ixabepilone, BMS 247550, BMS-247550, BMS247550;
jadomycin B, (1S,3aS)-1-[(2S)-butan-2-yl]-7-hydroxy-1,3a,5-trimethyl-2,8,13-trioxo-1,2,8,13-tetrahydro-3aH-benzo[b][1,3]oxazolo[3,2-f]phenanthridin-12-yl 2,6-dideoxy-alpha-L-ribo-hexopyranoside, jadomycin B;

Jexin, (+) Tubocurarine, (+)-Tubocurarine, 13H-4,6:21,24-Dietheno-8,12-metheno-1H-pyrido(3',2':14,15)(1,11)dioxacycloeicosino(2,3,4-ij)isoquinolinium, 2,3,13a,14,15,16,25,25a-octahydro-9,19-dihydroxy-18,29-dimethoxy-1,14,14-trimethyl-, (13aR,25aS)-;
JHW 015, 1-propyl-2-methyl-3-(1-naphthoyl)indole, JHW 015, JHW-015;
JTE 013, JTE 013, JTE-013, JTE013;
K 252, 3'-(S)-epi-K-252a, K 252, K 252a;
K-PAM, 2-Propenamide, 2-Propenamide, homopolymer, 2-Propeneamide;
K-SR, Acronitol, Addi-K, Apo-K;
kaempferol, 3,4',5,7-Tetrahydroxyflavone, 3,5,7-trihydroxy-2-(4-hydroxyphenyl)-4H-chromen-4-one, 4H-1-Benzopyran-4-one, 3,5,7-trihydroxy-2-(4-hydroxyphenyl)-5,7,4'-Trihydroxyflavonol;
kaempferol-3-O-(2,3,4-tri-O-acetyl-alpha-1-rhamnopyranoside), kaempferol-3-O-(2,3,4-tri-O-acetyl-alpha-1-rhamnopyranoside), KTARP cpd;
KAFA, 1-Acetamido-4-ethoxybenzene, 1-Acetyl-p-phenetidin, 4′-Ethoxyacetanilide;
Kaken, (1S-(1alpha(S*),3alpha,5beta))-4-(2-(3,5-Dimethyl-2-oxo-cyclohexyl))-2-hydroxyethyl-2,6-piperidinedione, .beta.-[2-(3,5-Dimethyl-2-oxocyclohexyl)-2-hydroxyethyl]glutarimide, 2,6-Piperidinedione, 4-(2-(3,5-dimethyl-2-oxocyclohexyl)-2-hydroxyethyl)-, (1S-(1alpha(S*),3alpha,5beta))-;
Kamalin, (E)-1-(6-((3-Acetyl-2,4,6-trihydroxy-5-methylphenyl)methyl)-5,7-dihydroxy-2,2-dimethyl-2H-1-benzopyran-8-yl)-3-phenyl-2-propen-1-one, (E)-1-[6-(3-acetyl-2,4,6-trihydroxy-5-methyl-benzyl)-5,7-dihydroxy-2,2-dimethyl-chromen-8-yl]-3-phenyl-prop-2-en-1-one, (E)-1-[6-[(3-acetyl-2,4,6-trihydroxy-5-methyl-phenyl)methyl]-5,7-dihydroxy-2,2-dimethyl-chromen-8-yl]-3-phenyl-prop-2-en-1-one;
Kaolin, Kaolin, Kaolinite;
Kathon 886, Kathon 886, Kathon 886 biocide, Kathon biocide;
KB 141, KB 141, KB-141, KB141 cpd;
Kemi, (+-)-Propranolol, (1)-1-(Isopropylamino)-3-(naphthyloxy)propan-2-ol, (2S)-1-naphthalen-1-yloxy-3-(propan-2-ylamino)propan-2-ol;
kenpaullone, 1-azakenpaullone, 9-bromo-7,12-dihydroindolo(3,2-d) (1)benzazepin-6(5H)-one, kenpaullone;
Ketamine, 2-(2-Chlorophenyl)-2-(methylamino)cyclohexanone,
Calipsol, Calypsol;
Keto-desogestrel, Keto-desogestrel;
Keto-pgflalpha, Keto-pgflalpha;
ketoglutarate, .alpha.-Ketoglutaric acid, .alpha.-Oxoglutaric acid, 2-ketoglutarate;
Kipca, 1,4-naphthalenedione, 2-methyl-, 1,4-Naphthalenedione, 2-methyl-, radical ion(1-), 1,4-Naphthoquinone, 2-methyl-;
KMD 3213, 1-(3-hydroxypropyl)-5-(2-(2-(2-(2,2,2-trifluoroethoxy)phenoxy)ethylamino)propyl)indoline-7-carboxamide, KMD 3213, KMD-3213;
KMTB, 2-keto-4-(methylthio)butyric acid, 2-Keto-4-methylthiobutanoic acid, 2-keto-4-methylthiobutyrate;
Kojic acid, 2-(Hydroxymethyl)-5-hydroxy-4H-pyran-4-one, 2-Hydroxymethyl-5-hydroxy-gamma-pyrone, 4H-Pyran-4-one, 5-hydroxy-2-(hydroxymethyl)-;
KR-31543, (2S,3R,4S)-6-amino-4-(N-(4-chlorophenyl)-N-(2-methyl-2H-tetrazol-5-ylmethyl)amino)-3,4-dihydro-2-dimethoxymethyl-3-hydroxy-2-methyl-2H-1-benzopyran, KR-31543;
KRM 1648, KRM 1648;
L 365260, L 365260;
L 740,093, 1-[(3R)-5-(3-azabicyclo[3.2.2]nonan-3-yl)-1-methyl-2-oxo-3H-1,4-benzodiazepin-3-yl]-3-(3-methylphenyl)urea, 1-[(3R)-5-(3-azabicyclo[3.2.2]nonan-3-yl)-2-keto-1-methyl-3H-1,4-benzodiazepin-3-yl]-3-(3-methylphenyl)urea, L 740,093;
L-454,560, L-454,560;
L-696,474, 1H-Cycloundec[d]isoindol-1-one, 15-(acetyloxy)-2,3,3a,4,5,6,6a,9,10,11,12,15-dodecahydro-6-hydroxy-4,10,12-trimethyl-5-methylene-3-(phenylmethyl)-, (3R*,3aS*,4R*,6R*,6aS*,7E,10R*,12R*,13E,15S*,15aS*), Cyto-Cytochalasin, L-696,474;
L-T3, (2S)-2-amino-3-[4-(4-hydroxy-3-iodo-phenoxy)-3,5-diiodo-phenyl]propanoic acid, (2S)-2-amino-3-[4-(4-hydroxy-3-iodo-phenoxy)-3,5-diiodo-phenyl]propionic acid, (2S)-2-amino-3-[4-(4-hydroxy-3-iodophenoxy)-3,5-diiodophenyl]propanoic acid;
LAAM, (-)-6-(Dimethylamino)-4,4-diphenyl-3-heptanol acetate (ester), (-)-alpha-Acetylmethadol, (1S,4S)-4-(dimethylamino)-1-ethyl-2,2-diphenylpentyl acetate;
lacidipine, Boehringer Ingelheim Brand of Lacidipine, Caldine, GlaxoSmithKline Brand of Lacidipine;
lactacystin, lactacystin;
lactisole, lactisole;
lamotrigine, 3,5-diamino-6-(2,3-dichlorophenyl)-as-triazine, BW-430C, Crisomet;

Lanol, (-)-Cholesterol, (3beta)-cholest-5-en-3-ol, (3H)-Cholesterol;
lansoprazole, 2-(((3-methyl-4-(2,2,2-trifluoroethoxy)-2-pyridyl)methyl)sulfinyl)-1H-benzimidazole, Abbot Brand of Lansoprazole, AG 1749;
lapatinib, GW 572016, lapatinib, N-(3-chloro-4-((3-fluorophenyl)methoxy)phenyl)-6-(5-(((2-(methylsulfonyl)ethyl) amino)methyl)-2-furanyl)-4-quinazolinamine;
laquinimod, laquinimod;
latrunculin A, latrunculin A;
latrunculin B, LAT-B, latrunculin B;
lavendustin A, lavendustin A;
LBH589, LBH 589, LBH589, NVP-LBH589;
leflunomide, Arava, Aventis Behring Brand of Leflunomide, Aventis Brand of Leflunomide;
lenalidomide, 2,6-Piperidinedione, 3-(4-amino-1,3-dihydro-1-oxo-2H- isoindol-2-yl)-, 3-(4-Amino-l-oxoisoindolin-2-yl)piperidine-2,6-dione, CC 5013;
Lendorm, 2-Bromo-4-(2-chlorophenyl)-9-methyl-6H-thieno(3,2-f) (1,2,4)triazolo(4,3-a)(1,4)diazepine, 2-bromo-4-(2-chlorophenyl)-9-methyl-6H-thieno[3,2-f][1,2,4]triazolo[4,3-a][1,4]diazepine, 2-Bromo-4-(o-chlorophenyl)-9-methyl-6H-thieno(3,2-f)-s-triazolo(4,3-a)(1,4)diazepine;
Lentinan, Lentinan;
leptomycin B, leptomycin B;
Leucovorin, 5 Formyltetrahydrofolate, 5 Formyltetrahydropteroylglutamate, 5-Formyltetrahydrofolate;
Leukotriene C4, Leukotriene C, Leukotriene C 1, Leukotriene C 4;
Leukotriene D4, Leukotriene D, Leukotriene D 4, Leukotriene D-4; leukotrienes, leucotriene, leucotrienes, Leukotrien;
Leupeptin, (2S)-N-[(2S)-2-acetamido-4-methyl-1-oxopentyl]-2-[(1-formyl-4-guanidinobutyl)amino]-4-methylpentanamide, (2S)-N-[(2S)-2-acetamido-4-methyl-pentanoyl]-2-[(1-formyl-4-guanidino-butyl)amino]-4-methyl-pentanamide, (2S)-N-[(2S)-2-acetamido-4-methyl-pentanoyl]-2-[(1-formyl-4-guanidino-butyl)amino]-4-methyl-valeramide;
Levamisole, Decaris, Dekaris, L-Tetramisole;
Levitra, 2-(2-Ethoxy-5-(4-ethylpiperazin-1-yl-1-sulfonyl)phenyl)-5-methyl-7-propyl-3H-imidazo(5,1-f)(1,2,4)triazin-4-one, 2-[2-ethoxy-5-(4-ethylpiperazin-1-yl)sulfonyl-phenyl]-5-methyl-7-propyl-1H-imidazo[5,1-f][1,2,4]triazin-4-one, 2-[2-ethoxy-5-(4-ethylpiperazin-1-yl)sulfonylphenyl]-5-methyl-7-propyl-1H-imidazo[5,1-f][1,2,4]triazin-4-one;
levobupivacaine, Abbott Brand of Levobupivacaine Hydrochloride, Chirocaine, levobupivacaine;
Levonorgestrel, Alcala Brand of Levonorgestrel, Aventis Pharma Brand of Levonorgestrel, Berlex Brand of Levonorgestrel;
levugen, (2R,3S,4S,5R)-2,5-bis(hydroxymethyl)oxolane-2,3,4-triol, 2,5-bis(hydroxymethyl)oxolane-2,3,4-triol, 2,5-bis(hydroxymethyl)tetrahydrofuran-2,3,4-triol;
liarozole, liarozole;
Lidocaine, 2 2EtN 2MePhAcN, 2-(Diethylamino)-N-(2,6-Dimethylphenyl)Acetamide, 2-2EtN-2MePhAcN;
lilopristone, 98,73, lilopristone, ZK 98.734;
Lipoate, (+)-alpha-Lipoic acid, (R)-()-1,2-Dithiolane-3-pentanoic acid, (R)-(+)-Lipoate;
Lipofectamine, LF 2000, LF-2000, LF2000;
lipoteichoic acid, lipoteichoic acid;
Lipoxins, Lipoxin, Lipoxins;
lissamine rhodamine B, lissamine rhodamine B;
lithocholic acid, (3alpha,5beta)-3-hydroxycholan-24-oic acid, 3alpha-hydroxy-5beta-cholan-24-oic acid, 3alpha-hydroxy-5beta-cholanic acid;
LMWH, 6-[5-acetamido-4,6-dihydroxy-2-(sulfooxymethyl)oxan-3-yl]oxy-3-[5-(6-carboxy-4,5-dihydroxy-3-sulfooxy-oxan-2-yl)oxy-6-(hydroxymethyl)-3-(sulfoamino)-4-sulfooxy-oxan-2-yl]oxy-4-hydroxy-5-sulfooxy-oxane-2-carboxylic acid, 6-[5-acetamido-4,6-dihydroxy-2-(sulfooxymethyl)oxan-3-yl]oxy-3-[5-(6-carboxy-4,5-dihydroxy-3-sulfooxy-oxan-2-yl)oxy-6-(hydroxymethyl)-3-(sulfoamino)-4-sulfooxyoxan-2-yl]oxy-4-hydroxy-5-sulfooxyoxane-2-carboxylic acid, 6-[5-acetamido-4,6-dihydroxy-2-(sulfooxymethyl)tetrahydropyran-3-yl]oxy-3-[5-(6-carboxy-4,5-dihydroxy-3-sulfooxy-tetrahydropyran-2-yl)oxy-6-(hydroxymethyl)-3-(sulfoamino)-4-sulfooxy-tetrahydropyran-2-yl]oxy-4-hydroxy-5-sulfooxy-tetrahydropyran-2-carboxyli;
LNAC, (2R)-2-acetamido-3-mercapto-propionic acid, (2R)-2-acetamido-3-mercaptopropanoic acid, (2R)-2-Acetamido-3-sulfanyl-propanoic acid;
lonafarnib, 4-(2-(4-(8-chloro-3,10-dibromo-6,11-dihydro-5H-benzo-(5,6)-cyclohepta(1,2-b)-pyridin-11(R)-yl)-1-piperidinyl)-2-oxo-ethyl)-1-piperidinecarboxamide, lonafarnib, SCH 66336;
Loperamide, Imodium, Loperamide, Loperamide Hydrochloride;
lopinavir, A-157378.0, ABT 378, ABT-378;
Loratadine, 4-(8-Chloro-5,6-dihydro-11H-benzo(5,6)cyclohepta(1,2-b)pyridin-11-ylidene)-1-piperidinecarboxylic Acid Ethyl Ester, Alavert, Claritin;

Lorazepam, AHP Brand of Lorazepam, Apo Lorazepam, Apo-Lorazepam;
Lorex, DEA No. 2783, Imidazo(1,2-a)pyridine-3-acetamide, N,N,6-trimethyl-2-(4-methylphenyl)-, Lorex;
lorglumide, lorglumide;
Losartan, 2-Butyl-4-chloro-1-((2'-(1H-etrazol-5-yl) (1,1'-biphenyl)-4-yl)methyl)-1H-imidazole-5-methanol, Cozaar, DuP 753;
Lovan, ()-N-Methyl-gamma-[4-(trifluoromethyl)phenoxy]benzenepropanamine hydrochloride, (+-)-Methyl-gamma-(4-(trifluoromethyl)phenoxy)benzenepropanamine hydrochloride, (+-)-N-Methyl-3-phenyl-3-(4-(trifluoromethyl) phenoxy)propylamine hydrochloride;
loxiglumide, loxiglumide;
LUF 5831, LUF 5831, LUF5831;
lupeol, lup-20 (29) -en-3-ol, lup-20(29)-en-3beta-ol, lup-20(29)-ene-3alpha-ol;
luteolin, 2-(3,4-dihydroxyphenyl)-5,7-dihydroxy-4-benzopyrone, 2-(3,4-dihydroxyphenyl)-5,7-dihydroxy-4H-1-benzopyran-4-one, 2-(3,4-dihydroxyphenyl)-5,7-dihydroxy-4H-chromen-4-one;
LY 117018, 6-hydroxy-2-(4-hydroxyphenyl)benzo(b)thien-3-yl 4-(2-(1-pyrrolidinyl)ethoxy) phenyl ketone, Lilly 117018, LY 117018;
LY 293111, 2-(2-propyl-3-(3-(2-ethyl-4-(4-fluorophenyl)-5-hydroxyphenoxy)propoxy)phenoxy)benzoic acid, LY 293111, LY-293111;
LY231514, LY231514;
LYCOPENE, (6Z,8E,10E,12E,14E,16E,18Z,20E,22Z,24E,26Z)-2,6,10,14,19,23,27,31-octamethyldotriaconta-2,6,8,10,12,14,16,18,20,22,24,26,30-tridecaene, .psi.,.psi.-Carotene, 2,6,8,10,12,14,16,18,20,22,24,26,30-Dotriacontatridecaene, 2,6,10,14,19,23,27,31-octamethyl-, (all-E)-;
lysophosphatidic acid, 1-O-oleyllysophosphatidic acid, 1-oleoyl-lysophosphatidic acid, LPA (lysophosphatidic acid);
Lysophosphatidylcholines, Lysolecithins, Lysophosphatidylcholine, Lysophosphatidylcholines;
Lysophosphatidylglycerol, Lysophosphatidylglycerol;
lysyl-arginyl-alanyl-lysyl-alanyl-lysyl-threonyl-threonyl-lysyl-lysyl-arginine, lysyl-arginyl-alanyl-lysyl-alanyl-lysyl-threonyl-threonyl-lysyl-lysyl-arginine;

M&B22948, 1,4-Dihydro-5-(2-propoxyphenyl)-1,2,3-triazolo(4,5-d)pyrimidin-7-one, 1,4-Dihydro-5-(2-propoxyphenyl)-7H-1,2,3-triazolo(4,5-d)pyrimidin-7-one, 1,4-Dihydro-5-(2-propoxyphenyl)-7H-1,2,3-triazolo[4,5-d]pyrimidine-7-one;
Malix, 1,2,3,4,7,7-Hexachlorobicyclo(2.2.1)hepten-5,6-bioxymethylenesulfite, 1,4,5,6,7,7-Hexachloro-5-norbornene-2,3-dimethanol cyclic sulfite, 1,4,5,6,7,7-Hexachloro-8,9,10-trinorborn-5-en-2,3-ylenedimethyl sulphite;
manidipine, 2-(4-diphenylmethyl-1-piperazinyl)ethyl methyl-1,4-dihydro-2,6-dimethyl-4-(3-nitrophenyl)-3,5-pyridinedicarboxylate, CV 4093, CV-4093;
manumycin, manumycin, manumycin A, UCFI-C;
maraviroc, 4,4-difluoro-N-((1S)-3-(exo-3-(3-isopropyl-5-methyl-4H-1,2,4-triazol-4-yl)-8-azabicyclo(3.2.1)oct-8-yl)-1-phenylpropyl)cyclohexanecarboxamide, maraviroc, Pfizer Brand of maraviroc;
Matrine, (+)-Matrine, .alpha.-Matrine, Matrene, (+)-;
MCYST-LR, (5R,8S,11R,12S,15S,18S,19S,22R)-15-(3-guanidinopropyl)-8-isobutyl-18-[(1E,3E,5S,6S)-6-methoxy-3,5-dimethyl-7-phenyl-hepta-1,3-dienyl]-1,5,12,19-tetramethyl-2-methylene-3,6,9,13,16,20,25-heptaoxo-1,4,7,10,14,17,21-heptazacyclopentacosane-11,22-dicarboxylic, (5R,8S,11R,12S,15S,18S,19S,22R)-15-(3-guanidinopropyl)-8-isobutyl-18-[(1E,3E,5S,6S)-6-methoxy-3,5-dimethyl-7-phenylhepta-1,3-dienyl]-1,5,12,19-tetramethyl-2-methylene-3,6,9,13,16,20,25-heptaoxo-1,4,7,10,14,17,21-heptazacyclopentacosane-11,22-dicarboxylic , (5R,8S,11R,12S,15S,18S,19S,22R)-15-(3-guanidinopropyl)-8-isobutyl-3,6,9,13,16,20,25-heptaketo-18-[(1E,3E,5S,6S)-6-methoxy-3,5-dimethyl-7-phenyl-hepta-1,3-dienyl]-1,5,12,19-tetramethyl-2-methylene-1,4,7,10,14,17,21-heptazacyclopentacosane-11,22-dicarboxyli;
Me-nle-asp-phe-NH2, Me-nle-asp-phe-NH2;
mead ethanolamide, 5,8,11-eicosatrienoyl ethanolamide (Z,Z,Z)-, mead ethanolamide;
MeAsO(OH)2, Arsonic acid, methyl-, DSMA (JMAF), MeAsO(OH)2;
Mebumal, 2,4,6(1H,3H,5H)-Pyrimidinetrione, 5-ethyl-5-(1-methylbutyl)-, 5-ethyl-2-hydroxy-5-(1-methylbutyl)pyrimidine-4,6(1H,5H)-dione, 5-Ethyl-5-(1-methylbutyl)-2,4,6(1H,3H,5H)-pyrimidinetrione;
Mechlorethamine, Bis(2-chloroethyl)methylamine, Caryolysine, Chlorethazine;
Medroxyprogesterone 17-Acetate, (6 alpha)-17-(Acetoxy)-6-methylpregn-4-ene-3,20-dione, 6 alpha Methyl 17alpha hydroxyprogesterone Acetate, 6-alpha-Methyl-17alpha-hydroxyprogesterone Acetate;
Mefenamic Acid, Antigen Brand of Mefenamic Acid, Apo Mefenamic, Apo-Mefenamic;
Megalomicin, (3R,4S,5S,6R,7R,9R,11R,12R,13S,14R)-4-[(2R,4R,5S,6S)-4,5-dihydroxy-4,6-dimethyl-oxan-2-yl] oxy-6-[(2S,3R,4S,6R)-4-dimethylamino-3-hydroxy-6-methyl-oxan-2-yl]oxy-7-[(2S,4R,6S)-4-dimethylamino-5-hydroxy-6-methyl-oxan-2-yl]oxy-14-ethyl-12,13-dihydroxy-3,5,7,9,

(3R,4S,5S,6R,7R,9R,11R,12R,13S,14R)-4-[(2R,4R,5S,6S)-4,5-dihydroxy-4,6-dimethyl-tetrahydropyran-2-yl]oxy-6-[(2S,3R,4S,6R)-4-dimethylamino-3-hydroxy-6-methyl-tetrahydropyran-2-yl]oxy-7-[(2S,4R,6S)-4-dimethylamino-5-hydroxy-6-methyl-tetrahydropyran-2-yl]oxy, (3R,4S,5S,6R,7R,9R,11R,12R,13S,14R)-4-[(2R,4R,5S,6S)-4,5-dihydroxy-4,6-dimethyl-tetrahydropyran-2-yl]oxy-6-[(2S,3R,4S,6R)-4-dimethylamino-3-hydroxy-6-methyl-tetrahydropyran-2-yl]oxy-7-[(2S,4R,6S)-4-dimethylamino-5-hydroxy-6-methyl-tetrahydropyran-2-yl]oxy;
Melarsoprol, Arsobal, Mel B, Melarsenoxid BAL;
Melatol, 5-Methoxy-N-acetyltryptamine, Acetamide, N-(2-(5-methoxy-1H-indol-3-yl)ethyl)- (9CI), Acetamide, N-(2-(5-methoxyindol-3-yl)ethyl)-;
meletin, 117-39-5 (NEUTRAL), 2-(3,4-Dihydroxy-phenyl)-3,5,7-trihydroxy-chromen-4-one, 2-(3,4-dihydroxyphenyl)-3,5,7-trihydroxy-4-chromenone;
melitten, D008555, melitten, melittin;
meloxicam, Abbott brand of meloxicam, Almirall brand of meloxicam, Boehringer Ingelheim brand of meloxicam;
Melphalan, 4-(Bis(2-chloroethyl)amino)phenylalanine, Alkeran, L-PAM;
Memantine, 1,3-Dimethyl-5-aminoadamantane, 1-Amino-3,5-dimethyladamantane, Axura;
menadiol, 2-methyl-1,4-naphthohydroquinone, 2-methyl-1,4-naphthoquinol, dihydrovitamin K3;
Menhaden oil, Menhaden oil;
menthofuran, menthofuran;
Meperidine, Demerol, Dolantin, Dolargan;
Mephenytoin, 5 Ethyl 3 Methyl 5 Phenylhydantoin, 5-Ethyl-3-Methyl-5-Phenylhydantoin, Mefenetoin;
mesalamine, 3-carboxy-4-hydroxyaniline, 5-amino-2-hydroxybenzoic acid, 5-Aminosalicylic acid;
Mesaton, (-)-m-Hydroxy-alpha-(methylaminomethyl)benzyl alcohol, (R)-2-Hydroxy-2-(3-hydroxyphenyl)-N-methylethylamine, 3-[(1R)-1-hydroxy-2-(methylamino)ethyl]phenol;
Meth, (+ )-methylamphetamine, (+)-(s)-deoxyephedrine, (+)-(s)-n-alpha-dimethylphenethylamine;
methanandamide, methanandamide;
methanethiosulfonate ethylammonium, 2-aminoethylmethanethiosulfonate, AEMTS cpd, methanethiosulfonate ethylamine;
Methimazole, 1 Methyl 2 mercaptoimidazole, 1-Methyl-2-mercaptoimidazole, Eli Lilly Brand of Methimazole;
methionyl-leucyl-phenylalanine, methionyl-leucyl-phenylalanine; Methorphan, (-)-3-Methoxy-N-methylmorphinan, 3-methoxy-17-methylmorphinan, 3-METHOXY-N-METHYLMORPHINAN, (-)-;
Methoxsalen, 8 Methoxypsoralen, 8 MOP, 8-Methoxypsoralen;
Methoxy-psoralen, Methoxy-psoralen;
methoxyamine, methoxyamine;
methoxychlor, 1,1'-(2,2,2-trichloroethane-1,1-diyl)bis(4-methoxybenzene), 1,1,1-trichloro-2,2-bis(p-anisyl)ethane, 1,1,1-trichloro-2,2-bis(p-methoxyphenyl)ethane;
methoxymorphinan, methoxymorphinan;
methyl chloroformate, methyl chloroformate;
Methyl glycine, (Methoxycarbonyl)methylamine, 2-aminoacetic acid methyl ester, Glycine methyl ester;
Methyl paraben, Methyl paraben;
methyl salicylate, 3M brand of methyl salicylate, esparma brand of methyl salicylate, Hevert brand of methyl salicylate;
methyl tryptophan, 2-Amino-3-(1H-indol-3-yl)-propionic acid methyl ester, 2-amino-3-(1H-indol-3-yl)propanoic acid methyl ester, 2-amino-3-(1H-indol-3-yl)propionic acid methyl ester;
methyl-dopa, methyl-dopa;
methyl-phosphorothioate, methyl-phosphorothioate;
methyl-Pyridinium, methyl-Pyridinium;
methylamine, aminomethane, methylamine, methylamine bisulfite; Methylamylnitrosamine, 1-Pentanamine, N-methyl-N-nitroso-, Methyl-N-amylnitrosamine, Methyl-N-pentylnitrosamine;
Methylene-tetrahydrofolate, Methylene-tetrahydrofolate;
methylenetetrahydrofolates, methylenetetrahydrofolate, methylenetetrahydrofolates;
methylglyoxal, 1,2-propanedione, 2-Ketopropionaldehyde, 2-oxopropanal;
methylnaltrexone, 17-(cyclopropylmethyl)-4,5-epoxy-3,14-dihydroxymorphinanium-6-one, methyl-naltrexone hydrobromide, methylnaltrexone;
methyloxidanyl, CH3-O(.), methoxy, methoxy radical;
methylparaben, 4-hydroxybenzoic acid methyl ester, methyl p-hydroxybenzoate, methylparaben;
methylphosphate, methyl phosphate disodium salt, hexahydrate, methylphosphate, methylphosphate diammonium salt;
Methylprednisolone, 6 Methylprednisolone, 6-Methylprednisolone, Medrol;
methylxanthines, methylxanthines;

Metoclopramide, 4 Amino-5-chloro-N-(2-(diethylamino)ethyl)-2-methoxybenzamide, Cerucal, Maxolon;
Metopiron, 1,2-Di-3-pyridyl-2-methyl-1-propanone, 1-Propanone, 1,2-di-3-pyridyl-2-methyl-, 1-Propanone, 2-methyl-1,2-di-3-pyridinyl-;
Metribolone, 17 BETA HYDROXY 17 ALPHA METHYLESTRA 4 9 11 TRIEN 3 ONE, 17 beta-Hydroxy-17 alpha-methylestra-4,9,11-trien-3-one, Methyltrienolone;
mevalonic acid, 3,5-dihydroxy-3-methylpentanoic acid, mevalonic acid;
micafungin, FK 463, FK-463, FK463;
miconazole, 1-(2,4-Dichloro-beta-((2,4-dichlorobenzyl)oxy)phenethyl)imidazole, 1-[2-(2,4-dichlorobenzyloxy)-2-(2,4-dichlorophenyl)ethyl]-1H-imidazole, Clclccc(COC(Cn2ccnc2)c3ccc(Cl)cc3Cl)c(Cl)cl;
Mictonorm, (1-methyl-4-piperidyl) 2,2-diphenyl-2-propoxy-acetate hydrochloride, (1-methylpiperidin-4-yl) 2,2-diphenyl-2-propoxy-ethanoate hydrochloride, (1-methylpiperidin-4-yl) 2,2-diphenyl-2-propoxyacetate hydrochloride;
Midazolam, Dormicum, Midazolam, Midazolam Hydrochloride;
Mifepristone, Contragest Brand of Mifepristone, Danco Brand of Mifepristone, Exelgyn Brand of Mifepristone;
MIII, 6-Methyl-5,7-dimethylthiopyrrolo[1,2-a]1,4-diazine, 7-methyl-6,8-bis(methylsulfanyl)pyrrolo[1,2-a]pyrazine, 7-Methyl-6,8-bis(methylthio)pyrrolo(1,2-a)pyrazine;
Milrinone,
Corotrop, Corotrope, Milrinone;
Mimosine, Leucaenine, Leucaenol, Leucenine;
mirtazapine, (N-methyl-11C)mirtazapine, 6-azamianserin, Celltech brand of mirtazapine;
Mit-C, 7-Amino-9.alpha.-methoxymitosane, Ametycin, Ametycine; mithramycin, (1S)-5-deoxy-1-C-[(2S,3S)-7-{[2,6-dideoxy-3-O-(2,6-dideoxy-beta-D-arabino-hexopyranosyl)-beta-D-arabino-hexopyranosyl]oxy}-3-{[2,6-dideoxy-3-C-methyl-beta-D-ribo-hexopyranosyl-(1->3)-2,6-dideoxy-beta-D-arabino-hexopyranosyl-(1->3)-2,6-dideoxy-beta-D-arabi, aureolic acid, Mithracin;
MitoTracker-Red, MitoTracker-Red;
Mitoxantrone, AHP Brand of Mitoxantrone Hydrochloride, Amgen Brand of Mitoxantrone Hydrochloride, ASTA Medica Brand of Mitoxantrone Hydrochloride;
mizolastine, Allphar brand of mizolastine, Galderma brand of mizolastine, Mistalin;
MLN8054, 4-((9-chloro-7-(2,6-difluorophenyl)-5H-pyrimidol(5,4-d)(2)benzazepin-2-yl)amino)benzoic acid, MLN8054;
mofarotene, 4-((2-(p-((E)-2-(5,6,7,8-tetrahydro-5,5,8,8-tetramethyl-2-naphthyl)propenyl)phenoxy)ethyl))morpholine, arotinoid Ro 40-8757, mofarotene;
Monensin, Coban, Monensin, Monensin A Sodium Complex;
mono-N-demethyladinazolam, mono-N-demethyladinazolam;
mono(2-ethylhexyl) phthalate, (2-ethylhexyl) hydrogen phthalate, 1,2-benzenedicarboxylic acid, mono(2-ethylhexyl) ester, 2-(2-ethylhexyloxycarbonyl)benzoic acid;
monoethylglycinexylidide, ethylglycylxylidide, L 86, L-86;
monomethylarsonic acid, disodium methanearsonate, methanearsonic acid, methylarsonate;
monoterpenes, monoterpene, monoterpenes;
monuron, 1,1-Dimethyl-3-(p-chlorophenyl)urea, 3-(4-chlorophenyl)-1,1-dimethylurea, 3-(p-Chlorophenyl)-1,1-dimethylurea;
MORIN, 2',3,4',5,7-Pentahydroxyflavone, 2',4',3,5,7-Pentahydroxyflavone, 2',4',5,7-Tetrahydroxyflavan-3-ol;
morpholine, C1COCCN1, morpholine, Tetrahydro-1,4-oxazine; morusin, morusin;
motexafin gadolinium, (PB-7-11-233'2'4)-bis(acetato-kappaO)(9,10-diethyl-20,21-bis(2-(2-(2-methoxyethoxy)ethoxy)ethoxy)-4,15-dimethyl-8,11-imino-3,6:16,13-dinitrilo-1,18-benzodiazacycloeicosine-5,14-dipropanolato-kappaN(1),kappaN(18),kappaN(23),kappaN(24),kappaN(25))gadolinium , bis(acetato-kappaO){3,3'-[4,5-diethyl-16,17-bis{2-[2-(2-methoxyethoxy)ethoxy]ethoxy}-10,23-dimethyl-13,20,25,26,27-pentaazapentacyclo[20.2.1.1 (3,6).1(8,11).0(14,19)]heptacosa-1,3,5,7,9,11(26),12,14,16,18,20,22(25),23-tridecaene-9,24-diyl-kappa(5)N(13), N(2, gadolinium texaphyrin;
Motuporin, Motuporin;
moxifloxacin, 1-cyclopropyl--7-(2,8-diazabicyclo(4.3.0)non-8-yl)-6-fluoro-8-methoxy-1,4-dihydro-4-oxo-3-quinolinecarboxylic acid, Actira, Avalox;
MPEG, 1,2-Dihydroxyethane, 1,2-Ethanediol, 146AR;
Muraglitazar, Muraglitazar;
mutalipocin II, ML-II, mutalipocin II;
mycophenolic acid, mycophenolic acid;
Mycose, .alpha.,.alpha.-Trehalose, .alpha.-D-Glucopyranoside, .alpha.-D-glucopyranosyl, .alpha.-D-Trehalose;
Myocol, (2R,3R,4S,5R)-2-(6-amino-9-purinyl)-5-(hydroxymethyl)tetrahydrofuran-3,4-diol, (2R,3R,4S,5R)-2-(6-aminopurin-9-yl)-5-(hydroxymethyl)oxolane-3,4-diol, (2R,3R,4S,5R)-2-(6-aminopurin-9-yl)-5-(hydroxymethyl)tetrahydrofuran-3,4-diol;

myricetin, 3,3',4',5,5',7-Hexahydroxyflavone, 3,5,7,3',4',5'-Hexahydroxyflavone, 3,5,7-trihydroxy-2-(3,4,5-trihydroxyphenyl)-4H-chromen-4-one;

myxothiazol, myxothiazol;

N-(2-cyclohexyloxy-4-nitrophenyl)methanesulfonamide, N-(2-cyclohexyloxy-4-nitrophenyl)methanesulfonamide, NS 398, NS-398;

N-(2-hydroxypropyl)methacrylamide, N-(2-hydroxypropyl)methacrylamide;

N-(3-(4-chlorophenyl)-2-(3-cyanophenyl)-1-methylpropyl)-2-methyl-2-((5-(trifluoromethyl)pyridin-2-yl)oxy)propanamide, MK 0364, MK-0364, MK0364;

N-(3-methoxyphenyl)-4-chlorocinnamanilide, N-(3-methoxyphenyl)-4-chlorocinnamanilide, SB 366791, SB366791;

N-(3-oxododecanoyl)homoserine lactone, 3-oxo-C12-HSL, 3-oxo-C8-HSL, 30-C12-HSL;

N-(4-(6-(4-(1-(4-fluorophenyl)ethyl)piperazin-1-yl)pyrimidin-4-yloxy)benzo(d)thiazol-2-yl)acetamide, AMG 628, AMG-628, AMG628; N-(4-(6-(4-trifluoromethylphenyl)pyrimidin-4-yloxy)benzothiazol-2-yl)acetamide, AMG 517, AMG-517, AMG517;

N-(4-cyano-benzo(b)thiophene-2-carbonyl)guanidine, KR-33028, N-(4-cyano-benzo(b)thiophene-2-carbonyl)guanidine;

N-(5-(((5-(1,1-dimethylethyl)-2-oxazolyl)methyl)thio)-2-thiazolyl)-4-piperidinecarboxamide, BMS 387032, BMS-387032, BMS387032;

N-acetylcysteine lysinate, L-NAC, N-acetylcysteine L-lysinate, N-acetylcysteine lysinate;

n-acetylmuramyl-1-alanyl-d-isoglutamine, 4-[2-[2-(3-acetylamino-2,5-dihydroxy-6-methylol-tetrahydropyran-4-yl)oxypropanoylamino]propanoylamino]-5-amino-5-oxo-pentanoic acid, 4-[2-[2-[3-acetylamino-2,5-dihydroxy-6-(hydroxymethyl)oxan-4-yl]oxypropanoylamino]propanoylamino]-5-amino-5-oxo-pentanoic acid, 4-[2-[2-[3-acetylamino-2,5-dihydroxy-6-(hydroxymethyl)tetrahydropyran-4-yl]oxy-1-oxo-propyl]amino-1-oxo-propyl]amino-5-amino-5-oxo-pentanoic acid;

N-acetylneuraminic acid, 5-Acetamido-3,5-dideoxy-D-glycero-D-galacto-2-nonulosonic acid, 5-acetamido-3,5-dideoxy-D-glycero-D-galacto-non-2-ulopyranosonic acid, N-acetylneuraminic acid;

N-desmethylclobazam, demethylclobazam, N-desmethylclobazam, norclobazam;

N-ethylmaleimide, 1-ethyl-1H-pyrrole-2,5-dione, Ethylmaleimide, N-ethylmaleimide;

N-methyl-N-(trimethylsilyl)trifluoroacetamide, N-methyl-N-(trimethylsilyl)trifluoroacetamide;

N-methylsulfonyl-6-(2-propargyloxyphenyl)hexanamide, MS-PPOH, N-methylsulfonyl-6-(2-propargyloxyphenyl)hexanamide;

N-oleoyldopamine, N-oleoyldopamine;

N-phenyl-1-naphthylamine, 1-anilinonaphthalene, 1-N-phenylnaphthylamine, N-phenyl-1-naphthylamine;

N,N,N',N'-tetramethylethylenediamine, CN(C)CCN(C)C, N,N,N',N'-tetramethyl-1,2-ethanediamine, N,N,N',N'-tetramethylethane-1,2-diamine;

N(6)-cyclohexyl-2-O-methyladenosine, N(6)-cyclohexyl-2-O-methyladenosine, SDZ WAG-994, SDZ-WAG-994;

N(6)-cyclopentyladenosine, N(6)-cyclopentyladenosine;

N3-IQ, (3-methylpyrido[3,2-e]benzimidazol-2-yl)amine, 2-Amino-3-methyl-3H-imidazo(4,5-f)quinoline, 2-Amino-3-methylimidazo(4,5-f)-quinoline;

Nadroparin, CY 216, CY-216, CY216;

naftifine, N-cinnamyl-N-methyl-1-naphthalenemethylamine hydrochloride, naftifin, naftifine;

nal-NH2, nal-NH2;

NALS, Akyposal SDS, Anticerumen, Aquarex ME;

nanchangmycin, nanchangmycin;

Naproxen, Aleve, Anaprox, Methoxypropiocin;

naratriptan, N-methyl-2-(3-(1-methylpiperiden-4-yl)indole-5-yl)ethanesulfonamide, N-methyl-2-[3-(1-methyl-4-piperidyl)-1H-indol-5-yl]-ethanesulfonamide, N-methyl-2-[3-(1-methylpiperidin-4-yl)-1H-indol-5-yl]ethanesulfonamide;

narbonolide, narbonolide;

NARIGENIN, ( )-Naringenin; 4?,5,7-Trihydroxyflavanone; H-1-benzopyran-4-one, ()-2,3-Dihydro-5,7-dihydroxy-2-(4-hydroxyphenyl)-4H-1-benzopyran-4-one, ()-Naringenin;

Narkotil, Aerothene MM, Dichlormethan, Dichlorocarbene;

Nasol, (+-)-Ephedrine, (-)-.alpha.-(1-Methylaminoethyl)benzyl alcohol, (-)-Ephedrine;

natalizumab, Antegren, natalizumab, Tysabri;

nateglinide, A 4166, A-4166, AY 4166;

Naxy, (3R,4S,5S,6R,7R,9R,11R,12R,13S,14R)-6-[(2S,3R,4S,6R)-4-dimethylamino-3-hydroxy-6-methyl-oxan-2-yl]oxy-14-ethyl-12,13-dihydroxy-4-[(2R,4R,5S,6S)-5-hydroxy-4-methoxy-4,6-dimethyl-oxan-2-yl]oxy-7-methoxy-3,5,7,9,11,13-hexamethyl-1-oxacyclotetradecane-2,10-di,
(3R,4S,5S,6R,7R,9R,11R,12R,13S,14R)-6-[(2S,3R,4S,6R)-4-dimethylamino-3-hydroxy-6-methyl-tetrahydropyran-2-yl]oxy-14-ethyl-12,13-dihydroxy-4-[(2R,4R,5S,6S)-5-hydroxy-4-methoxy-4,6-dimethyl-tetrahydropyran-2-yl]oxy-7-methoxy-3,5,7,9,11,13-hexamethyl-1-oxacy, (3R,4S,5S,6R,7R,9R,11R,12R,13S,14R)-6-[(2S,3R,4S,6R)-4-dimethylamino-3-hydroxy-6-methyl-tetrahydropyran-2-yl]oxy-14-ethyl-12,13-dihydroxy-4-[(2R,4R,5S,6S)-5-hydroxy-4-methoxy-4,6-dimethyl-tetrahydropyran-2-yl]oxy-7-methoxy-3,5,7,9,11,13-hexamethyl-1-oxacy;
nebivolol, alpha,alpha'-(iminobis(methylene))bis(6-fluoro-3,4-dihydro)-2H-1-benzopyran-2-methanol, Berlin-Chemie brand of nebivolol hydrochloride, Lobivon;
Nefazodone, 1-(3-(4-(m-Chlorophenyl)-1-piperazinyl)propyl)-3-ethyl-4-(2-phenoxyethyl)-delta2-1,2,4-triazolin-5-one, 2-[3-[4-(3-Chlorophenyl)-1-piperazinyl]propyl]-5-ethyl-2,4-dihydro-4-(2-phenoxyethyl)-3H-1,2,4-triazol-3-one, 2-[3-[4-(3-chlorophenyl)-1-piperazinyl]propyl]-5-ethyl-4-[2-(phenoxy)ethyl]-1,2,4-triazol-3-one;
nefiracetam, DM 9384, DM-9384, N-(2,6-dimethylphenyl)-2-(2-oxo-1-pyrrolidinyl)acetamide;
Nelfinavir, AG 1343, AG-1343, AG1343;
Neomycin, Fradiomycin Sulfate, Neomycin, Neomycin Palmitate;
Neopterin, 2-Amino-6-(1,2,3-trihydroxypropyl)-4(3H)-pteridinone, Monapterin, Neopterin;
Neostigmine, Neostigmine, Neostigmine Bromide, Neostigmine Methylsulfate;
Neut, Acidosan, Baking soda, Bisodium carbonate;
Nevirapine, BI RG 587, BI-RG-587, BIRG587;
NFBA, .alpha.,.alpha.,.alpha.-Trifluoro-2-methyl-4'-nitro-m-propionotoluidide, 2-Methyl-N-(4-nitro-3-[trifluoromethyl]phenyl)propanamide, 2-methyl-N-[4-nitro-3-(trifluoromethyl)phenyl]propanamide;
Nialk, 1,1,2-trichloroethene, 1,1,2-trichloroethylene, 1,1-dichloro-2-chloroethylene;
Nicardipine, Almirall Brand of Nicardipine Hydrochloride, Antagonil, Cardene;
Niflumic Acid, Donalgin, Flunir, Niflactol;
nimesulide, 4'-nitro-2'-phenoxymethanesulfonanilide, N-(4-nitro-2-phenoxyphenyl)methanesulfonamide, nimesulide;
niobium, 41Nb, columbio, columbium;
nitecapone, 3-(3,4-dihydroxy-5-nitrobenzylidine)-2,4-pentanedione, nitecapone, OR 462;
nitroanilide, nitroanilide;
nitroaspirin, 2-acetoxybenzoate-2-(1-nitroxymethyl)phenyl ester, NCX 4016, NCX-4016;
Nitrofurans, Nitrofurans;
NITROPYRENE, 1-Nitropyrene, 1-Nitropyrene [Nitroarenes], 1-Nitropyrene [Polycyclic aromatic compounds];
nitrosamines, N-Nitroso amines, nitrosamines;
Nitrosoanabasine, (+-)-1-Nitrosoanabasine, (+-)-3-(1-Nitroso-2-piperidinyl)pyridine, (+-)-N-Nitrosoanabasine;
Nitrosocysteine, 2-amino-3-(nitrosothio)propanoic acid, 2-amino-3-(nitrosothio)propionic acid, 2-amino-3-nitrososulfanyl-propanoic acid;
nitrosulindac, NCX 1102, NCX-1102, NCX1102;
Nizatidine, Axid, LY 139037, LY-139037;
NK 104, bis((3R,5S,6E)-7-(2-cyclopropyl-4-(4-fluorophenyl)-3-quinolyl)-3,5-dihydroxy-6-heptenoate), monocalcium salt, itavastatin, nisvastatin;
NK314, NK314;
NMDA, (NMDA), 2-methylaminobutanedioic acid, 2-methylaminosuccinic acid;
NN 703, NN 703, NN-703, NN703 cpd;
Noan, 1-Methyl-5-phenyl-7-chloro-1,3-dihydro-2H-1,4-benzodiazepin-2-one, 2H-1,4-Benzodiazepin-2-one, 7-chloro-1,3-dihydro-1-methyl-5-phenyl-, 7-Chloro-1,3-Dihydro-1-Methyl-5-Phenyl-2H-1,4-Benzodiazepin-2-One;
Nobiletin, 2-(3,4-dimethoxyphenyl)-5,6,7,8-tetramethoxy-4-chromenone, 2-(3,4-Dimethoxyphenyl)-5,6,7,8-tetramethoxy-4H-1-benzopyran-4-one, 2-(3,4-dimethoxyphenyl)-5,6,7,8-tetramethoxy-chromen-4-one;
NOC 18, NOC 18, NOC-18;
Nocodazole, Nocodazole, NSC 238159, NSC-238159;
nodularin, nodularin;
Nodularin v, Nodularin v;
nolatrexed, 3,4-dihydro-2-amino-6-methyl-4-oxo-5-(4-pyridylthio)quinazoline dihydrochloride, AG 337, AG-337;
Nonoxynol, Advantage S, Advantage-S, Delfen Cream;
noralfentanil, 4-MPPP, N-(4-(methoxymethyl)-4-piperidinyl)-N-phenylpropanamide, noralfentanil;
norbuprenorphine, norbuprenorphine;
Norclozapine, 3-chloro-6-(1-piperazinyl)-5H-benzo[c][1,5]benzodiazepine, 3-chloro-6-piperazin-1-yl-5H-benzo[c][1,5]benzodiazepine, 5H-Dibenzo(b,e)(1,4)diazepine, 8-chloro-11-(1-piperazinyl)-;
Nordihydroguaiaretic Acid, 4,4'-(2,3 Dimethyl-1,4-butanediyl)bis(1,2-benzenediol), Actinex, Dihydronorguaiaretic Acid;
Norethindrone, Conceplan, Ethinylnortestosterone, Micronor; noreximide, K 2154, K-2154, norborn-5-ene-2,3-cis-exo-dicarboximide;

norfluoxetine, norfluoxetine;
Norgestrel, DL Norgestrel, DL-Norgestrel, Neogest;
norharman, norharman;
norketobemidone, norketobemidone;
norlaudanosoline, (R,S)-Norlaudanosoline, 1-(3,4-dihydroxybenzyl)-1,2,3,4-tetrahydroisoquinoline-6,7-diol, norlaudanosoline;
normeperidine, normeperidine, normeperidine carbonate (2:1), normeperidine hydrochloride;
Nortilidine, (1R,2S)-2-methylamino-1-phenyl-1-cyclohex-3-enecarboxylic acid ethyl ester, (1R,2S)-2-methylamino-1-phenyl-cyclohex-3-ene-1-carboxylic acid ethyl ester, 3-Cyclohexene-1-carboxylic acid, 2-(methylamino)-1-phenyl-, ethyl ester, trans-(+-)-;
norverapamil, D 591, N-demethylverapamil, norverapamil; novobiocin, novobiocin;
NS-187, INNO-406, NS-187;
NSC 23766, NSC 23766, NSC-23766, NSC23766;
NSC 366140, 9-methoxy-N,N-dimethyl-5-nitropyrazolo(3,4,5-k)acridine-2(6H)-propanamine, NSC 366140, NSC 366140, me-methanesulfonate salt;
NSC 663284, NSC 663284, NSC-663284, NSC663284;
NSC-134754, 3-ethyl-9,10-dimethoxy-2-(1,2,3,4-tetrahydro-isoquinolin-1-ylmethyl)-1,6,7,11b-tetrahydro-4H-pyrido (2,1-a)isoquinoline, NSC-134754;
NU2058, NU2058, O(6)-cyclohexylmethylguanine;
number-one, number-one;
nutlin 3, nutlin 3, nutlin-3, nutlin-3A;
NVP-AEW541, NVP-AEW541;
Nylon, 6-Aminohexanoic acid homopolymer, A 1030N0, Akulon; O-(chloroacetylcarbamoyl)fumagillol, 5-methoxy-4-(2-methyl-3-(3-methyl-2-butenyl)oxiranyl)-1-oxaspiro(2,5)oct-6-yl(chloroacetyl) carbamate, AGM 1470, AGM-1470;
O-desethylreboxetine, O-desethylreboxetine;
O-Due, .beta.-Aminoethylsulfonic acid, 1-Aminoethane-2-sulfonic acid, 2-Aminoethanesulfonic acid;
o-quinone, 1,2-Benzoquinone, 2-benzoquinone, 3,5-cyclohexadiene-1,2-dione;
obovatol, 4',5-diallyl-2,3-dihydroxybiphenyl ether, obovatol; OCDD, 1,2,3,4,6,7,8,9-Octachlorodibenzo(1,4)dioxin, 1,2,3,4,6,7,8,9-Octachlorodibenzo(b,e)(1,4)dioxin, 1,2,3,4,6,7,8,9-Octachlorodibenzo-p-dioxin;
octanediol, octanediol;
Octoxynol, Octoxinol, Octoxinols, Octoxynol;
Octreotide, Compound 201 995, Compound 201-995, Compound 201995; Okadaic Acid, Ocadaic Acid, Okadaic Acid;
olanzapine, 2-methyl-4-(4-methyl-1-piperazinyl)-10H-thieno(2,3-b)(1,5)benzodiazepine, Eli Lilly brand of olanzapine, Lilly brand of olanzapine;
olefins, olefin, olefins;
oleoylethanolamide, oleoylethanolamide;
olmelin, 4'-Methoxy-5,7-dihydroxy isoflavone, 4'-Methylgenistein, 4H-1-Benzopyran-4-one, 5,7-dihydroxy-3-(4-methoxyphenyl)-;
olmesartan, 4-(hydroxy-1-methylethyl)-2-propyl-1-((2'-(1H-tetrazol-5-yl)-1,1'-biphenyl-4-yl)methyl)-1H-imidazole-5-carboxylic acid, CS-088, olmesartan;
olomoucine, olomoucine;
olomoucine II, olomoucine II;
Oltipraz, 3H-1,2-DITHIOLE-3-THIONE, 4-METHYL-5-PYRAZINYL-, 4-methyl-5-(2-pyrazinyl)-3-dithiolethione, 4-Methyl-5-(pyrazinyl)-3H-1,2-dithiole-3-thione;
omalizumab, Novartis Brand of Omalizumab, omalizumab, Xolair; omega-agatoxin, omega-agatoxin;
omega-Conotoxin GVIA, Conus geographus Toxin, Conus geographus Toxin GVIA, omega CgTX;
omega-N-Methylarginine, D-NMMA, L Monomethylarginine, L NG Monomethyl Arginine;
Omeprazole, Esomeprazole, H 168 68, H 168-68;
omeprazole sulfone, omeprazole sulfone;
onapristone, onapristone;
ONCB, 1-chloro-2-nitro-benzene, 1-Chloro-2-nitrobenzene, 1-Nitro-2-chlorobenzene;
Ondansetron, GR 38032F, GR-38032F, GR38032F;
ONO4819, AE1 734, AE1-734, methyl 7-((1R,2R,3R)-3-hydroxy-2-((E)-(3S)-3-hydroxy-4-(m-methoxymethylphenyl)-1-butenyl)-5-oxocyclopentyl)-5-thiaheptanoate;
Optef, (11beta)-11,17,21-Trihydroxy-pregn-4-ene-3,20-dione, (11beta)-11,17,21-trihydroxypregn-4-ene-3,20-dione, (8S,9S,10R,11S,13S,14S,17R)-11,17-dihydroxy-17-(2-hydroxy-1-oxoethyl)-10,13-dimethyl-2,6,7,8,9,11,12,14,15,16-decahydro-1H-cyclopenta[a]phenanthren-3-one;
OR 1246, OR 1246;
oroxylin A, oroxylin A;
Orphenadrine, Disipal, Lysantin, Mefenamine;
Osten, (Ipriflavone), 3-phenyl-7-propan-2-yloxy-chromen-4-one, 3-phenyl-7-propan-2-yloxychromen-4-one;
osteum, osteum;
OSU 03012, 2-amino-N-(4-(5-(2-phenanthrenyl)-3-(trifluoromethyl)-1H-pyrazol-1-yl)phenyl)acetamide, OSU 03012, OSU-03012;
Ouabain, Acocantherin, Acolongifloroside K, G Strophanthin;
OVEX, (17beta)-17-hydroxyestra-1(10),2,4-trien-3-yl benzoate, .beta.-Estradiol 3-benzoate, .beta.-Estradiol benzoate;
Ovex, (17-alpha)-19-Norpregna-1,3,5(10)-trien-20-yne-3,17,diol, (17beta)-17-ethynylestra-1(10),2,4-triene-3,17-diol, (8R,9S,13S,14S,17R)-17-ethynyl-13-methyl-7,8,9,11,12,14,15,16-octahydro-6H-cyclopenta[a]phenanthrene-3,17-diol;
oxaliplatin, 1,2-diaminocyclohexane platinum oxalate, 1,2-diamminocyclohexane(trans-1)oxolatoplatinum(II), 1-OHP;
Oxarol, (1R,35,52)-5-[(2E)-2-[(1S,3aS,7aS)-1-[(1S)-1-(3-hydroxy-3-methyl-butoxy)ethyl]-7a-methyl-2,3,3a,5,6,7-hexahydro-1H-inden-4-ylidene]ethylidene]-4-methylene-cyclohexane-1,3-diol, (1R,3S,5Z)-5-[(2E)-2-[(1S,3aS,7aS)-1-[(1S)-1-(3-hydroxy-3-methyl-butoxy)ethyl]-7a-methyl-2,3,3a,5,6,7-hexahydro-1H-inden-4-ylidene]ethylidene]-4-methylidene-CyClohexane-1,3-diol, (1R,3S,5Z)-5-[(2E)-2-[(1S,3aS,7aS)-1-[(1S)-1-(3-hydroxy-3-methylbutoxy)ethyl]-7a-methyl-2,3,3a,5,6,7-hexahydro-1H-inden-4-ylidene]ethylidene]-4-methylenecyclohexane-1,3-diol;
oxaspirodion, oxaspirodion;
oxatomide, oxatomide;
Oxazepam, Adumbran, Oxazepam, Serax;
oxcarbazepine, Desitin brand of oxcarbazepine, GP 47680, Novartis brand of oxcarbazepine;
Oxotremorine, Oxotremorine, Oxytremorine;
oxotremorine M, N,N,N-trimethyl-4-(2-oxopyrrolidin-1-yl)but-2-yn-1-aminium, oxotremorine M;
Oxymorphone, Bristol-Myers Squibb Brand of Oxymorphone Hydrochloride, Endo Brand of Oxymorphone Hydrochloride, Methylnaloxone;
Oxyntomodulin, Glicentin (33-69), Oxyntomodulin, Proglucagon (33-69);
Oxytrol, 2-cyclohexyl-2-hydroxy-2-phenyl-acetic acid 4-diethylaminobut-2-ynyl ester, 2-cyclohexyl-2-hydroxy-2-phenylacetic acid 4-diethylaminobut-2-ynyl ester, 4-(diethylamino)but-2-yn-1-yl cyclohexyl(hydroxy)phenylacetate;
p-ABA, 4-Aminobenzamidine, 4-Aminobenzenecarboximidamide, Benzamidine, p-amino-;
p-XSC, 1,4-bis(selenocyanatomethyl)benzene, 1,4-Phenylenebis(methylene)selenocyanate, p-XSC;
p-Xylol, 1,4-dimethylbenzene, 1,4-Dimethylbenzol, 1,4-xylene; Paclitaxel, 7 epi Taxol, 7-epi-Taxol, Anzatax;
paeonol, 2-hydroxy-4-methoxyacetophenone, paeonol;
palladium, 46Pd, paladio, palladium;
palmitoleate, (9Z)-hexadec-9-enoate, palmitoleate;
PALMITOYL, 16-Hexadecanal, hexadecanal, N-hexadecanal;
Palmitoylcarnitine, Hexadecanoylcarnitine, Palmitoylcarnitine, Palmitylcarnitine;
pamidronate, (3-amino-1-hydroxypropylidene)-1,1-biphosphonate, (3-amino-1-hydroxypropylidene)-1,1-bisphosphonate, 1-hydroxy-3-aminopropane-1,1-diphosphonic acid;
panaxadiol, panaxadiol, panaxadiol, (3beta,12beta)-isomer; panepoxydone, panepoxydone;
pantoprazole, BY 1023, BY-1023, pantoprazole;
Papaverine, Cerespan, Papaverine, Papaverine Hydrochloride;
Papite, 2-Propen-1-one, 2-propenal, 2-Propenal, homopolymer;
PAPP, 1-(2-(4-Aminophenyl)ethyl)-4-(3-trifluoromethylphenyl)piperazine, 4-(2-(4-(3-(Trifluoromethyl)phenyl)-1-piperazinyl)ethyl)aniline hydrochloride, 4-[2-[4-[3-(trifluoromethyl)phenyl]-1-piperazinyl]ethyl]aniline;
parecoxib, Dynastat, N-(((5-methyl-3-phenylisoxazol-4-yl)-phenyl)sulfonyl)propanamide, N-(((5-methyl-3-phenylisoxazol-4-yl)-phenyl)sulfonyl)propanamine, sodium salt;
Paroxetine, Aropax, BRL 29060, BRL-29060;
Parsal, 5-Amino-N-butyl-2-(2-propynyloxy)benzamide, 5-amino-N-butyl-2-prop-2-ynoxy-benzamide, 5-amino-N-butyl-2-prop-2-ynoxybenzamide;
Parthenolide, Parthenolide;
PC 314, PC 314, PC-314, PC314 cpd;
PCA 4230, PCA 4230;
PCSO, 2-amino-3-prop-2-enylsulfinyl-propanoic acid, 2-amino-3-prop-2-enylsulfinylpropanoic acid, 3-allylsulfinyl-2-amino-propanoic acid;
PD 134308, PD 134308;
PD 144795, PD 144795;
PD 180988, CI-1034, CI1034, PD 180988;
PD 98059, 2-(2'-amino-3'-methoxyphenyl)oxanaphthalen-4-one, 2-(2-amino-3-methoxyphenyl)-4H-1-benzopyran-4-one, PD 098059; pectin, pectin;
Pemetrexed, (2R)-2-[[4-[2-(2-amino-4-keto-1,7-dihydropyrrolo[4,5-e]pyrimidin-5-yl)ethyl]benzoyl]amino]glutaric acid, (2R)-2-[[4-[2-(2-amino-4-oxo-1,7-dihydropyrrolo[4,5-e]pyrimidin-5-yl)ethyl]benzoyl]amino]pentanedioic acid, (2R)-2-[[4-[2-(2-amino-4-oxo-1,7-dihydropyrrolo[4,5-e]pyrimidin-5-yl)ethyl]phenyl]carbonylamino]pentanedioic acid;
Penicillins, Antibiotics, Penicillin, Penicillin, Penicillin Antibiotics;
Penite, Arsenite, sodium, Atlas A, Chem Pels C;
Pentagastrin, Acignost, Gastrin Pentapeptide, Pentagastrin; Pentoxifylline, Agapurin, BL 191, BL-191;
Peplomycin, NK 631, NK-631, NK631;
peppermint oil, peppermint oil, WS 1340, WS-1340;
Pepstatin A, Pepstatin A;
Perazine, Perazine, Perazine Dihydrochloride, Perazine Maleate; Pergolide, Athena Brand of Pergolide Mesylate, Celance, Draxis Brand of Pergolide Mesylate;
Perillol, (−)-Perillyl alcohol, (-)-Perillyl alcohol, (-)-Perillylalcohol;
Perilymph, Perilymph, Perilymphs;
periodate, IO4(-), periodate, tetraoxidoiodate(1-);
perospirone, 2-(4-(4-(1,2-benzisothiazol-3-yl)-1-piperazinyl)butyl)hexahydro-1H-isoindole-1,3-(2H)-dione, perospirone, SM 9018;
perovskite, calcium titanium oxide, CaTiO3, perovskite;
PFPA, 2,2,3,3,3-pentafluoropropanoic acid (2,2,3,3,3-pentafluoro-1-oxopropyl) ester, 2,2,3,3,3-pentafluoropropanoyl 2,2,3,3,3-pentafluoropropanoate, 2,2,3,3,3-pentafluoropropionic acid 2,2,3,3,3-pentafluoropropanoyl ester;
Phebestin, Phebestin;
phen, .beta.-Phenanthroline, 1,10-Fenanthroline, 1,10-o-Phenanthroline;
phenolate, Phenol ion, Phenol, ion(1-) (VAN), phenolate; Phenols, Carbol, Phenols;
phenoxodiol, 7-hydroxy-3-hydroxyphenyl-1H-benzopyran, phenoxodiol;
Phenprocoumon, Falithrom, Hexal Brand of Phenprocoumon, Liquamar;
Phentermine, Adipex P, Adipex-P, AdipexP;
phenyl-propionamide, phenyl-propionamide;
phenyl-Pyridinium, phenyl-Pyridinium;
Phenytoin, 5,5-Diphenylhydantoin, Antisacer, Difenin;
pheophorbide a, (2(2)R,17S,18S)-7-ethyl-2(1),2(2),17,18-tetrahydro-2(2)-(methoxycarbonyl)-3,8,13,17-tetramethyl-2(1)-oxo-12-ethenylcyclopenta[at]porphyrin-18-propanoic acid, (3S,4S,21R)-9-ethenyl-14-ethyl-21-(methoxycarbonyl)-4,8,13,18-tetramethyl-20-oxo-3-phorbinepropanoic acid, 3-[(3S,4S,21R)-14-ethyl-21-(methoxycarbonyl)-4,8,13,18-tetramethyl-20-oxo-9-vinylphorbin-3-yl]propanoic acid;
phloretin, 3-(4-hydroxyphenyl)-1-(2,4,6-trihydroxyphenyl)-1-propanone, 3-(4-hydroxyphenyl)-1-(2,4,6-trihydroxyphenyl)propan-1-one, phloretin;
PHOB, 2,4,6(1H,3H,5H)-Pyrimidinetrione, 5-ethyl-5-phenyl-, 5-ethyl-5-phenyl-1,3-diazinane-2,4,6-trione, 5-Ethyl-5-phenyl-2,4,6(1H,3H,5H)-pyrimidinetrione;
phorate, O,O-Diethyl S-(ethylthio)methyl phosphorodithioate, O,O-Diethyl S-ethylmercaptomethyl dithiophosphate, O,O-diethyl S-[(ethylsulfanyl)methyl] dithiophosphate;
phorbol, phorbol;
phorbol 12-phenylacetate 13-acetate 20-homovanillate, phorbol 12-phenylacetate 13-acetate 20-homovanillate;
phosphatidylethanolamines, (3-Phosphatidyl)-ethanolamine, 1,2-diacyl-sn-glycero-3-phosphoethanolamine, 1-Acyl-2-acyl-sn-glycero-3-phosphoethanolamine;
Phosphatidylinositol 4,5-Diphosphate, Phosphatidylinositol 4,5-Biphosphate, Phosphatidylinositol 4,5-Diphosphate, Phosphatidylinositol-4,5-Bisphosphate;
phosphatidylinositol phosphate, PtdIns(4,5)P2, phosphatidylinositol phosphate, PtdIns(4,5)P2, PtdIns(4,5)P2; phytanic acid, 3,7,11,15-tetramethyl-hexadecanoic acid, Phytanate, phytanic acid;
Picibanil, NSC B116209, NSC-B116209, NSCB116209;
picric acid, 2,4,6-trinitrophenol, picrate, picric acid; pifithrin, 1-(4-methylphenyl)-2-(4,5,6,7-tetrahydro-2-imino-3(2H)-benzothiazolyl)ethanone hydrobromide, 2-(2-imino-4,5,6,7-tetrahydrobenzothiazol-3-yl)-1-p-tolylethanone, PFT alpha;
Pilot, 2-(4-((6-Chloro-2-quinoxalinyl)oxy)phenoxy)propanoic acid ethyl ester, 2-[4-(6-chloroquinoxalin-2-yl)oxyphenoxy]propionic acid ethyl ester, 2-[4-[(6-chloro-2-quinoxalinyl)oxy]phenoxy]propanoic acid ethyl ester;
pimecrolimus, 33-epi-chloro-33-desoxyascomycin, ASM 981, Elidel;
pioglitazone, 5-(4-(2-(5-ethyl-2-pyridyl)ethoxy)benzyl)-2,4-thiazolidinedione, Actos, AD 4833;
pipecoloxylidide, pipecoloxylidide;
piperidine, Azacyclohexane, C1CCNCC1, Hexahydropyridine;
piperine, ((1-5-(1,3)-benzodioxol-5-yl)-1-oxo-2,4-pentadienyl)piperidine, 1-piperoylpiperidine, piperine;
Pira, 1-((p-(2-(5-Chloro-o-anisamido)ethyl)phenyl)sulfonyl)-3-cyclohexylurea, 1-(p-(2-(5-Chloro-2-methoxybenzamido)ethyl)benzenesulfonyl)-3-cyclohexylurea, 5-Chloro-N-(2-(4-((((cyclohexylamino)carbonyl)amino)sulfonyl) phenyl)ethyl)-2-methoxybenzamide;
pirinixic acid, (4-chloro-6-(2,3-xylidino)-2-pyrimidinylthio)acetic acid, 4-chloro-6-(2,3-dimethylphenyl)amino-2-pyrimidinylthioacetic acid, 4-chloro-6-(2,3-xylidinyl)-2-pyrimidinylthioacetic acid;
Piroxicam, CP 16171, CP-16171, CP16171;
PKC412, PKC 412, PKC-412, PKC412;
plumbagin, 2-methyl-5-hydroxy-1,4-naphthoquinone, 5-hydroxy-2-methyl-1,4-naphthalenedione, 5-hydroxy-2-methyl-1,4-naphthoquinone;
Pluronic p 85, Pluronic p 85;
PMDT, 1,1,4,7,7-pentamethyldiethylenetriamine, 1,2-Ethanediamine, N-(2-(dimethylamino)ethyl)-N,N',N'-trimethyl-, 1,2-Ethanediamine, N-[2-(dimethylamino)ethyl]-N,N',N'-trimethyl- ;
PMPA, (((1R)-2-(6-Amino-9H-purin-9-yl)-1-methylethoxy)methyl)phosphonic acid, (R)-9-(2-Phosphonomethoxypropyl)adenine, (R)-9-(2-Phosphonylmethoxypropyl)adenine;
PMSF, .alpha.-Toluenesulfonyl fluoride, alpha-TOLUENESULFONYL FLUORIDE, alpha-Toluenesulphonyl fluoride;
PNPP, (4-nitrophenoxy)phosphonic acid, (4-nitrophenyl) dihydrogen phosphate, 4-Nitrophenyl dihydrogen phosphate;
Podophyllotoxin, Ardern Brand of Podophyllotoxin, Canderm Brand of Podophyllotoxin, Condyline;
polidocanol, aethoxysclerol, aethoxysklerol, aetoxisclerol; poly-gamma-glutamate, poly-gamma-glutamate;
ponicidin, ponicidin;
poractant alfa, Allphar brand of poractant alfa, Chiesi brand of poractant alfa, Curosurf;
posaconazole, Noxafil, posaconazole, SCH 56592;
potassium tellurate(IV), potassium tellurate(IV);
PQQ Cofactor, 2,7,9-Tricarboxy-1H-Pyrrolo-(2,3-f)Quinoline-4,5-Dione, 2,7,9-Tricarboxypyrroloquinoline Quinone, 4,5-Dihydro-4,5-Dioxo-1-H-Pyrrolo(2,3-f)Quinoline-2,7,9-Tricarboxylic Acid;
pranlukast, 8-(4 (4-phenylbutoxy)benzoyl)amino-2-(tetrazol-5'-yl)-4-oxo-4H-1-benzopyran, ONO 1078, ONO-1078;
Pravastatin, Apo Pravastatin, Apo-Pravastatin, Apotex Brand of Pravastatin Sodium;
Prazosin, Furazosin, Justac, Minipress;
PRDL, (11beta)-11,17,21-Trihydroxypregna-1,4-diene-3,20-dione, (8S,9S,10R,11S,13S,14S,17R)-11,17-dihydroxy-17-(2-hydroxy-1-oxoethyl)-10,13-dimethyl-7,8,9,11,12,14,15,16-octahydro-6H-cyclopenta[alphenanthren-3-one, (8S,9S,10R,11S,13S,14S,17R)-11,17-dihydroxy-17-(2-hydroxyacetyl)-10,13-dimethyl-7,8,9,11,12,14,15,16-octahydro-6H-cyclopenta[a]phenanthren-3-one;
Precursor mrna, Precursor mrna;
Prednisone, acis Brand of Prednisone, Apo-Prednisone, Apotex Brand of Prednisone;
pregnane, pregnane;
Pregnanes, Pregnanes;
Pregnanolone, 3 alpha Hydroxy 5 alpha pregnan 20 one, 3 alpha Hydroxy 5 beta pregnan 20 one, 3 alpha, 5 beta-Tetrahydroprogesterone;
pregnenolone 16alpha-carbonitrile, 3beta-hydroxy-20-oxo-5-pregnene-16alpha-carbonitrile, 3beta-hydroxy-20-oxopregn-5-ene-16alpha-carbonitrile, PCN;
Pregnyl, biogonadil, choriogonadotropin, choriogonin;
preussin, (+)-preussin, L 657398, L-657,398;
Primidone, Apo-Primidone, Apotex Brand of Primidone, Astra Brand of Primidone;
Proadifen, Diethylaminoethyldiphenylpropyl Acetate, Proadifen, Proadifen Hydrochloride;
Proanthocyanidins, Anthocyanidin Polymers, Condensed Tannin, Condensed Tannins;
Probenecid, Benecid, Benemid, Benuryl;
Probucol, Almirall Brand of Probucol, Aventis Brand of Probucol, Biphenabid;
Procasil, 2,3-Dihydro-6-propyl-2-thioxo-4(1H)-pyrimidinone, 2-Mercapto-4-hydroxy-6-n-propylpyrimidine, 2-Mercapto-6-propyl-4-pyrimidone;
Procetofen, Abbott Brand of Procetofen, AbZ Brand of Procetofen, Aliud Brand of Procetofen;
procyanidin B2. procyanidin B2;

Prodix, (8R,9S,10R,13S,14S,17R)-17-acetyl-17-hydroxy-10,13-dimethyl-2,6,7,8,9,11,12,14,15,16-decahydro-1H-cyclopenta[a]phenanthren-3-one, (8R,9S,10R,13S,14S,17R)-17-ethanoyl-17-hydroxy-10,13-dimethyl-2,6,7,8,9,11,12,14,15,16-decahydro-1H-cyclopenta[a]phenanthren-3-one, 17-alpha-Hydroxyprogesterone;

prolactin, polymeric, BBPRL, big big prolactin, big prolactin; Propafenone, Abbott Brand of Propafenone Hydrochloride, Aliud Brand of Propafenone Hydrochloride, Alpharma Brand of Propafenone Hydrochloride;

Propanesulfonate, 1-Propanesulfonic acid, Ammonium propanesulfonate, propane-1-sulfonic acid;

Propofol, 2,6-Bis(1-methylethyl)phenol, 2,6-Diisopropylphenol, Abbott Brand of Propofol;

propyl pyrazole triol, PPT cpd, propyl pyrazole triol, propylpyrazole triol;

propyne, 1-propyne, allylene, CC#C;

prostratin, prostratin;

protopanaxadiol, 20(S)-protopanaxadiol, protopanaxadiol, protopanaxadiol, (3beta,12beta)-isomer;

protopanaxatriol, protopanaxatriol, protopanaxatriol, (3beta,6alpha,12beta,20R)-isomer;

PS 15, N-(3(2,4,5-trichlorophenoxy)propyloxy)-N'-(1-methylethyl)imidocarbonimidicdiamide hydrochloride, PS 15, PS-15;

Pseudohypericin, Hypericum Extract, Phenanthro(1,10,9,8-opgra)perylene-7,14-dione, 1,3,4,6,8,13-hexahydroxy-10-(hydroxymethyl)-11-methyl-, stereoisomer, Pseudohypericin; Pseudomonas-exotoxin, Pseudomonas-exotoxin;

Psoralens, Furanocoumarins, Furocoumarins, Psoralens;

psychosine-3'-sulfate ester, psychosine-3'-sulfate ester;

PTBP, 1-Hydroxy-4-tert-butylbenzene, 2-tert-Butylphenol, 4-(1,1-Dimethylethyl)phenol;

pteridine, 1,3,5,8-tetraazanaphthalene, azinepurine, c1cnc2ncncc2n1;

Pterostilbene, 3',5'-Dimethoxy-4-stilbenol, 3,5-Dimethoxy-4′-hydroxystilbene, 3,5-Dimethoxy-4'-hydroxy-trans-stilbene;

PURAC, (+)-Lactic acid, (+-)-2-Hydroxypropanoic acid, (2R)-2-hydroxypropanoic acid;

Puromycin, CL 13900, CL-13900, CL13900;

putrescine, 1,4-Butanediamine, 1,4-butylenediamine, 1,4-DIAMINOBUTANE;

Pyocyanine, Pyocyanin, Pyocyanine;

Pyra, 1,2-Ethanediamine, N-((4-methoxyphenyl)methyl)-N',N'-dimethyl-N-2-pyridinyl-, 1,2-Ethanediamine, N-((4-methoxyphenyl)methyl)-N',N'-dimethyl-N-2-pyridinyl- (9CI), 1,2-Ethanediamine, N-[(4-methoxyphenyl)methyl]-N',N'-dimethyl-N-2-pyridinyl-;

pyranones, oxopyrans, pyranone, pyranones;

pyrazole, Pyrazol, pyrazole;

Pyrethrins, Pyrethrins, Pyrethroids;

pyridazine, 1,2-diazine, c1ccnnc1, o-diazine;

Pyrimethamine, Aventis Brand of Pyrimethamine, Chloridin, Daraprim;

pyrimidin-2-one beta-ribofuranoside, NSC 309132, pyrimidin-2-one beta-D-ribofuranoside, pyrimidin-2-one beta-ribofuranoside;

Pyro, 1,2,3-Benzenetriol, 1,2,3-TRIHYDROXY-BENZENE, 1,2,3-Trihydroxybenzen;

pyrogallol sulfonphthalein, pyrogallol sulfonphthalein;

pyrrole-2-carboxylic acid, 1H-pyrrole-2-carboxylic acid, 2-pyrrolecarboxylic acid, PCA;

pyrrolidine dithiocarbamic acid, pyrrolidine dithiocarbamic acid, Pyrrolidinedithiocarbamate;

pyrroloazepinone, pyrroloazepinone;

Qingkailing, Qingkailing;

quercitrin, quercetin 3-O-rhamnoside, quercetin-3-L-rhamnoside, quercetrin;

quetiapine, 2-(2-(4-dibenzo(b,f)(1,4)thiazepine-11-yl-1-piperazinyl)ethoxy)ethanol, AstraZeneca Brand of Quetiapine Fumarate, Ethanol, 2-(2-(4-dibenzo(b,f)(1,4)thiazepin-11-yl-1-piperazinyl) ethoxy) -, (E) -2-butenedioate (2:1) (salt);

Quicifal, 1-Ascorbyl palmitate, 6-Hexadecanoyl-L-ascorbic acid, 6-Monopalmitoyl-L-ascorbate;

quinazoline, 1,3-benzodiazine, 1,3-diazanaphthalene, 5,6-benzopyrimidine;

Quinolinium, Quinolinium;

Quinpirole, Quinpirole, Quinpirole Hydrochloride, Quinpirole Monohydrochloride;

quinuclidin-3'-yl-1-phenyl-1,2,3,4-tetrahydroisoquinoline-2-carboxylate monosuccinate, quinuclidin-3'-yl 1-phenyl-1,2,3,4-tetrahydroisoquinoline-2-carboxylate monohydrochloride, quinuclidin-3'-yl-1-phenyl-1,2,3,4-tetrahydroisoquinoline-2-carboxylate monosuccinate, solifenacin;

quinupristin-dalfopristin, quinupristin-dalfopristin, RP 59500, RP-59500;

R-138727, R-138727;

R-99224, (2Z)-(1-(2-cyclopropyl-1-(2-fluorophenyl)-2-oxoethyl)-4-mercapto-3-piperidinylidene), R-99224;

Raloxifene, ELi Lilly Brand of Raloxifene, Evista, Keoxifene;

raltitrexed, Arkomédika brand of raltitrexed, AstraZeneca brand of raltitrexed, D 1694;
Ramipril, Acovil, Almirall Brand of Ramipril, Altace;
ramiprilat, ramiprilat;
RAMP, RAMP;
Ranitidine, AH 19065, AH-19065, AH19065;
RAPA, (-)-Rapamycin, (3-(4-hydroxy-3-methoxycyclohexyl)-1-methylethyl)-10,21-dimethoxy-, (3S,6R,7E,9R,10R,12R,14S,15E,17E,19E,21S,23S,26R,27R,34aS)-;
rasagiline, 2,3-dihydro-N-2-propynyl-1H-inden-1-amine-(1R)-hydrochloride, AGN 1135, AGN-1135;
rebamipide, 2-(4-chlorobenzoylamino)-3-(2(1H)-quinolinon-4-yl)propionic acid, OPC 12759, OPC-12759;
reboxetine, 2-((2-ethoxyphenoxy)benzyl)morpholine methanesulfonate, reboxetine, reboxetine mesylate;
remifentanil, 3-(4-methoxycarbonyl-4-((1-oxopropyl)phenylamino)-1-piperidine)propanoic acid methyl ester, Abbott brand of remifentanil, GI 87084B;
renzapride, 4-amino-5-chloro-2-methoxy-N-(1-azabicyclo-(3.3.1)-non-4-yl)benzamide, BRL 24924, BRL-24924;
repaglinide, 2-ethoxy-4-(2-((3-methyl-1-(2-(1-piperidinyl)phenyl)butyl)amino)-2-oxoethyl)benzoic acid, 2-ethoxy-N-(alpha-(2-methyl-1-propyl)-2-piperidinobenzyl)-4-carbamoylmethylbenzoic acid, AG-EE 388;
Resiniferotoxin, Resiniferotoxin;
resiquimod, 1-[4-amino-2-(ethoxymethyl)-1H-imidazo[4,5-c]quinolin-1-yl]-2-methylpropan-2-ol, CCOCc1nc2c(N)nc3ccccc3c2n1CC(C)(C)O, R 848;
Retardex, acide benzoique, Ammonium benzoate, Aromatic hydroxy acid;
Riacon, (2-Mercaptoethyl)amine, .beta.-Mercaptoethylamine, 1-Amino-2-mercaptoethylamine;
Ribavirin, Dermatech Brand of Ribavirin, Essex Brand of Ribavirin, Grossman Brand of Ribavirin;
Riboflavin, Flavin mononucleotide, Pentitol, 1-deoxy-1-(3,4-dihydro-7,8-dimethyl-2,4-dioxobenzo[g]pteridin-10 (2H)-yl)-, 5-(dihydrogen phosphate), Riboflavin;
Rifabutin, Alfacid, Ansamycin, Ansatipin;
rifamycins, rifamycins;
Rifocin, Rifamycin, Rifamycin SV, Rifocin;
rimonabant, Acomplia, N-(piperidin-1-yl)-5-(4-chlorophenyl)-1-(2,4-dichlorophenyl)-4-methyl-1H-pyrazole-3-carboxamide hydrochloride, rimonabant;
risedronic acid, 2-(3-pyridinyl)-1-hydroxyethylidene-bisphosphonate, 2-(3-pyridinyl)-1-hydroxyethylidenebisphosphonate, Actonel;
risperidone, 3-{2-[4-(6-fluoro-1,2-benzisoxazol-3-yl)piperidin-1-yl]ethyl}-2-methyl-6,7,8,9-tetrahydro-4H-pyrido[1,2-a]pyrimidin-4-one, Risperdal, risperidona;
Ristocetin, Ristocetin, Ristomycin;
Ritonavir, ABT 538, ABT-538, ABT538;
rituximab, CD20 antibody, rituximab, Genentech brand of rituximab, Hoffmann-La Roche brand of rituximab;
Ro 13-8996, (2)-(2,4-dichlorobenzyl)oxy-[1-(2,4-dichlorophenyl)-2-imidazol-1-yl-ethylidene]amine, 1-(2,4-dichlorophenyl)-N-[(2,4-dichlorophenyl)methoxy]-2-(1-imidazolyl)ethanimine, 1-(2,4-dichlorophenyl)-N-[(2,4-dichlorophenyl)methoxy]-2-imidazol-1-yl-ethanimine;
Ro 23-7553, (1R,3S,5Z)-5-[(2E)-2-[(3aS,7aS)-1-[(1R)-5-hydroxy-1,5-dimethyl-hex-3-ynyl]-7a-methyl-3a,5,6,7-tetrahydro-3H-inden-4-ylidene]ethylidene]-4-methylene-cyclohexane-1,3-diol, (1R,3S,5Z)-5-[(2E)-2-[(3aS,7aS)-1-[(1R)-5-hydroxy-1,5-dimethylhex-3-ynyl]-7a-methyl-3a,5,6,7-tetrahydro-3H-inden-4-ylidene]ethylidene]-4-methylenecyclohexane-1,3-diol, (1R,3S,5Z)-5-[(2E)-2-[(3aS,7aS)-1-[(2R)-6-hydroxy-6-methyl-hept-4-yn-2-yl]-7a-methyl-3a,5,6,7-tetrahydro-3H-inden-4-ylidene]ethylidene]-4-methylidene-cyclohexane-1,3-diol;
Ro 23-7637, Ro 23-7637;
Ro 24-7429, 7-chloro-N-methyl-5-(1H-pyrrol-2-yl)-3H-1,4-benzodiazepin-2-amine, Ro 24-7429, Ro-24-7429;
Ro 31-6233, 1H-Pyrrole-2,5-dione, 3,4-di-1H-indol-3-yl-, 2,3-bis(1H-Indol-3-yl)maleimide, 3,4-bis(1H-indol-3-yl)-3-pyrroline-2,5-quinone;
Ro 31-7549, Ro 31-7549;
Ro 31-8220, Ro 31-8220;
RO4383596, (+/-)-1-(anti-3-hydroxy-cyclopentyl)-3-(4-methoxy-phenyl)-7-phenylamino-3,4-dihydro-1H-pyrimido (4,5-d)pyrimidin-2-one, RO4383596;
Robitet, (22)-2-(amino-hydroxy-methylene)-4-dimethylamino-6,10,11,12a-tetrahydroxy-6-methyl-4,4a,5,5a-tetrahydrotetracene-1,3,12-trione, (22)-2-(amino-hydroxy-methylidene)-4-dimethylamino-6,10,11,12a-tetrahydroxy-6-methyl-4,4a,5,5a-tetrahydrotetracene-1,3,12-trione, (2Z)-2-(amino-hydroxymethylene)-4-dimethylamino-6,10,11,12a-tetrahydroxy-6-methyl-4,4a,5,5a-tetrahydrotetracene-1,3,12-trione;
rofecoxib, Cahill May Roberts brand of rofecoxib, Merck brand of rofecoxib, Merck Frosst brand of rofecoxib;
roflumilast, 3-cyclopropylmethoxy-4-difluoromethoxy-N-(3,5-di-chloropyrid-4-yl)benzamide, roflumilast;
rokitamycin, 3''-O-propionylleucomycin A5, pro-leucomycin A5, rokitamycin;

Rolipram, Rolipram, ZK 62711, ZK-62711;
romidepsin, FK228, FR 901228, FR-901228;
rooperol, rooperol;
ropivacaine, 1-propyl-2',6'-pipecoloxylidide, AL 381, AL-381; roscovitine, 2-(1-ethyl-2-hydroxyethylamino)-6-benzylamino-9-isopropylpurine, CYC 202, CYC-202;
rosiglitazone, 5-((4-(2-methyl-2-(pyridinylamino)ethoxy)phenyl)methyl)-2,4-thiazolidinedione-2-butenedioate, Avandia, BRL 49653;
rosmarinic acid, (2S)-3-(3,4-dihydroxyphenyl)-2-[(2E)-3-(3,4-dihydroxyphenyl)prop-2-enoyloxy]propanoic acid, Rosmarinate, rosmarinic acid;
rosuvastatin, (3R,5S,6E)-7-(4-(4-fluorophenyl)-6-(1-methylethyl)-2-(ethyl(methylsulfonyl)amino)-5-pyrimidinyl)-3,5-dihydroxy-6-heptenoic acid, (3R,5S,6E)-7-{4-(4-fluorophenyl)-2-[methyl(methylsulfonyl)amino]-6-(propan-2-yl)pyrimidin-5-yl}-3,5-dihydroxyhept-6-enoic acid, (3R,5S,6E)-7-{4-(4-fluorophenyl)-6-isopropyl-2-[methyl(methylsulfonyl) amino]pyrimidin-5-yl}-3,5-dihydroxyhept-6-enoic acid;
Roxithromycin, 1A Brand of Roxithromycin, Alpharma Brand of Roxithromycin, Aventis Brand of Roxithromycin;
Rozevin, (2ALPHA,2'BETA,3BETA,4ALPHA,5BETA)-VINCALEUKOBLASTINE, (3aR-(3aalpha,4beta,5beta, 5abeta,9(3R*,5S*,7R*,9S*),10bR*,13aalpha))-methyl 4-(acetyloxy)-3a-ethyl-9-(5-ethyl-1,4,5,6,7,8,9,10-octahydro-5-hydroxy-9-(methoxycarbonyl)-2H-3,7-methanoazacycloundecino(5,4-b)indol-9-yl)-3a,4,5,5a,6,11,12,13a-octahydro-5-hydro, 1H-Indolizino(8,1-cd)carbazole-5-carboxylic acid, 4-(acetyloxy)-3a-ethyl-9-(5-ethyl-1,4,5,6,7,8,9,10-octahydro-5-hydroxy-9-(methoxycarbonyl)-2H-3,7-methanoazacycloundecino(5,4-b)indol-9-yl)-3a,4,5,5a,6,11,12,13a-octahydro-5-hydroxy-8-methoxy-6-methyl-, me;
RPR 121056, 7-ethyl-10-(4-N-(5-aminopentanoic acid)-1-piperidino)carbonyloxycamptothecin, APC cpd, RPR 121056;
RU 58668, 11beta-(4-(5-((4,4,5,5,5-pentafluoropentyl)sulfonyl)pentyloxy)phenyl)-estra-1,3,5(10)-triene,3,17beta-diol, Estra-1,3,5(10)-triene-3,17-diol, 11-(4-((5-((4,4,5,5,5-pentafluoropentyl)sulfonyl)pentyl)oxy)phenyl)-, (11beta, 17beta)-, RU 58 668;
ruboxistaurin, 13-((dimethylamino)methyl)-10,11,14,15-tetrahydro-4,9:16,21-dimetheno-1H,13H-dibenzo(e,k)pyrrolo(3,4-h)(1,4,13)oxadiazacyclohexadecene-1,3(2H)-dione, Arxxant, Lilly brand of ruboxistaurin mesilate hydrate;
rugosin E, rugosin E;
rutecarpine, rutaecarpine, rutecarpine;
Rutin, 2-(3,4-dihydroxyphenyl)-5,7-dihydroxy-3-[(2S,3R,4S,5S,6R)-3,4,5-trihydroxy-6-[[(2R,3R,4R,5R,6S)-3,4,5-trihydroxy-6-methyl-oxan-2-yl]oxymethyl]oxan-2-yl]oxy-chromen-4-one,
2-(3,4-dihydroxyphenyl)-5,7-dihydroxy-3-[(2S,3R,4S,5S,6R)-3,4,5-trihydroxy-6-[[(2R,3R,4R,5R,6S)-3,4,5-trihydroxy-6-methyl-tetrahydropyran-2-yl]oxymethyl]tetrahydropyran-2-yl]oxy-chromen-4-one, 2-(3,4-dihydroxyphenyl)-5,7-dihydroxy-3-[(2S,3R,4S,5S,6R)-3,4,5-trihydroxy-6-[[(2R,3R,4R,5R,6S)-3,4,5-trihydroxy-6-methyl-tetrahydropyran-2-yl]oxymethyl]tetrahydropyran-2-yl]oxy-chromone;
S-(beta-p-methoxypropiophenone)thiamine, MB 2, MB-2, S-(beta-p-methoxypropiophenone)thiamine;
S-Nitroso-N-Acetylpenicillamine, N-Acetyl-S-Nitrosopenicillamine, N2-Acetyl-S-Nitroso-D,L-Penicillinaminamide, S-Nitroso-N-Acetylpenicillamine;
S-Nitrosothiols, S-Nitrosothiol, S-Nitrosothiols;
S-phenyl-N-acetylcysteine, 2-acetamido-3-phenylthiopropanoic acid, phenylmercapturic acid, S-phenyl-N-acetylcysteine;
sabarubicin, 4-demethoxy-7-O-(2,6-dideoxy-4-O-(2,3,6-trideoxy-3-amino-alpha-L-lyxo-hexopyranosyl)-alpha-L-lyxo-hexopyranosyl)-adriamycinone, MEN 10755, MEN-10755;
sabcomeline, (R)-3-quinuclidineglyoxylonitrile-(Z)-(O)-methyloxime, alpha-(methylimino)-l-azabicyclo(2.2.2)octane-3-acetonitrile, sabcomeline;
Safingol, (2S,3R)-2-amino-1,3-octadecanediol, (2S,3R)-2-aminooctadecane-1,3-diol, (R-(R*,S*))-2-aminooctadecane-1,3-diol;
Safrole, 4-Allyl-1,2-methylenedioxybenzene, Safrol, Safrole;
SAGA, (1R,3S)-2,2-dimethyl-3-(1,2,2,2-tetrabromoethyl)-1-cyclopropanecarboxylic acid [(S)-cyano-[3-(phenoxy) phenyl]methyl] ester, (1R,3S)-2,2-dimethyl-3-(1,2,2,2-tetrabromoethyl)cyclopropane-1-carboxylic acid [(S)-cyano-[3-(phenoxy)phenyl]methyl] ester, (S)-alpha-Cyano-3-phenoxybenzyl (1R)-cis-2,2-dimethyl-3-((RS)-1,2,2,2-tetrabromoethyl)-cyclopropanecarboxylate;
SAHA, 8-(hydroxyamino)-8-keto-N-phenyl-caprylamide, 8-(hydroxyamino)-8-oxo-N-phenyl-octanamide, 8-(hydroxyamino)-8-oxo-N-phenyloctanamide;
saikosaponin, saikosaponin, saikosaponin B, saikosaponin B1;
Salicin, .beta.-D-Glucopyranoside, 2-(hydroxymethyl)phenyl, 2-(hydroxymethyl)-6-[2-(hydroxymethyl)phenoxy]oxane-3,4,5-triol, 2-(hydroxymethyl)-6-[2-(hydroxymethyl)phenoxy]tetrahydropyran-3,4,5-triol;
salvin, carnosic acid, salvin;

samarium, 62Sm, samario, samarium;
SAMe, (2S)-2-amino-4-[[(2S,3S,4R,5R)-5-(6-amino-9-purinyl)-3,4-dihydroxy-2-tetrahydrofuranyl]methyl-methylsulfonio]butanoate, (2S)-2-amino-4-[[(2S,3S,4R,5R)-5-(6-aminopurin-9-yl)-3,4-dihydroxy-oxolan-2-yl]methyl-methyl-sulfonio]butanoate, (2S)-2-amino-4-[[(2S,3S,4R,5R)-5-(6-aminopurin-9-yl)-3,4-dihydroxy-tetrahydrofuran-2-yl]methyl-methyl-sulfonio]butanoate;
sanguinarine, 13-methyl-2H,10H-[1,3]dioxolo[4,5-i][1,3]dioxolo[4',5':4,5]benzo[1,2-c]phenanthridinium, 13-methyl [1,3]benzodioxolo[5,6-c]-1,3-dioxolo[4,5-i]phenanthridinium, sanguinarine;
sapogenins, sapogenin, sapogenins;
Saquinavir, Invirase, Ro 31 8959, Ro 31-8959;
Sarasar, ( ) 4-(2-(4-(8-Chloro-3,10-dibromo-6,11-dihydro-5H-benzo(5,6)cyclohepta(1,2-b)pyridin-11-yl)-1-piperidinyl)-2-oxoethyl)-1-piperidinecarboxamide, (+)-4[2-[4-(8-Chloro-3,10-dibromo-6,11-dihydro-5H-benzo[5,6] cyclohepta[1,2-b]-pyridin-11(R)-yl-1-piperidinyl]-2-oxo-ethyl]-1-piperidinecarboxamide, 1-Piperidinecarboxamide, 4-(2-(4-((11R)-3,10-dibromo-8-chloro-6,11-dihydro-5H-benzo(5,6)cyclohepta(1,2-b)pyridin-11-yl)-1-piperidinyl)-2-oxoethyl)-;
Sarna, ()-Menthol, (+)-Isomenthol, (+)-Menthol;
sauchinone, sauchinone;
saxatilin, saxatilin;
SB 218078, SB 218078, SB-218078;
SB 225002, N-(2-hydroxy-4-nitrophenyl)-N'-(2-bromophenyl)urea, SB 225002, SB-225002;
SB 415286, 3-(3-chloro-4-hydroxyphenylamino)-4-(4-nitrophenyl)-1H-pyrrole-2,5-dione, SB 415286, SB-415286;
SB-705498, SB-705498;
scandium, 21Sc, escandio, scandium;
SCH 66712, SCH 66712, SCH66712;
schizandrer A, gomisin C, gomisin-G, PC 315;
scoparone, 6,7-dimethoxycoumarin, 6,7-dimethylesculetin, scoparone;
Scopoletin, .beta.-Methylesculetin, 2H-1-Benzopyran-2-one, 7-hydroxy-6-methoxy-, 2H-1-Benzopyran-2-one, 7-hydroxy-6-methoxy-(9Cl);
Score, 1-((2-(2-Chloro-4-(4-chlorophenoxy)phenyl)-4-methyl-1,3-dioxolan-2-yl)methyl)-1H-1,2,4-triazole, 1-((2-[2-Chloro-4-(4-chlorophenoxy)phenyl]-4-methyl-1,3-dioxolan-2-yl)methyl)-1H-1,2,4-triazole, 1-({2-[2-chloro-4-(4-chlorophenoxy)phenyl]-4-methyl-1,3-dioxolan-2-yl}methyl)-1H-1,2,4-triazole;
SDX 308, CEP 18082, CEP-18082, CEP18082;
Selegiline, Bristol Myers Squibb Brand of Selegiline, Bristol-Myers Squibb Brand of Selegiline, Deprenalin;
seocalcitol, 1(S),3(R)-dihydroxy-20(R)-(5'-ethyl-5'-hydroxyhepta-1'(E),3'(E)-dien-1'-yl)-9,10-secopregna-5(Z),7(E),10(19)-triene, EB 1089, EB-1089;
Sep-Pak, Sep-Pak;
Serad, (1S,4S)-4-(3,4-Dichlorophenyl)-1,2,3,4-tetrahydro-N-methyl-1-naphthylamine hydrochloride, (1S,4S)-4-(3,4-Dichlorophenyl)-1,2,3,4-tetrahydro-N-methyl-1-napthalenamine hydrochloride, (1S,4S)-4-(3,4-dichlorophenyl)-N-methyl-1,2,3,4-tetrahydronaphthalen-1-amine hydrochloride;
sertindole, 1-(2-{4-[5-chloro-1-(4-fluorophenyl)-1H-indol-3-yllpiperidin-1-yllethyl)imidazolidin-2-one, 1-[2-[4-[5-chloro-1-(4-fluorophenyl)-indol-3-yl]-1-piperidyl]ethyl]imidazolidin-2-one, Serdolect;
sevoflurane, BAX 3084, fluoromethyl hexafluoroisopropyl ether, fluoromethyl-2,2,2-trifluoro-1-(trifluoromethyl)ethyl ether; SEW2871, SEW-2871, SEW2871;
shikonin, shikonin, shikonin, (+)-isomer;
siderophore, ironophore, siderochrome, siderochromes;
Sildenafil, 1-((3-(4,7-Dihydro-1-methyl-7-oxo-3-propyl-1H-pyrazolo(4,3-d)pyrimidin-5-yl)-4-ethoxyphenyl)sulfonyl)-4-methylpiperazine, 5-[2-ethoxy-5-(4-methylpiperazin-1-yl)sulfonyl-phenyl]-1-methyl-3-propyl-4H-pyrazolo[5,4-e] pyrimidin-7-one, 5-[2-ethoxy-5-(4-methylpiperazin-1-yl)sulfonylphenyl]-1-methyl-3-propyl-4H-pyrazolo[5,4-e]pyrimidin-7-one;
silvestrol, silvestrol;
silybin, 2,3-dehydrosilybin, Alepa-forte, Ardeyhepan;
Sincalide, Bracco Brand of Sincalide, CCK-8, CCK-OP;
Sizofiran, Schizophyllan, Sizofilan, Sizofiran;
SK-7041, SK-7041;
SK&F 106528, recombinant soluble CD4, SK&F 106528, SK&F-106528;
SM 7368, SM 7368, SM-7368, SM7368;
Sodium pentosan poly sulfate, Sodium pentosan poly sulfate;
Sodium Salicylate, Sodium Salicylate;
sorafenib, 4-(4-(3-(4-chloro-3-trifluoromethylphenyl)ureido)phenoxy)pyridine-2-carboxyllic acid methyamide-4-methylbenzenesulfonate, BAY 43-9006, Nexavar;
sorbinil, CP 45634, CP-45634, D-6-fluoro-spiro(chroman-4,4'-imidazolidine)-2',5'-dione;
Sorbo, 1,2,3,4,5,6-Hexanehexol, 3,3'-Oxydi(1,2-propanediol), alpha,alpha'-Diglycerol;
Sorbose, (2R,3S,4R,5R)-2-(hydroxymethyl)oxane-2,3,4,5-tetrol, (2R,3S,4R,5R)-2-(hydroxymethyl)tetrahydropyran-2,3,4,5-tetrol, (2R,3S,4R,5R)-2-methyloltetrahydropyran-2,3,4,5-tetrol;
spiroglumide, 4-(3-chlorobenzamido)-5-(8-azaspiro(4.5)decan-8-yl)-5-oxopentanoic acid, CR 2194, CR-2194;
Spironolactone, Aldactone, Aldactone A, Alphapharm Brand of Spironolactone;
squamocin, squamocin;
SR 144528, N-(1,3,3-trimethylbicyclo(2.2.1)heptan-2-yl)-5-(4-chloro-3-methylphenyl)-1-(4-methylbenzyl)pyrazole-3-carboxamide, SR 144528, SR-144528;
SR 27897, SR 27897;
SR 48692, 2-((1-(7-chloro-4-quinolinyl)-5-(2,6-dimethoxyphenyl)pyrazol-3-yl)carbonylamino)tricyclo(3.3.1.1.(3.7)) decan-2-carboxylic acid, SR 48692, SR-48692;
SR 80327A, SR 80327A, SR-80327A;
SR 90107A-ORG 31540, SR 90107A-ORG 31540, SR90107A-ORG31540;
ST 638, alpha-cyano-3-ethoxy-4-hydroxy-5-phenylmethylcinnamamide, ST 638, ST-638;
stallimycin, stallimycin;
Stanozolol, Androstanazol, Methylstanazol, Sanofi Winthrop Brand of Stanozolol;
staurosporine, (5S,6R,7R,9R)-6-methoxy-5-methyl-7-methylamino-6,7,8,9,15,16-hexahydro-5H,14H-5,9-epoxy-4b,9a,15-triazadibenzo[b,h]cyclonona[1,2,3,4-jkl]cyclopenta[e]-as-indacen-14-one, Staurosporin, staurosporine;
Stearin, 1,2,3-Propanetriol trioctadecanoate, 1,2,3-trioctadecanoyl-sn-glycerol, 1,2,3-Trioctadecanoylglycerol;
Stereoisomerism, Stereoisomer, Stereoisomerism, Stereoisomers;
Steviol, Kaur-16-en-18-oic acid, 13-hydroxy-, (4.alpha.)-, Steviol;
Stevioside, 1-0-{(5beta,8alpha,9beta,10alpha,13alpha)-13-[(2-O-beta-D-glucopyranosyl-beta-D-glucopyranosyl) oxy]-18-oxokaur-16-en-18-yl}-beta-D-glucopyranose, 13-[(2-O-.beta.-D-Glucopyranosyl-.alpha.-D-glucopyranosyl) oxy]kaur-16-en-18-oic acid .beta.-D-glucopyranosyl ester, Diterpene glycoside;
STIL, (E)-3,4-Bis(4-hydroxyphenyl)-3-hexene, (E)-4,4'-(1,2-Diethyl-1,2-ethenediyl)bisphenol, (E)-4,4'-(hex-3-ene-3,4-diyl)diphenol;
stilbene-disulphonate, stilbene-disulphonate;
Stilbenes, Stilbenes;
Stim, 1,3,7-trimethyl-2,6-dioxo-1,2,3,6-tetrahydropurine, 1,3,7-Trimethyl-2,6-dioxopurine, 1,3,7-trimethyl-3,7-dihydro-1H-purine-2,6-dione;
Streptomycin, CEPA Brand of Streptomycin Sulfate, Clariana Brand of Streptomycin Sulfate, Estreptomicina CEPA;
Styrene, Styrene, Styrene Monomer, Styrol;
styrene-methylmethacrylate copolymer, styrene-methylmethacrylate copolymer;
SU 5416, 3-(2,4-dimethylpyrrol-5-yl)methylidene-indolin-2-one, Semaxinib, SU 5416;
SU 6668, (Z)-3-(2,4-dimethyl-5-(2-oxo-1,2-dihydro-indol-3-ylidenemethyl)-1H-pyrrol-3-yl)-propionic acid, (Z)-3-(2,4-dimethyl-5-(2-oxo-1,2-dihydro-indol-3-ylidenemethyl)-1H-pyrrol-3-yl)propionic acid, SU 6668;
SU 9516, 3-(1-(3H-Imidazol-4-yl)-meth-(Z)-ylidene)-5-methoxy-1,3-dihydro-indol-2-one, SU 9516, SU9516;
suberate, octanedioate, suberate;
suberosin, suberosin;
succinic semialdehyde, 3-formylpropanoic acid, 3-formylpropionic acid, 4-oxobutanoic acid;
Sufentanil, Abbot Brand of Sufentanil Citrate, Baxter Brand of Sufentanil Citrate, curasan Brand of Sufentanil Citrate;
Suldox, 4-amino-N-(5,6-dimethoxy-4-pyrimidinyl)benzenesulfonamide; 5-(4-chlorophenyl)-6-ethylpyrimidine-2,4-diamine, 4-amino-N-(5,6-dimethoxypyrimidin-4-yl)benzenesulfonamide; 5-(4-chlorophenyl)-6-ethyl-pyrimidine-2,4-diamine,
4-amino-N-(5,6-dimethoxypyrimidin-4-yl)benzenesulfonamide; 5-(4-chlorophenyl)-6-ethylpyrimidine-2,4-diamine;
sulfadoxine-pyrimethamine, Fansidar, Suldox, sulfadoxine-pyrimethamine;
Sulfamethazine, Sulfadimezine, Sulfadimidine, Sulfamethazine;
sulfamethoxazole hydroxylamine, sulfamethoxazole hydroxylamine;
Sulfaphenazole, Phenylsulfapyrazole, Sulfaphenazole, Sulfaphenylpyrazol;
Sulfasalazine, Alphapharm Brand of Sulfasalazine, Ashbourne Brand of Sulfasalazine, Asulfidine;
sulfate cellufine, sulfate cellufine;
sulfate-sulfate, sulfate-sulfate;
sulfidonitrogen(.), (NS)(.), mononitrogen monosulfide, nitrogen monosulfide;
Sulfinpyrazone, Anturan, Anturane, Apo Sulfinpyrazone;
sulfo-N-succinimidyl oleate, SSO cpd, sulfo-N-succinimidyl oleate, sulfo-N-succinimidyloleate;
sulfo-succinimidyl-oleate, sulfo-succinimidyl-oleate;

sulfogalactosylglycerolipid, sgg lipid, sulfogalactosylglycerolipid;
sulfones, sulfone, sulfones;
sulfonic acid, acide sulfonique, HSHO3, hydridohydroxidodioxidosulfur;
sulfonyl-phenyl-ethyl, sulfonyl-phenyl-ethyl;
Sulforafan, 1-Isothiocyanato-4-(methylsulfinyl)-butane, 1-isothiocyanato-4-(methylsulfinyl)butane, 1-isothiocyanato-4-methylsulfinyl-butane;
Sulindac, Aclin, Alphapharm Brand of Sulindac, Apo Sulin;
sulindac sulfone, (5-fluoro-2-methyl-1-(3,4,5-trimethoxybenzylidene)-3-(N-benzyl)-indene)-acetamide, 1H-Indene-3-acetic acid, 5-fluoro-2-methyl-1-((4-(methylsulfonyl)phenyl)methylene)-, (Z)-, Aptosyn;
sultopride, amisulpride, Barnetil, DAN 2163;
sunitinib, 5-(5-fluoro-2-oxo-1,2-dihydroindolylidenemethyl)-2,4-dimethyl-1H-pyrrole-3-carboxylic acid (2-diethylaminoethyl)amide, SU 011248, SU 11248;
Synthos, alpha-TCP, alpha-tri-Calcium phosphate, alpha-Tricalcium phosphate;
T 0070907, T 0070907, T-0070907, T0070907;
T 0901317, N-(4-(1,1,1,3,3,3-hexafluoro-2-hydroxy-propan-2-yl)phenyl)-N-(2,2,2-trifluoroethyl)benzenesulfonamide, T 0901317, T-0901317;
Tacrine, 1,2,3,4-Tetrahydro-9-acridinamine, 1,2,3,4-Tetrahydroaminoacridine, 9-Amino-1,2,3,4-Tetrahydroacridine;
Tacrolimus, Anhydrous Tacrolimus, Cilag Brand of Tacrolimus, FK 506;
tadalafil, Cialis, IC 351, IC-351;
Tamogel, (Z)-4-(1-[4-(Dimethylaminoethoxy)phenyl]-2-phenyl-1-butenyl)phenol, (Z)-4-Hydroxytamoxifen, 4-((1Z)-1-(4-(2-(dimethylamino)ethoxy)phenyl)-2-phenyl-1-butenyl)phenol;
Tamoxifen, ICI 46474, ICI 47699, ICI-46,474;
tandospirone, 3a,4,7,7a-hexahydro-2-(4-(4-(2-pyrimidinyl)-1-piperazinyl)butyl)-4,7-methano-1H-isoindole-1,3 (2H)-dione, SM 3997, SM-3997;
Tangeretin, 2-(4-Methoxyphenyl)-5,6,7,8-tetramethoxy-4H-1-benzopyran-4-one, 4',5,6,7,8-Pentamethoxyflavone, 4H-1-Benzopyran-4-one, 2-(4-methoxyphenyl)-5,6,7,8-tetramethoxy-;
tanshinone, tanshinone, tanshinone II A, tanshinone II B;
taurocholic acid, 2-[(3alpha,7alpha,12alpha-trihydroxy-24-oxo-5beta-cholan-24-yl)amino]ethanesulfonic acid, 3alpha,7alpha,12alpha-trihydroxy-5beta-cholanic acid 24-taurine, cholic acid taurine conjugate;
Taurodeoxycholic Acid, Deoxycholyltaurine, Sodium Taurodeoxycholate, Taurine Deoxycholate;
tauroursodeoxycholic acid, tauroursodeoxycholic acid, tauroursodeoxycholic acid, (3alpha,5alpha,7alpha)-isomer, tauroursodeoxycholic acid, monosodium salt, (3alpha,5beta,7alpha)-isomer;
tautomycetin, tautomycetin;
TAXOTERE, (2aR,4S,4aS,6R,9S,11S,12S,12aR,12bS)-12b-(acetyloxy)-12-(benzoyloxy)-2a,3,4,4a, 5,6,9,10,11,12,12a,12b-dodecahydro-4,6,11-trihydroxy-4a,8,13,13-tetramethyl-5-oxo-7,11-methano-1H-cyclodeca[3,4]benz[1,2-b]oxet-9-yl (aR,bS)-b-[[(1,1-dimethylethoxy)carbonyl]am, Docetaxel, Docetaxel anhydrous;
TBDZ, 1H-Benzimidazole, 2-(4-thiazolyl)-, 2-(1,3-thiazol-4-yl)-1H-benzimidazole, 2-(1,3-Thiazol-4-yl)benzimidazole;
TBHQ, 1,4-Benzenediol (1,1-dimethylethyl)-, 1,4-Benzenediol, 2-(1,1-dimethylethyl)-, 2-(1,1-Dimethylethyl)-1,4-benzenediol;
TCAT, 2,3,3-trichloroacryloyl chloride, 2,3,3-trichloroprop-2-enoyl chloride, 2,3,4-Trichloro-2-propenoyl chloride;
technetium, 43Tc, technetium, tecnecio;
Tegafur, 1-(2-Tetrahydrofuryl)-5-fluorouracil, 1-(Tetrahydro-2-furanyl)-5-fluorouracil, 5-Fluoro-1-(tetrahydro-2-furanyl)-2,4-pyrimidinedione;
Teleocidin, 13-Ethenyl-1,3,4,5,7,8,10,11,12,13-decahydro-4-(hydroxymethyl)-8,10,13-trimethyl-7,10-bis(1-methylethyl)-6H-benzo[g]diazonino[7,6,5-cd]indol-6-one, 6H-Benzo(g)(1,4)diazonino(7,6,5-cd)indol-6-one, 13-ethenyl-1,3,4,5,7,8,10,11,12,13-decahydro-4-(hydroxymethyl)-8,10,13-trimethyl-7,10-bis(1-methylethyl)-, (4S-(4R*,7R*, 10S*,13S*))-, Teleocidin;
telithromycin, Aventis brand of telithromycin, HMR 3647, HMR-3647;
Temazepam, 3 Hydroxydiazepam, 3-Hydroxydiazepam, AHP Brand of Temazepam;
temozolomide, 8-carbamoyl-3-methylimidazo(5,1-d)-1,2,3,5-tetrazin-4(3H)-one, CCRG 81045, CCRG-81045;
temsirolimus, CCI 779, CCI-779, temsirolimus;
terbinafine, (E)-N-(6,6-dimethyl-2-heptenynyl)-N-methyl-1-naphthalenementhamin hydrochloride, Lamisil, SF 86-327;
terephthalic acid, 1,4-Benzenedicarboxylic acid, benzene-1,4-dicarboxylic acid, p-benzenedicarboxylic acid;

Terfenadine, Aliud Brand of Terfenadine, alpha-(4-(1,1-Dimethylethyl)phenyl)-4-(hydroxydiphenylmethyl)-1-piperdinebutanol, Balkis Saft Spezial;
teriflunomide, teriflunomide;
terrein, terrein;
territrem A, territrem A;
territrem B, territrem B;
territrem C, territrem C;
tertiapin, tertiapin;
tetra-mu3-sulfido-tetrairon, 4Fe-4S, IRON/SULFUR CLUSTER, tetramu3-sulfido-tetrairon;
tetrachloroethene, 1,1,2,2-tetrachloroethylene, ethylene tetrachloride, PCE;
tetradecanoyl-phorbol-acetate, tetradecanoyl-phorbol-acetate; Tetrahydrocannabinol, 9 ene Tetrahydrocannabinol, 9-ene-Tetrahydrocannabinol, delta(1)-Tetrahydrocannabinol;
tetramethylrhodamine, tetramethylrhodamine;
tetramethylsilane, silane, tetramethyl-, tetramethylsilane, TMSCH2Li;
tetraphene, 1,2-benzanthracene, 1,2-Benzanthrazen, benz[a]anthracene;
Tetraprenol, (2E,6E,10E)-3,7,11,15-tetramethylhexadeca-2,6,10,14-tetraen-1-ol, (E,E,E)-Geranylgeraniol, 2,6,10,14-hexadecatetraen-1-ol, 3,7,11,15-tetramethyl-;
tetrasulfanide, HSSSS(-), SSS[S-], tetrasulfanide;
Thalidomide, Celgene Brand of Thalidomide, Sedoval, Thalidomide;
Thapsigargin, Thapsigargin;
thiamine disulfide, thiamine disulfide;
thiazole, 1,3-thiazole, c1cscn1, thiazole;
Thiazolidinediones, Glitazones, Thiazolidinediones;
thioacetamide, Acetothioamide, ethanethioamide, TAA;
Thioacetazone, Ambathizon, Amithiozone, Conteben;
thiobenzamide, thiobenzamide;
thiocoraline, thiocoraline;
Thiole, Divinylene sulfide, Furan, thio-, Huile H50;
Thiopental, Abbott Brand of Thiopental Sodium, Altana Pharma Brand of Thiopental Sodium, Bomathal;
thioredoxin dithiol, Reduced thioredoxin, thioredoxin, thioredoxin dithiol;
thioridazine, 10-[2-(1-methyl-2-piperidyl)ethyl]-2-methylsulfanyl-phenothiazine, 10-[2-(1-methylpiperidin-2-yl)ethyl]-2-(methylsulfanyl)-10H-phenothiazine, 2-Methylmercapto-10-(2-(N-methyl-2-piperidyl)ethyl)phenothiazine;
Thiostrepton, Bryamycin, Gargon, Thiactin;
thrombin receptor peptide SFLLRNP, thrombin receptor peptide SFLLRNP;
thrombin Tokushima, thrombin Tokushima;
Thromboxane A2, Rabbit Aorta Contracting Substance, Thromboxane A2;
thromboxane B2, (52,13E,15S)-9alpha,11,15-trihydroxythromboxa-5,13-dien-1-oic acid, thromboxane B2, TXB2;
Thyminose, 2-Deoxy-D-ribose, 2-Deoxy-erythro-pentose, 2-Deoxyribose;
thymol, 1-hydroxy-5-methyl-2-isopropylbenzene, 2-isopropyl-5-methylphenol, 3-p-cymenol;
thymoquinone, 2-isopropyl-5-methylbenzoquinone, 2-methyl-5-isopropyl-p-benzoquinone, dihydrothymoquinone;
Thyrotropin, Thyreotropin, Thyroid Stimulating Hormone, Thyroid-Stimulating Hormone;
Ticlopidine, 53 32C, 53-32C, 5332C;
Tilidine, Go 1261, Go-1261, Go1261;
tipranavir, Aptivus, PNU 140690, PNU-140690;
tirilazad, 21-(4-(2,6-di-1-pyrrolidinyl-4-pyrimidinyl)-1-piperazinyl)-16-methylpregna-1,4,9(11)-triene-3,20-dione monomethane sulfonate, Freedox, Pharmacia brand of tirilazad mesylate hydrate;
titanium alloy (TiAl6V4), Ti-6Al-V4 alloy, Ti6Al4V, titanium 6-aluminum-4-vanadium;
TMC-95A, TMC 95A, TMC-95A;
Tmndga, 1,1'-(2,3-Dimethyl-1,4-butanediyl)bis(3,4-dimethoxybenzene), 1,4-Bis(3,4-dimethoxyphentl)-2,3-dimethylbutane, 4,4'-(2,3-Dimethyl-1,4-butanediyl)bis-1,2-dimethoxybenzene;
TMSI, (Trimethylsilyl)imidazole, 1-(Trimethylsilyl)-1H-imidazole, 1-(Trimethylsilyl)imidazole;
Tobrex, (1S,2S,3R,4S,6R)-4,6-diamino-3-(2,6-diamino-2,3,6-trideoxy-alpha-D-ribo-hexopyranosyloxy)-2-hydroxycyclohexyl 3-amino-3-deoxy-alpha-D-glucopyranoside, (1S,2S,3R,4S,6R)-4,6-diamino-3-[(2,6-diamino-2,3,6-trideoxy-alpha-D-ribo-hexopyranosyl)oxy]-2-hydroxycyclohexyl 3-amino-3-deoxy-alpha-D-glucopyranoside, (2S,3R,4S,5S,6R)-4-amino-2-[(1S,2S,3R,4S,6R)-4,6-diamino-3-[(2R,3R,5S,6R)-3-amino-6-(aminomethyl)-5-hydroxy-oxan-2-yl]oxy-2-hydroxy-cyclohexyl]oxy-6-(hydroxymethyl)oxane-3,5-diol;
tocotrienols, tocotrienol, tocotrienols;

tofisopam, 1-(3,4-dimethoxyphenyl)-5-ethyl-7,8-dimethoxy-4-methyl-5H-2,3-benzodiazepine, dextofisopam, EGYT-341;
tolrestat, tolrestat;
Tolterodine, 2-[(1R)-3-(di(propan-2-yl)amino)-1-phenyl-propyl]-4-methyl-phenol, 2-[(1R)-3-(di(propan-2-yl)amino)-1-phenylpropyl]-4-methylphenol, 2-[(1R)-3-(diisopropylamino)-1-phenyl-propyl]-4-methyl-phenol;
toluene, Cc1ccccc1, methylbenzene, phenylmethane;
TOLUENE-DITHIOL, TOLUENE-DITHIOL;
topiramate, 2,3-4,5-bis-O-(1-methylethylidene)-beta-D-fructopyranose sulfamate, Cilag brand of topiramate, Epitomax;
Topotecan, 9 Dimethylaminomethyl 10 hydroxycamptothecin, 9-Dimethylaminomethyl-10-hydroxycamptothecin, Hycamtamine; Toremifene,
Fareston, FC 1157a, FC-1157a;
Tosylarginine Methyl Ester, Methyl N alpha Tosyl L Arginate, Methyl N-alpha-Tosyl-L-Arginate, TAME;
Tosyllysine Chloromethyl Ketone, Chlorotosylamidoaminoheptanone, TLCK, Tosyllysine Chloromethyl Ketone;
Tosylphenylalanyl Chloromethyl Ketone, Chlorophenyltosylamidobutanone, Tosylphenylalanyl Chloromethane, Tosylphenylalanyl Chloromethyl Ketone;
TPN+, CODEHYDRASE II, coenzyme II, NADP(+);
Tracer, Conserve, Spinosad, Tracer;
Tramadol, 1A Brand of Tramadol Hydrochloride, Able Brand of Tramadol Hydrochloride, AbZ Brand of Tramadol Hydrochloride; trans-resveratrol, (E)-5-(2-(4-hydroxyphenyl)ethenyl)-1,3-benzenediol, (E)-resveratrol, 3,4',5-stilbenetriol;
trastuzumab, Genentech brand of trastuzumab, Herceptin, Hoffman-La Roche brand of trastuzumab;
Trazodone, AF 1161, AF-1161, AF1161;
Tremode, 1,2-Benzisoxazole-3-methanesulfonamide, 1,2-benzoxazol-3-ylmethanesulfonamide, 3-(Sulfamoylmethyl)-1,2-benzisoxazole;
Tremorine, Tremorine;
Tretinoin, all trans Retinoic Acid, all-trans-Retinoic Acid, beta all trans Retinoic Acid;
Triad, Triad, Triad resin, Triad VLC;
Triamcinolone, Aristocort, Triamcinolone, Volon;
triazolam, 8-chloro-6-(2-chlorophenyl)-1-methyl-4H-[1,2,4]triazolo[4,3-a][1,4]benzodiazepine, Halcion, triazolam; triazoles, triazole compounds, triazoles;
tributylstannane, SnBu3H, TBT, Tri-n-butyltin;
trichostatins, trichostatin, trichostatins;
Triclosan, 2-Hydroxy-2',4,4'-trichlorodiphenyl Ether, Aquasept, Clearasil Daily Face Wash;
triethylamine, triethylamine, triethylamine acetate, triethylamine dinitrate;
Trifluoperazine, Allphar Brand of Trifluoperazine Hydrochloride, Apo Trifluoperazine, Apo-Trifluoperazine;
trimethylaminocarboxyldihydroboran, TCDB, trimethylamin-carboxyl-dihydro-boran, trimethylaminocarboxyldihydroboran; trioctyl phosphine oxide, trioctyl phosphine oxide;
Triolein, Glycerol Trioleate, Trielaidin, Trioleate Glycerin; tripterine, celastrol, tripterine;
triptolide, PG490, triptolide;
triterpenoids, Triterpenoid, triterpenoids;
troglitazone, 5-(4-((6-hydroxy-2,5,7,8-tetramethylchroman-2-yl-methoxy)benzyl)-2,4-thiazolidinedione), CS 045, CS-045;
Troleandomycin, Oleandocetin, Pfizer Brand of Troleandomycin, TAO;
TTNPB, (E)-4-(2-(5,6,7,8-Tetrahydro-5,5,8,8-tetramethyl-2-naphthalenyl)-1-propen-1-yl)benzoic acid, (e)-4-(2-(5,6,7,8-tetrahydro-5,5,8,8-tetramethyl-2-naphthalenyl)-1-propenyl)benzoic acid, (e)-4-(2-(5,6,7,8-tetrahydro-5,5,8,8-tetramethyl-2-napthalenyl)-1-propenyl) benzoic acid;
tubocapsanolide A, tubocapsanolide A;
Tunicamycin, Tunicamycin;
tyrphostin AG 1478, 4-(3-chloroanilino)-6,7-dimethoxyquinazoline, AG 1478, AG-1478;
tyrphostin AG-490, AG 490, AG-490, AG490;
tyrphostin AG17, tyrphostin AG17;
U 0126, 1,4-diamino-2,3-dicyano-1,4-bis(2-aminophenylthio)butadiene, U 0126, U-0126;
Ubizol, Androsta-1,4-diene-17-carbothioic acid, 6,9-difluoro-11-hydroxy-16-methyl-3-oxo-17-(1-oxopropoxy)-, (6alpha,llbeta,16alpha,17alpha)-S-(fluoromethyl) ester, Asmatil, atemur;
Ufur, 1-UFT protocol, 5-fluoro-1-(2-tetrahydrofuranyl)pyrimidine-2,4-dione; 1H-pyrimidine-2,4-dione, 5-fluoro-l-(oxolan-2-yl)pyrimidine-2,4-dione; 1H-pyrimidine-2,4-dione;
UK 157147, UK 157,147, UK 157147, UK-147,157;

Uprima, (-)-10,11-dihydroxyaporphine, 6a-beta-Aporphine-10,11-diol, 6abeta-aporphine-10,11-diol;
Uran, 238U, 238U Element, Uran;
uranyl acetate, uranyl acetate;
urethane, carbamic acid ethyl ester, ethyl carbamate, urethane; Urex, 2-Furfurylamino-4-chloro-5-sulfamoylbenzoic acid, 4-chloro-2-(2-furylmethylamino)-5-sulfamoyl-benzoic acid, 4-chloro-2-(2-furylmethylamino)-5-sulfamoylbenzoic acid;
urinastatin, acid-stable protease inhibitor, Miraclid, MR 20;
Urso, (3alpha,5beta,7beta)-3,7-dihydroxycholan-24-oic acid, (3alpha,5beta,7beta,8xi)-3,7-dihydroxycholan-24-oic acid, (4R)-4-[(3R,5S,7S,8R,9S,10S,13R,14S,17R)-3,7-dihydroxy-10,13-dimethyl-2,3,4,5,6,7,8,9,11,12,14,15,16,17-tetradecahydro-1H-cyclopenta[alphenanthren-17-yl]pentanoic acid;
USAN, (1-p-Chlorobenzoyl-5-methoxy-2-methylindol-3-yl)acetic acid, 1-(4-Chlorobenzoyl)-5-methoxy-2-methyl-1H-indole-3-acetic acid, 1-(4-Chlorobenzoyl)-5-methoxy-2-methyl-1H-indole-3-acetic acid & MAP-30;
valerenic acid, valerenic acid;
valspodar, 3'-keto-Bmt(1)-Val(2)-cyclosporin A, PSC 833, PSC-833;
venlafaxine, 1-[2-(dimethylamino)-1-(4-methoxyphenyl)ethyl]cyclohexanol, COc1ccc(cc1)C(CN(C)C)C2(O)CCCCC2, Elafax;
Verapamil, Calan, Cordilox, Dexverapamil;
verlukast, (3-(3-(2-(7-chloro-2-quinolinyl)ethenyl)phenyl)((3-dimethylamino-3-oxopropyl)thio)methyl)thiopropanoic acid, L 660,711, L 660711;
vesnarinone, vesnarinone;
Viagra, 1-((3-(6,7-Dihydro-1-methyl-7-oxo-3-propyl-1H-pyrazolo(4,3-d)pyrimidin-5-yl)-4-ethoxyphenyl)sulfonyl)-4-methylpiperazine citrate (1:1), 5-[2-ethoxy-5-(4-methylpiperazin-1-yl)sulfonyl-phenyl]-1-methyl-3-propyl-4H-pyrazolo[5,4-e]pyrimidin-7-one; 2-hydroxypropane-1,2,3-tricarboxylic acid, 5-[2-ethoxy-5-(4-methylpiperazin-1-yl)sulfonyl-phenyl]-1-methyl-3-propyl-4H-pyrazolo[5,4-e]pyrimidin-7-one; 2-hydroxypropane-1,2,3-tricarboxylic acid; Vigil, (+-)-Modafinil, (-)-2-((R)-(Diphenylmethyl)sulfinyl)acetamide, 2-((Diphenylmethyl)sulfinyl)acetamide;
vincristine, 22-oxovincaleukoblastine, leurocristine, vincristine;
vinflunine, vinflunine;
vinorelbine, 5'-nor-anhydrovinblastine, KW 2307, KW-2307;
vinpocetine, Armstrong brand of vinpocetine, Cavinton, Celltech brand of vinpocetine;
violacein, 3-(1,2-dihydro-5-(5-hydroxy-1H-indole-3-yl)-2-oxo-3H-pyrrol-3-ylidene)-1,3-dihydro-2H-indole-2-one, violacein;
Vira-A, (2R,3R,4R,5R)-2-(6-aminopurin-9-yl)-5-(hydroxymethyl)oxolane-3,4-diol, .alpha.-D-Arabinofuranosyladenine, .beta.-Adenosine;
voriconazole, Pfizer brand of voriconazole, UK 109,496, UK 109496;
vorozole, 6-((4-chlorophenyl)-(1H-1,2,4-triazol-1-yl)methyl)-1-methyl-1H-benzotriazole, R 76713, R 83839;
VX680, cyclopropane carboxylic acid(4-(4-(4-methylpiperazin-1-yl)-6-(5-methyl-2H-pyrazol-3-ylamino)pyrimidin-2-ylsulfanyl)phenyl)amide, MK-0457, VE 465;
Warfarin, 4-Hydroxy-3-(3-oxo-1-phenylbutyl)-2H-1-benzopyran-2-one, Aldo Brand of Warfarin Sodium, Aldocumar;
WAY-169916, WAY-169916;
Win 55212-2, (2,3-dihydro-5-methyl-3-((4-morpholinyl)methyl)pyrrolo-(1,2,3-de)-1,4-benzoxazin-6-yl)(1-naphthalenyl)methanone monomethanesulfonate, 6H-Pyrrolo(3,2,1-ij)quinolin-6-one, 4,5-dihydro-2-methyl-4-(4-morpholinylmethyl)-1-(1-naphthalenylcarbonyl)-, (R)-, WIN 55,212;
withaferin A, 5,6-epoxy-4,22,27-trihydroxy-1-oxoergosta-2,24-dienoic acid delta-lactone, withaferin A;
WLN: QR BG, 2-Chloro-1-hydroxybenzene, 2-Chlorophenol, 2-Hydroxychlorobenzene;
WLN: RVR, .alpha.-Oxodiphenylmethane, .alpha.-Oxoditane, alpha-oxodiphenylmethane;
WLN: ZSWR, BENZENESULFONAMIDE, Benzenesulphonamide, Benzolsulfonamide;
xanthohumol, 1-(2,4-dihydroxy-6-methoxy-3-(3-methyl-2-butenyl)phenyl)-3-(4-hydroxyphenyl)-2-propen-1-one, desmethylxanthohumol, xanthohumol;
Xaxa, 2-(ACETYLOXY)BENZOIC ACID, 2-Acetoxybenzenecarboxylic acid, 2-acetoxybenzoic acid;
Xylit, (2S,4R)-pentane-1,2,3,4,5-pentol, 1,2,3,4,5-Pentanepentol, Adonit;
Y 27632, N-(4-pyridyl)-4-(1-aminoethyl)cyclohexanecarboxamide, Y 27632, Y 27632, (4(R)-trans)-isomer;
yristate, (1aR,1bS,4aR,7aS,7bS,8R,9R,9aS)-9a-acetoxy-4a,7b-dihydroxy-3-(hydroxymethyl)-1,1,6,8-tetramethyl-5-oxo-1a,1b,4,4a,5,7a,7b,8,9,9a-decahydro-1H-cyclopropa[3,4]benzo[1,2-e]azulen-9-yl tetradecanoate, -one 9a-acetate, (+)- (8CI), Err:508;
Z 338, N-(N',N'-diisopropylaminoethyl)-(2-(2-hydroxy-4,5-dimethoxybenzoylamino)-1,3-thiazole-4-yl)carboxyamide, YM 443, YM-443;
zafirlukast, 4-(5-cyclopentyloxycarbonylamino-2-methylindol-3-yl-methyl) -3-methoxy-N-O-tolylsulfonylbenzamide, Accolate, Aeronix;

Zalcitabine, 2',3'-Dideoxycytidine, ddC (Antiviral), Dideoxycytidine;
zardaverine, 6-(4-difluoromethoxy-3-methoxyphenyl)-3(2H)pyridazinone, zardaverine;
ZD 9331, BGC9331, ZD 9331, ZD-9331;
Zearalenone, F 2 Toxin, F-2 Toxin, F2 Toxin;
Zeldox, 2H-Indol-2-one, 5-(2-(4-(1,2-benzisothiazol-3-yl)-1-piperazinyl)ethyl)-6-chloro-1,3-dihydro-, 5-[2-[4-(1,2-benzothiazol-3-yl)-1-piperazinyl]ethyl]-6-chloro-2-indolinone, 5-[2-[4-(1,2-benzothiazol-3-yl)piperazin-1-yl]ethyl]-6-chloro-1,3-dihydroindol-2-one;
zerumbone, zerumbone;
Zidovudine, 3' Azido 3' deoxythymidine, 3'-Azido-2',3'-Dideoxythymidine, 3'-Azido-3'-deoxythymidine;
zileuton, A 64077, A-64077, Abbot 64077;
Zocor, (1S,3R,7S,8S,8aR)-8-{2-[(2R,4R)-4-hydroxy-6-oxotetrahydro-2H-pyran-2-yl]ethyl}-3,7-dimethyl-1,2,3,7,8,8a-hexahydronaphthalen-1-yl 2,2-dimethylbutanoate, 2,2-Dimethylbutanoic acid (1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthalenyl ester, 2,2-dimethylbutanoic acid [(1S,3R,7S,8S,8aR)-8-[2-[(2R,4R)-4-hydroxy-6-oxo-2-tetrahydropyranyl]ethyl]-3,7-dimethyl-1,2,3,7,8,8a-hexahydronaphthalen-1-yl] ester;
zopiclone, 6-(5-chloro-2-pyridyl)-6,7-dihydro-7-oxo-5H-pyrrolo(3,4-b)pyrazin-5-yl 4-methyl-1-piperazinecarboxylate, AbZ brand of zopiclone, Aliud brand of zopiclone;
Zymosan, Zymosan, Zymosan A;
Trospium chloride, 2-hydroxy-2,2-diphenyl-acetic acid [(1R,5S)-spiro[8-azoniabicyclo[3.2.1]octane-8,1'-azolidin-1-ium]-3-yl] ester chloride, 2-hydroxy-2,2-diphenylacetic acid [(1R,5S)-3-spiro[8-azoniabicyclo[3.2.1]octane-8,1'-azolidin-1-ium]yl] ester chloride, 3-((hydroxydiphenylacetyl)oxy)-spiro(8-azoniabicyclo(3.2.1)octane-8,1'-pyrrolidinium chloride;
Valproic Acid, 2 Propylpentanoic Acid, 2-Propylpentanoic Acid, Calcium Valproate;

In preferred embodiments the present invention is defined as follows:

1. FMO3 inhibitor for use in the treatment of pain.
2. FMO3 inhibitor for use according to 1, wherein the FMO3 inhibitor is selected from Tenofovir and Methimazole.
3. FMO3 inhibitor for use according to 1, wherein the FMO3 inhibitor is selected from a compound comprising a phosphoric acid or ester group and/or a purine radical, the compound being Tenofovir.
4. FRK inhibitor selected from dasatinib, motesanib, Doramapimod, Pelitinib, sorafenib, Vandetanib and Canertinib for use in the treatment of pain.
5. FRK inhibitor for use according to 4, wherein the inhibitor is selected from compounds with a Kd of lower than 3000 nM, preferably lower than 2000 nM, especially preferred lower than 1000 nM, selected from dasatinib, motesanib, Doramapimod, Pelitinib, sorafenib and Vandetanib.
6. FRK inhibitor for use according to 4, wherein the inhibitor is selected from compounds comprising a substituted pyridine, quinoline, isoquinoline or pyrimidine group, selected from motesanib (AMG-706), Pelitinib (EKB-569), sorafenib (sorafenib), Vandetanib (Vandetanib) and canertinib ( CI-1033 ).
7. FRK inhibitor for use according to 4 or 5, wherein the inhibitor is selected from compounds comprising a substituted aniline group with a Kd value less than 1000 nM, selected from dasatinib ( dasatinib ), motesanib ( AMG-706 ), Doramapimod ( BIRB 796 ), Pelitinib ( EKB-569 ), sorafenib ( sorafenib ), Vandetanib ( Vandetanib ).
8. FRK inhibitor for use according to 4, wherein the inhibitor is selected from compounds comprising a chlor-, fluor- or chlor- and fluor- substituted aniline group selected from dasatinib, Pelitinib, sorafenib, Vandetanib and canertinib.
9. FRK inhibitor selected from compounds comprising a substituted aniline group, selected from dasatinib ( dasatinib ), motesanib ( AMG-706 ), doramapimod ( BIRB 796 ), pelitinib ( EKB-569 ), sorafenib ( sorafenib ), vandetanib ( vandetanib ), canertinib ( CI-1033 ) and imatinib ( STI-571 ) for use in the treatment of neuropathic pain, preferably trigeminal neuralgia, post-herpetic neuralgia, painful diabetic neuropathy, painful diabetic peripheral neuropathy, diabetic polyneuropathy, such as sciatic pain, radiculopathy, radicular pain, non-inflammatory neuropathic pain or a combination thereof.
10. LPAR3 modulator for use in the treatment of pain.
11. LPAR3 modulator for use according to 10, wherein the modulator is selected from compounds comprising N6-Benzyladenosine-5'-phosphate, p-Aminobenzoly PAB-J acid, NSC161613 and NSC47091.
12. LPAR3 modulator for use according to 10, wherein the LPAR3 modulator is an LPAR3 inhibitor.
13. LPAR3 inhibitor for use according to 12, wherein the inhibitor is selected from compounds comprising p-Aminobenzoly PAB-J acid, NSC161613 and NSC47091.
14. PDE4D inhibitor selected from cilomilast, Roflumilast, Filaminast, Piclamilast, V11294, Apremilast, Arofylline, Ati-Atizoram, Catramilast, Cimpyfylline, Daxalipram, Doxofylline, drotaverin, Efloxate, Etamiphylline, Etazolate, Etofylline, Broncholytine, Irimilast, oglemilast, Choline theophyllinate, Pumafentrine, Revamilast, Ronomilast, Tofimilast, Tolafentrine, Trapidil, GW 842470 (AWD 12-281), CDP-840, YM-976, CI-1018, D-4418, Lirimilast, SCH-351591, RPL-554, IPL-455903 (HT-0712), GSK256066, Zardaverine, Vardenafil, Tetomilast, IC485, L-826,141, ONO-6126, CI-1044, MK-0873, T-2585, R1533 (MEM-1414), Ronomilast, UK-500,001, AN2728, DE-103, Tofisopam, Dextofisopam, Levotofisopam (USAN) for use in the treatment of pain.

15. PDE4D inhibitor of 14 comprising a 1,2-dioxy-aryl group with IC50 value of 1100 nM or less, preferably 1050 nM or less, preferably 1000 nM or less, preferably 950 nM or less, preferably 950 nM or less, preferably 900 nM or less, for use in the treatment of pain, wherein the PDE4D inhibitor is selected from cilomilast ( cilomilast ), Roflumilast ( Roflumilast ), Filaminast ( Filaminast ), Piclamilast ( Piclamilast ), ( V11294 ), Apremilast ( CC-10004 ), Atizoram ( CP80633 ), Catramilast ( Catramilast ), Daxalipram ( Daxalipram/mesopram ), drotaverin ( Drotaverine ), broncholytine ( Glaucine Hydrobromide ), oglemilast ( GRC3886 ), Pumafentrine ( Pumafentrine ), Revamilast ( Revamilast ), Tolafentrine ( Tolafentrine ), (CDP-840 ), ( RPL-554 ), ( IPL-455903 (HT-0712) ), Zardaverine ( Zardaverine ), Tetomilast ( OPC-6535 ), ( L-826,141 ), Ronomilast ( ELB353 ), Tofisopam ( Tofisopam ).

16. PDE4D inhibitor of 14 comprising a 1,2-dioxy-aryl group substituted with an alkyl or flour-alkyl group, or 1,2-dioxy-aryl group condensed in a furan ring containing oxygen selected from cilomilast ( cilomilast ), Roflumilast ( Roflumilast ), Filaminast ( Filaminast ), Piclamilast ( Piclamilast ), ( V11294 ), Apremilast ( CC-10004 ), Daxalipram ( Daxalipram/mesopram ), drotaverin ( Drotaverine ), broncholytine ( Glaucine Hydrobromide ), oglemilast ( GRC3886 ), Pumafentrine ( Pumafentrine ), Revamilast ( Revamilast ), Tolafentrine ( Tolafentrine ), ( RPL-554 ), Zardaverine ( Zardaverine ), Tetomilast ( OPC-6535 ), ( L-826,141 ), Ronomilast ( ELB353 ), Tofisopam ( Tofisopam ) for use in the treatment of pain.

17. PDE4D inhibitor of 14 comprising a 3,5-dichlor-pyridine group or a pyridine group that is not condensed in a 2 ring structure, selected from Roflumilast ( Roflumilast ), Piclami-Piclamilast ( Piclamilast ), oglemilast ( GRC3886 ), Revamilast ( Revamilast ), GW 842470 (AWD 12-281), D-4418, SCH-351591 for use in the treatment of pain.

18. PDE4D inhibitor of 14 comprising a quinoline, isoquinoline or pyrimidine group selected from drotaverine ( Drotaverine ), Pumafentrine ( Pumafentrine ), Tolafentrine ( Tolafentrine ), Trapidil ( Seoanin ), D-4418, SCH-351591, RPL-554, GSK256066, T-2585, Ronomilast ( ELB353 ) for use in the treatment of pain.

19. PDE4D inhibitor of 14 comprising an aniline group, preferably carbonyl- or chlor-substituted aniline, selected from Apremilast ( CC-10004 ), Arofylline ( LAS31025 ), CI-1044, T-2585 for use in the treatment of pain.

20. PDE4D inhibitor comprising a purine ring, selected from V11294, Arofylline ( LAS31025 ), Cimpyfylline ( BRL-61063 ), Doxofylline ( Doxofylline ), Etamiphylline ( Etamiphylline ), Etofylline ( Etofylline ), Choline theophyllinate ( oxtriphyllin ), Theophylline ( Theophylline ) for the treatment of neuropathic pain, especially non-inflammatory neuropathic pain.

21. CAMK1D inhibitor selected from Bosutinib ( SKI-606 ), Pelitinib ( EKB-569 ), EKB-568, midostaurin ( PKC-412 ), tozasertib ( MK-0457, VX 680 ), SU-14813, Ruboxistaurin ( LY-333531 ), CGP-52421, for use in the treatment of pain.

22. CAMK1D inhibitor for use according to 21, the inhibitor comprising a chlor-substituted aniline group and is selected from Bosutinib ( SKI-606 ) and Pelitinib ( EKB-569 ).

23. CAMK1D inhibitor selected from bosutinib ( SKI-606 ), pelitinib ( EKB-569 ), EKB-568 and CGP-52421 for use in the treatment of pain.

24. Compound comprising a quinoline or isoquinoline group, selected from Bosutinib, Pelitinib ( EKB-569 ), drotaverin ( Drotaverine ), Pumafentrine ( Pumafentrine ), Tolafentrine ( Tolafentrine ), ( D-4418 ), ( SCH-351591 ), ( RPL-554 ), ( GSK256066 ), ( T-2585 ), Ronomilast ( ELB353 ), preferably the compound being an inhibitor of FRK, PDE4D or CAMK1D, for use in the treatment of pain, preferably neuropathic pain, more preferably non-inflammatory neuropathic pain.

25. Compound comprising a chlor-substituted aniline group, selected from dasatinib ( dasatinib ), Pelitinib ( EKB-569 ), sorafenib ( sorafenib ), Canertinib ( CI-1033 ), Arofylline ( LAS31025 ), ( T-2585 ), bosutinib (SKI-606), preferably the com-compound being an inhibitor of FRK, PDE4D or CAMK1D, for use in the treatment of pain, preferably neuropathic pain, more preferably non-inflammatory neuropathic pain.

26. dasatinib ( dasatinib ) for use in the treatment of pain.

27. AMG-706 ( motesanib ) for use in the treatment of pain.

28. BIRB 796 ( Doramapimod ) for use in the treatment of pain.

29. EKB-569 ( Pelitinib ) for use in the treatment of pain.

30. sorafenib ( sorafenib ) for use in the treatment of pain.

31. Vandetanib ( Vandetanib ) for use in the treatment of pain.

32. CI-1033 ( Canertinib ) for use in the treatment of pain.

33. imatinib ( STI-571 ) for use in the treatment of pain.

34. N6-Benzyladenosine-5'-phosphate for use in the treatment of pain.

35. p-Aminobenzoyl PAB-J acid for use in the treatment of pain.

36. NSC161613 for use in the treatment of pain.

37. NSC47091 for use in the treatment of pain.

38. cilomilast ( cilomilast ) for use in the treatment of pain.
39. Nicotinamide ( Nicotinamide ) for use in the treatment of pain.
40. Roflumilast ( Roflumilast ) for use in the treatment of pain.
41. Filaminast ( Filaminast ) for use in the treatment of pain.
42. Piclamilast ( Piclamilast ) for use in the treatment of pain.
43. V11294 for use in the treatment of pain.
44. CC-10004 ( Apremilast ) for use in the treatment of pain.
45. LAS31025 ( Arofylline ) for use in the treatment of pain.
46. CP80633 ( Atizoram ) for use in the treatment of pain.
47. Catramilast/Atopik ( Catramilast ) for use in the treatment of pain.
48. BRL-61063 ( Cimpyfylline ) for use in the treatment of pain.
49. Daxalipram/mesopram ( Daxalipram ) for use in the treatment of pain.
50. Doxofylline ( Doxofylline ) for use in the treatment of pain.
51. Drotaverine ( drotaverin ) for use in the treatment of pain.
52. Efloxate ( Efloxate ) for use in the treatment of pain.
53. Etamiphylline ( Etamiphylline ) for use in the treatment of pain.
54. Etazolate ( Etazolate ) for use in the treatment of pain.
55. Etofylline ( Etofylline ) for use in the treatment of pain.
56. Glaucine Hydrobromide ( Broncholytine ) for use in the treatment of pain.
57. GRC3886 ( oglemilast ) for use in the treatment of pain.
58. oxtriphyllin ( Choline theophyllinate ) for use in the treatment of pain.
59. Pumafentrine ( Pumafentrine ) for use in the treatment of pain.
60. Revamilast ( Revamilast ) for use in the treatment of pain.
61. ronomilast ( ELB-353 ) for use in the treatment of pain.
62. Tofimilast ( Tofimilast ) for use in the treatment of pain.
63. Tolafentrine ( Tolafentrine ) for use in the treatment of pain.
64. Seoanin ( Trapidil ) for use in the treatment of pain.
65. GW 842470 (AWD 12-281) for use in the treatment of pain.
66. CDP-840 for use in the treatment of pain.
67. YM-976 for use in the treatment of pain.
68. CI-1018 for use in the treatment of pain.
69. D-4418 for use in the treatment of pain.
70. Lirimilast ( Lirimilast ) for use in the treatment of pain.
71. SCH-351591 for use in the treatment of pain.
72. RPL-554 for use in the treatment of pain.
73. IPL-455903 (HT-0712) for use in the treatment of pain.
74. GSK256066 for use in the treatment of pain.
75. Zardaverine ( Zardaverine ) for use in the treatment of pain.
76. Vardenafil ( Vardenafil ) for use in the treatment of pain.
77. OPC-6535 ( Tetomilast ) for use in the treatment of pain.
78. IC485 for use in the treatment of pain.
79. L-826,141 for use in the treatment of pain.
80. ONO-6126 for use in the treatment of pain.
81. CI-1044 for use in the treatment of pain.
82. MK-0873 for use in the treatment of pain.
83. T-2585 for use in the treatment of pain.
84. R1533 (MEM-1414) for use in the treatment of pain.
85. UK-500,001 for use in the treatment of pain.
86. AN2728 for use in the treatment of pain.
87. DE-103 for use in the treatment of pain.
88. Tofisopam ( Tofisopam ) for use in the treatment of pain.
89. (R)-Tofisopam ( Dextofisopam ) for use in the treatment of . pain.
90. (S)-Tofisopam ( Levotofisopam (USAN) ) for use in the treatment of pain.
91. EKB-568 for use in the treatment of pain.
92. midostaurin ( PKC-412 ) for use in the treatment of pain.
93. tozasertib ( MK-0457, VX 680 ) for use in the treatment of pain.
94. SU-14813 for use in the treatment of pain.
95. lestaurtinib ( CEP-701 ) for use in the treatment of pain.

96. LY-333531 ( Ruboxistaurin ) for use in the treatment of pain.
97. CGP-52421 for use in the treatment of pain.
98. SKI-606 ( Bosutinib ) for use in the treatment of pain.
99. Roscovitine ( Roscovitine ) for use in the treatment of pain.
100. Tenofovir (PMPA) ( Tenofovir ) for use in the treatment of pain.
101. Methimazole ( Methimazole ) for use in the treatment of pain.
102. Remofovir (Pradefovir) for use in the treatment of pain.
103. Adefovir, preferably Adefovir dipivoxil (Bis-POM PMEA), for use in the treatment of pain.
104. Acetazolamide ( Acetazolamide ) for use in the treatment of pain.
105. Luteolin for use in the treatment of pain.
106. Method of treating or reducing pain in a subject comprising administering a compound of any one of claims 1 to 105 to a subject suffering from pain.
107. Method of preventing pain in a subject comprising administering a compound of any one of claims 1 to 105 to a subject suffering from pain.
108. Use of a compound, modulator or inhibitor as defined in any one of claims 1 to 105 for the manufacture of an analgesic or a medicament for the treatment of pain.
109. The method or use of any one of claims 1 to 108, characterized in that the compound is administered topically, enterally or parenterally, in particular preferred orally or rectally, intravenously, intraarterially, intramuscularly, subcutaneously, intradermally, intraperitoneally, transdermally, transmucosally or by inhalation.
110. The method or use of any one of claims 1 to 109, characterized in that the subject to be treated is a mammal, preferably a human.
111. The method or use of any one of claims 1 to 110, characterized in that the compound, modulator or inhibitor is provided in a medicament.
112. The method or use of any one of claims 1 to 111, characterized in that the compound, modulator or inhibitor is provided together with a pharmaceutically acceptable carrier or buffer.
113. The method or use of any one of claims 1 to 112, characterized in that the compound, modulator or inhibitor is administered in a therapeutically effective dosage, preferably a dosage of between 0.01 mg/kg and 1 g/kg.
114. Method or use according to any one of claims 1 to 113, wherein pain is selected from or associated with chronic or acute pain or hyperalgesia pain, somatogenic pain, neuropathic pain, psychogenic pain, heat induced pain, physical pain, nociception, hyperalgesia, rheumatic pain, headache, low back pain, pelvic pain, myofascial pain, vascular pain, migraine, wound associated pain, inflammatory pain, arthritic pain, diabetic pain, pain from cancer, somatic visceral pain, fibromyalgia, postoperative pain, phantom pain, trigeminal neuralgia, post-herpetic neuralgia, painful diabetic neuropathy, painful diabetic peripheral neuropathy, diabetic polyneuropathy, sciatic pain, radiculopathy, radicular pain, lumbar pain or any combination thereof.
115. Method or use according to 114, wherein pain is chronic pain.
116. Method or use according to 114 or 115, wherein pain is neuropathic pain.
117. Method or use according to 114, wherein pain is inflammatory pain.
118. Method or use according to 114, 115 or 116, wherein pain is non-inflammatory pain.
119. The method of treating or preventing pain in a subject comprising the administration of a therapeutic compound selected from the compounds of table 1.
120. The method of reducing pain in a subject or to treat train comprising the administration of an antagonist of one or more of the genes or gene products thereof, selected from genes CACNA2D3 (alpha-2-delta-3), PIK3CG or any gene selected from one or more of the genes listed in table 1, in particular selected from CG10033, CG10095, CG10142, CG10153, CG10158, CG10186, CG10186, CG10228, CG10265, CG1031, CG10332, CG10332, CG10481, CG10537, CG10550, CG1058, CG10583, CG10603, CG10603, CG10612, CG10641, CG10667, CG10686, CG10689, CG10689, CG10691, CG10706, CG10711, CG10728, CG10746, CG10800, CG10823, CG1086, CG10882, CG10932, CG10936, CG10954, CG10988, CG1100, CG1100, CG1101, CG11033, CG11081, CG11183, CG1119, CG11280, CG1130, CG11352, CG11456, CG11508, CG1152, CG11555, CG11577, CG11586, CG11590, CG11592, CG11594, CG11637, CG11637, CG11638, CG11642, CG11715, CG1180, CG1180, CG11820, CG11857, CG11865, CG11878, CG11893, CG11895, CG1193, CG11930, CG11942, CG11967, CG11992, CG12004, CG12030, CG12035, CG12052, CG12079, CG12082, CG12093, CG12131, CG12135, CG12199, CG12209, CG12235, CG12269, CG12290, CG12334, CG12373, CG12559, CG12637, CG1264, CG12641, CG12641, CG12645, CG12663, CG12749, CG12796, CG12797, CG12831, CG12878, CG12932, CG12938, CG12954, CG12975, CG13035, CG13047, CG13061, CG13069, CG13074, CG13096, CG13110, CG13130, CG13130, CG13162, CG13194, CG13196, CG13196, CG13243, CG13308, CG13319, CG13403, CG13559, CG13575, CG13623, CG1371, CG1387, CG13998, CG14028, CG1406, CG14065, CG14086, CG14214, CG14217, CG14240, CG14252, CG14274, CG14351, CG14362, CG14374, CG14374, CG14376, CG14506, CG14514, CG14590, CG14659, CG14710, CG14750, CG14755, CG14804, CG14818, CG14940, CG14952,

CG14980, CG15059, CG15120, CG15153, CG15167, CG15254, CG15307, CG15321, CG15324, CG15427, CG15570, CG15604, CG15604, CG15884, CG16778, CG1683, CG16840, CG16852, CG16854, CG16873, CG16899, CG16932, CG16975, CG17003, CG17027, CG1709, CG17137, CG17146, CG17150, CG17189, CG17234, CG1725, CG17255, CG17266, CG17293, CG17295, CG17521, CG1759, CG17612, CG17673, CG17697, CG17943, CG1800, CG1804, CG18069, CG18088, CG18130, CG18249, CG18332, CG18332, CG18350, CG18350, CG18350, CG18350, CG18372, CG1845, CG18480, CG18624, CG18624, CG18666, CG1915, CG1921, CG1921, CG1966, CG1968, CG1982, CG2038, CG2060, CG2079, CG2100, CG2109, CG2128, CG2158, CG2161, CG2257, CG2257, CG2286, CG2346, CG2371, CG2371, CG2503, CG2522, CG2747, CG2848, CG2848, CG2872, CG2872, CG2901, CG3000, CG30004, CG30004, CG30005, CG30039, CG30050, CG30073, CG30291, CG30342, CG30383, CG30384, CG30404, CG3083, CG3105, CG31065, CG31068, CG31110, CG31267, CG31299, CG31300, CG31623, CG31713, CG31716, CG31718, CG3181, CG3184, CG31841, CG31842, CG31876, CG31886, CG31908, CG31936, CG31955, CG31962, CG32016, CG32025, CG32045, CG32057, CG32121, CG3213, CG32148, CG32150, CG32176, CG32193, CG32219, CG32227, CG3224, CG32278, CG32296, CG32296, CG32313, CG32333, CG32346, CG3241, CG32531, CG32533, CG32540, CG32604, CG32614, CG32678, CG32678, CG32678, CG32678, CG3269, CG32698, CG3270, CG32703, CG32736, CG32779, CG32779, CG32779, CG32792, CG3291, CG3295, CG3298, CG33002, CG33106, CG33106, CG33106, CG33106, CG33106, CG33106, CG33128, CG33135, CG33147, CG33149, CG33166, CG33202, CG33261, CG33275, CG33275, CG3330, CG33346, CG33350, CG3344, CG33484, CG33500, CG33500, CG3351, CG33512, CG33512, CG33530, CG33547, CG33547, CG33653, CG33980, CG34059, CG34140, CG34140, CG34159, CG3421, CG34339, CG34341, CG34364, CG34383, CG34400, CG34401, CG34401, CG34416, CG3474, CG3520, CG3569, CG3581, CG3604, CG3613, CG3619, CG3619, CG3619, CG3707, CG3711, CG3717, CG3735, CG3905, CG3943, CG3949, CG3955, CG3981, CG4013, CG4040, CG4083, CG4094, CG4109, CG4217, CG42250, CG4247, CG4247, CG4260, CG4279, CG4279, CG4351, CG4365, CG4396, CG4459, CG4477, CG4502, CG4550, CG4560, CG4584, CG4587, CG4612, CG4633, CG4633, CG4648, CG4655, CG4673, CG4692, CG4698, CG4742, CG4775, CG4795, CG4798, CG4799, CG4806, CG4807, CG4830, CG4875, CG4875, CG4887, CG4946, CG4975, CG4977, CG5003, CG5012, CG5012, CG5013, CG5014, CG5014, CG5121, CG5134, CG5160, CG5179, CG5183, CG5198, CG5201, CG5219, CG5219, CG5261, CG5287, CG5330, CG5405, CG5411, CG5473, CG5499, CG5516, CG5519, CG5565, CG5580, CG5611, CG5643, CG5644, CG5703, CG5723, CG5725, CG5725, CG5727, CG5757, CG5788, CG5800, CG5818, CG5819, CG5821, CG5842, CG5884, CG5889, CG5890, CG5902, CG5934, CG5940, CG5949, CG5969, CG5977, CG5986, CG6006, CG6098, CG6136, CG6143, CG6177, CG6210, CG6249, CG6340, CG6395, CG6457, CG6496, CG6536, CG6549, CG6553, CG6571, CG6575, CG6582, CG6583, CG6605, CG6620, CG6637, CG6673, CG6703, CG6721, CG6724, CG6822, CG6824, CG6930, CG6930, CG6946, CG6948, CG6963, CG6971, CG6971, CG6972, CG6987, CG6998, CG7006, CG7007, CG7007, CG7010, CG7015, CG7025, CG7059, CG7081, CG7099, CG7100, CG7109, CG7129, CG7145, CG7160, CG7175, CG7175, CG7187, CG7194, CG7211, CG7223, CG7225, CG7292, CG7311, CG7342, CG7358, CG7371, CG7376, CG7422, CG7430, CG7443, CG7467, CG7494, CG7494, CG7497, CG7507, CG7556, CG7583, CG7636, CG7664, CG7708, CG7708, CG7712, CG7728, CG7730, CG7800, CG7800, CG7811, CG7816, CG7856, CG7873, CG7946, CG7957, CG8005, CG8008, CG8009, CG8014, CG8014, CG8029, CG8039, CG8048, CG8107, CG8110, CG8114, CG8203, CG8233, CG8288, CG8325, CG8326, CG8394, CG8432, CG8436, CG8440, CG8487, CG8520, CG8625, CG8631, CG8651, CG8732, CG8764, CG8849, CG8912, CG8914, CG8985, CG8985, CG9022, CG9022, CG9032, CG9067, CG9102, CG9160, CG9172, CG9231, CG9280, CG9311, CG9323, CG9350, CG9388, CG9447, CG9453, CG9460, CG9519, CG9537, CG9548, CG9603, CG9636, CG9636, CG9650, CG9696, CG9739, CG9742, CG9753, CG9753, CG9825, CG9825, CG9901, CG9901, CG9948, or an ortholog thereof, preferably wherein the antagonist is selected from an antibody or an inhibitory RNA, particularly a siRNA.

121. The method according to definition 1 or 120, characterized in that a the compound or antagonist is administered in a effective therapeutic dose.

122. The method of any one of definitions 1 to 121, characterized in that the compound is administered topical, enteral or parenteral, in particular preferred oral or rectal, intravenous, intraarterial, intramuscular, subcutaneous, intradermal or intraperitoneal, transdermal, transmucosal or inhalational.

123. The method of any one of definitions 1 to 122, characterized in that the subject is mammal, preferably a human.

124. The method of any one of definitions 1 to 123, characterized in that the compound or antagonist is provided in a medicament.

125. The method of any one of definitions 1 to 124, characterized in that the compound or antagonist is provided together with a pharmaceutically acceptable carrier or buffer.

126. The method of any one of definitions 1 to 125, characterized in that the compound or antagonist is administered in a dosage of between 0.01 mg/kg and 1 g/kg.

127. Use of a compound as defined in definition 119 or an antagonist as defined in definition 120, preferably further defined as in any one of definitions 121 to 126, for the manufacture of an analgesic or for the treatment of pain.

128. Compound as defined in definition 119 or an antagonist as defined in definition 120 for use in a therapeutic method for the treatment of pain, preferably as further defined in any one of definitions 121 to 127.

129. Method according to any one of definitions 1 to 126, use of definition 127 or compound of definition 128, wherein pain is selected from or associated with chronic or acute pain or hyperalgesiaain, somatogenic pain, neuropathic pain, psychogenic pain, heat induced pain, physical pain, nociception, hyperalgesia, rheumatic pain, headache, low back pain, pelvic pain, myofascial pain, vascular pain, migraine, wound associated pain, inflammatory pain, arthritic pain, diabetic pain, pain from cancer, somatic visceral pain, phantom pain, or any combination thereof.

130. The method of modulating the gene expression or gene function in a cell, wherein the gene is selected from one or more of the genes listed in table 1, in particular selected from CACNA2D3, PIK3CG, CG10033, CG10095, CG10142, CG10153, CG10158, CG10186, CG10186, CG10228, CG10265, CG1031, CG10332, CG10332, CG10481, CG10537, CG10550, CG1058, CG10583, CG10603, CG10603, CG10612, CG10641, CG10667, CG10686, CG10689, CG10689, CG10691, CG10706, CG10711, CG10728, CG10746, CG10800, CG10823, CG1086, CG10882, CG10932, CG10936, CG10954, CG10988, CG1100, CG1100, CG1101, CG11033, CG11081, CG11183, CG1119, CG11280, CG1130, CG11352, CG11456, CG11508, CG1152, CG11555, CG11577, CG11586, CG11590, CG11592, CG11594, CG11637, CG11637, CG11638, CG11642, CG11715, CG1180, CG1180, CG11820, CG11857, CG11865, CG11878, CG11893, CG11895, CG1193, CG11930, CG11942, CG11967, CG11992, CG12004, CG12030, CG12035, CG12052, CG12079, CG12082, CG12093, CG12131, CG12135, CG12199, CG12209, CG12235, CG12269, CG12290, CG12334, CG12373, CG12559, CG12637, CG1264, CG12641, CG12641, CG12645, CG12663, CG12749, CG12796, CG12797, CG12831, CG12878, CG12932, CG12938, CG12954, CG12975, CG13035, CG13047, CG13061, CG13069, CG13074, CG13096, CG13110, CG13130, CG13130, CG13162, CG13194, CG13196, CG13196, CG13243, CG13308, CG13319, CG13403, CG13559, CG13575, CG13623, CG1371, CG1387, CG13998, CG14028, CG1406, CG14065, CG14086, CG14214, CG14217, CG14240, CG14252, CG14274, CG14351, CG14362, CG14374, CG14374, CG14376, CG14506, CG14514, CG14590, CG14659, CG14710, CG14750, CG14755, CG14804, CG14818, CG14940, CG14952, CG14980, CG15059, CG15120, CG15153, CG15167, CG15254, CG15307, CG15321, CG15324, CG15427, CG15570, CG15604, CG15604, CG15884, CG16778, CG1683, CG16840, CG16852, CG16854, CG16873, CG16899, CG16932, CG16975, CG17003, CG17027, CG1709, CG17137, CG17146, CG17150, CG17189, CG17234, CG1725, CG17255, CG17266, CG17293, CG17295, CG17521, CG1759, CG17612, CG17673, CG17697, CG17943, CG1800, CG1804, CG18069, CG18088, CG18130, CG18249, CG18332, CG18332, CG18350, CG18350, CG18350, CG18350, CG18372, CG1845, CG18480, CG18624, CG18624, CG18666, CG1915, CG1921, CG1921, CG1966, CG1968, CG1982, CG2038, CG2060, CG2079, CG2100, CG2109, CG2128, CG2158, CG2161, CG2257, CG2257, CG2286, CG2346, CG2371, CG2371, CG2503, CG2522, CG2747, CG2848, CG2848, CG2872, CG2872, CG2901, CG3000, CG30004, CG30004, CG30005, CG30039, CG30050, CG30073, CG30291, CG30342, CG30383, CG30384, CG30404, CG3083, CG3105, CG31065, CG31068, CG31110, CG31267, CG31299, CG31300, CG31623, CG31713, CG31716, CG31718, CG3181, CG3184, CG31841, CG31842, CG31876, CG31886, CG31908, CG31936, CG31955, CG31962, CG32016, CG32025, CG32045, CG32057, CG32121, CG3213, CG32148, CG32150, CG32176, CG32193, CG32219, CG32227, CG3224, CG32278, CG32296, CG32296, CG32313, CG32333, CG32346, CG3241, CG32531, CG32533, CG32540, CG32604, CG32614, CG32678, CG32678, CG32678, CG32678, CG3269, CG32698, CG3270, CG32703, CG32736, CG32779, CG32779, CG32779, CG32792, CG3291, CG3295, CG3298, CG33002, CG33106, CG33106, CG33106, CG33106, CG33106, CG33106, CG33128, CG33135, CG33147, CG33149, CG33166, CG33202, CG33261, CG33275, CG33275, CG3330, CG33346, CG33350, CG3344, CG33484, CG33500, CG33500, CG3351, CG33512, CG33512, CG33530, CG33547, CG33547, CG33653, CG33980, CG34059, CG34140, CG34140, CG34159, CG3421, CG34339, CG34341, CG34364, CG34383, CG34400, CG34401, CG34401, CG34416, CG3474, CG3520, CG3569, CG3581, CG3604, CG3613, CG3619, CG3619, CG3619, CG3707, CG3711, CG3717, CG3735, CG3905, CG3943, CG3949, CG3955, CG3981, CG4013, CG4040, CG4083, CG4094, CG4109, CG4217, CG42250, CG4247, CG4247, CG4260, CG4279, CG4279, CG4351, CG4365, CG4396, CG4459, CG4477, CG4502, CG4550, CG4560, CG4584, CG4587, CG4612, CG4633, CG4633, CG4648, CG4655, CG4673, CG4692, CG4698, CG4742, CG4775, CG4795, CG4798, CG4799, CG4806, CG4807, CG4830, CG4875, CG4875, CG4887, CG4946, CG4975, CG4977, CG5003, CG5012, CG5012, CG5013, CG5014, CG5014, CG5121, CG5134, CG5160, CG5179, CG5183, CG5198, CG5201, CG5219, CG5219, CG5261, CG5287, CG5330, CG5405, CG5411, CG5473, CG5499, CG5516, CG5519, CG5565, CG5580, CG5611, CG5643, CG5644, CG5703, CG5723, CG5725, CG5725, CG5727, CG5757, CG5788, CG5800, CG5818, CG5819, CG5821, CG5842, CG5884, CG5889, CG5890, CG5902, CG5934, CG5940, CG5949, CG5969, CG5977, CG5986, CG6006, CG6098, CG6136, CG6143, CG6177, CG6210, CG6249, CG6340, CG6395, CG6457, CG6496, CG6536, CG6549, CG6553, CG6571, CG6575, CG6582, CG6583, CG6605, CG6620, CG6637, CG6673, CG6703, CG6721, CG6724, CG6822, CG6824, CG6930, CG6930, CG6946, CG6948, CG6963, CG6971, CG6971, CG6972, CG6987, CG6998, CG7006, CG7007, CG7007, CG7010, CG7015, CG7025, CG7059, CG7081, CG7099, CG7100, CG7109, CG7129, CG7145, CG7160, CG7175, CG7175, CG7187, CG7194,

CG7211, CG7223, CG7225, CG7292, CG7311, CG7342, CG7358, CG7371, CG7376, CG7422, CG7430, CG7443, CG7467, CG7494, CG7494, CG7497, CG7507, CG7556, CG7583, CG7636, CG7664, CG7708, CG7708, CG7712, CG7728, CG7730, CG7800, CG7800, CG7811, CG7816, CG7856, CG7873, CG7946, CG7957, CG8005, CG8008, CG8009, CG8014, CG8014, CG8029, CG8039, CG8048, CG8107, CG8110, CG8114, CG8203, CG8233, CG8288, CG8325, CG8326, CG8394, CG8432, CG8436, CG8440, CG8487, CG8520, CG8625, CG8631, CG8651, CG8732, CG8764, CG8849, CG8912, CG8914, CG8985, CG8985, CG9022, CG9022, CG9032, CG9067, CG9102, CG9160, CG9172, CG9231, CG9280, CG9311, CG9323, CG9350, CG9388, CG9447, CG9453, CG9460, CG9519, CG9537, CG9548, CG9603, CG9636, CG9636, CG9650, CG9696, CG9739, CG9742, CG9753, CG9753, CG9825, CG9825, CG9901, CG9901, CG9948, or an ortholog counterpart thereof, comprising administering a compound of table 1 to said cell.

131. The method of definition 130, wherein the cell is a nerve cell.

132. The method of definition 130 or 131, wherein administration treats, alleviates or prevents pain or hyperalgesia in a subject.

The present invention is further illustrated by the following figures and examples.

***Figures:***

**Figure 1. Thermal nociception in adult** *Drosophila.*

(**A**) Schematic representation of the thermal nociception assay in adult *Drosophila.* (**B**) Avoidance of noxious temperature of 46°C, but not avoidance of "sub-noxious" temperatures (25-35°C), is impaired in *painless* mutant (Painless (EP(2)2451) flies compared to the control strain Canton S (control). Data are presented as mean values +/- SEM. ~20 flies were tested per group, in replicates of at least four cohorts. Significant differences (P<0.001) were observed for temperature and strain responses. Further post hoc (Tukey's) analysis showed a significant temperature avoidance response at 46□C for control (*, P<0.05) but not *painless* flies when compaired to responses at 25°C. (**C**) To set up the experimental screening system, $w^{1118}$ (isogenic to the *UAS-IR* library) x *elav-Gal4* flies (Control, grey; n = 1706) and *painless* mutant flies (*painless,* blue; n= 1816) were tested for avoidance to noxious heat (46°C). Based on these data a Z-score >= 1.65 was calculated as a specific cut-off to identify lines for further screening. *Elav-Gal4* (also containing *UAS-Dicer* 2 (*UAS-DRC2*) for more efficient gene silencing) females were crossed to *UAS-IR* lines to knock-down the target genes in all neurons. All lines that exhibited a thermal avoidance defect (Z-score >= 1.65) were re-rested multiple times. (**D**) Results of the genome-wide screen. ~ 3% (622 transformants) of total lines tested (16051) exhibited a defect in thermal nociception, resulting in 580 candidate pain genes (622 transgenic lines). (**E**) Distribution of adult thermal nociception and developmental lethal hits for 16051 *Drosophila UAS-IR* lines. 1427 *elav-Gal4* x *UAS-IR* lines were developmentally lethal (lethal). Among the 14624 viable lines, 562 lines exhibited defective thermal nociception (pain). Additional 60 lines that exhibited defective nociception as well as a semi-lethal phenotype were labeled as pain & lethal.

**Figure 2. *Straightjacket* controls thermal nociception in adult *Drosophila.***

(**A**) Diagram of the $\alpha$2-$\delta$ family encoding peripheral subunits of $Ca^{2+}$ channels. (**B**) RNAi knock-down of *stj* impairs noxious thermal avoidance in adult *Drosophila* (% avoidance of noxious temperature). *stj-IR1* = Inverted repeat 1, *stj-IR2* = Inverted repeat 2, both crossed to *elav-Gal4;UAS-DCR2.* (**C**) Q-PCR for *stj*-Knock-down efficiency in *elav-Gal4>UAS-stj-IR1/2* adult fly brains. (**D**) Kinetics of temperature-induced paralysis for control and *elav-Gal4>UAS-stj* flies. (**E**) *stj-Gal4* driving expression of lamin:GFP to label nuclei and cell surface CD8:GFP to visualize axonal projections in the brain of adult flies. The pars intercerebralis is marked with an arrow. (**F**) Co-localization of anti-STJ immunostaining and *stj-Gal4>UAS-lamin:GFP.* The pars intercerebralis is marked with an arrow. (**G**) *stj in situ* hybridization in the leg of wild type ($w^{1118}$) flies. Of note, the sense control did not show any signal. DAPI counterstaining is shown as mark nuclei. All data are presented as mean ± sem. * P < 0.05, ** P < 0.01 (Student's t-test).

**Figure 3. *Straightjacket* controls thermal nociception in *Drosophila* larvae.**

1. (A) stj-GAL4 driven expression of UAS-CD8:GFP in larval body wall sensory neurons co-stained with anti-Futsch as a marker for sensory neurons. CD8:GFP expression co-localizes with sensory neurons (Futsch) in larval abdominal hemi-segments (A3) (top panels), and multidendritic sensory neurons (bottom panels). (B) Pan-neuronal knock-down of stj (UAS-stj-IR x elav), a mutant of stj (stj2), and stj mutant larvae over a corresponding deficiency Df (2R)Exel7128 (stj2/def) show severely impaired thermal nociception responses compared to w1118 x elav controls. painless larvae are shown as a control. The impaired larval thermal responses of a stj/def was rescued by reintroducing a wild type stj allele using the P[acman] system (stj2/def, stj+) (Venken et al., 2009). Percent responses ± sem to a

46oC heated probe are shown for the indicated time points. Mean response latency ± sem. P value was generated using a Kruskal-Wallis non-parametric test for median comparison with the Dunn's post-hoc test. All P values depicted highlight significance relative to control responses. stj rescue was also significantly difference from stj2 and stj2/def, (P < 0.001). At least 20 animals were tested three times per genotype.

**Figure 4. $\alpha$2$\delta$3 is required for thermal pain responses in mice.**

8. (A) Southern blotting of genomic DNA in $\alpha 2\delta 3$ wild type (+/+) and $\alpha 2\delta 3$ heterozygous (+/-) ES cells to confirm successful gene targeting. The endogenous wild type and targeted alleles are indicated. A 5' probe was used on Nhe I digested genomic DNA. (B) $\alpha 2\delta 3$ and $\alpha 2\delta 1$ protein expression in brain and isolated DRG lysated from $\alpha 2\delta 3$+/+ (+/+), $\alpha 2\delta 3$+/- (+/-), and $\alpha 2\delta 3$-/- (-/-) mice. Actin is shown as a loading control. (C) Using the hot plate assay, $\alpha 2\delta 3$ mutant mice (n=16) show a delayed acute thermal nociception response as compared to control $\alpha 2\delta 3$+/+ mice (n=12). Littermate mice were used as a control. Values represent the latency (seconds) to respond to the indicated temperatures. (D) CFA-induced inflammatory pain behavior. CFA (20 $\mu$l) was injected into the hindpaw of $\alpha 2\delta 3$+/- (+/+, n=10) and $\alpha 2\delta 3$-/- (-/-, n=21) littermates and mice were tested for thermal pain (54°C) using the hot plate assay on the indicated days. Days 1, 3, 5, and 7 indicate days after CFA injection. All data are presented as mean values ± sem. *p < 0.05; **p < 0.01; ***P < 0.001 comparing mutant versus control mice. # P< 0.05 comparing sensitization to baseline (day -2) of the same genotype (Student's t-test).

**Figure 5. Polymorphisms in *CACNA2D3* ($\alpha$*2*$\delta$*3*) associate with decreased acute and chronic pain in humans.**

(**A**) Schematic representation of the human *CACNA2D3* gene locus on chromosome 3p21.1. The positions of the SNPs assayed are indicated. Blue boxes represent exons. The relative gene position is given in megabases (Mb). (**B**) Homozygous carriers of the rs6777055 minor allele (C/C) were significantly less sensitive to heat wind-up induced sensitivity relative to the other genotypes (C/A or A/A). (**C**) Of 169 lumbar chronic root pain patients 1 year post discectomy those homozygous for the minor allele C/C at SNP rs6777055 and A/A at SNP rs1851048 were less sensitive than the other allele combinations. In each case, the homozygous minor allele is associated with significantly less pain. Note that genotyping was not always successful for every individual, hence, the slightly different total numbers in the chronic pain group. All data are presented as mean values ± sem. *$p$ < 0.05 (Student's t-test).

**Figure 6. $\alpha$2$\delta$3 is expressed in the brain and relays the pain signal to higher order brain centers.**

(**A**) $\beta$-Gal staining of whole brain slices from $\alpha 2\delta 3^{+/-}$ mice that carry the LacZ cassette. Different brain regions that are positive for LacZ expression are indicated. White lines indicate the brain slices displayed in Fig. 7A. (**B-C**) Quantification of % BOLD change and mean activation volume (in voxels) for (**B**) the thalamus and (**C**) the S1 somato-sensory cortex of $\alpha 2\delta 3^{+/+}$ and $\alpha 2\delta 3^{-/-}$ mice. Of note, it has been proposed that the S1 cortical region is involved in the localisation of nociception (Treede et al., 1999). The different stimulation temperatures are indicated. Data are presented as mean +/- sem. *$p$ < 0.05, **$p$ < 0.01 (Student's t-test comparing the respective control and $\alpha 2\delta 3^{-/-}$ groups). (**D**) Cross-correlation matrix of time profiles. Whereas the pain signal spreads from the thalamus to other higher order pain centers in $\alpha 2\delta 3^{+/+}$ mice (red areas), in $\alpha 2\delta 3^{/-}$ mice correlated activation can be only observed up to the level of the thalamus. Very weak activity is found in somato-sensory cortex (SC) for $\alpha 2\delta 3^{-/-}$ mice (green stripes). Data from the structures of left side of the brain are shown following challenge with noxious heat (55°C) at the right hindpaw. SI: sensory input; Th: thalamus; SC: somato-sensory cortex; AC: association cortex; LL: link to limbic system; LS: limbic system; HT: hypothalamus; BG: basal ganglia; C. cerebellum; M: motor cortex, P: periaquaeductal gray. Correlation-coefficients (cc) are given in the range from 0 (green), to +1 (red). n=20 for $\alpha 2\delta 3^{+/+}$; n = 18 for $\alpha 2\delta 3^{-/-}$.

**Figure 7. $\alpha$*2*$\delta$*3* mutant mice exhibit sensory cross-activation in response to thermal and tactile stimuli.**

(**A**) Second order statistical parameters maps showing only the significant differences of heat (55°C) and tactile (vibrissae) stimulation induced brain activation between $\alpha 2\delta 3^{+/+}$ and $\alpha 2\delta 3^{-/-}$ mutant mice. Activation was assessed by BOLD-fMRI. The three planes correspond to the white lines shown in Fig. 6A. The green/blue scale indicates increased peak activation (55°C) in $\alpha 2\delta 3^{+/+}$ control mice compared to $\alpha 2\delta 3^{-/-}$ mutant mice. The yellow/red scale indicates increased activation in $\alpha 2\delta 3^{-/-}$ mutant mice compared to $\alpha 2\delta 3^{+/+}$ control mice. Images depict significant differences of second order group statistics corrected for multiple comparisons over all mice tested (n = 20 for $\alpha 2\delta 3^{+/+}$ mice, n = 18 for $\alpha 2\delta 3^{-/-}$ mice). Arrows point to activated regions; note that for heat stimulation the S1/S2 somato-sensory cortex, the cingulate (Cg) cortex and the motor (M) cortex show significantly higher activity in $\alpha 2\delta 3^{+/+}$ controls. In $\alpha 2\delta 3^{-/-}$ mice, heat stimulation leads to significantly higher activity auditory cortex (AC), the visual cortex (VC), and the olfactory tubercle (OT), as well as the amygdala (Amd) and the hypothalamus (HT). For tactile stimulation, only one small region in the S1 somatosensory

cortex, ipsilateral to the side of stimulation (right), showed significantly higher activity in $\alpha 2\delta 3^{+/+}$ controls, whereas $\alpha 2\delta 3^{/-}$ mice again exhibited increased activation of the VC, AC, and OT, in addition to the caudate putamen (Cpu), S1 and S2 regions of the somato-sensory cortex, and the superior colliculus (SC). (**B,C**) % BOLD changes in the auditory cortex (AC), olfactory tubercle (OT), and visual cortex (VC) in control and a2d3$^{-/}$mice following (**B**) heat (55°C) and (**C**) tactile vibrissal stimu-stimulation. Data is presented as mean values ± sem. *$p$ < 0.05; **$p$ < 0.01 (Student's t-test).

**Figure 8. Thermal nociception in adult *Drosophila.***

**(A)** A schematic outline is shown of the thermal nociception assay we developed to assess the behavioral response to avoid noxious heat. The formula to calculate % avoidance is indicated. Of note, temperature at the top surface was measured at 31°C after 4 minutes. **(B)** Mean avoidance for all tested 16051 *elav-Gal4 -Gal4>UAS-IR* lines were ranked and a line of best fit was generated to display all avoidance responses. The cut-off used to score the *UAS-IR* lines with an impaired thermal nociception response is indicted as a dotted line (Z-score > 1.65 corresponding to a mean avoidance of 82%). **(C)** GO (Gene Ontology) classification of adult thermal nociception hits in *Drosophila.* Pie chart represents the fraction of genes corresponding to each functional category. Numbers indicate the gene counts from all the GO terms included in each functional category. **(D)** Gene set enrichment analysis (GSA). Significant GO terms are classified according to their parent terms - cellular components (CC), biological processes (BP), and molecular functions (MF). Numbers indicate the number of GO terms that constitute each functional class.

**Figure 9. Brain morphology in stj-knockdown flies and *stj-Gal4* expressing neurons and axonal projections in the *Drosophila* CNS.**

(**A**) NC82 staining of control and *stj*-knockdown brains. We imaged the entire and did not observe any structural abnormalities. Representative images are shown at anterior and medial regions of the adult *Drosophila* brain. NC82 labels presynaptic structures and has been previously used to assess brain morphology (Vosshall et al., 2000). (**B**) Nuclear and projection labeling of stj positive cells in the adult *Drosophila* brain. *Stj*-expressing nuclei are situated on the anterior surface and the pars intercerebralis (arrow) of the *Drosophila* brain. *Stj* projections cross the posterior brain and can be also found in the connection from the brain to the ventral nerve cord (arrowhead). Depicted are whole brain confocal images of *stj-Gal4>UAS-Lamin:GFP* and *stj-Gal4>UAS-CD8:GFP* fly lines. Composite images, and single images from anterior, mid, and pos-posterior brain segments are shown. (**C**) Nuclear and cell surface labeling of stj positive cells in the *Drosophila* ventral nerve cord (VNC) labels cell bodies throughout the VNC. CD8:GFP strongly labels neurons in the cervical connective, likely ascending neurons connecting the VNC to the brain (arrow and also see arrow head in B). Data are composite images from *stj-Gal4>UAS-Lamin:GFP* and *stj-Gal4>UAS-CD8:GFP* fly lines.

**Figure 10. *stj* and *stj* expressing nerves control thermal pain in larvae.**

(**A**) Temperature dose-response for control, *stj* mutant *($stj^2$),* and *stj* mutant with *stj* rescue (*$stj^2$/Df(2R)Exe17128, $stj^+$)* Responses were recorded at 44°C, 46°C, 50°C, and 53°C. *stj* rescue larvae exhibited responses that were not significant compared to control, and significantly faster compared to *stj* mutants at all temperatures tested (by Kruskal-wallis non-parametric test for median comparison with the Dunn's post-hoc test) **(B)** *$stj^2$* point mutant larvae exhibit normal mechanosensation. The Kernan scoring system used was as described (Kernan et al., 1994): 0 = no response to touch, 1 = a response of pausing mouth-hook movement, 2 = responding by withdrawing the anterior or turning away from the touch, 3 = a single reverse peristaltic wave away from the touch, and 4 = multiple peristaltic waves away from the touch. Wild type *Canton S* and *Painless* (*Painles*s(EP(2)2451) larvae are also shown. Larval sensitivities were assessed in first and second instar larvae. All data are presented as mean ± sem.

**Figure 11. Characterization of $\alpha 2\delta 3$ mutant mice.**

**(A)** $\alpha 2\delta 3$ mutant mice display normal inflammation in the CFA model. CFA (20 μl) was injected into the hindpaw of $\alpha 2\delta 3^{+/}$(+/+, n = 8) and $\alpha 2\delta 3^{-/-}$ (-/-, n = 10) littermates and mice were tested for paw swelling (paw width in mm) at days -2 (2 days before CFA injection), Day 1, and Day 7 using a spring loaded caliper. (**B**) $\alpha 2\delta 3^{-/-}$ (n=9) and $\alpha 2\delta 3^{+/+}$ littermate controls (n=11) show similar acute mechanical pain thresholds using the von Frey test. **(C-D)** $\alpha 2\delta 3$ mutant mice display no obvious phenotype in the open field, **(E)** tail suspension assays, and (**F**) Rotarod responses. Mean values ± sem from $\alpha 2\delta 3^{+/+}$ (n =12) and $\alpha 2\delta 3^{-/-}$ (n =10) mice are shown. (**G**) $\alpha 2\delta 3$ mutant mice display no obvious phenotype in the tail flick response. Mean values ± sem from $\alpha 2\delta 3^{+/+}$ (n =12) and $\alpha 2\delta 3^{-/-}$ (n =16) mice are shown. There were no significant statistical differences between the groups. (**H**) The IV-plots of peak calcium inward currents derived from $\alpha 2\delta 3^{-/-}$ DRGs (red circles, n=20) is identical to $\alpha 2\delta 3^{+/+}$ DRG neurons (black squares, n=20). (**I-K**) Characterization of voltage-gated calcium current behavior in $\alpha 2\delta 3^{+/+}$ and $\alpha 2\delta 3^{-/-}$ DRGs. (**I**) the maximal conductance (G) achieved upon

membrane depolarization is identical in $\alpha 2\delta 3^{+/+}$ (Gpeak 1.76 ± 0.18 nS/pF and Gsus 1.30 ± 0.14 nS/pF, n=20) and $\alpha 2\delta 3^{-/-}$ (Gpeak 1.65 ± 0.16 nS/pF and Gsus 1.26 ± 0.14 nS/pF, n=20) DRG neurons. (**J**) Half-maximal voltage of activation ($\alpha 2\delta 3^{+/+}$: V1/2peak -15.16 ± 0.71 mV and V1/2sus -14.82 ± 0.62 mV, n=20; $\alpha 2\delta 3^{-/-}$: V1/2peak -16.00 ± 0.93 mV and V1/2sus -15.05 ± 0.87 mV, n=20) and (**K**) the associated slope ($\alpha 2\delta 3^{+/+}$: Speak 5.20 ± 0.43 mV and Ssus 6.35 ± 0.44 mV, N=20; $\alpha 2\delta 3^{-/-}$: Speak 5.30 ± 0.43 mV and Ssus 6.81 ± 0.59 mV, n=20).

**Figure 12. Region specific fMRI activity.**

Region-specific quantification of (**A**) peak height and (**B**) activated volume for brain structures of the left and right hemisphere after a thermal pain stimulus (55°C) in $\alpha 2\delta 3^{+/+}$ (n=20) and $\alpha 2\delta 3^{-/-}$ (n=18) mice. MT: medial thalamus; VPL: ventral postolateral/posteromedial thalamic nucleus; S1: primary and S2: secondary somato-sensory regions; Cg: cingulate cortex; Amd: amygdala: HT: hypothalamus; M: motor cortex. % BOLD change indicates increased BOLD signals compared to baseline. Activated volumes are expressed in voxels. All data are presented as mean values ± sem. *$p$ < 0.05; **$p$ < 0.01 (Student's t-test comparing control and $\alpha 2\delta 3^{-/-}$ groups).

**Figure 13. Cross-correlation matrix of the somatosensory pain matrix and negative BOLD fMRI.**

(**A**) Cross-correlation matrix of time profiles of the structures of the somato-sensory pain matrix. Whereas the pain signal spreads from the thalamus to other higher order pain centres in $\alpha 2\delta 3^{+/+}$ mice (left-left: lower left, right-right: upper right; red areas), in $\alpha 2\delta 3^{-/-}$ mice correlated activation can be only observed up to the level of the thalamus. Moreover, much less correlated interhemispheric activity at the left right interac-interaction matrices (left-right: lower right) was observed in the $\alpha 2\delta 3^{-/-}$ mice. Data from the left side and right sides of the brain are shown for the average across $\alpha 2\delta 3^{+/+}$ (n=20) and $\alpha 2\delta 3^{/-}$ mice (n=18) following challenge with noxious heat (55°C) at the right hind paw. SI: sensory input; Th: thalamus; SC: sensory cortex; AC: association cortex; LL: link to limbic system; LS: limbic system; HT: hypothalamus; BG: basal ganglia; C, cerebellum; M; motor cortex; P: periaquaeductal gray. Correlation-coefficients (cc) are given in the range from 0 (green) to +1 (red). (**B**). Negatively correlated BOLD fMRI signals were found in the cingulate (Cg), motor cortex (M), and primary somato-sensory cortex (S1) exclusively in $\alpha 2\delta 3^{-/-}$ mice (right panels, n=18)) following challenge with a thermal pain stimulus (55°C). n = 20 for $\alpha 2\delta 3^{+/+}$ control mice. The yellow lines in the left brain image indicate the brain slices 1 and 2. The size of a voxel is indicated.

**Figure 14. BOLD fMRI signals in the entire mouse brain.**

Total BOLD fMRI activation for thermal pain stimulus (45°C, 50°C, 55°C): peak heights **(A)** and activated volumes **(B)** for $\alpha 2\delta 3^{+/+}$ (n = 20) and $\alpha 2\delta 3^{-/-}$ mice (n= 18). Note that at all temperatures and parameters measured, no significant differences were observed. For vibrissal stimulation significant stronger BOLD activity **(C)** and larger activation volume **(D)** were observed for $\alpha 2\delta 3^{-/-}$ (n=5) as compared to control $\alpha 2\delta 3^{+/+}$ (n=7) mice. All data are presented as mean values ± sem. **$p$ < 0.01 (Student's t-test comparing control and $\alpha 2\delta 3^{-/-}$ groups).

**Figure 15. Conserved regulators of thermal nociception.**

GO enrichment analysis for human **(A)** and mouse **(B)** orthologs of *Drosophila* thermal nociception hits. GO terms were pooled into functional categories for data representation. Numbers indicate the counts of GO terms included in each functional category. These GO terms are grouped according to their parent terms - cellular components (CC), biological processes (BP), and molecular functions (MF). **(C)** Global C2 data set for mammalian orthologs. Functional classification of C2 gene sets (MsigDb, Broad institute) found enriched in mouse and human orthologs of our primary fly candidate thermal nociception genes and their first degree binding partners. The numbers indicate statistical-statistically significant C2 genes sets grouped into each functional category.

**Figure 16. A global network map of thermal nociception.**

The systems network includes data from the significantly enriched *Drosophila* KEGG pathways and GO processes, mouse and human KEGG pathways and C2 gene sets. Pathways, processes and gene sets that share a role in a biological process were pooled into functional classes while the underlying genes that constitute them are depicted with a connection to their respective functional class. Functional classes (brown), genes representing direct hits with a thermal nociception phenotype (red), their first degree binding partners (green) and developmental lethal genes (blue) represent the nodes in the network. Only select KEGG pathways, biological processes and C2 gene sets are used to build systems map.

**Figure 17. PI3Kγ controls thermal nociception and capsaicin responses *in vivo.***

**(A)** Schematic representation of the human PI3KCG gene locus on chromosome 7qp22.3. The positions of the SNPs assayed are indicated. Blue boxes represent exons. The relative gene position is given in megabases (Mb). Normal and chronic refer to the healthy volunteers and chronic back pain patient cohorts, respectively. Wind up and heat tolerance are indicted for healthy volunteers. Significant values (dominant inheritance; t-test) are indicated. **(B)** Healthy volunteers carrying the G allele at SNP rs757902 were significantly more sensitive to heat wind-up induced sensitivity relative to the A/A genotype. **(C)** Of 160 lumbar chronic root pain patients 1 year post discectomy those carrying the G allele at rs757902 exhibited increased pain. Data in B and C are presented as mean values ± sem and numbers of carriers of the respective alleles are indicated. **(D)** Wild type (WT) and *PI3K*g$^{-/-}$ (KO) littermates show enhanced thermal pain thresholds in response to radiant heat (Hargreaves; n=22 for WT and n = 27 for KO mice). **(E)** *PI3Kg* KO mice also show enhanced thermal sensitivity in the hot plate assay (n=13 for WT; and n = 9 for KO mice) and **(F)** an exaggerated capsaicin-evoked behavioral response *in vivo* (n = 9 for WT and n = 13 for KO mice). **(G)** Thermal nociception in *PI3Kg*$^{-/-}$ (KO) mice recon-reconstituted with wild type (WT->KO) or *PI3K*g$^{-/-}$ (KO->KO) bone marrow. WT mice reconstituted with WT bone marrow are shown as controls. Hot plate assay; 54°C. n > 6 for each group. **(H)** Thermal nociception thresholds in response to radiant heat (Hargreaves test) in littermate control and PI3Kγ kinase-dead (KD) knock-in mice still exhibit enhanced baseline thermal nociception thresholds (n = 19 for WT and n = 23 for KD mice). Data are presented as mean +/- sem. **p* < 0.05; ***p* < 0.01 (t-test).

**Figure 18. PI3Kγ acts in DRG as a negative regulator of thermal nociception and capsaicin responses.**

**(A)** Representative temperature response ramps and Arrhenius plots for heat-activated currents measured in single DRG neurons isolated from WT and PI3Kγ KO mice. For temperature response ramps, red lines depict temperature ramp and black lines depict inward current. **(B)** Q10 as a measure of the rate of inward current changes in response to temperature. n = 37 for isolated WT and n = 29 for PI3Kγ KO DRG neurons. **(C,D)** Capsaicin sensitivity of DRG neurons isolated from WT and PI3Kγ KO mice. **(C)** Shows representative capsaicin responses from a single neuron. (**D**) Dose-response curves to capsaicin. The capsaicin EC50 is indicated. Numbers indicate numbers of single neurons tested with the indicated capsaicin doses at the respective data points. Electrophysiology data was generated by single neuron patch clamping. Data are presented as mean +/- sem. **p < 0.01, ***p < 0.001 by Mann-Whitney u-test.

**Figure 19. Flow charts for global bioinformatics analyses. (A)** Flow chart indicating the steps in the conversion of *Drosophila* candidate thermal nociception genes into mouse and human orthologs. **(B)** Flow chart indicating KEGG and C2 gene set analysis in *Drosophila,* mice, and humans. Data from all three organisms were pooled to generate a global network map. See Materials and Methods for details.

**Figure 20. A global network map of thermal nociception based on primary hits without binding partners.**

The systems network includes data from the significantly enriched *Drosophila* KEGG pathways and GO processes, mouse and human KEGG pathways and C2 gene sets based on analysis of primary screening hits without binding partners. Pathways, processes and gene sets that share a role in a biological process were pooled into functional classes while the underlying genes that constitute them are depicted with a connection to their respective functional class. Functional classes (brown), genes representing hits with pain phenotype (red), and developmental lethal genes (blue) are shown in the network.

**Figure 21. Basic behavioral analysis of PI3Kγ KO mice.**

PI3Kγ expressing control and PI3Kg knock-out (KO) mice exhibit similar behavioral responses in multiple paradigms. **(A)** PI3Kγ KO mice exhibit intact accuracy in a test for skilled reaching and **(B)** a slight decrease in cages crosses over a 24 hour period; but this trend failed to reach statistical significance. **(C)** In an open field test, control and PI3Kγ KO mice showed the same activity over a ten minute period and **(D)** similar levels of anxiety-related defecation. **(E)** Control and PI3Kγ KO mice exhibited similar coordination in the rotorod test. **(F-H)** No differences were detected between control and PI3Kγ KO mice in three models of learning: **(F)** Water maze, **(G)** T-maze, and **(H)** passive avoidance learning. (n=11 for control and n=12 for KO for the above tests). **(I-J)** Basal mechanical sensation was assessed by the von Frey test; PI3Kγ KO mice exhibited normal **(I)** force threshold and **(J)** latency. n=21 for WT and n=16 for KO mice. **(K)** PI3Kγ KO mice also exhibited a comparable sensitivity to acetone (n=21 for WT and n=16 for KO mice). Data are presented as mean +/- sem. In all experiments there we no significant differences (t-test).

**Figure 22. PI3Kγ KO mice show no difference in inflammatory pain responses.**

**(A)** PI3Kγ KO mice exhibit significant enhancement of baseline thermal nociception sensitivity but a comparable degree of thermal hyperalgesia following intraplantar CFA challenge. **(B)** A sensitization ratio (Baseline/CFA latency) shows similar CFA-induced sensitization in wild type (WT) and PI3Kγ KO mice 10 days after CFA injection. **(C)** WT and PI3Kγ KO mice exhibit similar paw swelling in response to CFA over the course of the experiment (data shown was recorded at day 8). Data are present-presented as mean +/- sem. *P < 0.05, **P < 0.01 by Student's t test.

**Figure 23. Testing of anti-pain compounds**

A: Compounds testing performed on pregabalin, cilomilast, zardaverine and roflumilast as described in Example 4.1.3.2. Data are presented as mean values ± SEM, n=8-10. Significantly different by Mann-Whitney U test compared to T=0 paw withdrawal thresholds (*p<0.05, **p<0.01, ***p<0.001).

B: Compounds testing performed on pregabalin, bosutinib and adefovir as described in Example 4.1.3.3. Data are presented as mean values ± SEM, n=8-10. Significantly different by Mann-Whitney U test compared to T=0 paw withdrawal thresholds (* p<0.05, **p<0.01, ***p<0.001).

C: Compounds testing performed on pregabalin, dasatinib and tenofovir as described in Example 4.1.3.1. Data are presented as mean values ± SEM, n=8-10. Significantly different by Mann-Whitney U test compared to T=0 paw withdrawal thresholds (* p<0.05, **p<0.01, ***p<0.001).

**Figure 24: Testing of anti-pain compounds, chronic inflammatory pain model**

A: Von Frey's hairs assay for Indomethiacin, Dasatinib, Tenofovir. Data are presented as mean values ± SEM, n=6-10. Significantly different by Mann-Whitney U test. *p<0.05, ** p<0.01, ***p<0.001 significantly different from post surgery withdrawal thresholds.

B: Von Frey's hairs assay for Indomethiacin, Cimilast, Zardaverine, Roflumilast. Data are presented as mean values ± SEM, n=8-10. Significantly different by Mann-Whitney U test. * p<0.05, **p<0.01, ***p<0.001 significantly different from post surgery withdrawal thresholds.

C: Von Frey's hairs assay for Indomethiacin, Bosutinib, Adefovir. Data are presented as mean values ± SEM, n=8-10. Significantly different by Mann-Whitney U test. *p<0.05, ** p<0.01, ***p<0.001 significantly different from post surgery withdrawal thresholds.

***Examples:***

**Example 1: Materials and Methods**

**Example 1.1:Fly stocks**

All *UAS-IR* transgenic fly lines were obtained from the VDRC RNAi library (Dietzl et al., 2007) with the exception of α2δ second hairpins, which were obtained from the Harvard trip stocks. elav with *UAS-Dicer* 2 was a gift from B. Dickson (Dietzl et al., 2007). *Painless*(EP(2)2451) was a gift from D. Tracey. *Df(2R)Exel712* was obtained from the Exelexis stock collection (Thibault et al., 2004). *NP1574-Gal4* was obtained from the Kyoto Institute of Technology Drosophila Genetic Resource Center (Hayashi et al., 2002). The point mutant stj lines $stj^1$ and $stj^2$, *the stj* deficiency *Df (2R)Exel7128,* and the *stj* P[acman] rescue ($stj^+$; 1259) flies have been previously described (Ly et al., 2008). These flies were generated by introducing a BAC containing the wild type *stj* locus into predetermined attP sites in the genome using ΦC31 integrase (Venken et al., 2009). *UAS-Lamin:GFP* was a gift from N. Stuurman. *UAS-shibire*$^{ts1}$ and *UAS-CD8:GFP* were obtained from Bloomington.

***Example 1.2:Drosophila*** **behavioral tests**

For adult thermal preference and avoidance of noxious heat ~20 four day old flies were placed in an experimental chamber (Nunclon dish 35mm x 10mm) sealed with scotch tape. All tests were performed in the dark. The bottom of the chamber was heated to 46°C over 15 seconds by floating on a water bath while the subnoxious zone was measured to be 31°C at the end of the 4 minute experiment. % Avoidance was calculated by counting the number of flies that failed

to avoid the noxious temperature compared to the total number of flies in the chamber. For experiments involving *UAS-shibire$^{ts1}$,* flies were transferred to the experimental chambers followed by a temperature shift to 30°C for 60 minutes. Larval pain assays were performed as described (Tracey et al., 2003). Since 3rd instar *stj$^2$* mutant larvae exhibit motor defects, we only used 1st and 2nd instar *stj$^2$* larvae and stage-matched control larvae for our experiments. Mechanosensation (Kernan score) was performed as described (Kernan et al., 1994). To set up the experimental assay and to determine a cut-off, where we would always hit *painless,* multiple control (F1 from *w1118* ♂ X virgin *elav-Gal4; UAS-DCR2* ♀) and *painless* mutant flies were tested for thermal avoidance and an avoidance histogram generated. For assessing noxious temperature-induced paralysis, wild type flies were placed in 5 ml polystyrene round bottom tubes (BD Falcon) and exposed to different temperatures (37-46°C with 1° increments) until 100% of flies were paralyzed. Basic coordination was assessed by tapping the test chamber on the bench and observing general coordination as flies move away from the site of impact as described previously (Dietzl et al., 2007).

### Example 1.3:Screening procedure

For screening, transgenic males harboring a *UAS-IR* transgene were crossed with *elav-Gal-4; DCR2-Gal4* females and F1 progeny were tested for avoidance of noxious heat (46°C) in our experimental chambers. In our pilot studies, a noxious temperature of 46°C was found to give the most robust differences between control and *painless* mutant flies. Of note, 46°C is the same temperature used to identify *painless* in larvae (Tracey et al., 2003). For the screening we chose a 4 minute cut-off, based on the difference between control and *pain* mutant flies. For each fly line, ~ 20 adult flies were transferred to the experimental chambers. During retesting flies were also scored for basic coordination before testing by tapping the flies to the bottom of the test chamber after loading and assessing whether flies were slow or uncoordinated when climbing back up the wall of the chamber. For noxious heat avoidance, flies were given a choice between the noxious and non-noxious temperature and after 4 minutes, the number of flies successfully avoiding the noxious heat and the number in the noxious heat zone (which were incapacitated by assay end) were recorded to calculate percent avoidance ((total-failed)/total)X100) for each test. The Z-score was generated as compared to wild type responses (*w$^{1118}$* x *elav; DCR2*). A Z-score was generated as follows: ((mean control avoidance - mean test avoidance)/standard deviation control). Flies failing to avoid the noxious temperature in the first round were confirmed with multiple retests. All *elav-Gal4>UAS-IR* lines with a Z-score >1.65 in the primary screen were tested an average of 6.78 independent times using second *UAS-IR* transformants. Based on previous use of this RNAi library (Dietzl et al., 2007; Mummery-Widmer et al., 2009) the false positive rate is estimated at 2-7%. To decrease the number of false positives we excluded 55 hairpins with potential off-target effects (more than 6 Can repeats and/or S19 score < 0.8 was considered unspecific), leaving 622 transgenic lines corresponding to 580 candidate nociception genes (Fig. 1E).

During hit confirmation for our screen, flies were also assessed for general coordination defects (Dietzl et al., 2007) that could potentially impede noxious thermal avoidance. In addition, to further test whether our hits had a defect that is specific to noxious thermal avoidance and/or exhibited additional alterations in innate behavioral programs (Benzer, 1967), we evaluated fly lines for general coordination by geotactic repulsion and phototactic attraction. To determine if our candidate thermal nociception hits were unable to avoid noxious heat due to increased temperature-sensitivity we assayed temperature-induced paralysis at 38°C, a system that was originally used to identity classic temperature-sensitive paralytic mutants such as *shibire* and *para* (Grigliatti et al., 1973; Siddiqi and Benzer, 1976). No significant correlations were observed between thermal nociception genes and phototactic attraction ($r^2$ = 0.0026), geotactic avoidance ($r^2$ = 0.00165), or temperature sensitivity ($r^2$ = 0,005742). Finally, to determine if our candidate pain genes are simply the result of increased temperature-induced paralysis at 46°C, we exposed pain hits to a chamber set to 46°C and recorded the kinetics of temperature-induced paralysis.

### Example 1.4:Functional annotation of pain hits

Of the 580 genes corresponding to the RNAi hits with thermal nociception phenotypes, 391 genes were annotated by GOstat. To find additional information about gene-function of the remaining 189 genes, we searched three other databases for functional information associated with the *Drosophila* genes and an additional 80 genes were functionally annotated using the following tools:

1. Panther db (http://www.pantherdb.org/).
2. Overlap with neuronal precursor genes published by Brody T., et al. (Brody et al., 2002).
3. NCBI Gene (Gene Ontology) (http://www.ncbi.nlm.nih.gov/gene).

### Example 1.5:Identification of fly orthologs in mouse and human.

To identify orthologs between *Drosophila* and mouse or *Drosophila* and human, we used pre-computed orthology

predictions obtained from Compara r49, Homologene (03/08), Inparanoid v6.1, Orthomcl v2 (Kuzniar, et al., Trends Genet 24, 539 (Nov, 2008). Since each database query outputs results using a different gene identifier, we transformed all the different identifiers to a single unique Entrez ID, for each gene. Both one-to-one, and many-to-many mappings were observed between the *Drosophila* and the mammalian genes. In cases where on *Drosophila* gene mapped to multiple mammalian orthologs, all the predicted orthologs of a given gene, were considered for further downstream analysis. In cases where a mammalian gene of interest had multiple *Drosophila* orthologs, the fly gene with highest Z-score for the corresponding RNAi hit was mapped.

**Example 1.6: GO classification**

Gene Ontology (GO) analysis was performed using GOstat (http://gostat.wehi.edu.au/). GO analysis was run with *Drosophila* genes whose RNAi hits have a Z-score >1.65. Over- or under-represented (p<0.1) GO terms with corrections for multiple testing (Benjamini - Yekutieli correction) were identified and compared to all *Drosophila* genes reported in the flybase (fb). Since terms that occur at a deeper level in the GO tree hierarchy, therefore containing lesser numbers of genes, are considered more biologically informative, we discarded terms containing more than 500 genes from further analysis. Significant GO terms were manually pooled and organized into "functional groups" based on their shared roles in a biological function.

**Example 1.7: Binding partner and pathway analyses.** Pathway analysis was performed using the *Drosophila* Pathway database in GeneSpring GX. Briefly, the Pathway database in GeneSpring GX contains binding partners from two sources: 1. those reported in open-source public databases like BIND and IntAct (IMEX consor-consortium) and 2. extracted from Pubmed abstracts using a proprietary natural language processing technique. The tool UI allows a query of the database to build networks of molecular relations (edges) amongst molecules (nodes) of interest. Data from the IMEX consortium only represent protein binding and promoter binding molecular interactions. Using filters in GeneSpring GX, we selected only binding molecular relations reported by the IMEX consortium to create this network, thereby including only experimentally proved physical interactions between the corresponding protein molecules.

**Example 1.8: Hypergeometric enrichment test.** A hypergeometric test, similar to the test used for GO enrichment analysis, was used to identify over-represented gene lists (C2 from Msigdb, BROAD Institute) and pathways (KEGG) amongst the pain hits. The hypergeometric test considers only the percentage representation of genes corresponding to a biological pathway in the pre-computed pain gene list. This analysis was performed on the gene list identified as mouse or human orthologs corresponding to adult pain hits (Z-score > 1.65) in *Drosophila.*

**Example 1.9: Generation of a systems map.** For the combined systems map, significant KEGG pathways, C2 gene sets and selected GO functional categories were manually grouped into uniform functional categories. Functional categories chosen for depiction were selected relevant to the biology of pain function. The complete list of functional categories is available in tables S4 and S5. Of these, 37 functional categories were chosen for construction of the systems map. For each functional category, the corresponding genes that mapped to the KEGG pathways, C2 gene sets and GO term, included in the category were extracted. *Drosophila* orthologs for these genes were found and assigned to the category and visually represented into a systems map. For the construction of *Drosophila* KEGG pathway systems map, pathways rendered over 90% enriched by GSA analysis, were manually annotated into functional categories. Pain hit genes and their binding partners that belong to any pathway were extracted and assigned to the appropriate higher level functional category. Data was represented as a systems map connecting these functional categories.

**Example 1.10:Gene set analysis (GSA)**

GSA uses lists of genes from biological pathways or processes, to find if the pathway or the process as a whole has been significantly altered by the experimental treatment. A null hypothesis is that "genes of a functionally irrelevant pathway are not clustered at the top of a rank ordered (based on Z-score) list of all genes in the experiment". The rank order list of 11293 unique genes with Z-scores was obtained from our screen assaying a total of 16051 UAS-IR transformants. Individual gene lists were constructed corresponding to 146 Drosophila KEGG pathways and 45 significant GO terms (corrected p<0.1). Binding partners were identified in yeast-2-hybrid screens reported in the biomolecular interaction network database BIND, i.e. binding partners experimentally confirmed to interact with the candidate thermal nociception genes. An enrichment score (Es) for a gene list (s) was calculated based upon the pain Z-score using the formula:

$$Es = \frac{\text{sum of average Z-scores of all genes in s}}{\sqrt{\text{(Number of genes in s)}}}$$

For every s, 100 bootstrap permutations were carried out to generate random functionally unrelated gene lists

of the same size and their enrichment scores calculated as Er. A gene set s is considered significant ($p < 0.1$) if Es > Er (90th quantile). This GSA approach removes any bias artificially created by the ad-hoc Z-score cut-off >1.65.

**Example 1.11:Imaging of nuclei and axonal projections in *Drosophila***

Brains and ventral nerve cords from *stj-Gal4>UAS-Lamin:GFP* and *stj-Gal4>UAS-CD8:GFP* fly lines were dissected in PBS, fixed in 4% paraformaldehyde in PBS for 30 min at room temperature (RT), washed three times for 10 min in PBS containing 0.3%Triton X-100, blocked for 1 hr at RT in PBT containing 5% normal goat serum, and incubated with primary anti-GFP (Invitrogen) and NC82 (Iowa hybridoma bank) counterstaining antibodies in blocking solution overnight at 4 C. Samples were washed three times for 10 min in PBT at RT, and secondary antibodies were applied in blocking solution for 2 hrs at RT. After washing three times for 10 min in PBS, samples were mounted in Vectashield (Vector Labs). Alternatively, to stain for STJ, adult brains were dissected in Grace's Insect Medium, fixed in 4% formaldehyde in PBS with 1% Triton X-100 (PBS/Tx), rinsed 2 times in 0.3% PBS/Tx and washed 2 times in 0.5% PBS/Tx for 15 min. Samples were blocked overnight at 4 C in 5% NGS with 0.1% PBS/Tx and the primary antibodies were incubated 3 days at 4 C in block solution. Samples were washed as was done after fixation and incubated with the secondary antibodies at 4 C for 2 days. Then samples were washed as before with the final wash step in PBS before being transferred to 50% PBS/glycerol and then mounted. Antibodies against STJ were raised in either rabbit (HH129) or guinea pig (125 or 127). Confocal images were captured on a Zeiss LSM510 Meta, Axiovert 200 M, and processed with LSM510 Image Examiner. For larval imaging, *stj-GAL4* was crossed to UAS-mCD8:GFP. Larvae raised at 22°C were dissected in modified HL3 solution (110 mM NaCl, 5 mM KCl, 10 mM $NaHCO_3$, 5 mM HEPES, 30 mM sucrose, 5 mM Trehalose, and 10 mM $MgCl_2$) (pH, 7.2) and fixed in 3.7% formaldehyde. Stainings were performed using standard protocols described (Verstreken et al., 2003). Images were captured using a Zeiss 510 confocal microscope (Carl Zeiss, Inc.) using a Plan Neofluar 40x, 1.3 N.A. objective and processed with LSM Image Browser 4.2 (Carl Zeiss, Inc.). Primary antibodies to GFP (rabbit, chicken, Abcam used 1:500) and FUTSCH were diluted 1:200. STJ antibodies were all diluted to 1:100. Secondary antibodies tagged with Alexa 405, Alexa 488, Alexa 568 (Invitrogen) or Cy3 were used at 1:200. In situ hybridizations on adult flies were performed as previously described (Couto et al., 2005).

**Example 1.12:Generation of α*2*δ*3* knock-out mice**

For gene targeting of α2δ3 in mice, a targeting vector was inserted into exon 15 of the murine α2δ3 gene. The linearized construct was electroporated into embryonic stem (ES) cells derived from the 129/OlaHsd mouse sub-strain. Correctly targeted ES cell clones were confirmed by Southern blotting and used to generate chimeric mice. Germline transmitted F1 mice were backcrossed to C57BL/6 females. All behavioral and fMRI studies were conducted in accordance with guidelines of the European Union Council (86/609/EU) and following Austrian regulations for the use of laboratory animals.

**Example 1.13:Physical examinations**

A physical examination of α*2*δ*3* mutant and control mice to assess general characteristics such as activity, behavior towards siblings, posture, grooming, breathing patterns and movement, abnormal discharge, malformations was performed. A step-wise necropsy exam was then performed to identify potential lesions and abnormalities in all organs. Harvested organs were fixed, paraffin-embedded, sectioned, stained using hematoxylin and eosin, and then assessed for possible histopathologies.

**Example 1.14:Serum chemistry and hematology**

Blood samples were collected by terminal cardiac puncture and serum was assessed for electrolytes, inorganic ions, lipid profile, glucose and creatine kinase, and basic markers of renal and kidney functions. Serum data were assayed in a Hitachi 912 automatic analyzer. Whole blood smears were stained with Wright's Stain in order to perform a differential leukocyte count.

**Example 1.15:Behavioral experiments**

For the hot plate assay, age and sex-matched wild type and α2δ3 mutant mice were acclimated to the hot plate apparatus (Ugo Basile, Comerio, Italy) and then tested for hot plate latency at 50-56°C. Jumping, biting, licking, and clutching of hind paws was considered a nociceptive response as described previously (Jourdan et al., 2001). For the tail-flick test, a light beam was focused onto the tip of the tail, and the latency to tail withdrawal was taken as a measure of the nociceptive threshold to radiant heat. The mechanical pain test was performed by applying an ascending series

of noxious von Frey hairs to the dorsal surface of each hindpaw until a hindlimb withdrawal response was observed (Whishaw et al., 1999). Inflammatory thermal hyperalgesia was produced in the mouse right hind paw by intraplantar injection of Complete Freund's Adjuvant (CFA) (20 μl of a solution containing 5 mg of CFA in 10 ml of a 1:1 emulsion of saline and mineral oil). Before and after CFA injection, nociceptive responses to heat were measured using the hot-plate test (54°C). To evaluate the inflammatory response elicited by CFA, paw swelling was measured using a spring loaded caliper (Mitutoyo, Japan). The rotarod test was used to evaluate basic coordination and balance. Mice were placed on a smooth rod that acts as a rotating treadmill (AccuScan Instruments, Columbus, Ohio). The rotarod rotates slowly at first and then progressively increases in speed. Mice were tested three times and the fall speed was recorded. Moreover, mice were evaluated for their response to a novel environment using an open field test (MED Associates, St. Albans, Vermont). Briefly, each animal was placed in the center of its assigned chamber. Activity of individual mice was monitored by photo-beam breaks and recorded for the ten minute test session. Mice were assessed for a "depression" phenotype using the tail suspension assay (MED Associates, St. Albans, Vermont). Each mouse was suspended from a metal hanger such that the end of the hanger is 1/8$^{th}$ of an inch or less from the base of the tail. Total times of immobility were recorded during a 6 minute period.

**Example 1.16: Further mouse behavioral tes**ts

PI3Kγ knock out (T. Sasaki et al., Science 287, 1040 (Feb 11, 2000) and kinase dead knock in (E. Patrucco et al., Cell 118, 375 (Aug 6, 2004)) mice are described.

Baseline thermal and mechanical sensitivities were assessed using the Hargreaves and von Frey test (Ugo Basile Biological Research Apparatus Co., Comerio, Varese - Italy) following acclimation with the test apparatus. For paw withdrawal latencies, responses were determined by testing left and right paws. The hot plate test was done using a microprocessor-controlled unit (Ugo Basile). For initial phenotyping, WT and PI3Kγ KO littermates were used. For subsequent tests, PI3Kγ KO mice were backcrossed >8 times to C57B6 mice and compared to sex and age matched wild type C57B6 controls. Inflammatory pain was induced by intraplantar injection of Complete Freund's Adjuvant (CFA) (20 μl of a solution containing 5 mg of CFA in 10 ml of a 1:1 emulsion of saline and mineral oil) and behavior was tested on a hot plate as above. Paw swelling indicative of inflammation was evaluated by use of a spring loaded caliper (Mitutoyo). Capsaicin behavior was assessed by time spent licking over 5 minutes following intraplantar injection of capsaicin (3 μg in 10 μl; dissolved in 5% ethanol, 5% Tween-80 and 90% saline; Sigma). For acetone-induced cold pain, a drop (50 μL) of acetone was placed against the centre of the plantar surface of the hindpaw and responses were recorded (I. Racz et al., J Neurosci 28, 12125 (Nov 12, 2008*).* Other behavioral test were as described previously. Briefly, for skilled reaching task, each mouse was placed on a restricted diet and conditioned in the reaching box for 5 days. On the 6$^{th}$ day mice were placed in the reaching box and reaching was scored for 5 minutes beginning with the first attempt. For circadian activity, mice were individually housed in cages with photosensors. The mice were monitored for 24 hours with a 12/12 light dark cycle and the number of cage crosses was recorded. For open field activity, mice were individually housed in clear plastic cages with infrared sensors and left for 10 minutes in conditions of low noise and dim light. Total horizontal activity was tracked and fecal pellets were counted at the end of the test. For rotorod, mice were trained for 3 days at very low speed and then tested while gradually increasing rotation speed. Mean latency on rod was recorded. For water maze, a 1.2 diameter milk pool was used with a 14 X 14 cm stationary platform hidden 7 mm beneath the liquid surface. Mice were placed in the pool in randomized quadrants and given 60 seconds to locate the safe platform. At the end of each trial mice that failed to locate the platform were placed on it for 10 seconds. After 14 trials over 7 days, the platform was moved to a new quadrant. Mean latency to find the platform is presented. For T-maze, mice were placed in a T shaped maze with one arm of the maze blocked off. On the second day the blocked arm was opened, and time spent in the new area was recorded. For passive avoidance, mice were placed in a clear plastic box with metal bars wired to distribute 0.12 milliamps built into the base. A black plastic platform was fixed in the center of the box. Mice were placed in the black "safe" platform, and when they step from the platform they were given a 1 second shock, and then removed from the apparatus. This process was repeated until mice no longer step from the safe platform.

**Example 1.17:Bone marrow transplantations.** Six to eight week-old recipient mice underwent a lethal total-body irradiation (1000 Rad). Freshly isolated donor bone marrow cells were then injected into syngenic recipient mice (5 x $10^6$ cells per mouse) 24 hours after irradiation. PCR analyses of blood cells and tail DNA indicated that ~ 95% of blood circulating leukocytes were of donor origin.

**Example 1.18:Western blotting**

Electrophoresis and Western blotting were performed using the Invitrogen Novex Mini-cell system as per the manufacture's instruction. Membranes were blocked with 5% milk powder in PBS. Primary antibodies were diluted in PBS/Tween/BSA. The following primary antibodies were used: mouse anti-Actin, dilution 1/1000 (Sigma); goat anti-α2δ1, dilution 1/500, (Everest Biotech Oxfordshire, UK), and rabbit anti-α2δ3, dilution 1/500. To generate the anti-α2δ3

antibody, rabbits were immunized with the peptide VSERTIKETTGNIAC conjugated to KLH. Serum was affinity purified against the immobilized peptide. Secondary antibodies were used at a dilution of 1 in 5000 (Promega, Madison, Wisconsin).

**Example 1.19:LacZ expression**

Since our $\alpha2\delta3$ knock-out mice carry a LacZ reporter, we used $\beta$-Gal staining as a marker to assess $\alpha2\delta3$ expression. Tissues from 7-12 week old heterozygote mice were analysed for LacZ expression. Organs from these mice were frozen, sectioned (10 $\mu$m) and analysed for LacZ expression using X-Gal staining followed by Nuclear Fast Red counterstaining. For whole mount brain staining, the brain was cut longitudinally, fixed, and stained using X-gal. The reaction was stopped with a PBS wash and then fixed with buffered formaldehyde. In addition to staining in the brain (Fig. 6A), moderate LacZ staining was detectable in some spermatogenic cells of the seminiferous tubules. LacZ expression was not detected in the sciatic nerve, eyes, Harderian glands, thymus, spleen, lymph nodes, bone marrow, aorta, heart, lung, liver, gallbladder, pancreas, kidney, urinary bladder, trachea, larynx, esophagus, thyroid gland, parathyroid gland, pituitary gland, adrenal glands, salivary glands, tongue, skeletal muscle, skin and female reproductive system.

**Example 1.20:DRG neuron cultures**

Lumbar dorsal root ganglia (DRG) with the cell bodies of primary afferents that project into the hind paw were harvested from adult C57BL/6J mice, treated enzymatically with Liberase Blendzymel (Roche) and trypsin (Invitrogen), and dissociated mechanically with a fire-polished Pasteur pipette as previously published (Obreja et al., 2002; Obreja et al., 2005). The resulting cell suspension was washed, plated on glass coverslips coated with poly-L-lysine/laminin (Sigma) and cultivated in synthetic serum-free medium (supplemented TNB™, Biochrom, Vienna) at 37°C in 5% CO2.

**Example 1.21:Patch clamp recordings**

Using the whole-cell voltage-clamp configuration of the patch-clamp technique, ionic currents and membrane potentials were recorded from isolated neurons after 16-24 hours at room temperature as previously published (Obreja et al., 2005; Obreja et al., 2002). Currents and the membrane potential recorded using an EPC10 (HEKA, Germany) and the Pulse v8.74 software (HEKA). Borosilicate glass micropipettes (Science Products, Hofheim, Germany) pulled with a horizontal puller (Sutter Instruments Company, Novato, CA, USA). For calcium current recordings the extracellular solution was composed of (mM); 130 NMDG, 20 TEACl, 5 CsCl, 2 $CaCl_2$, 1 $MgCl_2$ (Sigma), 10 HEPES and 20 Glucose (Merck) and adjusted to pH 7.3 with HCl (Merck). The intracellular solution contained 120 CsCl, 20 TEACl, 10 EGTA, 2 MgATP (Sigma), 10 HEPES and 20 Sucrose (Merck) adjusted to pH 7.3 with CsOH (Merck). Calcium currents were evoked by test potentials from -80 to 60 mV in 10mV steps from a holding potential of -80mV and digitized at 50 kHz. All recorded traces were corrected for leakage and capacitive currents using a P/5 protocol.

The individual recorded Calcium IV-plots were fitted with a modified Boltzmann equation with a high-voltage block. The conductance (*G*) is dependent on the amount and subtypes of $Ca^{2+}$ channels that are present in the cell. The activation is determined by the half-maximal activation of the $Ca^{2+}$ current (*Vh*) and the e-fold change around Vh (*S*) in the IV. Voltage-dependent block of the $Ca^{2+}$ current at high voltages is described by the maximal speed of the blockage (*Bs*) at the half-maximal voltage of the block (*Bh*). The reversal of the current

$$I = \left( \frac{G}{1 + exp\left( \frac{-V - Vh}{S} \right)^2} \right) * \left( \frac{1}{1 + exp\left( \frac{V - Bh}{Bs} \right)} \right) * \left(V - Vr\right) + A$$

is determined by the recersal potential (*Vr*). All data were analyzed in Origin 7.0 (OriginLab).

Alternatively, for PIK3CG, the following protocol was used: Using the whole-cell voltage-clamp configuration of the patch-clamp technique, ionic currents were recorded from isolated DRG neurons at -80 mV holding potential as previously published (Obreja et al., 2005; Obreja et al., 2002). The external solution (ECS) contained (in mM) 145 NaCl, 5 KCl, 2 $CaCl_2$, 1 $MgCl_2$ (all Sigma), 10 glucose and 10 HEPES (Merck), at pH 7.3 adjusted with NaOH. Borosilicate glass micropipettes (Science Products, Germany) were filled with internal solution (ICS) containing (in mM) 138 Caesium methanesulfonate, 2 $MgCl_2$, 2 $Na_2$-ATP, 0.2 Na-GTP, 0.5 $CaCl_2$, 5 EGTA (all Sigma) and 10 HEPES (Merck), at pH 7.3

adjusted with CsOH (Merck). After filling, electrode resistance was 3-5 MW. Currents were filtered at 2.9 kHz, sampled at 3 kHz and recorded using an EPC 9 and the Pulse v8.74 software (HEKA). Experiments were performed at room temperature and only one neuron was tested per Petri dish. An automated seven-barrel system with common outlet next to the cell under investigation (<100 μM) was used for fast drug administration and heat stimulation (Dittert et al., J Neurosci Methods 82, 195 (Aug 1, 1998). Capsaicin was applied at different concentrations (0.001, 0.01, 0.1, 0.5, 1.0, 5.0 and 10 μM; Sigma; 1 mM stock solution solved in ethanol) for 10 seconds each. Heat-activated inward currents ($I_{heat}$) were elicited by applying ramp-shaped heat stimuli at 120 s intervals with linear temperature increases from 25 to 50-55°C within 5 seconds. Current values were sampled at the rising phase of the temperature ramp, normalized to 24°C (bath temperature) and data represented as an Arrhenius plot (Vyklicky et al., J Physiol 517 (Pt 1), 181 (May 15, 1999). The temperature co-efficient $Q_{10}$ was used to characterize temperature dependence of the membrane. In the linear range, the activation energy $E_a$ was determined from the slope of the regression line (r>0.99) using the formula:

$$-E_a = 2.303R\log_{10}(I_2/I_1)/((1/T_2)-(1/T_1))$$

R gas contant (8.314 $JK^{-1}mol^{-1}$)
$I_1$ current at lower absolute temperature $T_1$
$I_2$ current at higher absolute temperature $T_2$

The $T_{threshold}$ was determined from the point of intersection of the two regression lines.

$Q_{10}$ was calculated using the equation: $Q_{10} = \exp(10E_a/(RT_1T_2))$.

**Example 1.22:fMRI and BOLD imaging**

Male mice were anesthetized with isoflurane and placed on a cradle inside the MR machine (Bruker BioSpec 47/40 (200mT/m) with a free bore of 40 cm, equipped with an actively RF-decoupled coil system and a quadratur head coil) under extensive physiological monitoring (respiration, temperature, heat function). The contact heat stimuli (40°C, 45°C, 50°C, and 55°C, plateau for 5 sec after 15 sec of heat increase) were applied at the right hind paw (presented at 3 min 25 s intervals, 3 time each temperature) using a custom made computer controlled Peltier heating device with no influence from and to the MR scanner. For tactile stimulation, the C1 vibrissa of the mice was moved with an air driven device integrated into cradle shifting the vibrissa by an inverted comb with an amplitude of 5 mm at 7 Hz. For the resting state measurement a series of 300 sets of functional images (matrix 64 x 64, field of view 15 x 15 mm, slice thickness 0.5 mm, axial, 22 slices) were collected using the Echo Planar Technique (EPI, single shot: TR = 2000 ms, TEef = 24,38 ms) within a 10 min recording period. Next a set of 750 functional EPI images where acquired with two times k-space averaging resulting in a TR of 4000 ms and a total measuring time of 50 min. Finally, 22 corresponding anatomical T2 reference images (field of view 15 x 15 mm, matrix 256 x 128, TR = 2000 ms, slice thickness 0.5 mm, TEef =56 ms, RARE) were assembled. Functional analysis was performed using Brain Voyager QX and our own software MagNan (Knabl et al., 2008). In brief, the following preprocessing was performed: Motion correction algorithm as implemented in BrainVoyager was applied (trilinear interpolation, motion detected always below 1 pixel). Slice time correction was performed with a Cubic Spline, followed by a high pass temporal filtering (9 cycles) and a 2D Gaussian smoothing of the data (FWHM kernel: 2 pixel, in-plane direction). Next a GLM analysis with separate predictors for each stimulation temperature was calculated. To detect significantly activated voxels statistical parametric maps (SPM) of these contrasts were generated for individual animals and FDR thresholded (q = 0.05, two sided). Different groups of significant activated voxels (n> 4) were labeled belonging to certain brain structures based on the mouse atlas from Paxinos (second edition). The corresponding peak activity was determined for each stimulation temperature based on the stimulus specific GLM predictors. The mean activity of each activated brain structure was averaged across all significant activated voxels and subjected to a statistical t-test comparing *α2δ3* mutant and control mice. A second level random effect analysis variance (ANOVA) was performed for Z-score maps between the different mice genotypes. Areas of significant group activation differences (corrected P < 0.05) were used as masks to only show the calculated peak activation maps in these regions (Fig. 7A).

To find brain areas which exhibit the most robust response differences between α2δ3 mutant and control mice, we performed a correlation based principal component analysis (PCA) of BOLD responses with temperature specific input vectors (activation probability, volume and peak height, time, symmetry, and width) per identified brain structure. We then calculated within the coordinate space of the first 3 principal components (88.85 % of total variance) the Euclidian distance for all predefined defined pain regions between α2δ3 mutant and control mice. Because the PCA vectors span a coordinate space optimized to cover the variance of the input vectors, large Euclidian distances indicate large differences in response properties between control and α2δ3 mutant mice for a given brain structure. Largest Euclidian distances were observed for the caudate putamen (left) and the primary somato-sensory cortex (left). Enlarged distances were

also found for the ventral tegmental area (VAT, left), the primary (right) and secondary (left) somatosensory cortex, the insula (right), entorhinal cortex (left), hippocampus (right) and motor cortex (left). The lowest Euclidian distances were found for the "sensory input" and thalamus, indicating highest similarity between control mice and α2δ3 mutants in heat-induced activation of these brain structures. Minimally 1 day after recovery from the functional experiments the mice obtained a single manganese injection (isotonic and neutral MnCl2 solutions: 0.4 mmol/kg, ip). 24h after the MnCl2 injection the DTI and MEMRI measurements were performed using the same scanner and coil system as mentioned previously. First we measured the DTI using an EPI SpinEcho approach (TEeff: 31.6, TR 5000 ms, Bandwith: 150 kHz, segments: 1, NEX: 2, 126 diffusion directions and maximal B-value of 600.00 s/m2 and total measuring time of 21min50s). Geometrical scan properties were matrix: 128x128, slices: 15 with 1.0 mm thickness, field of view: 18 x 18 mm. Fractional anisotropy maps were calculated using the Bruker PV 5.0LP3 software version and after segmentation and affine registration of the individual brains they were subjected to a voxelwise Student's t-test. For imaging of the T1 contrast of manganese uptake we used a modified driven equilibrium Fourier transform sequence (MDEFT, scan parameters: TR=15.00 ms, TE=5.1 ms, Bandwidth: 25kHz; FA=15°, inversion delay (ID)=1000 ms and four segments. The ID of the MDEFT preparation (responsible for the T1 contrast) was optimized for the contrast to noise ratio 24 h after MnCl2 administration. The geometric parameters of the 3D scans were: matrix: 256x256×64, field of view = 22×22×19.84 mm. 10 averages resulted in a total measuring time of 5h41min20s. Again the after segmentation and registration, voxel based Student's t-test were performed to asses potential differences in basal neuronal activity. The functional connectivity was assessed by thresholding the correlation-matrix at such a value that every node (brain structure) has at least one edge (connection). Now the total amount of connections were calculated over all structures of the somato-sensory pain matrix.

**Example 1.23: SNP of *a2d3* mapping in humans**

We genotyped 5 single nucleotide polymorphisms (SNPs) spaced evenly through a2d3 using the 5' exonuclease method (Tegeder et al., 2006). a2d3 haplotypes were identified using the SAS/genetics software package (SAS Institute, Inc.), which implements a modified expectation-maximization algorithm to reconstruct haplotypes from population genotype data. Linkage disequilibrium between SNPs was used to describe the non-independence of alleles. The PCR reaction mixture consisted of 2.5 ml Master Mixture (Applied Biosystems), 100 nM detection probe for each allele, 900 nM forward and 900 nM reverse amplification primers, and 20 ng genomic DNA in a total reaction volume of 25 μl. Amplification and detection were performed with an ABI Prism 7700 Sequence Detection System. Allele-specific signals were distinguished by measuring endpoint 6-FAM or VIC fluorescence intensities at 508 nm and 560 nm, respectively, and genotypes were generated using Sequence Detection System Software Version 1.7 (Applied Biosystems, CA). Genotyping error rate was directly determined by re-genotyping 25% of the samples, randomly chosen, for each locus. The overall error rate was < 0.005.

**Example 1.24:SNP mapping of the *PIK3CG* locus.**

We genotyped 5 single nucleotide polymorphisms (SNPs) spaced through the *PIK3CG* locus using the 5' exonuclease method. *PIK3CG* haplotypes were identified using the SAS/genetics software package (SAS Institute, Inc.), which implements a modified expectation-maximization algorithm to reconstruct haplotypes from population genotype data. Linkage disequilibrium between SNPs was used to describe the non-independence of alleles. The PCR reaction mixture consisted of 2.5 ml Master Mixture (Applied Biosystems), 100 nM detection probe for each allele, 900 nM forward and 900 nM reverse amplification primers, and 20 ng genomic DNA in a total reaction volume of 25 ml. Amplification and detection were performed with an ABI Prism 7700 Sequence Detection System. Allele-specific signals were distinguished by measuring endpoint 6-FAM or VIC fluorescence intensities at 508 nm and 560 nm, respectively, and genotypes were generated using Sequence Detection System Software Version 1.7 (Applied Biosystems, CA). Genotyping error rate was directly determined by re-genotyping 25% of the samples, randomly chosen, for each locus. The overall error rate was < 0.005.

**Example 1.25:Human genetic studies in healthy volunteers**

We genotyped 189 (for *a2d3)* and 192 (for PIK3CG) normal volunteers who had previously been phenotyped for ratings of experimental pain (Diatchenko et al., 2005). Included subjects were all pain-free Caucasian females 18-34 years of age taken from a larger prospective cohort study designed to examine putative risk factors for the development of temporomandibular joint disorder (TMJD). All subjects gave informed consent following protocols approved by the UNC Committee on Investigations Using Human Subjects. Volunteers were phenotyped with respect to their sensitivity to 16 experimental pain procedures corresponding to multiple pain modalities, including pressure pain, ischemic pain, and temporal summation of heat pain (i.e., windup). Heat sensitivity measures were obtained with a 10 mm diameter

computer-controlled contact thermal stimulator placed on the skin of the forearm. We used principal components analysis (PCA), as performed by the SAS statistical package (version 9.1, SAS Institute, Cary NC), as a data reduction exercise. From our raw pain scores, we derived a thermal windup score (Factor 3).

**Example 1.26:Chronic lumbar root pain**

We collected DNA from 169 (for *a2d3*) and 160 (for PIK3CG) Caucasian adults who participated in a prospective observational study of surgical diskectomy for persistent lumbar root pain (Atlas et al., 2001). The primary endpoint was persistent leg pain over the first postoperative year, using four 'leg pain' variables normalized to Z-scores with mean 0 and standard deviation 1. The primary pain outcome variable for each individual was the mean of these four Z-scores. Genotype-phenotype associations for each SNP were sought by regression analysis. The covariates were a number of demographic, psychological and environmental factors, including sex, age, worker's compensation status, delay in surgery after enrollment and Short-Form 36 general health scale. Stepwise regression (Zaykin et al., 2002) was applied to assess the association between pain scores and haplotypes with frequencies >1%, obtained from the Ensemble database v.38. These haplotypes accounted for 94% of chromosomes studied. If a haplotype was identified to be significantly ($P < 0.05$) associated with pain scores, phenotype-diplotype association analysis was performed by regression analysis. The collection of DNA and genetic analyses were carried out with the approval of the National Institute of Dental and Craniofacial Research institutional review board and informed consent was obtained from all sub-subjects.

**Example 1.27:Statistical analyses**

For analysis of adult heat-dose avoidance responses between control and painless flies (Figure 1B), a two way ANOVA was performed, followed by Tukey's post hoc test. For analysis of adult Drosophila avoidance response and RNAi knock down efficiency (Figure 2B-C) a Student's t test (with correction for multiple comparison) was performed. For analysis of larval pain behavior (Figure 3B and Figure S3A) we have performed the Kruskal-wallis non-parametric test for median comparison followed by the Dunn's post-hoc test. For mouse behavior, a Student's t test was used. For fMRI, the mean activity of each activated brain structure was averaged across all significant activated voxels and subjected to a statistical t-test comparing $\alpha 2\delta 3$ mutant and control mice. At the second level group analysis a standard analysis of variance (ANOVA) was performed for Z-score maps between the different mice genotypes. Areas of significant group activation differences ($P < 0.05$) were used as masks to only show the calculated peak activation maps in these regions. For human chronic pain phenotype, genotype-phenotype associations for each SNP were sought by regression analysis. The covariates were a number of demographic, psychological and environmental factors, including sex, age, worker's compensation status, delay in surgery after enrollment and Short-Form 36 general health scale. Linear regression was also used for the quantitative sensory testing associations in normal adult females, without covariates. For patch clamping studies, data were statistically analyzed by either Mann Whitney U Test for unpaired and Wilcoxon Signed Rank Test for paired comparisons. Unless otherwise indicated, data are represented as mean values $\pm$ sem.

**Example 2: Preliminary results**

**Example 2.1: Genome-wide screen for thermal nociception**

To identify genes required for nociception, we developed a high throughput behavioral assay to determine the response of adult *Drosophila* to noxious heat as a stimulus. When exposed to a surface at a constant temperature of 25°C, flies distribute evenly in the experimental chamber, but when given a choice between a noxious (46°C) and non-noxious (31°C) surface, flies rapidly avoid the harmful temperature (Fig. 1A,B). *painless* mutant flies respond normally to sub-noxious temperatures (≤39°C), but fail to avoid noxious heat (46°C) (Fig. 1B). Thus, adult flies can rapidly avoid noxious heat, and this complex innate behavior is dependent on *painless.*

Using this assay, we performed a genome-wide behavioral screen using the Vienna global *Drosophila* RNAi library (Dietzl et al., 2007). The pan-neuronal specific *elav-Gal4* driver line was crossed to flies containing *UAS-IR* (IR, inverted repeat) transgenes covering the expressed genome (Fig. 1C). Testing control flies (n = 1706) over many different days revealed that the vast majority avoided the noxious surface, with a mean avoidance response of 92 $\pm$ 6.4 % SD, whereas *painless* mutants (n= 1816) exhibited a markedly reduced avoidance response (51 $\pm$ 9.97 % SD). Based on these data, we set our primary cut-off at the 95 percentile of probability, corresponding to a Z-score of > 1.65 (Fig. 1C). At this threshold, we consistently observed impaired thermal nociception in *painless* mutant flies.

To identify novel genes regulating pain, we tested 16051 *elav-Gal4>UAS-IR* combinations targeting 11664 different *Drosophila* genes (82% of the *Drosophila* genome version 5.7) for effects on noxious temperature avoidance (Fig. 1D; Fig. 8B). Positive hits were retested, and 622 specific transgenic UAS-IR lines corresponding to 580 genes were identified as candidate thermal nociception genes (Fig. 1E). Approximately 9% of the neuronal *elav-Gal4* driven

*UAS-IR* lines were lethal, yielding no or few progeny (Fig. 1E). Gene Ontology (GO) and GO gene set enrichment analysis of the total screen data (Fig. 8C-D) showed a significant enrichment of genes involved in ATP synthesis, neurotransmission and secretion. We further annotated 80 nociception hits with previously unknown functions. These genes are CG10095, CG10096, CG10098, CG10158, CG10481, CG11033, CG11456, CG11577, CG11586, CG11590, CG11592, CG11820, CG11967, CG12004, CG12334, CG12785, CG12797, CG13096, CG13162, CG13623, CG1371, CG14351, CG14442, CG14514, CG14980, CG16725, CG16854, CG1804, CG18088, CG18130, CG18213, CG18249, CG18480, CG1968, CG2052, CG2747, CG30005, CG31103, CG31267, CG31955, CG3213, CG32150, CG3224, CG32792, CG33346, CG3996, CG4110, CG4351, CG4477, CG4946, CG5516, CG5565, CG5819, CG5969, CG5986, CG6136, CG6294, CG6340, CG6553, CG6583, CG6637, CG6724, CG6852, CG6901, CG7006, CG7042, CG7175, CG7358, CG7376, CG7556, CG7728, CG7800, CG8233, CG8325, CG8436, CG8771, CG9067, CG9288, CG9636, CG9650. These genes, as well as their orthologue counterparts, in particular human orthologs, or their respective gene products are preferred targets for therapy according to the present invention. KEGG pathway analyses of the primary thermal nociception hits and their respective binding partners revealed significant enrichment for oxidative phosphorylation, amino acid and fatty acid metabolism, ubiquitin-mediated proteolysis, and various signaling pathways such as Wnt, ErbB-, hedgehog-, JAK-Stat-, Notch-, mTOR, or TGFβ signaling. Thus, our nociception screen and the subsequent bioinformatic analyses have revealed multiple genes and pathways that relate to the expression of an innate nociceptive behavior, many of which had no previous functional annotations.

**Example *2.2:straightjacket* is a novel thermal nociception gene in *Drosophila***

One of the genes that we picked up in this screen was *straightjacket (CG12295, stj),* a member of the α2δ family of genes that function as subunits of voltage-gated $Ca^{2+}$ channels (Fig. 2A) and control the function and development of synapses. The fly *stj* ortholog in mammals is α2δ3, a close homolog of α2δ1 which is the molecular target of gabapentin and pregabalin, widely used analgesics for neuropathic pain in humans.

We confirmed that stj is required for noxious heat avoidance in adult *Drosophila* using a second independent hairpin (Fig. 2B). The two stj hairpins resulted in about 90% and 60% reduction of stj mRNA expression, respectively (Fig. 2C). Importantly, when flies were exposed to 46°C without given a choice to escape, *stj* knock-down did not alter the kinetics of temperature-induced paralysis (Fig. 2D), indicating a specific deficit in the nociception response. Of note, *stj* knock-down had no overt effect on brain morphology (Fig. 9A). Using a *stj-Gal4* line (Ly et al., 2008) driving *UAS-Lamin:GFP* to mark nuclei (Stuurman et al., 1999), we found GFP expression primarily in neurons in the pars intercerebralis and surrounding the subesophageal ganglion (Fig. 2E; Fig. 9B). *stjGAL4>UAS-CD8:GFP* labeling of axons (Lee and Luo, 1999) revealed broad projections throughout the central brain (Fig. 2E; Fig. 9B). Using antibodies raised against the *Drosophila* STJ protein, we confirmed STJ expression in *stjGAL4>UAS-Lamin:GFP* positive cells of the pars intercerebralis (Fig. 2F). We also found $GFP^+$ nuclei and projections in the ventral nerve cord (VNC) and ascending/descending axons from the VNC that innervate the central brain (Fig. 9C). *stj*-specific *in situ* hybridization revealed *stj* transcripts in the sensory organ (sensilla) of the leg (Fig. 2G) indicating expression in the peripheral and central nervous system of the fly. Further studies are required to fine map the site of *stj* action in the *Drosophila* pain circuit.

We next assessed whether *stj* also controls thermal nociception in the larval heat pain paradigm (Tracey et al., 2003). In larvae, we found expression of *stj-Gal4*>UAS-*CD8:GFP* in multidendritic sensory neurons (Fig. 3A). Pan-neuronal knock-down of *stj (UAS-stj-IR x elav-Gal4)* abrogated the larval response to noxious heat to an extent even greater than *painless* (Fig. 3B). Larvae carrying a *stj* point mutation and the *stj* point mutation over a corresponding deficiency (*stj*/def) also exhibited impaired thermal nociception. The extent of the defective thermal pain response was greater at higher temperatures (Fig. 10A). Restoring a functional copy of *stj* using the P[acman] system (Venken et al., 2009) rescued the thermal nociception defects in $stj^2$ mutant larvae, confirming the requirement for *stj* in this behavior (Fig. 3B; Fig. 10A). $stj^2$ mutant larvae showed wild-type responses to non-noxious touch (Kernan et al., 1994), indicating that these larvae are capable of responding to innocuous stimuli (Fig. 10B). Collectively, these data show that *stj* is required for avoidance of noxious heat in *Drosophila,* confirming the accurateness of the genetic screen.

**Example 2.3:Thermal analgesia in α*2*δ*3* mutant mice**

We next tested whether the fly *stj* data is predictive of altered nociceptive behavior in mammals. The closest *stj* ortholog in mammals is α2δ3 (mouse α2δ3 is 33% identical and 60% similar to the STJ protein and the domain structures are conserved throughout evolution (Ly et al., 2008). To examine the role of α2δ3 *in vivo* we studied α*2*δ*3* mutant mice generated by homologous recombination. Correct recombination and loss of protein expression were confirmed by Southern (Fig. 4A) and Western blotting (Fig. 4B). α2δ*3* mutant mice are born at the expected Mendelian frequency and are fertile. Extensive characterization of these mice showed no obvious anatomical or histological abnormalities, including apparently normal brain morphology. There were also no genotype-related or biologically significant differences noted between age and gender matched mutant and wild-type control mice for any of the parameters evaluated

at necropsy or by serum chemistry and haematology. Moreover, normal growth and body weights were recorded for mice at 49, 90, 180, and 300 days of age. Hence, by all anatomical and physiological parameters assessed, *α2δ3* mutant mice appear normal.

Importantly, similar to *Drosophila stj* mutants, *α2δ3* mutant mice showed a defect in acute thermal nociception in the hot plate assay, with diminished responsiveness at 50, 52, 54 and 56°C (Fig. 4C). In addition, *α2δ3* mutant mice exhibited delayed sensitization in the Complete Freund's Adjuvant (CFA) model of peripheral inflammatory pain (Fig. 4D), indicating that *α2δ3* contributes to the acute phase of heat hyperalgesia. CFA induced inflammation, as determined by paw swelling, was comparable between *α2δ3* mutant and control mice (Fig. 11A). By contrast, mechanical sensitivities (von Frey test) were unaffected in *α2δ3* mutant mice (Fig. 11B). *α2δ3* mutant mice were also evaluated for other behavioral tasks (Crawley, 2008): open field test to assess locomotor activity, general exploratory behavior, intra-session habituation, and general anxiety; tail suspension to assess behavioral despair; and a rotarod test to assess basic motor skills and coordination. In these assays no significant differences were observed between *α2δ3* mutant and control mice (Fig. 11C-F). Thus, genetic deletion of *α2δ3* in mice results in substantially impaired acute pain responses and a delay in inflammatory heat hyperalgesia.

**Example 2.4:α2δ3 SNPs associate with human pain sensitivity**

Since knock-down of *straightjacket* in *Drosophila* and knock-out of α2δ3 in mice results in impaired sensitivity to thermal pain, we expected that polymorphisms at the α2δ3 (*CACNA2D3*) locus might be associated with heat pain variance in humans. To assay for potential association of α2δ3 haplotypes relative to pain sensitivity we screened 4 single nucleotide polymorphisms (SNPs) within or close to the human *CACNA2D3* gene (Fig. 5A) in a cohort of 189 healthy volunteers subjected to a battery of experimental pain sensitivity tests (Diatchenko et al., 2005). Of these, the minor allele of the SNP rs6777055 was significantly associated with reduced thermal pain sensitivity, i.e, heat wind-up pain (Fig. 5B, recessive model). Wind-up measures successive increases in perceived pain intensity to a repeated noxious heat stimulus (ten heat pulses of 1.5 seconds each at 50°C each separated by 3 seconds).

Thermosensitive neurons have been also implicated in chronic pain in humans. To explore this we compared pain levels in 169 Caucasian adults who participated in a prospective observational study of surgical discectomy for persistent lumbar root pain, caused by an intervertebral disc herniation (Atlas et al., 2001) for an association with *CACNA2D3* SNPs. The minor alleles of two *CACNA2D3* SNPs (rs1851048 and rs6777055) were associated with less pain within the first year following surgery (Fig. 5C, recessive model). Importantly, the rs6777055 SNP C/C was significantly associated with less pain in both healthy volunteer and chronic pain cohorts showing a recessive mode of inheritance. In both the experimental and lumbar pain groups the minor allele frequency for rs6777055 was 0.2, that is ~ 4% of the human population is homozygous for this genetic variant. These data show that minor variants within the human *CACNA2D3* gene are associated with less induced pain in healthy volunteers and reduced chronic pain in lumbar back pain patients.

**Example 2.5:α2δ3 controls central transmission of pain signals to the sensory cortex and other higher order pain centers**

Nociceptive processing involves the relay of sensory information from primary nociceptor neurons to second order neurons in the dorsal horn of the spinal cord which then transfer nociceptive information to the brain stem, thalamus, and higher order brain centres. Since our *α2δ3* knock-out mice carry a LacZ reporter, we used β-Gal staining as a marker to assess *α2δ3* expression. In the brain, β-Gal labeled the thalamus, pyramidal cells of the ventro-posterior paraflocculus of the cerebellum, caudate, putamen, the dentate gyrus of the hippocampus, the olfactory bulb and olfactory tubercle, as well as diffusely throughout the cortex (Fig. 6A). The LacZ expression profiles were confirmed by Western blotting and quantitative PCR and matched *in situ* data from the Allen brain atlas. We did not detect LacZ expression in the spinal cord and DRG. Absence of a2d3 expression in primary sensory DRG neurons was confirmed by Western blotting (Fig. 4B). In line with these expression data, our behavioral experiments showed that loss of α2δ3 does not affect the noxious heat-induced tail flick response (Fig. 11G), a pain behavior mediated by a spinal reflex circuit (Pitcher et al., 1995). Finally, patch clamping showed that calcium current and kinetics were comparable among DRG neurons from control and a2d3 mutant mice (Fig. 11H-K) indicating no requirement for a2d3 in calcium channel function in these cells. These data suggest that *α2δ3* is not required for thermal pain processing in nociceptors and the spinal cord, but *α2δ3* may regulate thermal pain processing in the brain.

To address this, we employed non-invasive functional magnetic resonance imaging (fMRI) using the blood oxygenation level dependent (BOLD) signal to generate a activation maps of brain regions affected by noxious topical heat stimuli. In wild type mice (n=20), noxious thermal stimuli activate brain structures known as the "pain matrix" such as the thalamus (Fig. 6B), the S1 and S2 somatosensory cortex (Fig. 6C), the cingulum, amygdala, hypothalamus, or the motor cortex (Fig. 12). These areas are also involved in pain perception in human subjects. In both wild type (n=20)

and α2δ3 mutant mice (n=18), thermal pain-induced activation of the thalamus (Fig. 6B), the key sub-cortical pain relay centre (Price, 2000). Intriguingly, loss of α2δ3 expression interrupted the normal engagement of pain circuitry in the brain resulting in markedly reduced BOLD peak amplitudes and activation volumes in higher order pain centers such as the somato-sensory S1 and S2 cortices, cingulate, or motor cortex after exposure to noxious temperatures (Fig. 6C; Fig. 12). Impaired activation of higher order pain centers, i.e. sensory and motor cortices, were confirmed by calculation of Euclidian distances.

To additionally assess temporal information flow of the pain signal within different cerebral structures, we calculated a cross-correlation matrix of the response time profiles for each predefined region of the somato-sensory pain matrix (Fig. 6D). In control mice, the sensory input relays thermal-evoked neural signals to the thalamus, where it effectively spreads to other central brain centers such as the sensory and association cortex, limbic system, cerebellum, basal ganglia, and motor cortex. *α2δ3* mutant mice again exhibited normal activation of the thalamus but a reduced flow to nearly all the higher order pain centers, in particular the somato-sensory cortex (SC) (Fig. 6D). Moreover, whereas the pain signal spreads from the left (i.e. contra-lateral to the side of stimulation) in control mice, we found a considerable reduction in correlation coefficients of the pain signal from the left to the right brain in *α2δ3* mutant mice (Fig. 13A). In addition, we observed increased negative BOLD signals in the S1 somato-sensory, motor and the cingulate cortex on both hemispheres of *α2δ3* mutant mice (Fig. 13B), suggesting that genetic inactivation of α2δ3 not only results in impaired transmission of the signal to higher pain structures, but also in intra-cortical inhibition. Thus, loss of α2δ3 leads to impaired transmission of noxious heat evoked signals from the thalamus to higher pain centers.

**Example 2.6:Loss of α2δ3 results in sensory cross-activation**

Although we found a marked impairment in the activation of known higher order pain centers, one puzzling finding from our fMRI data was that we did not find statistically significant differences between control and *α2δ3* mutant mice in total activation volume and peak height when neuronal activity was surveyed in the entire brain (Fig. 14A). Since we had initially only focused on the pain matrix, we speculated that therefore loss of α2δ3 may result in hyperactivation of additional brain regions. Remarkably, noxious heat stimulation of all *α2δ3* mutant mice triggered significantly enhanced activation of the visual cortex, the auditory cortex, as well as olfactory brain regions (Fig. 7A,B). Thus, in α2δ3 mutant mice, noxious heat stimulation results in a significant sensory cross-activation of brain regions involved in vision, hearing, and olfaction.

To image basal neuronal activity integrated over a 24 hour time period, we employed manganese labeling (MEMRI) (Silva et al., 2004). We observed similar activity in all imaged brain regions among a group comparison of control and α2δ3 mutant mice indicating that the observed stimulus induced sensory cross-activations are not due to altered basal neuronal activity. Moreover, diffusion tensor imaging (DTI) showed no overt structural changes with respect to fractional anisotropy between the thalamus and higher order pain centers or the thalamus and the visual, auditory, and olfactory centers. Further, cross-correlation analysis of the time profiles of the structures of the the pain matrix from resting-state BOLD imaging showed no defects in spontaneous spreading from the thalamus to higher order pain centers in α2δ3 mutant mice. Finally, network analysis of this resting state brain activity showed no overt changes in total functional connectivity within the pain matrix (1087 connections in wild type versus 1040 connections in α2δ3 mutant mice) and also apparently normal multisensory-thalamo-cortical network connectivity (1922 connections in wild type versus 2099 connections in α2δ3 mutant mice). Although we cannot exclude subtle developmental changes in defined neuronal populations of *α2δ3* mutant mice, these data suggest that the observed heat induced sensory cross-activations (and defective transmission of the thermal pain signal from the thalamus to higher order pain centers) are not due to altered basal neuronal connectivities.

To assess whether loss of a2d3 also results in sensory cross-activation in other sensory modalities, we performed BOLD imaging in response to tactile vibrissal stimulation (Hess et al., 2000). Among control and a2d*3* mutant mice, we observed apparently normal activation of the brain region that encompasses the barrel field; the barrel field is the primary cortical somato-sensory brain centre for processing of vibrissal stimulation. Remarkably, although we observed apparently normal activation of the barrel field, tactile stimulation again resulted in sensory cross-activation of visual, auditory, and olfactory brain centers in a2d*3* knock-out mice (Fig. 7A,C). Thus, in α2δ3 mutant mice, noxious heat stimulation as well as tactile stimulation trigger sensory cross-activation of brain regions involved in vision, hearing, and olfaction.

**Example 3: PI3Kgamma locus**

Using a genome-wide neuronal-specific RNAi knock-down strategy in adult *Drosophila,* we performed the first global screen for an innate behavior, and identified several novel genes required for thermal nociception (see example 2). We report the construction from this data of an evolutionary-conserved, global network map, identifying molecular components and key pathways involved in thermal pain perception. This systems map predicts multiple novel candidate

genes and pathways that are conserved across phyla, providing a starting point for finding novel mammalian pain genes. One prominent pathway identified was phospholipid signaling and SNP mapping showed that a polymorphism in the phospholipid kinase PI3Kgamma locus is significantly associated with altered pain sensitivity in humans. Importantly, the role of PI3Kgamma in pain perception was confirmed in mutant mice that exhibit pronounced hypersensitivity to noxious heat and capsaicin. Using knock-in mice, pain hypersensitivity was mapped to the PI3Kgamma lipid kinase activity. Mechanistically, PI3Kgamma acts as a negative regulator of TRPV1 ion channel activity in DRG neurons. Our data provide the first systems map for a complex innate behavior in any species that allowed us to identify a previously unknown pain gene, PI3Kg, in mice and humans.

Using a genome-wide strategy of neuron specific RNAi knock-down in adult *Drosophila,* we report the first global screen for an innate behavior, avoidance of noxious heat, and identification of hundreds of novel genes required for its manifestation (see example 2). To interrogate this unprecedented resource beyond direct fly mammalian gene homologue comparisons we examined the *Drosophila* nociception genes using data-mining and bioinformatics to define a conserved global systems network of pain. Potential mouse and human orthologs of our candidate pain genes were first identified using compara49, inparanoid, inparanoid6.1, inparanoid6.1 to ensembl, homologen08, and orthomclv2 databases (fig. 19). Of the 580 candidate fly thermal nociception genes, 399 had human orthologs (table 1, cols. 2-4). Among known mammalian pain genes, our screen identified two fly orthologs of amiloride-sensitive cation channel 3 (Accn3), an acid-sensing channel that sets tonic pain thresholds, GDNF family receptor alpha2, responsible for maintaining the size and terminal innervation of cutaneous nociceptors, Protein Kinase G, implicated in promoting thermal sensitization in response to inflammation, the fly orthologs of the adenosine receptor Adora, important for both acute and chronic pain in mice, a potential homolog of the mammalian galanin receptor that contributes to neuropathic pain behavior in mice, a fly GPCR similar to mouse cannabinoid and lysophosphatidic acid receptors, and a fly homolog of the cholecystokinin receptor that influences thermal pain thresholds. Thus, our genome-wide functional screen for thermal nociception in flies identifies multiple known mammalian pain genes, showing that the inventive screen provided correct results of known and new pain associated genes.

Gene ontology (GO) analyses of human and mouse orthologs of the thermal nociception hits showed a marked enrichment of genes involved in neurotransmission and secretion, housekeeping systems such as mitochondrial structure, ATP synthesis, metabolism, or calcium signaling (Fig. 15A,B). We next generated an interaction map based on first degree binding partners for these mammalian orthologs, because we assume that our candidate thermal nociception genes perform many of their functions in protein complexes. All binding partners were identified in yeast-2-hybrid screens reported in the biomolecular interaction network database BIND, i.e. binding partners experimentally confirmed to interact with the candidate genes. Importantly, among the first degree binding partners in this interaction network, we found fly allatostatin C receptor 1, which has some homology with mammalian opioid receptors, Lmx1b which regulates central serotonergic responses to opioids, the nuclear factor-erythroid 2-related factor 2, which has anti-nociceptive effects by inducing upregulation of heme oxygenase-1, and tyrosine hydroxylase, the enzyme required for dopamine and catecholamines production. Thus, we present strong evidence that our screen identifies many known regulators of thermal pain and multiple uncharacterized genes and pathway previously never associated with pain perception.

To construct a systems map of thermal nociception we performed an enrichment analysis of KEGG pathways and Broad Institute C2 pathway gene sets, on the mouse and human "pain" orthologs and their first degree binding partners (in part shown in fig. 19). From both the mouse and human KEGG and C2 analyses, we found significant enrichment of genes involved in mitochondria, metabolism, calcium signaling, inflammation, cell adhesion, RNA processing, and neurotransmission. Finally, to generate a comprehensive global network map of thermal nociception, the KEGG pathways from *Drosophila,* mouse and human were combined with relevant gene sets from the C2 annotations to create a global "nociception network" (Fig. 16).

Importantly, the connectivity of the entire systems map remains intact even after omitting all binding partners (fig. 20), i.e., expansion of the pain network map by including binding partners does not introduce a bias. Moreover, all except one pathway in the network map has > 50% representation from the *Drosophila* pain hits alone. To test whether our bioinformatic approach is indeed valid for predicting pain genes, we measured the overlap of the direct candidate pain hits and their binding partners with previous microarray data from pain studies and all "pain" annotated genes from OMIM (NCBI). Intriguingly, we found a 35% overlap of our direct pain hits and a 37% overlap of the binding partners with the microarray and OMIM data. In contrast, 100 random gene lists gave a maximum of 6% overlapping genes and a minimum of 0%. Thus, our hypothesis-free systems map is indeed enriched for known pain genes, and we suggest, contains many others not yet discovered.

In example 2 we demonstrated a role in mammalian heat pain perception of a direct *Drosophila* thermal nociception hit, a2d3. Importantly, our bioinformatic analyses allowed us to expand our systems map to also include mammalian gene sets and genes that have no direct fly orthologs. We therefore wanted to validate whether this *in silico* approach has indeed also the power to identify novel mammalian pain genes. We first investigated whether polymorphisms in the gene sets predicted by our conserved network are associated with significant differences in pain sensitivity in humans. One of the genes identified by this approach was the catalytic p110 subunit of the phosphatidylinositol-3-OH kinase (PI3Kγ, PIK3CG). Class I phosphatidylinositol-3-OH kinases (PI3K) are lipid kinases that convert phosphatidylinositol 4,5-bisphosphate (PIP2) into phosphatidylinositol (3,4,5)-trisphosphate (PIP3). Mammals have four class I PI3K, three of which (α, β, and δ) are primarily activated by tyrosine kinase signaling pathways, while PI3Kγ is the principal PI3K that relays signals via GPCRs. Of note, PI3Kα, β, and d all showed no significant association with pain sensitivities in humans. PI3K and GPCR signaling were prominent nodes in our conserved systems map (Fig. 16; fig. 20). Although PI3Kgamma intersects with GPCRs implicated in nociception and PI3Kgamma has been the focus of literally thousands of studies, and is a major target for drug development, no distinct function has ever been ascribed to PI3Kgamma in the nervous system, and certainly none in pain.

To validate whether the PI3Kgamma nucleotide polymorphism found in our GWAS is indeed associated with pain in humans, we screened 5 SNPs within or close to the human *PIK3CG* gene (fig. 21A) in 192 healthy human volunteers who had undergone tests for acute experimental pain sensitivity. Of those, the SNP rs757902, located 5565 base pairs upstream from the human *PIK3CG* transcription start site, was significantly associated with a greater propensity to windup represented by the PCA-derived Factor 3 (Fig 17B, dominant model). Wind-up measures successive increases in perceived pain intensity to a repeated noxious heat stimulus (ten heat pulses of 1.5 seconds each at 50°C each separated by 3 seconds).

To confirm this *PIK3CG* association in an independent cohort, we compared pain levels in 160 Caucasian adults who participated in a prospective observational study of surgical discectomy for persistent lumbar root pain, caused by an intervertebral disc herniation. Again, a polymorphism in the SNP rs757902 was asso-associated with significantly increased pain during the first year following surgery (Fig. 17C, dominant model). The frequencies for the G allele in rs757902 were 3.1% and 3.4% in the healthy volunteer and lumbar pain groups, respectively, indicating that approximately 54-58% of the population carry at least one G allele at rs757902. We have therefore identified one common genetic variant of the human *PIK3CG* gene, rs757902, which is associated with an increase in heat pain sensitivity in healthy volunteers and heightened chronic post-surgical pain in lumbar back pain patients, a finding that indicates the strong predictive link between our bioinformatic analysis and human pain sensations.

To confirm that *PI3Kγ* indeed controls mammalian pain sensitivity, we analysed *PI3Kγ*$^{-/-}$ mice. These mutant mice exhibited normal posture and appearance and no significant differences were observed in hearing, sight, and vocalization. *PI3Kγ*$^{-/-}$ mice also behaved similar to littermate controls in a skilled reaching task (fig. 21A), built comparable nests, and displayed normal circadian behavior (fig. 21B), open field activity (fig. 21C and D), and motor coordination (fig. 21E). *PI3Kγ* littermate control and mutant mice also displayed a comparable ability to learn in the water maze, the T maze, and in a passive foot shock avoidance assay (fig. 21F-H). Furthermore, the overall morphology and histology of the central nervous system appeared normal in *PI3Kγ*$^{-/-}$ mice.

When we tested for pain responses, *PI3Kγ*$^{-/-}$ mice exhibited an exaggerated behavioral response to radiant heat plantar stimulation, using the Hargreaves test (Fig. 17D). We also observed an increased acute thermal response using the hot plate assay (Fig. 3E). In mammals, TRPV1 is the prototypical thermo-receptor, and is also the receptor for capsaicin, the active ingredient in chili peppers. We therefore tested whether PI3Kγ mutant mice also exhibit heightened reactivity to capsaicin. Importantly, we indeed observed a massive hypersensitivity in the response of the *PI3Kγ*$^{-/-}$ mice (Fig. 17F). In coltrast, the mechanical pain threshold using the von Frey test (fig. 21I and J), and the behavioral responses to acetone application (a cooling sensation) (fig. 21K) were comparable between control and *PI3Kγ*$^{-/-}$ littermates.

PI3Kγ is a key mediator of inflammatory cell migration to the site of injury. We therefore tested PI3Kγ mutant mice for potential defects in inflammation-induced pain sensitization, i.e. thermal hyperalgesia. *PI3Kγ*$^{-/-}$ mice developed comparable levels of thermal hyperalgesia (fig. 22A and B) following plantar CFA injection, indicating that inflammation-induced pain sensitization does not depend on PI3Kγ. CFA-induced inflammation, as determined by paw swelling, was comparable between mutant and control mice (fig. 22C). To further exclude a potential role of haematopoietic cells, we performed bone marrow chimeras; the requirement for PI3Kγ in thermal sensing was indeed mapped to non-haematopoeitic cells (Fig. 17G). PI3Kγ has been shown to act both in a kinase-dependent fashion, through conversion of PIP2 to PIP3, and in a kinase-independent manner. We therefore tested the behavioral response of PI3Kγ kinase-dead (KD) knock-in mice to radiant plantar stimulation. PI3Kγ KD mice exhibited a decrease in thermal nociception latency (Fig. 17H), comparable to the enhanced responses observed in complete PI3Kγ mutant mice. These data show that PI3Kγ functions through its lipid kinase activity, to modulate the behavioral response to noxious heat stimuli *in vivo.*

To test if the enhanced basal thermal sensitivity observed in *PI3Kγ*$^{-/-}$ mice is intrinsic to noxious heat TRPV1 expressing nociceptors, we employed electrophysiology on isolated wild type and *PI3Kγ*$^{-/-}$ dorsal root ganglia (DRG) neurons *in vitro.* Both control and *PI3Kγ*$^{-/-}$ DRG neurons responded to a thermal ramp (Fig. 18A), with *PI3Kγ*$^{-/-}$ mice exhibiting an increased steepness in the inward current response to increasing temperature. This translated into a massively increased Q10 value (Fig. 18B), a measure of temperature-dependent rate change in channel conductivity, indicating that *PI3Kγ*$^{-/-}$ DRG cells exhibit massive hyper-activation in response to noxious heat. In accordance with our behavioral data, isolated *PI3Kγ*$^{-/-}$ DRG neurons also exhibited increased sensitivity to capsaicin (Fig. 18C,D). These results provide the first genetic evidence that PI3Kγ plays a functional in role in the nervous system; moreover, we show that PI3Kγ functions as a negative regulator of TRPV1 channels, and, in consequence in the absence of PI3Kγ, DRG

neurons exhibit a markedly exaggerated response to noxious heat and capsaicin.

Our data provide the first systems map for a complex innate behavior in any species. This network map revealed many genes and gene sets previously reported to be involved in mammalian nociception. Importantly, the network also allowed us to predict a novel regulator of pain perception, PI3Kgamma. Although multiple GPCRs operate in the nervous system and PI3Kgamma is the main lipid kinase coupled to GPCRs, no behavioral defects had been reported before in PI3Kgamma mutant mice. Most importantly, these results directly translate to humans, since we found a PI3Kgamma polymorphism that significantly associates with altered pain sensitivity in healthy volunteers and pain levels in chronic back pain patients. Of note, humans carrying a minor allele variant at the *PI3K3CG* locus, as well as our PI3Kgamma mutant mice exhibit increased pain indicating that our systems approach using whole genome fly screening and construction of a conserved network map can identify positive as well negative regulators of pain perception. These data reinforce the extraordinary conservation of the neurobiological mechanisms of nociception, from its manifestation as avoidance of damage in flies to the complex sensation of pain in humans, a conservation that provides an opportunity from a fly screen to find novel human pain genes.

Our whole-genome, neuron-specific RNAi screen provides a global functional analysis of a complex, innate behavior. We have uncovered hundreds of novel target genes for nociception, a large proportion of which had completely unknown functions until now.

One of the screen hits was the calcium channel subunit *straightjacket*/$\alpha 2\delta 3$ and phospholipid kinase PI3Kgamma. In both larva and adult *Drosophila,* we show that *straightjacket* or PI3Kgamma are indeed involved in nociception, validating the screening results that yielded more than 100 genetic targets. Importantly, similar to the fly, genetic deletion of $\alpha 2\delta 3$ in mice also results in impaired acute heat pain responses. These results translate to humans, since we found $\alpha 2\delta 3$ polymorphisms that significantly associate with reduced pain sensitivity in healthy volunteers and lower levels of chronic pain in lumbar back pain patients. These data reinforce the extraordinary conservation of the neurobiological mechanisms of nociception, from its manifestation as avoidance of damage in primitive creatures like flies, to the complex sensation of pain in humans. Ligands and antagonists of the novel genetic targets were identified, that can be used in novel therapeutic approaches for the treatment and amelioration of pain and its symptoms.

### Example 4: Testing of anti-pain compounds

The basic principle of animal models of human pain involve the induction of a pain-like state in the organism resulting in characteristic behavioural and physical responses, such as hypersensitivity to touch (mechanical allodynia) and temperature (cold allodynia). The assessment of the efficacy of potential analgesics is determined based on said compound's ability to attenuate/ameliorate these symptoms.

### Example 4.1: Assessing chronic pain animal models - Chronic Constriction Injury

The Bennett and Xie chronic constriction injury (CCI) model is a model of mononeuropathic pain (Bennett and Xie, 1988). Rodents are subjected to a surgical procedure where gut ligatures are tied loosely around the sciatic nerve at the mid-thigh level. Symptoms of neuropathy develop in the operated paw over the following days including tactile allodynia and cold allodynia which are measured using Von Frey's hairs and paw withdrawal from a cold plate, respectively. Operated animals also exhibit other symptoms of spontaneous pain including thermal hyperalgesia , ectopic and spontaneous firing of sensory afferents, autotomy, licking and guarding of paw and sleep architecture abnormalities (Blackburn-Munro and Erichsen, 2005). Furthermore, electrophysiological studies have demonstrated the presence of both sensory nerve hyperexcitability and central sensitisation (wind up) in the dorsal horn and elsewhere (Blackburn-Munro and Erichsen, 2005). The Bennett and Xie model is thought to have predictive validity as an 88% concordance has been observed between activity in the model (any endpoint) and clinical efficacy in neuropathic pain (Kontinen and Meert 2003). Multiple drug classes including NSAIDs are ineffective in the clinical treatment of neuropathic pain and fail to ameliorate symptoms in the Bennett model despite the presence of an inflammation in the initial phase after surgery (eg Schafers et al., 2004; Takahashi et al., 2004, LaBuda and Little, 2005). Pregabalin, a drug approved for the treatment of various types of (chronic) pain, including neurophathic pain associated with diabetic peripheral neuropahty in humans, is effective at ameliorating symptoms of pain in the CCI model.

#### *Example 4.1.1. Test apparatus*

Mechanical allodynia was assessed by Von Frey's hairs. These consist of a series of wires of progressive thickness each of which bend when a critical pressure (1.4, 2, 4, 6, 8, 10, 15 g) is applied. Animals were placed in floorless Perspex testing boxes resting on a wire mesh tray. Von Frey's hairs were then applied through the mesh floor to the sole of the left and right hind paws until either the animal sensed discomfort and moved the paw or the pressure applied to the Von Frey's hair exceeded the critical level and it was observed to bend. Von Frey's hairs were applied in

ascending order until either a pain response (the paw was moved) was registered or the cut-off of 15 g was reached, using the Von Frey hair with the highest rating. Hairs were applied to each heel 8-10 times at a frequency of approximately 1 Hz. If a limb was moved in response to a probe, further testing was halted and the sensitivity to a mechanical stimulus was deemed to have been reached.

Sensitivity to cold (cold allodynia) was assessed by placing animal subjects on a cold plate held at 10°C. The latency (time) to lift the respective hind paw clear off the cold plate was measured. A cut-off of 180 s was applied.

***Example 4.1.2: Compound testing***

This example demonstrates the effectiveness of compound(s) according to the invention in ameliorating mechanical and cold allodynia in rats with peripheral mononeuropathy induced by loose ligation of the sciatic nerve as described in Bennett and Xie, 1988.

The test apparatus according to Example 4.1.1 was used for assessing mechanical and thermal sensitivities.

Male Sprague-Dawley rats were habituated to the mechanical test apparatus (Von Frey's hairs, described in Example 4.1.1) during 5-10 min periods spread over two days and once to the cold plate set at 10°C for 3 minutes. Following habituation animals underwent a surgical procedure requiring anaesthetization under isoflurane, shaving of the left (ipsilateral) hind limb and swabbing with antiseptic followed by administration of sodium pentobarbitone. An incision was made to reveal the left sciatic nerve which was tied off with four loose ligatures of chromic cat gut. The exposed muscle was sutured with non-absorbable silk and the wound closed with surgical clips.

The animals were monitored in the hours post surgery and on a daily basis thereafter. Animals showing signs of ill health or autotomy were removed from the study. Approximately 12 and 18 days after surgery the animals were reassessed for sensitivity to Von Frey's hairs and to the cold plate.

Animal subjects meeting pre-defined criteria for hypersensitivity to mechanical (ipsilateral hind-paw moved at a pressure of $\leq$4 g) and thermal stimuli ($\leq$78s withdrawal latency) in the operated (ipsilateral) hind-paw were allocated according to baseline mechanical and cold sensitivity scores to produce balanced treatment groups of 8 to 10 animals in each group.

*Example 4.1.3. Single dose/acute dosing*

Each group of animals according to Example 4.1.2 was administered a single dose of either a positive control drug (pregabalin formulated in a vehicle of 0.5% methyl cellulose to a concentration of 12 mg/mL and administered po in a 5 mL/kg dosing volume to give a dose of 60 mg/kg) or a compound according to the invention. Animal subjects were reassessed for mechanical and cold allodynia 30 min, 90 min, 180 min and 24 hours after administration of the respective compound, using Von Frey's hairs and a cold plate set at 10°C. The cold plate assessment was performed 5 mins after each Von Frey test.

*Example 4.1.3.1 Test with dasatinib and tenofovir*

The procedure as described in Example 4.1.2.1. was performed with the compounds according to the invention being dasatinib  and tenofovir. In total, five groups of animals were included: one group of 10 animals received the positivie control drug, pregabalin, at 60 mg/kg and four groups of 8 animals each received either 10 mg/kg dasatinib, 30 mg/kg dasatinib, 50 mg/kg tenofovir or 500 mg/kg tenofovir, respectively (Fig. 23C). Dasatinib was formulated in a vehicle (50% PEG:50% water) to concentrations of 2 and 6 mg/mL and administered orally in a 5 mL/kg dosing volume to give doses of 10 mg/kg or 30 mg/kg. Tenofovir was formulated in a vehicle (0.5% methyl cellulose) to concentrations of 10 and 100 mg/mL and administered orally in a 5 mL/kg dosing volume to give doses of 50 and 500 mg/kg.

*Example 4.1.3.2. Description of cilomilast, zardaverine and roflumilast*

The procedure as described in Example 4.1.2.1. was performed with the compounds according to the invention being cilomilast, zardaverine and roflumilast. In total, five groups of animals were included: one group of 10 animals received the positivie control drug, pregabalin, at 60 mg/kg and four groups of 8 animals each received either 3 mg/kg cilomilast, 30 mg/kg cilomilast, 20 mg/kg zardaverine or 1.8 mg/kg roflumilast, respectively (Fig. 23A). Cilomilast was formulated in water to concentrations of 0.6 and 6 mg/mL and administered orally in a 5 mL/kg dosing volume to give doses of 3 and 30 mg/kg. Zardaverine was formulated in a vehicle of 0.5% methyl cellulose to a concentration of 4 mg/mL and administered orally in a 5 mL/kg dosing volume to give a dose of 20 mg/kg. Roflumilast was formulated in a vehicle of 0.5% methyl cellulose to a concentration of 0.36 mg/mL and administered orally in a 5 mL/kg dosing volume to give a dose of 1.8 mg/kg.

*Example 4.1.3.3: Test with bosutinib and adefovir*

The procedure as described in Example 4.1.2.1. was performed with the compounds according to the invention being bosutinib and adefovir. In total, five groups of animals were included: one group of 10 animals received the positivie control drug, pregabalin, at 60 mg/kg and four groups of 8 animals each received either 60 mg/kg bosutinib, 200 mg/kg bosutinib, 2 mg/kg adefovir or 20 mg/kg adefovir, respectively (Fig. 23B). Bosutinib was formulated in 0.5% methyl cellulose to concentrations of 12 and 40 mg/mL and administered orally in a 5 mL/kg dosing volume to give doses of 60 and 200 mg/kg. Adefovir was formulated in 0.5% methyl cellulose to concentrations of 0.4 and 4 mg/mL and administered orally in a 5 mL/kg dosing volume to give doses of 2 and 20 mg/kg.

*Example 4.1.4: Chronic studies*

Each group of animals according to Example 4.1.2 was administered either a positive control drug (pregabalin formulated in a vehicle of 0.5% methyl cellulose to a concentration of 12 mg/mL and administered po in a 5 mL/kg dosing volume to give an effective dose (e.g. 60 mg/kg)) or a compound according to the invention. Dosing was carried out one or several times daily for two days to eight weeks. Animal subjects were reassessed for mechanical and cold allodynia either daily, weekly, biweekly, monthly or at the end of the study at one or several time points: 30 min, 90 min, 180 min, 12 hours, 24 hours, 48 hours, 72 hours and 1 week after administration of the respective compound, using Von Frey's hairs and a cold plate set at 10°C.

**Example 4.2: Assessing inflammatory pain animal models - Complete Freunds Adjuvant (CFA)**

Complete Freunds Adjuvant (CFA) consists of heat-killed mycobacterium suspended in mineral oil. When injected systemically into rodents, an immune response is triggered resulting in chronic inflammation of many organs such as skin, liver, spleen, eyes, bone marrow and particularly peripheral joints. The inflammatory response results in bone resorption and periosteal bone proliferation. Importantly this inflammatory state results in spontaneous pain and ectopic nerve cell firing. Thus, the resultant adjuvant disease exhibits polyarthritic symptoms.

When CFA is injected unilaterally into the limbs it elicits a monoarthritic-like condition and is thus used to model chronic inflammatory conditions. Unilateral injection allows the analysis of ipsilateral and contralateral effects of localised joint pain (Millan et al., 1988). Injection of CFA results in oedema of the affected joint, mechanical allodynia and mechanical and thermal hyperalgesia (Butler et al., 1992; Hsieh et al., 2010; Meotti et al., 2006; Staton et al., 2007). As these features resemble the clinical pathology of rheumatoid arthritis, CFA-induced chronic inflammation has been widely used as a model of this condition.

A wide variety of treatments acting via different mechanisms that have generated positive data in rat adjuvant-induced arthritic models have proven effective in subsequent clinical trials for rheumatoid arthritis (Hegen et al., 2008). Indeed, an analysis by Whiteside (Whiteside et al., 2008), concluded that activity in the rat CFA test could be used to predict the exposure needed for clinical efficacy. Both morphine-like opioids, steroids and NSAID analgesics can attenuate inflammation-associated allodynia/hyperalgesia and normalise nociceptive sensitivity (Jett et al., 1999, da Silva Filho et al., 2004).

***Example 4.2.1: Test system***

This example demonstrates the effectiveness of a compound according to the invention in ameliorating mechanical and cold allodynia in rats with inflammatory pain induced by injection of an adjuvant (CFA) into the limbs.

The test apparatus according to Example 4.1.1 was used for assessing mechanical and thermal sensitivities. 53 male Sprague-Dawley rats were habituated to the test apparatus and tested for basal sensitivity to mechanical and thermal stimuli in both the right (ipsilateral) and left (contralateral) paws using Von Freys hair and a radiant heat source, respectively, on the day prior to Complete Freunds Adjuvant (CFA) administration (Day 0). The following morning (Day 1), animals were subjected to a subplantar injection of 50% complete CFA in a 100uL injection volume into the ipsilateral hind-paw. Two days later (Day 3), baseline mechanical and thermal sensitivities were reassessed in the CFA-injected (ipsilateral) and non-injected (contralateral) hind-paws. 42 rats meeting pre-defined criteria for hypersensitivity to mechanical (ipsilateral hind-paw moved at a pressure of <4g) and thermal stimuli (>30% difference in latency time between ipsi- and contra-lateral hind-paws) were assigned to treatment groups of 8 to 10 animals in each group. One hour later, at T=0, drug administration commenced.

*Example 4.2.2. Acute/single dosing*

One group of animals according to Example 4.2.1 was administered a single dose of the drug indomethacin

(positive control), an NSAID with demonstrated efficacy in the CFA model and in human inflammatory/arthritic diseases. Indomethacin was formulated in 50% 0.1 M $Na_2CO_3$; 47.5% phosphate buffered saline (PBS): taken to pH 7 with 2.5% 1M HCl at a concentration of 2 mg/mL to give a dose of 10 mg/kg when administered intraperitoneally in a 5 mL/kg injection volume. The remaining groups of animals were administered a single dose of compound(s) according to the invention. 30, 90, 180 min and 24h post-dose, all groups of rats were re-assessed for mechanical allodynia and thermal hyperalgesia in both the treated and untreated hind-paws, using Von Frey's hairs and a cold plate set at 10°C in accordance with Example 0. All readings were compared to the basal sensitivity reading (T=0) of the (ipsilateral) paw in each animal.

*Example 4.2.1.1 Test with dasatinib and tenofovir*

The procedure as described in Example 2A was performed with the comopounds according to the invention being dasatinib and tenofovir. In total, five groups of animals were included: one group of 10 animals received the positivie control drug, indomethacin, intraperitonally at a dose of 10 mg/kg Four groups of 8 animals each received either 10 mg/kg dasatinib, 30 mg/kg dasatinib, 50 mg/kg tenofovir or 500 mg/kg tenofovir, respectively (Fig. 24A). Dasatinib was formulated in a vehicle (50% PEG:50% water) to concentrations of 2 and 6 mg/mL and administered orally in a 5 mL/kg dosing volume to give doses of 10 and 30 mg/kg. Tenofovir was formulated in a vehicle (0.5% methyl cellulose) to concentrations of 10 and 100 mg/mL and administered orally in a 5 mL/kg dosing volume to give doses of 50 and 500 mg/kg.

*Example 4.2.1.2. Test with cilomilast, zardaverine and roflumilast*

The procedure as described in Example 2A was performed with the comopounds according to the invention being cilomilast, zardaverine and roflumilast. In total, five groups of animals were included: one group of 10 animals received the positivie control drug, indomethacin, at 10 mg/kg and four groups of 8 an-animals each received either 3 mg/kg cilomilast, 30 mg/kg cilomilast, 20 mg/kg zardaverine or 1.8 mg/kg roflumilast, respectively (Fig. 24B). Cilomilast was formulated in water to concentrations of 0.6 and 6 mg/mL and administered orally in a 5 mL/kg dosing volume to give doses of 3 and 30 mg/kg. Zardaverine was formulated in a vehicle of 0.5% methyl cellulose to a concentration of 4 mg/mL and administered orally in a 5 mL/kg dosing volume to give doses of 20 mg/kg. Roflumilast was formulated in a vehicle of 0.5% methyl cellulose to a concentrations of 0.36 mg/mL and administered orally in a 5 mL/kg dosing volume to give doses of 1.8 mg/kg.

*Example 4.2.1.3. Test with bosutinib and adefovir*

The procedure as described in Example 1 was performed with the comopounds according to the invention being bosutinib and adefovir. In total, five groups of animals were included: one group of 10 animals received the positivie control drug, indomethacin, at 10 mg/kg and four groups of 8 animals each received either 60 mg/kg bosutinib, 200 mg/kg bosutinib, 2 mg/kg adefovir or 20 mg/kg adefovir, respectively (Fig. 24C). Bosutinib was formulated in 0.5% methyl cellulose to concentrations of 12 and 40 mg/mL and administered orally in a 5 mL/kg dosing volume to give doses of 60 and 200 mg/kg. Adefovir was formulated in 0.5% methyl cellulose to concentrations of 0.4 and 4 mg/mL and administered orally in a 5 mL/kg dosing volume to give doses of 2 and 20 mg/kg.

*Example 4.2.3. Chronic studies*

Each group of animals according to Example 2 was administered either a positive control drug (indomethacin, which was formulated in 50% 0.1 M $Na_2CO_3$; 47.5% phosphate buffered saline (PBS): taken to pH 7 with 2.5% 1M HCl at a concentration of 2 mg/mL to give an effective dose (e.g. 10 mg/kg when administered intraperitoneally in a 5 mL/kg injection volume)), or a compound according to the invention. Dosing was carried out one or several times daily for two days to eight weeks. Animal subjects were reassessed for mechanical and cold allodynia either daily, weekly, biweekly, monthly or at the end of the study at one or several time points: 30 min, 90 min, 180 min, 12 hours, 24 hours, 48 hours, 72 hours and 1 week after administration of the respective compound, using Von Frey's hairs and a cold plate set at 10°C according to Example 0.

**Example 5. Measurement of PDE4D activity and determination of IC50 values of test compounds**

The half maximal inhibitory concentration ($IC_{50}$) is a measure of the effectiveness of a compound in inhibiting biological or biochemical function. Concentration-response plots are used to determine the effects of an inhibitor on an enzymatic reaction. The cloning of catalytic domains of human PDE4D as well as the expression and purification of the PDE catalytic domains were based on the protocols described in Card et al., 2004. Measurement of phosphodiesterase

activity takes advantage of the selective binding of 5-AMP or 5-GMP (and not cAMP or cGMP) to yttrium silicate beads with embedded scintillant. 0.1-1 nM PDE was incubated with 50 nM 3H-cAMP or 70 nM 3H-cGMP (Amersham, 5-60 Ci/mmol) in 50 mM Tris (pH 7.5), 8.3 mM MgCl2, 1.7mM EGTA, and 0.01% BSA at 30C for 30 min in 384-well assay plates. The assay was terminated by adding one-third volume of 5 mg/ml yttrium silicate beads in 18 mM ZnAcetate/ZnSO4 solution (3:1).

A minimum of 30 min after mixing and centrifuging the reaction, hydrolysis was quantified by reading in a scintillation counter (Trilux, Conclusions Wallac). The cAMP and cGMP concentrations used, where possible, were far below the Km of the enzyme to ensure that the $IC_{50}$ values obtained are good approximations of Ki (Mehats et al., 2002).

## Example 6. Clinical study

Patients with a long term or chronic pain condition are eligible for the study.

After initial screening, a total of 20 to 200 patients are randomized to one of two treatment groups. Patients in one treatment group receive a compound according to the invention at a pharmaceutically active dose and patients in the other (control) treatment group receive either placebo or an active control drug at a pharmaceutically active dose. A preferred active control drug is pregabalin. Preferably, the study is carried out in a double-blinded fashion.

Treatment duration is between 1 and 15 weeks, and efficacy evaluation is carried out as the average of the pain scores recorded for the past 1 to 7 days (preferably 7 days) relative to the day chosen for efficacy evaluation, comparing the group receiving the compound according to the invention with the control group.

The pain scores are preferably assessed based on patients' daily pain diaries, in which they record their daily pain score on an 11-point (0 = "no pain" to 10 = "worst possible pain") numeric rating scale (NRS) [Arezzo et al., 2008].

The primary endpoint of the study is preferably a comparison of the average pain score for the last 7 available pain diary entries at the end of the treatment phase.

## Example 7. Painful diabetic peripheral neuropathy (PDPN)

A clinical study according to Example 6 is carried out with the following key inclusion criteria: patients are men or women ≥18 years of age with type 1 or type 2 diabetes with HbA1C ≤11% and painful diabetic peripheral neuropathy of at least 3 months' duration. Patients score at least 40 mm on the Short-Form McGill Pain Questionnaire (SF-MPQ) Visual Analog Scale (VAS) [Arezzo et al., 2008], or any other standard VAS. At randomization, patients have completed at least four daily pain diary entries (using an 11-point NRS) and should have an average daily pain score ≥4 over the past 7 days.

## Example 8. Post-herpetic neuralgia

A clinical study according to Example 6 is carried out with the following key inclusion criteria: patients are men or women ≥18 years of age with persistent pain for at least 6 months after the onset of herpes zoster rash. Patients score at least 40 mm on the Short-Form McGill Pain Questionnaire (SF-MPQ) Visual Analog Scale (VAS) [Arezzo et al., 2008], or any other VAS.

## Example 9. Mixed neuropathy

A clinical study according to Example 6 is carried out with patients selected according to inclusion criteria in Example 7 as well as Example 8.

## Example 10. Measurement of binding affinity to determine Kd values

The binding and selectivity of a compound for a receptor can be assayed, for instance, by carrying out biochemical binding assays such as KinomeScan kinase binding assays as described in Karaman et al., 2008. For an assay to determine the binding of a compound to the target FRK, the kinase construct NP_002022.1 may be used. The assay can be performed with either the full length protein or with a kinase construct that spans the catalytic domain.

## REFERENCES

Aleman, A., Rutten, G.J., Sitskoorn, M.M., Dautzenberg, G., and Ramsey, N.F. (2001). Activation of striate cortex in the absence of visual stimulation: an fMRI study of synesthesia. Neuroreport 12, 2827-2830.

Arthurs, O.J., and Boniface, S. (2002). How well do we understand the neural origins of the fMRI BOLD signal? Trends in neurosciences 25, 27-31.
Atlas, S.J., Keller, R.B., Chang, Y., Deyo, R.A., and Singer, D.E. (2001). Surgical and nonsurgical management of sciatica secondary to a lumbar disc herniation: five-year outcomes from the Maine Lumbar Spine Study. Spine 26, 1179-1187.
Basbaum, A.I., Bautista, D.M., Scherrer, G., and Julius, D. (2009). Cellular and molecular mechanisms of pain. Cell 139, 267-284.
Beauchamp, M.S., and Ro, T. (2008). Neural substrates of sound-touch synesthesia after a thalamic lesion. J Neurosci 28, 13696-13702.
Catterall, W.A. (2000). Structure and regulation of voltage-gated Ca2+ channels. Annual review of cell and developmental biology 16, 521-555.
Costigan, M., Scholz, J., and Woolf, C.J. (2009). Neuropathic Pain: A Maladaptive Response of the Nervous System to Damage. Annual review of neuroscience.
Cox, J.J., Reimann, F., Nicholas, A.K., Thornton, G., Roberts, E., Springell, K., Karbani, G., Jafri, H., Mannan, J., Raashid, Y., et al. (2006). An SCN9A channelopathy causes congenital inability to experience pain. Nature 444, 894-898.
Crawley, J.N. (2008). Behavioral phenotyping strategies for mutant mice. Neuron 57, 809-818.
Diatchenko, L., Slade, G.D., Nackley, A.G., Bhalang, K., Sigurdsson, A., Belfer, I., Goldman, D., Xu, K., Shabalina, S.A., Shagin, D., et al. (2005). Genetic basis for individual variations in pain perception and the development of a chronic pain condition. Human molecular genetics 14, 135-143.
Dickman, D.K., Kurshan, P.T., and Schwarz, T.L. (2008). Mutations in a Drosophila alpha2delta voltage-gated calcium channel subunit reveal a crucial synaptic function. J Neurosci 28, 31-38.
Dietzl, G., Chen, D., Schnorrer, F., Su, K.C., Barinova, Y., Fellner, M., Gasser, B., Kinsey, K., Oppel, S., Scheiblauer, S., et al. (2007). A genome-wide transgenic RNAi library for conditional gene inactivation in Drosophila. Nature 448, 151-156.
Dudycha, G.J., & Martha M. Dudycha (1935). A Case of Synesthesia: Visual-Pain and Visual-Audition. Journal of Abnormal and Social Psychology 30, 57-69.
Dworkin, R.H., O'Connor, A.B., Backonja, M., Farrar, J.T., Finnerup, N.B., Jensen, T.S., Kalso, E.A., Loeser, J.D., Miaskowski, C., Nurmikko, T.J., et al. (2007). Pharmacologic management of neuropathic pain: evidence-based recommendations. Pain 132, 237-251.
Eroglu, C., Allen, N.J., Susman, M.W., O'Rourke, N.A., Park, C.Y., Ozkan, E., Chakraborty, C., Mulinyawe, S.B., Annis, D.S., Huberman, A.D., et al. (2009). Gabapentin receptor alpha2delta-1 is a neuronal thrombospondin receptor responsible for excitatory CNS synaptogenesis. Cell 139, 380-392.
Field, M.J., Cox, P.J., Stott, E., Melrose, H., Offord, J., Su, T.Z., Bramwell, S., Corradini, L., England, S., Winks, J., et al. (2006). Identification of the alpha2-delta-1 subunit of voltage-dependent calcium channels as a molecular target for pain mediating the analgesic actions of pregabalin. Proceedings of the National Academy of Sciences of the United States of America 103, 17537-17542.
Hess, A., Stiller, D., Kaulisch, T., Heil, P., and Scheich, H. (2000). New insights into the hemodynamic blood oxygenation level-dependent response through combination of functional magnetic resonance imaging and optical recording in gerbil barrel cortex. J Neurosci 20, 3328-3338.
Hubbard, E.M., and Ramachandran, V.S. (2005). Neurocognitive mechanisms of synesthesia. Neuron 48, 509-520.
Hucho, T., and Levine, J.D. (2007). Signaling pathways in sensitization: toward a nociceptor cell biology. Neuron 55, 365-376.
Kernan, M., Cowan, D., and Zuker, C. (1994). Genetic dissection of mechanosensory transduction: mechanoreception-defective mutations of Drosophila. Neuron 12, 1195-1206.
Knabl, J., Witschi, R., Hosl, K., Reinold, H., Zeilhofer, U.B., Ahmadi, S., Brockhaus, J., Sergejeva, M., Hess, A., Brune, K., et al. (2008). Reversal of pathological pain through specific spinal GABAA receptor subtypes. Nature 451, 330-334.
Koester, S.E., and Insel, T.R. (2007). Mouse maps of gene expression in the brain. Genome biology 8, 212.
Kurshan, P.T., Oztan, A., and Schwarz, T.L. (2009). Presynaptic alpha(2)delta-3 is required for synaptic morphogenesis independent of its Ca(2+)-channel functions. Nature neuroscience 12, 1415-1423.
Lee, T., and Luo, L. (1999). Mosaic analysis with a repressible cell marker for studies of gene function in neuronal morphogenesis. Neuron 22, 451-461.
Lumpkin, E.A., and Caterina, M.J. (2007). Mechanisms of sensory transduction in the skin. Nature 445, 858-865.
Ly, C.V., Yao, C.K., Verstreken, P., Ohyama, T., and Bellen, H.J. (2008). straightjacket is required for the synaptic stabilization of cacophony, a voltage-gated calcium channel alpha1 subunit. The Journal of cell biology 181, 157-170.
Manev, H., and Dimitrijevic, N. (2004). Drosophila model for in vivo pharmacological analgesia research. European journal of pharmacology 491, 207-208.

Melzack, R. (1999). From the gate to the neuromatrix. Pain Suppl 6, S121-126.

Ogawa, S., Lee, T.M., Kay, A.R., and Tank, D.W. (1990). Brain magnetic resonance imaging with contrast dependent on blood oxygenation. Proceedings of the National Academy of Sciences of the United States of America 87, 9868-9872.

Petersen, C.C. (2007). The functional organization of the barrel cortex. Neuron 56, 339-355.

Pitcher, G.M., Yashpal, K., Coderre, T.J., and Henry, J.L. (1995). Mechanisms underlying antinociception provoked by heterosegmental noxious stimulation in the rat tail-flick test. Neuroscience 65, 273-281.

Premkumar, L.S. (2010). Targeting TRPV1 as an alternative approach to narcotic analgesics to treat chronic pain conditions. The AAPS journal 12, 361-370.

Price, D.D. (2000). Psychological and neural mechanisms of the affective dimension of pain. Science (New York, NY 288, 1769-1772.

Shmuel, A., Yacoub, E., Pfeuffer, J., Van de Moortele, P.F., Adriany, G., Hu, X., and Ugurbil, K. (2002). Sustained negative BOLD, blood flow and oxygen consumption response and its coupling to the positive response in the human brain. Neuron 36, 1195-1210.

Silva, A.C., Lee, J.H., Aoki, I., and Koretsky, A.P. (2004). Manganese-enhanced magnetic resonance imaging (MEMRI): methodological and practical considerations. NMR in biomedicine 17, 532-543.

Sokabe, T., Tsujiuchi, S., Kadowaki, T., and Tominaga, M. (2008). Drosophila painless is a Ca2+-requiring channel activated by noxious heat. J Neurosci 28, 9929-9938.

Stuurman, N., Delbecque, J.P., Callaerts, P., and Aebi, U. (1999). Ectopic overexpression of Drosophila lamin C is stage-specific lethal. Experimental cell research 248, 350-357.

Tegeder, I., Costigan, M., Griffin, R.S., Abele, A., Belfer, I., Schmidt, H., Ehnert, C., Nejim, J., Marian, C., Scholz, J., et al. (2006). GTP cyclohydrolase and tetrahydrobiopterin regulate pain sensitivity and persistence. Nature medicine 12, 1269-1277.

Thulborn, K.R., Waterton, J.C., Matthews, P.M., and Radda, G.K. (1982). Oxygenation dependence of the transverse relaxation time of water protons in whole blood at high field. Biochimica et biophysica acta 714, 265-270.

Tracey, I., and Mantyh, P.W. (2007). The cerebral signature for pain perception and its modulation. Neuron 55, 377-391.

Tracey, W.D., Jr., Wilson, R.I., Laurent, G., and Benzer, S. (2003). painless, a Drosophila gene essential for nociception. Cell 113, 261-273.

Treede, R.D., Kenshalo, D.R., Gracely, R.H., and Jones, A.K. (1999). The cortical representation of pain. Pain 79, 105-111. Venken, K.J., Carlson, J.W., Schulze, K.L., Pan, H., He, Y., Spokony, R., Wan, K.H., Koriabine, M., de Jong, P.J., White, K.P., et al. (2009). Versatile P[acman] BAC libraries for transgenesis studies in Drosophila melanogaster. Nature methods 6, 431-434.

Xu, S.Y., Cang, C.L., Liu, X.F., Peng, Y.Q., Ye, Y.Z., Zhao, Z.Q., and Guo, A.K. (2006). Thermal nociception in adult Drosophila: behavioral characterization and the role of the painless gene. Genes, brain, and behavior 5, 602-613.

Atlas, S.J., Keller, R.B., Chang, Y., Deyo, R.A., and Singer, D.E. (2001). Surgical and nonsurgical management of sciatica secondary to a lumbar disc herniation: five-year outcomes from the Maine Lumbar Spine Study. Spine 26, 1179-1187.

Benzer, S. (1967). BEHAVIORAL MUTANTS OF Drosophila ISOLATED BY COUNTERCURRENT DISTRIBUTION. Proceedings of the National Academy of Sciences of the United States of America 58, 1112-1119.

Brody, T., Stivers, C., Nagle, J., and Odenwald, W.F. (2002). Identification of novel Drosophila neural precursor genes using a differential embryonic head cDNA screen. Mechanisms of development 113, 41-59.

Couto, A., Alenius, M., and Dickson, B.J. (2005). Molecular, anatomical, and functional organization of the Drosophila olfactory system. Curr Biol 15, 1535-1547.

Diatchenko, L., Slade, G.D., Nackley, A.G., Bhalang, K., Sigurdsson, A., Belfer, I., Goldman, D., Xu, K., Shabalina, S.A., Shagin, D., et al. (2005). Genetic basis for individual variations in pain perception and the development of a chronic pain condition. Human molecular genetics 14, 135-143.

Dietzl, G., Chen, D., Schnorrer, F., Su, K.C., Barinova, Y., Fellner, M., Gasser, B., Kinsey, K., Oppel, S., Scheiblauer, S., et al. (2007). A genome-wide transgenic RNAi library for conditional gene inactivation in Drosophila. Nature 448, 151-156.

Grigliatti, T.A., Hall, L., Rosenbluth, R., and Suzuki, D.T. (1973). Temperature-sensitive mutations in Drosophila melanogaster. XIV. A selection of immobile adults. Mol Gen Genet 120, 107-114.

Hayashi, S., Ito, K., Sado, Y., Taniguchi, M., Akimoto, A., Takeuchi, H., Aigaki, T., Matsuzaki, F., Nakagoshi, H., Tanimura, T., et al. (2002). GETDB, a database compiling expression patterns and molecular locations of a collection of Gal4 enhancer traps. Genesis 34, 58-61.

Jourdan, D., Ardid, D., and Eschalier, A. (2001). Automated behavioural analysis in animal pain studies. Pharmacol Res 43, 103-110.

Kernan, M., Cowan, D., and Zuker, C. (1994). Genetic dissection of mechanosensory transduction: mechanoreception-defective mutations of Drosophila. Neuron 12, 1195-1206.

Knabl, J., Witschi, R., Hosl, K., Reinold, H., Zeilhofer, U.B., Ahmadi, S., Brockhaus, J., Sergejeva, M., Hess, A., Brune, K., et al. (2008). Reversal of pathological pain through specific spinal GABAA receptor subtypes. Nature 451, 330-334.

Ly, C.V., Yao, C.K., Verstreken, P., Ohyama, T., and Bellen, H.J. (2008). straightjacket is required for the synaptic stabilization of cacophony, a voltage-gated calcium channel alpha1 subunit. The Journal of cell biology 181, 157-170.

Mummery-Widmer, J.L., Yamazaki, M., Stoeger, T., Novatchkova, M., Bhalerao, S., Chen, D., Dietzl, G., Dickson, B.J., and Knoblich, J.A. (2009). Genome-wide analysis of Notch signalling in Drosophila by transgenic RNAi. Nature.

Obreja, O., Biasio, W., Andratsch, M., Lips, K.S., Rathee, P.K., Ludwig, A., Rose-John, S., and Kress, M. (2005). Fast modulation of heat-activated ionic current by proinflammatory interleukin 6 in rat sensory neurons. Brain 128, 1634-1641.

Obreja, O., Rathee, P.K., Lips, K.S., Distler, C., and Kress, M. (2002). IL-1 beta potentiates heat-activated currents in rat sensory neurons: involvement of IL-1RI, tyrosine kinase, and protein kinase C. Faseb J 16, 1497-1503.

Siddiqi, O., and Benzer, S. (1976). Neurophysiological defects in temperature-sensitive paralytic mutants of Drosophila melanogaster. Proceedings of the National Academy of Sciences of the United States of America 73, 3253-3257.

Tegeder, I., Costigan, M., Griffin, R.S., Abele, A., Belfer, I., Schmidt, H., Ehnert, C., Nejim, J., Marian, C., Scholz, J., et al. (2006). GTP cyclohydrolase and tetrahydrobiopterin regulate pain sensitivity and persistence. Nature medicine 12, 1269-1277. Thibault, S.T., Singer, M.A., Miyazaki, W.Y., Milash, B., Dompe, N.A., Singh, C.M., Buchholz, R., Demsky, M., Fawcett, R., Francis-Lang, H.L., et al. (2004). A complementary transposon tool kit for Drosophila melanogaster using P and piggyBac. Nature genetics 36, 283-287.

Tracey, W.D., Jr., Wilson, R.I., Laurent, G., and Benzer, S. (2003). painless, a Drosophila gene essential for nociception. Cell 113, 261-273.

Venken, K.J., Carlson, J.W., Schulze, K.L., Pan, H., He, Y., Spokony, R., Wan, K.H., Koriabine, M., de Jong, P.J., White, K.P., et al. (2009). Versatile P[acman] BAC libraries for transgenesis studies in Drosophila melanogaster. Nature methods 6, 431-434.

Verstreken, P., Koh, T.W., Schulze, K.L., Zhai, R.G., Hiesinger, P.R., Zhou, Y., Mehta, S.Q., Cao, Y., Roos, J., and Bellen, H.J. (2003). Synaptojanin is recruited by endophilin to promote synaptic vesicle uncoating. Neuron 40, 733-748.

Vosshall, L.B., Wong, A.M., and Axel, R. (2000). An olfactory sensory map in the fly brain. Cell 102, 147-159.

Zaykin, D.V., Westfall, P.H., Young, S.S., Karnoub, M.A., Wagner, M.J., and Ehm, M.G. (2002). Testing association of statistically inferred haplotypes with discrete and continuous traits in samples of unrelated individuals. Human heredity 53, 79-91.

Kuzniar, R. C. van Ham, S. Pongor, J. A. Leunissen, Trends Genet 24, 539 (Nov, 2008).

Sasaki et al., Science 287, 1040 (Feb 11, 2000).

Patrucco et al., Cell 118, 375 (Aug 6, 2004).

Racz et al., J Neurosci 28, 12125 (Nov 12, 2008).

Dittert et al., J Neurosci Methods 82, 195 (Aug 1, 1998).

L. Vyklicky et al., J Physiol 517 (Pt 1), 181 (May 15, 1999) Bennett G.J., Xie Y.K., (1988). Pain, 33, 87-107.

Blackburn-Munro G, Erichsen H.K., (2005). Current Pharm Des, 11, 2961-2975.

Butler, S.H., Godefroy, F., Besson, J.-M., Weil-Fugazza, J., (1992). Pain, 48; 73-81.

Hegen, M., Keith, J.C., Collins, M., Nickerson-Nutter, C.L., (2008). Ann Rheum Dis, 67: 1505-1515.

Hsieh, G.C., Chandran, P., Salyers, A.K., Pai, M., Zhu, C.Z., Wensink, E.J., Witte, D.G., Miller, T.R., Mikusa, J.P., Baker, S.J., Wetter, J.M., Marsh, K.C., Hancock, A.A., Cowart, M.D., Esbenshade, T.A., Brioni, J.D., Honore, P., (2010). Pharmacol Biochem Behav, 95; 41-50.

Kontinen V.K., and Meert T.F., (2003). In: eds Dostrovsky J.O., Carr D.B., and Koltzenburg, M., Prog in Pain Res Management 24, pp489-498, IASP press, Seattle.

LaBuda, C.J., and Little, P., (2005) J Neurosci Meths, 144, 175-181.

Meotti, F.C., Missau, F.C., Ferreira, J., Pizzolatti, M.G., Mizuzaki, C., Nogueira, C.W., Santos, A.R.S., (2006). Biochem Pharmacol, 72; 1707-17134.

Millan, M.J., Czlonkowski, A., Morris, B., Stein, C., Arendt, R., Huber, A., et al., (1988). Pain, 35, 299-312.

Schafers, M., Marziniak, M., Sorkin, L.S., Yaksh, T.L., Sommer C., (2004) Exp Neurol ., 185, 160-168.

Staton, P.C., Wilson, A.W., Bountra, C., Chessell, I.P., Day, N.C., (2007). Eur J Pain' 11; 283-289.

Takahashi M., Kawaguchi, M., Shimada, K." Konishi, N., Furuya H., Nakashima, T., (2004). Neurosci Letts, 356, 37-40.

Whiteside, G.T., Adedoyin, A., Leventhal, L., (2008). Neuro-pharmacology, 54, 767-775.

Jett, M.F., Ramesha, C.S., Brown, C.D., Chiu, S., Emmett, C., Voronin, T., Sun, T., O'Yang, C., Hunter, J.C., Eglen, R.M., Johnson, R.M., (1999). Characterization of the analgesic and anti-inflammatory activities of ketorolac and its

enantiomers in the rat. J Pharmacol Exp Ther, 288(3):1288-97.
da Silva Filho A.A., Andrade e Silva M.L., Carvalho J.C., Bastos J.K., (2004). Evaluation of analgesic and anti-inflammatory activities of Nectandra megapotamica (Lauraceae) in mice and rats. J Pharm Pharmacol., 56(9): 1179-84.
Card, G.L., England, B.P., Suzuki, Y., Fong, D., Powell, B., Lee, B., Luu, C., Tabrizizad, M., Gillette, S., Ibrahim, P.N., Artis, D.R., Bollag, G., Milburn, M.V., Kim, S.H., Schlessinger, J., Zhang, K.Y., (2004). Structural Basis for the Activity of Drugs that Inhibit Phosphodiesterases. Structure, 12(12):2233-47.
Mehats, C., Andersen, C.B., Filopanti, M., Jin, S.L., and Conti, M. (2002). Cyclic nucleotide phosphodiesterases and their role in endocrine cell signaling. Trends Endocrinol. Metab. 13, 29-35.
Arezzo, J.C., Rosenstock, J., LaMoreaux, L., Pauer, L., (2008), Efficacy and safetyof pregabalin 600 mg/d for treating painful diabetic peripheral neuropathy: a double-blind placebo-controlled trial. BMC Neurology, 16;8:33.
Karaman, M.W., Herrgard, S., Treiber, D.K., Gallant, P., Atteridge, C.E., Campbell, B.T., Chan, K.W., Ciceri, P., Davis, M.I., Edeen, P.T., Faraoni, R., Floyd, M., Hunt, J.P., Lockhart, D.J., Milanov, Z.V., Morrison, M.J., Pallares, G., Patel, H.K., Pritchard, S., Wodicka, L.M., Zarrinkar, P.P., (2008). A quantitative analysis of kinase inhibitor selectivity. Nat Biotechnol, Jan;26(1):127-32.
Neely et al., (2010). A genome-wide Drosophila screen for heat nociception identifies $\alpha 2\delta 3$ as an evolutionarily conserved pain gene. Cell. 2010 Nov 12;143(4):628-38.

## Claims

1. FMO3 inhibitor for use in the treatment or prevention of pain.

2. FMO3 inhibitor for use according to claim 1, wherein the FMO3 inhibitor is selected from Tenofovir and Methimazole.

3. FMO3 inhibitor for use according to claim 1, wherein the FMO3 inhibitor is selected from a compound comprising a phosphoric acid or ester group and/or a purine radical, the compound being Tenofovir.

4. Tenofovir (PMPA) ( Tenofovir ) for use in the treatment or prevention of pain.

5. Methimazole ( Methimazole ) for use in the treatment or prevention of pain.

6. Compound for use according to any one of claims 1 to 5, **characterized in that** the compound is administered topically, enterally or parenterally, in particular preferred orally or rectally, intravenously, intraarterially, intramuscularly, sub-cutaneously, intradermally, intraperitoneally, transdermally, transmucosally or by inhalation.

7. Compound for use according to any one of claims 1 to 6, **characterized in that** the subject to be treated is a mammal, preferably a human.

8. Compound for use according to any one of claims 1 to 7, **characterized in that** the compound, modulator or inhibitor is provided in a medicament.

9. Compound for use according to any one of claims 1 to 8, **characterized in that** the compound, modulator or inhibitor is provided together with a pharmaceutically acceptable carrier or buffer.

10. Compound for use according to any one of claims 1 to 9, **characterized in that** the compound, modulator or inhibitor is administered in a therapeutically effective dosage, preferably a dosage of between 0.01 mg/kg and 1 g/kg.

11. Compound for use according to any one of claims 1 to 10, wherein pain is selected from or associated with chronic or acute pain or hyperalgesia pain, somatogenic pain, neuropathic pain, psychogenic pain, heat induced pain, physical pain, nociception, hyperalgesia, rheumatic pain, headache, low back pain, pelvic pain, myofascial pain, vascular pain, migraine, wound associated pain, inflammatory pain, arthritic pain, diabetic pain, pain from cancer, somatic visceral pain, fibromyalgia, postoperative pain, phantom pain, trigeminal neuralgia, post-herpetic neuralgia, painful diabetic neuropathy, painful diabetic peripheral neuropathy, diabetic polyneuropathy, sciatic pain, radiculopathy, radicular pain, lumbar pain or any combination thereof.

12. Compound for use according to claim 11, wherein pain is chronic pain.

**13.** Compound for use according to claim 11 or 12, wherein pain is neuropathic pain.

**14.** Compound for use according to claim 11, wherein pain is inflammatory pain.

**15.** Compound for use according to claim 11, 12 or 13, wherein pain is non-inflammatory pain.

Fig. 1

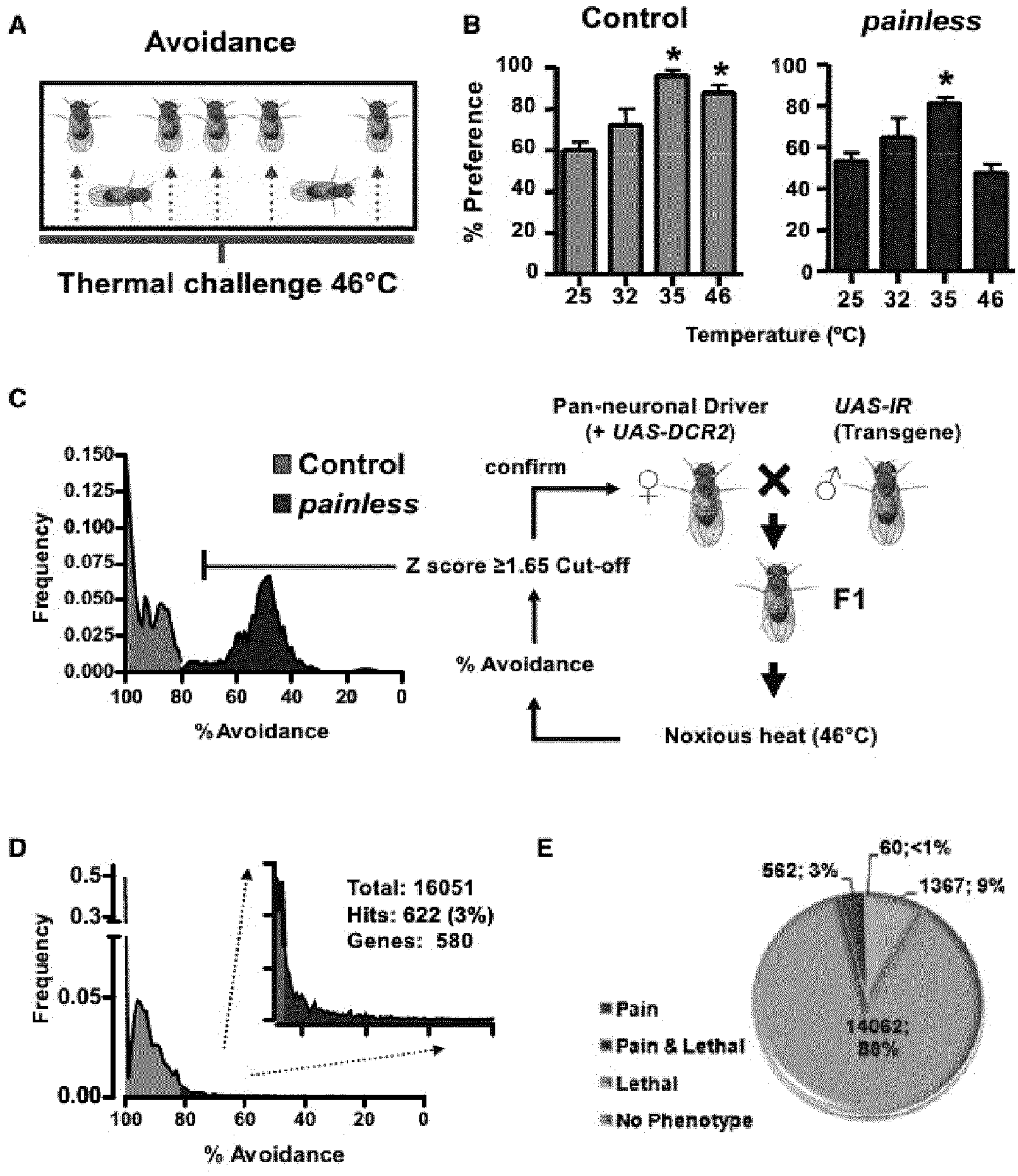
A
Avoidance
Thermal challenge 46°C
B
Control
painless
% Preference
100
80
60
40
20
0
25 32 35 46
Temperature (°C)
C
Control
painless
Frequency
0.150
0.125
0.100
0.075
0.050
0.025
0.000
100 80 60 40 20 0
% Avoidance
Z score ≥1.65 Cut-off
confirm
% Avoidance
Pan-neuronal Driver
(+ UAS-DCR2)
UAS-IR
(Transgene)
F1
Noxious heat (46°C)
D
Frequency
0.5
0.3
0.05
0.00
100 80 60 40 20 0
% Avoidance
Total: 16051
Hits: 622 (3%)
Genes: 580
E
60;<1%
562; 3%
1367; 9%
14062; 88%
Pain
Pain & Lethal
Lethal
No Phenotype

Fig. 2

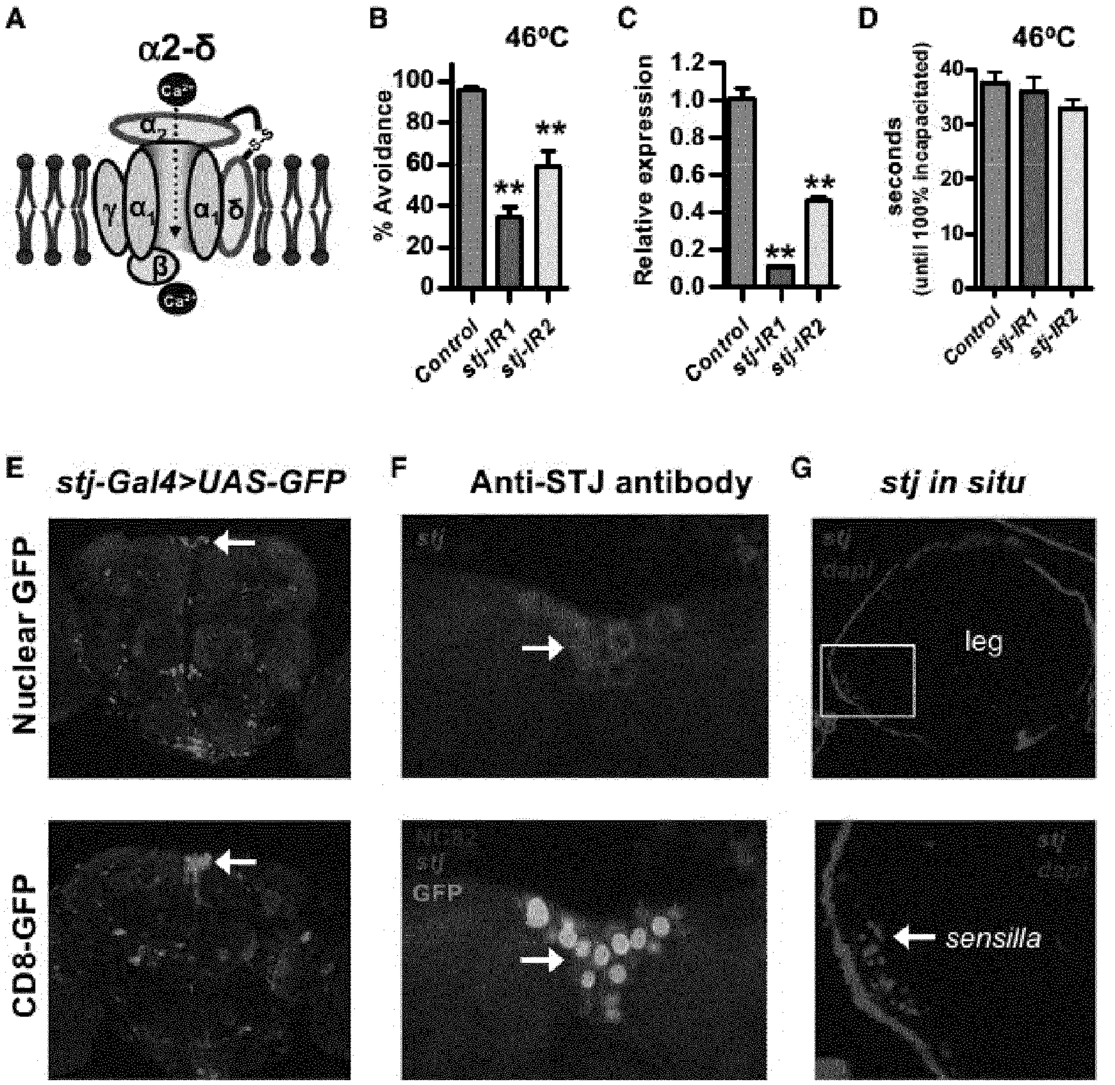
A
α2-δ
Ca2+
α2
S-S
γ
α1
α1
δ
β
Ca2+
B
46°C
% Avoidance
100
80
60
40
20
0
**
**
Control
stj-IR1
stj-IR2
C
Relative expression
1.2
1.0
0.8
0.6
0.4
0.2
0.0
**
**
Control
stj-IR1
stj-IR2
D
46°C
seconds
(until 100% incapacitated)
40
30
20
10
0
Control
stj-IR1
stj-IR2
E
stj-Gal4>UAS-GFP
Nuclear GFP
CD8-GFP
F
Anti-STJ antibody
G
stj in situ
leg
sensilla

Fig. 3

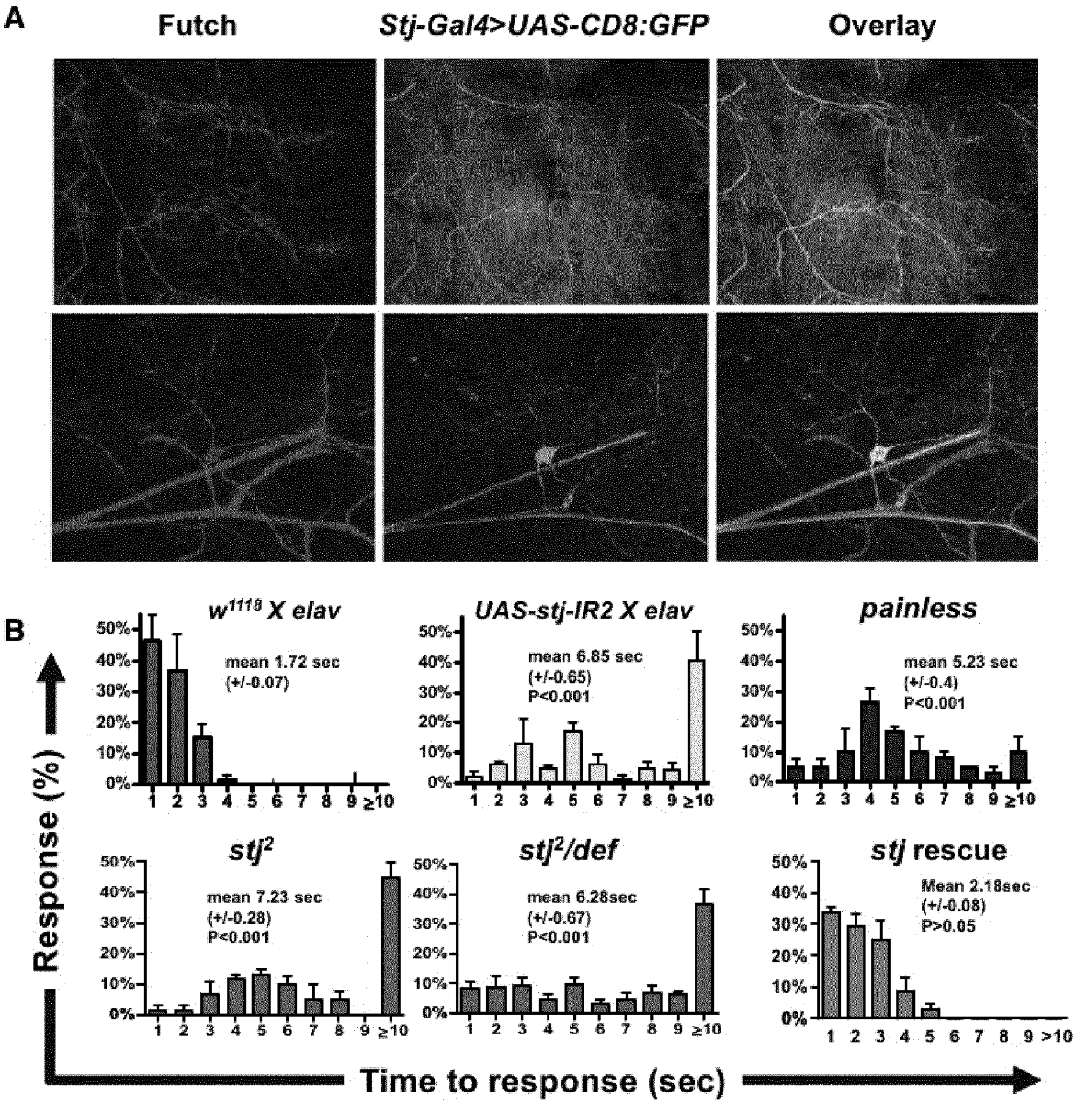
A
Futch
Stj-Gal4>UAS-CD8:GFP
Overlay
B
$w^{1118}$ X elav
mean 1.72 sec
(+/-0.07)
UAS-stj-IR2 X elav
mean 6.85 sec
(+/-0.65)
P<0.001
painless
mean 5.23 sec
(+/-0.4)
P<0.001
$stj^2$
mean 7.23 sec
(+/-0.28)
P<0.001
$stj^2$/def
mean 6.28sec
(+/-0.67)
P<0.001
stj rescue
Mean 2.18sec
(+/-0.08)
P>0.05
Response (%)
Time to response (sec)

Fig. 4

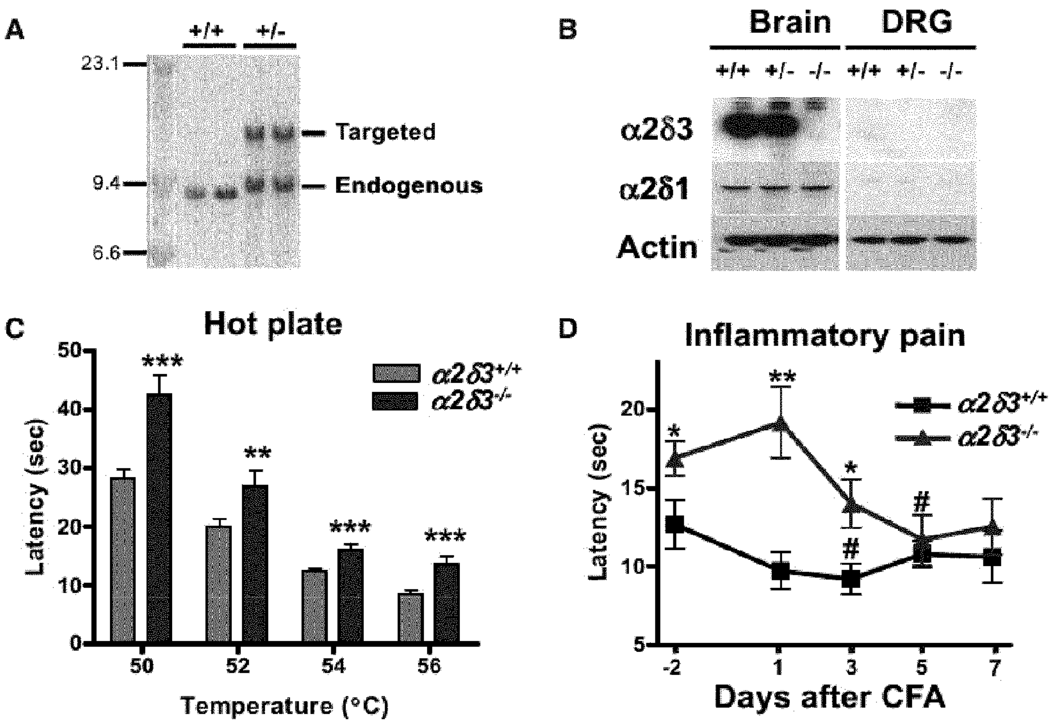
A
+/+
+/-
23.1
9.4
6.6
Targeted
Endogenous
B
Brain
DRG
+/+ +/- -/- +/+ +/- -/-
α2δ3
α2δ1
Actin
C
Hot plate
α2δ3+/+
α2δ3-/-
Latency (sec)
Temperature (°C)
D
Inflammatory pain
α2δ3+/+
α2δ3-/-
Latency (sec)
Days after CFA

Fig. 5

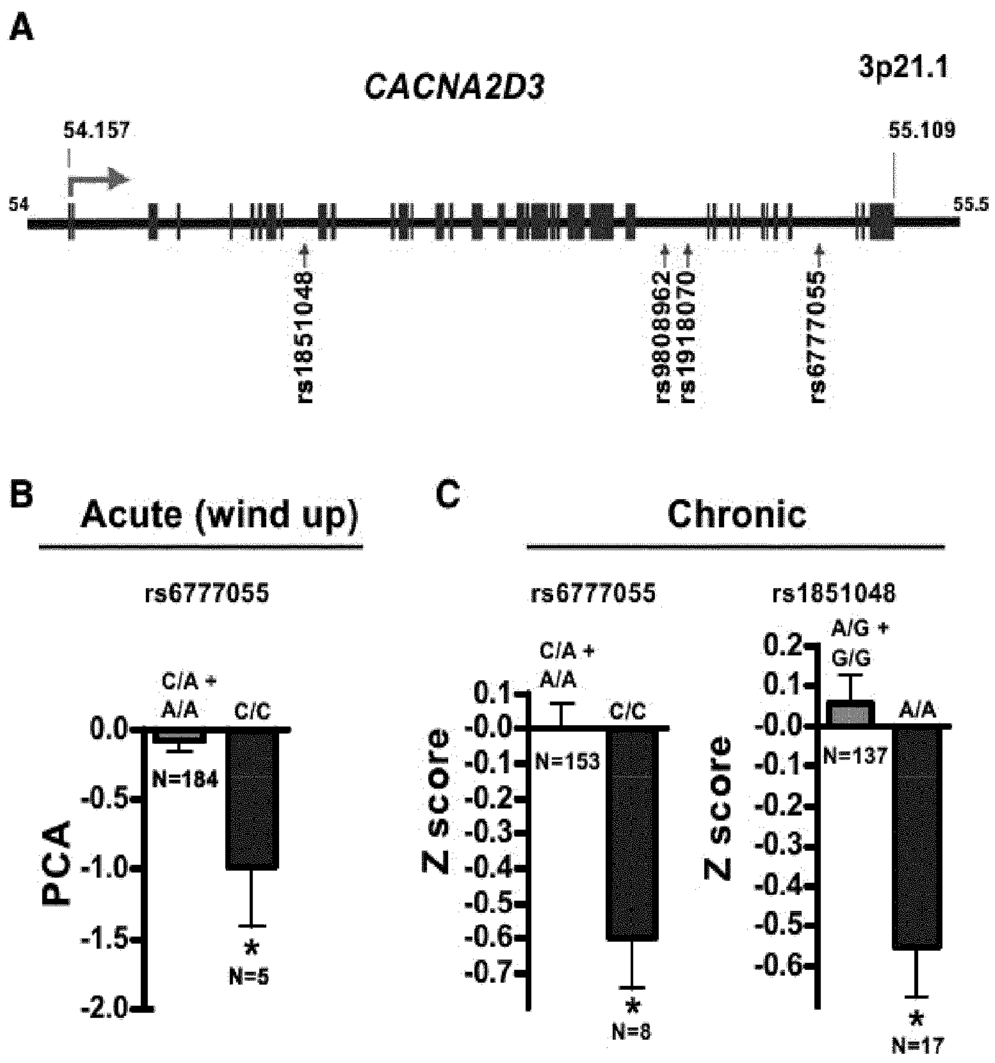

A
CACNA2D3
3p21.1
54.157
55.109
54
55.5
rs1851048
rs9808962
rs1918070
rs6777055
B
Acute (wind up)
rs6777055
C/A + A/A
C/C
N=184
N=5
PCA
0.0
-0.5
-1.0
-1.5
-2.0
C
Chronic
rs6777055
C/A + A/A
C/C
N=153
N=8
Z score
0.1
-0.0
-0.1
-0.2
-0.3
-0.4
-0.5
-0.6
-0.7
rs1851048
A/G + G/G
A/A
N=137
N=17
Z score
0.2
0.1
-0.0
-0.1
-0.2
-0.3
-0.4
-0.5
-0.6

Fig. 6

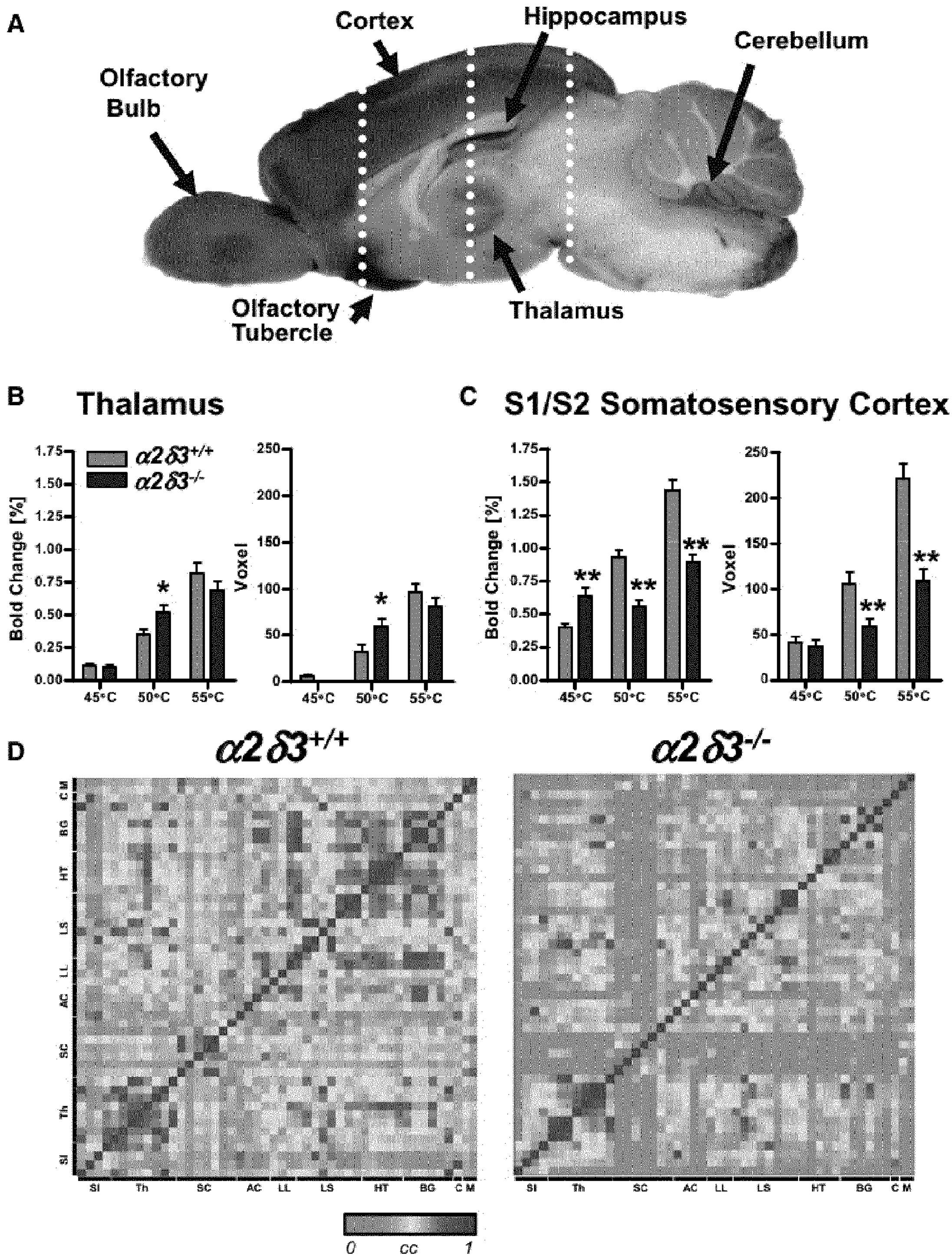
A
Cortex
Hippocampus
Cerebellum
Olfactory Bulb
Olfactory Tubercle
Thalamus
B
Thalamus
$\alpha2\delta3^{+/+}$
$\alpha2\delta3^{-/-}$
Bold Change [%]
Voxel
45°C
50°C
55°C
C
S1/S2 Somatosensory Cortex
D
$\alpha2\delta3^{+/+}$
$\alpha2\delta3^{-/-}$
SI
Th
SC
AC
LL
LS
HT
BG
C M
0
cc
1

Fig. 7

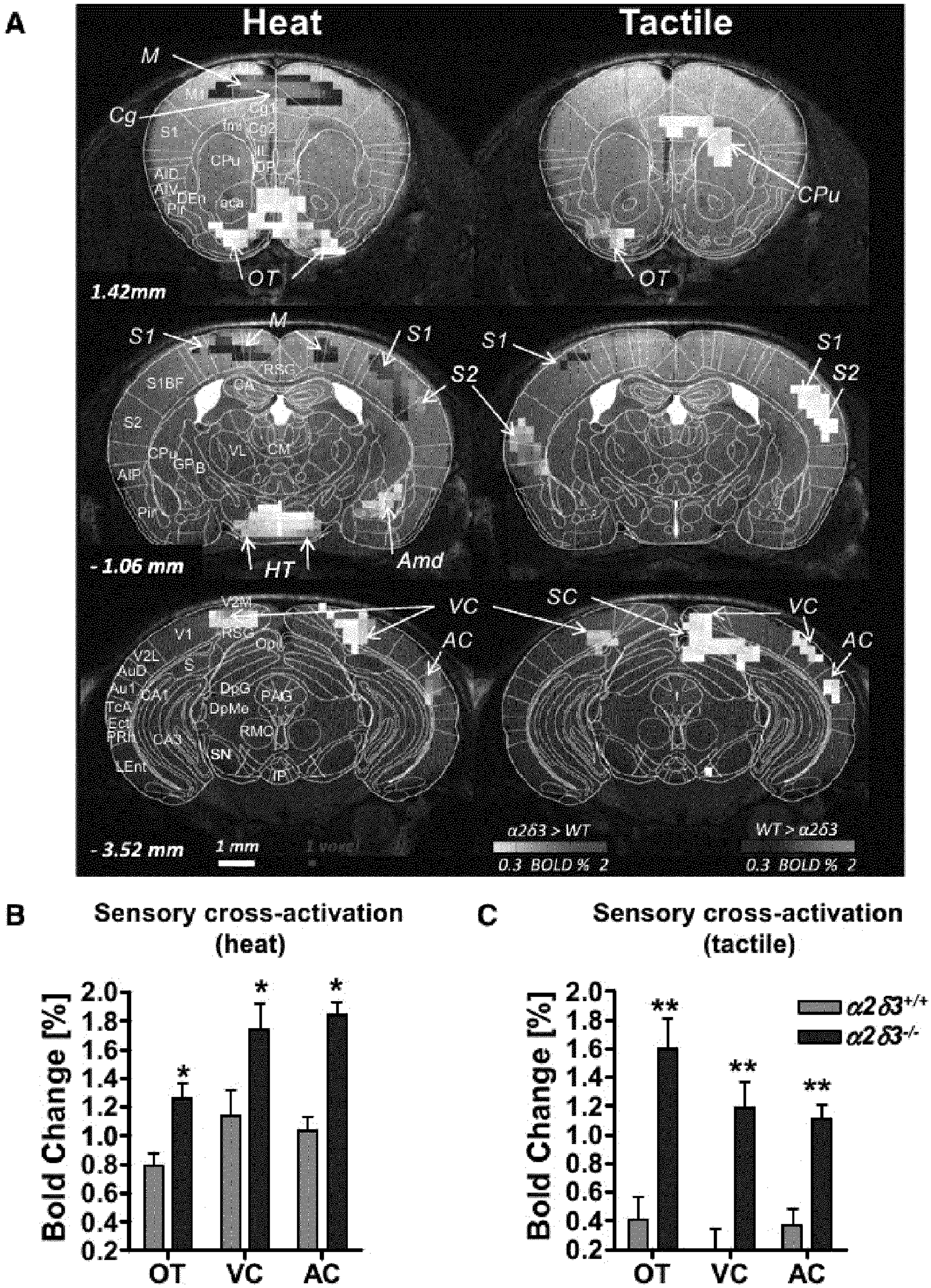
A
Heat
Tactile
M
Cg
CPu
OT
1.42mm
S1
S2
HT
Amd
- 1.06 mm
VC
SC
AC
- 3.52 mm
1 mm
α2δ3 > WT
0.3 BOLD % 2
WT > α2δ3
0.3 BOLD % 2
B
Sensory cross-activation (heat)
C
Sensory cross-activation (tactile)
Bold Change [%]
2.0
1.8
1.6
1.4
1.2
1.0
0.8
0.6
0.4
0.2
OT
VC
AC
*
**
α2δ3+/+
α2δ3-/-

Fig. 8

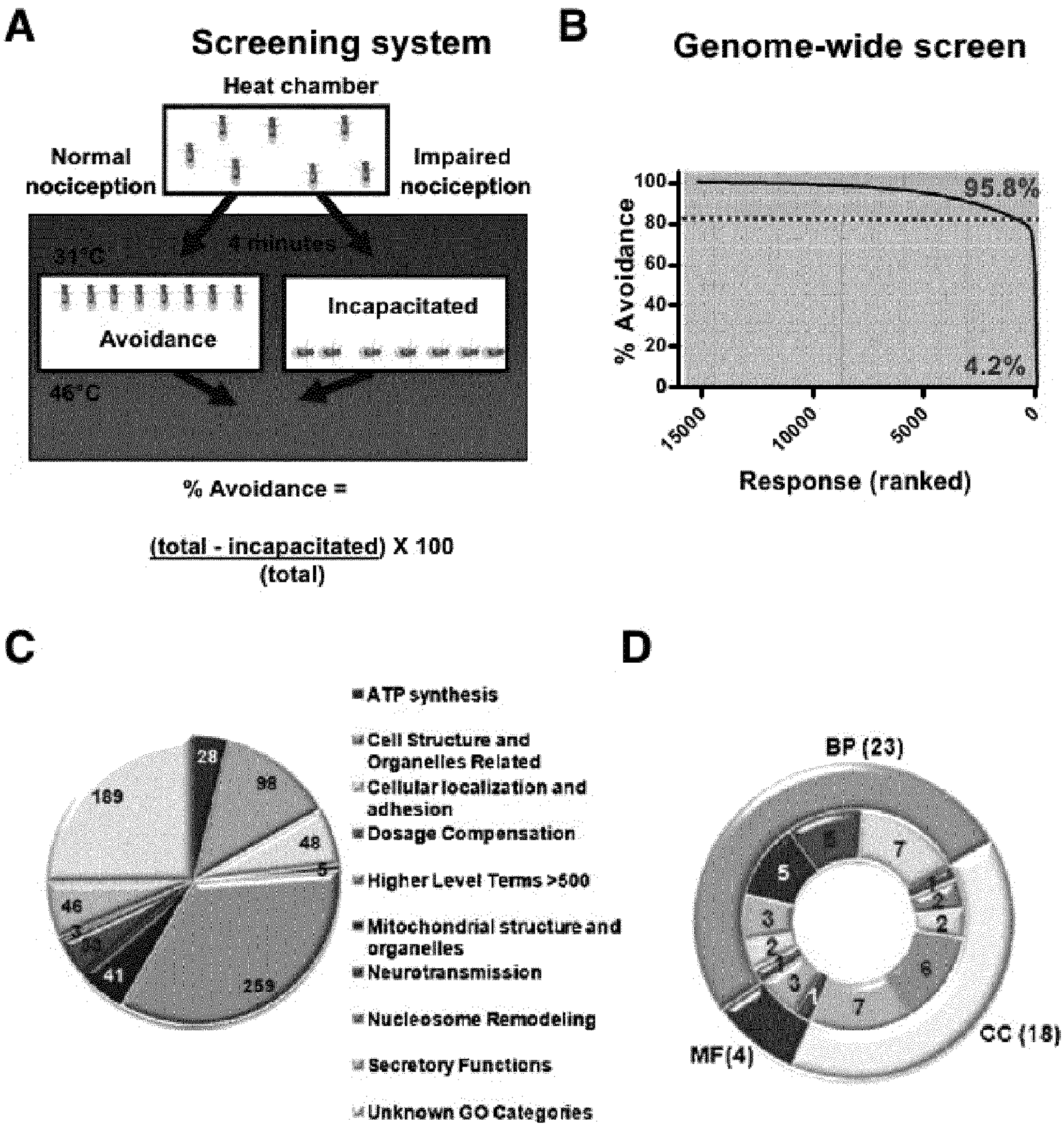
A
Screening system
Heat chamber
Normal nociception
Impaired nociception
31°C
4 minutes
Avoidance
Incapacitated
46°C
% Avoidance =
(total - incapacitated) X 100
(total)
B
Genome-wide screen
% Avoidance
100
80
60
40
20
0
95.8%
4.2%
15000
10000
5000
0
Response (ranked)
C
ATP synthesis
Cell Structure and Organelles Related
Cellular localization and adhesion
Dosage Compensation
Higher Level Terms >500
Mitochondrial structure and organelles
Neurotransmission
Nucleosome Remodeling
Secretory Functions
Unknown GO Categories
28
98
48
5
259
41
46
189
D
BP (23)
CC (18)
MF (4)

Fig. 9

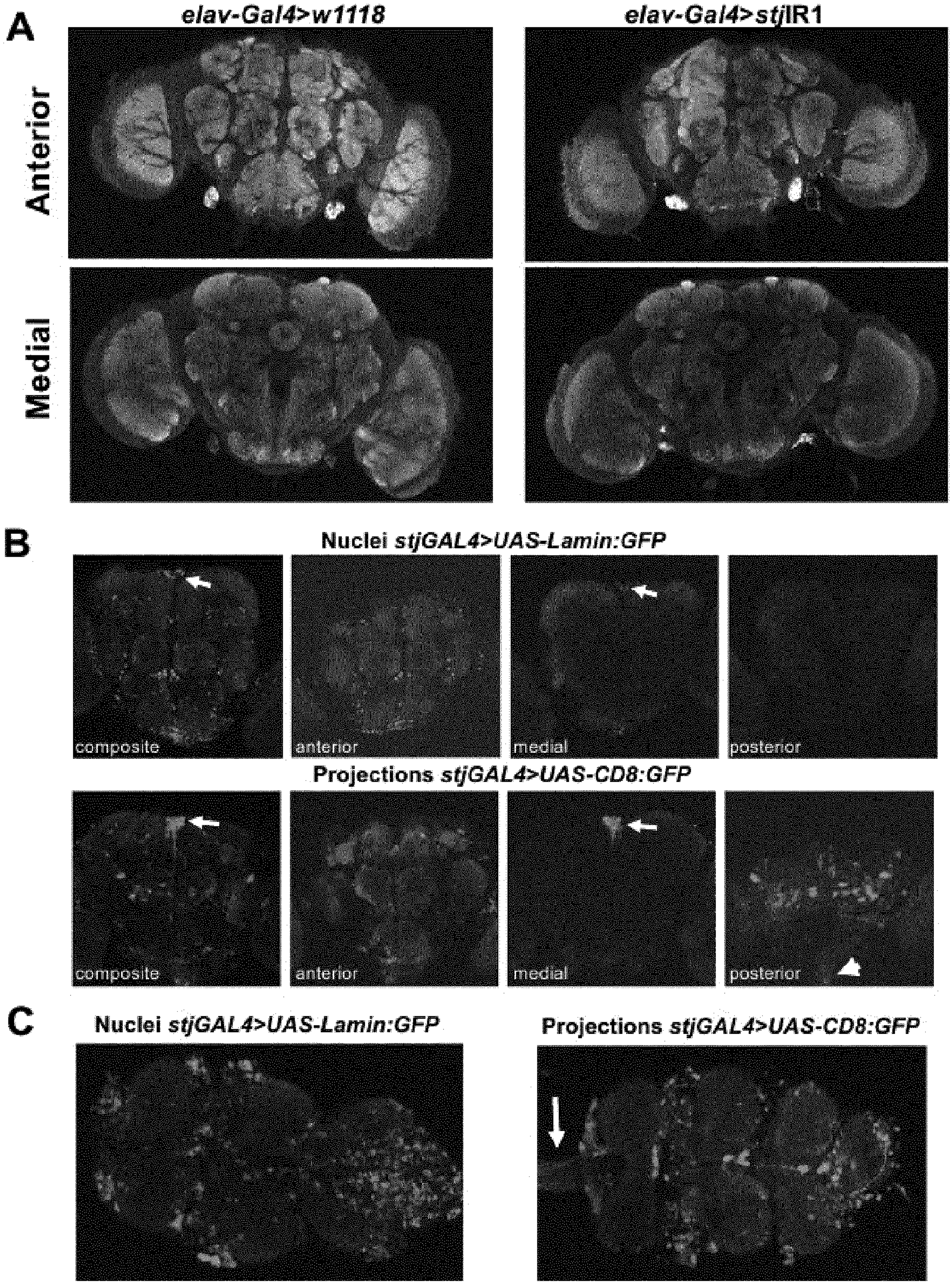
A
elav-Gal4>w1118
elav-Gal4>stjIR1
Anterior
Medial
B
Nuclei stjGAL4>UAS-Lamin:GFP
composite
anterior
medial
posterior
Projections stjGAL4>UAS-CD8:GFP
composite
anterior
medial
posterior
C
Nuclei stjGAL4>UAS-Lamin:GFP
Projections stjGAL4>UAS-CD8:GFP

Fig. 10

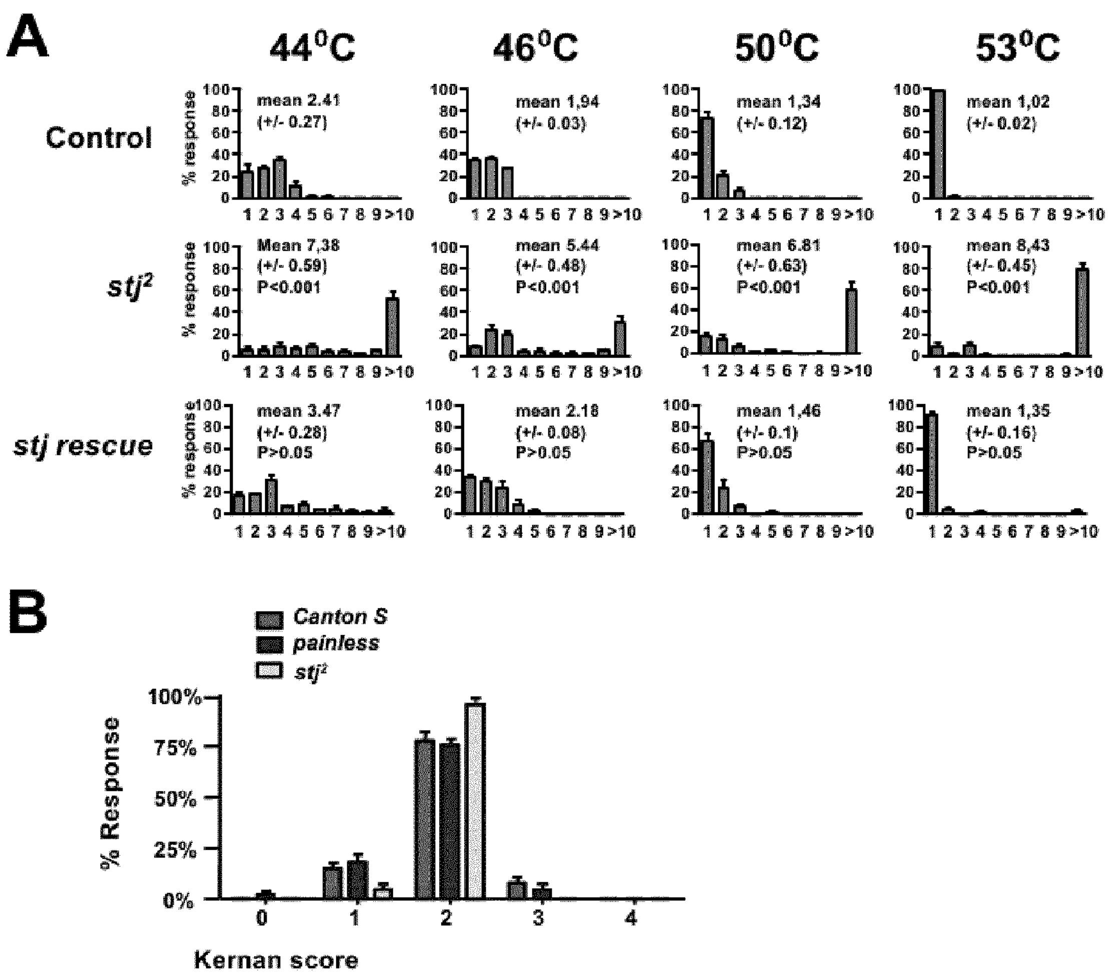
A
44°C
46°C
50°C
53°C
Control
mean 2.41
(+/- 0.27)
mean 1,94
(+/- 0.03)
mean 1,34
(+/- 0.12)
mean 1,02
(+/- 0.02)
stj2
Mean 7,38
(+/- 0.59)
P<0.001
mean 5.44
(+/- 0.48)
P<0.001
mean 6.81
(+/- 0.63)
P<0.001
mean 8,43
(+/- 0.45)
P<0.001
stj rescue
mean 3.47
(+/- 0.28)
P>0.05
mean 2.18
(+/- 0.08)
P>0.05
mean 1,46
(+/- 0.1)
P>0.05
mean 1,35
(+/- 0.16)
P>0.05
% response
1 2 3 4 5 6 7 8 9 >10
B
Canton S
painless
stj2
% Response
100%
75%
50%
25%
0%
0 1 2 3 4
Kernan score

Fig. 11

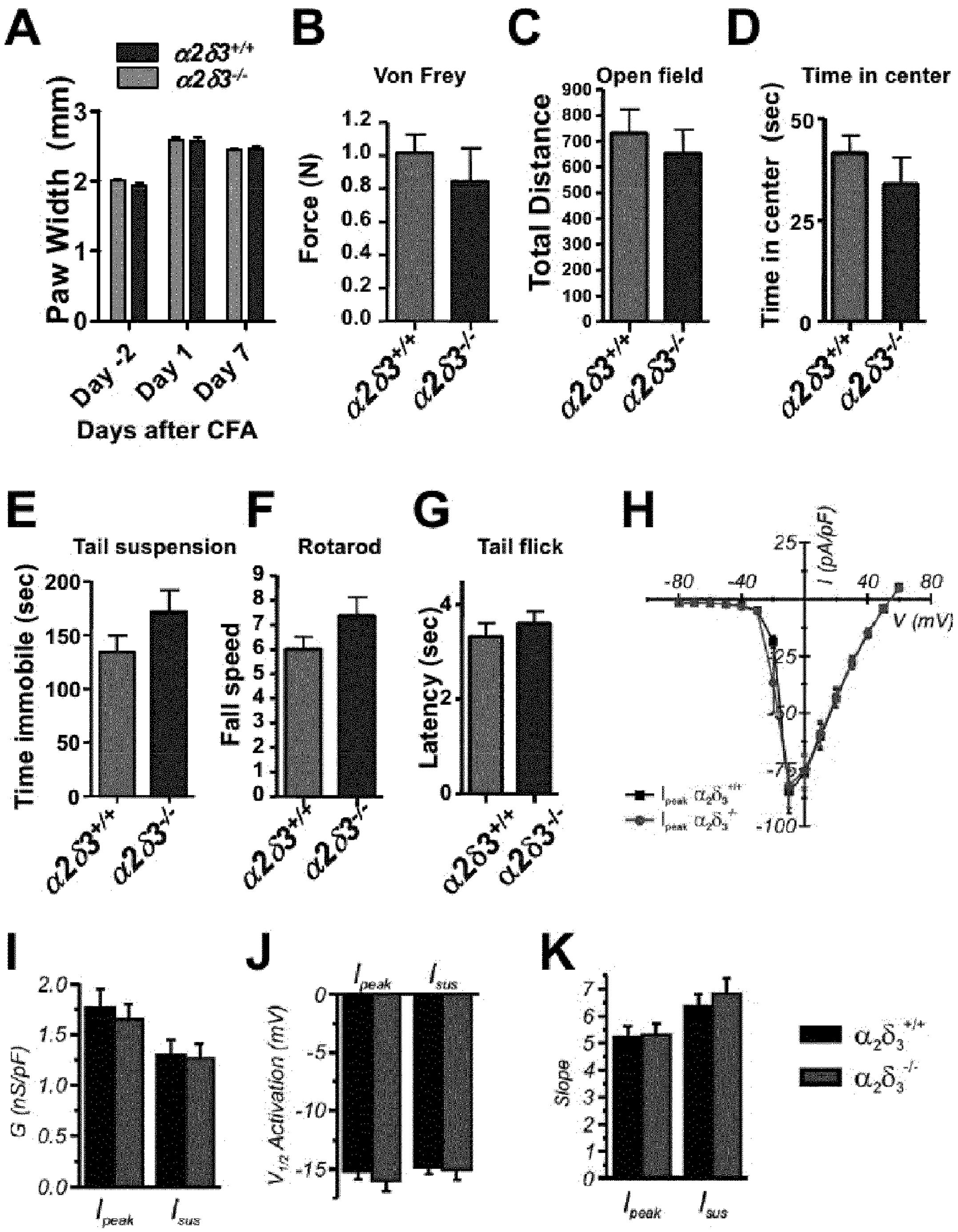
A
α2δ3+/+
α2δ3-/-
Paw Width (mm)
Day -2
Day 1
Day 7
Days after CFA
B
Von Frey
Force (N)
C
Open field
Total Distance
D
Time in center
Time in center (sec)
E
Tail suspension
Time immobile (sec)
F
Rotarod
Fall speed
G
Tail flick
Latency (sec)
H
I (pA/pF)
V (mV)
I
G (nS/pF)
J
V1/2 Activation (mV)
K
Slope
Ipeak
Isus

Fig. 12

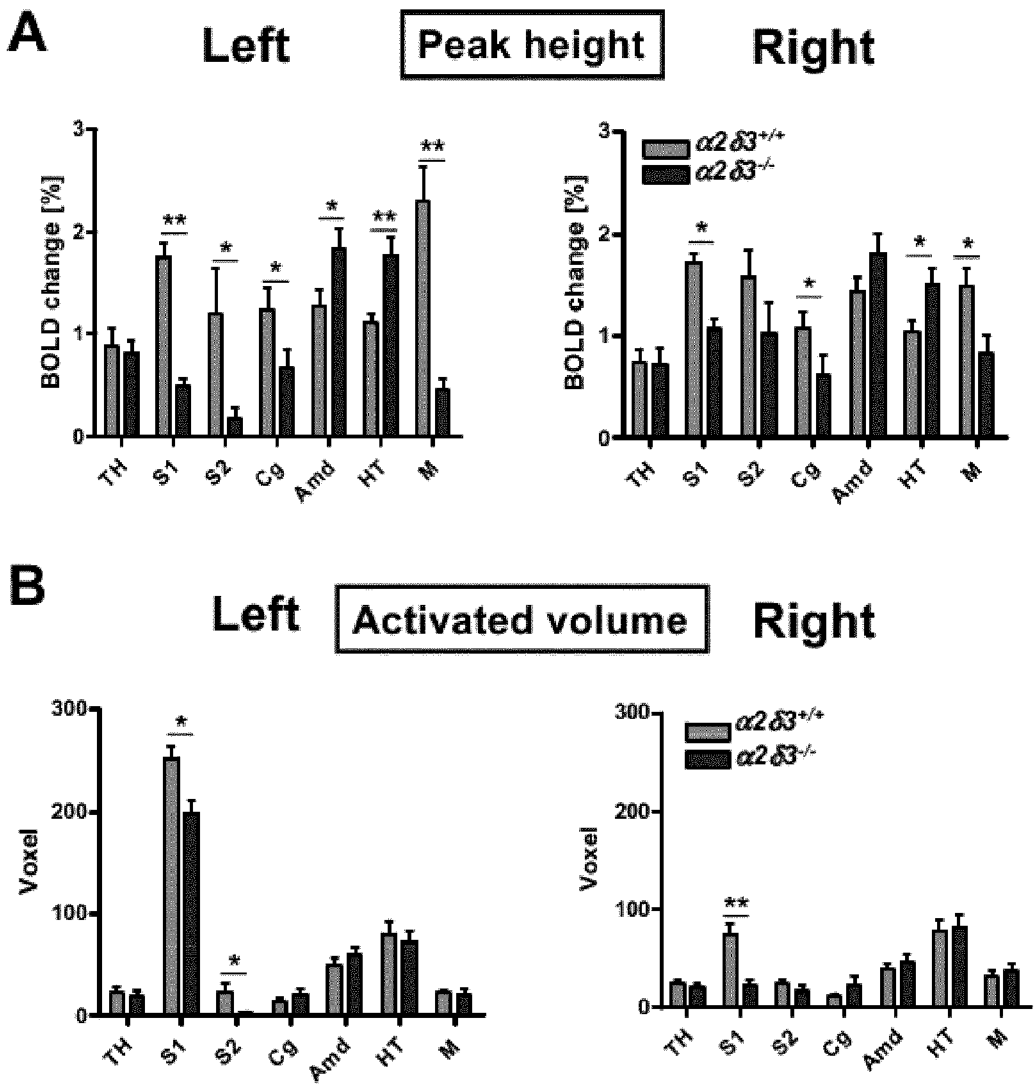
A
Left
Peak height
Right
BOLD change [%]
α2δ3+/+
α2δ3-/-
TH
S1
S2
Cg
Amd
HT
M
B
Left
Activated volume
Right
Voxel

Fig. 13

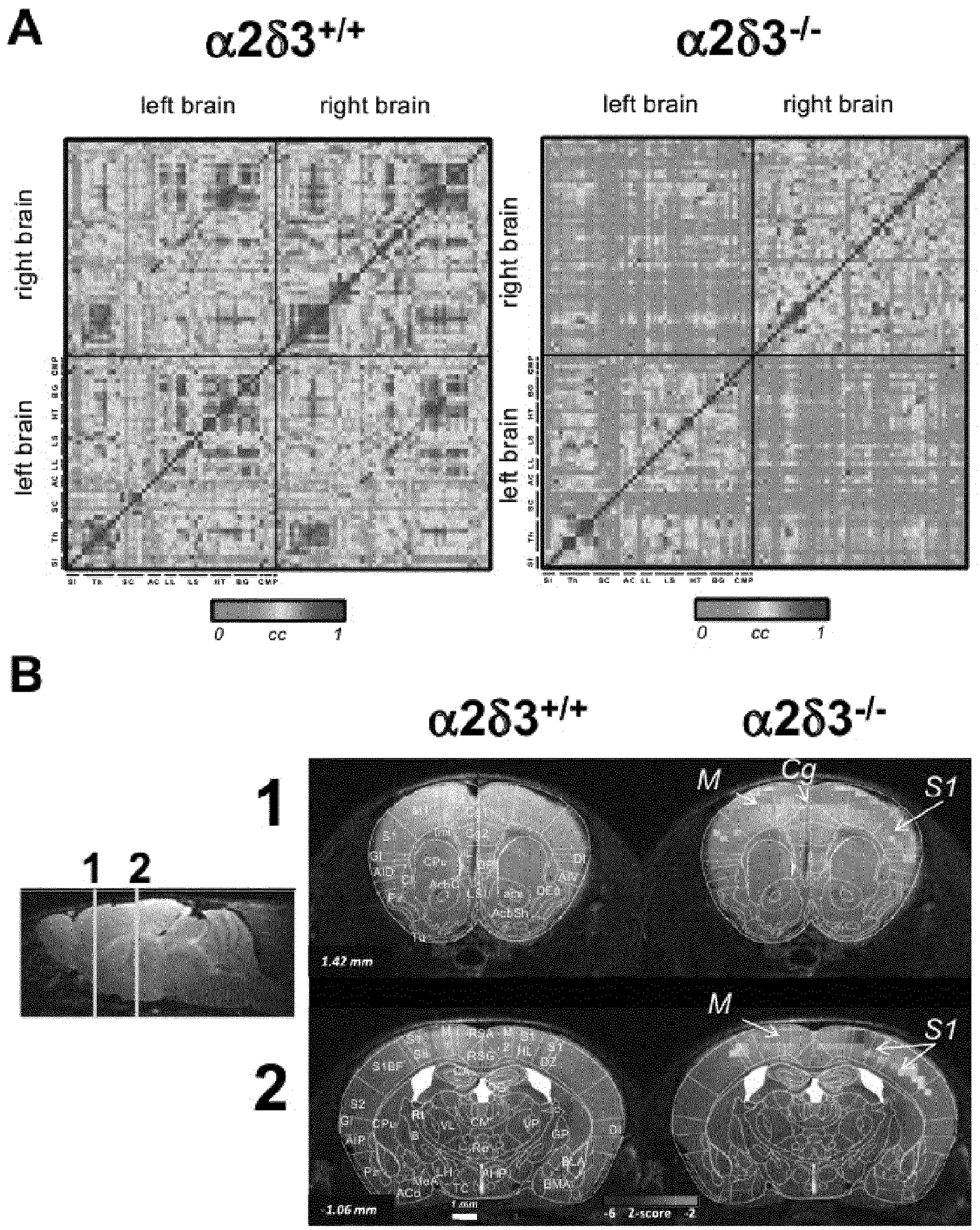
A
α2δ3+/+
α2δ3-/-
left brain
right brain
left brain
right brain
right brain
left brain
right brain
left brain
SI Th SC AC LL LS HT BG CMP
0 cc 1
0 cc 1
B
α2δ3+/+
α2δ3-/-
1
2
1 2
M
Cg
S1
M
S1
1.42 mm
-1.06 mm
1 mm
-6 Z-score -2

Fig. 14

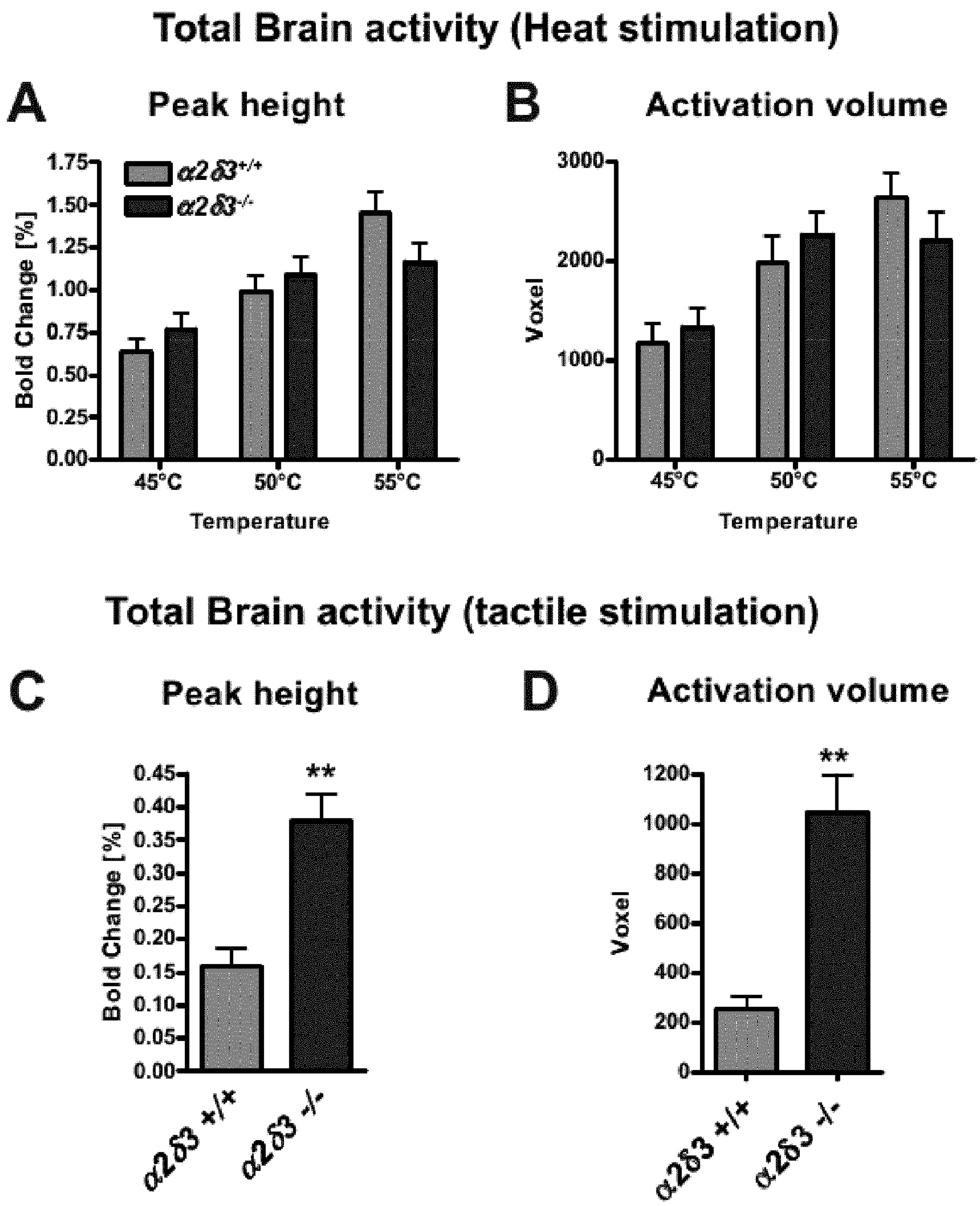
Total Brain activity (Heat stimulation)
A
Peak height
α2δ3+/+
α2δ3-/-
Bold Change [%]
1.75
1.50
1.25
1.00
0.75
0.50
0.25
0.00
45°C
50°C
55°C
Temperature
B
Activation volume
Voxel
3000
2000
1000
0
45°C
50°C
55°C
Temperature
Total Brain activity (tactile stimulation)
C
Peak height
Bold Change [%]
0.45
0.40
0.35
0.30
0.25
0.20
0.15
0.10
0.05
0.00
**
α2δ3 +/+
α2δ3 -/-
D
Activation volume
Voxel
1200
1000
800
600
400
200
0
**
α2δ3 +/+
α2δ3 -/-

Fig. 15

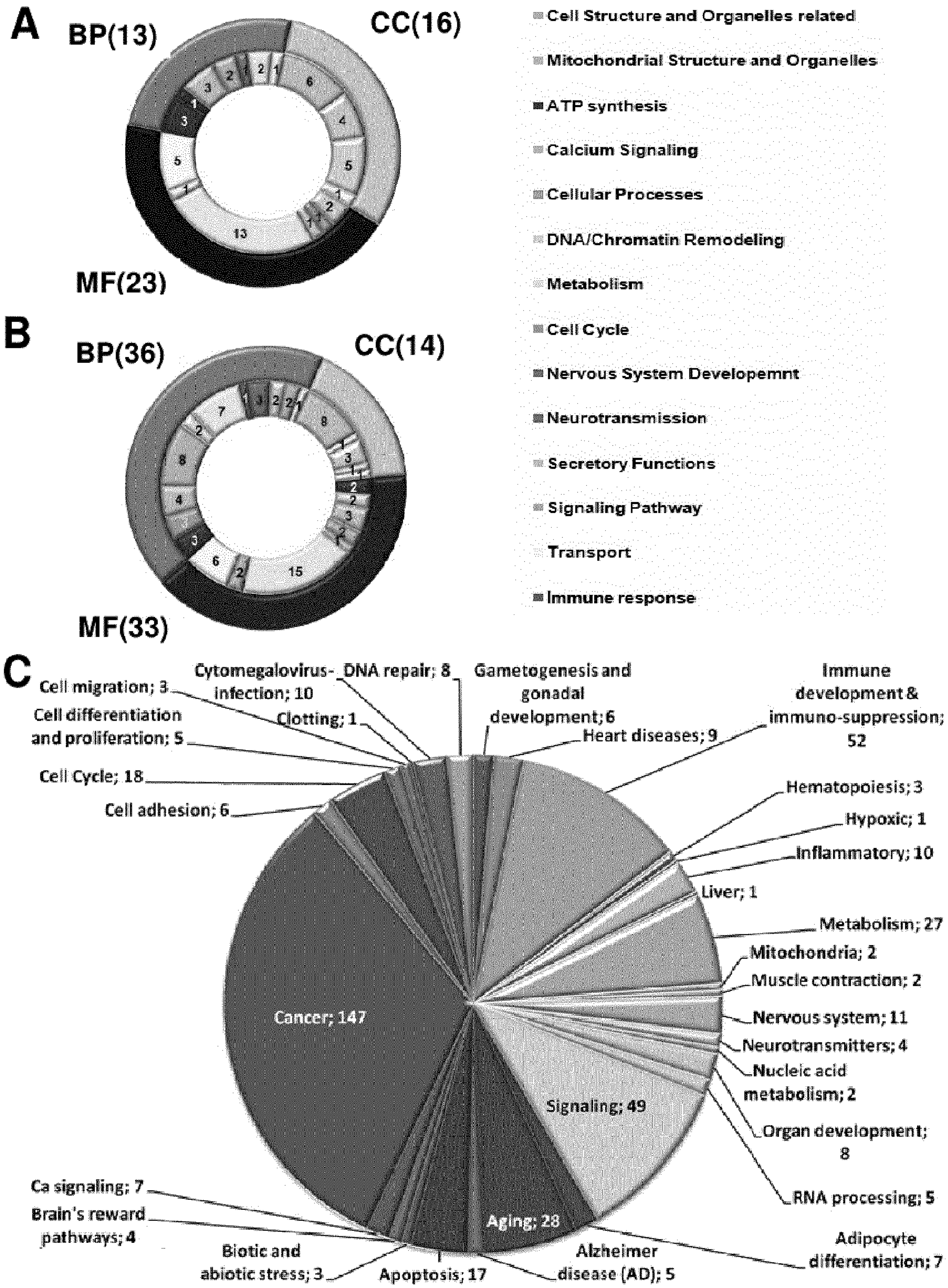
A
BP(13)
CC(16)
MF(23)
B
BP(36)
CC(14)
MF(33)
Cell Structure and Organelles related
Mitochondrial Structure and Organelles
ATP synthesis
Calcium Signaling
Cellular Processes
DNA/Chromatin Remodeling
Metabolism
Cell Cycle
Nervous System Developemnt
Neurotransmission
Secretory Functions
Signaling Pathway
Transport
Immune response
C
Cell migration; 3
Cytomegalovirus-infection; 10
DNA repair; 8
Gametogenesis and gonadal development; 6
Immune development & immuno-suppression; 52
Cell differentiation and proliferation; 5
Clotting; 1
Heart diseases; 9
Cell Cycle; 18
Hematopoiesis; 3
Cell adhesion; 6
Hypoxic; 1
Inflammatory; 10
Liver; 1
Metabolism; 27
Mitochondria; 2
Muscle contraction; 2
Cancer; 147
Nervous system; 11
Neurotransmitters; 4
Nucleic acid metabolism; 2
Signaling; 49
Organ development; 8
RNA processing; 5
Ca signaling; 7
Brain's reward pathways; 4
Aging; 28
Adipocyte differentiation; 7
Biotic and abiotic stress; 3
Apoptosis; 17
Alzheimer disease (AD); 5

Fig. 16

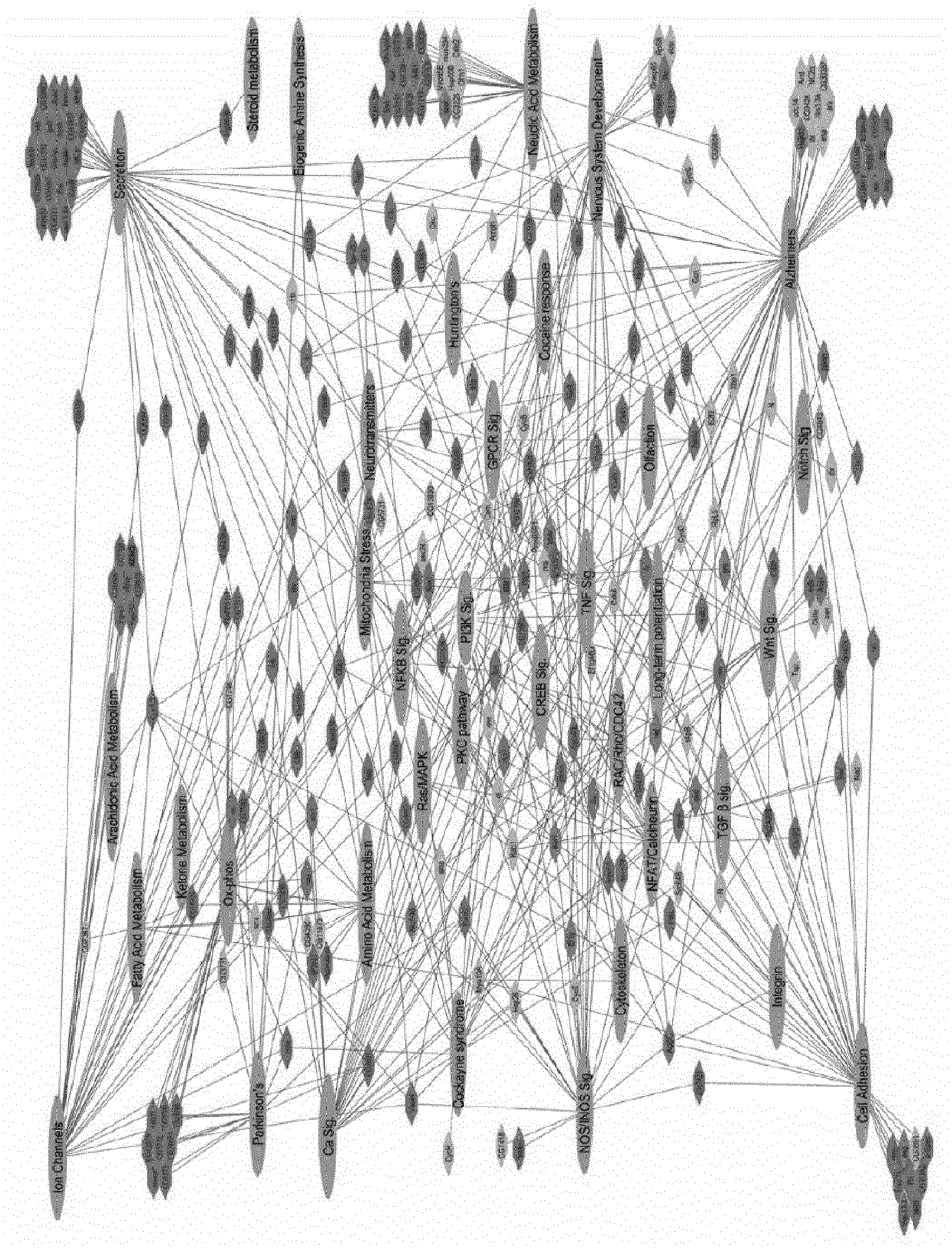
Secretion
Steroid metabolism
Biogenic Amine Synthesis
Neucleic Acid Metabolism
Nervous System Development
Alzheimers
Huntington's
Cocaine response
Neurotransmitters
GPCR Sig.
Olfaction
Notch Sig.
Mitochondria Stress
NFKB Sig.
PI3K Sig.
TNF Sig.
Long-term potentiation
Wnt Sig.
Arachidonic Acid Metabolism
PKC pathway
CREB Sig.
RAC/Rho/CDC42
Ras/MAPK
Ketone Metabolism
Fatty Acid Metabolism
Ox-phos
Amino Acid Metabolism
NFAT/Calcineurin
TGF β sig.
Cytoskeleton
Integrin
Cockayne syndrome
Ion Channels
Parkinson's
Ca Sig.
NOS/iNOS Sig.
Cell Adhesion

Fig. 17

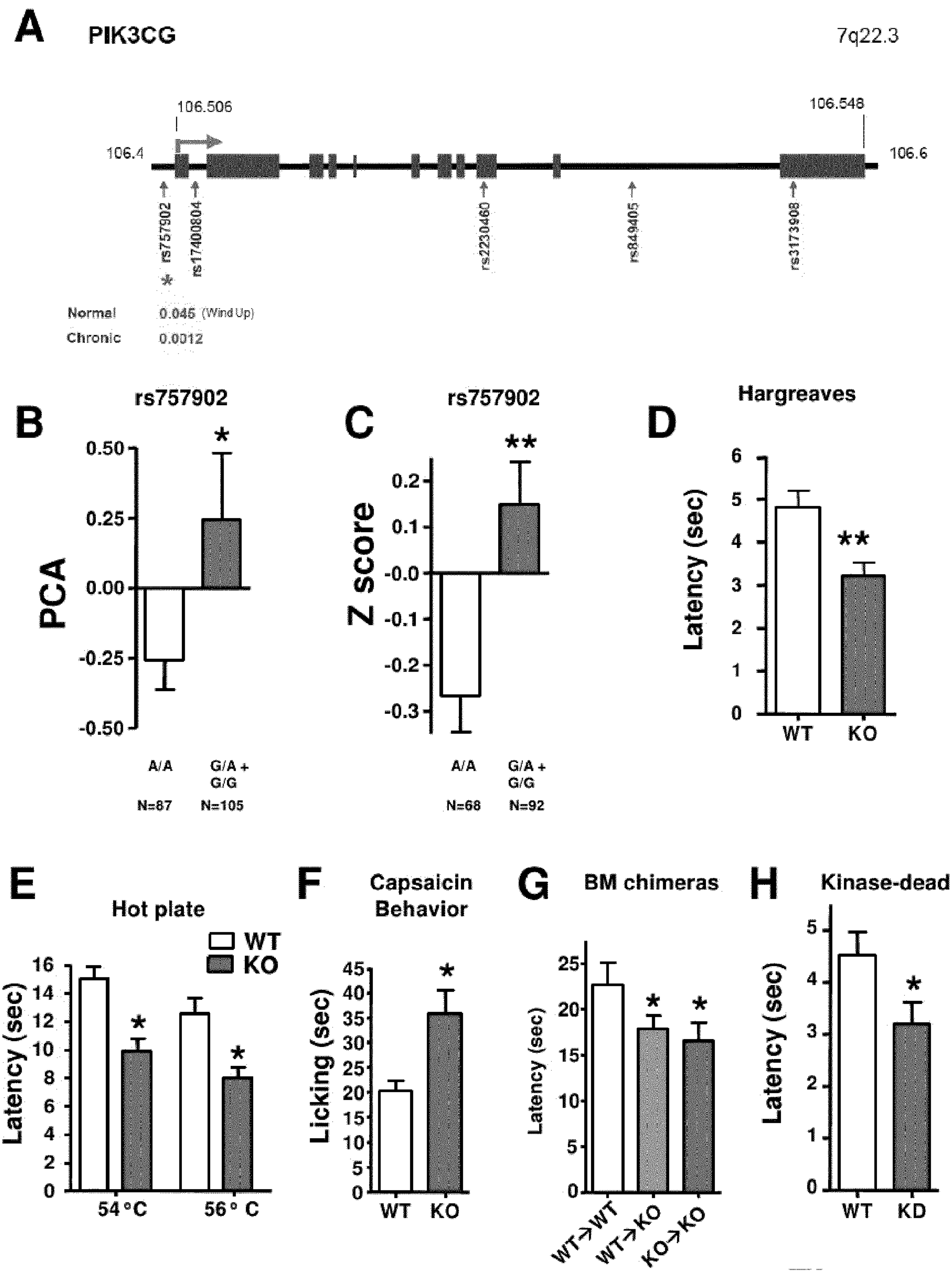
A
PIK3CG
7q22.3
106.506
106.548
106.4
106.6
rs757902
rs17400804
rs2230460
rs849405
rs3173908
*
Normal 0.045 (Wind Up)
Chronic 0.0012
B
rs757902
PCA
0.50
0.25
0.00
-0.25
-0.50
*
A/A
G/A + G/G
N=87
N=105
C
rs757902
Z score
0.2
0.1
-0.0
-0.1
-0.2
-0.3
**
A/A
G/A + G/G
N=68
N=92
D
Hargreaves
Latency (sec)
WT
KO
**
E
Hot plate
WT
KO
Latency (sec)
54 °C
56 ° C
F
Capsaicin Behavior
Licking (sec)
WT
KO
G
BM chimeras
Latency (sec)
WT->WT
WT->KO
KO->KO
H
Kinase-dead
Latency (sec)
WT
KD

Fig. 18

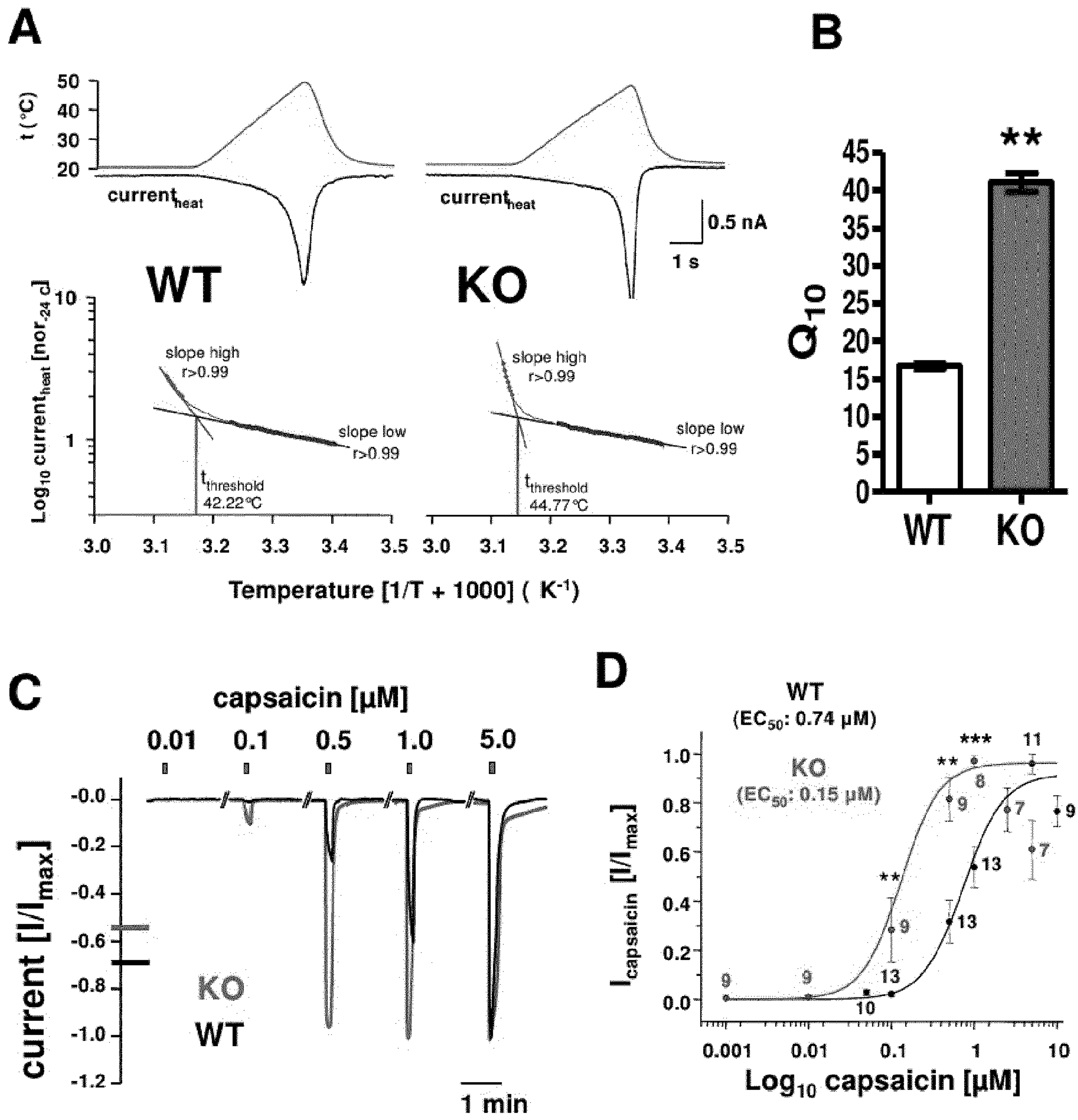
A
B
t (°C)
50
40
30
20
current$_{heat}$
0.5 nA
1 s
WT
KO
Log$_{10}$ current$_{heat}$ [nor$_{-24}$ c]
slope high
r>0.99
slope low
r>0.99
t$_{threshold}$
42.22°C
44.77°C
Temperature [1/T + 1000] ( K$^{-1}$)
Q$_{10}$
**
C
capsaicin [µM]
0.01
0.1
0.5
1.0
5.0
current [I/I$_{max}$]
1 min
D
WT
(EC$_{50}$: 0.74 µM)
KO
(EC$_{50}$: 0.15 µM)
I$_{capsaicin}$ [I/I$_{max}$]
Log$_{10}$ capsaicin [µM]

Fig. 19

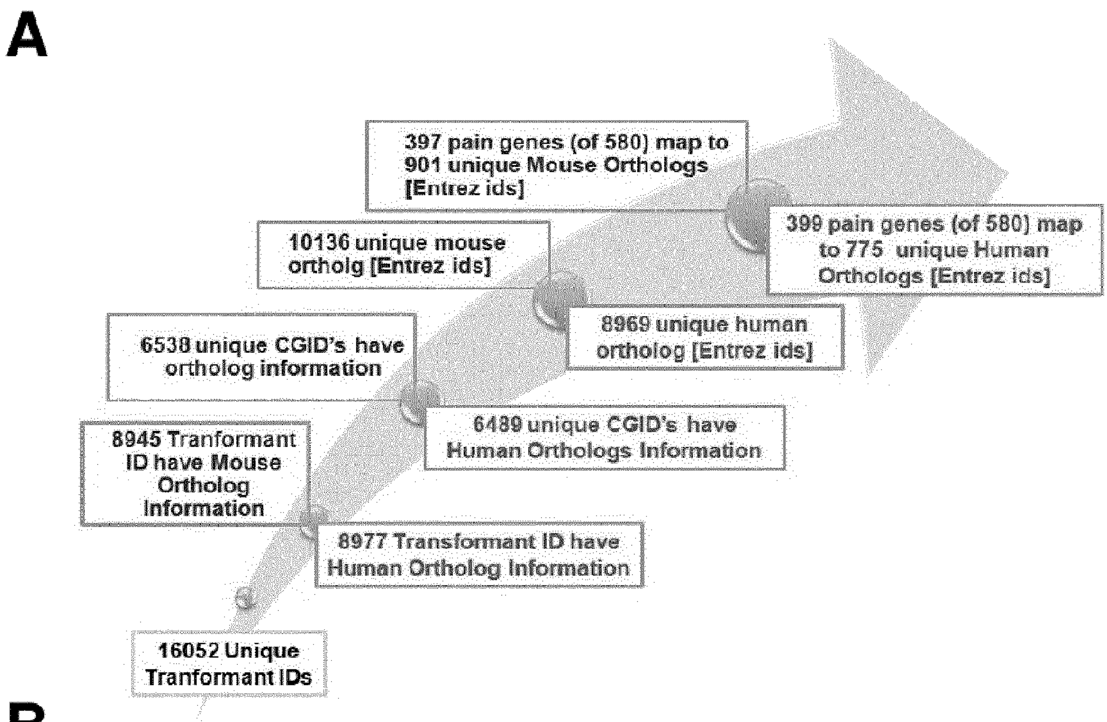
A
16052 Unique Tranformant IDs
8977 Transformant ID have Human Ortholog Information
8945 Tranformant ID have Mouse Ortholog Information
6489 unique CGID's have Human Orthologs Information
6538 unique CGID's have ortholog information
8969 unique human ortholog [Entrez ids]
10136 unique mouse ortholg [Entrez ids]
399 pain genes (of 580) map to 775 unique Human Orthologs [Entrez ids]
397 pain genes (of 580) map to 901 unique Mouse Orthologs [Entrez ids]

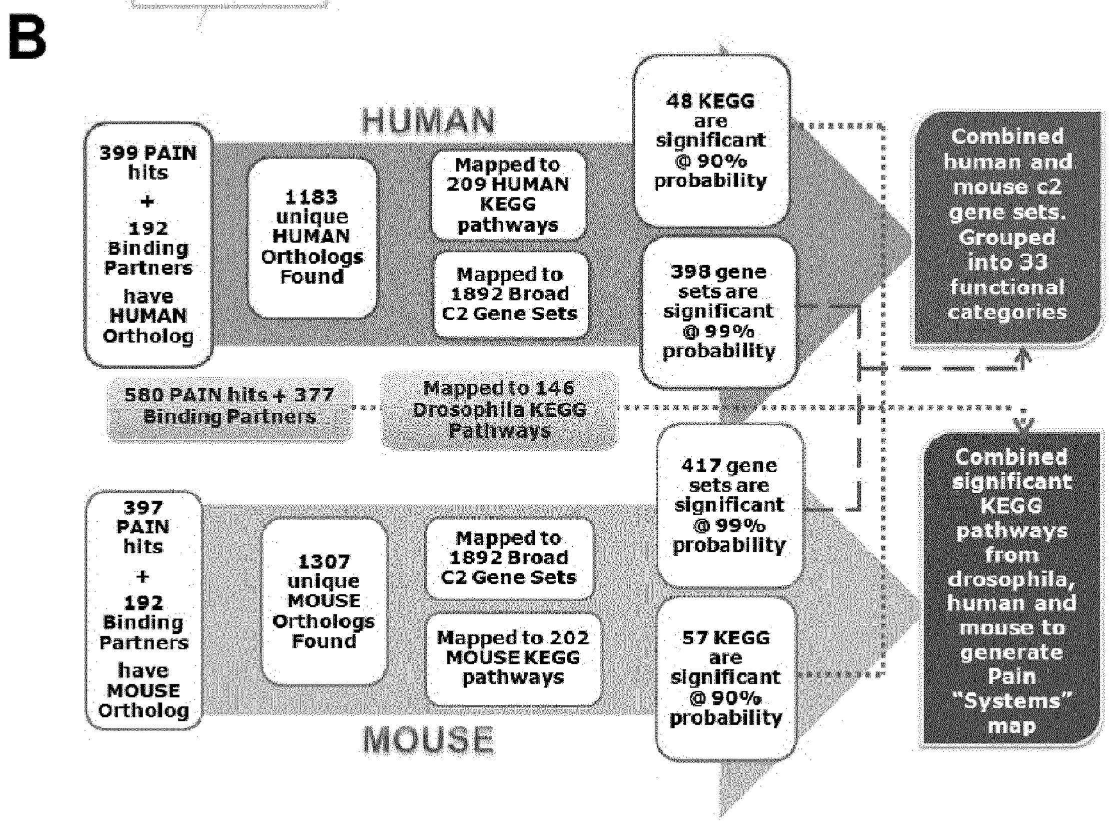
B
HUMAN
399 PAIN hits + 192 Binding Partners have HUMAN Ortholog
1183 unique HUMAN Orthologs Found
Mapped to 209 HUMAN KEGG pathways
Mapped to 1892 Broad C2 Gene Sets
48 KEGG are significant @ 90% probability
398 gene sets are significant @ 99% probability
Combined human and mouse c2 gene sets. Grouped into 33 functional categories
580 PAIN hits + 377 Binding Partners
Mapped to 146 Drosophila KEGG Pathways
397 PAIN hits + 192 Binding Partners have MOUSE Ortholog
1307 unique MOUSE Orthologs Found
Mapped to 1892 Broad C2 Gene Sets
Mapped to 202 MOUSE KEGG pathways
417 gene sets are significant @ 99% probability
57 KEGG are significant @ 90% probability
Combined significant KEGG pathways from drosophila, human and mouse to generate Pain "Systems" map
MOUSE

Fig. 20

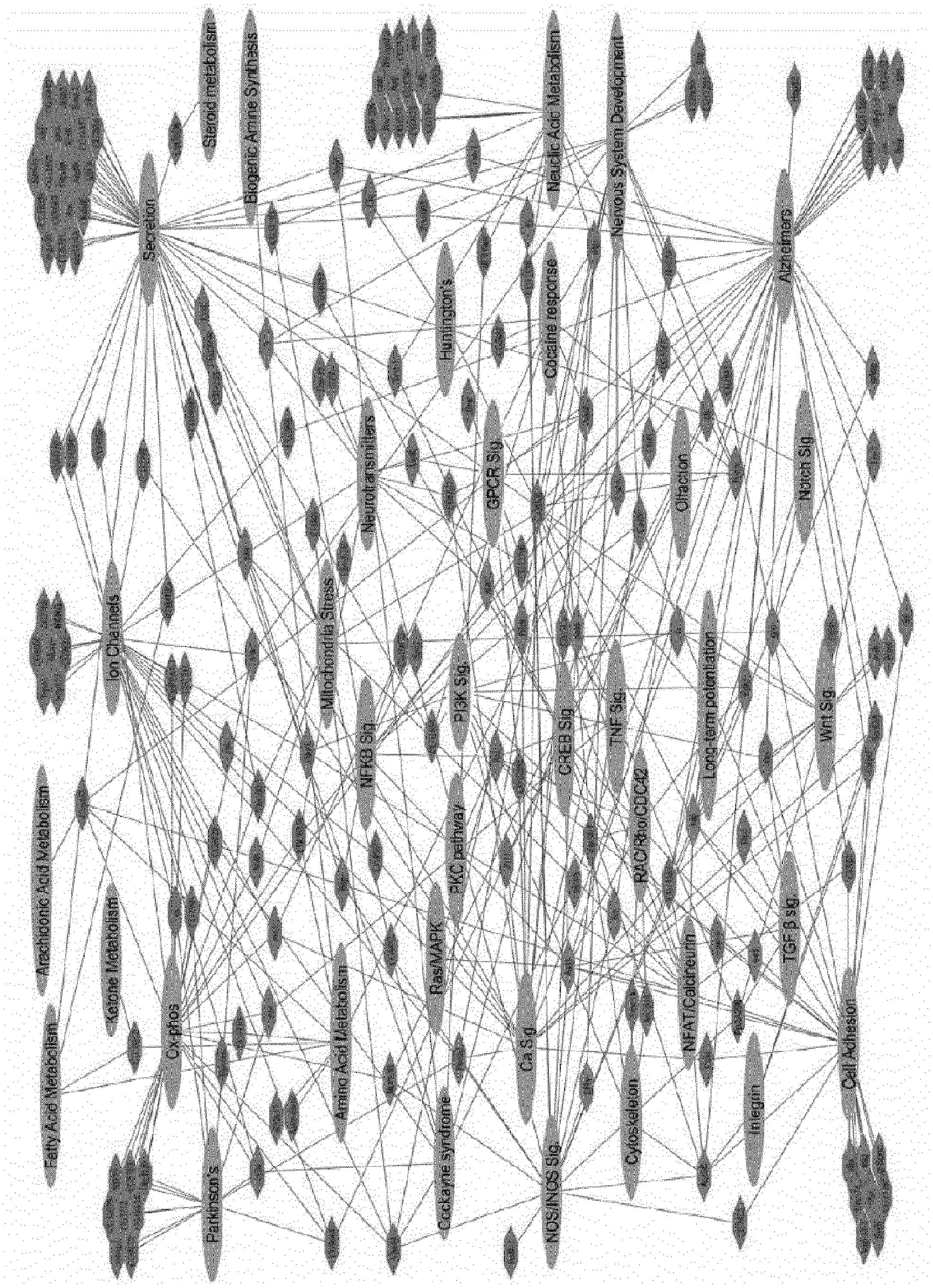
Secretion
Steroid metabolism
Biogenic Amine Synthesis
Nucleic Acid Metabolism
Nervous System Development
Alzheimers
Huntington's
Cocaine response
Neurotransmitters
GPCR Sig
Olfaction
Notch Sig
Ion Channels
Mitochondria Stress
PI3K Sig
CREB Sig
TNF Sig
Long-term potentiation
Wnt Sig
NFKB Sig
Arachidonic Acid Metabolism
PKC pathway
RAC/Rho/CDC42
Ketone Metabolism
Ras/MAPK
NFAT/Calcineurin
TGF β Sig
Ox phos
Amino Acid Metabolism
Ca Sig
Cell Adhesion
Fatty Acid Metabolism
Cockayne syndrome
NOS/iNOS Sig
Cytoskeleton
Integrin
Parkinson's

Fig. 21

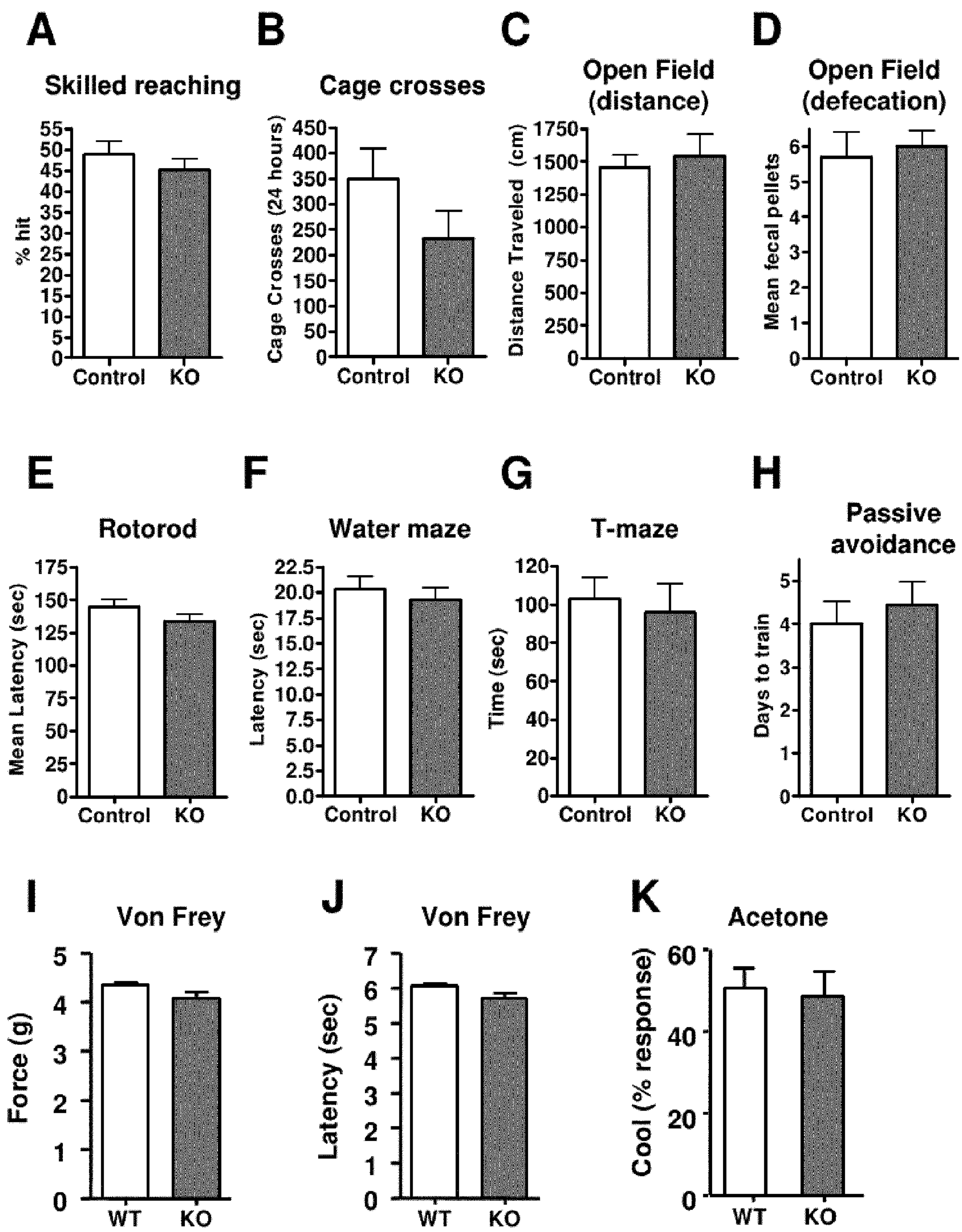
A
Skilled reaching
% hit
0
5
10
15
20
25
30
35
40
45
50
55
Control
KO
B
Cage crosses
Cage Crosses (24 hours)
0
50
100
150
200
250
300
350
400
450
Control
KO
C
Open Field (distance)
Distance Traveled (cm)
0
250
500
750
1000
1250
1500
1750
Control
KO
D
Open Field (defecation)
Mean fecal pellets
0
1
2
3
4
5
6
Control
KO
E
Rotorod
Mean Latency (sec)
0.0
25
50
75
100
125
150
175
Control
KO
F
Water maze
Latency (sec)
0.0
2.5
5.0
7.5
10.0
12.5
15.0
17.5
20.0
22.5
Control
KO
G
T-maze
Time (sec)
0
20
40
60
80
100
120
Control
KO
H
Passive avoidance
Days to train
0
1
2
3
4
5
Control
KO
I
Von Frey
Force (g)
0
1
2
3
4
5
WT
KO
J
Von Frey
Latency (sec)
0
1
2
3
4
5
6
7
WT
KO
K
Acetone
Cool (% response)
0
20
40
60
WT
KO

Fig. 22

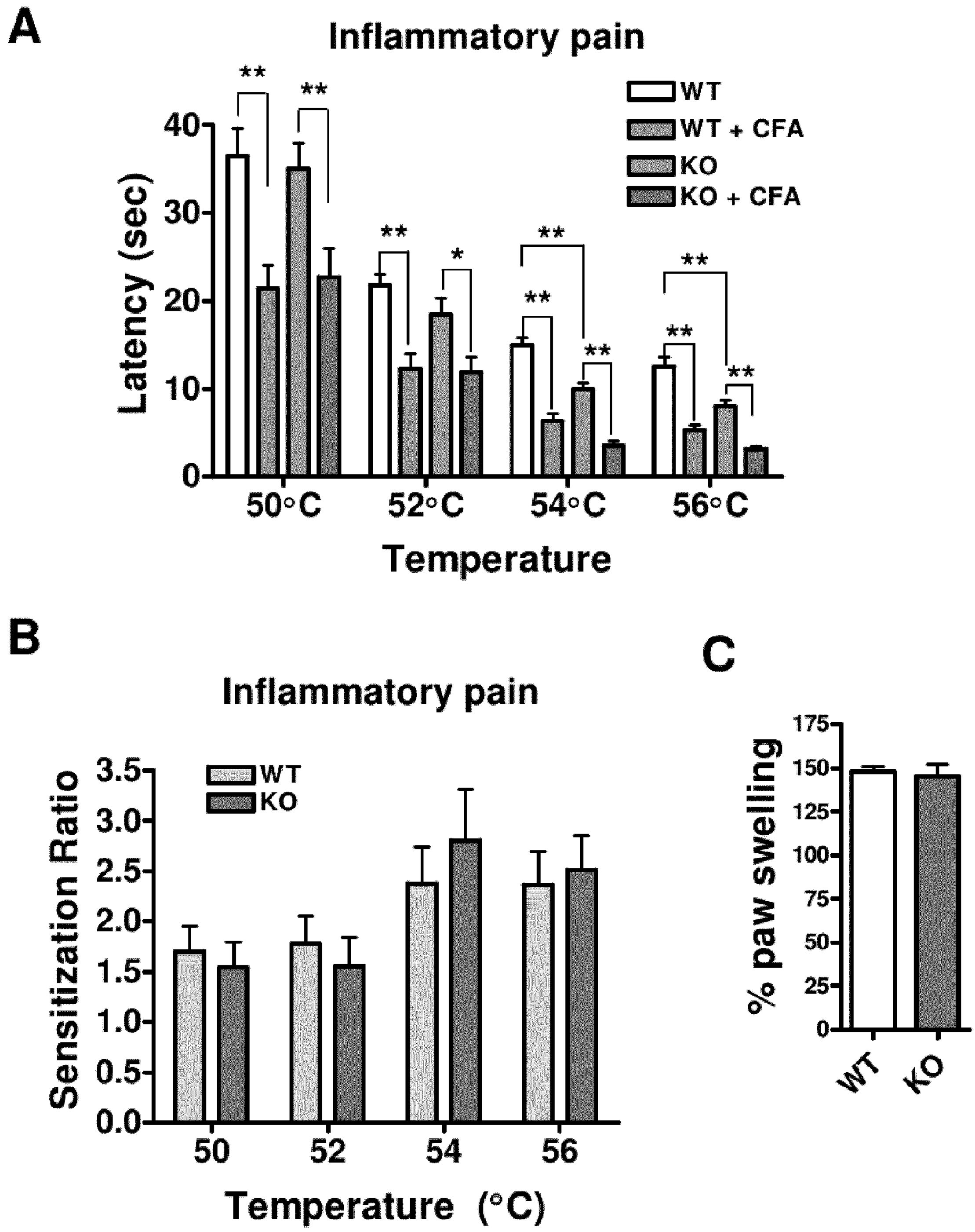
A
Inflammatory pain
WT
WT + CFA
KO
KO + CFA
Latency (sec)
0
10
20
30
40
**
*
50°C
52°C
54°C
56°C
Temperature
B
Inflammatory pain
WT
KO
Sensitization Ratio
0.0
0.5
1.0
1.5
2.0
2.5
3.0
3.5
50
52
54
56
Temperature (°C)
C
% paw swelling
0
25
50
75
100
125
150
175
WT
KO

Fig. 23 A

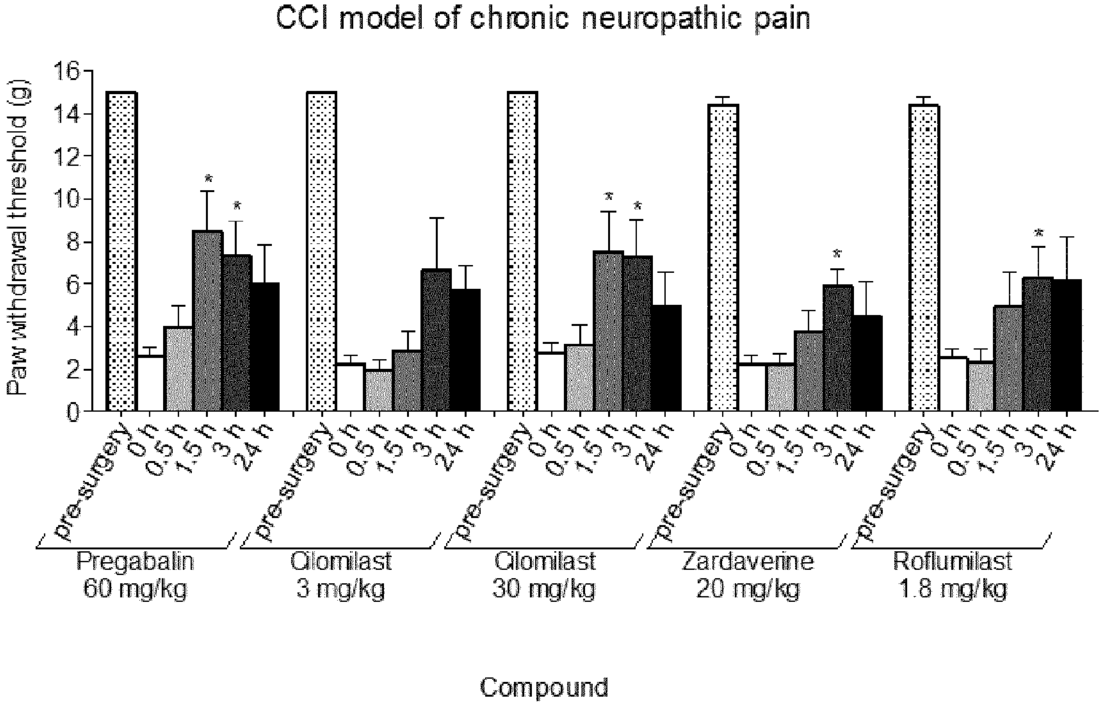


Fig. 23 B

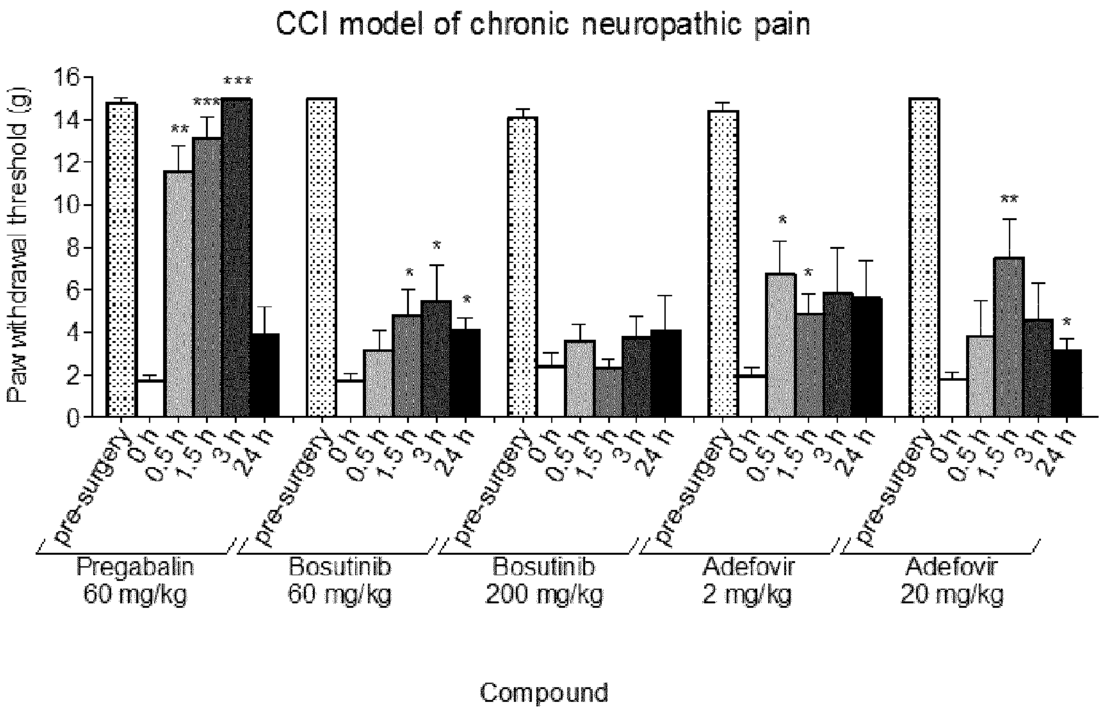

Fig. 23 C

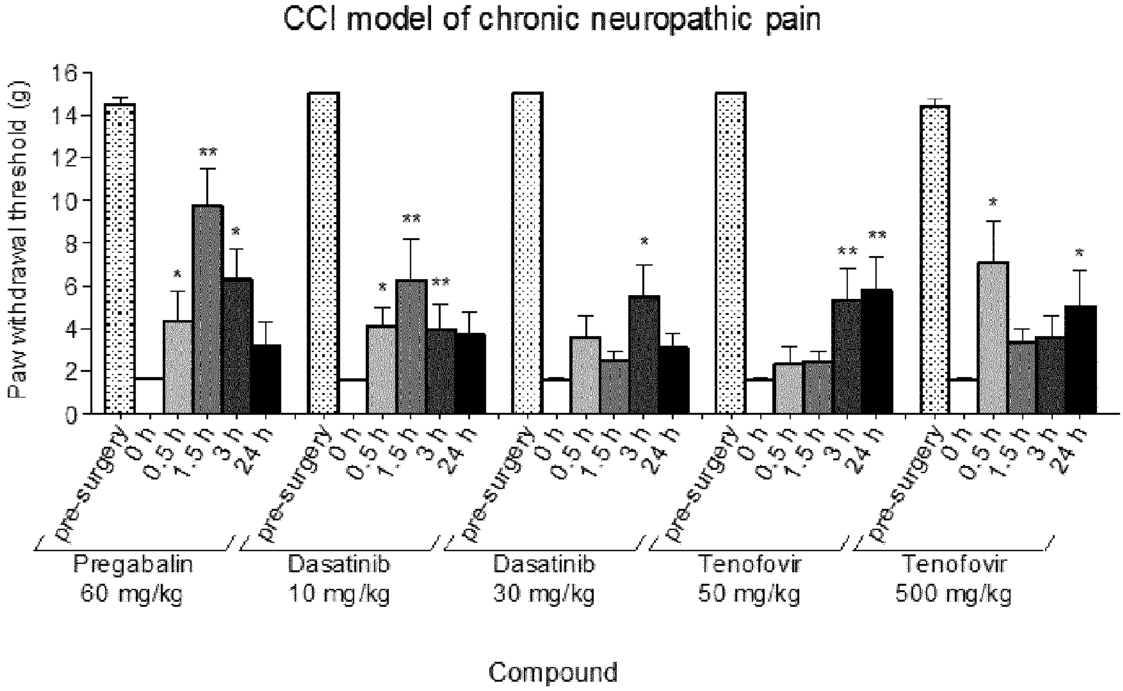


Fig. 24 A

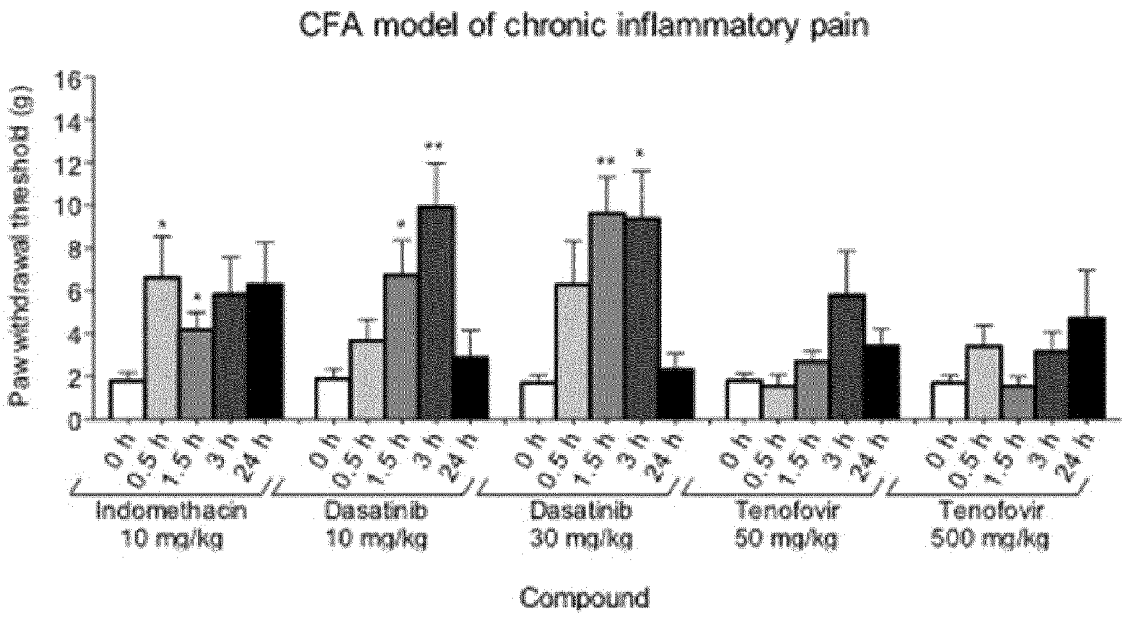

Fig. 24 B

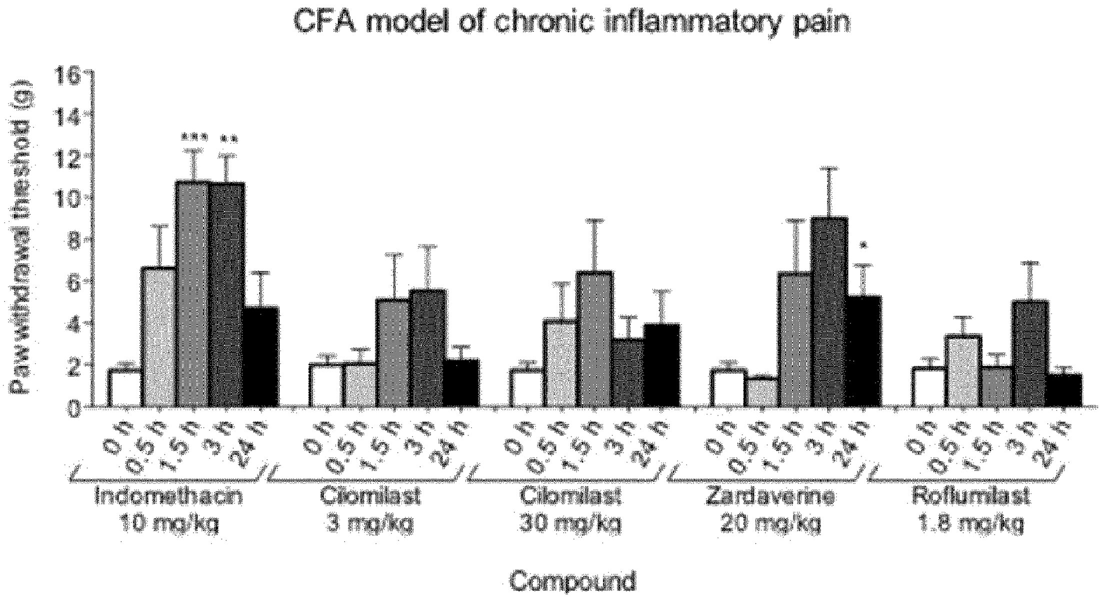


Fig. 24 C

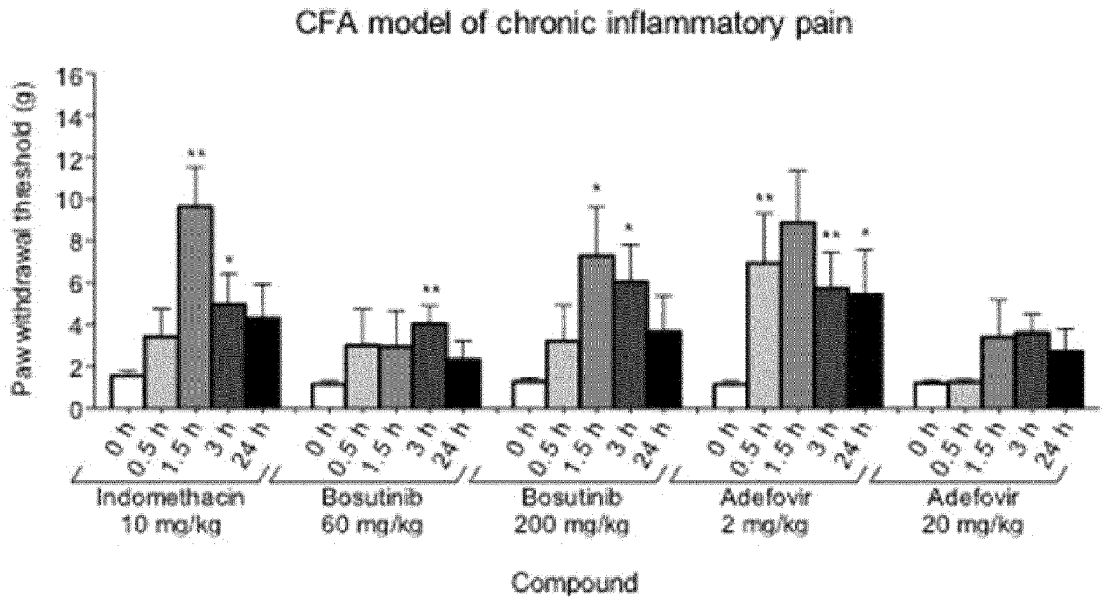

Europäisches Patentamt
European Patent Office
Office européen des brevets

# EUROPEAN SEARCH REPORT

Application Number
EP 13 17 4939

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MATAL JAROSLAV ET AL: "Experimental approaches to studies on drug metabolism and drug interactions in man: interaction of acyclic nucleoside phosphonates with human liver cytochromes P450 and flavin-containing monooxygenase 3.", NEURO ENDOCRINOLOGY LETTERS DEC 2006, vol. 27 Suppl 2, December 2006 (2006-12), pages 27-30, XP009173952, ISSN: 0172-780X<br>* page 29, column 1, paragraph 3-4 *<br>* figures 1,2 *<br>----- | 1-15 | INV.<br>A61K31/675<br>A61K31/4164<br>A61P29/00 |
| A | STOERMER ELKE ET AL: "Benzydamine N-oxidation as an index reaction reflecting FMO activity in human liver microsomes and impact of FMO3 polymorphisms on enzyme activity", BRITISH JOURNAL OF CLINICAL PHARMACOLOGY, vol. 50, no. 6, December 2000 (2000-12), pages 553-561, XP002715836, ISSN: 0306-5251<br>* abstract *<br>* page 556, column 2, paragraph 1 *<br>* figure 5 *<br>----- | 1-15 | TECHNICAL FIELDS SEARCHED (IPC)<br>A61K |
| A | JHAVERI MALHAR A ET AL: "Tenofovir-associated severe bone pain: I cannot walk!", JOURNAL OF THE INTERNATIONAL ASSOCIATION OF PHYSICIANS IN AIDS CARE,, vol. 9, no. 5, September 2010 (2010-09), pages 328-334, XP009155922, ISSN: 1545-1097<br>* page 328, column 1, paragraph 1 *<br>-----<br>-/-- | 1-15 | |

The present search report has been drawn up for all claims

1

| Place of search | Date of completion of the search | Examiner |
|---|---|---|
| Munich | 4 November 2013 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 4939

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HISAMUDDIN IRFAN M ET AL: "Genetic polymorphisms of human flavin-containing monooxygenase 3: implications for drug metabolism and clinical perspectives", PHARMACOGENOMICS, FUTURE MEDICINE LTD, UK, vol. 8, no. 6, June 2007 (2007-06), pages 635-643, XP009166173, ISSN: 1744-8042<br>* the whole document *<br>----- | 1-15 | |
| A | DATABASE EMBASE [Online] ELSEVIER SCIENCE PUBLISHERS, AMSTERDAM, NL; November 2006 (2006-11), PEIXOTO M C ET AL: "Antithyroid drugs for the treatment of graves disease: A randomized clinical trial", XP002715837, Database accession no. EMB-2006580579<br>* abstract *<br>& PEIXOTO M C ET AL: "Antithyroid drugs for the treatment of graves disease: A randomized clinical trial", ENDOCRINOLOGIST 200611 US, vol. 16, no. 6, November 2006 (2006-11), pages 344-348, ISSN: 1051-2144<br>----- | 1-15 | TECHNICAL FIELDS SEARCHED (IPC) |

The present search report has been drawn up for all claims

1

| Place of search | Date of completion of the search | Examiner |
|---|---|---|
| Munich | 4 November 2013 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRODY et al.** Overlap with neuronal precursor genes. Brody T, 2002
- Gene Ontology. *NCBI Gene, http://www.ncbi.nlm.nih.gov/gene*
- **KUZNIAR et al.** *Trends Genet,* November 2008, vol. 24, 539
- **T. SASAKI et al.** *Science,* 11 February 2000, vol. 287, 1040
- **E. PATRUCCO et al.** *Cell,* 06 August 2004, vol. 118, 375
- **I. RACZ et al.** *J Neurosci,* 12 November 2008, vol. 28, 12125
- **DITTERT et al.** *J Neurosci Methods,* 01 August 1998, vol. 82, 195
- **VYKLICKY et al.** *J Physiol,* 15 May 1999, vol. 517, 181
- **ALEMAN, A. ; RUTTEN, G.J. ; SITSKOORN, M.M. ; DAUTZENBERG, G. ; RAMSEY, N.F.** Activation of striate cortex in the absence of visual stimulation: an fMRI study of synesthesia. *Neuroreport,* 2001, vol. 12, 2827-2830
- **ARTHURS, O.J. ; BONIFACE, S.** How well do we understand the neural origins of the fMRI BOLD signal?. *Trends in neurosciences,* 2002, vol. 25, 27-31
- **ATLAS, S.J. ; KELLER, R.B. ; CHANG, Y. ; DEYO, R.A. ; SINGER, D.E.** Surgical and nonsurgical management of sciatica secondary to a lumbar disc herniation: five-year outcomes from the Maine Lumbar Spine Study. *Spine,* 2001, vol. 26, 1179-1187
- **BASBAUM, A.I. ; BAUTISTA, D.M. ; SCHERRER, G. ; JULIUS, D.** Cellular and molecular mechanisms of pain. *Cell,* 2009, vol. 139, 267-284
- **BEAUCHAMP, M.S. ; RO, T.** Neural substrates of sound-touch synesthesia after a thalamic lesion. *J Neurosci,* 2008, vol. 28, 13696-13702
- **CATTERALL, W.A.** Structure and regulation of voltage-gated Ca2+ channels. *Annual review of cell and developmental biology,* 2000, vol. 16, 521-555
- **COSTIGAN, M. ; SCHOLZ, J. ; WOOLF, C.J.** Neuropathic Pain: A Maladaptive Response of the Nervous System to Damage. *Annual review of neuroscience,* 2009
- **COX, J.J. ; REIMANN, F. ; NICHOLAS, A.K. ; THORNTON, G. ; ROBERTS, E. ; SPRINGELL, K. ; KARBANI, G. ; JAFRI, H. ; MANNAN, J. ; RAASHID, Y. et al.** An SCN9A channelopathy causes congenital inability to experience pain. *Nature,* 2006, vol. 444, 894-898
- **CRAWLEY, J.N.** Behavioral phenotyping strategies for mutant mice. *Neuron,* 2008, vol. 57, 809-818
- **DIATCHENKO, L. ; SLADE, G.D. ; NACKLEY, A.G. ; BHALANG, K. ; SIGURDSSON, A. ; BELFER, I. ; GOLDMAN, D. ; XU, K. ; SHABALINA, S.A. ; SHAGIN, D. et al.** Genetic basis for individual variations in pain perception and the development of a chronic pain condition. *Human molecular genetics,* 2005, vol. 14, 135-143
- **DICKMAN, D.K. ; KURSHAN, P.T. ; SCHWARZ, T.L.** Mutations in a Drosophila alpha2delta voltage-gated calcium channel subunit reveal a crucial synaptic function. *J Neurosci,* 2008, vol. 28, 31-38
- **DIETZL, G. ; CHEN, D. ; SCHNORRER, F. ; SU, K.C. ; BARINOVA, Y. ; FELLNER, M. ; GASSER, B. ; KINSEY, K. ; OPPEL, S. ; SCHEIBLAUER, S. et al.** A genome-wide transgenic RNAi library for conditional gene inactivation in Drosophila. *Nature,* 2007, vol. 448, 151-156
- **DUDYCHA, G.J. ; MARTHA M. DUDYCHA.** A Case of Synesthesia: Visual-Pain and Visual-Audition. *Journal of Abnormal and Social Psychology,* 1935, vol. 30, 57-69
- **DWORKIN, R.H. ; O'CONNOR, A.B. ; BACKONJA, M. ; FARRAR, J.T. ; FINNERUP, N.B. ; JENSEN, T.S. ; KALSO, E.A. ; LOESER, J.D. ; MIASKOWSKI, C. ; NURMIKKO, T.J. et al.** Pharmacologic management of neuropathic pain: evidence-based recommendations. *Pain,* 2007, vol. 132, 237-251
- **EROGLU, C. ; ALLEN, N.J. ; SUSMAN, M.W. ; O'ROURKE, N.A. ; PARK, C.Y. ; OZKAN, E. ; CHAKRABORTY, C. ; MULINYAWE, S.B. ; ANNIS, D.S. ; HUBERMAN, A.D. et al.** Gabapentin receptor alpha2delta-1 is a neuronal thrombospondin receptor responsible for excitatory CNS synaptogenesis. *Cell,* 2009, vol. 139, 380-392
- **FIELD, M.J. ; COX, P.J. ; STOTT, E. ; MELROSE, H. ; OFFORD, J. ; SU, T.Z. ; BRAMWELL, S. ; CORRADINI, L. ; ENGLAND, S. ; WINKS, J. et al.** Identification of the alpha2-delta-1 subunit of voltage-dependent calcium channels as a molecular target for pain mediating the analgesic actions of pregabalin. *Proceedings of the National Academy of Sciences of the United States of America,* 2006, vol. 103, 17537-17542

- **HESS, A. ; STILLER, D. ; KAULISCH, T. ; HEIL, P. ; SCHEICH, H.** New insights into the hemodynamic blood oxygenation level-dependent response through combination of functional magnetic resonance imaging and optical recording in gerbil barrel cortex. *J Neurosci,* 2000, vol. 20, 3328-3338
- **HUBBARD, E.M. ; RAMACHANDRAN, V.S.** Neurocognitive mechanisms of synesthesia. *Neuron,* 2005, vol. 48, 509-520
- **HUCHO, T. ; LEVINE, J.D.** Signaling pathways in sensitization: toward a nociceptor cell biology. *Neuron,* 2007, vol. 55, 365-376
- **KERNAN, M. ; COWAN, D. ; ZUKER, C.** Genetic dissection of mechanosensory transduction: mechanoreception-defective mutations of Drosophila. *Neuron,* 1994, vol. 12, 1195-1206
- **KNABL, J. ; WITSCHI, R. ; HOSL, K. ; REINOLD, H. ; ZEILHOFER, U.B. ; AHMADI, S. ; BROCKHAUS, J. ; SERGEJEVA, M. ; HESS, A. ; BRUNE, K. et al.** Reversal of pathological pain through specific spinal GABAA receptor subtypes. *Nature,* 2008, vol. 451, 330-334
- **KOESTER, S.E. ; INSEL, T.R.** Mouse maps of gene expression in the brain. *Genome biology,* 2007, vol. 8, 212
- **KURSHAN, P.T. ; OZTAN, A. ; SCHWARZ, T.L.** Presynaptic alpha(2)delta-3 is required for synaptic morphogenesis independent of its Ca(2+)-channel functions. *Nature neuroscience,* 2009, vol. 12, 1415-1423
- **LEE, T. ; LUO, L.** Mosaic analysis with a repressible cell marker for studies of gene function in neuronal morphogenesis. *Neuron,* 1999, vol. 22, 451-461
- **LUMPKIN, E.A. ; CATERINA, M.J.** Mechanisms of sensory transduction in the skin. *Nature,* 2007, vol. 445, 858-865
- **LY, C.V. ; YAO, C.K. ; VERSTREKEN, P. ; OHYAMA, T. ; BELLEN, H.J.** straightjacket is required for the synaptic stabilization of cacophony, a voltage-gated calcium channel alpha1 subunit. *The Journal of cell biology,* 2008, vol. 181, 157-170
- **MANEV, H. ; DIMITRIJEVIC, N.** Drosophila model for in vivo pharmacological analgesia research. *European journal of pharmacology,* 2004, vol. 491, 207-208
- **MELZACK, R.** From the gate to the neuromatrix. *Pain,* 1999, vol. 6, 121-126
- **OGAWA, S. ; LEE, T.M. ; KAY, A.R. ; TANK, D.W.** Brain magnetic resonance imaging with contrast dependent on blood oxygenation. *Proceedings of the National Academy of Sciences of the United States of America,* 1990, vol. 87, 9868-9872
- **PETERSEN, C.C.** The functional organization of the barrel cortex. *Neuron,* 2007, vol. 56, 339-355
- **PITCHER, G.M. ; YASHPAL, K. ; CODERRE, T.J. ; HENRY, J.L.** Mechanisms underlying antinociception provoked by heterosegmental noxious stimulation in the rat tail-flick test. *Neuroscience,* 1995, vol. 65, 273-281
- **PREMKUMAR, L.S.** Targeting TRPV1 as an alternative approach to narcotic analgesics to treat chronic pain conditions. *The AAPS journal,* 2010, vol. 12, 361-370
- **PRICE, D.D.** Psychological and neural mechanisms of the affective dimension of pain. *Science,* 2000, vol. 288, 1769-1772
- **SHMUEL, A. ; YACOUB, E. ; PFEUFFER, J. ; DE MOORTELE, P.F. ; ADRIANY, G. ; HU, X. ; UGURBIL, K.** Sustained negative BOLD, blood flow and oxygen consumption response and its coupling to the positive response in the human brain. *Neuron,* 2002, vol. 36, 1195-1210
- **SILVA, A.C. ; LEE, J.H. ; AOKI, I. ; KORETSKY, A.P.** Manganese-enhanced magnetic resonance imaging (MEMRI): methodological and practical considerations. *NMR in biomedicine,* 2004, vol. 17, 532-543
- **SOKABE, T. ; TSUJIUCHI, S. ; KADOWAKI, T. ; TOMINAGA, M.** Drosophila painless is a Ca2+-requiring channel activated by noxious heat. *J Neurosci,* 2008, vol. 28, 9929-9938
- **STUURMAN, N. ; DELBECQUE, J.P. ; CALLAERTS, P. ; AEBI, U.** Ectopic overexpression of Drosophila lamin C is stage-specific lethal. *Experimental cell research,* 1999, vol. 248, 350-357
- **TEGEDER, I. ; COSTIGAN, M. ; GRIFFIN, R.S. ; ABELE, A. ; BELFER, I. ; SCHMIDT, H. ; EHNERT, C. ; NEJIM, J. ; MARIAN, C. ; SCHOLZ, J. et al.** GTP cyclohydrolase and tetrahydrobiopterin regulate pain sensitivity and persistence. *Nature medicine,* 2006, vol. 12, 1269-1277
- **THULBORN, K.R. ; WATERTON, J.C. ; MATTHEWS, P.M. ; RADDA, G.K.** Oxygenation dependence of the transverse relaxation time of water protons in whole blood at high field. *Biochimica et biophysica acta,* 1992, vol. 714, 265-270
- **TRACEY, I. ; MANTYH, P.W.** The cerebral signature for pain perception and its modulation. *Neuron,* 2007, vol. 55, 377-391
- **TRACEY, W.D., JR. ; WILSON, R.I. ; LAURENT, G. ; BENZER, S.** painless, a Drosophila gene essential for nociception. *Cell,* 2003, vol. 113, 261-273
- **TREEDE, R.D. ; KENSHALO, D.R. ; GRACELY, R.H. ; JONES, A.K.** The cortical representation of pain. *Pain,* 1999, vol. 79, 105-111
- **VENKEN, K.J. ; CARLSON, J.W. ; SCHULZE, K.L. ; PAN, H. ; HE, Y. ; SPOKONY, R. ; WAN, K.H. ; KORIABINE, M. ; JONG, P.J. ; WHITE, K.P et al.** Versatile P[acman] BAC libraries for transgenesis studies in Drosophila melanogaster. *Nature methods,* 2009, vol. 6, 431-434

- **XU, S.Y. ; CANG, C.L. ; LIU, X.F. ; PENG, Y.Q. ; YE, Y.Z. ; ZHAO, Z.Q. ; GUO, A.K.** Thermal nociception in adult Drosophila: behavioral characterization and the role of the painless gene. *Genes, brain, and behavior,* 2006, vol. 5, 602-613
- **BENZER, S.** BEHAVIORAL MUTANTS OF Drosophila ISOLATED BY COUNTERCURRENT DISTRIBUTION. *Proceedings of the National Academy of Sciences of the United States of America,* 1967, vol. 58, 1112-1119
- **BRODY, T. ; STIVERS, C. ; NAGLE, J. ; ODENWALD, W.F.** Identification of novel Drosophila neural precursor genes using a differential embryonic head cDNA screen. *Mechanisms of development,* 2002, vol. 113, 41-59
- **COUTO, A. ; ALENIUS, M. ; DICKSON, B.J.** Molecular, anatomical, and functional organization of the Drosophila olfactory system. *Curr Biol,* 2005, vol. 15, 1535-1547
- **GRIGLIATTI, T.A. ; HALL, L. ; ROSENBLUTH, R. ; SUZUKI, D.T.** Temperature-sensitive mutations in Drosophila melanogaster. XIV. A selection of immobile adults. *Mol Gen Genet,* 1973, vol. 120, 107-114
- **HAYASHI, S. ; ITO, K. ; SADO, Y. ; TANIGUCHI, M. ; AKIMOTO, A. ; TAKEUCHI, H. ; AIGAKI, T. ; MATSUZAKI, F. ; NAKAGOSHI, H. ; TANIMURA, T. et al.** GETDB, a database compiling expression patterns and molecular locations of a collection of Gal4 enhancer traps. *Genesis,* 2002, vol. 34, 58-61
- **JOURDAN, D. ; ARDID, D. ; ESCHALIER, A.** Automated behavioural analysis in animal pain studies. *Pharmacol Res,* 2001, vol. 43, 103-110
- **MUMMERY-WIDMER, J.L. ; YAMAZAKI, M. ; STOEGER, T. ; NOVATCHKOVA, M. ; BHALERAO, S. ; CHEN, D. ; DIETZL, G. ; DICKSON, B.J. ; KNOBLICH, J.A.** Genome-wide analysis of Notch signalling in Drosophila by transgenic RNAi. *Nature,* 2009
- **OBREJA, O. ; BIASIO, W. ; ANDRATSCH, M. ; LIPS, K.S. ; RATHEE, P.K. ; LUDWIG, A. ; ROSE-JOHN, S. ; KRESS, M.** Fast modulation of heat-activated ionic current by proinflammatory interleukin 6 in rat sensory neurons. *Brain,* 2005, vol. 128, 1634-1641
- **OBREJA, O. ; RATHEE, P.K. ; LIPS, K.S. ; DISTLER, C. ; KRESS, M.** IL-1 beta potentiates heat-activated currents in rat sensory neurons: involvement of IL-1RI, tyrosine kinase, and protein kinase C. *Faseb J,* 2002, vol. 16, 1497-1503
- **SIDDIQI, O. ; BENZER, S.** Neurophysiological defects in temperature-sensitive paralytic mutants of Drosophila melanogaster. *Proceedings of the National Academy of Sciences of the United States of America,* 1976, vol. 73, 3253-3257
- **THIBAULT, S.T. ; SINGER, M.A. ; MIYAZAKI, W.Y. ; MILASH, B. ; DOMPE, N.A. ; SINGH, C.M. ; BUCHHOLZ, R. ; DEMSKY, M. ; FAWCETT, R. ; FRANCIS-LANG, H.L. et al.** A complementary transposon tool kit for Drosophila melanogaster using P and piggyBac. *Nature genetics,* 2004, vol. 36, 283-287
- **VENKEN, K.J. ; CARLSON, J.W. ; SCHULZE, K.L. ; PAN, H. ; HE, Y. ; SPOKONY, R. ; WAN, K.H. ; KORIABINE, M. ; JONG, P.J. ; WHITE, K.P. et al.** Versatile P[acman] BAC libraries for transgenesis studies in Drosophila melanogaster. *Nature methods,* 2009, vol. 6, 431-434
- **VERSTREKEN, P. ; KOH, T.W. ; SCHULZE, K.L. ; ZHAI, R.G. ; HIESINGER, P.R. ; ZHOU, Y. ; MEHTA, S.Q. ; CAO, Y. ; ROOS, J. ; BELLEN, H.J.** Synaptojanin is recruited by endophilin to promote synaptic vesicle uncoating. *Neuron,* 2003, vol. 40, 733-748
- **VOSSHALL, L.B. ; WONG, A.M. ; AXEL, R.** An olfactory sensory map in the fly brain. *Cell,* 2000, vol. 102, 147-159
- **ZAYKIN, D.V. ; WESTFALL, P.H. ; YOUNG, S.S. ; KARNOUB, M.A. ; WAGNER, M.J. ; EHM, M.G.** Testing association of statistically inferred haplotypes with discrete and continuous traits in samples of unrelated individuals. *Human heredity,* 2002, vol. 53, 79-91
- **KUZNIAR, R. C. VAN HAM ; S. PONGOR ; J. A. LEUNISSEN.** *Trends Genet,* November 2008, vol. 24, 539
- **SASAKI et al.** *Science,* 11 February 2000, vol. 287, 1040
- **PATRUCCO et al.** *Cell,* 06 August 2004, vol. 118, 375
- **RACZ et al.** *J Neurosci,* 12 November 2008, vol. 28, 12125
- **L. VYKLICKY et al.** *J Physiol,* 15 May 1999, vol. 517, 181
- **BENNETT G.J. ; XIE Y.K.** *Pain,* 1988, vol. 33, 87-107
- **BLACKBURN-MUNRO G ; ERICHSEN H.K.** *Current Pharm Des,* 2005, vol. 11, 2961-2975
- **BUTLER, S.H. ; GODEFROY, F. ; BESSON, J.-M. ; WEIL-FUGAZZA, J.** *Pain,* 1992, vol. 48, 73-81
- **HEGEN, M. ; KEITH, J.C. ; COLLINS, M. ; NICKERSON-NUTTER, C.L.** *Ann Rheum Dis,* 2008, vol. 67, 1505-1515
- **HSIEH, G.C. ; CHANDRAN, P. ; SALYERS, A.K. ; PAI, M. ; ZHU, C.Z. ; WENSINK, E.J. ; WITTE, D.G. ; MILLER, T.R. ; MIKUSA, J.P ; BAKER, S.J.** *Pharmacol Biochem Behav,* 2010, vol. 95, 41-50
- **KONTINEN V.K. ; MEERT T.F.** Prog in Pain Res Management. IASP press, 2003, vol. 24, 489-498
- **LABUDA, C.J. ; LITTLE, P.** *J Neurosci Meths,* 2005, vol. 144, 175-181

- **MEOTTI, F.C. ; MISSAU, F.C. ; FERREIRA, J. ; PIZZOLATTI, M.G. ; MIZUZAKI, C. ; NOGUEIRA, C.W. ; SANTOS, A.R.S.** *Biochem Pharmacol,* vol. 72, 1707-17134
- **MILLAN, M.J. ; CZLONKOWSKI, A. ; MORRIS, B. ; STEIN, C. ; ARENDT, R. ; HUBER, A. et al.** *Pain,* 1988, vol. 35, 299-312
- **SCHAFERS, M. ; MARZINIAK, M. ; SORKIN, L.S. ; YAKSH, T.L. ; SOMMER C.** *Exp Neurol .,* 2004, vol. 185, 160-168
- **STATON, P.C. ; WILSON, A.W. ; BOUNTRA, C. ; CHESSELL, I.P. ; DAY, N.C.** *Eur J Pain,* 2007, vol. 11, 283-289
- **TAKAHASHI M. ; KAWAGUCHI, M. ; SHIMADA, K ; KONISHI, N. ; FURUYA H. ; NAKASHIMA, T.** *Neurosci Letts,* 2004, vol. 356, 37-40
- **WHITESIDE, G.T. ; ADEDOYIN, A., LEVENTHAL, L.** *Neuro-pharmacology,* 2008, vol. 54, 767-775
- **JETT, M.F. ; RAMESHA, C.S. ; BROWN, C.D. ; CHIU, S. ; EMMETT, C. ; VORONIN, T. ; SUN, T. ; O'YANG, C. ; HUNTER, J.C. ; EGLEN, R.M.** Characterization of the analgesic and anti-inflammatory activities of ketorolac and its enantiomers in the rat. *J Pharmacol Exp Ther,* 1999, vol. 288 (3), 1288-97
- **DA SILVA FILHO A.A. ; ANDRADE E SILVA M.L. ; CARVALHO J.C. ; BASTOS J.K.** Evaluation of analgesic and anti-inflammatory activities of Nectandra megapotamica (Lauraceae) in mice and rats. *J Pharm Pharmacol.,* 2004, vol. 56 (9), 1179-84
- **CARD, G.L. ; ENGLAND, B.P. ; SUZUKI, Y. ; FONG, D. ; POWELL, B. ; LEE, B. ; LUU, C. ; TABRIZIZAD, M. ; GILLETTE, S. ; IBRAHIM, P.N.** Structural Basis for the Activity of Drugs that Inhibit Phosphodiesterases. *Structure,* 2004, vol. 12 (12), 2233-47
- **MEHATS, C. ; ANDERSEN, C.B. ; FILOPANTI, M. ; JIN, S.L. ; CONTI, M.** Cyclic nucleotide phosphodiesterases and their role in endocrine cell signaling. *Trends Endocrinol. Metab.,* 2002, vol. 13, 29-35
- **AREZZO, J.C ; ROSENSTOCK, J. ; LAMOREAUX, L. ; PAUER, L.** Efficacy and safetyof pregabalin 600 mg/d for treating painful diabetic peripheral neuropathy: a double-blind placebo-controlled trial. *BMC Neurology,* 2008, vol. 16 (8), 33
- **KARAMAN, M.W. ; HERRGARD, S. ; TREIBER, D.K. ; GALLANT, P. ; ATTERIDGE, C.E. ; CAMPBELL, B.T. ; CHAN, K.W. ; CICERI, P. ; DAVIS, M.I. ; EDEEN, P.T.** A quantitative analysis of kinase inhibitor selectivity. *Nat Biotechnol,* January 2008, vol. 26 (1), 127-32
- **NEELY et al.** A genome-wide Drosophila screen for heat nociception identifies $\alpha 2\delta 3$ as an evolutionarily conserved pain gene. *Cell,* 12 November 2010, vol. 143 (4), 628-38